# EUROPEAN PATENT SPECIFICATION

(11) **EP 1 761 650 B1**
(45) Date of publication and mention of the grant of the patent: **04.03.2009**
(21) Application number: 05766789.1
(22) Date of filing: 30.06.2005
(51) Int. Cl.: C12Q 1/68

(54) **HUMAN AUTISM SUSCEPTIBILITY GENE ENCODING PRKCB1 AND USES THEREOF**
FÜR PRKCB1 KODIERENDES HUMANES AUTISMUS-SUSZEPTIBILITÄTSGEN UND DESSEN VERWENDUNG
GENE HUMAIN DE PREDISPOSITION A L'AUTISME CODANT PRKCB1 ET PROCEDES D'UTILISATION

(30) Priority: 01.07.2004 US 584132 P
(43) Date of publication of application: 14.03.2007
(73) Proprietor: Integragen, 91000 EVRY (FR)
(72) Inventor: PHILIPPI, Anne, F-77310 St. Fargeau Ponthierry (FR); ROUSSEAU, Francis, F-91600 Savignay sur Orge (FR); BROOKS, Peter, 75007 Paris (FR); HAGER, Jörg, F-91540 Mennecy (FR)
(74) Representative: Chajmowicz, Marion
(86) International application number: PCT/IB2005/002381
(87) International publication number: WO 2006/003523

(56) References cited:
- WO-A-20/04037986
- MONACO A P: "A genomewide screen for autism: Strong evidence for linkage to chromosomes 2q, 7q, and 16p: International molecular genetic study of autism consortium, (IMGSAC)" AMERICAN JOURNAL OF HUMAN GENETICS 2001 UNITED STATES, vol. 69, no. 3, 2001, pages 570-581, XP002347957 ISSN: 0002-9297
- KUBO K ET AL: "PRIMARY STRUCTURES OF HUMAN PROTEIN KINASE C BETAI AND BETAII DIFFER ONLY IN THEIR C-TERMINAL SEQUENCES" FEBS LETTERS, ELSEVIER, AMSTERDAM, NL, vol. 223, no. 1, October 1987 (1987-10), pages 138-142, XP001042200 ISSN: 0014-5793
- PHILIPPI A ET AL: "Haplotypes in the gene encoding protein kinase c-beta (PRKCB1) on chromosome 16 are associated with autism." MOLECULAR PSYCHIATRY. OCT 2005, vol. 10, no. 10, October 2005 (2005-10), pages 950-960, XP002347958 ISSN: 1359-4184

### SEQUENCE LISTING

<110> INTEGRAGEN
<120> HUMAN AUTISM SUSCEPTIBILITY GENE ENCODING PRKCB1 AND USES THEREOF
<130> B0301WO
<150> US 60/584,132
   <151> 2004-07-01
<160> 63
<170> PatentIn version 3.1
<210> 1
   <211> 3411
   <212> DNA
   <213> Homo sapiens
<220>
   <221> CDS
   <222> (176)..(2197)
   <223>
<400> 1
   gggcgagtga cagccccggc tccgcgcgcc gcggccgcca gagccggcgc aggggaagcg      60
   cccgcggccc cgggtgcagc agcggccgcc gcctcccgcg cctccccggc ccgcagcccg     120
   cggtcccgcg gccccggggc cggcacctct cgggctccgg ctccccgcgc gcaag atg     178
                                                                 Met
                                                                 1
   gct gac ccg gct gcg ggg ccg ccg ccg agc gag ggc gag gag agc acc      226
   Ala Asp Pro Ala Ala Gly Pro Pro Pro Ser Glu Gly Glu Glu Ser Thr
               5                   10                  15
   gtg cgc ttc gcc cgc aaa ggc gcc ctc cgg cag aag aac gtg cat gag      274
   Val Arg Phe Ala Arg Lys Gly Ala Leu Arg Gln Lys Asn Val His Glu
           20                  25                  30
   gtc aag aac cac aaa ttc acc gcc cgc ttc ttc aag cag ccc acc ttc      322
   Val Lys Asn His Lys Phe Thr Ala Arg Phe Phe Lys Gln Pro Thr Phe
       35                  40                  45
   tgc agc cac tgc acc gac ttc atc tgg ggc ttc ggg aag cag gga ttc      370
   Cys Ser His Cys Thr Asp Phe Ile Trp Gly Phe Gly Lys Gln Gly Phe
   50                  55                  60                  65
   cag tgc caa gtt tgc tgc ttt gtg gtg cac aag cgg tgc cat gaa ttt      418
   Gln Cys Gln Val Cys Cys Phe Val Val His Lys Arg Cys His Glu Phe
                   70                  75                  80
   gtc aca ttc tcc tgc cct ggc gct gac aag ggt cca gcc tcc gat gac      466
   Val Thr Phe Ser Cys Pro Gly Ala Asp Lys Gly Pro Ala Ser Asp Asp
               85                  90                  95
   ccc cgc agc aaa cac aag ttt aag atc cac acg tac tcc agc ccc acg      514
   Pro Arg Ser Lys His Lys Phe Lys Ile His Thr Tyr Ser Ser Pro Thr
           100                 105                 110
   ttt tgt gac cac tgt ggg tca ctg ctg tat gga ctc atc cac cag ggg      562
   Phe Cys Asp His Cys Gly Ser Leu Leu Tyr Gly Leu Ile His Gln Gly
       115                 120                 125
   atg aaa tgt gac acc tgc atg atg aat gtg cac aag cgc tgc gtg atg      610
   Met Lys Cys Asp Thr Cys Met Met Asn Val His Lys Arg Cys Val Met
   130                 135                 140                 145
   aat gtt ccc agc ctg tgt ggc acg gac cac acg gag cgc cgc ggc cgc      658
   Asn Val Pro Ser Leu Cys Gly Thr Asp His Thr Glu Arg Arg Gly Arg
                   150                 155                 160
   atc tac atc cag gcc cac atc gac agg gac gtc ctc att gtc ctc gta      706
   Ile Tyr Ile Gln Ala His Ile Asp Arg Asp Val Leu Ile Val Leu Val
               165                 170                 175
   aga gat gct aaa aac ctt gta cct atg gac ccc aat ggc ctg tca gat      754
   Arg Asp Ala Lys Asn Leu Val Pro Met Asp Pro Asn Gly Leu Ser Asp
           180                 185                 190
   ccc tac gta aaa ctg aaa ctg att ccc gat ccc aaa agt gag agc aaa      802
   Pro Tyr Val Lys Leu Lys Leu Ile Pro Asp Pro Lys Ser Glu Ser Lys
       195                 200                 205
   cag aag acc aaa acc atc aaa tgc tcc ctc aac cct gag tgg aat gag      850
   Gln Lys Thr Lys Thr Ile Lys Cys Ser Leu Asn Pro Glu Trp Asn Glu
   210                 215                 220                 225
   aca ttt aga ttt cag ctg aaa gaa tcg gac aaa gac aga aga ctg tca      898
   Thr Phe Arg Phe Gln Leu Lys Glu Ser Asp Lys Asp Arg Arg Leu Ser
                   230                 235                 240
   gta gag att tgg gat tgg gat ttg acc agc agg aat gac ttc atg gga      946
   Val Glu Ile Trp Asp Trp Asp Leu Thr Ser Arg Asn Asp Phe Met Gly
               245                 250                 255
   tct ttg tcc ttt ggg att tct gaa ctt cag aaa gcc agt gtt gat ggc      994
   Ser Leu Ser Phe Gly Ile Ser Glu Leu Gln Lys Ala Ser Val Asp Gly
           260                 265                 270
   tgg ttt aag tta ctg agc cag gag gaa ggc gag tac ttc aat gtg cct     1042
   Trp Phe Lys Leu Leu Ser Gln Glu Glu Gly Glu Tyr Phe Asn Val Pro
       275                 280                 285
   gtg cca cca gaa gga agt gag gcc aat gaa gaa ctg cgg cag aaa ttt     1090
   Val Pro Pro Glu Gly Ser Glu Ala Asn Glu Glu Leu Arg Gln Lys Phe
   290                 295                 300                 305
   gag agg gcc aag atc agt cag gga acc aag gtc ccg gaa gaa aag acg     1138
   Glu Arg Ala Lys Ile Ser Gln Gly Thr Lys Val Pro Glu Glu Lys Thr
                   310                 315                 320
   acc aac act gtc tcc aaa ttt gac aac aat ggc aac aga gac cgg atg     1186
   Thr Asn Thr Val Ser Lys Phe Asp Asn Asn Gly Asn Arg Asp Arg Met
               325                 330                 335
   aaa ctg acc gat ttt aac ttc cta atg gtg ctg ggg aaa ggc agc ttt      1234
   Lys Leu Thr Asp Phe Asn Phe Leu Met Val Leu Gly Lys Gly Ser Phe
           340                 345                 350
   ggc aag gtc atg ctt tca gaa cga aaa ggc aca gat gag ctc tat gct      1282
   Gly Lys Val Met Leu Ser Glu Arg Lys Gly Thr Asp Glu Leu Tyr Ala
       355                 360                 365
   gtg aag atc ctg aag aag gac gtt gtg atc caa gat gat gac gtg gag      1330
   Val Lys Ile Leu Lys Lys Asp Val Val Ile Gln Asp Asp Asp Val Glu
   370                 375                 380                 385
   tgc act atg gtg gag aag cgg gtg ttg gcc ctg cct ggg aag ccg ccc      1378
   Cys Thr Met Val Glu Lys Arg Val Leu Ala Leu Pro Gly Lys Pro Pro
                   390                 395                 400
   ttc ctg acc cag ctc cac tcc tgc ttc cag acc atg gac cgc ctg tac      1426
   Phe Leu Thr Gln Leu His Ser Cys Phe Gln Thr Met Asp Arg Leu Tyr
               405                 410                 415
   ttt gtg atg gag tac gtg aat ggg ggc gac ctc atg tat cac atc cag      1474
   Phe Val Met Glu Tyr Val Asn Gly Gly Asp Leu Met Tyr His Ile Gln
           420                 425                 430
   caa gtc ggc cgg ttc aag gag ccc cat gct gta ttt tac gct gca gaa      1522
   Gln Val Gly Arg Phe Lys Glu Pro His Ala Val Phe Tyr Ala Ala Glu
       435                 440                 445
   att gcc atc ggt ctg ttc ttc tta cag agt aag ggc atc att tac cgt      1570
   Ile Ala Ile Gly Leu Phe Phe Leu Gln Ser Lys Gly Ile Ile Tyr Arg
   450                 455                 460                 465
   gac cta aaa ctt gac aac gtg atg ctc gat tct gag gga cac atc aag      1618
   Asp Leu Lys Leu Asp Asn Val Met Leu Asp Ser Glu Gly His Ile Lys
                   470                 475                 480
   att gcc gat ttt ggc atg tgt aag gaa aac atc tgg gat ggg gtg aca      1666
   Ile Ala Asp Phe Gly Met Cys Lys Glu Asn Ile Trp Asp Gly Val Thr
               485                 490                 495
   acc aag aca ttc tgt ggc act cca gac tac atc gcc ccc gag ata att      1714
   Thr Lys Thr Phe Cys Gly Thr Pro Asp Tyr Ile Ala Pro Glu Ile Ile
           500                 505                 510
   gct tat cag ccc tat ggg aag tcc gtg gat tgg tgg gca ttt gga gtc      1762
   Ala Tyr Gln Pro Tyr Gly Lys Ser Val Asp Trp Trp Ala Phe Gly Val
       515                 520                 525
   ctg ctg tat gaa atg ttg gct ggg cag gca ccc ttt gaa ggg gag gat      1810
   Leu Leu Tyr Glu Met Leu Ala Gly Gln Ala Pro Phe Glu Gly Glu Asp
   530                 535                 540                 545
   gaa gat gaa ctc ttc caa tcc atc atg gaa cac aac gta gcc tat ccc      1858
   Glu Asp Glu Leu Phe Gln Ser Ile Met Glu His Asn Val Ala Tyr Pro
                   550                 555                 560
   aag tct atg tcc aag gaa gct gtg gcc atc tgc aaa ggg ctg atg acc      1906
   Lys Ser Met Ser Lys Glu Ala Val Ala Ile Cys Lys Gly Leu Met Thr
               565                 570                 575
   aaa cac cca ggc aaa cgt ctg ggt tgt gga cct gaa ggc gaa cgt gat      1954
   Lys His Pro Gly Lys Arg Leu Gly Cys Gly Pro Glu Gly Glu Arg Asp
           580                 585                 590
   atc aaa gag cat gca ttt ttc cgg tat att gat tgg gag aaa ctt gaa      2002
   Ile Lys Glu His Ala Phe Phe Arg Tyr Ile Asp Trp Glu Lys Leu Glu
       595                 600                 605
   cgc aaa gag atc cag ccc cct tat aag cca aaa gct tgt ggg cga aat      2050
   Arg Lys Glu Ile Gln Pro Pro Tyr Lys Pro Lys Ala Cys Gly Arg Asn
   610                 615                 620                 625
   gct gaa aac ttc gac cga ttt ttc acc cgc cat cca cca gtc cta aca      2098
   Ala Glu Asn Phe Asp Arg Phe Phe Thr Arg His Pro Pro Val Leu Thr
                   630                 635                 640
   cct ccc gac cag gaa gtc atc agg aat att gac caa tca gaa ttc gaa      2146
   Pro Pro Asp Gln Glu Val Ile Arg Asn Ile Asp Gln Ser Glu Phe Glu
               645                 650                 655
   gga ttt tcc ttt gtt aac tct gaa ttt tta aaa ccc gaa gtc aag agc      2194
   Gly Phe Ser Phe Val Asn Ser Glu Phe Leu Lys Pro Glu Val Lys Ser
           660                 665                 670
   taa gtagatgtgt agatctccgt ccttcatttc tgtcattcaa gctcaacggc          2247
   tattgtggtg acatttttat gtttttcatt gccaagttgc atccatgttt gattttctga    2307
   tgagactaga gtgacagtgt ttcagaaccc aaatgtcctc aggtagtttg gagcatctct    2367
   atgagatggg attatgcaga tggcctatgg aaaatgcagc tgcataatta acacattatc    2427
   aaagtcctct tacaatttat tttccgcagc atgtcagcta agtagaccca atggggagag    2487
   aaaatgcctg ctttctttcc ctctttttct gcactgccat attcaccccc aaccatccaa    2547
   tctgtggata attggatgtt agcggtactc ttccacttcc gggcctggag cttggcttgt    2607
   atccaagtgt atggttgctt tgcctaagag gaatccctct atttcacctg ttctggaggc    2667
   accagacctt gaaaagaaca tgctcaaaat aaaatgttat ctgttatttt tgtaaactca    2727
   aagttaagat gatcaaagtt ctaaaattcc aagaatgtgc ttttagacgg tctcaatcta    2787
   aaagcacttc aaggggtcaa agggcaacca gcttgggtgc tacctcagtg ttgtagtttc    2847
   tgatacttta tgtctttgct caccctcatc cccaaactac ttgaaaaggg catttggcac    2907
   cactctctga aacaacacag tcactctagc aaggccccca aagggccctg gttttacatt    2967
   acatttcaaa ctttatttgc tttggggttt tgtttctgtt gttgttcaaa tgcaaaaaaa    3027
   agaaaaaaaa agaaaaaaaa aggtgactca cattgttaca catgctttaa aatatgtatt    3087
   caaatgttat taaccacaat gacgacctgc tttgatttaa ccaagaagac ggctgcggag    3147
   cctagcagac tcaggcctgt gggaatggga tttgttacaa atctaggttt gttactggct    3207
   tcagaaagct aattaagtgc tctgaaaaag acaccgtttc ttgaaacaaa gatggttgta    3267
   ttcctcactt tgatgttgtt ttgcaagatg tttgtggaaa tgttcatttg tatctggatc    3327
   tctgttatgt gccatttttc ttctagcatc gagatacaat aaaaaaaaaa aaaaagaaaa    3387
   aaaaaaaaaa aaaaaaaaaa aaaa                                          3411
<210> 2
   <211> 673
   <212> PRT
   <213> Homo sapiens
<400> 2
   Met Ala Asp Pro Ala Ala Gly Pro Pro Pro Ser Glu Gly Glu Glu Ser
   1               5                   10                  15
   Thr Val Arg Phe Ala Arg Lys Gly Ala Leu Arg Gln Lys Asn Val His
               20                  25                  30
   Glu Val Lys Asn His Lys Phe Thr Ala Arg Phe Phe Lys Gln Pro Thr
           35                  40                  45
   Phe Cys Ser His Cys Thr Asp Phe Ile Trp Gly Phe Gly Lys Gln Gly
       50                  55                  60
   Phe Gln Cys Gln Val Cys Cys Phe Val Val His Lys Arg Cys His Glu
   65                  70                  75                  80
   Phe Val Thr Phe Ser Cys Pro Gly Ala Asp Lys Gly Pro Ala Ser Asp
                   85                  90                  95
   Asp Pro Arg Ser Lys His Lys Phe Lys Ile His Thr Tyr Ser Ser Pro
               100                 105                 110
   Thr Phe Cys Asp His Cys Gly Ser Leu Leu Tyr Gly Leu Ile His Gln
           115                 120                 125
   Gly Met Lys Cys Asp Thr Cys Met Met Asn Val His Lys Arg Cys Val
       130                 135                 140
   Met Asn Val Pro Ser Leu Cys Gly Thr Asp His Thr Glu Arg Arg Gly
   145                 150                 155                 160
   Arg Ile Tyr Ile Gln Ala His Ile Asp Arg Asp Val Leu Ile Val Leu
                   165                 170                 175
   Val Arg Asp Ala Lys Asn Leu Val Pro Met Asp Pro Asn Gly Leu Ser
               180                 185                 190
   Asp Pro Tyr Val Lys Leu Lys Leu Ile Pro Asp Pro Lys Ser Glu Ser
           195                 200                 205
   Lys Gln Lys Thr Lys Thr Ile Lys Cys Ser Leu Asn Pro Glu Trp Asn
       210                 215                 220
   Glu Thr Phe Arg Phe Gln Leu Lys Glu Ser Asp Lys Asp Arg Arg Leu
   225                 230                 235                 240
   Ser Val Glu Ile Trp Asp Trp Asp Leu Thr Ser Arg Asn Asp Phe Met
                   245                 250                 255
   Gly Ser Leu Ser Phe Gly Ile Ser Glu Leu Gln Lys Ala Ser Val Asp
               260                 265                 270
   Gly Trp Phe Lys Leu Leu Ser Gln Glu Glu Gly Glu Tyr Phe Asn Val
   275 280 285
   Pro Val Pro Pro Glu Gly Ser Glu Ala Asn Glu Glu Leu Arg Gln Lys
   290 295 300
   Phe Glu Arg Ala Lys Ile Ser Gln Gly Thr Lys Val Pro Glu Glu Lys
   305 310 315 320
   Thr Thr Asn Thr Val Ser Lys Phe Asp Asn Asn Gly Asn Arg Asp Arg
   325 330 335
   Met Lys Leu Thr Asp Phe Asn Phe Leu Met Val Leu Gly Lys Gly Ser
   340 345 350
   Phe Gly Lys Val Met Leu Ser Glu Arg Lys Gly Thr Asp Glu Leu Tyr
   355 360 365
   Ala Val Lys Ile Leu Lys Lys Asp Val Val Ile Gln Asp Asp Asp Val
   370 375 380
   Glu Cys Thr Met Val Glu Lys Arg Val Leu Ala Leu Pro Gly Lys Pro
   385 390 395 400
   Pro Phe Leu Thr Gln Leu His Ser Cys Phe Gln Thr Met Asp Arg Leu
   405 410 415
   Tyr Phe Val Met Glu Tyr Val Asn Gly Gly Asp Leu Met Tyr His Ile
   420 425 430
   Gln Gln Val Gly Arg Phe Lys Glu Pro His Ala Val Phe Tyr Ala Ala
   435 440 445
   Glu Ile Ala Ile Gly Leu Phe Phe Leu Gln Ser Lys Gly Ile Ile Tyr
   450 455 460
   Arg Asp Leu Lys Leu Asp Asn Val Met Leu Asp Ser Glu Gly His Ile
   465 470 475 480
   Lys Ile Ala Asp Phe Gly Met Cys Lys Glu Asn Ile Trp Asp Gly Val
   485 490 495
   Thr Thr Lys Thr Phe Cys Gly Thr Pro Asp Tyr Ile Ala Pro Glu Ile
   500 505 510
   Ile Ala Tyr Gln Pro Tyr Gly Lys Ser Val Asp Trp Trp Ala Phe Gly
   515 520 525
   Val Leu Leu Tyr Glu Met Leu Ala Gly Gln Ala Pro Phe Glu Gly Glu
   530 535 540
   Asp Glu Asp Glu Leu Phe Gln Ser Ile Met Glu His Asn Val Ala Tyr
   545 550 555 560
   Pro Lys Ser Met Ser Lys Glu Ala Val Ala Ile Cys Lys Gly Leu Met
   565 570 575
   Thr Lys His Pro Gly Lys Arg Leu Gly Cys Gly Pro Glu Gly Glu Arg
   580 585 590
   Asp Ile Lys Glu His Ala Phe Phe Arg Tyr Ile Asp Trp Glu Lys Leu
   595 600 605
   Glu Arg Lys Glu Ile Gln Pro Pro Tyr Lys Pro Lys Ala Cys Gly Arg
   610 615 620
   Asn Ala Glu Asn Phe Asp Arg Phe Phe Thr Arg His Pro Pro Val Leu
   625 630 635 640
   Thr Pro Pro Asp Gln Glu Val Ile Arg Asn Ile Asp Gln Ser Glu Phe
   645 650 655
   Glu Gly Phe Ser Phe Val Asn Ser Glu Phe Leu Lys Pro Glu Val Lys
   660 665 670
   Ser
<210> 3
   <211> 82
   <212> DNA
   <213> Homo sapiens
<220>
   <221> misc_feature
   <222> (41)..(41)
   <223> SNP72 = A/G
<400> 3
   aaaaaattga agctggagat ttccagatgc atgggtccca rctgcactca tcaaaagtca 60
   ggacagaggt cagcatccgc ca 82
<210> 4.
   <211> 82
   <212> DNA
   <213> Homo sapiens
<220>
   <221> misc_feature
   <222> (41)..(41)
   <223> SNP75 = G/T
<400> 4
   gataagaaac aaactcagta gttcacgttg gttttcaaac kgtgagtata gaaatcaatg 60
   tgtgggtggt gtgttaagga aa 82
<210> 5
   <211> 521
   <212> DNA
   <213> Homo sapiens
<220>
   <221> misc_feature
   <222> (321) ..(321)
   <223> SNP76 = C/T
<400> 5
   ttttggactt tccctgggcc ctgcccaggg ctcacttggg cgggagcaga gcacagtgat 60
   tgagggtacc aagtggagcc aagtggcttg ggttcacttc ctggctctgc tacttcctag 120
   ccaagagatc atgggtgagc tttgaagcct ttccaggtct gagtagtaat agacccttcc 180
   tcctgaggca cccctcaagt cttccttaag gggcacttca tataatcctc agatttcatg 240
   aggtgcccag tgcctggcat gtagtgagca gcctgagagt gtcagctgtt ggctatgatg 300
   ggtgtcatta ctgttagtta ycacattctg gggttattac atgaacttga agctatgcgg 360
   actctcggtg gaggggaggg aagggcccgg cccctcttcc ttgtctgatg tctcctgcgt 420
   cctcctcagt gaaggtcatg ccttttgcca acttgatgag cctcctgggc ccctccatcg 480
   attccgtggc tgttctgcgg ggcatccaga aggtggcgat g 521
<210> 6
   <211> 82
   <212> DNA
   <213> Homo sapiens
<220>
   <221> misc_feature
   <222> (41)..(41)
   <223> SNP79 = A/G
<400> 6
   aaacatgagt taatacatat attttttaaa ggagctgtgc raagggcata catgataagc 60
   tattaagcca tagagtcagg ag 82
<210> 7
   <211> 519
   <212> DNA
   <213> Homo sapiens
<220>
   <221> misc_feature
   <222> (477)..(477)
   <223> SNP89 = A/G
<400> 7
   tcaacataac cttttcatag tcaagtgcta ctaaggtttc tgagattgtg caggggtgat      60
   tctgtgtgca aacaccagga aggaggagga gtcctgcaat ctggtttttt gtttttgggg     120
   ggttttgttt gttttgtttt tttttttttt gagacagagc ctcactctgt gcccaggctg     180
   gagtgcagtg gcgcgatctc agcttactgc aacctccacc tcctgggttc aagtgattct     240
   cctgcctcag cctcctgcgt agctgggact acaggcctgt gccaccatat ccagctaatt     300
   ttttgtattt ttagtggaga cagggtttca ccgtgttagc caggatggtc tcagtcttaa     360
   tctcctgacc tcgtgatcca cctgcctcgg cctcccaaag cgctgggagt acaggtgtga     420
   gccactgcgc ccagcccaat ctgggcttcc ttgccctcaa ttaattcctc cagcctrgca     480
   gtctgcatac tatggcccaa aggccggtgt ttgatccac                            519
<210> 8
   <211> 1000
   <212> DNA
   <213> Homo sapiens
<220>
   <221> misc_feature
   <222> (501)..(501)
   <223> SNP106 = C/T
<400> 8
   ttcaacaaag aagggtgagg cttgagtgtt caattagaga agctggccag ttgtggtatc 60
   atatcagctc tacatccaat gctcagtttt caaggggtca tgttgctctg aaaggcctgg 120
   tgaggttgat gagtgtctta atgaagccac ttgtgaaaga taatgtgaca tgtttgtcca 180
   tcacattgac gcaggaaagg agctttcaca aaagacagca cagaaatttt gcaggctcgg 240
   accagaaggg aatttgtcag tgtttagctt ggctgacatc ccacacccta ccttcttgtg 300
   cagttagaat cccttctagt gtaatgtgtg aaaaaccata ggccccctgc ccacaccctc 360
   ctagagctgt atcaccaaca gctttcaagt gtgtgcaaat tgggagacgg ctggatccca 420
   catctctggg tgacatttgc tgaaacaaat gcctgttaat aagtggcacg attcccttgg 480
   tctcagtgat aaagatgtac ytgttactgg gagcccttga gagaaatctt tggtgaaggc 540
   cttcacactc attttaattg gtgatagaca gatgtgttga gaaaacttac attttttttc 600
   tccatccgtc tcaattagaa tgttatgaaa tagcaccccc tttgctcttc gtgctttatg 660
   ccctagactt taaagactga caacgagtgg ttaaaaaatc tccattgctt caggatgcag 720
   tatcagctga tttggtggat taaatcgttt taaggtcctg agctttaatt cttacttcag 780
   tgattctcaa accttgtttt gcaaaacccc agagctttaa tcttaaggga tgagcctgct 840
   atttccaaga caacaaaatt atttctactt catattaata caaaacatta aaatatacat 900
   tgtctttgta atatagttta ttaattcaaa gcacttgcat tcccatttat ctggtcttca 960
   tacaaaacca tgtatgagtg agtaggcaga tggtcttatt 1000
<210> 9
   <211> 480
   <212> DNA
   <213> Homo sapiens
<220>
   <221> misc_feature
   <222> (280)..(280)
   <223> SNP107 = C/T
<400> 9
   tggcttgaat taacagaaga gccaaccata tgcaacttaa ccaaaagggg ctcatcaata 60
   aatatcatcc ccaaaataat aaagtctgaa agaaggaggt atgagtgtct caagaatgac 120
   agtgctctca gtcagtgttg ctacaaatct aggcacctgt tcatagtcat aatgtggctg 180
   ccatagctcc aacaattatg tcctcaccta acagcctcca gagcaggaag aaaggatgaa 240
   aagaaagagt ctttcctaca tgacccaaac tgggtccagy gcccacttcc agaccaatca 300
   ctgaaaggga aataatcatg ggtcatgcat gcactgtccc ctgggcacat tgccaccgaa 360
   caaaactaga aacaaagaag gaatcgccat tggatgggta gctaatagca tctgctacag 420
   cattcattct cacttccaaa tcccagatac ccacaagacc cccttcaatc agaactgcat 480
<210> 10
   <211> 82
   <212> DNA
   <213> Homo sapiens
<220>
   <221> misc_feature
   <222> (41)..(41)
   <223> SNP109 = A/G
<400> 10
   agggagaggg aggtgaagct ggggaaagct cagggatggg rcgagactat gctgtcatcc 60
   ctttactttc atgttgaata aa 82
<210> 11
   <211> 82
   <212> DNA
   <213> Homo sapiens
<220>
   <221> misc_feature
   <222> (41)..(41)
   <223> SNP111 = C/G
<400> 11
   ccaaattcca ctgagcagtg atcaattctt ggggcatctt sctctgtgtt cccagacatt 60
   ccttcaccct cattttctct cc 82
<210> 12
   <211> 479
   <212> DNA
   <213> Homo sapiens
<220>
   <221> misc_feature
   <222> (371)..(371)
   <223> SNP114 = A/C
<400> 12
   accaccatgc ctggctaatt ttaaaacaac ttttgtagag aaggggtctt gtgatgttgc    60
   aagactggtc ttgaactcct ggcttcaagc aaccctccca cctcagcccc caagtgttgg   120
   gattataggt gtgagccact atgcccctct ggagtgtcta ttttttaaat tgcctttctt   180
   tttctcttcc ctgcctcccc accaagaggc agcgtagaga agtgggcatt tgcacaggct   240
   accaggcaag tccctgtcgg tctgaatcct agctttgaga cttcccaggc atattactta   300
   gcctctctgt atttcagcct ccttgtctat aaaacggagt cagtaacagt accatagtgt   360
   ggctgagcag mtccaactca ttgcaatatc caagattctt ggaccagagc ttgacacaga   420
   gtatgctcag aaaatatctg ctcttatgat tgtccccaga ctaccccaat ggtggggca    479
<210> 13
   <211> 761
   <212> DNA
   <213> Homo sapiens
<220>
   <221> misc_feature
   <222> (501)..(501)
   <223> SNP117 = C/T
<400> 13
   tctgcaactc tggctagcat cattgataaa ttgtcttaaa agatgaagat ttgtatgcag    60
   gtggtttatt ggggatgtag ctcttgggaa caacatctat aagggataga gggaagcagg   120
   gaagaggtag ttgaactgtg gtgcaatagt tgtaatagag gccaaagcca attccatggc   180
   tggagctgag acggcccttc agaactggga caagccaatg catcaagtgg gcattgattg   240
   tgggctgctc tttggaaggc agcattacct tgggcaaagc agctcccttc ttcaggggag   300
   gagtcccagg gaggacacag ctgtgagcaa ttggttttcc cagcagctgg gggaatgagt   360
   gccttgtccc tatagatggt tttgaggggg ctgcatttgg gggaatgcct tctggggttc   420
   actgcgtgct tctatgtctt tcttagaggt tcatttcaaa ctataccttg gttcattcac   480
   agtactgatt tagttactat ycagattcat agactgtact tgctggcact ggaatatcat   540
   ggacagagag tcctaatctg actaggccta gggaatgaca tgggaatagc ttgtcctggg   600
   tcatccagct accagagagg agagctggga ttcatatcca accaacgata ctttatacct   660
   cacattactt ctgttatgct acacactcct gagtcacagg agattccaac ttgtatttca   720
   agcccagact gcatgacttc aatgtcctgt tgcccttaac c                       761
<210> 14
   <211> 825
   <212> DNA
   <213> Homo sapiens
<220>
   <221> misc_feature
   <222> (325)..(325)
   <223> SNP118 = C/T
<400> 14
   atctgactag gcctagggaa tgacatggga atagcttgtc ctgggtcatc cagctaccag    60
   agaggagagc tgggattcat atccaaccaa cgatacttta tacctcacat tacttctgtt   120
   atgctacaca ctcctgagtc acaggagatt ccaacttgta tttcaagccc agactgcatg   180
   acttcaatgt cctgttgccc ttaacccctg tatcagattg cagccccact ctggaaagtt   240
   ttcttgaatt cttctatgta tgttttttaa actgtgggtc atcactcatt agaagttcat   300
   gaagtcaata taataggtca agaayaccat gaagaaagca gaacagaaaa tatcacagtg   360
   catcactctc aggaagggta gatattgttt cacgaaactt tttttttttt tgagacaggg   420
   tctttgtctg tcgcacaggc tggagtgcag tggtacaatc atagctgact gtatcctcaa   480
   cctcccaggc ccaagagatc ctcccacctc attctcatga gtagctggga cttgaggcac   540
   atgccatcat gcctggctaa tttctaaaac tttttgtaga gatagggtct ccctatgttg   600
   ctcgggctgg tctcaaactc ctgagctcaa acagtcctcc taccttggct tcccaaagta   660
   ctgggattat aggcatgagc cactactcct ggcctcatac aatttttatt tgatataaac   720
   atatatagcc atgagtgaac tgggtcaaaa tgtaaaatgt gtctcttact gcaggtaact   780
   gtcaatgtag ttcgaatatc actgtgaaat atatagagaa gaggc                   825
<210> 15
   <211> 652
   <212> DNA
   <213> Homo sapiens
<220>
   <221> misc_feature
   <222> (201)..(201)
   <223> SNP119 = A/C
<400> 15
   tcctttgtag ggcgactaac atgaaggttc tgactcttaa cccatagagc cacaaaaaat 60
   agtcctagga aaatcatatg attttattta ttttaggaac agaaatggtc ccttccgaag 120
   tagaggcatt tctacttctt tcctaaacac catctctgct cagtgagctc tcccaccctc 180
   aggtactgtg tttcaggctt mttgctgtat tgctagaagt atcctcacct tcgagattaa 240
   gaaaagatat ttattgccgg gcacagtggc tcacgcctgc aatcccagtc ttttgggaag 300
   ctgagacggg aggatcactt gagcccagga tcttgagacc agccagggca gtatagtgag 360
   aactcatctt gaaaagaaaa atttattgag ctcctactgt atactgggga tacagcaata 420
   ggcaagatag cccttgctcc tcatgtaacc tacagataat aaacaagatt acaaacaaac 480
   aaaataagag gtcgttctag attatgataa gtgatggaaa agaaaaaacc tgtatctact 540
   tcttatagca gagacctgca ttgggagttg ctgtgatcaa acaggaaacg gaaggtggag 600
   tttgcttctg tatccctcca tccctgagtg gaggctgggt gcttctcacg ac 652
<210> 16
   <211> 401
   <212> DNA
   <213> Homo sapiens
<220>
   <221> misc_feature
   <222> (201)..(201)
   <223> SNP120 = C/T
<400> 16
   actttctttc acttaggtgt gaaaagacag gaagtacttg aagggtgggg tagactcaaa 60
   ggatagaatc ccacaggatc ccatagagcc cgggggcaat ttgaccagga aggggttctg 120
   aactccagaa gaggaggatg gagagtggaa acacaaagac taaaaggcag gctctagcat 180
   gacatccaga gagaggcctc yctcttctgg gcttaagcct actctcccag aaccttcact 240
   cagctccttg catcccaaag ggcagagtct aggctaagca taaaggatcc aatctatgca 300
   atggagggaa gtaagtgtcc aacacagagg cacctgatga aatacagcga ccagcccaag 360
   aagagcccct tggggctctg tttcttacat taggaaatct c 401
<210> 17
   <211> 401
   <212> DNA
   <213> Homo sapiens
<220>
   <221> misc_feature
   <222> (201)..(201)
   <223> SNP121 = C/T
<400> 17
   aaacacttct gcttggtccc tctagagaga agtctttaat tattcatctc tttccaagta      60
   attggcgaca agtcctgcag ctgggagttg gaataaactt ggttgctaaa agcatccatc     120
   acacataaaa caggggagat ctggctccaa agaatggtag atgtagatct tcccctacgg     180
   atttgttttc tgaagatctc yttatacact gacctcagcc ttttgctgac ctatccttgt     240
   gaactgaagg ctgaaaaaac caatgtagcc atggtccaca tccatttatc actttcagaa     300
   atggcccctg cactttatat ttcttcaggt cctgcaaaaa atattgaaaa tcctttacaa     360
   atagaggcaa ggtcaggcca tccctgtggg atttccaggg a                         401
<210> 18
   <211> 1472
   <212> DNA
   <213> Homo sapiens
<220>
   <221> misc_feature
   <222> (599)..(599)
   <223> SNP122 = A/G
<400> 18
   gcttcagggc atcagcctgg ggccacatca ggacctacct gcacgtaggt tgatcaggac      60
   cctggcaggg ctgtcaggta cagccacaaa gcaagctgtt tctgactgtg ttcccacact     120
   ttctcaggca tccaccctgg tgtatgaatt tgcttcttct ttataacaaa ctacctctaa     180
   actgcactct agtctacaga tcagctgggc aatgctgttg atctcggctg ggcttgctcg     240
   tgtgtctttg gtcagctagc aggttatctg gggcctgatt ggtctaggat ggcttcactt      300
   gcacatctgg cacttagatg tgtgttagct ggagcaatag ggggcctgga ccccgtgggt     360
   ctccttttcc agcaggccac ttgggcttgt tcacagggca gatgggccca gttcaaaggg      420
   tctgagaaca caacggtact gcaggatgag ctttggaact gctgcaccgt ctcttccact     480
   gcattctgca tacgggccct aaacaagtcc caagccttct cagattcaag acatggggga     540
   tgggctgtat ctcttatgga gagagtcaca tagcaagggt gtggctctag ggaggaatra     600
   agagtggtca agtgcagcct gccatgctgg ctccttcacc agcctgtcag ttatatgagc 660
   caatgggctg aatcatagtc atctttctct ccacatgcct ggcccgtgaa ggatttgcct 720
   gcccagcaga gaccgtggaa tgaatgtgtg ctccaggcac tctgccttct ctctcttctt 780
   tgacttctct aagctcgttc ctgcccctgt ccccctctgt ctctcttccc caggatcatg 840
   gcattagtcg gatccttccc atcattttca ttttagctcc agtatcacct ctttgagcct 900
   tccctgacta cccttccagc attctctaat cccatcacct tgtttttttt atcttttctt 960
   tttttttgag acggagtctc actgtgttgc ccaggatgaa gtgcaggggc acaatctcgg 1020
   ctcactgcaa cctgtgcctc ctgggttcag gtgattctct ggtttcagcc acccgagtag 1080
   ttgtgattac aggcatgtgc caccatgcct ggctaatttt tgtattttta gtagagacag 1140
   ggtttcatca tgttggtctg gctggtttcg aactcctgac ctcaggcaat ctgcccaact 1200
   cagcctctca gcctcccaaa gttatacagg tttttttttt tttttttttt ttaaatcttt 1260
   tcatagcatc tgtaactgtt taaaatatta gtttgacttc cttttctaga atgtaagctt 1320
   tgggagagcg gggtccttgc ctgtcttgtt ctctgttact tctccagctc ccagaatggt 1380
   ggctggcact cagggggtgc tcaacacaca taattgtcaa gaacatgcta catcaaggcc 1440
   tgagtggctt tgccacggct cttcttgtga ct 1472
<210> 19
   <211> 1001
   <212> DNA
   <213> Homo sapiens
<220>
   <221> misc_feature
   <222> (501)..(501)
   <223> SNP123 = A/G
<400> 19
   aacctgtgcc tcctgggttc aggtgattct ctggtttcag ccacccgagt agttgtgatt 60
   acaggcatgt gccaccatgc ctggctaatt tttgtatttt tagtagagac agggtttcat 120
   catgttggtc tggctggttt cgaactcctg acctcaggca atctgcccaa ctcagcctct 180
   cagcctccca aagttataca ggtttttttt tttttttttt ttttaaatct tttcatagca 240
   tctgtaactg tttaaaatat tagtttgact tccttttcta gaatgtaagc tttgggagag 300
   cggggtcctt gcctgtcttg ttctctgtta cttctccagc tcccagaatg gtggctggca 360
   ctcagggggt gctcaacaca cataattgtc aagaacatgc tacatcaagg cctgagtggc 420
   tttgccacgg ctcttcttgt gactgcaggc cttggtgcca gcagccaccc cagttccaag 480
   aaatggtctc ttgctggcca rcttagcagg aagaactggc agcgttcctg ttacagcact 540
   tccaggtggc tgcttttccc tgagtccatg tggcttctcc atgtctctct gttggagtca 600
   caagttgtgg aaattcactt aggccaactt agaccaagaa gtggaatagt taatagttaa 660
   atgtcagtct gggcaacata gcaagacttc gtctttacaa aaaattttta aaaatattag 720
   ctggtgcggt cgtgtgcacc tgtagtccta cctactcggg gggctgtggt gggaggatcg 780
   cttgagctca ggagttggaa gctgcagtga actatgattg tgccactgca ctccagcctg 840
   gatgacagag caagaccctt tatctaaaaa acaagaaaaa attaataaat aaaagttaaa 900
   tataaggtct ctggaggtat cctgtttggg ttcagattct ggctctgcca cttcctagtt 960
   gggtcacttt tgggaagtta cttaacctct ttgtgcctca g 1001
<210> 20
   <211> 701
   <212> DNA
   <213> Homo sapiens
<220>
   <221> misc_feature
   <222> (501)..(501)
   <223> SNP126 = A/C
<400> 20
   tgaatcaatt ctgtttctta agcagatggg agatcctgtg gctttttacc actatccaca 60
   gctctttttt tctttttctt ttcttttttg agacggagtc ttgctctgtc acccaggctg 120
   gagtgcagtg gcatgatctc ggctcactgc agtctccgcc tccctagttc aagccattct 180
   cctgcctcag cctcccgagt agctgaggtt ataggcgccc gccaccacgc ccagctaatt 240
   tttgtatttt tagtagagat ggggtttcac catcttggcc aggctggtct caaactcctg 300
   acctagtgat ctacctgcct tggcctccca aagtgctggg attacaggca tgagccactg 360
   tgcctggcct atccacagct ggtttcccct ctttgccccc ttttctggca ttctcttctg 420
   attcactctc aactctgatt tccaagctac ccagctcctt cccctagtgt gaatctgtat 480
   ttgcactgga tttattttta matgggaaat gaaagccact gtgtgctgga aaatatatat 540
   ttcagacacg aggaagctgg cggggaaata ttttttggat gtctttctag atctcttaaa 600
   aatcctgtga gccatcacca ggttgatctt gtagtaaggt ggccttggcc ttagtgtatt 660
   tctttttgac tcgatattgg gcggcaggag gtttgaggat t 701
<210> 21
   <211> 1000
   <212> DNA
   <213> Homo sapiens
<220>
   <221> misc_feature
   <222> (501)..(501)
   <223> SNP128 = C/T
<400> 21
   ttcaacaaag aagggtgagg cttgagtgtt caattagaga agctggccag ttgtggtatc     60
   atatcagctc tacatccaat gctcagtttt caaggggtca tgttgctctg aaaggcctgg    120
   tgaggttgat gagtgtctta atgaagccac ttgtgaaaga taatgtgaca tgtttgtcca    180
   tcacattgac gcaggaaagg agctttcaca aaagacagca cagaaatttt gcaggctcgg    240
   accagaaggg aatttgtcag tgtttagctt ggctgacatc ccacacccta ccttcttgtg    300
   cagttagaat cccttctagt gtaatgtgtg aaaaaccata ggccccctgc ccacaccctc    360
   ctagagctgt atcaccaaca gctttcaagt gtgtgcaaat tgggagacgg ctggatccca    420
   catctctggg tgacatttgc tgaaacaaat gcctgttaat aagtggcacg attcccttgg    480
   tctcagtgat aaagatgtac ytgttactgg gagcccttga gagaaatctt tggtgaaggc    540
   cttcacactc attttaattg gtgatagaca gatgtgttga gaaaacttac attttttttc     600
   tccatccgtc tcaattagaa tgttatgaaa tagcaccccc tttgctcttc gtgctttatg    660
   ccctagactt taaagactga caacgagtgg ttaaaaaatc tccattgctt caggatgcag    720
   tatcagctga tttggtggat taaatcgttt taaggtcctg agctttaatt cttacttcag    780
   tgattctcaa accttgtttt gcaaaacccc agagctttaa tcttaaggga tgagcctgct    840
   atttccaaga caacaaaatt atttctactt catattaata caaaacatta aaatatacat    900
   tgtctttgta atatagttta ttaattcaaa gcacttgcat tcccatttat ctggtcttca    960
   tacaaaacca tgtatgagtg agtaggcaga tggtcttatt                         1000
<210> 22
   <211> 1117
   <212> DNA
   <213> Homo sapiens
<220>
   <221> misc_feature
   <222> (727)..(727)
   <223> SNP131 = A/G
<400>
   tgtattctca cgtggcagag agcagagaga ggaaatcagc tctctcatgt ccctttttaa     60
   aaattcacat acatttaagt tcaagtgcag ttttattaca tgggtatatt gcatggtggt    120
   gaagtctgag ctttttgagt aaccatcacc caaataatgt acattgtatc tactaagtaa    180
   tttctcatcc ctcaccctct tcccaccctc ccaagtcact atcattccac actctatgtc    240
   cgtgcacaca cattataagc tcccacttgt aagtgagaac atgcggtatt tggctttctg    300
   tttctgagtt gtttcagtta aaatattgac ctctagttcc ttccacattg ctgcaaaaga    360
   tgcgatttca ttccttttat ggctgagtag tattctgtgg tgtatataga ccacatttgc    420
   ttcatccatt ttgagtccct tcttataagg gccttgatcc cattcagaaa gtctccatga    480
   tctgatctaa tctcatgatc taatcacctc ccaaaggccc cacctcctaa aagcaacaca    540
   ctggggggtt aggcttcaac caattaatct gtagaggaca aacacgttca gtccatagca    600
   ggaagcagtg ccgccatggg gaagagcatt gtcgtgaaac cttttgcccc tcttgttgag    660
   ttttctcctc cagcacaccc tgaacatgct gacagtgtca ccttcctctt tacttgtccc    720
   tcttccrctc caagttgcct cctggcttcc cagtgtctct gtgggcttga gaccagagct    780
   tgtcataaga gatagatgac gtcacttgat ggaaagagtc aagtgcagtg accagcacat    840
   agaaggcggc catcaacatt ggtttcatcc tttagagcaa agtttcccca agcgcctggt    900
   gtgcactggt gagatttggg gacggggtgg tacttaagac ctggcactaa ttaacactag    960
   atcactgtgt gggaaacata gtcccttttc tgttctcttc aaatccttct gatcgggctg   1020
   aggagaaagc ctcaggttgg tgctgatgtg cttttttctc ccagaccccc atcctaacag   1080
   aaacagagca ggtctcaggc tcgggccgtt aggcaat                           1117
<210> 23
   <211> 401
   <212> DNA
   <213> Homo sapiens
<220>
   <221> misc_feature
   <222> (201)..(201)
   <223> SNP133 = C/T
<400> 23
   ctgacactgc tgcaggactt catggtgaat tccttatttg gagggacagc ataggggccg     60
   ccagaaaata tcgccatggc cagaagcaga gcaagggaga gagggtttta aaaggcaggg    120
   ctgtaaacct cagcataaaa taagcagcca gcctctcatt tcagtcatgt aaacagtaat    180
   gtgtattcat taaaactgca ytagtctaag gagtccccag agatcagggg ctaacgggag    240
   aaggggtagg ggagaggctg ccaactgcac ctgctgtttt ctaacacagt gcaaacaggc    300
   caaaagtggg tttctggtgc atgtcacagc cagaaaaggc tgcccagagg ggcttcacag 360
   cccccatcca tcttgttaaa ggtaaacaga catggagaaa a 401
<210> 24
   <211> 758
   <212> DNA
   <213> Homo sapiens
<220>
   <221> misc_feature
   <222> (506)..(506)
   <223> SNP134 = C/G
<400> 24
   tatccattca tttgtcaatg gacagttgga ttgctctcat cttttggcta ttgtgaataa 60
   tgctgctatg aacaggagtg tacaaatatc tcttcaaggc cctgttttca attcttttgg 120
   ctatgaaccc agaagtggaa ttgctagatt aaatggtaat tctattttta atttgttgag 180
   aaccaccata cttgttttca tagtggctgc accattttgc attcccatca acagtgcaca 240
   aggatttttc cacatcctca ccagcacatg ttattttctt tttctttttt tgatagtagc 300
   catttaatgg gtatgaggtg gtatctcatt gtggtttttg atttgcattt tgctaatgag 360
   tgatgctgag catcttttca tgtgcttatt ggccatttat gtgtcttctt tggagaaatt 420
   ctattcaagt tgacttctat gattttgcat tatcacatct gtattgattc aagcaagcca 480
   actagaataa attctggcat ttaaascgat tttgtggttt ttctgcaaat aaattctgcc 540
   cccaaataac ctccaacttt ctggaagcag tcagcaggag tacagttctg aagataactt 600
   tctttaaaaa aggaaattca taaaatatca tgcatcttcc ttttttgaca ctaatggaac 660
   aatttaatgt aatttcagag ggaagcagag cccctggaaa ggctggtgtg ataagggaag 720
   gttacccagc tttcctgtca ggcggtgtgt gggagcag 758
<210> 25
   <211> 758
   <212> DNA
   <213> Homo sapiens
<220>
   <221> misc_feature
   <222> (506)..(506)
   <223> SNP135 = C/G
<400> 25
   tatccattca tttgtcaatg gacagttgga ttgctctcat cttttggcta ttgtgaataa      60
   tgctgctatg aacaggagtg tacaaatatc tcttcaaggc cctgttttca attcttttgg     120
   ctatgaaccc agaagtggaa ttgctagatt aaatggtaat tctattttta atttgttgag     180
   aaccaccata cttgttttca tagtggctgc accattttgc attcccatca acagtgcaca     240
   aggatttttc cacatcctca ccagcacatg ttattttctt tttctttttt tgatagtagc     300
   catttaatgg gtatgaggtg gtatctcatt gtggtttttg atttgcattt tgctaatgag     360
   tgatgctgag catcttttca tgtgcttatt ggccatttat gtgtcttctt tggagaaatt     420
   ctattcaagt tgacttctat gattttgcat tatcacatct gtattgattc aagcaagcca     480
   actagaataa attctggcat ttaaascgat tttgtggttt ttctgcaaat aaattctgcc     540
   cccaaataac ctccaacttt ctggaagcag tcagcaggag tacagttctg aagataactt     600
   tctttaaaaa aggaaattca taaaatatca tgcatcttcc ttttttgaca ctaatggaac      660
   aatttaatgt aatttcagag ggaagcagag cccctggaaa ggctggtgtg ataagggaag     720
   gttacccagc tttcctgtca ggcggtgtgt gggagcag                             758
<210> 26
   <211> 480
   <212> DNA
   <213> Homo sapiens
<220>
   <221> misc_feature
   <222> (201)..(201)
   <223> SNP136 = A/G
<400> 26
   atgcagttct gattgaaggg ggtcttgtgg gtatctggga tttggaagtg agaatgaatg      60
   ctgtagcaga tgctattagc tacccatcca atggcgattc cttctttgtt tctagttttg     120
   ttcggtggca atgtgcccag gggacagtgc atgcatgacc catgattatt tccctttcag     180
   tgattggtct ggaagtgggc rctggaccca gtttgggtca tgtaggaaag actctttctt     240
   ttcatccttt cttcctgctc tggaggctgt taggtgagga cataattgtt ggagctatgg     300
   cagccacatt atgactatga acaggtgcct agatttgtag caacactgac tgagagcact     360
   gtcattcttg agacactcat acctccttct ttcagacttt attattttgg ggatgatatt     420
   tattgatgag ccccttttgg ttaagttgca tatggttggc tcttctgtta attcaagcca     480
<210> 27
   <211> 575
   <212> DNA
   <213> Homo sapiens
<220>
   <221> misc_feature
   <222> (201)..(201)
   <223> SNP137 = A/C
<400> 27
   ccacattctc aggaagaacc atatttgatt tcatttagaa aacggaattc ttacttcttg      60
   aacaaagttg gcttttcttc tctgaacgtg gcctttgcaa atgcttgagt ctttgagtat     120
   ggtgatgact gagtgagagg agggatttgc ttggggcagc aggcacatct ggaaccattg     180
   accacaaacc tggctgagca magagccacc cggatgatgg gcttaatagg tacaaactgg     240
   gaacttcccc tcaggcatgc caactcagga ggttgtcacc agtggctgca gcttggagtg     300
   atctggggat cttttaaatg ccccaatgcc ctgcctctac ctctgtcagt gaaagcaggc     360
   tgtctgggag tagaatcaga gcagcagtat tcattaaagg ctctccaggt gactgtcatc     420
   agcatccagg tttgagagcc actgtgggga aataatctgt actttctata aagccagaag     480
   cagtcagttt tctgaacatc actgattaga ccgtagccat tttccagtat ggacaaagta     540
   ggttctctcc tgccatcccc cagctgcagc aaact                                575
<210> 28
   <211> 1102
   <212> DNA
   <213> Homo sapiens
<220>
   <221> misc_feature
   <222> (295)..(295)
   <223> SNP138 = A/G
<400> 28
   gctattacaa ccagactaaa agaagttcag tttgaagggc tgggaagaga gtgggtgagg      60
   gcagatttat ggcagagctt ctggctcaga tgttgcctct aattcagact ccatctcttg     120
   cttgactcca tgtctgggta agtgacttca acgctaagcc tcagtttcct caactgaaaa     180
   ccagaaataa cattgcttat ctcaataggt tataagcaga attaacacga agtctacaaa     240
   aatcataggc actcattaaa attagttccc ttgtagccag gcacagtggc tcacrcctgt 300
   aatcccagca ctttgggagg ccaagagggg tggatcacct gaggtcagga gttcaagact 360
   aacctggcca aactggtgaa accccatctc tactataaat acaaaaatta gccagacatg 420
   gtggtgggta cctgtaatcc cagctattct ggaggctgag gcaggagaat cacttgaacc 480
   tgggaggcgg aggttgcagt gagccgagat caggccattg cactccagcc tggatgacaa 540
   aagtgaaact ccatctcaaa aaacaaatta gttcccttgc atttgcccct gatgctcctc 600
   caactaagtc atcctatgac atttgaaagc ctttgctctg tagaattctt acttccagta 660
   tcacttggga agttctgacc tcggctaaga gtgaaaattg tgtgttaatt tacttgccat 720
   ctttgttcaa ttgatagtga ttatccagaa caatattaca agtgtttcta agccatccaa 780
   ggcactttag gaaggaacaa gaaaactaga acagttatag tgggtaccag agtagaaagt 840
   ggctacaaga atgccatata tttgccatct ctgccttcag cattaaagat gaaggaaatc 900
   aatgaggaaa aaatctggat ctgaagtctg aatttgcatt ttgattaatt tatcccaatt 960
   actttggtct cagttttcat aactgtaaaa tgatcgagat tttataagat gatgttgatg 1020
   atgatgatga tacctgccct atgtgtctca tcagagcact gagaatgata gatgtgttta 1080
   aaagctggcc ataagccaat gc 1102
<210> 29
   <211> 401
   <212> DNA
   <213> Homo sapiens
<220>
   <221> misc_feature
   <222> (201)..(201)
   <223> SNP139 = C/T
<400> 29
   attattcatc tttcctccca cgtgctttgc acatgacagt cactcagcaa ccctctgcag 60
   aactgagtca aacaggaaaa gtaatgccgc atatctgagc tgcagagtgt tcttatattc 120
   aaacacccat ccctatggct cctctgggat actggagatg actgccggca ggataacagg 180
   tgtgggagcc agggacaagg ygggaagagg gcttgctact cagggaatag agtaccccat 240
   tgatgctggt cactcttcct ttcctaccag ggatgctcag gcattccact ccttcacaga 300
   cagcaacctt gccctgagaa aagaaaggct tatgtgtgat tattcactat ggaaaataag 360
   gaggaaacag atcaggagaa acaggaaaaa taaagtttca c 401
<210> 30
   <211> 1103
   <212> DNA
   <213> Homo sapiens
<220>
   <221> misc_feature
   <222> (802)..(802)
   <223> SNP140 = A/G
<400> 30
   gcagagggtt gctgagtgac tgtcatgtgc aaagcacgtg ggaggaaaga tgaataatga     60
   gagcaatcga atgcccattc ccttgcatcc agggtgctct actagacctg cgttcttcca    120
   tcattctttc agcccacgtg gttgccaata ctgggagtta tcagtctttt ccatttttgc    180
   caatctaatg gataaaatat cttgtattaa tttgcatttc ccagatattc atgagattag    240
   gcatacgctt tttgggcact tgtatttttt tctctgggaa ttccctcttc gtattacttt    300
   gtcaattttt ctagtggctt ttttatttga gacagggtct tcctctgtca cccaggctgg    360
   ggtacagtgt ccagatcaca gctcactgta acctcaactc cagtgatctt cccaccttag    420
   cctccctagt aaatgggact acaggcacac accactatgc caggcttttt tttttttttt    480
   ttttgtagag acagggtttt gccatgttgc ccaggctggt cttaaactcc tgggatcaac    540
   tcatcctccc aacttagcct cccaaggtgc tgagattaca ggcatgagcc actgcacctg    600
   gtcccctgtc tttttttttt taattaattg atagtaactc tttatatatt ctagatgcca    660
   atccgtgatt tagtctaaaa ataaatcccc ttggtctgtc ctatgtcttt tcttttggtt    720
   tatgaaatct tttgtaatac aggaacgtca cacttccaca gtcaaatgtg tcagtcttct    780
   aaatagatat aggtaccttg trtatactcc ttgatatatc acattgattg gtggtgacct    840
   gtttgtctcc ctggctgtca gactgtgaga aacttgaggg caggggctgt actttattct    900
   tctctgtctc tctagggact agtgcaaagc tgggcacaga gttttgtgcc tgaatgagtt    960
   ttttttgtgt gtaaacgtgg tataaaacag gtcttgtgac tgaatgaggg aatgaatgaa   1020
   aaagccctgt cctgccctcc ctgcagagat ggggtctgct gcagagatgg gagtgagtca   1080
   gggagtgaat cacaagctct tcc                                          1103
<210> 31
   <211> 619
   <212> DNA
   <213> Homo sapiens
<220>
   <221> mist-feature
   <222> (354)..(354)
   <223> SNP141 = C/T
<400> 31
   aatttctcca tttgtaaaat gtatataatg atagtaccta ccacgcatgg ttgttaggaa     60
   gattaaatca attaatattt gtaaagtaga gcagccggct cattgtaagt attgggagta    120
   ttggttaaaa taattacaaa ataaacaatc tcacttagat acatccctta ccccctgtgg    180
   attatgtcaa gggtgaaaga aacagtgtgc ttgaaatcct gctgtgcgcc gagctgtata    240
   tcagaggtgt tatggtgatt cgtcatcatt ctcctgacca ctttccattt ttcttttaga    300
   gtcaacactg gccaatcttt agggaggagc tctgatcatt gagaaactga cacygtggaa    360
   gggggcattt ctgactctct cttcctagct ccatttgatc ttcctaaatc tttcaataat    420
   gtgtcattgg ggtaggacca aagaaaaacg gggtgaatgt acggttgctg ctttgtttcc    480
   catagtggga gtgactctga tgtttcacaa aggcaggggt ggagaggcag cgcctcgcag    540
   gaaagccttc ttgctaaatc gaggacttct gcacagaaat agatacaaac ttgcttactg    600
   tttaaataga atattagcg                                                 619
<210> 32
   <211> 1451
   <212> DNA
   <213> Homo sapiens
<220>
   <221> misc_feature
   <222> (674)..(674)
   <223> SNP142 = A/G
<400> 32
   tccatggact gaattcatac acacagaatt ttgtgggaac acagctgtgc ctgtttgttt     60
   atgccttatc tatggctgcc tttgcgctac aaggatggaa ttgagtagtt gtgacagaga    120
   ccatgtggcc tgcaaaacct aaaatactca gtctcttatc cttgaagaaa aagtttgtca    180
   gcctctggtc tagaacattg cctggctctt ggtaaggtta aatacatgtt tggaactttt    240
   tatttattga aaataatata cattttgttc tccacctaac cttttaaggt aagtattgtt    300
   atccctgtcc tagagatgag aaaactggga ctggaattct gtactgtcac ttccaggggg    360
   caaacacctc atgaggtttt tttttttttt ttggcagtct tgctctgtct cccaggctgg    420
   actgcagtgt cgcgatctca gctcactgca gcctctgcct cctggggttc aagtgattct    480
   cctgcttcag cctccccagt agctgggatt ataggtacct gccccctctc cccgctaatt    540
   cttgtatttt tagtagagat agggtttcat catgttggcc aggctggtct tgaattcctg 600
   acctcaagtg atccacccgc cttggcctcc caaagtgctg ggattacagg tgtgagctac 660
   catgcctggt ctcrtgagtc ttgttcatca cttacctcct tttgttcttc cagcttcagt 720
   ttcaggttca gggggtacat gtacaggttt gttacatggg taaattgcat gtccctggag 780
   tttggtgtac taatgatttc atcacccagg tagtgagcat agtacctgat agtagttttt 840
   gatcctcacc ttccaccctc aaggaggtct cagtgtctgt tgttcccttc tttgtgtcat 900
   gagtgcctga tgtttagctc ccacttataa gtgagaatat gcagcatttg gttttctgtt 960
   cctgtattaa ctcacttagg atagtggcct ccagctgcgt ccatgttgca gcaaagagca 1020
   tggtttcatt cttttttatg cctgcatagt attccatggt gtttatatac cacattttct 1080
   ttatcccatg caccgtggat gggcacctcc gttgatttct tgtctttgct attgcatcac 1140
   ttactcttga atctggagca gtgcctagca cataatagac attcaggaca tgtttgttga 1200
   gcaatgaatg gatgagttag gaatcaatta catataaatc aataatcaat aatcatcaat 1260
   gatgaatttc tatgaacaaa tatgaaaatt agatcggccg cattgtagag atgaggagac 1320
   agccctgtag aaggaaagtg cctcatcaaa atgcaacatc gctgtgaaag tgtagacttg 1380
   gagctccagc tgaagtcatc tgtgtatttt ctaagctgtc ccctctacca tcctgccttc 1440
   tccgggagct g 1451
<210> 33
   <211> 1001
   <212> DNA
   <213> Homo sapiens
<220>
   <221> misc_feature
   <222> (501)..(501)
   <223> SNP145 = A/G
<400> 33
   tacctagtct tagagtttgc aaatgataga cacaggatat acacctgatc atcagactcc 60
   agaattcaca ttcccaacca ataagtaata caaatacaag taagcaatac aatcacagac 120
   acacagaaca aaaaaagtta aagtagcaat taataaagta ggctaaaacc aggtagagtt 180
   gataaatata atcataatct gcttctttaa catgatagaa agataaaaat agacaagaaa 240
   gaacacaaac ttctggaaaa aaaataacga gagactacct aacaatgcaa tatgaagaaa 300
   gaaacaaaga aagagaaaag gtgtaatatc atcccagata agatgctaca atttaaacac 360
   gttgagaaga acactgtgga taatttccaa ggaaaatata aattccaaaa gtttgtttga 420
   gtagaggtga aaaactttcc tgtgtggttg aagtgtagag agggagaggg aggtgaagct 480
   ggggaaagct cagggatggg rcgagactat gctgtcatcc ctttactttc atgttgaata 540
   aaagggaaag ttaaacatgg aactacgaca gggggaaaag agagaataat aaaaatatta 600
   tttggggtaa tacggttttc tatctggaaa actcaaagga agcaactaaa actccacaaa 660
   agctatttga gtttttttaa aaaatgcttt aaaataaata tgctaacaat ctattgcttt 720
   cctattgttt ttgtttgttt gtttgttttg tgttttgaga tggagtctcg ctctgttgcc 780
   caggctggag tgcagtggca tgatattggc tcacttcaac ctccgcctcc tgggttcaag 840
   caatctactg cctcagcctt ccaagtacct gggactacta caggcaccta ccaccacgcc 900
   cagctaattt tttttgtatt tttaatagag acggggtttc accatgttgg ccaggctggt 960
   ctcgaacttc tggcctcaag tgatctgcct gcctcagtct c 1001
<210> 34
   <211> 816
   <212> DNA
   <213> Homo sapiens
<220>
   <221> misc_feature
   <222> (316)..(316)
   <223> SNP147 = A/G
<400> 34
   attattattt aagagtgttg cccaggctgg tctcaaattc ctgggctcaa gcaatcctcc 60
   tgccttggcc tcccaaagtg ttgggattcc aggcgtgagc cagtgcaccc tgcttgttag 120
   ggtgttccta atatcagggc ccaggaatga ggaggaaatg aatttatcct gctaccatga 180
   atctcaaagt atgactactg ctagtgggac aagagatgct tgtaggtaaa atgtgcaaaa 240
   aatttttgtt ttaatcatta taaatgtatt ttaatgtgga ttgggaaaaa tgtatctagt 300
   agtggttttg catttrtgat aaggatatag aatttcctac ctaagtaaag atttttaggt 360
   taaacaaaag ataaactgat ttgaggacaa tattaagtga atgacagtat gggtgatata 420
   aggatatggc aaaagttttg gtacaaatgt gtttaaagtt tagggtacat tgttgattgg 480
   cctacccagc agccattcac agccttcatc cttctggcag aggctacctc tcatgatgga 540
   agctaaaaat gcctaatact tgcttttccc ggctctcttg tagctgctga atgaggatgt 600
   gacccaatcc tgctaatggg aactgagggg aagacctggg aaagagtcta ggaaagattt 660
   ttcctttctg ataagagaga gacatccctt cccttgtcct cttgggcatt tttgtacaag 720
   gatataatgc ttggagttgt ggcagccctt ttgggtcaat gagggacaca ttgccgacat 780
   cctgagcatg gcagagtgga aaacttaaaa gaaccg 816
<210> 35
   <211> 838
   <212> DNA
   <213> Homo sapiens
<220>
   <221> misc_feature
   <222> (638)..(638)
   <223> SNP149 = C/T
<400> 35
   cccttccttt gtcctctggc tcttactagc ctccaatccc ttatactttc ccaacaccat     60
   gcccacacct ttagaaacag tcctttcatt gaacctttct taaacactct tcactttagt    120
   atgccatctg tttcctgcgg ggcccctgac tgatgcatct gctttgtctt gcctcatttg    180
   ctcctgctgt agatggaaca tgtctgcaaa catcgttaca gttgacccca tggaaagatg    240
   gcaaccctag tggaggaatt agccttcttc ctgcattcgt tttttttttt tttttgagac    300
   aaagtctcgc tctgttgccc aggctggagt gcagtggtac gatctcagct cactgcaacc    360
   tctgcctcct gaattcaagc aattctcctg cctcagcctc ccaagtagct gggactacag    420
   atgtgcacca ccatactcag ctaatttttg tattttcagt agagataagg tttcagcata    480
   ttggccaggc tggtctcgaa ctcctgatct caagtgatct ccctgtctca gcctctcaaa    540
   gtgctaggat tacaggcgtg agcgactgca cccagccatc ttccctgcat tcataattga    600
   tctcagaaaa tgttctgatg gagttggttc tctatcaytg ggagaacgaa ctcttataac    660
   ctcagacctg gatcccatgg catccctttc acccaggagc agtgtatgtt ataagaagag    720
   tgaaggagaa actttgatgc gctgatgaaa gtaataacta attattataa gcttgcttaa    780
   ccacaataac cgctattacc tagtaaagtt ggcataaaaa gtaaactaca agccagtc      838
<210> 36
   <211> 401
   <212> DNA
   <213> Homo sapiens
<220>
   <221> misc_feature
   <222> (201)..(201)
   <223> SNP150 = C/T
<400> 36
   atctgaaagt gcaccatcct gctgaaatca catcctgttc acataacact gcagtccata     60
   gcttctgaat ccacacagaa gttctcgtgg gcgataccaa atgccaagag gatgccttag    120
   aaggagtctg gagatactcg aggattggcc tctctagctc agaggttaca ctgtcatcaa    180
   atgtccaaag agctcttgta yagaattttt gtggtgcagt tctaaaggag aagtaggact    240
   gaggtccatc tgtgcaggct aacgaagaac agcccatact gaccaactgg ttgctacata    300
   ataatattag ctccccatcc gaggatagta aagctcaatt gccaggcatg ctgtagaggg    360
   catagctgtg gtagatgtgg gtttctacca tgttggtggt t                         401
<210> 37
   <211> 594
   <212> DNA
   <213> Homo sapiens
<220>
   <221> misc_feature
   <222> (394)..(394)
   <223> SNP151 = A/G
<400> 37
   ggtattcttt ttcaaaaaaa aaaaactaag agccagcatt tatagatata attctgtcct     60
   aaacacttta catataataa ttcttcattc ttcattacac cctgtgagat aggttatcat    120
   tgttataatt ccatttcaca gatgaggaaa ctgaggtact cagagactaa atgacttgct    180
   caaggtcact tgtgagaggt ccagctctag gccctctggc ccgtggggct atgctcttac    240
   ctagtacctc aaactgcctg gtagacctca accctgtgta cctgcacaaa tggtcctgaa    300
   gcaggcagct ttcttccctg agagctgcct ttccagatat gcactgccaa aggtctgtga    360
   ccctacagcc ttggcctgat ttggtccaga tgcrtgtgga catctctccc aaacaggatc    420
   aaattggcct cttctaggag tttagaattt ggaatcaagg gtgctggttt tagtctggac    480
   tggtcacaca gtttttgaca actgtttatt gtacgggaaa agtgtccagc ttgatgaatt    540
   ctcacacagt gaatttttct ttgtaagcag agccccagaa gcccttcttt gctc          594
<210> 38
   <211> 1350
   <212> DNA
   <213> Homo sapiens
<220>
   <221> misc_feature
   <222> (699)..(699)
   <223> SNP152 = C/G
<400> 38
   aaagagtgtg gactttggaa ctagcctgcc tgggctccaa tcttggctct ggtacctact      60
   agctgtgtat cattgttttc attgttttgt tttgttttat tttgttttgt ttgagacagg     120
   atctcactct gttgttgggg ccagagttca gtggtgcaat catagctcac tgcagcctcc     180
   aattcctggg atcaagtgat cctcccacct cagcctcctg agtggttagg accacaggca     240
   tacaccacca cacctggcta attttaaatt tttttgtaga gatggggtct tgctatgttg     300
   cccggatggg tcttagcctc ccaaagtgct gggattacag gcatgaacca ttgtgtccca     360
   ccttagctgt gcactttaaa taagtcactt gacctctctg ttcctgtttc ttcatttaca     420
   aggtgaagat gataatatac tacttcttag ggtgagtatt aaataagtta acctatgcaa     480
   agaggttaaa acagtgccta gcatatcata gaaatgtttg cttgacccag aggcctgaac     540
   ctgctttgcc ttcagtgttg gtctttaggt cctggcttca ctccttaatg ctgcatctca     600
   gcctttcctc atgaaacttt ctgtctttga tccttttcac tccttaactg tgaatctccc     660
   aaattccact gagcagtgat caattcttgg ggcatcttsc tctgtgttcc cagacattcc     720
   ttcaccctca ttttctctcc atggccaaat cctatagtgc ctcctaaata attatagctg     780
   acatgtatgt agcacctatc atatgccacg tgctgttcta agtgattgat atatgctaac     840
   tcatttaatc ctcaccctat gagaaagatg cttttttaac acctatttta tagaggagga     900
   aactgaggcc cagagaagtg aactgacttg ccaggtcaca taactaataa gcagagtctg     960
   gatttgagtt tggctctaga gcctgctctt agccaccatg tcccatcctc actgagagga    1020
   cccttgttca actccactaa ccctctctgg acaagtagcc tctggtcctg tctccctgct    1080
   tcctctctgt ctcactggcc taccctttcc acacctttct aaaaaccaaa tgtgatcatg    1140
   tcatcctctt tgttgaaaac atgtcaatgg cttcccattg ttcttgggac aaagtaaata    1200
   ctctttgaag tggctgcgaa gcagggtgga catacgtcct cctctgccaa gcttgcctat    1260
   aaaccaaatt ggtttgttct gttgtgtcag cgtaccacaa ggctggtgcc cttgtcatcc    1320
   tcacaagagt ccctgttgga agatacagga                                     1350
<210> 39
   <211> 668
   <212> DNA
   <213> Homo sapiens
<220>
   <221> misc_feature
   <222> (292)..(292)
   <223> SNP155 = C/T
<400> 39
   tgattttttc tcttgggaat aataaaatca agaggtttta tgttctgtgt gcaggacact 60
   gtgcttgagg ttttataaag tggatacggt tattggaccc attttatggc tgaggaacca 120
   gagactcagg gtggttaagt cacttccctg aggtcacaca gctaactctg gagcctgcgc 180
   ttgttaattt ccctctgttc tgagggtatt tccaactggg accttcaatt taaccttgcc 240
   tacctacccc atgggaagta gagcctcagg agctttcata gcttgtaaat gyccagggat 300
   gacacagtct gaacttgcaa tttgcttttc ctttctcctc tatggatgct gttagcttgt 360
   gttttctctc tgtgtgcctc atttcagctt ccttattcct gcagttttac ttcagcccaa 420
   ccagatttca gcccacctgt gggcaaagct gcacagccca ttcagactga gagagacttt 480
   tctttttctt tctttctttc tttctttctt tctttctttc tttctttctt tctttctttc 540
   tttctttctc tttctttctt tctttccctc cctccctccc ttctttcttt ctttcttttt 600
   ttctttcttt ctttccttct ctctctttct ttctttcctt ccttccttcc ttctttcttt 660
   tcctccct 668
<210> 40
   <211> 842
   <212> DNA
   <213> Homo sapiens
<220>
   <221> misc_feature
   <222> (346)..(346)
   <223> SNP156 = A/G
<400> 40
   ttccacactg tggcccctgg gcatgaagga gggcccaggc cagggatggg acaggctcta 60
   ggtggcctgg aggtgtctag tcagtgtggc ctgggtgacg aggatctgat gtaaaatgag 120
   actaggggag ggattgaggc agagatggaa gctgtgtctt tttttttgtt tttgagtcag 180
   agtttctctc tgtcactgga gtgcagtggt gccatcatgg ctcactgcag ccttgaattc 240
   ctgggctcaa gcgatcttcc caccttggct gggattacag gcatgagcca ctgctcccag 300
   ccaggaagct gcatctttga atgcgatcag aaccaaaccc catagrggga gtgatggctt 360
   acaggccatg aggattccca gactcatctc aggcttggtc aatacccaca gcatcaaggc 420
   taggaaggga atgtattttg gttaaaggca ggactgatac ttatttattt attttgagac 480
   ggagtctcgc tctgtcaccc aggctggagt gcagtggcgg gatctcggct cactgcaagc 540
   tctgcctccc aggttcacac cattctcctg cctcagcctc ctgaatagct gggactacag 600
   gcgcccgcca ccacgcccgg ctaatttttt gtatttttag tagagacggg gtttcaccgt 660
   gttagccagg atggtcttga tctcctgacc tcgtgatccg ccagcctcgg cctcccaaag 720
   tgctgggatt acagacgtga gccaccgcac ccggccaggc aggactgata tttaacctgc 780
   atgccctggc actactgtac cagtggtgga aatattgaaa tactctgaaa ccgatgggta 840
   aa 842
<210> 41
   <211> 401
   <212> DNA
   <213> Homo sapiens
<220>
   <221> misc_feature
   <222> (201)..(201)
   <223> SNP177 = C/G
<400> 41
   gactaaattt ttcacacatc ctattgtgct tatttgccta tccttataga agggtgagca 60
   cagacatttc tgtgtccttg ttctaacatt tcatatctgg ataaagggag acaggagtct 120
   tttgtatttt ttggaattct gttttgtcag atcaggagtc ctgttcctga catttgggcg 180
   gggctacagt ggagacaggt scataacagg cacccctcaa gccagatgga gagcccatct 240
   tcctcacgtt ggttctgtgc atctcaaagt cagaaggggt cctgctcctc tctgaggggg 300
   cacacgtgtc ctatttggag gcagtagtgg cagcgtcctt cactcactct gtggcgtact 360
   ggccaccgtg aacacacgct gtggcaccat gcccctgccc g 401
<210> 42
   <211> 691
   <212> DNA
   <213> Homo sapiens
<220>
   <221> misc_feature
   <222> (223)..(223)
   <223> SNP178 = A/G
<400> 42
   ttctcaaaaa ggctgactac agcacatttt gggaaaactt ttgcagagaa aatagcatca     60
   tgaagtcaag tccatggtca cctaaatgca gttgagtgcc aggatggcaa agtggcaccc    120
   tatgcaccct atgtagggtg aaaggtagaa atgctggaag agcatgacaa gagtgtattt    180
   acagtcaggc agtgggggtt gcaatttggg aggatagggt garaagatca cttgaggtca    240
   ggaattcaag actagtctga gaaacatagc caactcccat ctctacaaaa aaattttttt    300
   ttgtttaatt agctgggcat ggtggcacat gcctatagtc cccagctact caggaagctg    360
   aggcaggagg atcgcttgaa cccaggagct cgaggcttca gtgagctgtg attgcactat    420
   tgcactccag cctgggtgac agagtgagac cctatctcta aaagcaacaa caacaataac    480
   aacaacaaca agaaatttac gatgagggat cttcatagca ttctttcaga ataagaaact    540
   acttaatgga caaaaagtag gtaaaaatat acagtaattt aacgatataa ctaagaaaac    600
   tgtgtaagtg tgtatagaga gagagtgaga actttgcctc ctatgtacat gtgatataca    660
   ttttttttct gctgtccatg gaagagttac a                                    691
<210> 43
   <211> 796
   <212> DNA
   <213> Homo sapiens
<220>
   <221> misc_feature
   <222> (596)..(596)
   <223> SNP179 = G/T
<400> 43
   caggcttaga catttcttca aaagtgctca ttcactgccc accattttga tggtgacttg     60
   gactggaatg gtagtcatgg tgaatgaaag aagtagacag agggccgggt gcggtggctc    120
   acacctgtaa ttccagcact ttgggaagct gaagcaggca gatcacttga agtcaggagt    180
   tggacaccag cctggccaat atggttaaac ccagtctcta ctaaaaatac aaaaaaatta    240
   gctaggcatg gtggtgcacg cctgtaatac cagctactcg gaaggctgag gcacaagaat    300
   ttctctgggt ggcagaggct gcagtgagcc aagattgtgc cactgcatga tcagtaatgc    360
   tctgttgccc aggctgcagt gcaatggtgc gatctcggct cactgcagcc tgggcaacag    420
   agcgagactt catctcaaaa aaaaaaaaaa taagtagaca gagaagtgat atattttgga    480
   ggctgagtca actaggcttg ctgatggatg gaatctggat tggctctatt gacagaatga    540
   tgctatatag ttaacgaatg gaattgattg tgctaatgac ttcatgaaat accctkgggg    600
   tagatcttcc cagagaatag ttttatcaaa tggaaagccc ttggctgtga ccttgagtta    660
   cattgctggc tctgctccta cctgaaataa atccctttcc ttctacgggg acaaataaac    720
   accattttca gctacctaag agcagcaggt gtataggaca catccttgct ttacaaagaa 780
   aatgtttggg ggaaga 796
<210> 44
   <211> 401
   <212> DNA
   <213> Homo sapiens
<220>
   <221> misc_feature
   <222> (201)..(201)
   <223> SNP180 = A/C
<400> 44
   tcagttttgc aaaatgattt ttttctgtcc tggggcagct ggggtgacat ttcagaacac 60
   caaggaaaag atttgctaca caaacagagc atttgtctgg gccagtaatt ggcttggaat 120
   aggagactgt tttatccgca tgcaagaaat tgtgtccctt tctttctagc aagagcctgc 180
   aaatgcttgt ttatgcctga mttataatcg ctgcatactg tagttgttga ttaaaaaaaa 240
   tgtttccaac agaccgaaaa ttgttctcag gctcacagag gaatttcagt tgaccttgaa 300
   catgagaaat gtaatccctg ggcgataacc aattagcacc cggaatgact cagaaattac 360
   tttcaagcct cttatctgga ggaattctga gtgacagtaa a 401
<210> 45
   <211> 528
   <212> DNA
   <213> Homo sapiens
<220>
   <221> misc_feature
   <222> (201)..(201)
   <223> SNP181 = A/G
<400> 45
   aatgctattt gctttctctc ttagcattga gttctagggc tctaaatcgc agacatgaaa 60
   aggcaaccat tagtgtattc ctgtttctga aagcacatcc cacacagcaa gataaaggac 120
   gagtttaatt tgtgcctcaa ttttaaaatg ttcgctcttt gatgcagtgt gtgctaggca 180
   ttgtcccctt ggaacaatgg ratatttcaa aggacaaagg acagggctcc tgtcctctga 240
   aggctcactg agtaggggcc acaaacccag gtgttttcag gagccaggca aacaacaaga    300
   aggcgaaaga gcttggcatc acccagtgtt agcagatccc cttcttcaaa agaagctggg    360
   gatctgggtt ttcatatctc caaattttca acagttggca actaattaaa tattaaaatg    420
   acaacaaact ctgtgcagac cagaccaaca gggttatcag acttcttttt ttttttttcc     480
   agggttatta gacttctgat ctagattcta gaagttaaat taacatgc                 528
<210> 46
   <211> 898
   <212> DNA
   <213> Homo sapiens
<220>
   <221> misc_feature
   <222> (698)..(698)
   <223> SNP183 = A/G
<400> 46
   gaaaatggcg gatgctttgt tagccatggt tcctatatgg taacagtgga atatgcaccc     60
   catgagggag aaataaacct tgaagtttta agctgctgtg gggaagaggg gatctcaaca    120
   tgcctgaaat cacaccggtc atctctgccc accctcttcc ccacacttcc aaacttgctc    180
   ctcctccgct tcttctcatc tcagtggaaa caaccagcat cctgccagtt ggtcagaaca    240
   gaggctgggg agctgtcctt catctccaat ctcccttctc ccttgcccaa tctgctaccc    300
   atctggggtc agtctgtctc ccaaatagat ccactttgtt cagtctacac tgcagacaac    360
   tgagtccatc attttttttt cttttaagac agggtctccc tttatcactc aggctggagt    420
   gcagtggtgc aatcatggct cactgcaacc tccaactcct gggctcaggg gatcctccca    480
   cctcagcctc ccgcgtagct gggactacag gcacacacca ccatgcctgg ctcatcattt    540
   ctctccttga taatgcagtg acttcctagt gatcactcag cccacgtgct ggggggtctc    600
   ctctccactc agagtgacct gttaaatatg tatggtcata ccatagccct gcttcaaacc    660
   tttcaaaggc tgtctgtcat gctcatagtc agcctgartt tcttaaggtc ctttcaacac    720
   tttgtctgcc tcctgtgcca tatctgcctt gcatttcatg atccagccag acgatgtttc    780
   attccttgaa tgtgtcttcc cctctgttcc ataaatgctg ttcctgctgc caataccagt    840
   ttatctactt cagcaacccc aacatacctt ttggacacag tatggaggac ccaatccc      898
<210> 47
   <211> 1097
   <212> DNA
   <213> Homo sapiens
<220>
   <221> misc_feature
   <222> (601)..(601)
   <223> SNP184 = C/G
<400> 47
   acatgagctt ttaactccac agttttctct ctgctaaacg tgaatccagt aacgaagtcc     60
   tggcagctgc aggtctccca ctcatggctt ccagtttttg cctcattcca aagcacctgc    120
   tgccactctg agccccattc cacagctcat ggcaaggagt ccctctccca ttgcactgag    180
   gacacacgtg gccctcactc atccatttat gcaacaaata tttagttggg aactatacta    240
   gttgtcagga aaaccatgga gaatgagaaa tattccgtgt ccacaacagt ctccaattta    300
   ttggaaggaa acaagtgaac aggcagttat cctccagtat ttatgggaag agaagtggat    360
   gaaggaagaa aatcagagga gaacctagtg cagacagaga gaatcgatga gggcttccct    420
   gagccatgaa aatgtgaacc ccaagctggg ttgctgaagg ggagagaata gccatttgta    480
   ccacagtagc agcagcaggt gcaaaggccc tgtggttgga ggggcctggg acatccaggg    540
   gagtgaaagg agaatgaagc tacattgcag agaagaagca ccctagcaat gatggagctg    600
   saaagccagg tagggcctaa acacacaagt ccatgaaagc acctcgatct ttaccctgaa    660
   aatgatggga tgccactgag acattttaag caggagggga cttggttgga tgagaggctc    720
   actgtggttg tgtgtgaaac ccagtgtaag ggaaatcact gtgccttgtg caggtgagag    780
   atgatggcag gtagtgatgg ttgagatgga aagaagtaga tgaattctag agacagctgg    840
   gaggaaagaa atgacagttc tggttatggt tgagacatga gggataaaag aaagatggag    900
   ttgcaaagga tgtccgacag gtttcaggct tgtggagtga tgcatggcta tgcttttcat    960
   ggggacaggg tgccggagct gcaggaacta cagcttccca ttgtgtgcct gtgccttcct   1020
   acaagatcgt cctcaaaccc tgagcatgtt ttctgtgtgc ttcctttttg cagagggcca   1080
   agatcagtca gggaacc                                                  1097
<210> 48
   <211> 954
   <212> DNA
   <213> Homo sapiens
<220>
   <221> misc_feature
   <222> (366)..(366)
   <223> SNP186 = A/G
<400> 48
   gctgaaagaa gcttcagcac agaagatcta tgtaaaaatg gtatttactg gaaaacagaa 60
   atagcccaaa tggtatattc ataataaata caaaaaaaaa cgaagtgact ctctcctctc 120
   tctttctgtc cctctctcat gcacgtgcat ctatctatct atctatctat ctatccatct 180
   gtctatctaa tctatcatct atctacctat catctttctg ttatctatct acccatcatc 240
   tatctatcat ctatcatcta tatctatcat ctgtttatca tctatcatct atttacctat 300
   tatctgtcta tcttattatc tatcatctgt ctacgtatca tcaatcaatc aatcatctat 360
   ctgtcrtcat agaaagattt tcataacata tcaaataaaa ataaaagcaa attatggaac 420
   attatgattg gcaatataaa atgtgtatag accaattcct tttatgtaat atactcatgg 480
   tccctgcttt cattaaactt tttcttcaac atttcattat gaaaatttaa aaaatggaaa 540
   agttgaaatg attgcataat gaacatctat aacacccacc acctggattc cacaagtaac 600
   atttgactgt atttgcttta tcatgtatcc atctgtctat ccgtccatca acgctttttt 660
   aatgcatttc aaagcaagtt gcagacatcc atacacttta catttgcaga taactgcaaa 720
   tgcactttac attcagcatg tataccattt aaccagaatt caatatttgc aatgattatt 780
   ttaaaggtaa aattacacac actgaaatgc agctatctta agtatatcat tctatgagtt 840
   ttgaaaaacg catagttttt aaaatgcagt ggaggtatac acattcattt aaaaaatcac 900
   acaggcatga aagcaactgt gatgtttagt atgttccatg ggcacctgcg ggag 954
<210> 49
   <211> 496
   <212> DNA
   <213> Homo sapiens
<220>
   <221> misc_feature
   <222> (201)..(201)
   <223> SNP187 = A/G
<400> 49
   aataatccca ggacactgaa ccaatgacat taaatgcctg ggtgtatcac taaggaggct 60
   gtggagggtc tggtggtggc cccttccact agggtacccc tagaaacttg gcatttgtgt 120
   gacactctcc aagatgctgg catcaacttt tgtagctcaa aattcagacc gtacaacaga 180
   gttgactata gtcttggcca rgttttccaa atattagtca tttatgcacc tgttatgggt 240
   ttttccttgc cattacatgc ctcctttatt cgatcatatt tttctttaaa gtgactttaa 300
   cttgacttcc ttttcttctt cttaacattg ccctaagcaa taatatatgt gaaatcacaa 360
   gtttgatgta ccagttatac atttatttta atgtgtttgg tttgatatac cagttatata 420
   tttattttaa tgtgttttag gaaaatatat taaaagaaga gtaattttgt tcttgtacta 480
   gtacctgaac cacatt 496
<210> 50
   <211> 1023
   <212> DNA
   <213> Homo sapiens
<220>
   <221> misc_feature
   <222> (370)..(370)
   <223> SNP197 = C/T
<400> 50
   catggtagtt cttcctcgac acagcactgg gtcattggct cattgagctc agagcccaag 60
   cttttgttag tacatcatgt tgagttgcca ccaaaatggc cttgacaatg atgatacaaa 120
   caagaacaaa aagcagcagc taacagtaat aggttgggca ctctactcag cactctagct 180
   gtattatctc attttatcct cctgatacct ctattggaaa ggtactatta ctaactttaa 240
   tttacagaca agaatgctga cattcagaga agttaagtaa ctggtgcact gtcacacagc 300
   tgctaagtag tggagctaag atttgctctg caagactcta gaaccccaaa cccggaagcc 360
   tttgctaacy tacctgtgcc ttaacttcac attcctggtt ttggcttata gaatattttc 420
   actttacagc caggcacagt ggctcacacc tgtaacacca gcactttggg aggtgtaggc 480
   aagtggattg cttgaatcca ggaggtcaag accatcctgg gcaacatggc aagatcccat 540
   ctctacaaaa aattagctag gagtgatgca cgcacccgta gtcccaccta cccaggaggc 600
   tgtggtggga gggtcaactg agcctgggag gtcaaggctg cagtgagcca tgattgcatc 660
   actgcactcc agcctgagct acagactgat actttgtctc aaaaaataaa ataaaataaa 720
   ataaaatata aaatattttc actcttacga tcacatctaa tcttcacaaa agcactataa 780
   gtattatggt ccacatttct attcaatgga aactgacccc cagaaagatg aaatggctca 840
   agttccacaa tcagtaagtg gcagttccag ggcttgagct caggttttct aatagccctt 900
   tgggtgtttt tttttttttt tttttttaat actatggaag ttactccgct tatcctacag 960
   ggatattgca aggattagat aagattttat ccatgggaat gattattaaa atttagggag 1020
   cac 1023
<210> 51
   <211> 600
   <212> DNA
   <213> Homo sapiens
<220>
   <221> misc_feature
   <222> (530)..(530)
   <223> SNP198 = C/T
<400> 51
   gaattgacgt tcctcccagc atgccttgca actgagttat tgctgggcca tggcagcaaa      60
   tttggggagg aagtaacttc tgtccttcaa tgactcataa aatctaattc tatttaaaaa     120
   aaaaaagagg tgcatatgtt caattgaaaa gaaaacagtt aaaatagatg ttttgctaaa     180
   gaaacttaac tgcctgtgcc cactggcagt tgggaaattg catgaatgtg ccgggctgac     240
   aattgtccct gagaaaaact gcaaaaggaa tggaagaaac ataattcttt cgtacctatg     300
   cagcaaggtg cacctccagt ccagctctaa taatgccggc aacctccatt tcatgtttat     360
   tatattttat tcaccatctc tttcaatcct ccctgattat gcccacttta aagatgatga     420
   aatccatatt cagagctgtt aagtgacttc ctagaggtca tctagcaagc aatcacagat     480
   ccagattcac ctgactgtgg agtacaggct tattatccct ttgtgcttcy aggacaattt     540
   gcctaaacca tctaatgagc aaatctctga aaactcaacc cccttctggg agcttttgca     600
<210> 52
   <211> 841
   <212> DNA
   <213> Homo sapiens
<220>
   <221> misc_feature
   <222> (452)..(452)
   <223> SNP199 = A/G
<400> 52
   ggttgggaga caagtcacgt ttgggtggat ggtcttaggc agtgcatcat aatctggtct      60
   taccctacct ttcccctttt ttcttttata tgactccatg aattttactc cccctctcca     120
   gcaataatag ccatcaaatc aggaacgtaa gtgcttagtt gtattttcca caatatacgt     180
   taaaacaaat acaaagccat aaaggtgaaa ccatcataca tccatggact acctaaaatg     240
   atctcctacc cattttggga aacacggccg tttggaaatt tatctggccc ctgtccctcg     300
   cctgggaatc cttaacatct ggcactgtgg attatccaca gaaacatctc tggcaataga     360
   gttgctcctg gcatagagtg ggcatcatta agtgtttgtt cagaaataaa aggacagtga 420
   gatgccaacc gtaacattta atagcttagg crctggtgtg ttgaagccac atgctcagta 480
   cagttcaaag atgatagatc ttagggtttc cctgcatatg gttagcggta gaaacatacc 540
   acaaaacacg accttaccta cagtggagcg atcccctgat gtggggattc cgactaatga 600
   gtctgggggg caagccgagt tcttccaagc agaaagcaag caagtgaaat ctctctctca 660
   cttacttctc tctctgtggc caatgccatc tgctagcttt aaaaataatt tttaaaacaa 720
   gtgttttggt tctcttcaac gtgcagcaaa gacagatgct atttttaggc ttgttttcta 780
   ttaaaaaaaa aaaaaaagcc aaagaaccag gggctgggtg tcggcagttc tggcttactg 840
   t 841
<210> 53
   <211> 965
   <212> DNA
   <213> Homo sapiens
<220>
   <221> misc_feature
   <222> (501)..(501)
   <223> SNP200 = C/T
<400> 53
   gtggttgtgt gaacctgtag tcccagctac tggagaggct gagacaggag gattacttga 60
   gcctgggagg ttgaggctgc agtgagcaga gatcacgcca ttgtactcca gactgggcga 120
   cagagtgaga ccctgccaca gaaaaaaaaa aacaaaaaaa ttggttaact tgtgctctcc 180
   tgtaacctgc cagcttctgt cttgatgtac cactaaatct ccatgttcct tctctcccat 240
   cctggactgg aagctctttg aggggaggac acatccacac atccatacaa tgtctagctc 300
   agaacctttc acacaagatg tacccactgg aggcagcatc atgaggggtt gagagtgtag 360
   agtctggaac ccaatgccta aggttcagac cctggctttg cagcagctta tcagctgaga 420
   gaccactggc aagttactta ccctttctga gcttcaactt tctcctcttt agaagggaga 480
   gaatgattat acttgtcaca ytgagttttg tagagggaca agtgagacaa aatatgtaaa 540
   gcattcagct catcacgtgg taagtaccca agaaagctta atttcattat tgttgttgtt 600
   ataattatta ggcactccca catttgataa atgcacatat gactgttaca tataagataa 660
   agttctatgt gctgtatgga agattttttt aaatggtgaa ttaaatgctt gttatatggc 720
   cagcatcatg ttagacactc tacacatgtg tccttgttta atattctgtg ggcaagtgaa 780
   aaatgtccca tctacaggtg aagagaaagc ccaaaattag attaaataac tgctcagtgt 840
   tgcagttagt aagtagtgca acaagacttc aaatactgta ttgacttaaa aggtgaaatc 900
   tttgcttcac tttcccacca cctcttctcc cggttttata gcccctcaca atagctttgc 960
   aactt 965
<210> 54
   <211> 929
   <212> DNA
   <213> Homo sapiens
<220>
   <221> misc_feature
   <222> (648)..(648)
   <223> SNP201 = C/T
<400> 54
   gaaaaaatat ttagccaggg aagaaaggtg ttctgcttaa tatgagatac ttcttttttt 60
   tctgacacca gctaagaacc tgaagctcag acagacacca tggctcatac ctgtaatctc 120
   agtgttttgg gaggctgagg ttggaggatc acttgagctc aggagttaga ggccagcctg 180
   ggcaccatga cgagatctcg tctctataaa aaataaaaaa cttagctggg catggtggcg 240
   cacagctgta gtctctccta aaaataaaac aaaactcaaa gctctaaaac cacagctatt 300
   tatttatttc taattcatgt ccctgactgc ttagaaaggc cctatatctt ccttggggat 360
   tatcattgta gattttatgt gtccatttgc taatatttat tgagaactta ctataagcca 420
   ggccctaagt gcttaacaaa catcgtctca cttaaccttg agaataactc tgggagggag 480
   atgctaccat ccctgcttta caaacaagga aatggagatg caaggttttg aaagcttgcc 540
   caaaatcatg gagctagtaa gaagcagttc ccaaattctg accccggttt ttctttgccc 600
   atgttcttta tcttacacca ctctccgtct tagatgcctt cactccaygg gggtgaagga 660
   tcaaggtgct cggtggcaat ggtctgtggt tcctgtgatg gggagactga ttctgggttg 720
   ttggaaatgt gtttcctctg ctgagtcaat tccatcccct tcggttcaaa aggcagctac 780
   agtctcttct ttttagaaca cagatttcat catgtcattc ccctgcttag gaacatggca 840
   agttatagga attagcaatg ataacctctc ctggggagtt ttctaagatc ttattgcctc 900
   tccagagagc ccccagagct catctagaa 929
<210> 55
   <211> 401
   <212> DNA
   <213> Homo sapiens
<220>
   <221> misc_feature
   <222> (201)..(201)
   <223> SNP203 = A/G
<400> 55
   acaggcatca tgggtgattt ttcactctac atgattttca acttgggtgc tgacattagg 60
   acctaactcc tatgtgggat gtgcctctac taaaatagaa ccaactgggt tcttgagaag 120
   attaaatatg gaactacaag tttattggaa gaaaatccac tcccattgta ggaaacagga 180
   gaggaaataa ttagtgttcc rgtcaattga gttgtgattc agggctagtt tctaatcaat 240
   caggaagccg cactgggatc tgggatccat ggatccggct cactggccag gcaggtggta 300
   aaattaaaag attagcattt caggtgctaa gatgatacct attccaaagc cccttttcct 360
   aaactaacta cacactctcc ctttcatctt ccactattaa t 401
<210> 56
   <211> 858
   <212> DNA
   <213> Homo sapiens
<220>
   <221> misc_feature
   <222> (273)..(273)
   <223> SNP206= C/T
<400> 56
   gtgaaccaaa acctcactgc cctttctttc ctcccatcct aatcttacac aaatgtcaag 60
   ccttggctca atttcctcac cctccataaa cctctcctga caaagccagg ccacaaaaat 120
   tcctgagaaa accacgtgtg aaatgcactc ccatttttct taaaagccca agttttagag 180
   ctaggcagag gtggagtctg cacccagctc ttactgactc taccacttcc tctctggagt 240
   ccttactgtt cctcaacttc tctgagtgtt caytgccttt ttattcaaat gggaaaaata 300
   gtatactcac ctcattgggt tattatgatg atcagggaag ctcatgcatg taaataaaga 360
   agtagcagct cggcgcggtg gctcttgcct gtaatcccag cactttggga ggctgaggca 420
   ggcagatcat gaggtcagga gatcgagacc atcctggcta acatggtgaa accccgtctc 480
   tactaaaaat acaagaaaat tattagctgg gcatggtggt gggcgcctgt aatcccagct 540
   actcgggagg ctgaggcagg agaatggcgt gaacccggga ggtggagctt gcagtgaccc 600
   gagatcgcgc ctctgcactc cagcctgggt gacagagtga gactccgtct caaaaaaaaa 660
   aaaaaaaaaa aagaagtagc tagcttagaa tgtaagtgct cagttaattt ccccaccctg 720
   gaggatacac tttatatgac aagtacatgc ttgtgtgtga gaccacaaat tatcttgcga 780
   gggggcaaca ggctgagtga ctgtccaaca gtgtaggaat gaatttctgg gttcattcct 840
   tccccatcat cagcaagt 858
<210> 57
   <211> 550
   <212> DNA
   <213> Homo sapiens
<220>
   <221> misc_feature
   <222> (89)..(89)
   <223> SNP207 = A/G
<400> 57
   ccagccccct tataagccaa aagctgtaag tagcccattc tctctgactg ccgggtattc      60
   acacacaagg tattcttgcc tgtgcctcrt gttttccaaa tgctccctga ggggtagacg      120
   tcaatgtgtc tactggaaca tgggtgtatg tattcacaca tatgcacaaa atcactggtt      180
   actaagatgg acattttcca ttggcccagc ttagtctcta taaagatgga aacgacgatg      240
   cctattgaat ttttgtttca tcgattcaat tattgtaact gtcttagctg acaactttct      300
   gagaatggtt cttgggtgag cagagttcag atccattttt ggactgtgat aaaggatcta      360
   attttgccct cattctttct cttggttcaa ctgtgtcatt ttttcttcat actgacattg      420
   aaaaggtcat gttgtttatc tcaacagtat ttaaatttaa ttctgaaaca tacactatgt      480
   ctgagtcagt aaataagatg gcagttcagc agccagctcc cacccaaggc aaaacagagt      540
   gaagcagaga                                                             550
<210> 58
   <211> 1348
   <212> DNA
   <213> Homo sapiens
<220>
   <221> misc_feature
   <222> (251)..(251)
   <223> SNP209 = A/T
<400> 58
   ctgatcgtca atgctctctg ggcagggctt gaggagtaca acctgatgag aagcagacgt 60
   taagatcccc tttcctccac tcctgacatt ggagcaacca tccagtcctg tctagacatc 120
   actacaatgc tcattccctc cttccttcca ttctagcctg tgtcaggcct cactaactcc 180
   aaacccactt gtatgacaga ctgattccaa tcatttctgc tcttgttacc gattcttatc 240
   tgcactttac wtaaagcccc ataattttga ggcatctgga cagtaggaaa ggctgaaaac 300
   ctggcaagct tttatagctt gcaaaggtcc ttgtcctatg ctctgttagt atcatttcca 360
   aaatactact tgggctatct gatcacatcc cattgcccat atcctctgag agtctaaatg 420
   ctgaactgct tcagaaacta aatcatactc acaggacttg gtttctcagc aattttatca 480
   tctgaaaaca ttgtttatgt agctgaggtc aaaagcttca agtactgctt taatgagctg 540
   cgaatctgtt catgtgacat tctcttccaa acccaattgc cctttacact gtatgagtaa 600
   gtgcctctgt gtgtgtgtgt ctgaatgtgt gtgtttgatg tgctgacatt tctgaattga 660
   atccagaaag tgtaaccttt gtatgtttgc tgttaaaact ccaaattcaa gccttaagaa 720
   aaggatctaa cacttactca atgtgattta tttaagcaca gaacctaact cccccaaagt 780
   atcaaatact caagagaaca attcattttt gaaaattgtg atttaaaata attctcataa 840
   aacctttact gttcaaatat attagaaaat atattgcatt tttgtctgca tcaggcactg 900
   ttttaactgc cttccatgaa ttgcctcatt taattctctc aaccctgtaa ggtagggtta 960
   tcaccacagt atagatgagg aactagagct tggaaaagtt aaggcatttg cccaaggtcc 1020
   agcaactaga aagcagagga gccaggactt gatccctgga ctgtacactg cacaagaccc 1080
   ttgtaaagat tattagaagc acttggagcc cctggtaagg tttgtgattg tgctaccagg 1140
   agagtcatga taacaccatc gaatggtgtc tgttacataa tagaaaaaga ctgagtattc 1200
   ttacaattca aatgatttaa atttatttta aaatattgca acaaataagt agcatcttca 1260
   cagcttttat aattcgttgt caggatttta aaagatgcaa atcttatttg ttccttaggg 1320
   ctttgatttt gaggctgaca aagacggc 1348
<210> 59
   <211> 401
   <212> DNA
   <213> Homo sapiens
<220>
   <221> misc_feature
   <222> (201)..(201)
   <223> SNP210 = A/G
<400> 59
   gcattcgagc tttgttgtga acaccggaag taacatgccg agcgcctggg ggatggaaac 60
   tcctatagca ccccacaggc taacagcaag caggacaaga caaaaagggc aggtgggaca 120
   tggtagagat ggaccctacc caggaaacag ctccatcagc atcttagcct gccccactct 180
   agccacacat acccacgtgt rctcctgagt tcagtgtgcc cacctcactc ccacaccctc 240
   acatagactt ggcaagagta aggagggaac tccatagaga cattttacct atctcagggg 300
   agcagccaca aagaagcaag tcttgtaaaa ggtcttttgc aaaggagagt gaacccagca 360
   atgagagatc cttaacagct agtgcccatt agggggctaa a 401
<210> 60
   <211> 511
   <212> DNA
   <213> Homo sapiens
<220>
   <221> mist_feature
   <222> (201)..(201)
   <223> SNP211 = A/T
<400> 60
   ggctaaacct aaagcctggg tggtgatggc tcaaacgcta atgagtcagt gaatccttac 60
   cgaccccctg gcctttataa tctgaggcaa ctttggctgc agcccgggaa tgtgcagggc 120
   actagggaat acaaggcctt cttccctggt tgtcttgtaa taaaacagcc atggggttgt 180
   ccctccagtc cgagagactg wgatgaggcc tacatagcag cgatgtggtc aggtaaaaat 240
   caggaaccca ctgaaatctt gggcaagcca ccctgcctgc ttgtgcctcg gttctctcat 300
   atgtcatata taggaggtga ggactccagc tccacctgcc ccaggtgggt gtggtgatga 360
   tgaggaaaga caagaggctt gcaaggaccc tgaagaggtc ggagcatcat acagattcct 420
   ttattagccc acattctgat gttccctggt gagacttgcc ccaagcaatt gctagtaaat 480
   gggggttaat ttcttctcca cctccctact g 511
<210> 61
   <211> 401
   <212> DNA
   <213> Homo sapiens
<220>
   <221> misc_feature
   <222> (201)..(201)
   <223> SNP213 = C/T
<400> 61
   acattcaatg acttacttct tttcttagaa aatttccacc acatttctat ccccaagcca 60
   acatacaatg tgaaatgaaa gccagtgcgt ggagtgcagc tgctaaaaat tttcagcaca 120
   gggctctttc tgactctgct catgagatgg tatcagccac ccaatgactg gcgtatcttg 180
   gtcctgtgtc tttcttctta ygctgtgtta atgtgtttac tttccatttg gcagagagac 240
   aagagagaca cctccaactt cgacaaagag ttcaccagac agcctgtgga actgaccccc 300
   actgataaac tcttcatcat gaacttggac caaaatgaat ttgctggctt ctcttatact 360
   aacccagagt ttgtcattaa tgtgtaggtg aatgcaaact c 401
<210> 62
   <211> 731
   <212> DNA
   <213> Homo sapiens
<220>
   <221> misc_feature
   <222> (201)..(201)
   <223> SNP214 = C/T
<400> 62
   atctcaacat ataaaggcat ggtaccgaag tttaggcttg tcaattggat gaccacagcc 60
   aacttaagaa cttcaaaccc ttgaggacaa atttctcaaa tcctcagagc ttattcctgc 120
   catgttcttt ctctaacctc acatacaagc tcagatgctg gtgaaatcaa gaagaaatgc 180
   aaagcaagat aaataggaac yaaatctggg ataccaatcc agttccactt gtcatgtcct 240
   ttgcaaggac atagatggag ctggaggcca ttatcctcag cagactaaca caggaacaga 300
   aataccacac attctcactt ataagtggga gctaaatgat gagaacacat ggacacatag 360
   agtggaacaa cacacactgg agcctatcag gtggtggagg atgggaagag ggagagggtc 420
   aagcaaaata actaatgagt actaggctta atacctgggt gactaaataa tctgtacaac 480
   aaatccccat gtcaccagtt tacctatata acaaacctgc agatgtaccc cctaacctaa 540
   agtaaaagtt aaagaagcaa aatgaataaa tgccctaaag ccctgtacct caggtccttt 600
   atctttgctt gttttgatgt gggactgcat tgacctacac agaattctgc tgctcattac 660
   ttcctagaca agcaatgttg gctccaaaat ggaatcccaa tttccaaaca caacaaagtt 720
   ttagctctag g 731
<210> 63
   <211> 387468
   <212> DNA
   <213> Homo sapiens
<400> 63
   cagaggagga tgggagcgat gtgaccacaa gccagggaag ccagggaatg ctgacagcct     60
   ccagaagcca caaaaggcaa ggaagcgttc ccctctgaaa cctccagagg gaatgtggcc    120
   ctgctgacac tgtgacttat ggtttctggc ctccagaatg atgagagaat aaatttttgt    180
   tatttctagc caccctgttc tgttataatt ttttatggca gccacaggtc tggagcaaag    240
   ggaccttgga ctccattctc agccacagca acctgccccc tcactgcctc tgaaatcttt    300
   caagtgacca gcattcactc cttcatgagc agagtgggcc tggggggctc tggtggcaag    360
   gaggacccat gcccaatcta cggggagctg ctgccctgca gctcctgttg gcactatgcc    420
   atgttgccct aatctcttgc ctgatctgct cagttgacct cttcactgct cttcctgcct    480
   ccaccctttc tcctctaatt tccacatagt agctagggta agcttttaaa atgaaaaccc    540
   cataagttgt atcaggccac tcctcagctt ccaaccctcc aatggcttcc attcactgga    600
   gtgaaatcca aactccttat cacatttgca aagccctttg gggccctgct acctccctga    660
   cctcatctca taagtcctct ctcctctctg ttcatccaac cactgaggcc ttctcgctat    720
   ttctccaaca taccaagctt gatactcccc taggctcttt gcattttctg ttttctctgt    780
   ctggaaactc tcgatagtcc cctggctcac cctctcactt tattcaggcc ttactcagat    840
   gtccccatat tataaaggat ttccctgtgg tctttataat aatgctttat ttttatttac    900
   aaacattcta ttatacacca ccaggtatgg attcgttagt tggttaggtt attactcccc    960
   cttccccatt agactataag ctccactagg gcagggacct ttgtctggtt tgtttaacag   1020
   tatctggaac agcacacagt aggtgcttaa taagcaattt gttgattgaa agaacaaatg   1080
   tatgagtgag ggaggaatgt aggctccatt tagctctatc tttttcccta gataagtcag   1140
   aatctggata tttaagtaaa attctatgac ccgtgaactt aaaaaacaac aacaacaaca   1200
   acaacccact gggctagctt aaaaaaaata gagcatctgg agccaccagt ttgagaccct   1260
   gacctacagg aatctgggtc caatgggaac cacatttctc atcctcactt ggtgcaacgc   1320
   cttcaggaac caagtggctc ttttccatcc acagcccttg catccccagg ccccggcgcc   1380
   atgaggtact gtttgttgtt acagaccaaa gtcatccagg gcacttcatt caaaaggcag   1440
   gatgtgggct gtgagcttcc agctcatcag ttcaaaggaa atctacttca gcagcaatgt   1500
   ctcctgggat gttttcccaa gcctccctgg gaggtggtgg ggcctcttca aggactaata   1560
   atagacaggc acatccagtt tcccaaacat gtcaacctta aaagtgaatg ctattttttc    1620
   ctccttacct ttgaattact atgtaggaga gggacattct attattgcag cccttgctct   1680
   cgacatgcta cctaataatg acttaccact cagcagcttc caggcaaaat tgtcatagtg   1740
   ggacctgagc tcgtgcaact ccagcctgcc ccccttcatc caccactact caggttcacc   1800
   cctttgtcac caggatgctt tgtagtcagg ccagcccaat caccattcag caacggtatg   1860
   actgtgggtg tatcttaatc actctgaacc tccccatcct tatccataat gaaagttacc 1920
   agatctacat agggttgttg cgggggctaa ataagacagc acaagtacct gatgcccagc 1980
   aatcacttga tatttattat taaacaccta ctgggtgtga gtgggggtaa gaagtgccga 2040
   cctttaagtc ttcattcatt cacttgttca tccatgccct cactcaatca accaatattt 2100
   actggggtct tgggaggtgt caggcactga gcttggtgct gggcgacatg gtggtgaaca 2160
   gcccccctgc tctcacagag cctaggtctg gtgaaggagc tcacagccta atgaagctct 2220
   gcgttcccag agtgggggac atggtggcag tgggtatctt taggccttgt gcatgaacag 2280
   ggcgctaaat aacattgaat tacacattgt agacaattat taccctttca gttaccttca 2340
   gatcacatca aggagagagt cctggttgga tagtaatgtc tttaacaccc ctctagcatt 2400
   tattaatttc ctctcttaac aaataaaaga tgacttcagt cgaagatgct taggacagat 2460
   gacggcacct ggagatattt taataatgta gatacctctt gctgttcaaa ctcagaccaa 2520
   aagagatagg ctttttttcc cccagagggt gcacaaatac gaccagaatt tgtgaagacg 2580
   agtcagaaat gaatgaaatt tggaaaaata ttgatctact gaaatccttc ctccccacac 2640
   tattagccct atgttacagt tggggaaacg gagtcgtttt gcagagggga tggacagaag 2700
   gtagggagtt ctcttccaaa cgtgcaggag gcaagcaaag ccaagaatct tctctgtggt 2760
   ggagttagag acatataaaa taaagatcgc tcctccccta cctctgcaga acgtgtgtgt 2820
   gtatgtgtgt gtaagtgtgt gcggccacaa gcctttccga atgagtgaca gcgggagccc 2880
   atccctccag gagacgcgtg cagaatgacc aatgggatgg atgggggtgg atgggtacca 2940
   gtctccgcag aggccggggt ggaattcgct gcgccccacc ccttccaccc gctccccttc 3000
   gccccgtagg tctttccact ctcgctcctc ccctgggcac atctcctgaa cgcagccccg 3060
   ggggccgagg acggggtggg gtggggggcg aggctcgggt ccgacgaccc cgggctgcgg 3120
   tcccggcgct gcagagctgc ggctgtgcac gcttagccgc gaggcccgcg gtagcccggg 3180
   cgccgatatg taaagcagct ggcagcgctg ggcggggcct gggcgcgatg caaatgagga 3240
   gggcggggct ggcccggggc tccgcctccc tcccccgcag ctggggccag cggtgccaag 3300
   cgcagctgga cgagcggcag cagctgggcg agtgacagcc ccggctccgc gcgccgcggc 3360
   cgccagagcc ggcgcagggg aagcgcccgc ggccccgggt gcagcagcgg ccgccgcctc 3420
   ccgcgcctcc ccggcccgca gcccgcggtc ccgcggcccc ggggccggca cctctcgggc 3480
   tccggctccc cgcgcgcaag atggctgacc cggctgcggg gccgccgccg agcgagggcg 3540
   aggagagcac cgtgcgcttc gcccgcaaag gcgccctccg gcagaagaac gtgcatgagg 3600
   tcaagaacca caaattcacc gcccgcttct tcaagcagcc caccttctgc agccactgca 3660
   ccgacttcat ctggtgagcg cgcgcgcgca gggcaccttc ccgggccccc gagggcagcg 3720
   ccgcgccagg gaccccctct ccgcgccctc tgcgccctcc gcgccctccg caccctggga 3780
   ccccgcgtct ccggactccc ggctccggac cctgctgccc gggactcccg gatggacagt 3840
   cctgccgttg ccctgtcccc accctggtcc cagacgggcc gccgcggggc gcctcctgcc 3900
   ctctcctgct ctcaggcgcc tctagagcgc ccaggggcgg cgtcgcggga gcctttgctc 3960
   cacctgacta ggagcgcgcg gggtctgtgc ctgccctgga gggcagcgcc tcgggtgctc 4020
   tccgacccgg ggttccctat ctctccgcct gcttccgggc gcgaggagcc ctcgcccccc 4080
   accccttgtt tccggggggg gcggcgccct gggtgtcctt ctctatctcc ctgcgggcat 4140
   gggacatcct ttctcactcc tctgtgcctc cgggcagcgc cctgtgttat ctcccattgc 4200
   ccctccccga gggcctgggt tcccctttcc actcctcggt cacatcactg cgggcccctt 4260
   tcttccccag tccctccagt agtggggcat cctttcctcc ttcccagtcc ccctcccaga 4320
   ggacaccacc gccgcggggt cactctcgcc ctccctctga atgcgtcttt atctcttctc 4380
   ttttcccgag ggtgctcggg gcatctatgg gtacatctgt cgcctgcctt cagcccctac 4440
   cccgacggaa acgctcccca ctatcccgcc acctggtggt cgcagcctcc tctcttctgc 4500
   aggagtgaag gcagatgggg gttacagccg agctcccacc tacccccaca aaggcggaag 4560
   actcttgggc acccgcctgt ggctgggagt ttgcacctgg ggtacagagg cagggaggaa 4620
   ggcgggtgac tctgtgggta actagctgga ggctgggccc cccgggctgc ctgacataca 4680
   cctccttctg cttttgcagg ggcttcggga agcagggatt ccagtgccaa ggtaggctct 4740
   ggggctttgg ggatgctatt tgtgggaaga gagggtgaaa aatactttat agaagaagtt 4800
   actgagttag gcagagagtg aaagaatcac gttggtcgga gtgacctccc aggctaggaa 4860
   ttcttcacca caacagggtc ctttcaaggg gtgtgtgtgt gactggggcc gatggcgctt 4920
   gggagtctta catgccaagg aagttcacct acccttcctg ccttcccggc tctggaagag 4980
   tcaaagcggt ctcctgaagc aatcctggca tggtcagttc cgctggggga gaaagtgttt 5040
   tcccgggacg tttctgggag aacctgtcct ccttagttcc cctttctctg cccatgtccc 5100
   ttccttatct cacccaggga ggttctgcct ctccctgcct tgcagaggtc tctgcaggtg 5160
   gctgccgctc ctctgcagat ggtgcatccc ctagaaaggc gactgtgttt gtcgcctggg 5220
   cctcctctct ctctgaaaga agtatttcca gagggagtgt ttctcagatc ctggtttaaa 5280
   tcaccctatc ttctgggttg aaacagaaag gtctccaggg cactggttac ctgagttctt 5340
   gcatctggct tgagatcctg gactctaacc aggggtatca aacctgctgg tggtgagttg 5400
   atcacgtgta aagtgtcccc ctcccccgtc tttggggctc atgtctaaaa gacagattgc 5460
   aaattggctc tccaggacca aaagttaccc gcagctctgt gtagccctta gggtgtctta 5520
   cttgtgaatt tgatgtgaac gttttagaac tgggagattt cacatacaca tcctaatttc 5580
   caactttaac tttgctgaga aatggtctca ctttcgcttg aaaacaatcg tttggagctg 5640
   agagccgtcg tcgtagatgg ggaacagcct taccattttg tctcagttga tcccagtggg 5700
   gaacacttca cccatttctg gcccctgtgg gcatttaagt ttgcaacctc tcatctggaa 5760
   agtttgtgtg ttatgttttc tttttaattt aaattttaat ttttcactct tgggcttggt 5820
   aagattgagt ttggtaagaa acggcacatt tctggggtat tgtatcttta ggggcaaagt 5880
   gaccaccacc ctggtgagct gggtttaaat caaaagaaag tatttgagtg tggaagagtc 5940
   tgacgtgatt tagcactgac tgacagtttt gtggctgcac ctccaccctc cagggaaaag 6000
   taaagtgatc cctagtgagt gtaggtggca tcgtcagtgg tgcagaatgt ttgcttgctg 6060
   gcagcatggc actgtgctcc tctgagactc ttgcatactc ttctgagatc ggggtcccgg 6120
   tgggctcagt gagccctgcc aacctaccca gcctgtctta ccaagatttg gatcctctgc 6180
   accacaccag aggactcctc aagtctcttt aagtggcaac aacttaggcc ctggtgcaaa 6240
   caaatttgga ttgttttcta gaactttgac ccctcaaaca aaatgccaat ggtgtcttgg 6300
   caccggaatg tgtaatttct tctttgaggt tgcctttcac ttccatgaat cagtttcagt 6360
   ctttgtctct tgctgagatc gggggcgatt tttaaacatt tttaccacgg atcttctatg 6420
   ccacctgctc tgctgttatt gatggatggg aaagtcactt tggaatgtgc aaggcatgac 6480
   acatttgaaa taggagatcc tttaactcaa agtctggaag ataataggaa ttttctgatg 6540
   ttaagagaaa aaggcagaat taaagtttgt aatgaaatat gggcgctcta gagataggag 6600
   tgtctttttt ttttttttga cagggtctca ctcttttgcc ccggctggag tacagtggta 6660
   tgatcagagc tcactgcagc ctcaaactca tgggctcaag tgatcctcct gcatcagcct 6720
   cctgattaat tgggactaca agtacgcacc accatgcctg gctaatttta aaacaacttt 6780
   tgtagagaag gggtcttgtg atgttgcaag actggtcttg aactcctggc ttcaagcaac 6840
   cctcccacct cagcccccaa gtgttgggat tataggtgtg agccactatg cccctctgga 6900
   gtgtctattt tttaaattgc ctttcttttt ctcttccctg cctccccacc aagaggcagc 6960
   gtagagaagt gggcatttgc acaggctacc aggcaagtcc ctgtcggtct gaatcctagc 7020
   tttgagactt cccaggcata ttacttagcc tctctgtatt tcagcctcct tgtctataaa 7080
   acggagtcag taacagtacc atagtgtggc tgagcagctc caactcattg caatatccaa 7140
   gattcttgga ccagagcttg acacagagta tgctcagaaa atatctgctc ttatgattgt 7200
   ccccagacta ccccaatggt ggggcagctg gcacgagaga agactggctc tgcagggttg 7260
   ctttgctatg gaactgcctg ccaggtaaga gtcaaaactc agccatgaac ttctggtcac 7320
   tctgtgtagc tgctttgaca gcatattcca tttgggcagc agtagggagg agtgagggga 7380
   ggggcagatt ggcagtccct gaaattgaga tgtttagaat gggaacatat aggaaagagg 7440
   gaaatttatc cctggtactc agcattgcct gggactaatt ctggaaccac tgaggagttc 7500
   cttagtatct gattgttctt ggtatcagat atttcctctg aaagtaaatc cacttacgat 7560
   ggaatcaaca gtttatacaa aacacataag gttttatgat ctcacttaag tcagcaatcc 7620
   agagcccagg gcctcaggtt cctttgagtt ttctgctcca ctatccctgg ggagtgacaa 7680
   ttatctcatg gtccaaaaag gatgctagac gctggagtcc tcacaccctt ctttcagcta 7740
   gcaggaagga agaaaggggt aaagaagggc atatagcaca tgctcggtgt cacttgttta 7800
   agttgctcaa gatccttcta cctacatcct cttagctagg gtttaatcac atacccacac 7860
   cttgctgtga gggagcatgt gggaaatgta gtgtttattt caggcagcca ctatttagag 7920
   gatgtttcca tttcaaaaat acaagcagag agaggatgtt gtctctgcca ctctggtagg 7980
   aggtgtgtag gatgatcgca aggcaaagaa accattgaga gtaaccttca ggctctatta 8040
   cattgtcatg gaaattgggg acactgctat gggttgggag aggaaggatg gaaaactcat 8100
   aaaccttggg aaattatgga tttctgtgtg ctcaggggag cttaagaata gattttcttg 8160
   ctttcgtatg atgataggac gaaactctag gttgactgcc tcataatttc tccctttttg   8220
   tttccactct gtctatttct tataaaacca ctctctggga ggtaatttaa gaatgtgttt  8280
   gatgacaggg aactggggct gaagatccct attgtaaaat tgcctaaatt ctagagtaga  8340
   agtggaactc ctttgacaag caaggcagat ttctctctat ggcatttttt tctttgcctt  8400
   ttatttattt atttatttat tgagacagag tctcactctg ttgcccaggc tgcagtgcag  8460
   tggcaccatt tcgactcacg gtaacctcca tctcctgggg ctcaagcgat tcttctgcct  8520
   cagcctccca ggttgctggg attacggggg cctgccacca cgcctggcta atttttgtat  8580
   ttttagtaga gatggggttt catcattttg gccaggctgg tcttgaactc ctgacctcag  8640
   gtgatccacc cgcctcggac tcccaaagtg ttcagattac aggcatgagc tacagtgctg  8700
   gctgtttctg tggcattctt tacaggaact taggacctgc atttgagctt taattttaat  8760
   taaagtcttc tctaagaaga tttttggtag agagaaaaac cttcttcttt tattttaaat  8820
   tttgttttta gagacagggt ctcactgtgt ggcccaggct ggagggcagt gacatgatca  8880
   tagctcactg cagcctcaaa ctcctgggct caagtgattc tcctgcctca gtgtcctgag  8940
   tagctgggac tgcaggtgtg tgtcaccacg gccctttttt tttttttttt ttttttttga    9000
   gacggagtct tactctgtca tccaggctgg agtgcagtga cgtgatctta gctcactgca  9060
   acctccactt cccaggttca agtgattctc gtgcctcagc ctcttgagta gctggaacta  9120
   caggcacgtg ccaccatgcc cggctaattt ttatattttt agtagaggtg gggtttcgcc  9180
   acgttggtta ggctggtctc gaactcctga cctcaggtga tctgcccgcc ttagcttccc  9240
   aaagtgctag gattacaggt gtgagccact gaacctggcc aaatacatat tttttaattt  9300
   taaaattttt tgtagagata gtggatcttg ctttgctgcc tgtgcctggt ctcaaactgc  9360
   cagcctcaag ccaggagtct cattctgctt tggcctccca aagtgtttgt tgtttttgta  9420
   aaggaagtga ctttggtaga aatagagagg caataaaata tagagattca gaattcttag  9480
   caatgagttg gagtccagta gaattgggta tgtatctctg atggagccgc ttactccctg  9540
   tgtgaccttg ggcaagtcgt cccccagcag caactttggt ctcctcatct gtaaaatggg  9600
   gataatagca cctaactcat agggttatgg tgaagaataa tgaggtggtg catgagaaat  9660
   agccacagca agtatcaatg actctaagtg agaccatcag tattacttga ttagctgtag  9720
   gaaactgtgt caaagtggga cggagttcat tctggaactt gttaaatgac taggcctctt  9780
   tcatgtactt agatagtttc tcttcttggg tatatttctt cctgggtatt gggtaacacc  9840
   agtgagtgct cagttcagtg tctgggccca gaaacccatg cctggttagc attgtctttt  9900
   cttctgagaa agatctgaac ttgccatgga ctctctagaa aaaccccagt gtacaaattt  9960
   tctatgaaaa gctagaattg atgattataa tgagtaagtt ggctcttcct tttcccatgt  10020
   agattagaaa acatgttttt aaatcttcta attgaaatta ttcttaaaaa accccaaagt  10080
   ctgacatgac tccaactatt gttgttttca tcaatatcaa tcctatcaaa tcaaagttta    10140
   ttttagtgtg taattaacac atgtagacta gaaaagtttg cactttttta tatcacaaaa  10200
   aaggttaatt ttattttcta tggaaagcta gaattgatga taatgagtaa gtcagctctt  10260
   cttttcccca tgtagattag aaaacaggtt tttaaatctt gtagttgaaa ttattcttaa 10320
   aaaaccccaa atcatgacat gaatccaacc attcttgttt ttatcaatat tcaccctatc 10380
   aaatataaag tttattttag tgggtaatta acatgtgtag attagcatag catatgtgta 10440
   tttatataac aaaaaatgaa tcaaaagtta attttatttt gggcagtgtt ccctagaaac 10500
   ttataatatg gccccatgca cttcaggttg aattttctta gtaaaatcat aaagaggtcc 10560
   attttttcaa aaaatcacca agtcaggctg caagtttaga ggttacttct attgtttcac 10620
   ttctctttct tcccagaagc agaccccaga gacctgtgag ccaaactcca ctcacctttt 10680
   agcttattct aaatgtgtcc tgcgggcttc tctgtgaata aaaggcagag gctttttgac 10740
   ttcgtgtttg atgatttggc tttttctttt tttttaccac ctgacggggg atttcaggct 10800
   caatgtccca atcgaaataa gaatctttga gtgggttaac attgagtctt aaaaaggagg 10860
   gctgatttgt tggagaggca acaacgaatt catttttcac tgtagagcac tcaggaggag 10920
   tctgaagctt tgtcactggg caacagaatg cctttctaag taaatatgga aaatatgacc 10980
   tttgaaaatc tagctgatat gatatataga ttcttaggat ataattttta aaaaatatat 11040
   tatttccaag cactaatgat actccagaga tactgcccac aaccagatgc agcatgttta 11100
   tagtgtatct aggccaaaaa aaaaaaaaaa aaaaaaaaaa aatagaagat acagaagaag 11160
   gcaattggtc aaaagattaa ctgaaggttt gaaaataatt ttatacacat ttgtactgtt 11220
   ggggtctctc ttctctctgt ctctgcttat ccttctgtct ctgtctcttt gtttccatca 11280
   cacatgcaca cacacacacg tgtatacaca cacatatatg taagtagatc aatatgttat 11340
   aattttcaca atacgcatct tcttttgcta cttttcagat acaggtcatt aaattactaa 11400
   gtataattta aaatatatgg tctggccttt taactctgca gttcaattaa ttcttgggta 11460
   gataggggga tctgctgaaa agtttgtctc tgacgccttc aaaggtaagt cccttatgtt 11520
   ctggcatttg ctctaataac atgaattctt tgacttcatt catccaatct cttcattgaa 11580
   attgtgtgaa attccataga tagaatgtta gcctttttac agagaataca acatccacag 11640
   tgtttacata tggcatttgg aggtgtgatg gctgcttgaa aatccagtag gaaatttgtc 11700
   tattcaccac atacattcat ttattcatcc atccatccct ccatttatcc acccacctct 11760
   ctattcatca tccgtttttc tgtttatcta tttgtccatc catgcatcca tcccttcact 11820
   ccattctcct tctaaaagtt cttttttttg agacggagtc ttgctctgtc gcccaggctg 11880
   gagtgcagtg gtgcaatctt tggctaactg caagctccac ctcccaggtt cacgccattc 11940
   tcctgcctca gcctcccgag tagctgggac tacaggcgcc caccacctcg cccggctaat 12000
   tttttgtatt tttagtagag atggggtttc accatgttag ccaggatggt atcgatctcc 12060
   tgacattgtg atcctcctgc ctcggtctcc caaagtgctg agattatagg cgtgagccac 12120
   cgcgcccagt ctcttttttt tgtttagaca aactctcact ctttcgccca ggctggagtg 12180
   cagtagtacg atcttggttc actgtaacct ccgcctccca ggttcaagtg attctcctgc 12240
   ctcagcctcc caagtagctg cgattacagg tgcccaccac ctcgcccagc taatttttgt 12300
   atttttagta gagacagggt ttcaccatgc tggccaggct ggtctctaac tcctgacctc 12360
   aagtgatctg cccacctcaa cctcccaaag tgctgggatt acaggagtga gccaccacac 12420
   ctggcctcac tgattaagtt ctatgtgtca agttctattt gtcaggaact attttaggtg 12480
   acagaataca atggtgaatg aaactgagaa aatttctgtt gtcatggagc ttatgatgta 12540
   gcggggggag ataagacatt ctacagatca tttcactcat atttaacctc atttttatta 12600
   ggtactttga agaaaatgga ctgggtaccg ggagaatata taatgggaag ctaacctatg 12660
   taatggctgg ggatgcagtc agtggtctgg gaattaaaaa aaaaaagccc aacttctcat 12720
   tctcattttc tgaggtaact ggagaaagtc tgagttgtca aggaactaga gctaaaaatc 12780
   tgtattgtaa aactgcccat atcctggata atatatgcaa cagggctgaa ggcagtggct 12840
   catgtctgta atcacagcac tccggaaggc caaggcaaga ggattgcttg aggccaggag 12900
   ttcgagacca gcctgggcaa cataacaaga ccctatctca attaaaaaca aaacaaaata 12960
   tgtgcaggag acttatcttt gtgacattct atggagaaac cgtaaatatt tatttgtatc 13020
   atggctgcaa gtaagatgag taagtatttg gtagaactag cctgttacct tgagatggct 13080
   tctttcccag tttgacagag aacttcatct cctcttagtt aataactcag ataatgagct 13140
   gacttccttt ttgtttaggg tttacagttt gaggtggctg ttctaaagaa gaaaaatcag 13200
   acaatttcag aggtggagga gcaagtctgg gccaatagat tgaaaataaa taaaaagaga 13260
   gagcacggca tttcagtgag aggcattttg agctgaaagt cagttttcag ggagcacaag 13320
   attactcaca ttgcctgcca aataccttcc tcaccctgtg tccatctctg atatattaca 13380
   actcttcatt cctttggctt aggcagtatt ttctcttgtt cagaagatac tttttaagta 13440
   gtttaacaat aaacagtgtt gactcactta tagagaaagg acttttgtca agtctgtctt 13500
   tctgatttca ttaaggagaa agtctcagtt ggtgttaata tatctttaac aaccctctaa 13560
   cacaaatctg ggcacttagt agatatctgt tgaatattct ttttttttaa ttataaagga 13620
   actacaggga attatgagaa tgtaaaacta tgggctgggt atggtggctc atgcctgtaa 13680
   tcccagcact ttgggaggct gaggtgggtg gatcatctga ggtcaggaat tcgagaccag 13740
   cctggccaac atggtgaaac tccttcacta ctaaaaatac aaaaattagc caggtgtggt 13800
   ggcatgtgct tgtagtccca gcttctcggg aggctgaggc aggagaatcg cttgaacccg 13860
   ggaggcagag gttgcagtga gccaagattg cgccactgca ctccagcctg gcgacagagc 13920
   gagactccgt ctcaaaaaaa aaaaaaaaaa aaaaaaagtt agtttctacc tataaaccaa 13980
   cttttttttg agtcagcgag ggtacacttt tacctaatgt tgagcctggt tctgttctac 14040
   cctgagcttg attctgtact ggctattcct ccttgtgaac agagaaggga agttcagtgt 14100
   ctttcctgga aatttttggt gttgtaagga tgctcagtgt tcctgaaggt tgagaggaac 14160
   agcaaggaca ggatctggta gagatgtcgc ttttattcag aagtgctaag ctgttaatca 14220
   ttaccatggt tgttattacc ttttaattgc catttgtcct gcttgtttgt ggtctcctgg 14280
   gcatctcctt ccccctcagc ccacaagcag aaagttgccc tacagagtca ggaggagaac 14340
   tcaaacccag accagagagc tctgatccac tcctatgggt cttggaactg gctaagtaga 14400
   tggaaattaa ctgattcaag atatgcaaca ccattagttc aaccatagct gtgttcctgg 14460
   ggtgaattct agctttcttt tttctttcat cttgctactt cccagagacc agactttggg 14520
   cctctaggga aggagtatca aattgggctt gagagttcaa gctcaaaaca gacctgattt 14580
   taaatcttgg ttctgcttct ccatatcttc atgacctttg ggtgggtaaa atatgatcct 14640
   gcctctatct atctatctat ctatctatct atctatctat ctatctatct atctatctat 14700
   ctgtccatct atctatctat ccatccatcc acctatccac caatacatcc atccgtctgt 14760
   ccatccatcc atccatccat ccatccatcc atccatccat ccacccagct attcttccat 14820
   tcattcatcc aaacatccac ccatccaccc agctattctt ccattcatcc atccaaccat 14880
   ccatccatcc atccatccat ccgtctggct atccaaccgt ttttccattc atttaatata 14940
   tatttattgg atgcctacca catatcaggt actgttctag gcattggaac tagaacagtg 15000
   aacagaacag catcgctggc ttcatggaat ttacattcta gtagaataac agtaataagt 15060
   gaataacata gaacgaatat attgtataat gtcaggttgc catatgggct ttgaagagaa 15120
   gtaagacact gtcctcaccc ttaaagatct tcctggtctt ttaaaaggag aacagcacat 15180
   ggccaattag ttctgagaca taatgtaaag tgctacacca agaatgtaaa aacactttgg 15240
   atggcatccc tactcctcct cggaccggtg aagattccac agaggaggtg acatttggga 15300
   acagtcttac agcaggagtg ggagttcccg gattgacaag gggatgaggg caggggaggg 15360
   tgtttcaggc acaggacagg acaacgcagt tgagatagtg aggagagaga atagggcaca 15420
   tgcagggaac aggaagaaga gggtgtgagg gagggaggag caggtgggag gtgaacgtca 15480
   tatcagaggg gttggcccct gtgaaggcag tgtagtgact aggaagtggt tgttgtcact 15540
   gttttaaagt gggatgtgtg ggggtgagtg aaatggccaa atgtttgaaa aagaggagca 15600
   tgagatgcct ttggcaacaa tacttgccat gactcaaatc ctgaaattct aggcctgcag 15660
   tgatggaagt caggcaggag aaactgaaat ggccatttag aagcagtggg ggtgccttac 15720
   cggagcacct cgtggctgtt gctatttttc acaccctaaa gagacatttg gaatttcatg 15780
   tcagtcatat tgttgattgt tctattttga gggtggttac tagttatttt tatggttcta 15840
   acaataggga catctctctt cactgtatcc ttagacgcgg agggccgtag aaatgaattt 15900
   tcctctgaaa ggcatcctcc cactccctct ccctctccac tgctgtcacc cagtgggtac 15960
   gtgtttattg agcacatgct gcacatggtg gtgaagagtt ggtgttttgg agaaagaaag 16020
   gcttcgattt aaatcttgac ttcttgctgt gggaccttaa gcaagttact taacctcttc 16080
   gagcttctgt tttttcatag ctaaaaggat ataataataa aacctacttt atgtgaagtg 16140
   tttcttagca ccctacctga catatataca gtaagcactt gataaatggt agctatttct 16200
   gttcttccta ggactgggca ctgtccttgt ccttactgag tttataatcc tgttggggag 16260
   gcaagatgaa tgctcttgaa tcaaggagac gacgcaagaa tggatgtgct tacatgtcgt 16320
   ggtcaatagc attagctgca ccttacagtg gatcttagag agaaatgctt ggttggggcc 16380
   ggagaggtga tttgagtgtg agtgagcaca gtgctttctg agaaaccctt tcctggcctg 16440
   caacacttta atttgtggga aacctcagga agtagaattg caatctgtga tcgccccatc 16500
   ggctgacctc acttcaaaaa tgtattggta gcatttcaac tttaaataga tttgtgacca 16560
   tttcaccctt ctaaatttgg gttcccttca ccctttgggt ggagtgttta tatccctttt 16620
   cggttgaaga agctgacaga tagtgtaggt gaaggtggag ggtgaagtct gaaggacggt 16680
   cgcttgtctc attgggatag tcagggcagt gatttttgtg ggatggccct atattgaaga 16740
   tcttctttcc ctccctaaag gtagagacca ttctttagag tttagtgagt gctgcaaatg 16800
   aagatttgat cgttatttgg atttttttgc atgtgtatgt gtggattttt tttggtagtt 16860
   atccagatac attttgtggc aaaatttatg gctgagggta ttgataattg tttctgagac 16920
   aaacagtgca cttagggaaa caggattgac atatagagta gagaaggact tctttttatt 16980
   tcagttggat attcatcttt tggtggctag ctttttttaa aaaaactatt aatattattt 17040
   ttgattgaca aatcataatt gatggggtac aatgtgatgt tttgatacat atatacaatg 17100
   tgtcatggtt aattcaaact aattaatata accatcagct tgtttactta ttttttatgg 17160
   tgagacattt gaaatttact tagttatttg aaatatatga tacacttatt attgactatg 17220
   ttaccctgct gtgcaataga tctccaaacc tattcccctt gtctatctga aactttgtat 17280
   cctttgatca acaactcctt ccctcatccc agattctggt aaccatcctc ctactctcta 17340
   cttctatgac tacgacttta ttagattcca catatacgtg agatcatgct gtatttgtct 17400
   ctgtgtttct ggcttatttt acttagcata atgtcctcca gattcatgta tattgtcaca 17460
   aatgatataa tttctctctt ttttttaaag tctgggtagg attctattgt gtgtatacac 17520
   catactttct ttattcattc atcctctgtt gaacacttag gttgattccc tatctttgct 17580
   attgtaagta gtactgcaac aaacatggga atgcacatat tctttgacat attgatttca 17640
   gttgctttaa atatatgccc agaagtggga ttacttaaaa aaaatttaat ttaatttttt 17700
   ttaatttgag actgagtttt gctcttgttg cccaggctgg agtacaatgg tgcgatctca 17760
   gctcactgca acctccacct ctcaggttca agacattctc ctgcctcggc ctcccaagca 17820
   gctgggatta cgggcatgca ccaccacgcc tggctaattt tgtatttttg ctagagacgg 17880
   ggtttcactg tgttggtgag gctggtctca aactcctgac ctcaggtgat ccacctgcct 17940
   tggccttcca aagtgctgga attacaggtg tcagccacca tgctcggtgg tagttcactt 18000
   ttttgttttt ttgagaaact tttagtactg tttttcataa ggctatacta acttacattc 18060
   ccaccaacag tgtatgggtc cccttttctc tacatcttct ccaacatgtt attttttatc 18120
   tttttgataa aagccattct aacaggtgtg aggtgatatc tcactgtggt tttaatttgc 18180
   atttccctag tgcttagtgt gctgaacatt ttttcaagta cctattggcc atttgtgtgt 18240
   cttcttttga gtaatttctg ttcagatccc atttttaaat caggttattt gtttacttgc 18300
   tattgagttg tttgagttct ttgtatattt tggatattaa cttcttatca gatacagcat 18360
   ttgcaaatat ttttcccatt ctgtgggctg tctcttcact ttgttaattg tttcctttgc 18420
   tgtgaggaag ctttcagttt gatgccctcc catttgtctc cttttgcttt tgcttaatca 18480
   ttgcccaaac caatgttgtg gagtttttcc cttatgtttt tctctagcag ttttatagtt 18540
   tcaggtttta agtctttaat caattttgag ttgattgtgt atgtggtttg taagataaag 18600
   gtctaattgc attcttctac atgtggatat ccagttttcc caacaccatt tactgaagag 18660
   actgtccttt ccctcattgt gtgtttttgg cacctttatc aaaaatcagt tggctataaa 18720
   tgtgcaggtt tatatctggg ctttctatcc tgttccattg gttgatgcat ctgtttttat 18780
   gctagtaccg tgctgttttg attacaattg ctttataatg tattttgaaa ttaagtcgtg 18840
   tgattcctcc agctttgttc tttttgctca atgttgtctt ggctatttgg ggtcttttgt 18900
   ggtcccatat gaacttaggg attgtttttt ctatttctgt gaaaaatgac attggaattt 18960
   tgataagaat tgcactgaat ctgtagattg ctttggctag tatggacatt ttcacaatat 19020
   taatttgtct agtccatgaa cacggaatat cttttcattt atttgtgttt tcctcagttt 19080
   ctttcatcag tgttgtatag ttttcagtat acagatagtt tgcttccttg gatacattta 19140
   cacctaagta attttttttt tgctgctatt gtaaattagt ttgttttctt aattttctgg 19200
   tctggtagtt tgttattaga taagctagta tataaaatta gaataattag ataatctaat 19260
   atagaaacac tactgattgt atgttggttt tgcatcctgc agttttattg aatttgttga 19320
   tcagttctaa cagctttttt agtggattct ttagggtttt ctgtatataa aatcaccttg 19380
   tcaacaaata gagacaagtt cacttcttcc cttcctatta ggatgccttt tatttctttc 19440
   tctttcctaa ttactctggc tgggacttcc agtactctat tgaaaagaaa gggtgagagt 19500
   gggcgtcctg atcttagagg aaaggctttc aacttttcag tgctgagaag gatgttagct 19560
   acgggtttgt cataatagag cccatagtgt ttattgtgtt gagtgcattt cctttatacc 19620
   ttattgctga gtactttttt catgaaagaa tgttaatttt tttcaagtgc ttttttttcc 19680
   tgcatctatt gagatgatca tatgattttt atcctttatt ttgttaacat gggtatcaca 19740
   tttattgatt tctatgtgtt gaaccatcct tgcatcccag ggagaaatcc cacttgatca 19800
   tggtgaataa aattctttta atgtgttctt caatttggtt tgctaatatt ttgttgagga 19860
   tttttccatc tatgttcatc agggatattg gtttatagtt ttcttctctt gtgtttttgt 19920
   ttggctttgg tatgatagtc ttggataatg agtttagaaa tactccctta attttttgga 19980
   agagtttaag aagaattgat tgacatccag atcaagaaac agaacatgct ggtaccctag 20040
   aagcatcatc tcacaatccc tatcaaccgt atccttcagg agtaatcact atcctaacct 20100
   ctatcaccta tgattacttt catctgttcc tgaacttatg cccacttttc tgttatgcaa 20160
   ctccattgac ttatgcccat ttttctgtta aaaaatttaa aaagtgcaga gctttaagtt 20220
   actgaggctg agcctgagaa atgttagctg ggactttttt agttttgcat agaggaagtg 20280
   aaatccgtat gggagacagg caccttctgg aatcacccat tggggaatgg tgccgtttgc 20340
   cttctttggg gaacatctgc ttcctgtttc aatgttgccc atcggttcca gactgttcaa 20400
   caaggaagcc agggcattct gacagttttt ccacacaata atttagctct gtaaatacta 20460
   ggaaaacttg agctcacaaa ctatttttag ggcttaacat ttcaaaaatt ctgtattagt 20520
   aagaatctat taaagctgct gtaacagaca aaccttgaag tctctgtggc ttgacagaag 20580
   ggagtttatt tcttactcat gtcactaacc agtgcaggtg gtcctggtca ggacatcttc 20640
   cacagagtca cacaggaccc cagggtcctc cccattgggc cagtggaagg ggaaagggaa 20700
   attgagaaag cacactcatt tcacaacaac ttcagctggg gaaggaagtc acatcacttc 20760
   tgctcacatt ctatttgcca gaacccaggc aaagaggcaa ccaacggaag ggggtcctgg 20820
   gacatgtcga ctagctctat gtccaggagg aaaagaaaac aggttttgat gaactcagta 20880
   aatctcactc cagtctccaa agccataatt tcctttgtct ttctttgcgg tgatgtatct 20940
   agttttatct ctcacttgac tggtaaaact aagatgcaaa actctggacg tctttatctt 21000
   atcaaacata gcagggaaag agtgagttct atttgtgttc ctaaaagtga aataaagaag 21060
   cacttttctt gtttttagtt attagaacaa tctggctctt gtcagacttt ctagtttttc 21120
   aatcagtcag aattcttgcc tctctggttg cctgttgtat taatccattc tcatgctgct 21180
   aataaaggca taactgagac tgggtaattt ataaaggaaa gaggtttaat tgactcacaa 21240
   ttcagcatgg ctggggaggc ctcacaatca tggcagaagg caaaggaagg tcaaggcact 21300
   tcttacatgg cagcaggcaa gagagcgtgc acaggggaac tgccctttat aaaaccatca 21360
   gatctcgtga gacttattca ctatcacgag aacagcatta gaaaacccac ccccatgatt 21420
   ccattacctt ccgccaggtc cttctcatga cacatgggga ttatgagagc tacaattcaa 21480
   gatgagattt gggtggggac acagccaaac catatcacct gtggacatcc caagcagatc 21540
   tctttgaggc cgggttagtt ctcttctaag ctgacttgtt atatagaaga atcctttgtt 21600
   ggttctactt ccagatattt gactggacac ccaacaaatc aattatctat tgctggatag 21660
   cacataacct caaggcttag tggcttaaag tagccactgt tttatttagt tttagtttct 21720
   gtgggtcagt aatttgggct gggctcagct gggtggtctg tctgatctgg gcagggattg 21780
   gctgttcttg gctgggctca tttaccttcc tggggcctca gcagggatgt ctaggacaac 21840
   tggggcctct cttcaggtct ttcatcttcc agtgggctat cctgagcatg gtcatatgct 21900
   gatgggtgca ttctctgggc atgagcaagg gctgcagggc ttcttgaagc caaggctcag 21960
   aacatgcata acatcacttc tgccatattc cctggtcaaa gcaaatcaca agcccacccc 22020
   agaagtaaag gaggaaatag agaagagaat agagatgcct cctcttgatg ggagaagctg 22080
   caaataattt gtgatatttt ttggaaccca ccacactaat ttcagtgtgg tcctcgcaat 22140
   tgcttggctg tctaaaaaca tactccagtc tagtgtgcat ttctgaaaat gaacttgatc 22200
   aaaggcaggc cccaaatgag tgctcaacat ttacacctga taataatact acctacctcc 22260
   tagggaaatg gtagggccag atgagatgct gtatgcaaag gatacggcca ggacttagtg 22320
   cacacgcaac actgaatact accaccatga ctactgaata ctgccatcaa atactaccgt 22380
   tactaccact tggcactccc taaaagcttg agattcagag aaaggaagac aaagcccctg 22440
   tacttgagga gggagagaat aaagagagcc ggcgggggag ggggtggcag gagagagaga 22500
   gagagagagg cgtgtgagtg agttcttgca acacagtgtg gtcagtgtga tcacagagct 22560
   gtgatggagt gcggatgagg tctgatgact tactggtcaa gagcatgggt tcaggcctca 22620
   gttcctcccc aatcacatgt ggggtcttag gcatctttct ttttaatctt cagtttcttt 22680
   gtctgtgaaa tgaagataat ggcacagagc ctacctccca gagttctatg tggattaaat 22740
   gagacaggca aacaacaaca aaaaccacaa acaaacaaaa aacaaaaatg aaaacaaaaa 22800
   tcatgtgatt gcttattaaa tgttagttat agaaaaaaat ataagttctt aaatgtgccc 22860
   ttgtaaaaat gtcctaaaga cctcatcttt atttagttct tctgcaactc tggctagcat 22920
   cattgataaa ttgtcttaaa agatgaagat ttgtatgcag gtggtttatt ggggatgtag 22980
   ctcttgggaa caacatctat aagggataga gggaagcagg gaagaggtag ttgaactgtg 23040
   gtgcaatagt tgtaatagag gccaaagcca attccatggc tggagctgag acggcccttc 23100
   agaactggga caagccaatg catcaagtgg gcattgattg tgggctgctc tttggaaggc 23160
   agcattacct tgggcaaagc agctcccttc ttcaggggag gagtcccagg gaggacacag 23220
   ctgtgagcaa ttggttttcc cagcagctgg gggaatgagt gccttgtccc tatagatggt 23280
   tttgaggggg ctgcatttgg gggaatgcct tctggggttc actgcgtgct tctatgtctt 23340
   tcttagaggt tcatttcaaa ctataccttg gttcattcac agtactgatt tagttactat 23400
   ccagattcat agactgtact tgctggcact ggaatatcat ggacagagag tcctaatctg 23460
   actaggccta gggaatgaca tgggaatagc ttgtcctggg tcatccagct accagagagg 23520
   agagctggga ttcatatcca accaacgata ctttatacct cacattactt ctgttatgct 23580
   acacactcct gagtcacagg agattccaac ttgtatttca agcccagact gcatgacttc 23640
   aatgtcctgt tgcccttaac ccctgtatca gattgcagcc ccactctgga aagttttctt 23700
   gaattcttct atgtatgttt tttaaactgt gggtcatcac tcattagaag ttcatgaagt 23760
   caatataata ggtcaagaat accatgaaga aagcagaaca gaaaatatca cagtgcatca 23820
   ctctcaggaa gggtagatat tgtttcacga aacttttttt ttttttgaga cagggtcttt 23880
   gtctgtcgca caggctggag tgcagtggta caatcatagc tgactgtatc ctcaacctcc 23940
   caggcccaag agatcctccc acctcattct catgagtagc tgggacttga ggcacatgcc 24000
   atcatgcctg gctaatttct aaaacttttt gtagagatag ggtctcccta tgttgctcgg 24060
   gctggtctca aactcctgag ctcaaacagt cctcctacct tggcttccca aagtactggg 24120
   attataggca tgagccacta ctcctggcct catacaattt ttatttgata taaacatata 24180
   tagccatgag tgaactgggt caaaatgtaa aatgtgtctc ttactgcagg taactgtcaa 24240
   tgtagttcga atatcactgt gaaatatata gagaagaggc tctgggggct catgaacttg 24300
   gcccatggac tgttgaggct ttttttttaa tctttagaga tgatataaat gtagtgcttt 24360
   gaattcttta taattagaag ttttattctt accccatcac catgtcccaa gataattttt 24420
   attatcatct ttcagcttta acatatcaca gacaaaagtt atttttccaa atagaatttt 24480
   aagtatggta taagaggttt ttagaaatgg aatgattggt tttggcggaa ctgcctggcc 24540
   tgacctgtca ctcgggtgaa gtgtcagaga gctgtgtcga atgctggcgg agaactgtcc 24600
   ctctcctgca gagagaagga gctgcttatc ctgggggata atgtgggttg agaaagaagt 24660
   gttttatgag cagaatcagc acagagggag ggaggaggag gtgtttaggg gctgcagaga 24720
   gccgccagaa gatgtgggaa cagtatgcca gcacggaggt gggtcagatc cccatggtgg 24780
   gactctgctg agggttgcct ccagccgaga cttccaaggg ctgggattca gtgtgtgttt 24840
   atgttctctg gtgtgcctga taggtggggg cccacatgac agctggcctc tctgtgtttc 24900
   ccttctggcc gtctctgatt tggggcccaa gggccatagg ggggccaggg cactccagat 24960
   gggacgaaaa gtcaagtcca agaaaaaaat gaaatccggg ctaggcttgt ccctgcttga 25020
   ggatgtgaga gagagagaga gagtgtgtgt gtgtgtgtgg gtgtgtgcgc acgtgcgtct 25080
   gcagtcccta gaaggatctc tattcattgg acagagaggc caagggaatt ttccatttcc 25140
   tggatgtctt caaagatttc aaatgccatt aacttgagct gacatttatt gagtactcac 25200
   tcggtgactg gcactgttct aagtacttta catgtattaa cttatttaat cctcacaaca 25260
   gccctgtgtg gctgctactg ttattgttgc cattttatgc tgagaaaaca gcacaaagaa 25320
   ttgagaaatt tttccaaagt tacagttagg attcaaaaat agacttggtt ttaggattgt 25380
   gtaacttcag aattgatggt gacaatgacg ttgatgatga ggatgatgat gatgatgatg 25440
   aaggcagcta atatttcagt gtctgcaatg agctgggcat tttcacaaac tttatccctt 25500
   tgaatcttta ccttccacta agatttgctg ctattcttat cccttttgta gataagaaag 25560
   caagcttaga gaagataaaa catttgctta agtaactgca gagcgtagat tgaattcagg 25620
   tccattcgag tcagactgtc tgttcaccaa ggtgaagggc tccgggggtc acagaggagc 25680
   accatggtga ggaaggcccc gtaagagtgt ggcagaatgg caaccaggaa ggatctggta 25740
   tacgggatgg tgataaggcc catctctcat ggccaggaaa gtgtgcctgc cacagcaatg 25800
   aatgaaactg gcaacatttg acaggaaggg gaaattctct agtgggagga atacttccat 25860
   caaactgttt tttttttttc ccagtggggg agtttgcaca tgtgtagatt tttttagcca 25920
   tagccttgaa taaggaattc agaaagtggc ttctagtttg aggactttca ttggccacgt 25980
   gatcacagta ggctgatttc ttcatgccta agttaccagg ctgggaggac aggctgagaa 26040
   ctatgcaagc tgttgagtca cttggaagga agagggttac atacagcagg cactttcatg 26100
   gaataatact tccctgaaga gctgcactaa tttattgagg cactgagctg gatgcttcac 26160
   acacatgatc tcattgaatg tccacagcac cctaggaggt tgatctcatt attaacttca 26220
   tgaagtgact gaggcttaga gatattaaat agcttccagc ctgggaaaca tagtgaaacc 26280
   ccatctctgc aaagaataaa aaaaaatgag ctaggtgtgg tggctcgtgc ttatattcac 26340
   agctacttag gaggctgagg tgggaggatc ccttgagctt gtgagggttg aggctgtagt 26400
   gagctttgat ggcaccactg cactccagtc tgggtgacag agtgagaccc tctctctaaa 26460
   caaacaaatc ccaggagaca ttaattaact tgccactggt ctctaagcta agtagttgaa 26520
   gagggcaaca aacccaggtg gtctgactcc taggcttgaa ggtccaggtt cagctactcc 26580
   accagtatcc ctggtcagtc tttggccagg gcacagaagg tggcatatca agttgtgggg 26640
   tctgatttgg aaagtgtgag atccctcctt tgtatctttt ccaaagtcca ctcagaagag 26700
   agtagtgtaa aaggagtgat agcacagcgt ggtctggctg gcatagtcag ccgccctgtg 26760
   atgggcaggg ggaagctgct ttctgtcctc aagcagcccc taaacttgta ggccagactc 26820
   ataaatcaca cattgataac tcagtgtgat gtgtgctgta atggtggaga ggggggagaa 26880
   atgccaggag cagctcacct cccagggtcc tggggaggct gcacagagga gcctgagttc 26940
   tcagttgggt tttgaggcag aggaagcagt gaaagagaga ggaagaatgt cagtgaggtg 27000
   cggggagaag caagagagag aagacacttg gcaggaagaa gtagttgaag tacccgggga 27060
   tgtttagctt gaagaataga aaatttggag gaggcagaga aaaggcagga aactttaaaa 27120
   gcaggagaga aagacctcca ggttcccaaa gggagcatgg cacttctcaa gggggtgagg 27180
   ttgacagcat taggagagag agagagagaa agagagagag agaaagagag agagcgagag 27240
   agttgtcaag gaagggaagg acccatggag gggagagcac aggtgaaaat gagaagtgat 27300
   gggaaaatgg gtgggaaata aatgaagttt tgaggccagt tgagaaatct tgcttggtgg 27360
   tttctatttt ctttgtgaag cagaaaaaaa gaggaattag acagacctgc attcaaatct 27420
   catgacccca ggcaaatttc ttatccttag cttcttcatt ttgaaaaagg ggtgaatgat 27480
   aataggacct acctaataag agttgtcagg atcaaatatg ttagtgcttg tgtaagatgc 27540
   agtggcaccc tggcacttag taccaaaaaa aagtactata aatgttgtca cctattgagg 27600
   gagtccgggt ttgggtgctt gatacataga aatatcatga atagcagcag agaatacaag 27660
   aagaagagaa agcttggggg agaagataga gaattatgcg ttgggtatgt tgaggatgag 27720
   gtgcctgtgc catatccggg tgtaggtgcc caaaggcaac tggaagtcat gagaagtcag 27780
   ggatagaggt aaatgtttgg gagttgttaa tacaggggtg catttttaaa atatctgaag 27840
   tatgttactg gtggcactca aagattttta atgatgtaag gtattcaaca acatggaact 27900
   acgtagtttt aaaagaaatt ctcttgtagg ccgcgtgtgg tggctcacac ctgtaatccc 27960
   agcactttga gaggccaagg tgggtggatc acttgaggtc aggaattcca gaccagcctg 28020
   gtgaacatgg tgaaacccca tctctactaa aaaaaaaaag aaacaaaaca aatcaaaaca 28080
   aaaaaaccgg gcatggtggt gtgtgcctgt actcccagct actcgggagg ctgaggcagg 28140
   agaattgctt ggacccggag tcagaggttg cagtgagcca agatcgcgct actgtactct 28200
   agcctaggca acagaatgag actcagtata aaaaagaaaa aaatgttttt ttcttttaat 28260
   tcttcgggtt aaggagagtg tcttagtttg gtgctatcat gtcctaaaca tctttctaat 28320
   gcatgcaaat ctccctttta agctaaaaga atgaagcaat ctcagactca gagctatgta 28380
   cctgctagaa tttaataatt taaattttca ttgtgttctt catactcacc ttctatttat 28440
   agcaagtgtt actagctctc caattctgga ggttgtttca gataggatgc attagttctt 28500
   tttttttttt tgagacaagg tcttgctctg tcacccaggc tggagtgcag tggcacaaac 28560
   acagctcact gcagcctcaa cctcccaggc tcaagcaaac ctcccacttc agcctcccag 28620
   gtagctggga ctacaggcat gtcccaccat tcctagctaa ttattttatt ttttatagaa 28680
   acagggtctc actgtgtttc ccaggctggt cttgaatttg ggctcaagca atcctcacac 28740
   ctccgcctcc taaagtgctg ggattacagg catgagccac catgcctgac ctgctttggt 28800
   tcttaaaaac agaatatctg gcgagcctaa caaggaggct ggtggttcca gggttggttt 28860
   aatcaaagac ttaacactgt catcaagcgc ctctcagtta tttgctctgc catcctcaaa 28920
   atgtgatcag tgtctccctt cttggtccca ggaaggctgt gatagctccc agaatcacat 28980
   actcacaggg caaagcagga aggaagggga cataggacaa aggatttgtg tttaagtatt 29040
   tcatgtcaca tttgcatgtg gtgcttggag ctgtggcagc tatgttttga ccatgaggga 29100
   caaggttggc aagctgtaaa tggcagtgta aaaagaggga gaggatctgt ctccttcaca 29160
   atatccttga gcccctgaat tcactaatct tgatatagct ctatctcttg aaattttgtt 29220
   ttgttagata attctcttac tgcttaaaac gtcttaagat gggttttctg ttctttccag 29280
   tagaatgcat tgtatctcac acaatttgag gttattgcct caacctactc tgaatcattg 29340
   ctcctccccc ttttttcctg gttgattagc ttctcttgag attgtgtctt atcttcctgg 29400
   ttttttgtat gtcgggtaat tttggattgt atcctggaca ctgtaagtgt tgtgctgtgg 29460
   agactctgga ttcattcgta tttctctgaa aagcattgat gtttttgttt tagtaggcca 29520
   ttgacttggt tggacagtag actgtctctg ggacaccagc tcagatgtca gttctgtttt 29580
   ttattttatc cttgagtctt ctttgggact gccccatgca tgcatggttc aagagtcagc 29640
   cagacatttg ggtggggttt agacacagaa tttggggctc ttctctcttg gctgtctcta  29700
   ttatggaatt ccttcctcac tttccagaag ccgtgagtga cctgaaacct gtcctctggt  29760
   tctgtacacc agaaagactt gaggcttcat gttgaagttt tagctgacag cagcttgcgt 29820
   tcaggggaaa agctgtaaaa tgaccattcc cttcttccaa gtttcaacct ctctgcagaa 29880
   tctgctttct tttgttcact ttcgagtaat ttcaggaaaa aatttttttt tcgttgagag 29940
   tttttacttg ttacctatgg aaaggttggg tctagctgaa gcttgcttgc ccataacaga 30000
   aacgagactc ttttctgtct aacacatggg aaaactaacg cccagagagg tgaagtcatt 30060
   tccccaattg tcagaggcac ttgagccaga gtgactccat cttgagtaag ggctaggaaa 30120
   ataagaccgg gacttgctgg gctgcattcc cagaaagaaa ggtattccta gcctctagat 30180
   gtctacagtt aagggaacag attgataatg tttactaaac agagccaggc ttggcagtgt 30240
   ccagctatcc cgatatcttg agaacaaagg cattcctaat tttgctttaa ggataataat 30300
   attgattatt gcaaaacaga gtaattaaga aaattaatcc tttatcacaa acccttgtag 30360
   cagaacacat ctctccatga tttttaaaat catatatata tgatttatat atatgattat 30420
   ataatatata acatatatga tcatataatc atatatatgc atgtgtatgt gtatatatat 30480
   acacatgcat atatatgtat atatgtgtat acatacatac gtatatatgt gtatatatac 30540
   atacatatat atgtgtatat atatacatat atatacacat acacacacac acacacacac 30600
   acacacacac gaatatttta cctagggtgg acgtgctcct tctcttactt ttaggaaccc 30660
   cctactctgt ctatggagta actgttcttt caccacttta ctttcttaat aaacttgctt 30720
   ttgctttgca ctgtggactc accctgaatt ctttcttgtg caagatccaa gaaccctttc 30780
   ttggggtctg gatcaggact cctttcctgt aatacaatgt cacagagctc ttaagtggca 30840
   gagcttgggg ccaaacttag gctgctgcac ccgatgtccc agctcctctg ccctatctag 30900
   ctgtggattt gtgcctcgaa atagaattgc attttgaatg cagtttcatt ttcgtatgga 30960
   aagtgtctat cctgtaaatg taactgtagc agccccaaag ctgagctggg cagttttatg 31020
   gcagcctgga gtcagtcttg tccgcttcca atcagggttc ctggactggc tcctatatta 31080
   aaattggtat tagtagacaa tgttccaggc agagttttgg atagaaacta tccatcagca 31140
   ccaagaatgt gcctgatttg gtaatctttt taccaaagta aaagtcagta gtaagttgtg 31200
   gttaaaagag aggaagttca gagttcagta cgttatttct ctctctctct ctctttgttt 31260
   aaataaaaga ggaagttact gctcttattt ttgtaccagc ccgtcagtaa tgggtacaga 31320
   gtaaaatctt agctaagctg aggtcaaact tcaaaactta gtaataagga tctcccttgt 31380
   tcctttgtag ggcgactaac atgaaggttc tgactcttaa cccatagagc cacaaaaaat 31440
   agtcctagga aaatcatatg atttatttta ttttaggaac agaaatggtc ccttccgaag 31500
   tagaggcatt tctacttctt tcctaaacac catctctgct cagtgagctc tcccaccctc 31560
   aggtactgtg tttcaggctt attgctgtat tgctagaagt atcctcacct tcgagattaa 31620
   gaaaagatat ttattgccgg gcacagtggc tcacgcctgc aatcccagtc ttttgggaag 31680
   ctgagacggg aggatcactt gagcccagga tcttgagacc agccagggca gtatagtgag 31740
   aactcatctt gaaaagaaaa atttattgag ctcctactgt atactgggga tacagcaata 31800
   ggcaagatag cccttgctcc tcatgtaacc tacagataat aaacaagatt acaaacaaac 31860
   aaaataagag gtcgttctag attatgataa gtgatggaaa agaaaaaacc tgtatctact 31920
   tcttatagca gagacctgca ttgggagttg ctgtgatcaa acaggaaacg gaaggtggag 31980
   tttgcttctg tatccctcca tccctgagtg gaggctgggt gcttctcacg accagggcct 32040
   ctgagatttc tttttttgtt ttttttaaat tattttagat taagggagta catgtgcagt 32100
   ttcgttacgt ggttatggag cgtgatgcta aggttagggt ttctattggt cgcgccactc 32160
   acatagtgaa cctagcatct gataggtagt tttttgaccc ccttccccta tgccctctcc 32220
   ctcttggggt ccctagtgtc ggttgtttcc atctttatgc ccctgtgtac ccaatgttta 32280
   gttcccactt cgaagtgaga acatgtggta tttggttttc tgtttctgca ttaatttgct 32340
   taggataatg gcctccagct gcatccatgt tgctgcaaag gacacaatcc aaataagcac 32400
   aatcagaaat gaccaagctg acattatgac tgatcccaca gaaacacagg agatcctcag 32460
   ggatgactat gaacacctct atgcacacaa gttagaaaac ctagaggaaa tgggtaaatt 32520
   tcctttgcac acaacctcac aagattgaac gaggaagaaa tagaaatcaa gatttatttt 32580
   ctgttttgtt tgtgtgtgtg tgtgtgtgtg tgtgtgtgtg gttttttgtt tgtttgtttg 32640
   ttttttggac aggatctcat tctgtcactc agactgaggt acagtagcac aatcacacct 32700
   cactgcaacc ctgacctcct gggctcaggt gatcctccca cctcagcctc cagggcagct 32760
   ggaactacag gcatgtacaa caatgcccgg catatatata tatatatata tatatatata 32820
   tatatatata tatatatata tatgtgtgtg tgtgtgtgtg tgtgtgtgtg tgtgtgtgtg 32880
   tgtgtgtgtg tgtgtatatg tatatttaaa tttttatata taaatatata tctatctttg 32940
   tagaaatggg gttttgccat gttgtgcagg ctgaaattga gatttcctaa tgtaagaaac 33000
   agagccccaa ggggctcttc ttgggctggt cgctgtattt catcaggtgc ctctgtgttg 33060
   gacacttact tccctccatt gcatagattg gatcctttat gcttagccta gactctgccc 33120
   tttgggatgc aaggagctga gtgaaggttc tgggagagta ggcttaagcc cagaagaggg 33180
   aggcctctct ctggatgtca tgctagagcc tgccttttag tctttgtgtt tccactctcc 33240
   atcctcctct tctggagttc agaacccctt cctggtcaaa ttgcccccgg gctctatggg 33300
   atcctgtggg attctatcct ttgagtctac cccacccttc aagtacttcc tgtcttttca 33360
   cacctaagtg aaagaaagta ggataagaac agcagttgtt aacttgattg attgtaaatt 33420
   gccttcatta gaaaatgtta attatcgccc ctgggaaggg attaatcctt taggtctcta 33480
   ggttactaga ctggcaaaag ccaagtgtgc cttggttgac tgtttctttc tgagagtaaa 33540
   gagaaaacat caggggaggt agaaacctca gtcatggggt aatcattatg gccatgttta 33600
   tccccataca cgtatcttct gctgattctg gagtcagcta gattttgtga cccagcatag 33660
   atgatatagt gacattgtcc tgctgtgtta aaaaatttaa gttcataaga aatgcataag 33720
   tacaatctca tcaggataaa ttacacaaca tagataagat tgaagacctt ttaactcttt 33780
   tgacgtctca atttagtccc ctctaccttc cccagaagcc tattgtttgt aaatttggtg 33840
   tctcttatag aactctgtag cattccaaag tctgaacttt ctcccatgca tatggccact 33900
   tccctgttga tgaacattta gattcttttt tggaggggtt gtgtctatta caaacagcat 33960
   ggcaatgagc atctttctac acatctcatc aatatgcaag tgtttctcgt gttcctcgga 34020
   agtataatgg cttagccctg gggtatacac gattcagctt ttaataggtt ttgccaaatg 34080
   ttctccagaa tagctggacc actgtatact cccaccagca gtgtgtgttt ccgtctcact 34140
   gcatctttcc cagaccatga tattatcagg ccttaacact tttgccaatc tgatgggaga 34200
   aaaatggcat ttaattttaa tgatcctttc ctgtcccctt ctcttctctt ctcttccctt 34260
   cccttcccct tcctttcttc cttcccttcc ttcccttcct tcccttcctt cccttccttc 34320
   ccttccttcc ttccttcctt ccttccttcc ttccttcctt ccttccttcc ttccttctct 34380
   ctctctctct ttctttcttt ctctttcttt ctttctcttt ctttctttct tttctttctt 34440
   catttccttt cctttctttc ctttcttact ttccgagtct cactctgtcg cccaggctgg 34500
   aatgccgtgg catgatctca gctcactgca acctccgcct cccaggttca agcgattctc 34560
   ctgccttagc ctccttagta gctgggatta ctgccacctc acctggctga ttttttttgt 34620
   atttttagta gagacggggt ttcgccatgt tggccaggtg ggtcttgaac tcctgacctc 34680
   aggtgatcca ccggcctcgg cctcccaaag tgctgggatt acaggcgtga gccgctactc 34740
   ccagccacct cttcttacta gtgagactga acatcttttt acatatttat tggacaattg 34800
   tatttcatct tctatgaatg tctccctccc cactttttta taatttcata ttgatttgta 34860
   ataattttta tttcaatcta ctagacactg atcctttgct cgttgtttgt gtttgtgttg 34920
   tctattttca tcaagtcctg ggtcctgtgt cactttagtg ccataaggga atgacagact 34980
   cccttattat tggtttagct cttgaagcct aacaaagtgt ttccaaacag ttcattattc 35040
   ccactttaag gatgtgaaga ctgaggctca gggtcacaac ttgtgcaatc ttgggtagac 35100
   tttgaatctc tgagcagact tagaagtaga ccaaactgct gtgagccaca gggagattat 35160
   tattgctaac ttttccctgt tagccaaaca cttctgcttg gtccctctag agagaagtct 35220
   ttaattattc atctctttcc aagtaattgg cgacaagtcc tgcagctggg agttggaata 35280
   aacttggttg ctaaaagcat ccatcacaca taaaacaggg gagatctggc tccaaagaat 35340
   ggtagatgta gatcttcccc tacggatttg ttttctgaag atctccttat acactgacct 35400
   cagccttttg ctgacctatc cttgtgaact gaaggctgaa aaaaccaatg tagccatggt 35460
   ccacatccat ttatcacttt cagaaatggc ccctgcactt tatatttctt caggtcctgc 35520
   aaaaaatatt gaaaatcctt tacaaataga ggcaaggtca ggccatccct gtgggatttc 35580
   cagggagcag tgcactgacc cgttttgcag aatgggaagc aaaaaataaa actcagactt 35640
   agacagctgg cataagcaag gacttcccta ttctgagtgc caaatgtcaa tttgaaatac 35700
   ttgaactttt attctctggg acatgtactg gcattagtaa gaatttctat ttattgagca 35760
   tttatttggg gcaggcagat gataagttat acacattaat tatcttattt gattttcaca 35820
   atgacctatc agttacataa ttgattgtct gcattttatg gataaataac ctgaggctca 35880
   gttatttgac ttgctcaagg tgatgtaaca agtaaatggc agagccatga tttaaataac 35940
   aaaacgcatg ctcttaatca ccacattaat gtcttgatat caattaatag aaccatttcc 36000
   cctccatttt ctctttagtt tcagaactgg taagagtttg tctaataaga tttttataaa 36060
   gtggtgacat gaatggcttg tggggatgga gactggggcc ggctgccaag ggtacccatc 36120
   tcacacgaag catcaggtcc acttgggttt gtttttggct gattacaatc ttgaatcaaa 36180
   cttttcagta cagctggtct cccaagaact ttctaatgtt gatttgagct tccctgaatg 36240
   aatctcagaa aataagaaag ggagagagaa tgcctgtgta ttgagcttga cccatttcac 36300
   agatgccgaa atagaagtga ggcgacttgc ccagggtccc tgtggtggat tgtgttgttg 36360
   tctcaattat ttgctcccac ctcatacaag gattatacat ttcacacatg gctatgtgac 36420
   atacatgtct acttgcagaa gggacacact ttccttccca ctgatgtgga gcattggcca 36480
   atggaacgtg agcagacaca atctactcta catttaaaca gaggctttga gaagctattg 36540
   tgtggtttag ccattgttct tcccacctct gccacaagac caagaaaggg ctgttgcctc 36600
   agctgcgtcc tgggatggaa aagacacagg aagtagaatc acagagccac agacaactgc 36660
   agtgacacgt agcacgagta agagctaaac ttcgaatatt gtaagtcact agaattttgg 36720
   ggttgcttat tattgcactt acctaccaaa agctgactaa tacagtcata tagccagtaa 36780
   cttgactaag ctggagcttg agccaggtcg aactcccaat cctatccatg gtctttttct 36840
   tatttcagtc ttccctaata ttccattcta acttacatca tattttctgt gtctgtgtgc 36900
   tgtctgtcct tttttctttt aaataaatct ttttctacca aagatataga acaggaaata 36960
   atatttttct ttaaagagtc tgttttttca tttcaaccca tttattttga aggaaatttt 37020
   atcatcgtat aaatagaaaa acagtgtcat cacaaataat tcataactat aagcagtggc 37080
   tcacgcctgt aatcccagca ctttgggagg ctgaggtggg cggatcacct ggggtcagga 37140
   gtttgagacc agcctgacca acatggtgac actccatctc tactaaaaat acaaaaatta 37200
   gccaggcatg ctggcagagg cctgtaatcc cagctactca ggaggctgaa gcaggagaat 37260
   cgcttaaacc tgggaggcgg aggttgcggt gagccgagat cgcgccattg cactctagcc 37320
   tgggcgacag agtgagactc catctgaaaa aaaaaaaaag ggtaatttaa acaatattat 37380
   gaagttctag ctaggtactg ttggcagaca agaatctgat tttgaaatat cgtctctcta 37440
   tgttccaaaa aggagtttgg caaatattag aatggtgttc aaaacataca agcaccaaac 37500
   tgatactttc tcctttgata aatcaggaag attaaaaaag aatcagaaga acaatttctt 37560
   cctagtatgg cttggtgtga tgagtgtcat ctgtattcca tataaaattc tttcagatat 37620
   caccaatgag tgatatgttc attacaatgg ggggtgtccc tttctttggt tcatgtataa 37680
   taactggaaa catgtatata gcatgtggta tgtgccaggt actcttcaag atactttagt 37740
   tatttaatct attgtcagat aaggaaacag aggcttggag aggctaaaca acttatccta 37800
   gctagaaaga ggcagaattg gggcttcaag tcaggtctgt ttgattccag aggcaacatc 37860
   ctaaagcaaa gtttccttct aactgctcac ttgggacttt taaacgaagc cattaggtgc 37920
   ttcactttct tgtggcatgc cagagtggta gctgcttcag ggcatcagcc tggggccaca 37980
   tcaggaccta cctgcacgta ggttgatcag gaccctggca gggctgtcag gtacagccac 38040
   aaagcaagct gtttctgact gtgttcccac actttctcag gcatccaccc tggtgtatga 38100
   atttgcttct tctttataac aaactacctc taaactgcac tctagtctac agatcagctg 38160
   ggcaatgctg ttgatctcgg ctgggcttgc tcgtgtgtct ttggtcagct agcaggttat 38220
   ctggggcctg attggtctag gatggcttca cttgcacatc tggcacttag atgtgtgtta 38280
   gctggagcaa tagggggcct ggaccccgtg ggtctccttt tccagcaggc cacttgggct 38340
   tgttcacagg gcagatgggc ccagttcaaa gggtctgaga acacaacggt actgcaggat 38400
   gagctttgga actgctgcac cgtctcttcc actgcattct gcatacgggc cctaaacaag 38460
   tcccaagcct tctcagattc aagacatggg ggatgggctg tatctcttat ggagagagtc 38520
   acatagcaag ggtgtggctc tagggaggaa tgaagagtgg tcaagtgcag cctgccatgc 38580
   tggctccttc accagcctgt cagttatatg agccaatggg ctgaatcata gtcatctttc 38640
   tctccacatg cctggcccgt gaaggatttg cctgcccagc agagaccgtg gaatgaatgt 38700
   gtgctccagg cactctgcct tctctctctt ctttgacttc tctaagctcg ttcctgcccc 38760
   tgtccccctc tgtctctctt ccccaggatc atggcattag tcggatcctt cccatcattt 38820
   tcattttagc tccagtatca cctctttgag ccttccctga ctacccttcc agcattctct 38880
   aatcccatca ccttgttttt tttatctttt cttttttttt gagacggagt ctcactgtgt 38940
   tgcccaggat gaagtgcagg ggcacaatct cggctcactg caacctgtgc ctcctgggtt 39000
   caggtgattc tctggtttca gccacccgag tagttgtgat tacaggcatg tgccaccatg 39060
   cctggctaat ttttgtattt ttagtagaga cagggtttca tcatgttggt ctggctggtt 39120
   tcgaactcct gacctcaggc aatctgccca actcagcctc tcagcctccc aaagttatac 39180
   aggttttttt tttttttttt tttttaaatc ttttcatagc atctgtaact gtttaaaata 39240
   ttagtttgac ttccttttct agaatgtaag ctttgggaga gcggggtcct tgcctgtctt 39300
   gttctctgtt acttctccag ctcccagaat ggtggctggc actcaggggg tgctcaacac 39360
   acataattgt caagaacatg ctacatcaag gcctgagtgg ctttgccacg gctcttcttg 39420
   tgactgcagg ccttggtgcc agcagccacc ccagttccaa gaaatggtct cttgctggcc 39480
   aacttagcag gaagaactgg cagcgttcct gttacagcac ttccaggtgg ctgcttttcc 39540
   ctgagtccat gtggcttctc catgtctctc tgttggagtc acaagttgtg gaaattcact 39600
   taggccaact tagaccaaga agtggaatag ttaatagtta aatgtcagtc tgggcaacat 39660
   agcaagactt cgtctttaca aaaaattttt aaaaatatta gctggtgcgg tcgtgtgcac 39720
   ctgtagtcct acctactcgg ggggctgtgg tgggaggatc gcttgagctc aggagttgga 39780
   agctgcagtg aactatgatt gtgccactgc actccagcct ggatgacaga gcaagaccct 39840
   ttatctaaaa aacaagaaaa aattaataaa taaaagttaa atataaggtc tctggaggta 39900
   tcctgtttgg gttcagattc tggctctgcc acttcctagt tgggtcactt ttgggaagtt 39960
   acttaacctc tttgtgcctc agtttttcac ctacaaaaat agagcaaagt gtaaaaatat 40020
   gaactacctc acagggttgt caggaaaact aaattttgtg tatggaaagc actgattgct 40080
   gagcagatgg tgagcatctc tgagtaggag ctcctgctgc cattattatt attattatta 40140
   ttggaggtta tcatgtgtga ttgaagagca ggaacttcag ctgggcttca ggagcaactg 40200
   gttagaaaaa cggaagactt ggcccggcgt ggtgtctcat gcctgtaatc ccagcacttt 40260
   gggaggtcga ggcgggcaga tcatctgagg ttaggagttc aagaccagcc tgaccaacat 40320
   ggtgaaaccc cgactctact aaaaataaaa taaaaaataa aaaaaaaatt agctgggtgt 40380
   ggtagcggat gcctgtaatc ccagcatttt gggaggtcga ggtgggtgga tcaactgagg 40440
   tcaggagtcc aagaccaggc tggccaacgt ggctaaaccc cgtctctact aaaaacacac 40500
   acacacacac acacaaaaaa aaaaaaaaaa aaaagaaaaa aaaagttagc tgagtgtggt 40560
   ggtgggcgcc tgtaatccca gctacttggg aggagactga ggcaggagaa ttgcttgagc 40620
   ctgggaggtg gaggttgcag tgagccgaaa tcgtgccatt gcactccagc ctgggcgatg 40680
   gagtgaaact ctgtcaaaaa aaaaaaagag agaaaaaaga aaaaaagaaa aatggaagac 40740
   ttatcgggaa tgcaggcagc tgtgcgattt cagcttctct ttgtttttgg accatgggtt 40800
   ttctggttct tctttggttc ctgcaggtct gactttctcc tttttctgtt gatggtcttt 40860
   cttggcctgt gtttgcaatg ggtcaaccat gtgctttctg gccatacttc acacttactt 40920
   cccaactggg acagatagca tttcttaagt ctaattccaa acttctggga taatctgatt 40980
   gcccttcatt gagtcagaag ccagtggata gggacatgga agatgctata gctgggctcc 41040
   acccttgcag gtgagaggag cagtttcagg aaagtctgcc aggcatgagc tgagacctta 41100
   agagatgtca gctagaatta gccactgtca gtatatctgc ttctgggtcc cactcttcat 41160
   cctgcttttc aaggttttca aatatacact cctactcagt ctcatgaaaa ttattatttt 41220
   taaaataccc tttagcacat ttacctctaa tgatactaga ttcttgcctc ctgcctctgt 41280
   aaatcactcg gtcacttccc gatcttatat ttggctgtca agaatgtgga aatctcacag 41340
   aacccttgaa tttcaaagtt ggaagggaaa attgcttgtc tatgcttata aaaaatacac 41400
   acctacaccc acgtacacat gtatttacta aaatagtatt aggctaaata ttcagttgta 41460
   cagctttctg atttcattaa caatgtctgt ctttccgtgt cagaagagat agacatttga 41520
   aaaaacggca gcgtaggatt ttgttctatg gatgagctaa aagttctttc acctgtccac 41580
   aaacttccac ctagtccgtg tgccttataa aacacaaatt ctgaaatccc atgtaacgtg 41640
   tcagctagat tctgcctatc actgagtaga gatgaaccat cacatctcat gttctagatt 41700
   tggggttaca aattcaaatt cctacagggg ccaggcaact gtcttagatg tgtgaaacgt 41760
   cctagatata agaaatgcct gtaaatgaaa ccttaagagt ttttataagc atacaaataa 41820
   gtatgtaata ttttgaaaca tacttatttt ttggtgtacc cacacaggcc tagcatcacc 41880
   agattcttct ctcttttttt ttttttttgt tttgccccaa gagaggcagg aaatgagtat 41940
   atttatggga gctttcctaa ttgtgaagtg ttggtaagtt cattattttt aaaaaactaa 42000
   aaaccaactt gcaggccaaa caaaatgtgg gccaccaatg tgtaatcact gatgtagata 42060
   atgcacttca attaatctaa aatcatcaac tattttggca gccctctcag actgctgact 42120
   cactgagctt ttcatctgat gaaatcctta agtgattcac aaatacttaa tctgaaaacc 42180
   tcttaatcta acttgtgttc ctggacttgt aaattgggtt tctggacctt aatacttgct 42240
   catgttaaat ccattctgtt acttatgttt aaggtcctac atctgtcttc cagcctgatc 42300
   acccatcttt tcagccatgt gttgtttttg aattcaactc atttgatcct ttctctatgt 42360
   cctttttttt tttttttttg agacggggtc ttgctctgtt gcccaggctg gagtgtggta 42420
   gcacgatcag gactcaatgc aacctcgact tcctgggctt aagcgatcct cctgcctcag 42480
   cccccacaag tggttgggac tacaagcacg tgccaccaca cctggctaat ttttttgaat 42540
   tttagtagag atgaggtctc actatgttgc ccaggctggt cttgaactcc tgagctcaag 42600
   taatcctccc agctcagtct cccaaagtgc tgggattaca agcgtgaacc accatgccca 42660
   gcctctctgt gtcttatgat aaaaaaaata ctggatgaga caagactgag gaggaagacc 42720
   cagggaccct ttttagcaga gcttaccagc gggtgtccaa ggattgaatt tcccttccta 42780
   gacatgtttt gcttcaacta aaaagatata tatatatata tatatatata tgatgtatat 42840
   cacacacata tgtgtatatc acacacatat gtatgtatat cacacacatg tgtatatcaa 42900
   acacatatgt atatcacaca catatgtatg tatatcacac acatatatgt atgtatatca 42960
   cacatatatg tatgtatatc acacatatat atgtatgtat atcacacaca tatatgtatg 43020
   tatatcacac atatatatgt atgtatatca cacatatata tgtatgtata tcacacatat 43080
   atgtatgtat atcacacata tatgtgtgta tatcacacat atatgtgtgt atatcatata 43140
   tatatatgca tgacccatat ttaaaagtag gatatttcat aacgaaatac cagtattagg 43200
   tatcttttga aaaagtgtcg taactgacag cccaagtctg cattccttca tggcaccagg 43260
   gctagaactg agaagtagct gactttttgg ggtggatatg ggctccccta gtcatgctat 43320
   atgcctcatt ttatgtgact ggccgggccc cataggcgtt tatgtttgcc actcttttag 43380
   aggtcccttt aaggttgaca tatattcatt gattgatcaa ctcatttaca cacgcatgca 43440
   gtcatttttt ggacccctgt ctatgtgctc tgcacaatgc gaggtgtggg atataatggt 43500
   gaccaagaca gattccaacc actcactcac agatcccaca gcctaccagg gaagccagat 43560
   atggaacaat tatatacgta attttacttg taagtgccac aaagacaaat tgtattaagt 43620
   agtaccatgt ggctgtggaa gtttaacttg tcactaatcc accaaactct gagaagacag 43680
   catattttcc gtcattccca aaagactatc acaggcaaca ttgaggaatg ctttgttaaa 43740
   ctccagggat atattccaca tcacacttcc cccgtccacc agattagcaa ctgtccaagg 43800
   acaaaacaaa acacacacta aaaaggaaat ggggttagtc cgatatgtct ccatctcctt 43860
   atgctggttc ttttgtctgc agaagccacg gtttcttttt ctttttgaaa aaaaaaaatg 43920
   acatttgcgt acctctctgc gtttctcttt ctccaggatt cttctgggat cctaggcagt 43980
   gtggtttggt gatctcactg gtgagatctc ttggagccct ggaatgcaat tcatctgggc 44040
   cagaagagtt gaactaattt aaggacatct gagtgcttca ctgatgtatt tgttctgctc 44100
   tgtcccttca aaataccact actcaaaaca atgttcagct ggagaaagtg gttaacactg 44160
   gaattttctt tttttctgag acctaagtga cagggaaggg tggatgaagg aaacatatcc 44220
   tttgaaaatc ctttagttag aatcctttgg ttgcaagtaa cataaatgaa ctctgattaa 44280
   tgtaagcaaa aaagaatttg ttagaaggat atgggggggg agggatggac agagatgtga 44340
   aggaaaagat gaagaaccag gctttagaaa gtcctgaaac caaatgaatt ctagggatct 44400
   aggaagcagg gactaatgac tgtttccttc aggacaccag agatggggtg aataactctg 44460
   taaaagatgc aaatttccca cctgggtaac atagtgagac cctgtcctta aaaaaaaaca 44520
   gtagccaggc acagtgcctg tagtcccagc tatgtggagg ctgaggcaga aggatcactt 44580
   aagcccagga gctcaagact gcagtgagct atgatggcac tgctgcaccc cagcctgggc 44640
   agcagaggga gaccctgtct cttaaaaaac aaatgctaaa ttttagagag aatagagaga 44700
   atcttttctt aaaaaggtgc taagtctggg gaaggagctt ctgattgtcc tgccttgagt 44760
   cacacgacca tctctgactt gttgacccct ctacaatccc atataagact ccccaagagt 44820
   agattctgag caggtaaagc ccatgggtgt tcccaaaagt cggcctcaac ctgaagccac 44880
   ttattcactc tgagctcatc accaatggca tgccgcactg tcaaaactct ctttctgtgc 44940
   atttgtctgg gccactcaac tcaggaaagt tagttgttct gtgtgagtcc ccatgttctc 45000
   actgatttca tttcagtctt taagctaaaa atctgtgtaa atggtaatgg agaatggcta 45060
   catcacactt agtatgtgtc aaagcagtat cctaagtgcc ttagtggtat cagcttattt 45120
   aagactgaca gtactctgag gtgcagtcac ttcttttttt ttttttcctt tttttcaaaa 45180
   cagagtctca ctccgtcatc cacactggag tgcagtggca tgatctcggc tcaccgcaac 45240
   ctccgcctcc cagggtcagg tgattctcat gcctcagcct cctgagtagt tgggactaca 45300
   ggcatgcacc accactcctg gctaattttt gcatttttag tagagatggg gtttcaccac 45360
   gttggccagg ccggtcttga actcctgacc tcaggtgatc cacccacctg ggcctcccaa 45420
   agtgctgtaa ttacaggcgt gagccaccgc acccggcctg tcacttcttg tcattcacag 45480
   tagttatgtt ctgtaaagtc actgcaaaca ctgaattaat aaacactgaa ccattgttcc 45540
   taggtgaaat acaaagttgg gttcctgtga gcctttggtc acagcatttt catccactga 45600
   tcaacccgta agcttattct gtgtgtgttt ctgtttaaag acaccttaat atatattgtt 45660
   gactcattaa catctaactc actgccaaca gcactccaac tcgtgcctga tcaaagctta 45720
   tataacacac gcatgacaca tctcggactc cttgcactta ggaacaccag atagcacttc 45780
   accattaccc tatgggacca ttttacagca acgtcaccct caaaaagcag aaaaatggga 45840
   aaaagcatgc tgctaatata ccacagagag aatgcttgtt tacagcatga gagctgaagt 45900
   ctgaaggtag aacatggttt tgttcatcct cagctgggaa cacatgttgg ctgactcaaa 45960
   tattttgcca ctccgtacat gtccgtgagt gatcatgaaa gaaccttgag tatggatttg 46020
   gcggttataa ataaatttta gcaagaggtg aatttgcaaa taatgaggat tgactataac 46080
   ccctgttatt agctctagtt aacagatggg ggaatggagg taaacagctc tctgcaggtc 46140
   ttgcagccag ggagtgtaga ggaggctttg tcattctggg tgcacagcct gcgtccttaa 46200
   gaggcaatgt agtgttggct gggtgtggtg gtggttcatg ctgtaatcct agcactttgg 46260
   gaggctgggg tgggtggatt acttgaggtt aggagttcaa gaccaggctg gccaacatgg 46320
   tgaaaccccg tctctactaa aaatacaaaa aaatcagtgg ggcgtggtgg cacgcgcctg 46380
   taatcccagc tactcaggag gttgaggcag gagaatcact tgaacttggg acgcggaggt 46440
   tgcagtgagc tgagagcgtg tcactgcact ccagcctggg cgacagaatg agactccatc 46500
   tcaaaaaaaa aaagaggcag tgtagtggtt gagtgcagtg tagctcctgc tgagagctgg 46560
   ttttcatgtt ttgtgggcac acaggaagta gagaatgctg agagctgggc actgaagcag 46620
   taggagatat gggagtggga aagtggtttt ctttagtaag tctgggctgt tttcatagcc 46680
   taggatgaag ttcagcaata ctcaaccttg aggttttcca ggcaccttct ccttgacatg 46740
   aatctcctgc aaacagatgt catagtaatc tgggaataaa cctttgttgt tggtccttta 46800
   gctatccttt tttttttttt tttttttttt tttttttttt tttgagacgg agccccactg 46860
   ttgccaggag tgcagaagca tgatctcagc tcactgcaac ctccgcctcc ccagttcaag 46920
   tgattctcca gcctaggcct cccgagtagc tgggattaca ggcatgcacc accatgcctg 46980
   gctaagtttt gtatttttag tagagacggg gtttcaccat gttggctagg ctggtctcga 47040
   gctcctgacc tcaagtgatc cgcccgcctc gacctttcaa agtgttggga ttataggcgt 47100
   gagccactga gcccggtgcc ctggtccttt agctttctaa gacagcagca tagtagttca 47160
   gagcataggc tcttgggcca tagcttcctg agttcgaatc ctagctctgc tgttttttaa 47220
   actctgtgac cttgggcaag tcttaacctc tctgtgcctc agttttcttg tctgcaaaat 47280
   gggattaata aaaacaccga tctcatagga ttgttgtgaa aatcgagtga gtatgtttat 47340
   agtgtttagg aaagttcctg gtacacaaga catgctctga aagtgtatta atattatttg 47400
   tccttagact cagtagtact ctgggggaat cgaatcccca cccctaagcc aggcagcatg 47460
   gtagaagcta ggtcagctgg caggctcctg ggtcttttcc attctgctgc aggagatgcc 47520
   tgactcacct cttgtgctca cacagatgac agctaaatag aagggctctt tcctcctagt 47580
   attagcttaa ttataggggc tccctagaac agtggggaaa atgagctttg agaatccccc 47640
   caccacacac acaaggccca cactagcacc tgaaagagga gtgaacattg cttcctgcct 47700
   ccttctcccc ctccttctca catatgcctt tgtctctgtg attctgcacg tttaattttt 47760
   aatcccggga tgggttctat tagtttttgt gtattttctc tgccctggtt tgagctccaa 47820
   aaggtctgtt tgggtctctg tgggtgtctg acagcctctg caggcctttc tgaattatat 47880
   aactcaagct ttgtattttc tatttgttac tgggggttct ttctgtgcta taattttact 47940
   cttagaaaat gaagtagggt actagaattt tctctgggat cccctagggt ttccttctga 48000
   atattttttc ctgccatatt ttcttcgcta aaacattttt ttttcttatt tcagaagcaa 48060
   tacatcttca ttatagaaca ttacaacaag atgcagagaa agaaaataat cttatcaagc 48120
   agaaatgtta ataccttgct atctacccat gtctctctgt ctctgtctcc tcaaacacac 48180
   acaaatgggg tcaagatgac ataagagact atctgctttt caattcacat tttacgatct 48240
   ttttttttgt atggttctgt agtctccact gcttggcttg acgatcttta acaggtgccc 48300
   tccggatggt catgggggta ttttccattt ctcactgtta caagcagtgc tctgatgaaa 48360
   caccttttgc tggcattcct aattatgtcc tgagagcaga cttctagaag ttttcagcag 48420
   tgaggttaaa gggaatgttt gcctctcctc ttcactctgt gatgctcctg ggggcccagg 48480
   cccatgagtc tctgtgtcca tcttcccaag atgaaagaca aagaatgggc agagcattga 48540
   cagggccctg tgttgactcc ttttctctct tttcccagtt tttttttttt tttttgagac 48600
   agagtctcgc tctgttgccc aggctgaagt gcggtggcgc ggtcttggtt cactgctaca 48660
   tttgcctccc aggttcaagc aattctcccg cctcagcctc ccaagtagtt gggattacag 48720
   gcactcacca ccacacccag ctaattttgt atttttagga gagacggggt ttcatcatgt 48780
   tgcccaggct ggtcttgaac tcctagcctc aagtgatcca ctcaccttgg cctcccaaag 48840
   tgctgggatt acaggcgtga gccactgcac cctggcccat tcccgttttt taaaaaaata 48900
   ttttattcct tttagactat ttagccacag ttttccattc tattccttgg gataagtgaa 48960
   aacttgattt ttaaaaaata attacaaaat tttaaaatca tggtaaaaaa cacataacat 49020
   aaaatttacc atcttaacta tttttttaag tgtacagtat cattttaagt acatttacat 49080
   tgttgtgcaa cagttaggac ttttcatctc gtaaaactga aactctatac ccactggcaa 49140
   taacttccca tttcttcctc cccaccgagc ctctggtaac caccattctg cttcctgttt 49200
   ctatgagttt ggctatttta gatgcctcat gtaagtggac tcttgcagta tttgtctttt 49260
   catggctggt ttcttccttt ttttcttttc ctttctttct ttctttcttt ctttctttct 49320
   ttctttcttt ctttctttct ttctttcttc cttccttcct tccttccttc cttccttcct 49380
   tcttcctttc ctttctttct ttcctttttt tttttttttg acagggtctc cctgtttctc 49440
   gcaggccagg ctggagtgca gtggggcaat cacagctcat ggcagccttg acctcctgtg 49500
   ctcaagggat cctcccatct cagcctcctg agtaactggg acttcaggca cacactacca 49560
   tgcccggtta atttttttct tgatttttaa tagagacaag gtcttgctat gttgcccagg 49620
   gttgtctcaa actcctggcc tcaagagatc ctcccatctc agcctcctga gtagctggga 49680
   ttacaggtgc acactaccat gcccagttaa tttttttctt tgatttttaa tagagacaag 49740
   gttcttgcta tgttgcccag gctggtctca aactcctggc ctcaagtgat cctcccacct 49800
   cagcctctca aggtgaaggg atgagcctcc caaagtgaag gcgtgagcca ttgctcctgg 49860
   gatgactggt ttatttcact tagtgtaatg ccctcagagt tcattcatgt tgtagcatat 49920
   gtcagaattt tcttcctctt taaggttgat taatattcca ttgtatgaat aggcaacatt 49980
   ttgcttatat attaatcggt caatggacat ttgagttgct ttcacctctt ggctattgtg 50040
   aataatgctg ttgtgaacat gggtatacaa atatcttttt tttttttttt tttgtagaga 50100
   tggggtctcc ctatgttggc caggcttgtc tcaaactcct tggcccaagc aatcctccta 50160
   cctcagcctc ccaaagtgct gagattacag gtgtgaacta ctgcacctgg ctgagactcc 50220
   acttccaatt cttttggata tagatcctga agtggaattg ctgatggtag aaactgtctc 50280
   gggaagattt ttctggggat ttggcttcta actgcaccac agctccagtg gaaacatcta 50340
   ttcattaaac tggcatttat tgaggaccag ctttgttcca gtcactgaac aaggctgggg 50400
   gcatagcgtg gcttacatag gtccccaccc tccatggagc tgatattcag caagaagcca 50460
   gacaatacac aggtaagcaa attgaaaaaa acaggatgag atccactggc gataaatgtc 50520
   atgatactga atcaggattt gacaacagag tgatgtggtg agtggctact ttagatcaga 50580
   aaggccttcc taactgggtg acaggtgacc tgagaactac aaggtgggga ggagctagcc 50640
   acacagaatt tgggaaaata ccattctggg ccctgggatg agcaatggaa aggtcttgag 50700
   gaggaaagtg gctaggcgtg atccttgttg ctggggcgtg gtgggtgtgg ggccagatca 50760
   taaggggccc cacaggatga gcctctgtgc taagtgcagt gggaagccac tggagagttt 50820
   taggtaggcg agatgcaaag gatcagattt gtatttaaca caccttccat tggctgccaa 50880
   gtagagaatg gattgttggg gactggagca gaagtgggag gatgagtcag gagtcaattg 50940
   cagatttcta ggtgagagat ggtgtctttg tctgcacggg cagcagtgca gagggagaga 51000
   cacaggcaga tttgggaggt aaaaggactt actggtaaat gggatatgaa ttggaagagg 51060
   gaagcaaatg cacagctggg cacctggtac actttacata tgacgtcttt gaacttcgta 51120
   gcaatcccat aaccacattt tgtagatctg tgttgtccag tagaaatata acatgagcca 51180
   taaacctgag ctgtgtatgt aatttaaaat gttcttagca atcacattaa aatggtcaaa 51240
   ggaaacaggt gaaatgaact gtgagaatgt tttctttaac tcagcatatt ccaaatattc 51300
   ccatatcaac atgtaatcaa tataaaatta ttaatgagat attttacttc cccgcatagc 51360
   aagtcttcaa catttgatat gtattttaca cgtacagcac atctcaattc aaccacactt 51420
   caagtactca gtagccacat atggctagtg gttaccatat tggtagcaca gttatagatg 51480
   aggaaagaga gtctcagaga gtataagttc ttatgcaagc ttatagaatt aggacttgaa 51540
   ccctaatttg actctggcac ccgtgttgtg ctcaaagcac tgcatccacc cccaatgatg 51600
   aagacatttt caatgtaagc aaaagtcctg gagatttccc cacatcatat acatggggac 51660
   ataggaacta acatttgtca tatatttata ttttcctttc caaagcagat caaccccaag 51720
   ttaccttccc ttagctctcg ttttctgcct ctctttgcaa atatcacttc attaaaaatt 51780
   gtgaacattc tctttgcctt cccttatggg cttcctgttt gaacaccaca tccagggcac 51840
   cctgggaact gaaccatctc cttttctttt tctttttgag tcagggtttc actctgtcgc 51900
   ctagactgga atgcagtggt gtgaccgtag ctcactgcag cttcaatctc ttggcctaaa 51960
   acaatcctcc cacctcagcc tcctaagtag ctgggactac aggaacacac caccatgcct 52020
   ggctaatttt tttattttta ttttttgtag ctgcaaggtc tcactgtgtt gcccagactg 52080
   gtctcaaact cctggactca agcagtcctc ccacctcagc ctcccaaagt gctaggatga 52140
   caggtgtgag ccacaatgcc tagcccatat ctccttttct tttgtggcat tctagtagcc 52200
   ctttggttca tgcgatcatt tagttgttca gcaaatatgg atgattgaat ggatataata 52260
   ggtgcagggc actcccaggt tctcaagatg gatgatcttg ggcaagttat ttaatttctc 52320
   tggattttag tttccttgcc agcaaactca agataatagt aatgcctact tttaagtagt 52380
   tattgtgaag attataagga attaacaata acgataataa taagtaataa tgatacatgt 52440
   tgtttattag agcctgctat atgccagaca ctaactctat gatttaacct tctcctgcta 52500
   ccctttactc ttattatttc tttaggagtg aaattcacta atcattaagc tttttaacta 52560
   actgaatcaa ttctgtttct taagcagatg ggagatcctg tggcttttta ccactatcca 52620
   cagctctttt tttctttttc ttttcttttt tgagacggag tcttgctctg tcacccaggc 52680
   tggagtgcag tggcatgatc tcggctcact gcagtctccg cctccctagt tcaagccatt 52740
   ctcctgcctc agcctcccga gtagctgagg ttataggcgc ccgccaccac gcccagctaa 52800
   tttttgtatt tttagtagag atggggtttc accatcttgg ccaggctggt ctcaaactcc 52860
   tgacctagtg atctacctgc cttggcctcc caaagtgctg ggattacagg catgagccac 52920
   tgtgcctggc ctatccacag ctggtttccc ctctttgccc ccttttctgg cattctcttc 52980
   tgattcactc tcaactctga tttccaagct acccagctcc ttcccctagt gtgaatctgt 53040
   atttgcactg gatttatttt taaatgggaa atgaaagcca ctgtgtgctg gaaaatatat 53100
   atttcagaca cgaggaagct ggcggggaaa tattttttgg atgtctttct agatctctta 53160
   aaaatcctgt gagccatcac caggttgatc ttgtagtaag gtggccttgg ccttagtgta 53220
   tttctttttg actcgatatt gggcggcagg aggtttgagg attgtgatac taacaacatt 53280
   aacaacaaca aactttcatt gattgattgc catgtgctgg aaatgtgcta ataaattaca 53340
   tttattttct ctttgaatcc tcaaaccaac tctttgagac aagtgtgttg tattacagct 53400
   gtagaaactg aggtcctaag aggtcagtag atttgctgaa ggtcacagag ctgagatcca 53460
   aatctaagca tcacagcaag aagtatcctt aaccatcatc tttgctgttg gttggcatta 53520
   gtctggctgt gctaagaaag tttcctttga cttgcagatg gatcaaggct tttcttgaga 53580
   gaggcttctc ttgcctggtt ctccttgatt cttttactct aggagactca gttcttctca 53640
   aatccctgtc tgtgggactt tgggcaaaac attcaacgtc atctttacct cttagagtca 53700
   ctgcgagggt tggactatgg tgtgttaggt gttttttttt tttttttttt tttttttgat 53760
   gaagtctcac tctgtcgccc aggctggagt gcaatggcat gatcttggct cactgcaacc 53820
   tccgcctcct gggttcaacc gattcttctg cctcagcctc ctgagtagct gggattacag 53880
   gcacgcacca ccacgcctga ctaatttttg tatttttagt agagacgagg tttcactatg 53940
   ttggccaggc tggtctcgaa ctcctgacct caagtgatcg gcctgccttg gcctcccgaa 54000
   gtgctgggat tacaggcgtg agccaccgtg cccagccgtg ttatgagttg aacagatttc 54060
   ttggcttcag caggggctca gtatgggcct atcatcattt tcatttatct ccttttatgc 54120
   ctcatgctct atctgtcccc ttcatctctg cctgtagcag ccacagaccc tctctttcct 54180
   tggtgcatgt ccctccttcc cactttccta atttcagcct gacttagttc tctacctgtc 54240
   agcttggact taaggaaagg aagttctctt ttcttggggg gtcactgggg ccatgggcgt 54300
   ctttccactc catttctttt ccaaagctgc ctctggaacg aatctgtaaa tcccagggca 54360
   ttgcatgagt tttcttttct ttctttcctt ctttctttct ctctctttct ttccttcttg 54420
   ctttcttgct ttctttcttg ctttcttgct ttctgtcact gaaagcaaat attgtagaca 54480
   gatatgcttg taggatagaa ataaaatgcc ccattctttg aaacatattt tgttttttat 54540
   tttagattca tggagtatgt gtgcaagttt gtcacatggg tatattgcgt gatgctgagg 54600
   tttaggcaat gaacttgtta cccaaatagt gagcatagta cccagtaggt agtttttaaa 54660
   ccgttacccc cttctctccc tcctttccga gttcccagtg tctgttgttt ccatatttgt 54720
   gtctgtgtat accccttgtt tagcttccac tataggtgag aacatgtggt atttggtttt 54780
   ctgtttttct gtgataattc acttaggata atggccttca gctgcatcca tgttgctgca 54840
   aaggacatga tttcattttt tttatggctg tgtagtattc catagtgtat atgcaccaca 54900
   ttttctttat ccaatccatt gttgatgggc acctaggtgg attccatgtt tttgctgtta 54960
   tggacagtga tgcaataaac atgagagtga aggtgtcttt ttggtggaat gatgtgtttt 55020
   cctttgggta tatacccagt aatgggaatg ctggattgaa tggcaattct atttttagtt 55080
   ctttgagaag tctctaaact gcttcccaga ggggctgaac taatttacaa tttcaccaac 55140
   agtgtaagag cgtccttttt tctccgtaat ctcaccaagg cactgcataa gctatctgag 55200
   tggccagcag gcagaatgct tgaaggattc tccagctggg ggtgctgggc ccaaaggctg 55260
   gcagctgtat ggtcctggcc tttttctctc cagcagggtc cccatgatct tcccaggtct 55320
   agttttttct tgagcacacc tgcatctgac agttaattgc aagaatctga cttgctgaac 55380
   tctcatttct ccctcctcct ttagcgcatc aagtcccctt ctttgttttc ccagcttggt 55440
   tcttgcaagg actacctaga ggaggacttc aggcaatccc tttctccctg gtgggctgct 55500
   gtgttctcac caataaatca atgctctctc tagtttcagg ctgcctctta ggacagatgc 55560
   tgataccaag tgacttttga ggttgttgag ttgagacact tggaccaaac agcggaaggc 55620
   acagcttggc tcagctaaag gaagctccgt gaaagctctc tgagtgggcc tattggctcc 55680
   atgagttggc aggctggccc tcctctagga gatggtgggt cctcatgggc tgtagcaggg 55740
   ccaggccaca ggagcagact gctggaggag aacatttagg gccatcttcc ttgctgcctt 55800
   tctgtgatct cttcacccag gaagctgaat gctgttggca agcaaaaggg tgcttggtaa 55860
   ccatttcttt tttaagtgtg tgctggtgcc agggagggag aaatcctcaa aacccattgt 55920
   gtctgactca ggcaccactg gtttggccat aaccgctagt ttatcctccc tccctctgcc 55980
   accctcgtca tgtttctcga ccacaacctg tctggacctt ccctgggttg gccagggctt 56040
   gataatcttt ttctgtaagg agttatccct tcctcatctg ccaggatggg gctactgtgc 56100
   agggactggg taacaggctt gaagttgcaa gagggctgga agggtctcac tagcaagcac 56160
   acattatgtc ttatataggt ccctggcctt gtgcttcaga ccctatcaca gcttttccac 56220
   agctggggct gttgagaatg taagtggtga catagctgga tggctaagat ggggggagta 56280
   tgagacctgc aagcgtgtga gtctcctttt gctagcagag tgtgaatgaa atgtatttat 56340
   tttcccaata aattcgacat gactgatcat tcatccaccc acttacccac ccatctactc 56400
   actaacccac ccactcagtc atctacacag ctgttcactc gtccatccat ccatccatcc 56460
   atccatccat ccatccatcc atccatccat ccatccgtcc atccacattc aatgtagtgt 56520
   aatgattaag agcatagagt ttggagttat cctgcttagg tttgaatccc agctctgcca 56580
   ctcaccaagc atgtgatctt ggttcagttt taaaacctct ccttgtctca gtctcctcag 56640
   ctataaaatg agattgatgt tgaaattaat aacagagcct accttacggc attataaagg 56700
   ttatgtgagt cagtatgtgt aaggtgcttt cagtagtgcc tgggtcatag cagtgctatc 56760
   taagtgttag ctattagcat tatggcatct actcacctgt ttatcatcta taatagtcat 56820
   taaaggtctt caataaatat ttctggtctt tggccttgca ggccaatggt aggattgcac 56880
   tgcttgcctc ccttgtgggt aggtggggcc gtatgagtag ctctgaccag tgagttgtga 56940
   tccaaagtga tgtatgttca cttttatgct gggacatgta attgctggtt ctagattatc 57000
   cagaaaaaat ctttgagtta tggctgctct atcaacttgc tcctggagac atagaacttc 57060
   ttccttagct gatcaggcat gaggctgtag tatgtgagaa aaacaaactt ttacctattt 57120
   tgagggttat tactgcagga taacccagtt ctatctctgt ctctctcatc tatcatctat 57180
   ctatttacaa taattccagt ctcattttat ttcccttccc ttttcttttt ggcacctatt 57240
   actagctggc attttcttat attttttgga gtttcctccc aaaatttatt tgttattaat 57300
   tataagaata tgttttccat gagaaaaagg acttattcac ttatctaatt ccaatagtta 57360
   gaatggtact ttacacatag tagacactca gcacatattt ttaaaacaat aaatcagtta 57420
   atgaatggat ctacttgact tttagtaggt cctattgaac actgcattcc acagctcctt 57480
   gaaaatcctt actgaggcag ctaatattta agaaggaaca ccctgacctt tcactgtcca 57540
   gttttattgc ctgaatcaag aaaaatatcc ctgagataga cctcactgcc tctctgcaaa 57600
   cctcttaata cccttagtca attgtaagaa gctgcttgga accctgtaag atggtctttg 57660
   gccagatcaa gtcttgctcc tcttcctctg aatcttgtag gggcctttaa tctttctgta 57720
   actgtgattc cccagaacag gatgcctttg ttagcctagc atcttttctg ttacttctat 57780
   gaaacaagga gagacgctcc ctgggaccct taactatttc tggaagcaaa atgacccttg 57840
   ccccactgaa gacttaggag cctggtcact tccaaccggg cttttcatct ttgtgactcc 57900
   cagtcatggg caagaggcta gctagctcca atcctaatgc ccgtgccatc aactgcttca 57960
   tttctggttt cttccttgct tttgtgatac cctcattgac tctttccact gtaccctgtg 58020
   gacttgcctc tatgggacac ttcctcccca tcctgcctga agaggaaaga gcctgccttt 58080
   cttctgggaa catcatccca ttcagaaaca acaacctttc tggagtcagg agtttgcttt 58140
   cctcttgttc tttattgctg cttcctggag attgcattct tgtttaaaag ctcacctgta 58200
   agactctgat caaatgtcca ttcttccctc tcacttcatc cctgaagatg aatgggacat 58260
   ttatctttat atatgtatgt atgcctaggg gaaaaaactg aaggaacatt atgtatatat 58320
   ataatgcgtg tgtgtgtgtg tgtgtgtata tatataatgt gtgtgtatat atatatataa 58380
   ttttttttga gacagggtct tgctagactg gagtgcagtg gagtgatcat ggctcactgc 58440
   agcctcgacc tcctgggctt aagtgagcct cccaactcag cctccctagt agctaggact 58500
   acaggcatgt gccaccatac ccagctaatt ttaaaatttg tatttattat tatttttggt 58560
   agagatgggg tctcactatg ctgcctgcgc tgttcttgaa ctcctgggct caagcaatcc 58620
   tcctgtctca gcctcccaaa gtgctggaaa tacaggcatg agacactgca tccagtctat 58680
   gatattctta tgtttttaca atgtgcttat tattgagtag ggaaattatg agtgatgtgt 58740
   gtgtatgtat gtgtgtgtat acagatgtat gcatctgtgt tcaatttttc ctgttgagag 58800
   tgagtaaaca aatatttttg aaaataataa aatacataaa tgaggggcag gggctctctt 58860
   ggacacttcc aaaagtactt tgctcaattt tctatgttag gcctatctgc aagaagcata 58920
   agggtttgtc aggagctatt atgattattt attctacaaa tcccagttca acaaagaagg 58980
   gtgaggcttg agtgttcaat tagagaagct ggccagttgt ggtatcatat cagctctaca 59040
   tccaatgctc agttttcaag gggtcatgtt gctctgaaag gcctggtgag gttgatgagt 59100
   gtcttaatga agccacttgt gaaagataat gtgacatgtt tgtccatcac attgacgcag 59160
   gaaaggagct ttcacaaaag acagcacaga aattttgcag gctcggacca gaagggaatt 59220
   tgtcagtgtt tagcttggct gacatcccac accctacctt cttgtgcagt tagaatccct 59280
   tctagtgtaa tgtgtgaaaa accataggcc ccctgcccac accctcctag agctgtatca 59340
   ccaacagctt tcaagtgtgt gcaaattggg agacggctgg atcccacatc tctgggtgac 59400
   atttgctgaa acaaatgcct gttaataagt ggcacgattc ccttggtctc agtgataaag 59460
   atgtacctgt tactgggagc ccttgagaga aatctttggt gaaggccttc acactcattt 59520
   taattggtga tagacagatg tgttgagaaa acttacattt tttttctcca tccgtctcaa 59580
   ttagaatgtt atgaaatagc accccctttg ctcttcgtgc tttatgccct agactttaaa 59640
   gactgacaac gagtggttaa aaaatctcca ttgcttcagg atgcagtatc agctgatttg 59700
   gtggattaaa tcgttttaag gtcctgagct ttaattctta cttcagtgat tctcaaacct 59760
   tgttttgcaa aaccccagag ctttaatctt aagggatgag cctgctattt ccaagacaac 59820
   aaaattattt ctacttcata ttaatacaaa acattaaaat atacattgtc tttgtaatat 59880
   agtttattaa ttcaaagcac ttgcattccc atttatctgg tcttcataca aaaccatgta 59940
   tgagtgagta ggcagatggt cttatttatt acttcgttca atatatagat ttttgagtgc 60000
   ctaccggtat acagcactga acgaaagaga aaaatactag tttggatggg agctcaacac 60060
   aatctgaggg gatatattgg acccattttg cagaggaaga aactttggtt caggaaggga 60120
   aagtgaactc tgaagtttga cataaagtta gcttcagaac tagaatatct gccttctgat 60180
   ctcagcctat cacctatctc aggttctgtc tccacgatac catgtgattg gaactggctc 60240
   attaacctgg ggatggatca atcagcatct tttttttttt tttaacagga ttggatggaa 60300
   atctgcattc cttgatcact cagtctatag ggtgaactta caatggccct catgcttaat 60360
   cttgaaatac agggatggtc tgatggagtg tgctggagac cctgctgaga tctttggaca 60420
   actcctctcc agtccactac ctgaatcctt tctcttctcc tcaaaaaaat ctgagactat 60480
   ccaaatgaag agtaaataaa gggagaagta agaaggaaac atgagacaga gaggcagcag 60540
   gagcagaatg aaacttgggg agcagaggga gaagccaaga aaacttactg gacttggagc 60600
   caataaattg gccatggcat gaaggtttct gccagcaata tggcagccca ttccagctct 60660
   ttccattttc ttttcccagc tgtgctgcca agcaccatgc ttaggctggt ttaacagaga 60720
   ttatgctagg aggattttct aacattcatg tcctgattac cccttatctg attccttagc 60780
   agtagccttc agtggcctca gagaattcca ggttcagctg tctgaacagt tcaatgactg 60840
   tttgtaagtt ctgacttacg atcaaggcag gaaatgtgaa aagcattttt aaaaaggaaa 60900
   aaaaccatcc ggcgttgtta acatttttgt tattccagta ttttcccatg catataaata 60960
   tgtgtacttc ttttattttt cacaagagta ctctcacacc ataatattgt tttataaccc 61020
   tcttttccac tgtgctctag tgtaaacatt ttactgtacc aaatcttttt ccacaaaagg 61080
   atttttaatt actgcataga tttctattga acggatgtac taaaatttac ctaaacagct 61140
   cttattattc aacatgattc aaccattgtt acaaataata tagttgtata tttatcttta 61200
   tggaaagcta tttctgaata ctcctaagga taagttcatg tattagttag gttttgctgt 61260
   gtaacaaatc atcgcacaac ttagtggtgt caaacaatga ctatttactt acctctgcag 61320
   gtctgcattt tgggccaggt agttctgctg gactaggctg gaatcagtgg acataggctc 61380
   tggttactta tgcatttgca atcagctggg ggactgcatg ggatgatggt ggtatgtcat 61440
   cttccagcag gccaggttgt ttccatagca gttagagttg caagagcagc aaggaggcaa 61500
   accctgatgc acaaactttt tttcaagttt tggttgcatc acatttgctt ctgtcccata 61560
   ggccaaagca tgtgacatgg ccaagcccgg agacagtttg agaagacatt accaaaggca 61620
   tggctacaaa aggcatgaaa gtctcaggac cattgttgta atcaactcat cacagtcccc 61680
   aaaatgggag tttctgggtc aaaaagtgtg gccatttaaa ggttttcatt gtcacattgc 61740
   tctccagaga aattgtaact aatttacaca ccacctagag tacatgcatt tccaaattca 61800
   cttgatcact ggatataatc attggtttaa tttcccccca gaaaataata ttattgcttt 61860
   tgaagagtac atgctttgtg agggaagctg tcttgaatgt ccagcttcca ggtgcaagcg 61920
   cctgcatgtg tgcacaggtg acaccatgta gaaccgaaga accacctggc tgagtctagc 61980
   caacccagag aattgtgagg gaaaagaata tttttttaag gcagaagaaa aatgtatgtt 62040
   cattgtaaaa acctgaagca atgtggtacc tttatcatca atatcttccc cagtatttga 62100
   ttcattctga cattatgttg ttgggttaca tttatttcta ttacatttag gacctttgag 62160
   tctatattca ttagtgaaat ttgtctgcca tttactcttt ttttttggtt tgctgtcttt 62220
   gacaagtttt tgctattttc atcaaaacat ctgggaagtc ttccatcttt tcttgttttt 62280
   taatgcttag gaagagcata aatggcaaag gatttagaat attttttaga agtttgagtg 62340
   gatttaccat acccccaaag caaaacaatt tctgggcctg gtgccttttc caagattagt 62400
   tatttgataa ttttttctgg tgtatttcat gatcattttt ggtatacttt caaatgtgtt 62460
   atgttttttt aagaataagc atttaattga gtctctaatt tattagcata aaactatata 62520
   tattgtcctg taatgatgtg aaaaattgcc ttgccatctg caattctctt ttctcattct 62580
   tactgagttc ctatttttag tttttgaaaa ttttcttatg agctttgcca gagtgtcact 62640
   gttttatttt tcccctcaaa caaccacctc ttaaatttat tcttcagttc aacttttttc 62700
   tgtttccaga tgcattaatt tctgcttttt aattatttcc tcagttttat cactcttcct 62760
   agtttcttga gctaaatgtc cctatgtact ttcaattata tctactgatt tgtcaaaaat 62820
   tggatagtat gctcgtctcc tactatgata gtgatttttc aacacctttg tattttttta 62880
   aacaaatttt gttttgtaca tttctaatgt acttttatgt tataggagtt agagtttcat 62940
   gattctccag tcttattgtg aagttactca gatctgagtt caaattctcc tgtgctgcct 63000
   accaaatgat gaaccagttt tagattctcc taaaagcttg gtgataataa tgccagtgtt 63060
   gcttattttt aatacttaaa ttaaatgata taattgatgt taagtgctca tcatagtgcc 63120
   aggcacatgg aacacacact gctattggtt atattgttat tattgatgta ctctttatga 63180
   atacaaaata tccatctttg tcctatgtaa tatttttatg ctttacattt tatttgtctg 63240
   ataattaata tttctgctca tggcagcttt tcccaccttt tcattttcat tttggatcat 63300
   tttattttca gcaagtctca tgggaaccat gtatatttga attctatttt ttctttaaat 63360
   tatgaatctt tgtcctttaa tgggataatt caatccattc acattttttt tttttgcaag 63420
   acacagcatt tggcaagtgt ttacttcttt cttctcccaa tccactttcc tgcctttgaa 63480
   gattttagaa ccagaattta tgaaaaaaaa taaatatatt tgatttccca tgaattgcta 63540
   ttgacacatg cattatttca tgcaattcaa gactttttta aagatgtgac tctcaaactt 63600
   acttcattac tcacttttta tgttttttgg acccatttcc ctcttattga attaattgat 63660
   tgattcatta attcaatcaa caaatattta ttgaaaacat actgtatgca gacactgtgc 63720
   tagggtctga gagaaacgta tttgatgtaa acctggtctg tgtcttcact gaatctacat 63780
   ttagattcac aaaattagag acacaaaaat agatgacaaa ttaaagcaga tataaaggaa 63840
   accaataagg gtctcaagga gagattagtg aacgtgcatg tgagtatcta tttagcaagt 63900
   ttaatgaggg gatgcttttt gtgaaggtga catttatgct ggcagcttaa gaagtggcat 63960
   tagctgtgtg aagagaggaa ggaagaaagg cattccaggc agaaggacta gcatatgcaa 64020
   agggcttgaa aggggaaaga gccttatcct aatgttttca tagaaattat ttttaaatac 64080
   tcaacaatta gcttttgaat taaaggaatg cacacaagtg aagttaatct cttataggca 64140
   aaaccaaatg aaggattgga aaatgcctct gaagagtcag ctggaaggct cttcatccat 64200
   ccatccaacc atccatccat ccatccatcc atccatccat ccatccatcc actcatccat 64260
   ccattcatcc atccatccac ccacccatct acccacccat ccatccatcc attcacccac 64320
   ccactcatcc atctatccat tcatccatcc atccaataag cagtcattga gtgcctactg 64380
   tgtgccagac cctgtgccag gtgttgatca caaataatac aagcacctcc tggagagtag 64440
   agcctagttg tgtacatggg atggagggcc catgataata aagctggtac ttctcatgga 64500
   gtgggatggg ccaggggcat ggggaaggca cacgtgctgt gctctgtaga gctctgagca 64560
   gggagggacg cagtgggaaa gagctgagaa caattcctgc cagatggctt tattctttcc 64620
   cctgaaggaa gtggggactt cgtgctgtga catgatacac aagaacagtg tttcctgtgg 64680
   gtttgcaagg gcattttgaa tataaacact acagctcgct gatgtgtgaa aaaattattt 64740
   tttttgcttt ttcagacttt gtgaagggat aatcagatca attttataag tctggaaaac 64800
   aggtatttgg ggtgaatttt aatgcacaca ttatagtggc tggaacccta accaaaggca 64860
   gctgtcacct gttggtgggt tctcttaggc acgttgtttg ttcatctagc tgtcacctgg 64920
   cagcttatgc cctccagttc ctggaactga ccagttatct gcaactgact aagttctggt 64980
   actggatcct gtgtgatttg actctaacct ccaagaacct tactgtcttt agctgtgaaa 65040
   tgcggagaag actggactac ttgatctctc aacactctat atatctttaa agttaaatca 65100
   tttgttcagt catttgccct ttcttctgcc atttctcttt ccctctctcc catccatcca 65160
   tccatccacc catccaccca cccatccata tatctattta atatttatat tatttatgtt 65220
   atttatttat ttatttattt tgcaatggag tcttgctctg ttgtccaggc tggagtgcaa 65280
   tggcgtgatc tcggctcact gcaacctccg cctcccaggc tcaagcgatt cttctgcctc 65340
   agcttcctga gtagctggga ttacaggcac atgcaccacc atgcccggct aatttttttt 65400
   ttttttttga gacagagtct tgctctgtcg cccaggccag agtgcagtgg tgcgatctcg 65460
   gttcactgca agctccgcct cccaggttca cgccattctc ctgcctcagc ctcccgagta 65520
   gctggaacta caggcgccca ccaccacacc tcgctaattt tttgtatttt tagtagagac 65580
   agggtttcac cttgttagcc aggatggtct tgatctcctg acctccagtg atccgcccat 65640
   ctcggcctcc caaagtgctg ggattatagg cgtgagccac cgcactggac cctgtctaat 65700
   atttaattga gtgcctgttg tgtgctaagt actgtgctgg gttctcatcc agtggccagt 65760
   aaattagaca atcctcccat catagagctt gccatctagt agggagacag acaagcaaat 65820
   aattacacag gtaataatac aattacagtt atgataagta ttacaaatga gaagaatggg 65880
   gtaggtgagc atgtgtatta aggctatcgg tctcattttg agggccagag aactctttct 65940
   tgacaaaatg aagtttaaac aaaaactagg tgaagattgg cagaaaagca tcccaggcag 66000
   agggcacagc cagtgtgaag gccttgggat aggaaggggc atggccacat caagggaggt 66060
   aaaaatacat aacaaaataa taatatgagt aacaatggca actactactg aaggaggacc 66120
   gcctatttgc cagggaccat gcgatcctta actgtgtggt tttgatcaaa tcatctaaac 66180
   tccttgcacc ttggtctatg catctataaa atggggtgat agtgatcgtg tatacttcat 66240
   caggttcttc tgaggcgcca atgaagtaac gtctgggaaa tccttaggtc tatgcctgga 66300
   ccataggaag tgtttaagag acagccgttg tgactgttat ttttatgaat ggtattgggt 66360
   gagaaaaagt tcgtgttgca tctcagggct tgccttggac cctgttgatg atttctatgt 66420
   tgaggtcact cttggagttt ctgcagaagt agaactcttg gaagtctgct attcagcaga 66480
   tgagtgaaac aagtgcttct ccctccgctc ccacagaaag aggggcagag atgccagcca 66540
   cagcttggct ttcaggcagc aggcacattg ggatcagtga ttggcatttg caacattggt 66600
   tggcatcaca gagagcatgg aaggtcaagg taataattat caaggacagt ttaagggaaa 66660
   cagtacattc cattccttat ccattgtaag accagtacat tccctgttct ttaactcttg 66720
   ggaagacatc tcccccagat ctctctgctg aggatcaggg ccatttctct gttaaaaatg 66780
   tcaaacagca acttaccaga ggaaacagaa tggtggaaat gccatctcag cattcaagaa 66840
   gccccagtac caaactttgc atgtatatac gtttgaaaca agctttgtaa attacttttg 66900
   ttcatttgta attacttatc cataagaact ctctctctct ctctctctct ctctctctct 66960
   ctctctctct gtgtgtgtgt gtgtgtgtgt gtggtttgtt gtcacctggg ctggagtgca 67020
   gtgatgcgac catggctcac tatattcaaa atcctgggct catgcgatcc tcccacctca 67080
   gccttctgag gagctgggac cgtaggcacg tgccaccagg cccagcaaat aaaatttatt 67140
   tatttattta tttatttatt tatttattta tttatttatt tatttattta ttgtagagat 67200
   agggccttgc tatgttgctc aggctggtct cgaactcctg gcctcaagtg atcctcctgt 67260
   ctcagcctcc caaagtgcag ggattacagt taggtatgga gagagaagga aataaggaag 67320
   acacagttat tactctttag gacccataat ttaactcaag tgattttgcc cccaggtggt 67380
   atttggcaat gtctggagac attttgtttg tcacaacttg gggtggtggg ggttgctact 67440
   ggaatctagt gggtagaggc cagggatgct actgaacaaa atatacaaga cagcctcaca 67500
   acaaagaatt gaccagtcca cagtgtccac agtgccatgg ttgagaaacc ttgatctaac 67560
   tgcttatgac ataccacttc cataaagccg agagatatca attcttatgc agttatcact 67620
   gtgtacagga aattgtgtgt ttgtacatgt gtgtgcatga atgtgtagat ataaagggat 67680
   aaaaccttta tcttcctttc ctaggtattt ttacctctgt attatcaatt gaaataattt 67740
   gtttttgaat atacaatgca aagtttgaaa tcctacaggt acaacaaggt atacagtgaa 67800
   taaagaatct ccccattcca cttctgtcct caagtcttca gttctctgga ggagtactac 67860
   tgtcattagt ctcctatata ttagtagaga gacattccgc atatgaacct tataccctta 67920
   gtagcacctc acacatattt ttttccttgt tgcctttttt tttcctatgt gatgacgtat 67980
   cttagagata ttttcatatc agcgcatata gagcagcctc attcatttta acagctgcac 68040
   aggttttttt tttttttttt tttttttttt tttttgtggc tgtcccatac ggtcttctgt 68100
   tgatgggcat aggttatttc tagtcttttg cataacaaac agtgctggag tggatctcct 68160
   tgtacctaac tgtggacaca catgtgcaag tacgtctgta atccaaaatc ctgcaagagg 68220
   aatttctagg ttaagatggc atgtgcattt taaatttggc tagagatttc tgcagtgatg 68280
   tctgatcctg tgcaccgtgc attcatcatg tgacccattt ctagtccttc ttgaccgccc 68340
   cctctcctat ggggatcctg agctgatagg gtcacttctg tctctccccc acactggaac 68400
   agctctctct aatctacaag attaattttt tttcctaatc aaaacacaat tgccaaggat 68460
   agtttgaaaa atattaattg tgtctgtcat cctggggctc tgagcacgtg agcgtttggg 68520
   atataatctt tgcaaattgc ctatagttca tacagcacag caagagcctc tctctctgca 68580
   gccagtcctt tatgatcgat catacccacc ccctctccca atgggctgta tcctaagggt 68640
   aagtagacag catgggttct cttgcttaaa aaaaatcgtt aactcaacgg gggcgcctgc 68700
   tgcttgattg ggagtatgtc ggcatttaga gcctgctggc tgcaatctca ctacgcagac 68760
   tggaagttgt ggggtgtttt tatcagcagg aaacagcttt gagagcggaa ggggagtgag 68820
   agtagatgga tagatgaggg aattcgaaga ggaggaagcc gtgggagaaa tgtcttcttt 68880
   taaagacaga caaggcagat ttctccagcc catctctctg gaatgtcaag taggaatgaa 68940
   atgggaagtt gggttaattc taccaggcct gcccagcaac cagcctgttc aggttcccca 69000
   ggagttgcag cctgttgtct gagactgtga ggttctcttc cctgaagtgt cttgtgggaa 69060
   ttggctggtg gcagcatttt ctctgaggtc ccaaaaattc gagtggtcaa gaagacaccc 69120
   tgcaatgatc attccccaac attggaggcc ataaaagtca gtctgggttt gcaaatacac 69180
   actggaagtc tcagagctgg tgcagacatc tcagagctgg tgcagacaga cactgtggct 69240
   gagtgtgtgt gccttgttcg ttatggtttc actgctactg gattggtacc tggtacacag 69300
   taggcactga aaaaatattc actgaaagaa agagaaaact tgatggcagt tgtaggtctg 69360
   ggtctgttgg aatttttacc cccaggaaga gatttgattt taactcaaag ccagttgata 69420
   aacagcctca cccagcagac agagggcagc ttgggggaaa ctggagggta tgtagggtgg 69480
   ggtggggctc ttatctgtat ggacaatgcc atatattttc tgaaacttgg atttttctcc 69540
   ttataaggga gaagatccaa gaagacaatg aaaaaacatt tgcccctgcc tttagaaaaa 69600
   ataacctttt aatttttgga ataatttcag atttttagaa aagttgcaaa acatttacag 69660
   agagttccac tgcatacccc tcacccaaag tttcccccta atgctaacat cttatgcaac 69720
   tgtgatgcct ttgcccctac cattttgagg ttaacaatta agttagtata atacttattc 69780
   tgtaccgagc tctattctat atcattttcc tgcagttgtt tatttgaccc tttcagcatc 69840
   ctttgaggaa taagcactgt tatagcagtc cccattttac agacggggaa gctgaggctc 69900
   agctagactc tagctagtgt tcgaaaccta agttgtctgg ctccagtctc tcacccctca 69960
   ccattattat ctgcacagta atgatggtaa ctgtcatctc ttgatgataa tgcgttcagg 70020
   aactgagctc aaaagttttt ccctccctcc cttcctccct tccttccttc cttccttcct 70080
   tccttccttc cttccttcct tccttcctcc ctccctccct cccttccttc cttccttcct 70140
   ttttttgaga tagggtctca ctctgtcacc cagtctggag tgcagtgtca cgatcgtagt 70200
   tcactgtaac cttgaactac tgggctcaag ccatcctccc acctcaactt cttgagtagc 70260
   tttgactaca ggcataagcc atcatgtcta cctaattttt agttttttct tttctttttt 70320
   tttttttttc ctttttctgt agagacaaag tcttgctatg ttgcatggct ggtctcaaac 70380
   tcctggcctc aggagccctc ctgcttcagc ctccccaagt tctgggatta caggtgtgag 70440
   ctgccacgcc cagcctactt atgttatttc taaaccctcc cctgactcag agctgtggta 70500
   aagagccccg cttttgttgt cctgctcagg acaaaaatac atggccccag gcttttgtga 70560
   gtaggatccg gaagggaact taagtgtgtg tgtgtgtgtg tgtgtgtgtg cacccacgtg 70620
   tgtgtggggg agtgatagag attctcctgg tttctgtcct agttttttct aagcgtatgg 70680
   agacaggtct gctccgtaca tctggggatt ggtattacca catcatttcc cccctaacag 70740
   cagcaaagat tctcaaagct tcatcagagc atgaacaatc agaaatgcct taattttgtt 70800
   agtcacaata aataatactt tgattaatta gcaagcacat gccttgggaa tatgagaaag 70860
   taaatgattt ctaccctaga tctttcagat agctgggagg gaagagagta ttaaaatacg 70920
   cttttaacta tgggttttgg atggagaaga ggagaatgcc ggagagggga aagcagcagt 70980
   ggaattgttc ttggagatag gagaaactga aactctttcc acccagggtc tttgcagaaa 71040
   gtggttccca ctgcctggaa tactttttct tctctttaca tggtgagctc tcactctagt 71100
   ctcaacttaa atgtctcccc ctttgacagg cccttgctaa catcctgttt caagttcctt 71160
   ctggatattg attaggatgg taagcttcat gaggttaagg atctctgttt catccactgc 71220
   tccatcctct ggtgtctgca acgacgctgg cacaccatag gcccttatta aatatgtgtc 71280
   gactaaatga atatgtggtc ttattaaata tgtgtcgact aaatgaatat gtggtctatt 71340
   tcaagtcctc gtttgtgtct gccctagaac ttagcactgt ttgccattag cttgtctatt 71400
   tccttgtgaa tagctagatt ccctcatgag ctataagggc cacgatggca gggggtgtgt 71460
   ctaccttgtt caccaagggt acccaggatg aacctggcct gcagctaagg gggtgggctc 71520
   tagggtcaga ttacttgggc tcaaatccca gatttcctat gttccagctg tgtgatttct 71580
   gctaattgtc aagccactct gtgcctcatt ttccacatag gtgcaagagg ggtgataata 71640
   gcatctatct cttggggttg ttgaaagaat taaatatgtc agtattggct aaaacactta 71700
   gaggaagctg ggcgtggtgg ctcatgtctg taatcccagc actttgggag gctgaggcag 71760
   gtggattgct tgaggccagg agttcaagac cagcctggcc aacatggcga aaccccatct 71820
   ctactaaaaa tacaaaaatt agccagacat ggttgtgggt gcctgtgatt tcagctactt 71880
   gggaggctga ggcagaagaa tcgcttgaac ccgggaagtg aaggttgcag tgagctgaga 71940
   tcgcactact gcactccagc ctgggtgaca gagcgagtct tcatctcaaa agaacaacaa 72000
   caacaacaac aaaaaaccac aacacttaga agagtatcta gcatatagta agtgcttcag 72060
   tgctttatac atgtcagtta ttggtagtac tcaaaatatt tgggatttaa aagttggggg 72120
   atttttacat aaaaggcact tttcctcttt gagtggtaaa atctccccta agaaagaggc 72180
   aaggacagat ggagatgatc tgagaggccc aagagatcct taaaaccagg ctttttaaga 72240
   aatctgatgt tttatttctc cgtattgtca tggaaaaggc cagcttttag catgcctggt 72300
   gtttctggtg ttttcttctc ttcactgttt tctacatttc tttctttgca ttggtccttt 72360
   tttttctttt tttttttttt taataaaaaa aaaatagcac agctcggagt cttgcttgct 72420
   gacttactgc tagagtgtgg gatccattgc ttccatttag agaagcagcc ggccctggaa 72480
   tgtggccgca gggctgccag gctgactctg tgagctgagc tggcgaggaa tgcactttgc 72540
   agcttgcact gagtttggcc ttacgtctgt ggaatttctt ctctcttctt actcattcgt 72600
   gtcgttaaat aaataacaac acaagcagtg catcctcccg gttataatgg caaacagtac 72660
   aatgtgttac aacacacagt agcttgaaga ggatatctct ctcctgtggt agatgcttca 72720
   ttccccagaa cttcactcct cacccccaga ataggttgcc cagttaacag gatgcctggt 72780
   tcagtctgca ttttgggtaa acaaggaaca cctttttagt ataagtatgt cccaaatatt 72840
   gcatagcacc aacttatcct aaaacactat ttgttgttta tgtgctattc aggtttaact 72900
   gggcattctg tagttttatt tgctaaatct gacaactcca gagagtctcc tagaaccagt 72960
   ttcctgtatt ttcttccagt aattttccaa gcatgtgcaa cctatggata tcttggcaga 73020
   cttactcctg gtagaattcc catcacctag aacagtgctt aggacattgt aggtgcttaa 73080
   taaatatggg ttgaatgagt gattgactct gtacccaccc catttttctt cacataagtg 73140
   gaaatatatt atagtgttgt ttcatatgta gcttttcctt ttaataatgt ctcatgggga 73200
   ttgatacata gagctgcctc atactcctct aagggtttcc tagcattccc ttttatgatg 73260
   cataatgtat ttaaccagtc ccctgatgat cgacattggt gctgttgaga gtctttagct 73320
   attataaaca ataaggtagt gagcattcct atatatgttt acctctgtgg gtttgacgac 73380
   gtgcaaggat aggtgtcgca ttggaatcat gaggacggag gatatgtgct ttaaaatgtg 73440
   gattgatgtt gctgaattgc tttctgtaca ggatctgttc acacttccct cagacagctt 73500
   gttccccatc tccttaccaa catagaatat tatcaaactt tttcattttg ccaatctgat 73560
   cagtgaaaaa ctagtatctc gctatcatct taatttacat ctctttgctt atgagaacat 73620
   ttttatgttt gaaagctcta tttctttttc tgtgaactgc ttcttctttt ttgcctattt 73680
   ttttctactt attgattttt aaccggtttt tgtttgtgaa agaacatgtc tgttagcctg 73740
   gcatatgttt tatatataat gtaatttata aaattggtgt ttaactttga aaaagtgatt 73800
   tattatggat tttttaaact ctgtagacat taaaaaattc ctcagtagtc aatttatgag 73860
   tcttttcttc cacatattct acattttgtg tcttgcttag aaaggcctct tactcaatga 73920
   gattatagag gaattctccc atattttctt ttactacttt aacttctttt tttttgcatt 73980
   taaatcttta atccatctgg aatttatttt ggttttagga gggaggtgag gatctagctt 74040
   tattttttac cagatggcca gccagttgtc ccaataccat ttattgaata atccatcgct 74100
   tcccacagaa ttgaaatgct gcctttatca tatattaaat tgtggaggag ggattttata 74160
   ctcattttct gacagcctcc agtgccagct aggcagcaag gattttaggg ggtttcagct 74220
   gactagaggc aaagatcctg acctgaagga taaccgcaat ttattaaacc agaaatactc 74280
   tgtgtaggaa cagtgggttt cctgggtggg gacagggttg gggtggggga aagtgagtgt 74340
   cccgtgctgg gaggaattta gttacagcaa gatttctcaa ccttggcatt attgacattg 74400
   gggggtggaa taggtctttg ttggagcctg tcccatgtag tgtaggatgt ttagcagcat 74460
   ccttggtctc tacccagtat acaccagtat taacttcacc ctgatttgta acaatcaaaa 74520
   atgtctccaa acattgccct gggtggcaaa atcatcttca gttgagaatt actgctttat 74580
   caagcctggg tcgagcagac ctcccatcca tttcttttaa acagaggcag ggtcttgttc 74640
   cgttgcccat gcagtgcatg atgtgatcat agctcactgc agcctcaaat tcctggctca 74700
   agccatcctc ctgcctcagc ctctggggaa gctaggacta taccacatgc ccagctaatt 74760
   ttttagtttt tgtagagaca gggtcttact atgttgcata ggctggtctc aaactgctgg 74820
   ccacaagcaa tcctcttgcc taggcctcct gaagcactgg gattatgggc gtgagccact 74880
   gtgcccagcc tgaacagacc gttgattaga tgcatgtgag gccctttctt gttctgagag 74940
   tctgtcatca gcagtatcaa tggctccaga tgtctgggag gcaggaaggg aggtgcctat 75000
   ctatctcaga gaccagaaca cccagatttc tggtatgagt gtggggtctg tggggaaagg 75060
   gtggaaccat accggtcgac tccgggagga acagaggttc ccacctagcc ctacaggtga 75120
   atcttgcaaa actccctagg acctgcctgg gagcctagcc attccccctg ctaaccacac 75180
   ccccagacaa caaggcctgg ccatggatcc aggaacaatg actctgagaa ccggaggtgg 75240
   cagatgtttt gtgatttaac agcatatcgg tttattgtct tatttttctt aggagtaaaa 75300
   gagtccatgg gtgtattgtt gggggcagca ggaagcacac gtgtctattc ggagatttgg 75360
   ggcagcaggt ggggagagaa agtccatcta gacgagggag gtgtgggagt gagatggaag 75420
   aggagaggga agtgggtgga atccctggga gctgggggaa ggaggaaaac acagagagga 75480
   gctggctgag gtttgagcaa ggcctctgga gattggtgag agctggggcg gcggggattc 75540
   tttatgacag tgctgcagag ggaaaaagga ttgttgctga gttgggtgga ggtaggtagg 75600
   agtgatttga agaaggaggt ccccttggga agttgcatta gtgggtgaat gggggtttta 75660
   taggccaggc agagaatatg gggagggcaa ctccagcaga tgaacagctt ttgcaagaca 75720
   cctgtgtgga gcatgtggca ccacctgagc gtcctcccca ggtgcggctc taacacaggt 75780
   gcaggttatg ttgggagata gagagccaga actcaggagt gccttgggtg ccttgctgta 75840
   agatttgggc tttcccttta tttctttttt tttgagacgg attctcgctc tgtcgcccca 75900
   ggctggagtg cagtggcgcc atctcggctc actgcaagct ccgcctcccg ggttcatgcc 75960
   attctccagc ctcccgagta gctgggacta caggtgccca ccaccgtgcc cagctaattt 76020
   ttttttttgt atttttagta gagacggggt ttcaccgtga tctcgatctc ctgacctcat 76080
   gatccagccg cctcagcctc ccaaagtgct gggattacag gcgtgagcca ccgcgcccag 76140
   cctgggcttt ccctttattt caaaacactc ctcacccact caatatagaa aaccaaaaaa 76200
   tagaggtgag caaaaggaag agagtgaaga acagcgtggt gtcacccacc cagtggccac 76260
   ctccataaac gctgtccagt ttcctctgca cgtccttagc atgctccctg cacacccact 76320
   gggacaccag ctttgtgaag gcaggggttt gttttgtttt gtttacagag ctaggcgaac 76380
   agttggcact cagtaactgt gtaatgaatg gatctgtact gcaaacctga tatcacttgc 76440
   tcttttcact tcgaagttca tgattcgctt ccttgttagt agataccttt ctgggacagg 76500
   tttttaatgg gcacaaatta ttggactggg taaataggcc acatttcacg gaaccaacac 76560
   cctcctgctt tttatcagtt ttcttccaca ttcctctatc acaagtgctt tcatttgttt 76620
   gggctgctgt aaccatgggt ggcttaagtg acggaaatgt attttctcac agttctggag 76680
   ctggaggtct gaggttcttg gcgagaccct cctcttggct tatagataac ctctttctcc 76740
   ttgtattctc acgtggcaga gagcagagag aggaaatcag ctctctcatg tcccttttta 76800
   aaaattcaca tacatttaag ttcaagtgca gttttattac atgggtatat tgcatggtgg 76860
   tgaagtctga gctttttgag taaccatcac ccaaataatg tacattgtat ctactaagta 76920
   atttctcatc cctcaccctc ttcccaccct cccaagtcac tatcattcca cactctatgt 76980
   ccgtgcacac acattataag ctcccacttg taagtgagaa catgcggtat ttggctttct 77040
   gtttctgagt tgtttcagtt aaaatattga cctctagttc cttccacatt gctgcaaaag 77100
   atgcgatttc attcctttta tggctgagta gtattctgtg gtgtatatag accacatttg 77160
   cttcatccat tttgagtccc ttcttataag ggccttgatc ccattcagaa agtctccatg 77220
   atctgatcta atctcatgat ctaatcacct cccaaaggcc ccacctccta aaagcaacac 77280
   actggggggt taggcttcaa ccaattaatc tgtagaggac aaacacgttc agtccatagc 77340
   aggaagcagt gccgccatgg ggaagagcat tgtcgtgaaa ccttttgccc ctcttgttga 77400
   gttttctcct ccagcacacc ctgaacatgc tgacagtgtc accttcctct ttacttgtcc 77460
   ctcttccgct ccaagttgcc tcctggcttc ccagtgtctc tgtgggcttg agaccagagc 77520
   ttgtcataag agatagatga cgtcacttga tggaaagagt caagtgcagt gaccagcaca 77580
   tagaaggcgg ccatcaacat tggtttcatc ctttagagca aagtttcccc aagcgcctgg 77640
   tgtgcactgg tgagatttgg ggacggggtg gtacttaaga cctggcacta attaacacta 77700
   gatcactgtg tgggaaacat agtccctttt ctgttctctt caaatccttc tgatcgggct 77760
   gaggagaaag cctcaggttg gtgctgatgt gcttttttct cccagacccc catcctaaca 77820
   gaaacagagc aggtctcagg ctcgggccgt taggcaatga tgtcaagcca gaatgaacta 77880
   acattatttt gttctcgttg tgttttattt tcatgggaaa cttagagtta tattcagaga 77940
   tattgatttt tttaatccca tttaaaaata atgttttaat tgagataaaa ttcatataat 78000
   gtaaaattca cattttaacc atttaaaatg tacaattcag tggttttaaa catatttata 78060
   atgttgtaag ccatcaccac tatttaattc cagaacattc tcatcacccc cgaaagaaac 78120
   cctgaacccc attagcagtc gctctccatt ttttttctct cttccctgcg gcccctggta 78180
   accaacaaac taattccgtt tctgtagatt tgcctatcct agacattttg tataaataga 78240
   atcatacaat gaatgatcct ttgtgactgg cttcttagaa taatgttttc aaggctcatc 78300
   caaggcatgt atcagtacct cattcctctt aatggttaga tcactttcca ttgggtggat 78360
   ggaccatatt ttgtctattt attcatcaat tagtgagcat ttgagttgtt tccacttttt 78420
   ggctgttata aatagccata aacgtatata tgcttttttt tttttttttt ttttttttga 78480
   gacaaggtca cccaggctgg agcgcagtgg cacaatcaca gctcattgca gccttgaact 78540
   cccaggctca agtgatcatc ttccacctca gcttcccaga tagctgggac tacaggcatg 78600
   tgccactatg cccaactgat ttgtttattt tttgtagaca cagggtctca ctttattgcc 78660
   caggctgttc ttcaactcct gagctcaagt gatccaccca cctcggcctc ccaaagtgct 78720
   gggattacag gcatgcgcta ccatgcctgg tccaattttt tgtataagca tgttttcaat 78780
   tctcttgggt gtcttcctag gagtggaatt gctgggccct ataagagctc tgtgtttaac 78840
   ttttgcaggc catttcctga gtgactttaa gttgaaaagt aagtcagttt aaagaaaact 78900
   attaatcata ttgcagatgg tgtgtggaca tgacaaacgt cataaacgtg gtttgcagtg 78960
   atggaagagt agaaatcctg cttggacatg acctcctcca tcaactgttt cccactcacc 79020
   tttccagcct gtggtgcctc tgttcccagg agcctcagtg cagccacgct ggcctctgaa 79080
   ctgtcttaca tgagatgtcc ttcttcaaag atactgccgt ttgtcttgtc ctgggatatg 79140
   cgcgacccac attttttttt tttttttttt tttttgagat ggagtctcac tgtttgcccg 79200
   ggctggagtg cagtggcagg atctcggctc actgcaacct ccgccttctg ggttcaggtg 79260
   attctcctgc ctcagcctcc cgagtagctg ggatttcagg tgcgcaccac catgcccagc 79320
   taagttttgt atttttagta gagacggagt tttgccatgt tagccacgct agtctcgaac 79380
   tcctgacctc aggtgatcca cccacctccg cctcccaaag cgctgggatt acagacatga 79440
   gccactgtac ctaactgcgc caccccttta taccctccag ggagctggac ttttcagaga 79500
   tcatctttca cttagaatct accgtttaac ccttaattgc atttcccctc atattattat 79560
   actcactaat gattttgttg gagaatcttt tgcctctgct ataagccact tgagggtagc 79620
   atctatattc aagtagtcca ttgttcctct catagtgcag cctcacctga tcaatggtga 79680
   acagctgctg atgaacagaa ccttccctgg aggcccctgt tctactgaat cttggtcagc 79740
   cccgtgtcct gatgcttgcc ctgcttgctg ttactttttg cctgtggttt tactgtttag 79800
   ttaacttctt cttttccttt ctttctttct tctttttttt tttttttttt tttttgagac 79860
   agagtctctc tctgttgccc aggctggagt gcagtggcgt gatctaggct cactgcagtc 79920
   tccgcctcct gggttccagc gattctcctg cctcagcctc cagagtagct gggattgcag 79980
   acgtgtgcca cctcacccgg ctaatttttg tatttttagt atagatgggg tctcaccctg 80040
   ttgggcaggc tggtctcgaa ctcctgacct caggtgatcc accctctttg gcctcccaaa 80100
   gggctgggat tacaggtgtg agccaccgtg cctggccttt atttatttat ttatttattt 80160
   attttttgat atggagtcat gctctgttgc ctaggctgga gtgcagtggt gcaatcaaag 80220
   ctcactgcag cctcgaactc ctgggctcaa gttgtcctcc tgcctcagcc tcccaagtag 80280
   ctgggactac agatatgtgc caccacgccc agctaattgt tgtatttttt atagagactg 80340
   gtcttgaact cctgggctta agcgatctgc ccaccttgac ctctccaaat gctgggattg 80400
   caggcaaatc agattgtttc acagacttca gaatggaggc tgaggttcaa ggaggtggac 80460
   accaggcagc cctgggaggc catcttccat ccagtatgta gagtaaggaa caggaaagca 80520
   ggtgtaccga gaggcaagaa ccatgaatat ggacaaggag aagccgagag aacacaaggg 80580
   gagacccaac tgtcttccag ttcccagttc tcttcctttc cttcagccag accgaccttc 80640
   ctcctattgg gcccaaatca tgtcaaaaaa aaaaaatctg actggccagg cttagtcaag 80700
   gaggggatca tagagtccca gtgccatggg tgagggaggc agtcagtgta gacatcatat 80760
   cactggggag ataagccttc aatggtgccc actattgggt gaaaaggcag acctggagct 80820
   ctgaacttca gcttcccctt cttcccaagg ttgtagaagt gggaaagcca agctagtcat 80880
   ttctgagtgc gggaggagaa taatgtgaca aaaatggtta acatcagact agctagcctt 80940
   gaaagcttga gtctgaacgg tgatatctta ggagagggga aaaaataagc atgaagctgt 81000
   ggctctgctc catttctggg gaaggaagct cttctgactt ttctggctca gtgcaggaag 81060
   ggttgaagaa tctcttaaaa tctcctgagt cctctttgtc cttcatctca gaattcctgt 81120
   gcaggtgatg tttggctgta tgctccacga aaggaatagg gaacttggct ccctccctcc 81180
   agggactgtt gctgctcctc tgagtttcct tagcttaagc tgtgccccct gtgtgccatt 81240
   tgccttggga cagagatggc ccccattttc caatcctgtt gtaggggtgg gaatgctccc 81300
   cagtatgtga aaatatcccc aagctgtgtt ttttttttct tttcttttgg ggcaggctct 81360
   tgtgttgccc aggctggagt gcagtagcac aatcatagct cactgcagcc tcaaattccc 81420
   aggctcaagc aatcttgctt cagcctcccg agttcctaag tagctgagac tacaggcatg 81480
   tgcctccctg cccagctaat tttcatttta ttttattatt tttgggatga ggtcttgtta 81540
   tgttgcccgg gctcatgtca aactcctggc ctcaagtgat ccttctgcct cagcttcctg 81600
   aacagctgaa atatccctag gcttttaagc ctcagttact taatcaacac agtggggcca 81660
   gtacagccca tcattggcat ttgtggattt ggcagtggtg gttccttttc tcattagcta 81720
   cccagaagat ccttagcaca gcatcataag tcactgtctt gaaactccac cctgaattgg 81780
   atgcttggtg agcatctggg agaggcagaa tggtgttgga accaagattg tgggctctgg 81840
   agtgagattt ctctgtaagt ctggacacca ccccttggtt gggtgatctt gggcaaatta 81900
   attaatgtct ctatgcctca ttttccttgc ccataagtgc ggatgacata gcacccacct 81960
   cacaaggctg ttgggaggtt taaatgagtt aagacctgca cagcccttag aacagggagc 82020
   tttcaatatg tgttggccac agctcttgtg cccaatcgat ttcactcatt tgggcaatgc 82080
   ctgggttcta gatgaagggg gaggaggctg cctgtagtgt cttcttcatg ggccagtcca 82140
   gctctgcagt ggactgtact ggtgatgtcc accgtgcctc agcttttaag catatattct 82200
   ccttgaaaac tatttaagca gaacacactg gggtcaagcc agtccaagcc agatgtttgg 82260
   ggtcctgggt gcttctgttt tctccatgtc tgtttacctt taacaagatg gatgggggct 82320
   gtgaagcccc tctgggcagc cttttctggc tgtgacatgc accagaaacc cacttttggc 82380
   ctgtttgcac tgtgttagaa aacagcaggt gcagttggca gcctctcccc taccccttct 82440
   cccgttagcc cctgatctct ggggactcct tagactaatg cagttttaat gaatacacat 82500
   tactgtttac atgactgaaa tgagaggctg gctgcttatt ttatgctgag gtttacagcc 82560
   ctgcctttta aaaccctctc tcccttgctc tgcttctggc catggcgata ttttctggcg 82620
   gcccctatgc tgtccctcca aataaggaat tcaccatgaa gtcctgcagc agtgtcagag 82680
   ccattgcctt gccccggaag gagtgaactg tgtagaacaa aagcagaggc tgggagctgt 82740
   agttcacgcc tgtaacccca gcacttcggg aggcagaggc agaaggattg cttgagccca 82800
   agaggtctag accagccctg acaacacagt gggaccccag tctctataaa aaatacaaaa 82860
   tacaaaaata caaaaattga tgacctgcac ctgtagtccc agctactagt ggacactgaa 82920
   gtaggatgat tgcatgagcc caggaggtgg aggctgcagt gagctttgat tgcaccactg 82980
   cattccagcc tgggcaagag tgagactctc tatcaaaaaa aaaaaaaaaa aaaaaaagca 83040
   gaaagatgga ttctttattt ttttaattta aaaatacttt ttacaattct taaatgcttg 83100
   cttatgatag aacaattaga aaatgaaggt atacaaagag aagaaaataa aaatcacctc 83160
   taattccact actcagaaaa aactcgatcg ttaacattcc tgtttatgtt ttagcatatt 83220
   ttaatcaatc tacatttata tgtattttaa attaaaatta gaaaccaaac ttaatacatg 83280
   ttattcagaa atctgctttt taccctacct cacaattaat atatgatgaa tgtctttccg 83340
   agttaagaaa tatagatctg cagtatcttt tctttaattt taattttaat tttttagaga 83400
   caggatcttg ctcagttgcc caggctggag tgcagtgggg tgatcacagc tcactgcagc 83460
   ctcaatctcc tgggctcaag cgattctccc acctcagcct gccgagtagc tgggactaca 83520
   ggtgcatgcc accacatgtg gctaattttt tttttttttt tttttttgag acgaggtctt 83580
   actatgtagc ccaggctggt cttgaactcc tggccacaaa caagcagttc tcccacctca 83640
   gccttccaaa gtgctgggat tacaggcttg agcaccatgc tcgatattgc agtatctttt 83700
   tttatgcatc attgtatcct attctataat tgtttctatg gtctcatttg tagaaaggtt 83760
   aacattaaat ttcgaatagt tatatgtact cactggttca aaattaaaaa ggcacaagag 83820
   ctttaatact tttattgaaa gatttctctc ctatccctag cccttaggcc actctgttca 83880
   cctccttttt ggcacccaat attcaactaa taataccaat ttattgtgta tacttctgga 83940
   aatagttcat aatgtacatg cacatcatat acatttattt ttctcttcct caaacacgta 84000
   cattcaaatg ttagcttacc ttgctttctt cacttcacaa tatcctgtat cttggtgttt 84060
   gttccatgtc agtgcatata gaacatcctc attctttttt ttgaaacaga gtctggcttt 84120
   gtcacccagg ctggagtgca gtggtgtgat cttggctcac tgcagcctct gcctcctggg 84180
   ctcaagcaat cctcctacct tagcctccta agtagctggg actacaggca cacaccacca 84240
   tgactggctg atttttgtat tttttttttt cattgttgtt ggtagagaca gggtttgtcc 84300
   ctgttgccca ggctggtctt aaactcctgg gctcaagtga tagggtcacc tgggcctccc 84360
   aaagtgttgg ggttacaaga gtgagccact gcacccaatg tctcattcta tttttattta 84420
   tttatttttt gagacagagt ctcgctctgt tgcccagacc ggagtgcagt ggtcttgggt 84480
   cactgcaacc tccactttcc aggttcatgt gattcttcca cctcagtttc ctgactagct 84540
   gggattacag gtgtgtgcta ccacacccgg ctaatttttg tgtttttagt agagataggg 84600
   ttttgccatg ttgcccgagc tggtctcaaa ctcctgactt cagatgatcc acctgcctcc 84660
   caaagggttg ggattactgg cgtgagccac cacgcccggc cacctcattc tgttttatga 84720
   ttgcatcgtg gtccatagta tgggcatccc tggggtgttt ccaaaattgg gatatattga 84780
   ctcagaaacc cttcctgtgg ttctgtccaa gctccttgtt catatgacct ggcctggggt 84840
   gactggagga aggatgtcca ttcggagatt aggagggact gcagctctcg attatgaatg 84900
   ggattggcct ccacaggcct gagtgacaag gacaaagcag agctggtgag ccccatgcca 84960
   ggtggcggtc actggtggaa ggccatttgt gacttctctg cagttctcca gatggaccac 85020
   atttaaaaca gaggcactgc cattcatttc tacttggttg aggaaaggtt tcatcgcagg 85080
   cccagagaga ggatcagatg ttttttgcag tttctggcaa gacattagtg ggtatcagag 85140
   ggtaagtgat ctcgtaaagc tgactcaaag caagagaaaa tttcgtgtct gtcttagaaa 85200
   cacatttgcc tccaaaggga aggcagggtg tggaggacta atcaacgcat ttcacatttg 85260
   gtttctttgt ctttcttggt gatcttacgt gactagccct gaatattttg catatgtcaa 85320
   ggaaacattt caaattacta agatgagagg aaaatatggg gaatgaataa aatggggttt 85380
   atcagcaaga acagcaaaag acttcttaga gccggctggg gattgttttg tgaatctcta 85440
   aacaagaggg gcagtttgac ttggggatgg gggatgtttt gacacttgga gggatgggag 85500
   ggagccaaca aagatgtaaa gcaaaactgg gatggtggtg atggagaagc agtgggggtt 85560
   tctatgggaa gacataggaa ataatggtct gtccttcaca gagtaggtga ggtctactag 85620
   aagatactta tcttttccct aaatttcagt caacttcaca tgattaagtt tccattatta 85680
   aagacttgtt ctttgtgtta cctttttatt attattatta ttattatttt tttttttttg 85740
   aaatagagtt tcactctgtc acccaagttg gagtgaattg gcgtgatctt ggctcactgc 85800
   aacctccacc tcctgggctc aagagattct cgtgcctcag ccacccgagt agctgggact 85860
   acaggcacac accaccaggc ctaactaata tttgtatttt tagtagaggt ggggtcttgc 85920
   catgttggcc aggctggtct cgaactcctg ggctcaagta atccacccgc ctcggcctcc 85980
   caaagtgctg ggattacagg catgaggcac catgccaggc cttttgtgtt acttttgtaa 86040
   gagatgaaat gaaactaata tctaatcaaa tctaagcatc aagcaattaa aaatattttc 86100
   ttaaataact attgagttaa ataggactca aaagtacaat tgcagagagg ctataaatga 86160
   aaaaaaatag agtgctatat aaaacaatgg ataagatcag ccaatgctgt gcttgcagat 86220
   aagttcatag ctttaatact tttatgatta aggattgaga tgacttgctt tgagcataat 86280
   ctggctattc acttattcta aggaacttgt catggctcaa acatgtttct agtatcctct 86340
   ttagaaattc cgtttagata cagttttctt tattaaacct tctaaggtga ctatttcaaa 86400
   gatgaaacat tcattttgtt gtttaaatta acacgtattt tttaaagggt tatgtaaatg 86460
   tgttatccat caagtatatc tttgagcctt catcacagtg tgatgtaagg gatataagtt 86520
   gttaaggatc cagcagtaga gaaaatttct gcacaaaagt aactatagct ctattacttt 86580
   tggaatattc cctaggatac atccaaaacc aaatcaaatt atactactta taaaacacat 86640
   ttggaaagat actatgtgaa tatgctttga atactgaggc ttatgttaga acagagtcat 86700
   ggacgaattt gcaaaaaaaa aaaaacctcc aaaatttcct ggagtctgac aaaaataccg 86760
   gatagggttc ctgaatatat tttcatgatc tttgctatat aaccagatat ctctatattt 86820
   tgaaatggat catattttat tagtttttct tagtacacaa gcaacatgct atcattgcag 86880
   atgccagaaa gtaggatcac attgttgtgc tatagatctc cagaacttct ttatcttgca 86940
   aaactcaaaa actctatcat ttctcaaact caaaactcaa caattctcca ttccctcttc 87000
   ctccacccca tggccaccac cattctactt tgtctctatg aatttgaata ctataggtat 87060
   ctcatataag tggaatcatc cagtatttgt catcttaaga tttctttctt tgtgtaacgt 87120
   tatcaaggtt gatccacatt gttgcgtgtg tcagaagttc ccttctcttt taaaggctga 87180
   atatttcatt gctttataga ctgcactttt ttatccattc atttgtcaat ggacagttgg 87240
   attgctctca tcttttggct attgtgaata atgctgctat gaacaggagt gtacaaatat 87300
   ctcttcaagg ccctgttttc aattcttttg gctatgaacc cagaagtgga attgctagat 87360
   taaatggtaa ttctattttt aatttgttga gaaccaccat acttgttttc atagtggctg 87420
   caccattttg cattcccatc aacagtgcac aaggattttt ccacatcctc accagcacat 87480
   gttattttct ttttcttttt ttgatagtag ccatttaatg ggtatgaggt ggtatctcat 87540
   tgtggttttt gatttgcatt ttgctaatga gtgatgctga gcatcttttc atgtgcttat 87600
   tggccattta tgtgtcttct ttggagaaat tctattcaag ttgacttcta tgattttgca 87660
   ttatcacatc tgtattgatt caagcaagcc aactagaata aattctggca tttaaaccga 87720
   ttttgtggtt tttctgcaaa taaattctgc ccccaaataa cctccaactt tctggaagca 87780
   gtcagcagga gtacagttct gaagataact ttctttaaaa aaggaaattc ataaaatatc 87840
   atgcatcttc cttttttgac actaatggaa caatttaatg taatttcaga gggaagcaga 87900
   gcccctggaa aggctggtgt gataagggaa ggttacccag ctttcctgtc aggcggtgtg 87960
   tgggagcaga gagtggcatt ctctgcatac tcttggggag aagagtgggt gagacaggct 88020
   gctcagggct ggggcagagc ccaggggaag gggatggaag gggaagaaca gcccttcaag 88080
   agtcctgcag aaattggtgg aagttattta acagaagtgt tcggctccac ccagcacatt 88140
   ctgttgcctt ctacatacag agtgtgttag tctgttctca tgctgctaat aaagacacac 88200
   ctgagaccag gtaatttata aaggaaagag gtttaatgga ctcacagttc cacatggctg 88260
   gggaggcctc acgatcatgg cagaaggcaa atgaggggca aagtcacgtc ttacatggtg 88320
   gcaggcagga gacagcatgt gcaggggaac tcccatttat gaaaccgtca gatcacctga 88380
   gatttattca ctaccatgag aacagtatgg gggaaaccac ccccatgatt cagttatttc 88440
   cacctagccc cacccttgac atgcagggat tattacaatt caaagtgaga tttgggtggg 88500
   gccacagcca aaccatatca cagggtaatg aagaatgtgt gcccaagtag tagagggctt 88560
   aagaaaacca ctcttgggct ctgagtctct ctaggtctca gtttcctcat ctttcaaatg 88620
   gcaatattaa taagacccac ctcataggga ttgtgtgggg tttaaatgag aaaagacagg 88680
   taaggtgctg gtaccttata agtgattaag ttgccattaa ggtattgggg acttagtgct 88740
   tgtccttgaa gagctttcgg tctcgtgagg agacagcctg atcgttataa actattatag 88800
   aaaatgggaa gaaataaggg ttgaggtgtg ataaagatgt gctaggccag gcaagatggc 88860
   tcacgtctgt aatcccagca ctttgggatt acaggtgggc agatcacttg aggccaggag 88920
   ttcaagatca gcctgaccaa catggcaaaa ccctgtttct actaaaaata caaaagttag 88980
   ctgggtgtgg cggcacccat ctgtcatccc agctacttgg gaggctgagg caggagaatt 89040
   gcttgaatct gagaggcaga ggttgcagtg agccaaaatt gcacaactgt actccagcaa 89100
   gactctgtca aaaaaagaaa aaataaataa aaaaagatat gctataccct catatcacaa 89160
   taatgccagg aaatagatca ttaattctga accctaattc ttgggtgggc atcaaggcag 89220
   gcctcacaag aagaaggcat ttgagttgaa tcttataggc tcagctgggt tccaacagca 89280
   aagacttagg gaaagggcag agcaggcagg ggaacagtaa gagcaaaggc ttgaaggcat 89340
   ggaagttcat gggtaattac tgactgagat gtttggtgtt atggtgcata gaacactatc 89400
   ggatgataat gaaaacattg aattccagca tgtttagaag gacattggct gataaaaatg 89460
   acgtcttaat agttatctgt ggacaaattt gtaaatgtaa aagagttgta gactcaaatc 89520
   ctacagaaat taggcaggag acctaaatga gtgaagtggg tgggtataaa acagaggtgg 89580
   tggcaggaac ccatgttgaa ttgaatagtg ggtgttttgt ttaaaggggc accagtagta 89640
   atttttcctc tgttggaata tgatcctagg gctgccagct cttctacttt ttcaagagaa 89700
   gccagaaatc tggatttcat gcaaaatttc ttgtttttct ttttattaaa gacatggtat 89760
   ggaccaaaaa aagatgtctc taggctagat ctctgcaacc tctcatctat actctatttg 89820
   catttttaaa gactatatgc aaatgttatt tagcagagct acctagtttt tttgtgtgat 89880
   gatagcatgt gaaagagcta tgatacatct tggaaatgtc aactttgaaa tatacagtga 89940
   ctgtgttggt cactacagaa tgatctctgc agaaacttca gttacatttt ctctaaatga 90000
   cacctttgct ttgacaacac attttcaaaa agaaaaatag caaactaccc ttcctccagg 90060
   gccttagcca cagtagctcc tgccagcata acacaatgct gcaccttact gagggatcag 90120
   gaaatgtggt ttgatttata attatggctt catgggtaga atggtggcca atgtccacat 90180
   gagccacact cagtggctaa gatccagagg gcatttaatt taggtagcca gtgttacaga 90240
   catagtttag atttttgcag ccttgaccaa tcttaataga tctgtctgtt ctttagagtt 90300
   accttttttt cctgcctcct agctacctac accttttaat gctaaatgta gttagctagt 90360
   gaatgctgtt atttgaatct taaaatttca ggttgggaca gaaccttaaa attcatgtat 90420
   ttcaaccacc ctttgtatga ggcaggaatc ttggttatga caggtgacag aaaattcaaa 90480
   ctagcttaat tcagaaggga atttattgcc tttataaaac caaaaagtcc aagggtagct 90540
   gccttcaggt attgtctgat ccaggggttt gttatgtttc caggacatgg tttcctttat 90600
   tccatctctg cttatgtatt ggctcccttc tcagatggaa gagactcccc tcatcaaggt 90660
   gagatggcta tggaagctct actcacattt cctctaggtt caagtccatg tagagaagag 90720
   cacctctttc tcttgttata tctcacaggc tctaaattag ctactagggc cagtgaaaaa 90780
   gcaatgtgct gattgaccac ccttatctac tcttggagtt ccggatggaa ttaacactac 90840
   ctgaattatg tggaccaaga gtgggaaagg aatagttctc cagaaggata tttggtattg 90900
   ttattaaaat aaggttaatt gaatactcac tgaaccatct aatgcttgaa ttcccttcat 90960
   agcacgccat tggtgactgt ccaatcttca gttactaaca tgctaccccc aaaatggtgt 91020
   gtttgtgcag ctctaataat gacagggttt ctcctaatac tgagatgata ttggcatctc 91080
   catagttcca gacattattt ctggtcccaa ccataatcaa cctgttttga cacgggtgaa 91140
   catgtaaggg aagggattgc tcattgtggt cacagaagta acctacccaa tagaagcaca 91200
   ccaacttgaa gcatgcttcc atgatcactg agggaggaaa atgattggca agtctcgtat 91260
   tggtccttga agcgctccct ataaacactt gtcactgcca agcgtgtatc actggccaaa 91320
   gcaagtcaca ttgtgatatg attagacttt gtgtccccac ccaaatctca tcttgaattg 91380
   taatccccat aatccccatg tgtctaggga gagaactggt gggaggtgaa tggatcatag 91440
   gggcagtttc tcccatgctg ttctcatgat actgagtgag ttctcacaag agctgatggt 91500
   tttataaggg gctcctcccc cttcactcct cactcttctc tctcccgtag ccatgtgaga 91560
   aggtccaagc ttgcttcccc ttcgccttct gtcataattg taagtttcct gaggcctccc 91620
   caaccatgcg gaactgtgag tcatttaaat ctctttcctc tagaaattac ccagtctcgg 91680
   gtagtatcaa gatagcagtg tgagaaccga ctaatacgca tggccatgcc taacttcaaa 91740
   gcggctgggc aagtatcata ttgctgtgta cttgggagga ggggagaatc agaatatttg 91800
   atctggcgac caccacacaa ctgaacttct ctatttctgt gttcttcttg ttaaccatct 91860
   agtttctgtt tcctcttgtg acacatatat taattaattt ttcaataccc aaacacaagc 91920
   ctctaatatt tatctttgtt tagttacttc ctaataaact aagatatctc tacatcctga 91980
   tcctaaaatt caaaggctcc acgtatacca tggtgactag ttaattccaa tatattgcat 92040
   agttgaaaat tgccaagaga gtagatttta agtgttctca ctacgaaaaa atgcatatgt 92100
   taaatagctt gatttagcca tttcactatg tatacatgta ggaaaacatc atgttgtaca 92160
   ctacagatgt atacaatttt tacttgtcaa ttataaacaa caacaacaaa actgcaaagt 92220
   ctccttagac ccacccaatc acatgttgtc tgctcattta atgagtaaat gctatatagg 92280
   ttcattctgg acctgggtaa aatgatttac ggactgggac caagtgggag gaccctattt 92340
   caattcatta ggtaaggtct tccaggtcaa tatttgacca ttgattggca catctggggt 92400
   atagttattt aactgccttc tagtttacct caatatgacc cctcagtgga gggaaaatgg 92460
   agcacaatga ctagaagaat gttgagagat gaggaaagag agaatgggat ggaaagaaca 92520
   taaaatagga aagcattgac attgtcactt cttggctatt tcttcctgta aaattttgcc 92580
   tggagtgccc tacaatgggg tccccatcct ctgccctgat aacatcttag tggatcactc 92640
   ctctctccct tttcattctg caggacttgc ccattggggc tctttcagac ctgactggtc 92700
   attgggtatc tgtggccatg ataatcactc tcttcctgga cgtgaccctc ctctcgttaa 92760
   ccaggatgcc tgtgttctta aggttggccg tttccaagga cagagagctt gcatcccatt 92820
   tctaggggac tggggatcca aggactccat tcttgacatt gtcttgtcag ccttgaagtg 92880
   ccgaaaccca gcatccctgt ctgaggtcat cctgtctctc ttccaaacat gaggacttct 92940
   taaatttttt aaaaaaagtt tttacttgta taaattgaga aggcacaagt gcaattttgt 93000
   tacatggata tgttatttag tggtagaatc tgtgcttttg gtatgtccat cacccagata 93060
   gggtaccctg tactcattaa gtgatttctt atccttcact cccccaccca acccttccaa 93120
   gaaggatgac tattactcaa cagtcattca gtagccattg gagaatgact attattccat 93180
   actctgtgtc tatgtgtatt atatagttcc catttataac taagaatatg cattatttga 93240
   ctttctcttt ctgagttttt tttttttaag ataatggctt tgggttccat cctgttgctg 93300
   caaaagacat gatttcagac tgggcatggt ggctcatgcc tgtcatatta gcacactggg 93360
   aggctgacga aggaagatca cttgagccca ggagtttgaa accagcctga gcaacttggt 93420
   gagactccgg ctctataaaa attaaattag ccaggcatcg tgttgtgtac ctgtggtccc 93480
   agtttcttga gaggctgagg caggaggatc tcttgagccc aggagttcaa ggctgcagtg 93540
   agctatgatc acacaactgc cccccagcct gggtgacaga gtgagaccct gttttagtca 93600
   ggacttcaag accagccttg ccaacatggt gaagccccat ctctgcaaaa atacaaaaat 93660
   tagctgggca tgatggcggg tgcctgtagt cccagctact caggaggccg aggtggaaga 93720
   atcacttgaa cccgggaggt ggaggttgca gtgagccgag attgccccac tgcactccag 93780
   cctgggcgac agagtgagac tgtttcaaaa aaaataaata ataaataaat aaataaaaat 93840
   aaaatcaaag gtcttactct tttttatggc tgagtagtat tctattgtgt gtatgtatat 93900
   atagatatac atacatatac cacacacata cgtgtatata tgtatatata tggtggtatg 93960
   tgtgtctata tacacacaat gatatgtata ttaatatata tgtatatata aatatatata 94020
   cattttcttt atccagtcat ccattgattg atgcttaagt tgattccata tctttgctat 94080
   tgtactgtga taaacgtatg agtgcaggca tcttttatat aataatttct tctttggata 94140
   gctgcccaaa attcaggtaa aagatctcag tctacaaggc agctcccaag cttcaaaaaa 94200
   ttgtcctgcc agatttttaa aagcaagttt tctcaaaagc ttcgatgtat acattggtcg 94260
   atttatttat ccattaaagt atttattgaa tacttcttac tgcgtatggt ggtggtggtg 94320
   ctgaggtgtc tcacagtgaa cacaacaggc gtagcctctg ctattaccaa aattattgtc 94380
   tggtaaagaa tgcagatggc taaccaggca ggtgcctgta cggtgagata tgtgctgtga 94440
   tggctgtaag cgcatggtgt gagcgtagag ttgggtgctt aattgaaggc atcaggaagg 94500
   cttccaggac taaatgatat caaagtccca ccttaaggat gacaggttta actaagtaaa 94560
   ggaggatgtg gaaagaaggg ggagagtgct ccaggcagag gaaatagtat atacaaaagc 94620
   acaagaggat tgaagaacca aaatcacttt ggcatggctg gaatgaaggg catgtttgtg 94680
   tattttcagg gtaggggatg tgtttaggga caagctcaga gaaaaggaag taaggaaccc 94740
   tgtcataaag ggctcatctg tcctatgaaa gagtgtgtac attttattct gagagtacag 94800
   agccttttct tttttctaag cattacatga ttagatttgt cttttagaaa ccactctggc 94860
   tgccgttaaa aatgaatttc acagtgacaa ggctggagga aggatcttgg gttaggaggc 94920
   ggtggtagta atctaggcag gacttgagga cgcctgagtg gtggtgagga aggagagagg 94980
   tggatggaag attcaagaga tatttagggg gtgttattga gagaacttag tgattgattt 95040
   ggcgtgggag gaatgagaaa gtcagggatg gctccctggt ttctggcttg ggtactaggt 95100
   gcgctggtgg tgcttttgac agagatagaa ccatgggagg aggaacatgt tttgagggaa 95160
   ggctgtctcc cttttagaca cctagttggt gtccaaatgg cacagaccag ttggcgtgtg 95220
   gatatcttgg ggcttagggc gtggatctgg gctcagggtg gagatctgag ccttagctgc 95280
   acatcagtag tcattgcagt catggagtgt gctggggttt tgccaagagc tcatgtagat 95340
   tcaggcgaga agagaggagg ggcccaggct gggaatactg gctttggaaa tactcttgaa 95400
   atatatagaa atgaaagaat gtatagggct gtttccacag acgtttattt tttattttta 95460
   tttttttgag atggtgtttc actcttgttg cccaggctgg agtgcaatgg catgatctca 95520
   gctcactgca acctccacct cccaggttca agcaattctc ctgcctcagc ctactgagta 95580
   gctgggatta tagatgccca cgaccatgcc cggctaattt ttttgtattt ttagtagaga 95640
   cacggtttca ccatgttggc caggctggtc tcgaactcct gacctcaggt gatccaccca 95700
   ccttggcctc ccaaagtgct gggattacag gcatgagcca ccgcgcccgg cccacagaca 95760
   tttactgagc aagagcttgt gcaaggcact ctggtatgca tgcagtcggg gaccctctga 95820
   tgaatggaac tcaggccttg tataaggaag tgacctccat cagagggtgg agacgctgtc 95880
   agtgaacatc catcacagac aagtgactga gctaggagct ggggtaaggt agaaagagga 95940
   ggacagtggg cgatatgagc tgggaaatgc acctctgttg ggggactggg gagggcttcc 96000
   tggaggaggt gattttggta tctagtggtt ttgttttgtt ttgtttgttt tttttgtttt 96060
   gagatggggc cttgctcttg ttgcccaggc tggagtgcaa tggcatgatc tcggctcact 96120
   gcaacctccg cctcccaggt tcaagcgatt ctcctgcctc agcctcccaa gtagctagga 96180
   ttacaggcgc ccacaaccat acctggctaa tttttgtatt tttagtagag acggggtttt 96240
   gccatgttgg ccaggctgtt cccgacctcc tgacctcgtg atccgcccgc ctcggcatcc 96300
   caaagtgttg ggattacagg cgtgagccac tgcgccaggc ctgtatccag tgtttaagga 96360
   gatgttttgt cagataaagc tggagttttg taatggagtt ttggagatgg ggaagggcag 96420
   actggcttaa gagggtgctg cataagagat ggctcagagg agagaaagtg tacgatgttt 96480
   tcaggacaaa ttagtccttc accatggcag gaatctaggc ggccgaagga catttgtggg 96540
   aaattgtagc agaaaatacg aggaggactt tgatgccagg ccacagagtt tgcagtcctt 96600
   tttggagact caggggagtt attgaacaat tagaaactaa aggctgatgt gacctccaca 96660
   ggatactggc taaacgcatg aactcttgaa ccagatggac ctggatttaa gtctggattc 96720
   tgccatttcc ttgctgtgtg gtcttaggca gattattaac ctctctgagc ctcagttttc 96780
   tcatctgtca aataggagtg aaatacctca tagagggttg tgaagagtga agtaaactaa 96840
   tgtggagaag gcattctgca gtgtctggaa ttcagcgacc cctcagtaca tgttaacaca 96900
   ctaatacttt ctacgtaatc atacctgtaa caacacagag acgatggtat tatctttaca 96960
   gcttgttcgc ttctgtatcc tgagcatcta ggacagtgcc tggcatgtaa tggatgctgg 97020
   agaaatattt cttgggcaag aaatgaattg caccagtttc tttttgtaag ggatctattt 97080
   ttttcttgtc tggaaaaata ctttgataaa accttaggga aaagatgaaa ttccctttct 97140
   tctggagcct agatttcaaa gaggagaggt tgagtgctct gcaaagtaga aggagaagat 97200
   tttgggcagt gaggaggaga gaattgatga gatggtgggg cacagcgtac tatgaaatgt 97260
   gactcttccc ccgggacccc cgaaaaaatc ttctgtaaca ttttatatgt gtaagacatc 97320
   tctctaccat ctgctatggt cgaaatggga tttgtagaga gctcatgtga ttctcaaacc 97380
   tcctagtaac tctgcttaca tggagagaca gctgtctcca agccagctgt tgtcttttgg 97440
   aaagctttaa tctgggggaa gataacacct tgagaaaaga tgtcaggatt ggaggcagat 97500
   gttgacggag ccagtttcta atgccccaag gcagccacac tttgtatgga ttgtatggat 97560
   gggcaggtgc acaaggtgag ttattttttt ccctacccag aaatttatgg aagcctggca 97620
   tggtggctca cccctataat cccagcactt tgggaggcca aggtgggcag atcacttgag 97680
   cccaggagtt cgaaaccagc ctgggcaaca tggtgaaacc ctatctctac taaaaataca 97740
   aaaattagcc tggtatgggg gtatgcacct gtagtctcag ctactcagga ggctgaggtg 97800
   ggagaactac ttggacccag gaggtcgagg cttcagtgaa ccatgttcat gccaccacac 97860
   ttcagcctgg gtgagagagt gagaacctgt ctaaaaaaaa atatgaagtt gctcttttga 97920
   agagcattct aatgacttat ctggagattc taggaaagaa attaaattca gctttcaatg 97980
   tctgtctttc tagatttctt ccttctaacg ttattgttgt tatgtcctca aacaaaaaca 98040
   tttagtactt attatgagct tcatagggtt gttgtggatt aatacatgta aagctcttag 98100
   tgcaatacac gacacataga taacatttaa ttttattatc gttactgttg ggtagcactc 98160
   agttcatgaa attcccacac acagtcaagt ggtaaatgct attactttgt aaatatcatc 98220
   gatgaggaac ctgaagctca cagaggttga tgacttgcct aggatcgctc agaaagcagg 98280
   cagcagagct gattgccatg gtaaccattt ccaagcttca gtgcccagag atggacccag 98340
   tctggcatgg gcttatcccc tggagcactg tgagtgtctt gacagataag taaaggaagt 98400
   ctttaatttc tgaaaaatgc ccagctgtga ttggcattgt taggggtcag tgaagacacg 98460
   ctcaaagacc ttactgtcca gcttttttat ctttcacctt taatgccgct gttagcccca 98520
   ggagctaacc aaaggggagt tttctttatc agttccaaga gggatgccat ggttgtccca 98580
   agattctgga gctaaaggtg ggaccaggac cagaattcag tttctgtgtt tccaaattca 98640
   gttctattct cctctcagta gaccaggagg cctccagaca agacagatgg cagacagcta 98700
   cagtagttta aaatagcctg agctcacttc tgaggtagac atgtgcgtgg agtgagctgg 98760
   aagacaggga cccctgtgtg ggaaagattt tggcaaagag aagcccagtg gggagttctg 98820
   atgctaggct gagtgtcatc aggttggggt gagggtgtga gaagagacct gagcaggtgc 98880
   caacctgagg ctgagtgggg aggagagaag cgagaggaca ggtgccaggc aagatgcggc 98940
   actgccaatc agtagaagct tcctccctcc tttccagctg tgctaccgag acggggaggt 99000
   caggtggtcg ttctgggagg gctggtgcct tttacttccc ggccttctgg cctgctgggt 99060
   cttggagtcc agtgcaaaat aatgaagttc ctataaagac agtttgtata gatcctttta 99120
   ggatgacttg aaagcctatg catttcctta acaatgatca attcttggct aatcctcttt 99180
   catccatatc cgcacccaat gcttttcact ccatttccat actggatgtt ttgaaataaa 99240
   tcccagacac catataattt cagtaattat caatctcaat atcacagaaa gtcaaataga 99300
   cattatatat cttctaatat gatgcaaata gaagtggcca atgctatcta tgcaatatta 99360
   ttactaaaga tctaaccaaa tttacaggaa atacagagaa tatgtaggaa attctacata 99420
   gaaataactt gatttcttca cgaaaccaat ggcatggaag tgtgaaaaaa atagaattaa 99480
   aagatatagc aatctaagat agtgtgtgga cctcatttgg aaataaattc aaacaaatca 99540
   tccataaaga aaacattgtg agataatcaa ggagaaggaa acattaactg aaatataaaa 99600
   tgataataaa gaattgttat tgcttttact gggtgggata atggaattgt ggttgctttt 99660
   ttgttgttct tatctgtgga gatatgctga agaatttatg ggtaaaataa aactaagcga 99720
   tgctaggtgg ggaggaacct atgcatttcc tattttttgg caaaaaggcc ccaaggtgta 99780
   gtccctagca cctatgttcg aacaatgtga cccagcattc ctgctgatcc ctgactgaac 99840
   caggtatggc tccacttgat cctctctctg gggactggga gttagcattc agaaatgcca 99900
   gctagcctcc atcaacagca gactggaagg atcaggcatc tcaggaatca tgcaatgtgc 99960
   atcaaaaaat agagaaagct tatcttcaga gagacagaca agtgaagctg gctcagaggt 100020
   catgtggacc agagggatgg gggaagcagt tgccttggtt cctgacaatc ctgcattgaa 100080
   gtcaaactgc tgttcctttc cctggtttct gtttccttgt aacagactta ttcctttgct 100140
   tgaactgggg gcttttagtg gggttctgtt gcttgcaaat cttaagtact ctgagaaaca 100200
   gctaaggaga tgaagatgga aaactttgtc atgaagggaa tgggttactg gaatctttct 100260
   ggagggagaa tttcagaatt acgaggatgc agttctgatt gaagggggtc ttgtgggtat 100320
   ctgggatttg gaagtgagaa tgaatgctgt agcagatgct attagctacc catccaatgg 100380
   cgattccttc tttgtttcta gttttgttcg gtggcaatgt gcccagggga cagtgcatgc 100440
   atgacccatg attatttccc tttcagtgat tggtctggaa gtgggcactg gacccagttt 100500
   gggtcatgta ggaaagactc tttcttttca tcctttcttc ctgctctgga ggctgttagg 100560
   tgaggacata attgttggag ctatggcagc cacattatga ctatgaacag gtgcctagat 100620
   ttgtagcaac actgactgag agcactgtca ttcttgagac actcatacct ccttctttca 100680
   gactttatta ttttggggat gatatttatt gatgagcccc ttttggttaa gttgcatatg 100740
   gttggctctt ctgttaattc aagccagaag attcttaatt gatacagtta ggcttcataa 100800
   tttggaggtt tgggaggaaa atgaggtgaa agagcatgca ttgttgggga agataatatt 100860
   ttaaatcaga aaattaacat tttctaccag aagaccttca attattatat tatagactgg 100920
   tttgcagagc cttttgagag tggtgtgaac tgatatccat ccatccatcc atccatccat 100980
   ccatccatcc atccatccat catcttctgt ttgttgattt tctatccatc catccattca 101040
   tccatccatc tatccatcca tccattcgtc catccatcta tccatctatc cactccatcc 101100
   atccatccat ccatccatcc atccatccat ccacccatcc atccatcttc tgtttgtcga 101160
   ttttctattc atccatccat ccatccatcc atccatccat ccatcatctt gtttgtcaat 101220
   tttctaccca tccatccatc catccttcca ttcatccatc catccatcca tccatccacc 101280
   tacctcatcc atccatccat ccatccatcc atccatcatc ttctgtttgt tgattttcta 101340
   cccactcatc catccatcca tccatccatc catccatcca cccatttgac tttcttttta 101400
   cgaaactttc cctttgatca atggaggatt tccttcctgg acttgcttgg ttctgtctat 101460
   tatcagctgg gtataaaatg cttttccttc ttcagttagg tcacgtgtag agaattgata 101520
   ttagatagct atggtgagga gtgaatggga ttctgcctgg cctctctcta gggacacttt 101580
   gccttctgca gtgtctggca tttctaagaa acgttgattt ttaggcattg aggaaaaaag 101640
   ctgttttttt tttttttttt tgacattaaa agctctagtt ttcccttatt ttattccctg 101700
   gagcttcttt caacatattc aagcactgtt ggcttcaggc gtggctttgt gggctggaaa 101760
   gggccagggt atgttttcag aggaatcctg tgggattgaa ttaggaatgc tgccttcagg 101820
   ggataagtat tattttaata ggaagatatt ctttattttt tgattcttag gaattgctgt 101880
   ttctcatgta gatgtactta cctttgttac attaataagt tccataaaat attttcagcg 101940
   tatagaattt ttatatatct agaccattgt ctgtttcagg ttaggaattg tcagtgttta 102000
   tacgtgcgcc taatcccttg tagtctgttc gttactggga tggtggtgat gggggcacta 102060
   gagggtggaa tttgcgcaca tggagagagg agatggtgca tgtcagtgtg ttttccagtt 102120
   gaggcccttg gggttgtggg tggggaggaa agaggaggag gaggaagcca gtgtggtagg 102180
   agtgtgatcc acattctcag gaagaaccat atttgatttc atttagaaaa cggaattctt 102240
   acttcttgaa caaagttggc ttttcttctc tgaacgtggc ctttgcaaat gcttgagtct 102300
   ttgagtatgg tgatgactga gtgagaggag ggatttgctt ggggcagcag gcacatctgg 102360
   aaccattgac cacaaacctg gctgagcaca gagccacccg gatgatgggc ttaataggta 102420
   caaactggga acttcccctc aggcatgcca actcaggagg ttgtcaccag tggctgcagc 102480
   ttggagtgat ctggggatct tttaaatgcc ccaatgccct gcctctacct ctgtcagtga 102540
   aagcaggctg tctgggagta gaatcagagc agcagtattc attaaaggct ctccaggtga 102600
   ctgtcatcag catccaggtt tgagagccac tgtggggaaa taatctgtac tttctataaa 102660
   gccagaagca gtcagttttc tgaacatcac tgattagacc gtagccattt tccagtatgg 102720
   acaaagtagg ttctctcctg ccatccccca gctgcagcaa acttctcact aatgggcatg 102780
   ctttgagaaa caccaaactg cagggaccca aaggagcata caattaacgt taaatcacac 102840
   cagctttggg catatatatc cctcctccac cccaaaatct cttttaaaat agtgttttaa 102900
   atgtattttt tgtagtatgt tcacaggact caaaaaaaaa tacaaaatgc tgcactattc 102960
   acaataacaa agatatggaa tcaacccagg tgcccatcag tggcagaata ctatgcagtc 103020
   ataaaagaga atgaaatcat atcctttgca gcaacatgga tggagctgga ggccattatt 103080
   ttaagcaaat tagagcaaga acagaaaatc aaatactgca tattctcact gataagggag 103140
   agctaagcat tgagcacaca tagacataaa tatgaaacta gacactggac tactggaagt 103200
   tagagggagg ggtgggttta aaaactacct gtcgagcact gtgctcactg ccagggtgac 103260
   gggatctgta caccaaacct cagcatcttg cattattccc gtgtaacaaa tctgcacatg 103320
   tacctactgt atctaaaatg aaagttgaaa tttaaaaaat gtttaaaatc cacacagttt 103380
   taaagtctct tattctcttc tgttctccat ccacccattt cccaccccct tctctggata 103440
   tcttttcaga aatcttttcc ttcaccccct ttgttgtcac acaaaagatg acatactgca 103500
   acccctattc tccaccttgt tttttttttt tcctgctaat atttggctag taagcaccag 103560
   catggtgtaa tgactggcat ttgttctgat ttgttgagca tcctcagtga ctggcagaag 103620
   ctcacatggt gctgccagtc tttagatatt tgaattactt cccctgtcca ccccacgtct 103680
   agaagatctc tctaagcaat gcaaagacag cttccttata ctacttttta aaatagatgt 103740
   agagtatata tatatttgag acagggtctc gctctcaccc aggttgcagt acattggtgc 103800
   gatcttggct tactacaacc tctgcctccc aggcttgagt gatcctccca cctcagcttc 103860
   ctgcgtaggt gaggtcacag gtgtgcacca ccacacccaa ctaatttttt tttttttttt 103920
   tttttttttg catttttggt agagacagag tttcaccatg ttgctcaggc tggtctcgaa 103980
   ctcaagcgat ccacctgcct tggcctccca aaatgctggg attacagatg tgagcctggc 104040
   ccaagtattc attttttaat agatgtaaag tatggataat tcataattta ttaaacaagt 104100
   cctgtaggga tggactttag gtgatttcca atttggggcc attacaaaca atgccattaa 104160
   gaatagtgtg gccaatgcat cacttcagat gtgtagaaat ccatctgtag agttcccata 104220
   agtgctattt cttggtcaag gggcatgtgt agagtatctt tagcgacggg aaatttcacg 104280
   aaggcatcac attttccagt ttgctgtagc aggcagctct gcttctgaaa ttgtagtagg 104340
   tacagggttt tcttttcttt ttttccattg gtggctttga ggaactttca gatgggatag 104400
   ttgaaaaatg tacatctaat ggttttagag gagatgcttc tggagctgca gttctcctag 104460
   ggcctttctt gaatgacctg caggcaccat ctggtgtttg ttaaaaatgt ggaccctgag 104520
   ccctattaga ctctttgagg caaggcccaa taaattttta acacaagctt cccaagcgat 104580
   gcttctgtgc actgaagttt cagaaccact gttctaaggg atttgttgta gaacaatgcc 104640
   gtgagaggac atcttttcat atgattagca cagtggaacc acactggttt actcgggaca 104700
   aagtctgcga ctgccatgaa cattgcaagc tcaataatgc caagcaaaga ctgagtcttg 104760
   gcatcgggtt gaaaataaca tttggctatc aacctctgat gcagcattta caagtttgcc 104820
   ctcaaatgcc catagaatag gaggaacagg gtcaacaaca ctgaaaatag tcagataacc 104880
   agttgctgga atctcagtga aatatccatt aactcaaagg cagatggcac tggattggat 104940
   ctacccttca cagtcaacac ctccaacaag atggaacaag accaactgga aacagctttg 105000
   tctatcttcc actgtcctat tcctagacta agagactcag cttcgtacca agtgggaagt 105060
   cactctcttc tgctcaaaat taagtttttg aaatgaccac aggcctctta cgatggctaa 105120
   ttttcctaca ttcattcaga gaagccagca agggaagggg aggcagttaa cttgtgcagt 105180
   ggtttgtgta gggaactgct ggtggtgaat tgaacgttca gttgtgtggc tgccatacgt 105240
   taggtgcata gaagagaccg aaggaactag ggtgtgagtg cattgtggaa tcagagaatc 105300
   agattgtgga ggtgggagaa gagtctgagg cagttcaaga gagctgcagt ggatgagtgc 105360
   ttgctaaaat cccagtgcta tatgtgtagt tctgccctcc aaccatggct tgagaagagg 105420
   actggaccat tactagcgaa aacatcacta ggaagggcag cctatacaac caatcttctg 105480
   gcagacacag ggcctggata tatctttcca aagaataacc cagtacttaa gacaatgcag 105540
   ctgcgtttac agagttttga agggaaatgt tatcatctac atatttgatt ctcagtcaaa 105600
   ctgtcactta tgtgtgaagg caacagaaag acagttttca gatataaaaa ggcactgcaa 105660
   atctaccacc actgaaaaag ttaattgaag atgtactctg attgagagca gatccaaaat 105720
   taggaactta agaatgaaga agtcatggta tggaggaact gggatgagca cttccagtaa 105780
   ttaaacataa tattgagtcc aaataattgt tataagtctc caattaagtg tcagacaaca 105840
   ataattattt gtgaatgagt agctatatgg tgtaaaatta gcttcatgat ctatttgagt 105900
   gcaaaatcct agcatataaa tgaagactgg gaagtagata acataggagc aaggatatga 105960
   aaactttact taatctatca tcttttattg gttttactta atttttcatc tttcattgtg 106020
   gaagacagtc agaaaacatg ctgttaactt tctttaatca caatgaagta aaactagaag 106080
   ttaataacaa aggttataaa aaatcaatga cttaaatata aaatactttc cttaatgacc 106140
   ctgggacgtg ttcaacatag tgctgtatgt tcctgttggt ccttaccatt gcaatacggc 106200
   aaaaaagaaa gaaagaaaga aagcatacaa agaaagaaat aaaactgtat ttatttgcaa 106260
   acgacatgcg tatctgtgca gaaaatacca aggaatctat aaaacaaacc aatcaaccat 106320
   aaacaagcca tcctacaaca cataagtgaa ttcagcaagg ttgccagata caggggaaac 106380
   atacaaaaaa caattgtatt tctataaact agcaatgaat acgggaagac tgaaattgaa 106440
   aatataatac tattgacaat tgattgctca aaggaaaaaa ataattagct gtaaatccaa 106500
   caaaacattt gcaggactta tgtactgaaa actgtaaaat gttgatgaaa gaaataaaga 106560
   atatctacat aaatgaagac atactgtgtt catgaattga aagactcaat atagtaaagg 106620
   tagcagctat ccccaaattg atatacaggt ttaatgtgat tcttataaaa atcccaacaa 106680
   aattactttg tagatataaa taagattatt ctataattta tatgagaagg caaaggaact 106740
   agcatagcta aaacagtttt gaagaagtag aataaaatgg gaggaatcag tctaccgtag 106800
   tcaagactat gtgatattgg tggagggaca gacacataaa tcaatagaac agaatagata 106860
   acccagaaat acacccacac aagtatgctc aattgatttt tgacaaaggc acaaaaggag 106920
   ttcaatggag aaatgaattt tcaacacata gtgctagaac aattggatat ttattggcca 106980
   aaaaatgaac ctcagcccaa gtcttgcact ttatacaaaa attaactcaa aattgatcat 107040
   gctattaaat gtaaaatata aaactataca acttttagaa gaaaacatag gagaaaattt 107100
   ttggaatcta gggtcaggga aaatttctta gactttacac caaaatcatg atccataaaa 107160
   ggaaaaattg aatagtcgga cttcatcgaa actaaacgct attgttctgc aaaacgctgt 107220
   tgagagaatg aaaaggcaaa ctacaatctg gaccaaatat ttgcaaatca cttatctgac 107280
   aaaggactat tatctaggat acataaagag ccctcaagtt tcagctgtaa gaaacaaaca 107340
   atccaattaa aaaagggcaa aagctatcaa cagacatttc accaaagagg gaacacagat 107400
   gacaaataaa cacatgaaaa gatgttctac atcattggct tttaggaaaa tgcaaattaa 107460
   aagcacaatg agatatcact acagatcaaa atgtcaaaaa tgaaaaataa tggtaatgcc 107520
   aaattctagc aagggaggct gaggaacaac tagatcaccc acacatggtt gctgagaatg 107580
   ggaactgcag tagccactct ggaaaactgg cagtttttta aaacatgcca actgccctat 107640
   gacccagcag ttgcactctt gggcatctat cctagataaa tcaaagctta aaggctcata 107700
   caaaaacctc tgtgtgaata tttatagcaa gcaaggaaaa aaaaacccac aagagaaacc 107760
   taaggtgaac tggagtaaga atatagatga gaatatataa tattttatat tctgatgaaa 107820
   tatagtgtta ggatatataa aaaggattgt gttgatacct ctaagagtta gtcctggaaa 107880
   acagaacaaa gcaaagcaaa caaaagagcc aataaaacat ctacttctaa ttatatattt 107940
   aatcatatat atagatcata tatatatcac gcatatattt atgtaacata tacaatgaat 108000
   atatatagca aatatatata tgaacaacat gcaagttaga agtgagaaag gacttataaa 108060
   tccagagata gaaaaattat aagacgagat gtgctattac aatggtgttc agactggtac 108120
   tctcggattt atgtcgtagc ttttccacta ctgttttctg accttgggtg attatttact 108180
   ctctcagtct caatctcctc cactataaaa cagggataat aacgcagtag ttgtatggat 108240
   ttggttaaat aatgcatgca aatagctcaa cccagtgttg gatgcctcat agaactagga 108300
   tgatagctgt ttttcaattt ttggtattaa gtttgaaagt ccaagaagac agaaatgctc 108360
   aaattgtcta ccataaaaga tagactgcct agattagggg ttggcaaatg ttttcttaca 108420
   ggactagata gtaaatattt taggtttatg gggcatctga gcaactactc aatgctacta 108480
   ttgtagcatg aaggcagccg tggacaattt ataaagaagt gagcatggct gtgttccaat 108540
   aaatctttat caaaacaggt ggcaggcctg caggctgtag tttactggcc tctagtctaa 108600
   accaataaca agaaggaagt aatgaaagtt ttcaaaatcc tcaccaaaac ctatataaca 108660
   aatgatcctt ttggttttta aaacatttct ctagaccata gggaaaaagg acactgtctc 108720
   acttctctcc tcttacccta gcatcataca tacacaaaaa tctaacaaag ataaggcaaa 108780
   aaataaagcc atggtctaat ttcacttata aacacagaga aaacagtcct aaataaaatc 108840
   ttggcaagtt gaatcctcta ggatattaaa agaacaatgc atcatgatca aggaagcttc 108900
   attctagaaa tacattatga tttattatta ggaagtctat taatacgtcc catcaatatg 108960
   tcaaaggaaa aatatgatca tagatgcaaa aaaaagcatt tgttaaaatt tcaatatttg 109020
   ttcttggtga aaattcttgg agaaaggact aagaacaaaa agatactttt tcagcatgat 109080
   gaagactata tgtggccaac cagtagccaa aaatatactt aattttgaga tacttaagga 109140
   attcccatta aagttaagaa taaatgaaag atggttgata gcaacactgt tatttgtcat 109200
   tgttttggat gttctagtca atgaaacaag aaaagaaaca tataaatact aattacatat 109260
   tggaaaaatg aataccaaat taacactatt cacaggttat attatttatt ttagcaactc 109320
   taaaggaatc cattgaaaaa ctattagtac aaataacaga attagttaag agggctgatt 109380
   acaaaacatg tcaaccaaaa tcaataactt tcttagagag catctctaac ttggtggatg 109440
   acataatgga aaaataatcc cttttggtct ccagatgcca ccagctccct tttcctcctc 109500
   cgcatgcttg gcagaaggcc agaggaagta ctgtagtcct ctactacaga gggaagtgta 109560
   ggaaactgga atattaacca tggattggca atctgtgtgt ctaaaggaaa gtggtttaca 109620
   ttttctttta aacgtcacac ttggcatgcc agatccccat ggcgttggag cataccaaag 109680
   tgatagagcg tgggcataat atgtttcagg caaaatgttt agcatttgca cagttaggtt 109740
   ctcaccaatg gtgtgttcct tccatagaca tgtgagcctg gagcttggag cattggagtt 109800
   tgagaaatgg cttgatgagg gcattgagaa gcactagaaa ggatatggat agcaactcga 109860
   ttcaaatttt atttttgtct acttttctaa gtttgtttac ttcaagcact taaactccac 109920
   ttctttgagc ctttgttgcc ttgcctacat actgaggata attaaacctt tctcctaggg 109980
   tcttgtagca gtaaatatga ttggatgaaa aatacgaaag catgtcaaca actgcttgtt 110040
   aaaagttagt tacctttctg cttccttctt atgcttgact agctcccttg actctttgaa 110100
   aacatcagaa agcctagcct taaactcctt tttggaaact aagcattggg tcccagagcc 110160
   tgaggtttct taacgtctgc ccctagtctg gttcagcttc tgcctgtttc tcaactttgc 110220
   tctcatttca ggaaaggaga caaatgaatt attcacgatt attgtctctt gaggggcagt 110280
   gactgtctct tctatcttgt tcttccactc ccctccggag catccagagc aactctagaa 110340
   ataggagtgt agaaataaaa cccaggaaag agggacttta gaaacaagag gcaatgccat 110400
   ggacaggggt ggcagaactg ttgcccttcc agtctttgct ctgccatcca ccagacttgt 110460
   tgcatgggaa actcagtcaa actctgaagc ttcggagtgt tctccactat aaaaggagaa 110520
   aaatgatcat ctaccttagg gtggtgttgt aaaggtcaca tatgtgtggc caggcacagt 110580
   ggcttatgcc tgtaatccta gtgctttggg aggctgagat gggaagatca cttgaggtca 110640
   ggagttcaag accaacgtgg acaacatagc aagaccccat ctctacaaaa aaagttaaac 110700
   aatactaacc aggctagttg acatacacct gcagtcccag ctacttggga ggctgaggcg 110760
   ggaggatcac tggagcccag gaggtggagg ttgcagtgag ccatgattgt gccactgcat 110820
   ttcagcctgg gtgatagagt gagactctct ctctctctct ttttaaagat acatgtgtga 110880
   atgtgtctta tttaactatg ttacacatac atataaatat aaattctgtt tattaatagc 110940
   tcatataata tacagactct gcgcctgtac aaatgtaaag gcaatgcatt ttacgtagga 111000
   aaaatggttg agtcgaatga tttaattttt ttacaaatca cacaatactt gcatgaaata 111060
   gcaacaggtg cgaacctcct aggcagatag gagtgcagtg ctttgtttca cagataccat 111120
   caagcctaac ctttattgtg ggcttactat gtgtcaggcc tgttctagga tcttgttaat 111180
   gaactgattc atttttttct cataacaaca tagaggggaa aagatcacat ggggaaggca 111240
   ccaggtccct ggactttgcc tcgcgtgctc ctggcatttt gtagagaggt aaggacttgg 111300
   ttccttatgg acttttaagc tcatgattgg tttggtctgg tttcaattct catcttcatg 111360
   tatgctctta gaaataatct cattttagga gagaaattca gagtcaacac ggaaactgaa 111420
   aagctctagt actgcctgaa gtctcagaat aaagggtgcc ttcattttca cttttccaaa 111480
   taatctatga gagggacttt tagtagatcc gtaagatgtg atactaacat agtatctgca 111540
   tattgtgaaa aatttataac tgtatccata aaggttattt gactccattg cctcaaagtc 111600
   tatataatta gaattccatg atgtcatctt tgagctttaa tatagcttaa aattatcctt 111660
   ctgtaggttg tgtttctttc tgaaacattt taaatttaga aaattaactt gttaaacttt 111720
   tttaagttaa atctttagat ttttttttat tcaccctgct gtgggggagt cagaatgaca 111780
   aatgcttgaa tcatcaaagt caaatgttag ggcattttag cccctctggg aaagctaagg 111840
   tctccatggc ccaagaatct aactctactt atggggatga tgatctcaca tgatctcatg 111900
   atctctgtgt tttgccaaca ttccttcttg gcaggactta ggagtgcaga tgggccccag 111960
   agagcatgcc ttgctcaagc caggtccccc tcctgctgcc actttgggtg gctggaaaaa 112020
   cctcacaaag ggtgcagtgt ctgcggttgg gagactggtc tcaattttcc tccatcacct 112080
   gctcataaac tctcatgggt ttctgggtca cagtgcagcc ctaggtcaat cttccaaaag 112140
   ctgatttgct gaatttcatg tgaattgact agcttcatgt cagcatttta gggaacattt 112200
   gattggtctt catgcctgct ttctgccttt gcaggctgag aaacaatgag gggagagaat 112260
   gagggacaga gagaggaggg gactgagggg ccaaggaggt tccgatgagc aaaaatggaa 112320
   gagaaagaac gtcagagagg aggctattgg gggatgagga gagagaccag agatcaagaa 112380
   gcatgaaggt attgagggcc agagagggga cttctaggga catggagagg ctgcaggaca 112440
   gagaaagggc taggggaacg ggacagtggt tctttcattt caatgatcaa agttcccagc 112500
   tttttgacac cacaggggca ccctgacaat tctggcaata agaacatgaa aggcctggtc 112560
   tttatttcac tcaattcctg ctatgtgtgg tgagtgtggg tgagccaagg ggaaggtgat 112620
   cctattgtca ggaggtaatt taccatgaat aggggatgat atggaaataa tgtgtgtgat 112680
   ccttcccctg ccactgttgg gatgtctttt taatttcctt ccctcatttg tcacagccgt 112740
   gaaaatactt tttctgatat gatgaatgac agatggcagg gtgccggcag cccttctgga 112800
   gggatgggag gttgtgtgtg tccacgatag gggcccaata agtactggct gaatgagaaa 112860
   atgaggagcc tcactgtggg ctttctttgg ggtgaatgga ggtgctgagt gacctctcag 112920
   cttcctagaa gtcacaggcc agaagccgtg gaatctcagt ggtggaaagt cctactgatt 112980
   tgaggatcag ggagggagag aatcagcaat ggtgtgctga taaatgttta gtagttggct 113040
   ctctggtaaa aaagaaaaag aaaaagaaaa agaaaacaaa aacaaaacaa aacaaaaaaa 113100
   acaaacaatg aacaaccctg gtatgcagtg cttgccactg gtcagtttcc atggtcagtt 113160
   tctcaccatg ggcaatttca tgtgccatca ctgaacacag agtagggaag agatccacgc 113220
   cattggctca caagctggcc ctaccacacc accaggagga atatatattg ggcaacgact 113280
   aatagcaata gaaaaactca tgcttctttc tcctggcatg acttcatgta tatgacatac 113340
   atcctctcat tttatgcttg tagcaactct gaaacctaag atttatttcc tcattgcagg 113400
   tggggaagcc aaagctcaga ggggttaaat aatgtttctg ttattgtaca gcaaatcagt 113460
   ggcagagtgg attctaagat tcgtgccttc tctactcact tcactgggct gtcagaggtt 113520
   aagggaagag ttacataagc cacctctgat tattagagaa tgacagggct ggctaattct 113580
   gcctggatag tattgaagag gagcttcatt caggggcacc gctttgctaa agagaccacc 113640
   caaagaatga gtagttttga gtcaccaggt accctgaagt ggtacctgaa ggtacagctg 113700
   gtatgatgga tgacagatgc cggtaccttg ctgtcatcca tcatatcagt tgtcagttac 113760
   caggtagacc tagcggtaaa gggtaagtta tatctcttgc ctaaatgtca gtttcctcat 113820
   ctgtaaaatg ggaccctgaa agtctacttt agagggctat agggttgtta agatttaatg 113880
   agttaagtcc ataaagaacc taacgtagtg tcagacacat aatagatcca gagaaatgct 113940
   gattcttagc aggctaactt tttctttttg aattcctctt taggccagaa ctgactagta 114000
   taggcttttc aaactttagc gtgcagaaga atcacctgtg gatctaatta agatgcaggt 114060
   tgtgatccag taggtctggg gaggggccgg gcctggaatc ctacatttct aacaagctgc 114120
   cagatggtgc tgattctgct agtccataga tcacacgttt tctttcattc tttctttgtc 114180
   tttgtctttt ttttttttga gacagggtct tgtgccgtca tccaggctga agtgcagtgg 114240
   tgtgatctca gctcactgca acctccacct tctaggctta agcgattctc ttgccttagc 114300
   ctcccaagta gctgggatta caggcatatg caaccactgc tggctaattt ttgtattttt 114360
   agtagagacg gggttttacc atgttggcca ggctggtctc gaacttctga tctcaaacga 114420
   tccacctgcc ttggcctccc aaagcgctgg cattacaggc gtgagccacc atgcccagcc 114480
   catggatcac actttgagta gcaaggggct aagggatcct gacatagcat tttggggtcc 114540
   tgcagcttca acttactctg ctgcctgttc ccatgacctc taagactgct cttctcgaga 114600
   cagcatcctt gttctgcact ggttcctttg gagcagctgc tcagccctgc taaaggcatc 114660
   ttgctcagtg caatattagt ttgctgcgga agatctggga cctgggctcc tctcctcatt 114720
   ctccttgatc ttagttttgg ttctgccagg actttgctgg gcaatctttg tcatgtcctt 114780
   tccccttttt agattcaatt tcttgatttg caaaacaaga cagtaggaga gagtcacttt 114840
   ctagtttagg cattctgaag ttctgtgaat ctgtgatacc agctggttgg tgcattttcc 114900
   tgccaggcca taagcagtac ttgcctttat ttctgggctc cagtgggggt cttcagagat 114960
   acatagatgc atatagtcag gacaagagtt aggaggagta aaggtaaggg ttcaatccaa 115020
   accttttgga atagtaacag ggtggcccag agagatgaag aatgattggg gcacaggaat 115080
   cttcttgctt tggggcctaa acgacatctc cagggaatgg ctactcattg ggcacatatg 115140
   agccaggcac tgtgccaaga gctaatggga gacacttgtc attctttctg gccacacact 115200
   ctggagccgc tttttttttt ttttttttta atatttgtga attcgccacc tcataaaccc 115260
   atgtctcttt aatgcacaac tcagaaaccc actcgcccaa cctcccttgc agctagagca 115320
   cagacatgtg acctaggatc tgtcaatcag gtgagtcctt gctggactct gaattggtgg 115380
   ctagaagcag cagacactgt gtttgatctg ttctggaggg ggaggtgtta agtatgtcca 115440
   gactgcagag tcagctatgg tggggcttct agcaagatct gtcccttgtg aatgttgaat 115500
   attcacagca tctgtagtgc agattgcctg gtctggactc tggagtggtg gcagcgggtt 115560
   ctgtactgtg gtcaagatgt ttcttctggc tttctaaatg tgtcctaatt ggattctcag 115620
   ggcctcccaa agcttgtctt tcctggttaa actggttaga gtgggcttcc atgtttataa 115680
   ataagaatct tgactgctta caacacttta tgtgtgttat ctcattgaat tcttttttta 115740
   atttttgaga tggagtcttg ctctgttgcc caggctggag tgcagtggtg cgatcttggc 115800
   tcactgcaac ctccacctcc agggttcaag cgagtctcat gccgcagcct cccaggtagc 115860
   tgggattaca ggcacacagc accatgcctg gctaattttt gtatttttag tagagactaa 115920
   taaggttcac catgttggcc aggctggtct caaactcctg acctcaagtg atccacctgc 115980
   cttggcctcc caaagtgctg ggattacagg tgtgagccac cgtgcctggc cctccttgaa 116040
   ttctcacagt aacctagtga ggtaggtgtt attatcctct tcttgtaact gaagaaactg 116100
   agtcaagaag agatgaacta acttgctcaa ggttccactg tcgataagtg gaggcccagg 116160
   atttgagtcc agatcctctg gttccaaagc acacattctt aaccaccatg catctcctgt 116220
   cagagatttg tgggtgtgga aaggtccttg caaatcactt gtcagtagta cagggtttca 116280
   tgagatctac acctgggctg ggcacgcctg ttggtttttt actgacaacg cataattttt 116340
   cgaggagtgg taatcagagg caaccgttct agtgacagtg attcaggcgt aaacacttgg 116400
   ctttggccca gaggttgcac tgtgtgagac cacaggtgaa cactagaatg tttacactct 116460
   tccggagtct gtaggtggat gccagggaca gagaatgaga ttgtaaccca gagaaagaaa 116520
   gaaaagaaag aacgaacaaa caaaagaaag aaagaacgaa cgaatggaca aaagaaagaa 116580
   cgaatgaaag aaagggccag cccctggggc ctccttagat aaggaagaga aaggagacct 116640
   tgcctcaagc tcagaaactg gttaaaacac gagcccagtc acctagtgat aagcagtgct 116700
   tggttctatt aactctgaca acatagtact atcttgggca gagctctttg gagaagcagg 116760
   ttggctaact ggccaacctc agttgattct catggtcttg gaactttcct gggggtccaa 116820
   cttcctccgt tctgcccctc ttttttctct accttctatc gttttattct ttgtctcctt 116880
   tatttttctt caacaaatat ttttgagcac ctattgttaa caattcaacc aatatttatt 116940
   gggtgtttgc tcggtgccac ttacagttct aagtgctgga gatacagcag tgaactagcc 117000
   agacaagctg tcatagagtt tccaatttag tggcagtggg gtagtccagt aggcatgaag 117060
   ccaggcaggt accagttgtt gaggtggtgg tagttccacc cagtactagg aagataacat 117120
   tcttatcccc attttacaga tgaagacact gaggcaagag agcttaaatt aattgcctga 117180
   ggtctcacag gtaggctggg atctctgtga gatctaggag tgccagagcc acttgggtcc 117240
   agccacccac acctcttccc tgtgcctcag gctgggatga cacccagagc ctctcctttc 117300
   tccctcacct gggcccagag accacctcct tccctctcct tgcattgctg ctgcccatgc 117360
   taaccgaagg agcctcgccg tggggcggat gtggcctccc cagtggccca cttcctcctg 117420
   cttctgccaa cagcagcatt ggcccctatg atttgaattt tttttttttt tttttttttt 117480
   tttctgttga tccctctagc ggcaccagat gtgggtgggg tggagggtgg gtacacccac 117540
   actcccttaa gcctggaagt aacctaggtt ccagactctg tgtgtccctg aatggtaata 117600
   gtgactctct tcttagctgt ctgtcccact gggtcctccc actccccaac tggtgctcct 117660
   gcttttcctc agaatggggg acataaatct ccttctagtc ccaacccatt gtcaggcagg 117720
   gcttcctcct ggagggctga cttcccagga ctgggccagc taaaagctaa gcagtatggg 117780
   taagagcacg gggttctgga gtcagacaga cctaagttca aaccccagta ctgccgtgga 117840
   caagctgtgt ggcctcagac aagttgatta acctctctga gttcctttta aaataggctg 117900
   aggtctgtag ggataaggta cctgaggtct tagtgcctgg cacagagtaa acccacgtgt 117960
   tggctgtttc cattattggg tagaacagag gggcctcctg gcatcagtgg gcattttggg 118020
   cctgctgctg tcttgtctat ctaggccttg gttttgcctt tcaggctggt gcctctttgc 118080
   agtttcactt tagcaagtga aactttattt ttcctgtttc tcctgatctg tttcctcctt 118140
   attttccata gtgaataatc acacataagc ctttcttttc tcagggcaag gttgctgtct 118200
   gtgaaggagt ggaatgcctg agcatccctg gtaggaaagg aagagtgacc agcatcaatg 118260
   gggtactcta ttccctgagt agcaagccct cttcccgcct tgtccctggc tcccacacct 118320
   gttatcctgc cggcagtcat ctccagtatc ccagaggagc catagggatg ggtgtttgaa 118380
   tataagaaca ctctgcagct cagatatgcg gcattacttt tcctgtttga ctcagttctg 118440
   cagagggttg ctgagtgact gtcatgtgca aagcacgtgg gaggaaagat gaataatgag 118500
   agcaatcgaa tgcccattcc cttgcatcca gggtgctcta ctagacctgc gttcttccat 118560
   cattctttca gcccacgtgg ttgccaatac tgggagttat cagtcttttc catttttgcc 118620
   aatctaatgg ataaaatatc ttgtattaat ttgcatttcc cagatattca tgagattagg 118680
   catacgcttt ttgggcactt gtattttttt ctctgggaat tccctcttcg tattactttg 118740
   tcaatttttc tagtggcttt tttatttgag acagggtctt cctctgtcac ccaggctggg 118800
   gtacagtgtc cagatcacag ctcactgtaa cctcaactcc agtgatcttc ccaccttagc 118860
   ctccctagta aatgggacta caggcacaca ccactatgcc aggctttttt tttttttttt 118920
   tttgtagaga cagggttttg ccatgttgcc caggctggtc ttaaactcct gggatcaact 118980
   catcctccca acttagcctc ccaaggtgct gagattacag gcatgagcca ctgcacctgg 119040
   tcccctgtct tttttttttt aattaattga tagtaactct ttatatattc tagatgccaa 119100
   tccgtgattt agtctaaaaa taaatcccct tggtctgtcc tatgtctttt cttttggttt 119160
   atgaaatctt ttgtaataca ggaacgtcac acttccacag tcaaatgtgt cagtcttcta 119220
   aatagatata ggtaccttgt atatactcct tgatatatca cattgattgg tggtgacctg 119280
   tttgtctccc tggctgtcag actgtgagaa acttgagggc aggggctgta ctttattctt 119340
   ctctgtctct ctagggacta gtgcaaagct gggcacagag ttttgtgcct gaatgagttt 119400
   tttttgtgtg taaacgtggt ataaaacagg tcttgtgact gaatgaggga atgaatgaaa 119460
   aagccctgtc ctgccctccc tgcagagatg gggtctgctg cagagatggg agtgagtcag 119520
   ggagtgaatc acaagctctt ccgtggctca gctgacattt caaaagccct gagcagagaa 119580
   gcccccctca ttccaagaga gcctccgcct ctttgagcag gcacagtgaa gctctgctga 119640
   ttatcaaact ctgtgtgtgt atgtgcacgc acgtcagggg tttagcagtt aaaactctta 119700
   gtgctgcata ttctgcgtat gtgtgaacaa ggctttaaaa aatctctcag aggacctgtg 119760
   tatgtgtgtg ggtgctgggg gctgcataga gagagagtta aaagagatga gggggtgttt 119820
   agaatcgttg caatgtattg catttgtttc tgggagggtg gggcgactgg agggggttaa 119880
   atagctccca gaggcgctct tgtgagcgga gcttcctgaa atctctttat ctgtggccac 119940
   atgctgttct tggttgtcca cgtggttccc tggtgtccct gttgctgaag gcacttctgt 120000
   tttgttcttg gaggcttggc ttctcattgc cattttctaa agcagtagag agtgccagtt 120060
   agattgttag acccaaataa tctgaaatgt gtgaaaaaat agtaaatatt tactgtacct 120120
   tttgtggaac aggttattgc catggaaatg cactgtacac gaatctggat gatggaggga 120180
   agctggctca ggagctatgg gagtgtttgg ataccatgcc gcctgaattg gcaggtttag 120240
   gagtgagtgc cgtgggttta gaatcacagt gtggttgggt gaggctgaaa cacttggtcc 120300
   ccagtcaacc tgtactgaga ggggcattta ttcattgctt cccacatgca gggtgcttcc 120360
   tagagcattc tccatgcatt attgcgttta atcatcatgc caccccaatg aggtgccatt 120420
   atcctccctg ttttacggag acacagagtg tttgatctac ttgcttgaag gtacgcagtc 120480
   aatgaatggc agagccccct gactctgttc tctggagctt gatccttccc tgctcagctg 120540
   tttctctcac tgtgatttac cctagagaaa gctaaccagc tgtctaccgg ggttttattt 120600
   tccaatattg acttcggggc tgacatttag aaaccatcaa caatttccag cttctcttga 120660
   aaatgtgcac aatctggcta ctgtgtgggg cttccctagt ggctgctccc gagcagccat 120720
   ccaggttagc cgggcatcag ctctgcagtt ggcgctgggc atcctcctgt tgcagctggg 120780
   gagaaagcat ggggtatcac acccaggggc tccttgaatt aattgagaac tgtctgttga 120840
   gagctcctca tcctgttaga cttgacattt aatttagctg ttgttttcat ttgccaatta 120900
   gagcttttga gattcttttg attttcttcc tgtggtctca aggaatttgt gggtggttga 120960
   gtgaagatct tcatggcagg aaaggaaggc tgcaacagga gatgtaatgc gtgagcacat 121020
   tcattcattc attcattcat ttacttgttt attcatttgt tcaacatatt cattgagtat 121080
   tggatgttga agttacaaca gtggaagtat ggcaataaat aagacagacc cagtcactgc 121140
   tctcaaggag catatagtca aagagggcag tcagacatca actatgatgc acgttacagg 121200
   gaaggcatcc atcttttttt tttttctgtg tcacccaggc tggaatgcag tgatgtgatc 121260
   tcagctcact gcaacctctg cctcccgggt tcaagcgatt ctcctgcctc agccttccaa 121320
   gtagctgaga ttacaggcac ccaccaccat gcccggctaa ttttttttta aattttatta 121380
   ttattatatt ttaagtttta gggtacatgt gcacaatgtg caggtttgtt acatatgtat 121440
   acatgtgcca tgtagtagag acggggtttc atcatgttga ccaggctggt cttgaactcc 121500
   tgacctcagg tgatctgccc accttggcct ctcaaagtgc tgggattaca ggcatgagac 121560
   accacgcctg accagggaag gcatcgatct tagactgact tagtggtgtc taggtctctg 121620
   aaggaggtgt cagagaaggt ctccttggct aatctttgtg accaccggag ctctgttagt 121680
   ttcataagac ataagtctca gcaactgttg cagaaagacc cttcctcagt aaggatgatg 121740
   ggctcctcgg gctcttgccc ttcccgtcct gggaggaacc ttctatcaag agccatggct 121800
   agtcatttca tccattggga atgagcttac tggctccatt tatgagagtt agtttagact 121860
   gaggagtaaa ggaggagtga aggagtttgg gagttgtcag tgataagcat gcacatttgg 121920
   agttgacagg acagcatgga cactagagcc tgaacacctt agttcagctt agctgcactg 121980
   cctattatgt gatactgggc tggtttctta actgttctat gcctcagttt ccccatctga 122040
   agttgggaat aaaatactca tctcataggg ttattgtgaa gagtaaatga gtcaagctgg 122100
   gtgtagcggc tcatgcctgt aatcccagca ctttggaagg ccgaagtggg tggatggctt 122160
   gaggtcagga gtttgagaac agtctggaca acacgatgaa accctgtctc tactaaaact 122220
   acagaacatt agctgggatt acacatatgc aggtgtgtaa tcccatgaaa cacatttcaa 122280
   ataattgcta aaatttttca agctgtggca taagagacaa cagtcctgaa gaggactgaa 122340
   aggtcttggg atgcagggga gcttaatgta ttaaaaggtg acattttgga tcagtaggga 122400
   aaaaattaca cacaggtgtg tgtgtaatcc cagctacttg ggaggttgag gcacgacaat 122460
   cacttgaatc tgggaggtgg aggttgtagt gagccaagat ggcaccactg cactccagcc 122520
   tgggcgagcg agactgtctc aaacacacac acatgcacac acacacacac acacacagcc 122580
   aggcaaagga tgaagagaag cagttaataa atgtgagcca ttcacgtgtg tattatgatt 122640
   atcacgtgat atcagccagt cctgcttgag actcactttt ccgacagttc tttcttcggt 122700
   ggactgtacc catgcccctg tgtgccatat atttgtgatt tccagccttc ctttctgata 122760
   acttcaattc attgctcttc ccaggcaagt ccttcaatgg ttatggggtg ccgcgcttgc 122820
   cctcatcccc agctgagtgt tctgcgtttg gggacttgtg tttataagtg tgggaggctg 122880
   tcccctgggg gttactgccc agacagttag ggttgtccat aataacagaa aagtgcatgg 122940
   agagagaagg tgcaggtccg cctatttgat gttgggtagg atgtgctgct tgatgaccgg 123000
   tgaaacattt acttctccca ccatgggcga gaccttggat gtgtccttta cctctctctt 123060
   tgtcacttct gtcctgaact gcccatgagt gaccaggggc ttccgacaga agtgtctcac 123120
   aatccaacca cacagcagaa aggcattttc ctttggattt gagctttgtc cctgcactga 123180
   ctttttgtgt catctcctct tcattcaatg tgagatactc accttgattc atagaatgtt 123240
   ttctcattcc aggtttgtat ttttttaatc accaaaagaa gcacttatca taaaagactg 123300
   tgatgatgta taactgtgta tttagtttga aaatcttcac cttatagaag caataaacaa 123360
   tatttaggtg tattatcctg tgaaattttt ataaatataa gactttatta cgaataaaca 123420
   aaaaaagaga tcatatccta tatacttttt ttataatttt ttttgagaga gggccttgct 123480
   gtgtcatcta ggctgaagtg cagtggtgtg atctcagctc actgcaacct ccacctccag 123540
   ggctcaatcc tcccacctca gactctcaag tagctgggac tacaggcacg cgccaccacg 123600
   tccagctaat ttttgtaata tttttttttt gtagagatgg ggttttgccc tgttgcccag 123660
   gctagtaact ttttccctta ttaatacctt atttagatcc accttatgtt ttaaatggct 123720
   gcagagtgtt tcataatatg gccaccatat tatttacatt catatttata atttgtctaa 123780
   aacattagca ataggcattt ggattatttc caagttttct ctataatgaa cagtgcaaca 123840
   gtgaacactc ttgggcactg tagtggggat ttccatagaa caattcttag aaatggggtg 123900
   tttagggcaa aggatattaa catttttagt tgtaatagat attgctaaat tgctctcaaa 123960
   tgttgtgcca atttatgcac ccaccaacag cgcacgaaag tgcctcttag aaaatgtctt 124020
   ttagaagata aaatacctat tgttaagcca tgaaacacat ttcaaataat tgctaaaatt 124080
   tttctagctg tggcataaga gacaagagtc ctgaggagga ctgaaaggtc ttgggatgca 124140
   ggggagctta atgtattaaa aggtgacatt ttggatcagt aggaaaaaaa atggcttatt 124200
   taatacatgg tgctggcata attgggaggc catgtggaag gatgctgagt ttgattccta 124260
   actcatacaa aaataaatta caggtggagt aagctatagg cagtaaaaaa aaaaaaaaaa 124320
   aaaaaaaaaa agaatattag aggaagtttt aggagcatac ttctgtaacc ttgagtgagg 124380
   gaaatccaga agccataaag aaaaagatta gacagtttca attatgtaaa aataatttaa 124440
   tcttttaaaa ggagaatgta ttcatgaatt gcttatgtaa taagaaagac tattaaaaat 124500
   aggtccagta gataatgtac aatggtttcc aatgtgaata aagttgaagt taataaagaa 124560
   aagaaagttt taggacatgt cagggagttt ttgttcaagg attgagattc tgcaatgttt 124620
   attctaaata aagatgaatg atagactagt gcacatgtag taaactagta aatgtgtagc 124680
   ccataaacct tgtaagccct ggaagggtaa agaaaaaggg aaggaaatgt tgttttctgc 124740
   ctctttattt tttgttgttt tgagaaagtt gagtacccac ccaaagcccc aggtgtccca 124800
   ggagagagaa gattgaaaac atgccatcaa aacaggagct tcagcctcag ccacaagctc 124860
   cagtgtttac tgggggccag tgcccttatt cccacgtgaa gcatctgcct tttttgcccc 124920
   cttgaagtct gagtctctcg tctgttcctg gagattttta gccaagcccc tcctccccac 124980
   ccctcacctt agtatttgta ccaccctctg aatcaacccc aaagacaccc ctttgtactc 125040
   ttcccttcat ctggccatca ccagggtact gtggtcattg aagctgagtg tgtgccctca 125100
   ctttccgtaa actcacagtt cctttattct gcaccttcag ggattgcaaa tcgttaaggg 125160
   tatgccttca aataccagcc ctctgacttc ctagctcaat aacctcacac agctctatat 125220
   ctcagtttcc tcatctgaaa aacagtcatg aaatggcacc acctcctagc attaatgtga 125280
   agattgttat tattatgtgt tattaattat tattatgtta tcctattaat atatgagaca 125340
   gaatctcact ctgttgccca ggctggagtg cagtggcaca atcttggctc actgcaacct 125400
   ctgcttccca agttcaaggg attctcctgc ctcagcctcc tgagtagctg ggattacagg 125460
   tgtgtgccac cacgcctggc taatttttgt atttttagta gagacagggt ttcgccatgt 125520
   tggccagggt ggtcttgaac tcctgatctc aggtgatctg cccaccttgg cctcccaaag 125580
   tactgggatt acaagtgtga gccactgcgc ctggcctatc ttattattat tattattatt 125640
   actttttgag atgggatctc actctgtcac ccaggtggga gtgcagcagt gcaatctcag 125700
   ctcactgcaa tgtctgcctc ctgggttcaa gcgattcttc tgcctcagcc tcctgagtag 125760
   ctgggaccac aggcacccac caccatgccc ggctaatttt tgcattttta gtagagatgg 125820
   ggtgtcacca tattggccag gctgatctcg aactcctgac ctcgtgatcc acccacctcg 125880
   gcctcccaat gtgctgggat tacaggtgtg agccactgcg cccagcctct ttttattatt 125940
   atacttagca cggcacttgg cacttaggaa atgttcagta tatgttagct attattggct 126000
   actgttattt cttcttcttc tcctcctcct cttcttcttt ctcctcttat tcctcctcct 126060
   cccccactcc cttccacccc ctcttcttat tcctcctcct tctcctcatt attattttaa 126120
   gtacctcctt accaatgcct acagaaatag actgacctag taccaggatc tagaggagtt 126180
   caatcaagtt cgtctgttgc cttcaaggta acagcgtagt ggtggttccg agccagatgc 126240
   cagacccagt ctgcctttgt tcaaatcctg gttttacact taatactgta ggtacctcgg 126300
   gcaagttacc taacatttct tcacctcaat ttctccattt gtaaaatgta tataatgata 126360
   gtacctacca cgcatggttg ttaggaagat taaatcaatt aatatttgta aagtagagca 126420
   gccggctcat tgtaagtatt gggagtattg gttaaaataa ttacaaaata aacaatctca 126480
   cttagataca tcccttaccc cctgtggatt atgtcaaggg tgaaagaaac agtgtgcttg 126540
   aaatcctgct gtgcgccgag ctgtatatca gaggtgttat ggtgattcgt catcattctc 126600
   ctgaccactt tccatttttc ttttagagtc aacactggcc aatctttagg gaggagctct 126660
   gatcattgag aaactgacac tgtggaaggg ggcatttctg actctctctt cctagctcca 126720
   tttgatcttc ctaaatcttt caataatgtg tcattggggt aggaccaaag aaaaacgggg 126780
   tgaatgtacg gttgctgctt tgtttcccat agtgggagtg actctgatgt ttcacaaagg 126840
   caggggtgga gaggcagcgc ctcgcaggaa agccttcttg ctaaatcgag gacttctgca 126900
   cagaaataga tacaaacttg cttactgttt aaatagaata ttagcgatta tcttttatcc 126960
   agctggagtc tcctcggttt ggtactgcct caaatgagca gcctgcaatc ttgggtgtca 127020
   cttcctcact ggttctgctt tccataactg tgttgaaatc actcccccag aaggtcacca 127080
   aaagggaaaa tattagtgtg tgtttattgg cacgatcttg caggtgctgg gctaagtgta 127140
   gacatgattt attttcattt aatcttcaag gtagttctag gcgatgtccc cccactttgc 127200
   agatggggac actgatgtgc aggaaatgaa gtgacatgct gtagcactgt tggtgctctg 127260
   ctgtgtcccc tcacccttcc atttcattgc attctggcct ggcttccaat gggcagcaac 127320
   tgcagctgtt cacctgaggg cttgctctgc cgtagtccat tcggtccata ggcatgtcag 127380
   gctgtggaag cacaggagga gaggtgaggg atggtgcagg agagaggaga ggatgattaa 127440
   tgtcccccag ggcccatcct caaccaatga cttatgggaa acaacctcct agagttgtat 127500
   taagctccag tgacccagaa tggtaacaag ctcacaaaca cttccttttt taaaaaattt 127560
   cacaaaaata ctttattata cccatacctc cttgttttag cccatcccag tacggtggta 127620
   tcttcagaac acttccactt tcccctctcc tcacctcttc cctcccaccc ttgagatgta 127680
   atctttggac cacttttact tctttgcttt ctctcagcac cctttctccc ctactctatg 127740
   gtgactttgt gtttttgaat tgaagtcgtt tcatgtctgt gtcttttttt tttcttcaac 127800
   tgttatttta agttctaggg tacatgtgca ggatgtgcag atttgttaca taggtaaaca 127860
   cgtgccatgg tggtttgctg cacagatcga cccatcacct aggtattaag cccagcatcc 127920
   attagctatt cttcctgatg ctctccctct tccctcccca ctctgacatg gcccagtgtg 127980
   tgtcattccc caccaccaaa tgtccatgtg ttctcattat tcagcgccca cttataagtg 128040
   agaacatgca gtaaaacact gcctttatag gcttccttcc cttcactgtc ttacttcccc 128100
   actccctact gctgctctct gggatcacct ctcacagaag ctgtttgtac tttaatcatt 128160
   gtctgagagt ctggggtatc ccaaactaag ataattgccc aaggtcttag ctaaattcaa 128220
   cagccctatt tgtctttatc ctccttgtgg caatgccact actaatggat gtcccttctt 128280
   aaaattcttc cccattttgg cttttgtgac acgtaataga attcttctac ttctgattat 128340
   ttctttcctt tttcattaat tggcagctct ttctttttaa aaaaatttgc tagatttttg 128400
   cttatttttt tttttaagaa atgaggtctc attatgttgc gcagactggc cttggaatcc 128460
   taggctcaag cgatcctccc atctcgtcct ccgaagtgct gggattacag gcgtgagcca 128520
   ctgtgcccat ctttcttttc ctttctttag ccacccagtt ttaggtacct ccagtatccc 128580
   agtattttag ttgtaagtga cagaaaatct gactcaaatt ggtttatgta taacaaaatg 128640
   taagttattg gatcacatac ttgggaatct taggataggt ggcttcaggg aagatatgat 128700
   caaggtgttc agcaatctga ttctctctcc atcatgattc agctttcctc tgtattgctt 128760
   gattctcagg catgtgcttg actaatgagg caacaacaac ttgcaggtcc aatcttatct 128820
   tcatcaatgg tatcttaact acatcagtgg tataggactg cttctttcct tagagtccca 128880
   ctgtaagttt tgggtttcat tctcattggc ctgtgttgag tgatgtatac atttctaaac 128940
   caatcacagt tgccacggag atgtaataca ggttgagcat ctcttatcca aaatgcttgg 129000
   gaccagaagc atttctgagt gtggattttt tttttcttca gattttgaac tatttgcatt 129060
   ttacttgcca gttgagcatc ccaaatccaa aaagttaaaa tccaaaatgc tccaatgagc 129120
   ctttccttgg agcatcatgt cagtgctcaa aaggttttgg gttttggagc atttcacatt 129180
   ttgggttcct ggatttgggg tgccctcatt tcccaggctg ggtcacataa tcacccttgg 129240
   agctaaggtt agagttgagc cctactccat aaggactgag agtggggaga ggtgatttcg 129300
   caaaggaaag tcaaagtgct gtcacatgag gggatgggta ctggagaggc aaaaacaaca 129360
   gatgttcata ctccgcatct ctgggttcat ttatctgctc ttctctttct ctgtgaatca 129420
   tctgatgatt tccactcctg cctgcctgag taggactctg aaactcctgc ctccagatct 129480
   gactccactt ctgagttgta gcctcctttc ccccaccctc tagagttagg catttccatt 129540
   tggtggtgcc atgcagacct gaaactcagt gtgccacaac cctcgccacc tcccacctgg 129600
   tttcgctccc taacgtccct gtctttgtca gtggtgccag ttgggtttcc atctctccct 129660
   catccgtccc aacccccacc taatttccag ccccattgtg ctgcttttga ttccttgaat 129720
   gcacctttgc aatgcttttc cttggtttgt aaccctcttt agcatattct tggtttggtg 129780
   aacccctggc catatttaga tatcagctca gaattcactt atttgggaac ctctaacctg 129840
   gtcacccatc tctgccaagc ctgagttaga tgctctttcc ctgagttcta ctaggccttc 129900
   atctcgtcta ccacagcact tctcactcag actccaatga ctggctccat tgcctgcctt 129960
   ccccgtaagg gcaggggcac atctgtgtaa ttcaccattg tatcttcagc agtccagcct 130020
   gacaattgag tctaggctgg tgtccaacaa accagtcttt gttttttttt tcttttattt 130080
   taattttgat agttttgggg gagcaagtgg tttttttggt tacatggata agttctttca 130140
   tggtgatttc tgagattttg gtgcacccat cactgtaaca gtatatgtgg tatccaatat 130200
   gtagtctttt attcttcacc ccctcccacc attccccgga gtcctcaaag tccattatat 130260
   cactcttatg cttttgcatc ctcatagctt agcttcattt acaagtgaga atatatggta 130320
   tttggttttc cagtcctgag ttacttccct tagaacaatg gcctccaact tcatccaagg 130380
   tgctgcaaag gccattattt cgttccgttt tatggctgag tagtattcca tcatgtatgt 130440
   gtaccacatt ttctttatcc actagagata aaccaggttt tgaattcagg atccacctct 130500
   cctgagctgt gagcacatgg gcaagtttcc tcaccagtcc aagcctgttt tctcatctct 130560
   aaaatggaga caatgacggt tcctccctca ctgagcactt gtgagaagta agtggtgatc 130620
   acggttacaa agtgttcagc acagtgcctg gaacacggta agcgttcaat cagtgccaat 130680
   gatcatttat tatactcact atgaacatta tttttacctc atgcagtgtc tagaacataa 130740
   tcaactctca acaaatgaca gctcttatta ttagccaaga atggaacctg aaacatagta 130800
   cgctccatgg atcattgaag ctttgttttt gaaagtttga aattaacaaa aagagccttt 130860
   gatgtttccc ttgctgcaca ttcttcccct ccaggtggag tgggcagttt ccactgggtg 130920
   ctggaaccaa ccctcctgta gttggaggtg atcttctctg atgtcactag ggtgccgagg 130980
   tgatgtgtca ccagcacttg tttgcttgtg tgatgtaatg agggtggact tgcttctcct 131040
   agggaagagc ctcattaagg ttagcaaggg gcttgttcct gggaagttct aaggaccgcc 131100
   tggggctgcc acataaggaa ctcccctttt ctcattttgt ctccggtaag ttgttattga 131160
   tctgtacatg atgtttctct ttcttaccag ccgtgggaat gggccatcct ctggcatcac 131220
   tgttgccttg aacgttacca tcaccaccgt tgccatgaag tcatgacagc agcaccattg 131280
   gctctggttt attgggtgtc taccatggac agtagtaggg ctagacattt agtatactcc 131340
   agggctactg cggggattaa atgaggtatg aagcatctat aaccccacgg tccaataggg 131400
   tagccactag ccatgtgtgc ctactgagat atacatttta attaaagtta aataaagaaa 131460
   aaattcaatt tatcatgcac agtagccaca tttcaagtgc tcagtatgtg ctcatggctg 131520
   atgtagctgg cagtgaagat ggggaacatt gtcatcatca cagaaaattc ttccacagcg 131580
   ctgatctaga tagaacagga ttcagtccgc tgtggcttgt ggatccatgg actgaattca 131640
   tacacacaga attttgtggg aacacagctg tgcctgtttg tttatgcctt atctatggct 131700
   gcctttgcgc tacaaggatg gaattgagta gttgtgacag agaccatgtg gcctgcaaaa 131760
   cctaaaatac tcagtctctt atccttgaag aaaaagtttg tcagcctctg gtctagaaca 131820
   ttgcctggct cttggtaagg ttaaatacat gtttggaact ttttatttat tgaaaataat 131880
   atacattttg ttctccacct aaccttttaa ggtaagtatt gttatccctg tcctagagat 131940
   gagaaaactg ggactggaat tctgtactgt cacttccagg gggcaaacac ctcatgaggt 132000
   tttttttttt tttttggcag tcttgctctg tctcccaggc tggactgcag tgtcgcgatc 132060
   tcagctcact gcagcctctg cctcctgggg ttcaagtgat tctcctgctt cagcctcccc 132120
   agtagctggg attataggta cctgccccct ctccccgcta attcttgtat ttttagtaga 132180
   gatagggttt catcatgttg gccaggctgg tcttgaattc ctgacctcaa gtgatccacc 132240
   cgccttggcc tcccaaagtg ctgggattac aggtgtgagc taccatgcct ggtctcatga 132300
   gtcttgttca tcacttacct ccttttgttc ttccagcttc agtttcaggt tcagggggta 132360
   catgtacagg tttgttacat gggtaaattg catgtccctg gagtttggtg tactaatgat 132420
   ttcatcaccc aggtagtgag catagtacct gatagtagtt tttgatcctc accttccacc 132480
   ctcaaggagg tctcagtgtc tgttgttccc ttctttgtgt catgagtgcc tgatgtttag 132540
   ctcccactta taagtgagaa tatgcagcat ttggttttct gttcctgtat taactcactt 132600
   aggatagtgg cctccagctg cgtccatgtt gcagcaaaga gcatggtttc attctttttt 132660
   atgcctgcat agtattccat ggtgtttata taccacattt tctttatccc atgcaccgtg 132720
   gatgggcacc tccgttgatt tcttgtcttt gctattgcat cacttactct tgaatctgga 132780
   gcagtgccta gcacataata gacattcagg acatgtttgt tgagcaatga atggatgagt 132840
   taggaatcaa ttacatataa atcaataatc aataatcatc aatgatgaat ttctatgaac 132900
   aaatatgaaa attagatcgg ccgcattgta gagatgagga gacagccctg tagaaggaaa 132960
   gtgcctcatc aaaatgcaac atcgctgtga aagtgtagac ttggagctcc agctgaagtc 133020
   atctgtgtat tttctaagct gtcccctcta ccatcctgcc ttctccggga gctgccaaag 133080
   tgcagcgctg atgcctcctc tctctgtccc acagcctgac ttttgttcag ctcctgcttc 133140
   tcttcttcac caactgccag cttcagctgc tttgtggcac cctggggctt gaagcttcat 133200
   ttctggtgct gtggcaccac aaagctctgc cccagtgtca cttgagtcca agtagatcag 133260
   ccaagtttga atgatctctc tcttggccac ttcagtctat gaagctggag ttgagaccat 133320
   ctgtgatttt acacattcgt tacagcaaaa ccaagagggc catggggtaa gagggccatt 133380
   aagctagagg ctgagcaaaa ctctgctctt gaagcacctt ctgagatggc ttctcatgga 133440
   acgtctgcgc aaagtggctt cacatccaat taagtcaaag atgcattaaa tgaatccagt 133500
   ttgtaactgc ttccaaccat gaagtcacag ttccatctct tgccctggga ggggcttctt 133560
   tcaggctctg aggactggag ccctggagac tttattgcct tacggtttcc tcctctacca 133620
   tcagatgttt ctttttcatg tgttctggcc tggatttgaa tagactgcct ggatttgaat 133680
   gctgccttca cctctttctg gtgtgtaatc ttggacaagt taccttcttt ctcagtgcct 133740
   cagtttcctc ttcagtacaa taaggataat aataagacct accacatagc tgtagagagg 133800
   attaaaggag ccaatatgtg gaaagtattt acaacagtgt actgcatagt agcttttcta 133860
   taagtgatta ttattattat cccttcgaat ctaaaatccc tctagtttgt ttcagaggag 133920
   caaggattgt gtctcgcctg cctttaaatc cctttaagtt gggtgtctca agcccaacca 133980
   tctgcaggag actagaaggg gacttacatg agtgaattga atcaggtgtg gatttctgtg 134040
   aactagagaa tgcaaggctg tctgaaaggg gaactattcc ttattcatgc atcacatata 134100
   cactggtgtg ccaagccctg ttctgcatct ggggacacag cagagaatga accgggcaaa 134160
   attccctgcc cccttggagt tgatgttgta gtggactaag ctctgatgac tctgttgctg 134220
   catgagaatg tgggcccact gctgacaaaa cttctgattt ttctgtaaga aaagccggaa 134280
   acctggattt gtaaaaaccg tgaactctcc tagcttaaga attggcagtg gtttccattt 134340
   tttttttttt ttaaagcaca gtacatgctg tgaacccaag agccagtttg cagcccctgc 134400
   cttaagacac tcctctaatt gctctgggag gaggcctgtc tctgccctgc aaatcagcag 134460
   ctggtcggat ttcaaggtcg ttggtgtgga gggcgcctcc tccctctcct gcgtggaggt 134520
   tattgtggct ttttctgccc acagagcaga tgacgctgcc accaggcagt gagttagaat 134580
   attccttctc ttatccttcc ttttggatgg tggccttggc tgggttccca gccccctgac 134640
   agcagaagac aaaaggatgg aagcagcttc ctcatagtca cccatggggg cgatagggtg 134700
   gtgatggtga tgtggagaaa atgcattggg tcaggacagc cacctttgaa gccagaaaat 134760
   taatgatctc tgttgcctga atcttttcaa agtggagaag taatttaaaa gcttgtgtgg 134820
   gaaggattga gaatgggatt gagaaaagaa gtttaggttg ctatcaggaa caattcctaa 134880
   cagcaggagg tttttggaga ggcagtggtg aaattccttg gaaatttttg tgtggtgtct 134940
   tttttctttt tttctttctt tctttcctct tttttgtttt tgtttttctt tttttgagat 135000
   agagtctcat tctgtcacat aggctggagt gcaatggcac aaccccggcc cactgcaacc 135060
   tccacctccc aggttcaaga gattctcctg cctcagcctc ccgagtagct gggactacag 135120
   gcacccgcca ccatgcccag ctaatttttg tatgtttagt agagacaggg ttttgccttg 135180
   ttggccaggt tggccttgaa ctcctgacct caagtgaggt ccacctgccg tggccttcca 135240
   aagtgtttgg attataggcg tgagccactg cgccgggcct tttatggtgt ctttgaagtg 135300
   cttgtttaaa tttacatgtg gttgcctctc agtggggtgc atgctctctg gttttcccca 135360
   agccccacca ctccctgttg tgctttaccc acttgcctga cttagttatg cttactggcc 135420
   tggcctttgt gggtctttga gtttgtgcct tctgctctga aggaagcatg cttgagatga 135480
   tggaatctag gttcaaacct acctgcccta tctgcccttt actgacttgt gtgactgtgg 135540
   acaagttcct ccacccctga ggttgtcagg accctgcagg aaatagaacc attcaggtgg 135600
   ctgcaagaag aaagtttcat gaaaggacta tttagggaga cttgggcaag gttaagatta 135660
   caaataagga atgtggaagc acctagggcc ttgcaacagc aggaggcggt taccgtctgt 135720
   aaagaggaag aagcaagcgg agggaatagt ttggagccta ttgagagctg gggctaagaa 135780
   ggaaggcatc gccagcagga accatggtcc tggagggatg aaccgcttcc caaatagcag 135840
   taccgaggca gggaggaaga gggggaagaa acaccccagc ttctcttcac ctctcccatt 135900
   gcctgttggc ctcacccact ggctgaaccc agctggaaac cagaaggacc agatgatgaa 135960
   ttctgtaagg gtcagccttt tggggctcag ctcacagcag aatcagagag aaaaaaatgg 136020
   atctgagagg tgaaacaagg aacaagcagc acgtaactct cagcttccct accttgaaag 136080
   tggaggagaa tgagataact gaccttggaa ggttattggg tggctcatgt actctcgaag 136140
   ggaagggcct ggtcaatatt aagtactcat aaatgctgag attcagtgtg ggtttcaaac 136200
   cgtttctagg agctaaaggt cctgagaaag ggcttcagtt tcagtatcaa gagacattct 136260
   caggtgggca gatctcttga agccaggagt tcgagaccag cctgcccatc atggtgagac 136320
   cccatctcta ctaaaaatac aaaaattagc tggatgtggt ggtgggtgcc tgtaatccaa 136380
   gctactcagg aggctgaggc aagtgaattg cttgaacctg ggaggcggag gttgcagtga 136440
   gctgagatcg cgccactgca ctccagcctg ggcgacaggg tgagaccctg tctcaaaaac 136500
   caaaaccaaa aagacattct cctccttccc ctgcagtggg gcccactggg aatggttgca 136560
   tcttccatgg ggagtttaaa gtgaatgtgt agtggctaag agcaaagacc ctagaaccaa 136620
   gtaggtggac actgtggtag tccatcagtg tggtagacac tgatctttct gttactatag 136680
   attaatggcc ttgtacagta tgcactattt tgtgtctgcc ttctttcact taaaataatg 136740
   tttttgagac tcacgcatgc atgtgcctgt attggagtct attgagagct ggtttccagt 136800
   cctgattgcc actgacagtc aagttactta acttttcatg ccttattttc tcatctgtaa 136860
   aatgaggatg ttagtagttt ctacctcatt gggttctgag gattaaaatg agttaatcta 136920
   cgtaaagagc ttaacacagt gtctaacaca tagtagacaa tcaatagatg ttagctattg 136980
   taattgccat catcgtcatc atcatcatca ttatcatcat catcgtcacc atctccatgg 137040
   atcagctcaa atcaaaactg acccattgtc aatagaaaaa ttctctgtgg gatgaagtcc 137100
   atgccaccag gcctctcggg aacttcattg atcataccat tatcttctca tcctcccaca 137160
   gatatgatat ttcagggagt gattgatagg tgtgtgtgtg tgtacgtggg gaagggtggg 137220
   cttgtgcctg actccaggga cagagtgctt tgttatgcag aatagcttgc ctgtcatccc 137280
   actacaggcc ccttccttga aaagttccag gctatttttg agccctcatg gctcacatgt 137340
   tcctttctac atcatgtcaa tgccagtaca gaaaaattga aaccttttgg cagcatctaa 137400
   agcaaaagag atttcaaagt gcatggagga tgagtgttgc tcaaatgaca gggtattaaa 137460
   acgtgacaag aaggctgagt taagagactg caggcagctt gtgtgtttgt atttggacct 137520
   tgctggattt cttgggaaat cagtggagtt gtccaaggtt aatgtcatct ccaagaaggc 137580
   accaaagggt agtaacactc cttcaccctc atttgtacat acactctgca agagcggaag 137640
   atgatgcagc cagtggagtg ggcggttgtc ccagagcacc acctgggctc tgagctacag 137700
   gattctgcag gggaaagcac ctgaggacag agtgaggtgg gttggtgcca cctgggagct 137760
   ccaggctttt gtgtgctgat gtgcaggcag atactggagc cagaccgaat tgtgtgcgaa 137820
   atgtgggtct aagagtgaag gcaccaaacc ctcacttttt ttactttcat tcagccttcc 137880
   catttttctt caaagggcag aatatgactt tggagtcagg tagacctgaa ttcaaatccc 137940
   tcttcttctt cagtgagtag ctaaatcttt ccaatgcttt atttaaaagg aatcacgatc 138000
   acactttcag ggttgttgag aggtttagag attgtggtgt tacagggccc ctggggctca 138060
   aaaggtgttt gctgaacccc tgtcttgtgt taaatagtca caatagtccc aaggggagga 138120
   tgaacaggta gcactgccat ttcataaaca ggtgactgag gtgcagagtg gcttatccat 138180
   aatgacacag gaggtgaatg ctggagctgg attgaaggct gagtcattgg actctcctct 138240
   gagggaggca gtacagagta gtggtcaggg cttgaccaac ctctagagtc catgggcaga 138300
   ttttctgggc ctctggctta gttttcctca tgcataaatg gaatgtgggg attatagtag 138360
   atgtactttc tatggttgtg aaggttacat atacatgaga taattctata aattcttagc 138420
   atgtgcttga gttacagtaa atatgcaatg aagtattcac tactgtttta gtattatttt 138480
   tattagaagc cgtggcagta gttaccatca ttgtcaccat ctccatcgat cagctctaat 138540
   caaaactgac ccattgtcaa cagaaaaatt ccctgtggat gaagtacatg ttgtaggtgt 138600
   agcgggcaat cgtgatcgcc atcagaaatt catattgggg tttggtttac aagtgttggc 138660
   ctctgggagt gtttcctacg tgctccttct ctcctcccct ttgaggacgg atggtgtgat 138720
   ggttcctctc catttggtcc ctcctctggg accagcattc tcctgccttc tttgtcctac 138780
   tctgtgtccc aggaggtaga cccctgtgga ctgtatctcc tggactccct tgctggttga 138840
   cttttgtctg agttaagcca aagggaggtg gcagcaagag acgagaaggt gggaggagag 138900
   agagtttggg gtatttccac ctgcagctcc ctctgtccat ctctggcccc gctctgtgat 138960
   gctagccccc gcgaggttgc ccatctctca gggtttcctc tcttacttgc ccctgttacg 139020
   tcatttcatg ggactatacc tttagcttta ggtgcagaac agttttccac agttaaatgt 139080
   ctttaattga acatctagaa tgcaaaacat attataagat aatgtgttgg attgttttcc 139140
   tttaaaaaat ggtacaacca ctttgcaaaa caatggcagt ttcttattaa gttaaacata 139200
   gagttacagt atgatacagc aattccactc ctaggtattt atccaagagg aattaaattg 139260
   tatgcccaca caaaaaatga atgaataaat taagacttat aaatgaatgt tcatagcagc 139320
   tttattcata ataaccagat actgttaaaa aaaccccaga atattgatca gtagatgaat 139380
   ggataagcaa actttagaat aatcatataa tggaatatta ttcagcagta aagatgaact 139440
   aactgctaat acagacacgt gcatgcgcga gtctcaaaaa cattatttta agtgaaagaa 139500
   ggcagacaca aaatagtgca tactgtacga ggccattaat ctatatagta acagaaagtg 139560
   gatcagtgtc taccacaggc tgggggtgtg tacagggaaa ctttttaggt ggtgcccatg 139620
   ctctgtatct caattttggt ggtacatgcg tataatacat ttgtattgtc ttgtatcaaa 139680
   gtatgcctca tgaagttcag gtatatgaaa atttgtattt gctttattat atttaaatat 139740
   cagagaatgc aaaattgtaa aaagaaaaag aaatcctgaa agtcctttta actagagaca 139800
   accaccacca tccacatttt atctatttcc tttcagtttt taattttttc ctgtacttag 139860
   gttttcccct tcactgtctt atttaaataa aaaagaaagc aaatggaaag tctcccatac 139920
   gaaggccttt ttgctatgag tttaccttgt ctaagggcat ctagcatgat ggtgagaagg 139980
   ttttgtcttg tataactaaa atgtattggg aggaaatgaa aatgtttagc ctggtgaagg 140040
   ggaaacattg gggagaaaag tgagggagag tgtggtctac agtcgtgagc tttaaatcac 140100
   aaccaatcag ccaattaaat actgagctcc tgttatgtgt cagtattagg aagacagtag 140160
   tgaacaaaac atatggctcc tctgatttaa tggagttaac agtctgggag ggaaagatat 140220
   ttttattatt tgcaacaaca atagtaatag ctaatattta tgatgagcta gtgcctatta 140280
   tggtgttaag gtttagtttt ttcttttagt gtttttaatg tgttgtcaca tttaatccca 140340
   gcaactccat gagtaggtac tcttaattct catttcatgg ttgaaaataa taaagtggct 140400
   tggagatgtt aagtcaaatg gggtcataca gtgagcatgt taaacctaga ctggcaggtc 140460
   aggacggaag tgatatttaa tcctcagtga aatgattttt ttctttttaa tttttatttt 140520
   tattgagaca gaatctcact ctgttgccca ggctggagtg cagtggcaca atcttggctc 140580
   actgcaacct tcacctcccg ggttcaagca attctcctgc atcagcctcc caagtagctg 140640
   ggattacaag cacccgccac cacgcccggc taatttttgt atttttagta gagatgggga 140700
   tttcaccatg ttagccaggc tggtttctaa ctcctgacct caggtgattc acctgcctcg 140760
   gcctctcaaa gtgctgggat tacaggcgtg agccaccggg cccggcctct ttcagtgttt 140820
   ttaatgtgtt gtcacattta atcccagcaa ctctatgagt aggtactctt tttattccta 140880
   ctttatggtt gaaaataaca aagtgacttg gagacattaa gtcaaatggg aacatacagt 140940
   gagcatgtta aacctagatt ggcacgtcag gacagaagtg atatttaatc cccaagaaaa 141000
   taatatattt tttttctttt tttgagacag ggtctcactc tgttgcccag gctggagtgc 141060
   attggcatga tcacggctca ctgcagcctc gacctcctgg gctcaagtga tcctcccacc 141120
   ccagctacct gggtacaggc atgcaccacc ttgccccgct actgttttgt gtttttgcag 141180
   agatagggtt ttgctgtgtt gcccaggctg gtctcaaaca cctgggctca agcaattcac 141240
   ctttcttggc ctcccaaagt gctgggatta caggcgtaag gcactctgac cagctggaaa 141300
   tgatatttaa tctgatatct gaaggatgat aggagttaaa caagaatcaa gtgtcagaga 141360
   ccagagatat gaggtctcat atatatatat ttgctcaggg cagggggctg gttttgggtg 141420
   ataattaggg gctagactga acagaattag tgtttggaag tgagtggtgg tggtggtgag 141480
   ggaggagaga agggtgagtc ccagggtaag ggatgcaggc acatcctgag aaatgaggca 141540
   gaagaatgag aacttggctt ggggcatgtt ctgttcatct gaatggtggt gctgaggaga 141600
   caggtggaca gagaggtgtg gggttcaggg aggagcacag gactgagagg gaccagcagg 141660
   aatagaattc ctgaagtccc ttggattgac aagatgcttc agtgagagag agtccacagg 141720
   gtagagatca gcacctagga cagagcctcg agaacttcct ctaggatagg gaagggctgg 141780
   agaacaggag gaggagccaa ggagaggagg aaggggccaa gagggcagga tcaggactga 141840
   ggcaggccga cttctgcgca gttgaagaac tctaatgtca gagctgtcct taggaggcag 141900
   agaacccctc gtcctggaag agtgcaggga gctgcttggt gaccatcctt tggggatgtc 141960
   atggaaaaga tatttgcatc aatctggggt gagattaggt gtctggattg gttcaggttc 142020
   ttttgtcatt tgtaagcaca caaaagaact ctagttatct aaaggaaaaa aaaaactgga 142080
   aatattatgg gttggcacac gagtggtgcc aggcctcaga gagtacaggg cccagggtgg 142140
   ctctggggat ctgggtggca ggagcgaatg gatgaccttc acagggtgac accatcggga 142200
   ggaatcagct ccagctgttt tctgtccttg tcccaagaat caaatcccag ggagaacagt 142260
   gtgattcccc aatgttgtga ggttcagtca tgtgcaaggg gaaagccggg cacctcgatt 142320
   gacagcccca ccaggactgc ataccgtgga gaaaggatgg cttcccaaag aaaaacaaca 142380
   ttccagctac cagagaaaag ggagtggacg ctgggcaggc aaagccaaca gacacttttt 142440
   gtagtctcca tggtcctgtg tatgattcag tgatttttgc agtattcact tagcaagtca 142500
   ttcctcgttg cagaattgtt gctagtgttt ttcctggctg gggattaagc tttttccatt 142560
   tgcctatttt agagtgagtt gggggcctgg gattgacacg catttgtctc tgggcttcaa 142620
   ggtagcccag agattatgcc acctcaggag gcacaatttg gtggctgtca ctccttgtca 142680
   tgtcaacaaa gggtgtggct tgtggctctg tggcctctgc tttaccagca aggggaggat 142740
   gctggtagta gcaaagggtc taaatcgctg ttgcatcagc gaggtggatg tgcctatcct 142800
   aaaggcacct actgagaaga tgcccaagta acagacactc atgggcacag tagacctgtc 142860
   aaacctcata gttcctggct gactctcttt gggtgacctg agtcacacgc ccacgcctga 142920
   acacccactg aaactcctag gtgcattacg tcttctgcaa tatctaaagg aattaaggga 142980
   aggatgaaac tctgtatctc aactttcctg aattcaccag tatggactga tggaattgag 143040
   tgctagtaac accaagctcc taaataataa taataaaggc ttacttatag gaacgtacga 143100
   agcacaagag aaataagggc ccccacggcc cacccttcaa cttctcacat ttggtttgtt 143160
   agtcaggact cctttggtca caagagacaa aaaaaacaat tcaagttggt ttaggaaaag 143220
   aggcatttat tggcttgtac agaggagttg agagttcttt aggtctggct ggatttaggt 143280
   gctccagcat ggtcatgggg catctcttgg ctcagctttt ctctgtgttg gcttcattct 143340
   caaaaaaact tttccttggt gatggtaaag atggcagatg cagcactttg ggagcctgag 143400
   gcaagaagac cacttgaggc caggagttcg aaaccagcct ggtcaaccta gtgagacccc 143460
   atctctacaa aagaaaaaaa aattcaggtg taatggtgtg cacctgtagt agctactagg 143520
   gatgttaagt gggagttgca tgaatccagg tgctcaaggt tgcagtgagc cataatcgca 143580
   ccattgcact ccaccttggg tgacagagtg agattctgtc tcaaaaaaac ccacaaaaac 143640
   cgccccccca aaaacggatg gcagatgcaa atttattccc taccagttta acaactctgc 143700
   tggaaaataa accctctttc ctaataattc cagcaaaagt cctgaagctg attctctttg 143760
   ggtgtcctga gtcacacgcc catgcctgaa tccccactgt ggcccaggtc ccatgtccac 143820
   ccttagagcc agaggatgag ggcagcctta gctggcccac acaatcagtg agccagtgaa 143880
   gagcgattcc tcacaggaga agcatggagg atgggtgctg tcaggcagaa acaagacatg 143940
   ccaccacact tggttttggg gttaggcctg cttctggtct gagcaatctg ctgctggata 144000
   tggctggtga gtgaagagat attgatgtgt catcccctta gaccatggca ttgtgtcctg 144060
   ctctctgcat ctttgggggc ctgatcaaaa gcgggaagtc atggggtcaa ggacagtctc 144120
   cacgtgccac ccaaacagga aggatgtccc cagcaggtgt tctgctcagg gtctggctct 144180
   cccagggttg ttactgcaga gggaagggta ggggcagctt tcttctggtg gatttattta 144240
   cttctcaggc ctgttctttt cctcatgttg gctcaactgt tttatcttgc ttagttaaat 144300
   aaatttggta acattataaa ggaaaacagt cttttccggc agttgctgac cactgcaaaa 144360
   ttactgctct ttcttttgtt tttccttttt agtgctgggc tgtgaataag caaatgaagt 144420
   ttgctatctc ttcctcccct cttccaggaa gaaataatca ccatcagaac tttaagagtt 144480
   tcccataaga ctgggcaaat tagaggaagg acctctatac ggtggaacag atgcaattgt 144540
   gcaaacagaa tggggttatt ctctacatat ggagatggaa cagaccctcc cagatgtgtt 144600
   agtgtgggga aaagcaggtg gtggaggtgc gttcagagta gcagtgttca gctctcccaa 144660
   gcacattcaa atcacccagg agcttttaaa acgtactgat gcctgcccac caccctctcc 144720
   tcacagcccc attaaatcag aatctctggg agtgggactt gagcactttt cattttaaaa 144780
   gctccctggt gactcctggg gagccaggac tgagaactag tgctgtagaa ttgtatatgg 144840
   gcaaaacagc agggatctag ataaaaaaag aaaactgaag tagggaatat ctcagtccgt 144900
   acctacagat agcctcacac atgtggtcgc gcatacagag aacgtctcta gaaggataca 144960
   caggagactg gaaagaacgg ttctctccag tgatgaagta cagtggtgat tctcaactgt 145020
   atactcgttt gtaatgttgg aattttgaag ccacaagcat aaatgctcac tgtgtactga 145080
   aagctggcca gacagggctg taagatgacc cctgaactct agcttgtctg tgttggggcc 145140
   ggctgcccac agattcacac aaaccatctc cctgcccgtg gtctctgctt gccctcctct 145200
   actcagctgc aatgagtggt ttccgagtgt gggagagacc agaagagctc attcattcat 145260
   tcattcgctc acatttttag taagaattag gtataagcca ggcacgtggt aggcacgtga 145320
   atctgatctc accttcacaa ccgtatgtga aattggtggt cttatctcca ttctaagatt 145380
   ggggatattt taacacagaa attcatacaa ccaagctgta aatatttgag gccgaatgac 145440
   cagctaagag agcttataaa ctggcagcac ttagatgtgt ttggatggcc tgtacagaac 145500
   tttttaaaaa aatcctattt cacgactctg gaccagtgaa cccagggcca ctgttggcta 145560
   gagctgagtg tcccttggaa gggcatatgc ccctcagttc agcgcagtcc ccccatatcc 145620
   cttcccttct gtgtcacacc agccttgcat cactcagtca caggcctagc ctctgcaggc 145680
   atttgcgttt gtcacccctg tgttaaggta ggtaggatga gtgaactgtc gtctgtcctg 145740
   cagggatgct gagctgaagt gtcctgtgga atgtgccatt tctattccaa tctggccctc 145800
   tgtctgctgg ctggaaaacc aagctccttt gctttaagtt tcatttgtgg actgcatggg 145860
   atgggctgtg ggtattgatt tgtttgtgga aaagatgtgc acaataggga atgtgtaaaa 145920
   tacatcatgg aaatggatgg ttaatgccgc ctggctggag tgtggggcag tggagtgaca 145980
   agagatgaga gtcaagttag atggtggggt atactgtggg tagatgccgg gctaagcatt 146040
   ttgaacttta acctttaggc tactgctgtc caataagact ttctatggtg ctggaaattt 146100
   cttatatctg tactatccaa tgttgtctcc agacgcatgt ggcatcaagc acttgaaaag 146160
   tagctggcat gaatgaagat ttttattggc attttagatt ttatttaatt aatttacttt 146220
   tacatagctc catgtggctg gtagctacca cactgggcgc tgcagattta ggcaatagca 146280
   gtggatgtgt tggagcaggg aaataatatc aaagctgaat cttcagaagg tgatttttcc 146340
   aggagtgtac aggccgcatg gggtgagcag aagtaggtag ggataatatt gacacttctt 146400
   agcatctatt aggcacttct tgtgtccaca cactgtgcta agataactca cttattcctc 146460
   ataacaactc tatgaggaaa gtactatcat tgtccctatt ttatagacta gaaaatgaga 146520
   cacagcaaag acaatgggca tattcaaggt cacacagcta atcaacagtg gcacacggtg 146580
   tagaatccag gcagctcagc tgtagagctt gtaccatgaa tccttccatc ctggaaggaa 146640
   ataaatgaac cttcattgag aatatacctt ctgctgtccc ctggtgctgg gcattcacac 146700
   gtaaaatctc atttagattt ttgttaacac tcatgtgcca ttcggtggat ttagatcctt 146760
   tgtgtttatg gaaacttggg ctcataggga tgagatatca aggcaactgc aatctctgtg 146820
   gacaccagtt aaggctttat tgtaaaattc catgcctgtg ggggaaatca caggctgtgg 146880
   gagtcggggg gaatgtacag gaagatatgg cataaaaggt gttgcgaagg aagaatcagt 146940
   gggatttagc aactgactga atggcggggg tgaggaaagc gggggaggct ttctcctgga 147000
   acagtgagat gaatgacaaa cacacatgta actgcactgt gtgagttgag agtgtgggct 147060
   ctagaggaag acaaactggt ttcaaataca agtgttaaca cggactagct gaacaacctt 147120
   gggcaaatta cttctctccg agcatcagtg tccctgttaa tataacgagg accgaaggca 147180
   ggaggatcac ttgaggtcag gagtttgaga ccagcctggg caacatagtg agatcccgtc 147240
   tctagaaaaa acttaaaaaa ttagccaggc atggtgatgc atgcctgtag tcccatctac 147300
   tcaggaggct gaggccagag gatcacttga gcccaggagt ttgcggctac agtgagctgt 147360
   gattgcacca ttgcactcca gcctgggtga cagagcaaga ctttgtctct ataaaagata 147420
   taatgagaat catagcacct tcctcacagg actgtgatga ggctcaggtt aagcacggat 147480
   atgaaagccc tttgcaaact caatagcatg accctgtgag tcactcttct tagtgctggt 147540
   gagaccagcc attgggcaga aatggtgacc agaactttct gtctttgatc atggaaacag 147600
   ttcgggaaat tgcctggggg aaccccactt catcttaggt acttggtaga gtttaatgcg 147660
   gataagtctg tgggtcagct ttgacctagg aggaaagagt ggttctccca gcagctgtag 147720
   agcaaaatct gcatgccagt ggctccaact taaatgccct tttccaccaa acagtttgca 147780
   tgtgttgtct tatttgagct gcacaacaac cttatataca taggtactgt gataaaataa 147840
   ggctcagaga ggtggagaca ctgagctaat gtccttcagc ttataggtag taaaatgagt 147900
   gttcaaacga aggtcctcct gcttccagaa acttagcctg taattcctgt aggtgatggg 147960
   cttctcattt cttctcctac cacagatatc tgcaactagc gctagaattt ctatcccaga 148020
   ttgacatgga tttaagggta aaggttgctt cctaagaatg tcagttttcc actatagacc 148080
   ccagtaagaa ttgtgtacaa gaggggaatc gactccaaac agaatatata tatatatatg 148140
   atttaattta gtcctctttg gaatgcaatg agtaaatttt agtggttaga gtaaaccact 148200
   aaaatttggg agttaggggt gaagtgcagt gtgaagcaga gttgagatct cagtgctttt 148260
   cctggggcag aaacatccga gagggcccct gtcacccaag cttttggcta cagtctactc 148320
   cttccatctg ggattaacca ggcgtttctt atccttgctg atctagtgtt tcttattgct 148380
   gctgtaacaa attaccacaa atttcctggc ctaaaatggc acacatttat tatcttatac 148440
   ttctagatgt caacattctg aacagcaggt cttccggggc tgaaatcgag gtgtcagcag 148500
   ggctgccttc cttctggagg ctctagggga caatctgttt cttacctttt ctagaggcca 148560
   cccacattcc ttggcttgcg ttcccttcca gaaatggcac aattccaacc tctgcttcca 148620
   tcatcacgtc tcatcctctg actccgagtc tcttccctcc ctcctataag gacccctgtg 148680
   atcacactgg gctcatccag ataatctagt ataaccccct ttctcaagat ccttaattta 148740
   atcacatctg caaaatcaat tttgtcatgt aagtaatata ttcacaggtc ccaggattag 148800
   gacacggata tctggtggtg gtgtcagggg gtcgggggta ggggatggga acattattct 148860
   gcctacagag ctggcaggtg gcctttcttc tttcctttct tttcctttcc tttccttccc 148920
   ttcccttccc tttcctttcc ctttcccttc ccttttttcc ctttcttttc tccttccctt 148980
   ccttttttcc ctttctcttc cccttcccct tcccttcccc ttcccttccc cttccctttc 149040
   ccttcccctt cccttcccct tcccttcccc ttcccttccc tttcccttcc ccttcccttc 149100
   cccttccctt ccccttccct tccccttccc ttccccttcc cttccccttc cccttccctt 149160
   cccttccctt ccccttcccc ttcccctccc cttccccttc ccttcccctt cccctcccct 149220
   tccccttccc ttccccttcc ccttcccttc ccttcccctt cccttccctt tcccttcccc 149280
   ttcactttcc cttccccttc ccttcccttt cccttccttt tcccttccac ttcccctttc 149340
   ccttccactt ccctttccct tcaccttccc cttccctttc ccttcccctt ctctttccct 149400
   tccctttccc ttccctttcc ttttcccttc ccttcccttt cccttcccct tcccttccct 149460
   ttcccttccc tttcccttcc ccttcccttt cccttccctt tccctttccc ttcctcttcc 149520
   cttccccttc ccttcccctt cccttcgcct tcccttcccc ttcccttccc cttcccttcc 149580
   ccttcccttc ccttcccttt cccttctcct tcccttcccc ttcccttccc tttcccttcc 149640
   ctttcccttc cccttccctt tccctttccc ttccctttcc cttccccttc cctttccctt 149700
   gcctttccct tccccttcgc ttctgcttcc cttccccttc tcttctcttt tctttttttt 149760
   ctggagtcag ggtctcattc tgttgcccag gctggagtgc agtggtgcta acacagctca 149820
   ctacagcctt gatttcccag cctcccgggt tcaaccaatt ccccccacct cagcttccca 149880
   agtagctggg accacaggtg cccaccacca agcctggcta attttaaaaa ttttttagag 149940
   atggagtctt gccatgttgc ccaggatggt cttgaactcc tgggctcaag caatcctcct 150000
   gcctcgacct cccaaagtgc taggattata ggtgtgagcc actgcactca gctgcaggtg 150060
   gcctttctga tcttgcctgt aataagggag ggtgggaggt ggatagagcc ggagacatct 150120
   ttttcattct ctgctggagc tgagtagcaa tttccccttt taaacaggcc atgtgtgcag 150180
   tcccctacct ggccgtcaga cattttcatt gctcacctgg cccctaggca tttgagtttc 150240
   agaccccatt ttgtagttct agtaaagcct tgctatagac tggggtcaga gggctgcagg 150300
   ctttcaacct ttgtagagca aggggttatt gaataaatta tgaagcatct ggagattctg 150360
   ggctcttaac tgggtacctg tttgaaagta gggtctagta catttttttc agtagctcag 150420
   taaatgttgc ctcttcttaa aaaaaaaaaa aacagaaaac aaaaactggt cttactctga 150480
   atttgccaaa tagaaatgag gggcgtttta tttgtgtcac aaaaggattt gatttcttta 150540
   aataggcagc cctactattt tcttcaaagt gttttagtta atgggaaatt tgatttgcac 150600
   aacttttcag aaacacactg tgtaaaggaa ttacctgcac cagaagctca tgaggttttt 150660
   caaagtctgt tctcagcttc tctgcacatc atgctggggt tcacttggcc tattgattct 150720
   gcctcagtgg ggatggacta ttcatttctg tgggtaattg gaagcctacc taaagtgacc 150780
   ttgtcaatgg ttggattgac aaatatccag gctggtggga tgtcaggcga tcatccagga 150840
   gctacatcta ccatgaagtt ggttatgttt tctccttttt ctatgactga tgatattcat 150900
   tttttggttc attcattcac caaacattta tttactgaat atcaattatg taccaggaat 150960
   tgtggtagac actaggagac tagagatgag ctatctttat ccccttctgt ctccaaaggg 151020
   gataaagatg aataagataa taattgattt ttttttttga gacagagtct cgctctgtct 151080
   cttaggctgg agtgcagtgg cgtgatctcg gctcactgca gcctctgcct ccctggttcc 151140
   agtgattctc ctgcctcagt ctcctgggta gctgggatta ccggggcctg ccaccacact 151200
   ttgctaattt ttgtattttt tgtagagaat aactgatatt ttagatgttt ggaagaaagg 151260
   ttgtagaata aagcacctac ttgaaaatgg gggaaactac tttcctaaat atttctgggt 151320
   caaagaataa attaaaacca tactaataga ccacttagaa acttgataca atgatgggac 151380
   taaatataaa acataggatg cagccaaaac tgtacttaaa agagggtttt tagccataac 151440
   atctttaaat gtttaatgaa gaggaatggg aataaattaa ttgagcagtc aattcaagaa 151500
   gctttaaaaa gaacagtgga taatattagc tgacatttgt agaatagtca tgatatggtt 151560
   ggcatggttc tgggcatgga ataactcatt tagttctgac aacaccactg tgatatgggt 151620
   cctattgttg ctgtcatttt ccagagagga aactgaagta caggaagatg aggtagtgta 151680
   cctagtctta gagtttgcaa atgatagaca caggatatac acctgatcat cagactccag 151740
   aattcacatt cccaaccaat aagtaataca aatacaagta agcaatacaa tcacagacac 151800
   acagaacaaa aaaagttaaa gtagcaatta ataaagtagg ctaaaaccag gtagagttga 151860
   taaatataat cataatctgc ttctttaaca tgatagaaag ataaaaatag acaagaaaga 151920
   acacaaactt ctggaaaaaa aataacgaga gactacctaa caatgcaata tgaagaaaga 151980
   aacaaagaaa gagaaaaggt gtaatatcat cccagataag atgctacaat ttaaacacgt 152040
   tgagaagaac actgtggata atttccaagg aaaatataaa ttccaaaagt ttgtttgagt 152100
   agaggtgaaa aactttcctg tgtggttgaa gtgtagagag ggagagggag gtgaagctgg 152160
   ggaaagctca gggatggggc gagactatgc tgtcatccct ttactttcat gttgaataaa 152220
   agggaaagtt aaacatggaa ctacgacagg gggaaaagag agaataataa aaatattatt 152280
   tggggtaata cggttttcta tctggaaaac tcaaaggaag caactaaaac tccacaaaag 152340
   ctatttgagt ttttttaaaa aatgctttaa aataaatatg ctaacaatct attgctttcc 152400
   tattgttttt gtttgtttgt ttgttttgtg ttttgagatg gagtctcgct ctgttgccca 152460
   ggctggagtg cagtggcatg atattggctc acttcaacct ccgcctcctg ggttcaagca 152520
   atctactgcc tcagccttcc aagtacctgg gactactaca ggcacctacc accacgccca 152580
   gctaattttt tttgtatttt taatagagac ggggtttcac catgttggcc aggctggtct 152640
   cgaacttctg gcctcaagtg atctgcctgc ctcagtctcc caaagtgcta ggattacagg 152700
   catgaaccac tgcgcacagc tatttgcttt cctattttta aacaatgacc agttgggaaa 152760
   caaaatgaag acatgtcaaa tttacaatag cataaaaaag aatcccttag atgatgctaa 152820
   agggaaaatg tgtaggactt atatggagaa aataagagtt caacaagggg acaatcattt 152880
   cctgatggaa agactcattg ttgtcaaggc atcaattatc ttgaatttaa tttctaaatt 152940
   taacacaatt tcaatcaaag tcccaatcat tttttggagg ctgagggttg actgaataca 153000
   atcccaataa aaattccatt ggtattttta aaatcttgat aaactgattc taaagtttac 153060
   ttggaagaat aaatatgcag tgatagccaa gaaaatgttg aaaataagga taatgagggt 153120
   ggacttgccc tgccaagata taacatatta cagagctact ataattaaag cccatattgc 153180
   tatactagaa gacagatcag tggcacagaa tagaaggccc aaagacagac cccagtatat 153240
   ataattatta aacatataat aaagttggca tttgaaatca gtggggaagg gatgaattat 153300
   tcaataaagg atgctgggac aattggttac ctctttggaa aaaaatggca aattggattc 153360
   ccatttcaca tcagattcca agaaattaga catggatcaa agagtttaaa gtggaaatga 153420
   tgccatataa aagtacaaaa aaactccaaa gagtaatatt tatatgacct tggagcatga 153480
   agaaggctgt ttgaggtgta cttggactgt ctttctttgt ccagttttcc cttttagtgg 153540
   tttaaaagga atacatagta gtttttatat ttttatttat ttatgtattt atttttgaaa 153600
   cagggtctca ttaggtcacc caggctggag tgcagtggtg cagtcatggc tcactgcagc 153660
   cccaacttcc tgggctcaag cgatcctccc acctcagcct ccggagtagc tggactgcag 153720
   gcatgcacca ccatgtctgg ctaatctttt attttttgta gagatggggt ctcactatgt 153780
   tgcccaggtt ggtcttaaac tcctgggctc aagtgatcct cctgccttgg tgtcccaaag 153840
   tgctgggatt acagatgtga gccaccacac ccggtttcta ttcttatttt taatacttaa 153900
   ttgttaacac gtatatttaa acttataatg tcctatatgc aatttctcaa tatcaataat 153960
   cttttgagga atataagact cagcacattt tcatcttgta accctcctgc aactcccatt 154020
   tcttatgact tgggttttct ctacttctct atctcttcct ctgttgaaaa cttttcatat 154080
   tatggaaaaa attaaacata tataaaagta acattagtat aatgcatcct tgtgtatgca 154140
   tcacccagtt caataatgac ccatcatgat caatttcttt ttcatctgta atcctcccct 154200
   ttctctcccc accttggcat actgaattat tttaaattat tttatccata aagatgttaa 154260
   tgtatcctca aagataagga ttctataagc aaagaaaaac aagaccatta tcacaccaaa 154320
   tatttttcat tccctaatat cagtaacaat aattccctta tgtcatcaga aagtgagtat 154380
   atccatttcc ctgattgcct tacagttttt taaactgttg gtttttttcc acttagaatc 154440
   caaacaaggt ccacatgtta aatttagttg atatgggttg ataggtgcag caaaccacca 154500
   tggcacatgt atacctgtgt aacaaacctg aatgttctgc acgtgtatcc cagaatttaa 154560
   agtaaaatta aaaaaattta gttgatatgt ttcttaaatt cctttgaaaa tatacgtttc 154620
   tcgtcctatt ttctccccac ttgcagtcct ctcttgtagt ctatttgttg aagataccat 154680
   attctttgcc ttgtagtttt ctacattctg ggtatttggt tggggtgggg gggggtgtgt 154740
   ttgctgatcg catccctgtg gtgtcattta atatgttcct ctgtcacttg tattttctgt 154800
   aaatctgtat ttgcatcaag agacttgaac caatcatatt tgattatttg acaagtgtac 154860
   ttcataggta gtgaagtgag ctttcctctg taggcaagtg gtatcttttt gtgaccttag 154920
   caaccataga tgaatgtcac ctagactcat tgtgtcacta gggatggcct tgaggtttcc 154980
   tgttctattt tcatttacta agcatgcact ttagaaaggc tctgagctgc tccctgaagc 155040
   atggcacccg tcctccagaa acccgcagtc tagaggttgt aaaagaagat ttgaacacca 155100
   agactgtaaa agatgatttg aacaacaaca acaacaacaa agcagtttta ttttgtatac 155160
   attctttcat tgtacctccc ccagagggat ataacctcca aataggggag ccataccata 155220
   gatgcattaa attcacacca attcaaatgt gtgtatatat gtatttgctt gcagactact 155280
   aaagtgacaa tgtaaacgtt gctgcctatt tttcccactc ttctattaga ttgacgttgc 155340
   ttttggtgag atggggcatg agtccaccat cctgtcccct ctgtttccac atgaccctca 155400
   tcgtatgaac acctctctta agtgtgattc taggatttaa agatacatat ttcttatgca 155460
   ttatgcacct aaacacaagc caactacttc atctggcacc tgatccaaga aacaacattc 155520
   tgttccaaaa tcaatagaag acaaggcact ttaagggatc ttcaagggag tgtaattttg 155580
   agcatatgga atctttctaa ccctagctaa ctttagaagt ttaactccta tgcaagatga 155640
   agcatctatg ttcccaagag tttgacttca gcaaaggaca agctgcagaa gctgacatta 155700
   ttgtttattt tggcataaag gctttacttg ttggtgaagt tcaagtttaa tgatctcttc 155760
   ctccctttct ctctcttcct cctctccgct ttccttcttc ccttcctccc accctgattt 155820
   tctcttttgc agtttgctgc tttgtggtgc acaagcggtg ccatgaattt gtcacattct 155880
   cctgccctgg cgctgacaag ggtccagcct ccgatgtaag taatgggcat cgattgcttt 155940
   tctctgtcca cagtcaatgc tgccttgtga ttaaatgtga gtgagcattc ttagacgagt 156000
   aagtgtggac gatctcactc taaggcatgt ggtggtccct atagcttttg agacagtttt 156060
   ccttttagtg gaaaacaaaa ttttggaggg agtgtggctg gataggaagt cagctagttt 156120
   gtctactatg attttgctaa tgtcttcact ttgagagctg gagcacggag ggtgagaggc 156180
   aggggcctac ttggtagtgt ggctgcaaaa aggtagtttc ggtggtactt tgcagtgcac 156240
   gttatggcac tttgttaggg tgttctttct tttgttattg ttattattat ttttattatt 156300
   tttcagatgg agtcttgctc tatcgcccag gctggagtgc agtggagcaa tctcagctca 156360
   ctgcaagctc cgcctcctgg gttcatgcca ttctcctgcc tcagcctccc gaggagctgg 156420
   gactacaggc acctgccact atgcctggct aattttttgt tttgtatttt tggtagagac 156480
   ggggtttcac cgtgttagac aggatggtct cgatctcctg acctcgtgat ccgccgcctc 156540
   ggcctcccaa agtgctggga ttacaggtgt gagtcactgt gcccggccta ttattattat 156600
   tatttaagag tgttgcccag gctggtctca aattcctggg ctcaagcaat cctcctgcct 156660
   tggcctccca aagtgttggg attccaggcg tgagccagtg caccctgctt gttagggtgt 156720
   tcctaatatc agggcccagg aatgaggagg aaatgaattt atcctgctac catgaatctc 156780
   aaagtatgac tactgctagt gggacaagag atgcttgtag gtaaaatgtg caaaaaattt 156840
   ttgttttaat cattataaat gtattttaat gtggattggg aaaaatgtat ctagtagtgg 156900
   ttttgcattt atgataagga tatagaattt cctacctaag taaagatttt taggttaaac 156960
   aaaagataaa ctgatttgag gacaatatta agtgaatgac agtatgggtg atataaggat 157020
   atggcaaaag ttttggtaca aatgtgttta aagtttaggg tacattgttg attggcctac 157080
   ccagcagcca ttcacagcct tcatccttct ggcagaggct acctctcatg atggaagcta 157140
   aaaatgccta atacttgctt ttcccggctc tcttgtagct gctgaatgag gatgtgaccc 157200
   aatcctgcta atgggaactg aggggaagac ctgggaaaga gtctaggaaa gatttttcct 157260
   ttctgataag agagagacat cccttccctt gtcctcttgg gcatttttgt acaaggatat 157320
   aatgcttgga gttgtggcag cccttttggg tcaatgaggg acacattgcc gacatcctga 157380
   gcatggcaga gtggaaaact taaaagaacc gtgggtccat gatgatgctg atgagctgtt 157440
   gataccactc acagtccttt tatccccaag ctggatagat gggagaagaa gctttttttt 157500
   tggccaggca cggtggctca cgcctgtaat cccagcactt tgggaggctg aggcgggcag 157560
   atcacaaggt caggagatcg aaaccatcct ggctaacatg gtgaaacccc gtctctacta 157620
   aaaatacaaa aaattagcca ggcatggtgg tgggcgcctg tagtcctagc tccttgggag 157680
   gctgaggcag gagaatggcg tgaacctggg aggcagagct tgcagtaagc ggagatcgca 157740
   ccactgcact ccagcctggg cgacagagtg agactctgtc tcgaaaaaaa agaaaaaaaa 157800
   gaagcttttt tttttttttt cttttttttt ttaagcaaag tcttactctt tgaagccttc 157860
   tcttttattt ttgttttttt ctcttttgag acagaatctt gctctgttgc ccaggccaga 157920
   gtgtggtggc atgatcatgt ctcactcact gcagcttaac ctccctggct ctagcaaccc 157980
   ccccacctca gccgcccacg tagctgggcc tacaagtgca tgccaccaca cctggctaat 158040
   tttttatttg cttgtaaaga taggttttcc ctatgttgcc caggctggtc tcgaattcct 158100
   gggctgaagc cctcctccta ccttgacctc ccaaagtgct agaattacag gcatgagtca 158160
   ctgtacctga ccctgggggc attcttttac atgcagctga aactgtccta gctgagaagg 158220
   ctttccacca tgcacagtct ttcattgccc aataatgaac tgcattcagc ataccccaat 158280
   gcagccatca cccctagttc tcttactttg tttctgcttt gaatcccctt cttgaaattt 158340
   ctcccattgg ctttgtctcc attactggtt ctgttaccca cttagacttg acagctgaca 158400
   gcttgcccaa agtgagtagt gactatcttc tgtcattgga tttctttagc tcctgctggt 158460
   cttctgcaaa tggtcatctg ccaatttgct gcaaactggg gtgggagttt gtggggaggg 158520
   catatctgtt gccatagcaa cacaaagatg ctggagcata tgttggatat tctttgcttg 158580
   gaaaatgtgg taattgaggt taagaactcc aagaaacatg gaacaaccaa agtccttgga 158640
   aatctgtttg caaagacaaa gtttgagact gtttgtcatg ttctctcaga tcctatgccc 158700
   tagcccttgt agtgcaattt cttctctcct ccctagcaaa ataggcaggg gagtttgggg 158760
   agactgccct actcagtatg cagctctgag tgttcatgca gactgaaact tgatactcaa 158820
   cttgcagcca agaagatggt gtttcccata ttctatctat ctgctgaatg tttctgttgc 158880
   ctaagacaga aactcagctc aaactgactt atatgaaaag gaaaattttg ggtccttgaa 158940
   atagaaaagg cctaagggtt gacttggctt caagcacagt gaatcaaagt tctaaataat 159000
   gtcctcaaga acctgtttct ccccttcttg gcttagcctt tgtctgtgtt gattttattt 159060
   tcaggaaggc ttgtctccta tgcgagcatg gtggctgcaa cacctttaat cttatatctt 159120
   atcagtgctg cagtgcagta acaagagcta aagtctcatt agaccatgaa tcacatattg 159180
   ccagaagaat gggttatgct gattggccag gcctgggtat atgctgctgt ggtgtgaatg 159240
   ttttgtttct ccaaacttat atcctgaaat cctaacaccc aaggtatggt gttagaaggt 159300
   gggccccttg ggaggtgatc agttcatggg ggaagagccc tcataaatga gatagtgccc 159360
   ctataaaaaa gaccccagag atatccgtca ccccttctac catgtgaggt tgcaatgaga 159420
   agacagttgt gtatgagaaa gtgagctctc actggagacc gaatcttcca gtgccttgat 159480
   cttgtacttt ctagcctcca gaactgtgag gaataagttt atcatttaaa aatcactcag 159540
   gttttggtat tttgttacag cagccagaag ggactaggac agaaaatttt ggtactaaca 159600
   gtgtatagag gaacaaaatt ttaaggatgg gttttctgaa ttggtttcgg ggatttggta 159660
   attggctgcc aaatctgatt agatttaaga atgctaatta ccctatttct agtagtaaag 159720
   agagcctgga tagtccgtgg catgatctgt ttatagagat acacaaatca tctgcattgg 159780
   atactcctaa tcaaccattt ataagaagca aggagccaag tgattcgata tatgataccc 159840
   aaacactttt ggaaaactaa ggcatataat gacttgctcc taatgtctct ggacaaagtg 159900
   gagaaagaaa aagatgagct cagggatttg aattcccagc tcaaagagtt tcataaatga 159960
   tctaagagct tctaggtgtg ccctgaagga gagccttcta gcctgtagtt gcagggctga 160020
   aatttctgaa aattaaatgc acaacctcat cctgcaattg gctgaattac aatgcaaatt 160080
   ggactcccca ccacacaggg tgtctgctct aaaagcaagg gcaagggcat tgatcagaaa 160140
   agaatgggat cccataaggt gggaagactc tgatgaagac cctaatgaag ctggggacat 160200
   tgagccccta aattctgaca agtctttttt ttaccagtgg aagtggcctc cttacctcaa 160260
   gcaaaagtgt catccccact cagggtggca ttggcctttc catcttgtct aagggaacta 160320
   accctgcatt gccctaagaa acagtaatgg ctttccctga ggcagttgcc atgccaggca 160380
   atgctgattc ttgtcagcac cgaatcccac caccccttca agtcctagca ggctcctaaa 160440
   cgtgagatgc actgtgtgac ccacggggag gtatgctata ccccaaaaga actatttgag 160500
   ttttctaatt tatacaagca gaaccatagg gaatatgcat gggaatgata ttaagggggt 160560
   aggataatgg tggaagtaac atgaagttga atcaggctga atttattgat agaagttcac 160620
   taagcagaga ttctgaatgt aatgttccag cttaaggagt taggaagggc tctaacagtt 160680
   tagctggttg gctgaaacat ggattaaaag atggcctatc atgagtgagt tggagatgcc 160740
   tgacctcccc tggtttaatg tagaggaaag gattcaaaag cttagggaga ttagaatgcc 160800
   agagtggatt tgtcatttaa tacccgctca cccatgctgg gaaggtcaga cataccttta 160860
   agaaaggtat gtctctgtca atactttgag aaatatattt gtgaggggag ccccagcgtc 160920
   ctttaagagc tctgtgatcc ctcttctcta tagaccaaac cttactatgg gaaccacagc 160980
   cacttaattg gaaaacttaa atgcaatggg aataattggg tcctggagta tcaggggcca 161040
   tttggtggca ctcaactgtt aaaggcaagg taggtgcggt taccttaatg gatcgcagag 161100
   ttacaacaac aatcggaata gcctgactca tgcagaccta tggcactgcc taatcatggt 161160
   gttgctagaa gtgaaatagg tgggaagcct actaaattct tacttgatct gtatagcaga 161220
   aaaattctag gtgaagtgaa cagaagtcta acttgaatca taaaaataga gaatcatagc 161280
   ccctcagtcc attcccagac ttgagccagt ttatagaccc agaacccttt gaatgaaagg 161340
   gaggtaaaat ttccttgaga aagcgattat actgctaaaa gtttatcctg ttaatctttc 161400
   tcccagcctt gcccaaaggg acctactgct ttttatcagg ataactgtgc actagagaaa 161460
   aggaaatgat gagacctttt ggggactact gaacactagt tctgaactga cactgattct 161520
   aggagaccta aaatgtcact tgtccctcca gtcagaatag gggcttatgg tggtcaggtg 161580
   atcaatggaa ttttagctca ggtctgtctt acagtggatc cagtgagtcc ccaaacacat 161640
   cctgcagtta tttccccagt tccagaatgg ataattagaa tagacatact tagcagttgg 161700
   cagaatcccc atactggttc tttgacctgt agagtgaggc ccactatgat gggtgaagcc 161760
   aagaggaagc cattagaact gccccagcct aggaaaacag ccaaccaaga gcaatactac 161820
   atccttagag ggacagcaga gatcagtgcc atcatcaatg acttgaaaga tgcagaggta 161880
   gtgattccca ccatatccct tattcagctc tcttatttgg cttctgcaga agacagatga 161940
   atctagaaga atgtcagtgg ataatcataa gcttaaccaa gtgatcactc caagtgtagc 162000
   tgctgtatca aatgtggttt cattggttga gcaaattaat tcatcctctg gtagctagta 162060
   tgtagctatt gatttatcaa atgccttttt ctccatctct gtccataaga cccaccagag 162120
   cagttttatt gcagctggta aggctagcaa tacaccttca ctgtcctact tcaggggtat 162180
   atcaactctt cagtcgtatg tcataattta gtttgcaggg atcttgatca tttttccctt 162240
   ccacaagcta tgacaatggt cctttacatt gatgacatta tgctgatttg tcctagtgaa 162300
   tgagaagtag caactactct agacattggt aagacatttg tgtgttagag gatgggaaat 162360
   aaatccaact aaaatttagg gaccttcttc ctcagtgaaa tttctagggg tcagtggtgt 162420
   gtggcatgtt aagatacccc ttttaacgtg aaggataagt tgttgcatct ggacccttct 162480
   acatccgaga aagagacaga tggcctactg ggcctacttg gattttggag gcaacacatt 162540
   cctcatttaa gtgtgttatg ctggcctact taccaagggg ccccaagagc ttctggtttt 162600
   cagtagggcc cagaacagta gaaggctctg caacaggtcc agactgctgt gcaagctgct 162660
   ttgccacttg ggccatctga cccagcaaat ccaatggtgg ttggttagtg gcagagaggg 162720
   atactgtgtg gggcctttgg caagccttta taggtgaatt gaagcacaag tatttaagat 162780
   tttggaatat agtcctgcca taatccacag atagctagtt tcctattaag agacagctct 162840
   tggcctgcta ccgggccttc gtggaaactg acgatttgac catgggccac taaattgccg 162900
   tatgacccga attgctcttc attaactggg tcttatctaa accaccgagc cataaagtgg 162960
   gcatgcacag cagcacttca tcatcaaatg gaagtggtat gtatgtgatc aagcctgggc 163020
   aggctctaaa ggcacaagta cgatacatga agaaatggcc tggatgcctg tgctccccac 163080
   tcttgctacc ctgccttctc tctcccaact tgcacctttg ggctcatgag gagttagttc 163140
   cctatgatca gttgacaaag gaagagcaga ctaggaccca gtttacagat gtttctgtac 163200
   ggtgtgcaga caccacccag aagtggacag ctacagcact gtagtcactt taggacatcc 163260
   ctgagggaca atagtgaagg gaaatcttct cggtgggcag aactttgagc agtgcacctg 163320
   gttgtgcact ttgcttggga ggagaagtgg ccatacatgt gactatatcc tgattcatgg 163380
   gctgtagcca attatttgac tggctgggca gggacttgga aggaatatga ttgaaaaaat 163440
   tggtgacaaa aaagtgtggg gatgaggtgt gtggatagac atctctgagt gggcaataaa 163500
   catgaaaata tttgtgtcct aggtgagtgc ttaccaaagg gtgacctcag cagaggagga 163560
   ttttaataat tgacttgata gaatgacccg ttctttggat accaggcagc ctctttcccc 163620
   agacaccctg tcatcacaca atgggctcat gaacaaagtg gccgtggtgg cagggatgga 163680
   ggttatgcat gggctcagca gcatagactt ccactcatca aggctgacct ggctatggcc 163740
   accgctgagt gctcaatctg ccagcagcag agaccaacac tgagttcctg atatggcacc 163800
   atgcctaggg gttatcagcc agctacctgg cggcaggttg attacattgg accccttcca 163860
   ttatggaaga ggcagcattt tatccttatg gaaatagaca tgctggatac agatttgcct 163920
   tccctgcctg cagtgcttct actgaaactg ccattcatgg acttagagca tgctttattc 163980
   actgttatga aattccacac agcattgtgt ctgatcaaag aaatcacttc aaagccaaag 164040
   aggtgggcag tgaactcaca ctcatggaat tcactggtct taccatgttc cccatcattc 164100
   tgaagcagct ggcttgatag aatggtggaa tggccttttg aagagtcagt tatgttgcca 164160
   gctaggtagc aataccttgc atgcctgggc aagattctcc agaaggctat atatgctctg 164220
   aatcagcatc tagtatatgg tgctttttct ctgatagcca gaaatttatg gatctgggaa 164280
   tcaaggaagt ggaatgggag tggtaccact cattattacc cctagtaatt ttaccaccag 164340
   caaaattttg ctttcttttc ccatgacttt atgctctccc agcatggagg tctcagttcc 164400
   agagggagga atacttccag caggagacag aacaatgatt ctattgatag ttaacactgc 164460
   cacccagcca ctttggggtt cccatgcctc tgagttaaca ggccaagaag gaagttatga 164520
   agttggctgg agtgattgat ctggactact aaggggaaat tggacactac tctacaatgg 164580
   agaaaaggaa aaatgtgtct ggatacagga gatccctaaa gacatctctt agtattacca 164640
   ttacctgtga ttaagatcaa tgaaaaacta caacaactca atacaggcaa aactatgaat 164700
   ggcccagacc ctttaagaat gaaggtttgg gtcaccctgc caggtaagga accaccacca 164760
   gctgaggtgc ccactgaagg taaagggaat acagaatagg taatagaaga aagtagttac 164820
   aaataccagc tatgaccgtg tgaccagtta cagaaacaag gactgtgatt ggcatgatta 164880
   ttggcattat tttgttatgg atatgtttgt gtgtatatat acatatggtg agcaaatatc 164940
   tttgttttct ttgctctttt agttctttat catgtaacgt aagatgtatt gactttatat 165000
   cagtattttt atgtcttagt atttaagtta taggatacca ggacaagagg aatcattact 165060
   caaggacttc atctcctttt ctggggaggg gattattgca ttttcagttg tacaaagtct 165120
   aattgtatca catcaggtgg aattatgtcc ttgttattct ttatttggag gttctttatt 165180
   taagaaggtg tgtatggatg ccaagttgag aaagggtgga cttgtgatgg ttaattttgc 165240
   acatgcagtt aaccttgact gggctaaggg gggtacccag atagctggta atacatgatt 165300
   tctgggcatg tctgtgaggg tgttttcaag aaattatcat ttgaatcagt agactgagta 165360
   gaaaaattgc cttcattgac ataggcaggc accatctaat ctgttgagga ctcagaacaa 165420
   aaaggcagag gaagggtaaa ttcactctct ctttttgagc tggaacatta attttttccc 165480
   gcctttgggt gttggtgctc caggttctca gtccttcaga cgtggaccag gacctactcc 165540
   attggcacct atggttctca ggtctttggg ctcagatttc aaacattgga ctaaactata 165600
   cccctggctt tcttggttct ctggcttgca gatggaagac tgtaggactt cttggcttcc 165660
   ataattatgt acaccaattc acgttattga tttttttttg gagaaccctg actaatacat 165720
   tgtgcctaca ccgtagatga gggcgtgagt gccccttcca tggattgaga gtggggaaga 165780
   ggtagtttct ctgaggaaag ctagagaaac ggtattgtgt cctggttaaa atcacggatt 165840
   atggagccag cattcctagc ctcagatctg tgtgaccttg gacaaataac ttaaatccat 165900
   ttatgcctta gattcctcag ctaaaaaatg agttaaataa tagtttctac ttgatagcat 165960
   tgttatgagt atcaaataca tagatatctg taaaagcact taggacagtg tctggtgcat 166020
   ggcaagagct atatatgtga tgatgatgat gacaatgaca atgaataaca gtgcacacat 166080
   gctgagcagc cagcaaggac aggtgtcttg tcagacagac caggaaaggg tgaagaatga 166140
   gcaggttctc tgtctgccac ctggcagtcc ttcctagaag tgtggaggct ctgtaagtgg 166200
   catgagaccc ctctctgtat gaatgaagga gtgcagccat tcattgcctt gagcatgagg 166260
   agagcaggaa gtactccctg cagccagcat gcataggatt tggcagccta cctgaaatga 166320
   ttctatctct ggatacagtt ccaaaaaggt cagttggcac caaggatgat gaattggtcc 166380
   agacacctga actggcttga ctcagttcat tgaccttttt ctgctcatgg ttggaggcat 166440
   gattaaagac ctagccacac ctgcttgccc acctggccag actctccttt agctgcccct 166500
   gatactcatg caggtatgga ggggtgccct ctccttctgc atgaccttgg aggaactggt 166560
   gtgcagcaag ttcgtgaagc agaaacacac aggcggtgac cttggacaag gctccatcca 166620
   cgataaagga tgtgcttggt tgcatcctct accctctgct gttccaagcc cagtctgagc 166680
   caggcaatat tatggatatt agtagagctt tgtgctgttg gcattatcct acagctatgt 166740
   tcacatcata tcacattgag gcaattttat gaaccaggca ctgtcccgtg aacctagcta 166800
   ctgagccaac cattcaataa caatgagaat agacaacatt tatgggcact cgccattgga 166860
   gggccacaaa gtgtggcaga gttcatggat gatgctcctg gtcacccaca gtcatccttc 166920
   ttgttcttga tctcaccagg caggaaatcc tcaattcatc tgtctcttgt acctcttaga 166980
   gagaccaggt ctctggtact gtcataagca ttacttatgg acattatagg ttattcagca 167040
   ctcagaatac tgaccacata cacagcctcc tgtgacatct cagaacaaga aacagaggag 167100
   ttgtctctga gggttgggac tagggtgagt caagtgaggt actttccctg ggcacagttt 167160
   aaggggttct tgtctgtgtc tgtggatagc tggttggttg gctggtgggt ggatgatcta 167220
   agatggcccc tttcacatga ctgacgacga gcaggttgtc ggcctgtgtg atttggctct 167280
   cgtccatgtg gcatcatcct ccatccaact ggctagctct gacttgttca tatggtggtt 167340
   tccaggctcc aagaagagca agagagcatc tcccaacact ttttaagcct ttgtatcatg 167400
   tttgctaatg tcctaaaaag ctagtctctg gccaagtcca gattcaagtg gtagaaaaat 167460
   agattccagt gcctcattgg agaggaagta tctatggtta atctattgca gatcctgtta 167520
   acccaagagg ccatagcaga acagaacccc tggagaccat cacactggac tttataaaaa 167580
   tctgcatgtt atagtgtggc tcagagtcat agatggcctc atcaaaagaa tgcacctaat 167640
   ttattccctg cctcaatcgc cagtggactt aaccacctct agctataagt ggttaatgtg 167700
   gcagtttatt gatcacgtca atggtggtta agagtatgga ctctgaaatc agacatacct 167760
   gaatttgagt cttgattctg cccagatact cattaggaaa acttgggcat gtcgttcgac 167820
   ttctctatgc cctagtctcc ttatatgtaa aataggatgt tagtaccaac cacataggtt 167880
   tatagtgata attttaaaaa tatttaaaat gagaaagagt tcacaaacaa aagacaaatg 167940
   aatttcaaag cccttacatg aaagcgcttg gcacagtgca tagcaagccc ttggtacatg 168000
   tagctaaggc catttccatt gctccaccta ggtccataat caagaatgta ctgacaattc 168060
   tctacattct tccacccaga gcatgccctt atgaggtgat tacagtttcc atttccatta 168120
   cttcacacaa acctcaaatt ctgtgatgca gtgctaagaa aagaactcaa actttggagc 168180
   cagaaaatgc tggtgtcaaa ttccaaatgg tctctgctat gtattgctcc aatgaccttg 168240
   ggaaaataac tttaagttct gtgaccctca gtttatgtct ttaaaataga gaaaataata 168300
   cctaaataaa gcggtttttt tttttttggt aactattcaa taggagactg cttgaaccat 168360
   ccctagaata atgtctggca caaagaaggc actcaatacc tggttttaaa atcattatta 168420
   atgtctggag gagcctctcc tgctctataa gggatacagg ttaccctctg acctgccaag 168480
   aaataatagc taataataat agctaacttt attgaacagg gtctctgggc aggcagtaca 168540
   aaaggactct ttgtctggtt tgtctaaaga tacctttttc aacactttat ggctttagaa 168600
   ataatttctt atgatttata gtcaagttac tgagtaaaac ccactgttag gtcctgtgat 168660
   ctcctaattc catcactaaa tcaggtcaat tagagatggt agacactaat gttgacctta 168720
   tccttttgat caagtctcac tttgtgataa gatagattaa aaatcataaa cttgttaaac 168780
   ttgttaaatg ttgaagattt gataaaacag aaatgaaagt attaaatgta tgtatacatt 168840
   aaagtatgta aataagtgag aacatgtttg aaaatctggt tgaaatggat gattttctca 168900
   taaaattgtc aattttgtct caaaaagaaa ttgaaaacca gaataggcaa ataaccgaga 168960
   aagaaatgaa aacattattc aaggatcttt tcttctcgta aaaccccaga caaatatgtt 169020
   tcatgggtaa aacttcttaa attttagagc aacgaagaat ttctgttcat ttaaaattgt 169080
   acctgattac aaagaagaaa taaaccttta caattttttt taaatgaaaa tgagagaaat 169140
   ccagctcaaa ttagcagtag gaaaagaggg cccattgact cctgagaagg acattgggag 169200
   tagagtaaga gcttcaggac caaggacctc aaaggctgga actgaagctc cagtacttgc 169260
   aagatacttt tccatttctg ctttattggt tagttatttt gctctgtaac aaaccattcc 169320
   aaaatttagt ggcttgaaac atcagttatt tgtggtttct catgattgtg tgggttactt 169380
   gggtgattct tctgacctgg gtttatttgg gtaatccctg ctgggctctg tcctgggtct 169440
   tggtcagcta gtggctgggc cgatgctgga ttacctagaa cagccttgct agcattgact 169500
   gagggctggc aggctgtcga tctgggcatt tcatttttcc tccatgcgtc ctcttttcct 169560
   ccaccaggct agcttagttt cacggttcca agcacggcaa gagagggaaa tcccaaagca 169620
   caggtgtgtg tgtgtgtatg tgtgtgtgcg tgcgtgtgtg tgtgtgcgtg cgtgcgtgtg 169680
   tgtgtgtgaa atggagtctc tttctgttac ccaggctgga gtgcagtggt gtgatctcgg 169740
   ctcaccgcaa ccctcccaga tacaagtgat tctcctgcct cagcctcctg agtagctcgg 169800
   attataggca cccaccacca cgcctagcta atttttgtat tttttgtaga gacggggttt 169860
   caccatgttg gccagctggt tttgaactcc tgacctcaag cgttctgcct gcctcagcct 169920
   cccaaagtgc taggattaca ggtgtgagcc actgcgcctg gccagcatag gtgtttttta 169980
   agcctttgtt tgtgtcacag tggttatgga caagatcaga tttggggtgg agaaacaaac 170040
   ttcatctctg gatgggaaga atagcaaagt cacatggcca aggagcatgc ggggatggga 170100
   gacattggtg gccattgtgc agtctaccac actgaccctt atctgctgtg ccagatgcct 170160
   cgtttgccct tccaatccac tctgctttct ctgagagact aagccataat gactgcttca 170220
   actggggctc ccttaccctc tgacctgacc tctggttgga ctcaagggga ggggctaata 170280
   ggagacttgg ggaggggaga gtgagaccct ccccgagtca gtcagtattt attatggagt 170340
   catcttcact ctaccttttg actataggtt gctgctcctc tacagatcat cttctctatg 170400
   taattctctc cttctgggtt ccaggagcta ctcccttcct ttgtcctctg gctcttacta 170460
   gcctccaatc ccttatactt tcccaacacc atgcccacac ctttagaaac agtcctttca 170520
   ttgaaccttt cttaaacact cttcacttta gtatgccatc tgtttcctgc ggggcccctg 170580
   actgatgcat ctgctttgtc ttgcctcatt tgctcctgct gtagatggaa catgtctgca 170640
   aacatcgtta cagttgaccc catggaaaga tggcaaccct agtggaggaa ttagccttct 170700
   tcctgcattc gttttttttt tttttttgag acaaagtctc gctctgttgc ccaggctgga 170760
   gtgcagtggt acgatctcag ctcactgcaa cctctgcctc ctgaattcaa gcaattctcc 170820
   tgcctcagcc tcccaagtag ctgggactac agatgtgcac caccatactc agctaatttt 170880
   tgtattttca gtagagataa ggtttcagca tattggccag gctggtctcg aactcctgat 170940
   ctcaagtgat ctccctgtct cagcctctca aagtgctagg attacaggcg tgagcgactg 171000
   cacccagcca tcttccctgc attcataatt gatctcagaa aatgttctga tggagttggt 171060
   tctctatcac tgggagaacg aactcttata acctcagacc tggatcccat ggcatccctt 171120
   tcacccagga gcagtgtatg ttataagaag agtgaaggag aaactttgat gcgctgatga 171180
   aagtaataac taattattat aagcttgctt aaccacaata accgctatta cctagtaaag 171240
   ttggcataaa aagtaaacta caagccagtc tcatacatga gtctagatga acatatccca 171300
   aataaaatag tgaatgctat tttattgttg tcacggacat tatgcattca aataatgtgg 171360
   aggaaacaat ctagagataa aacaacaaag aaaattatga tcaatataac aaaatatagg 171420
   aataaagata aaaatatgcc agcaaggaac cgtggaatct ggaaagcata aatttagatg 171480
   gtagaagtga tagcagaatg ttacccaacc aatgtaaatg gattgaaatt ctctactgaa 171540
   aggcaaagac tatgagattg gatgagcaat gtacaactct tggcagtgta taaaagatgc 171600
   ttaaagcaca ggttttaaaa ataaaaggga caaaagtgta tcatgaaaat acatatataa 171660
   aaatgaaaca tggtcttaat aatgatatca aaagaaatag gactcaaaac aaaaagcata 171720
   aacgggggca gatgtcccag cagatcactt gagctcagga gtttgagacc agcctgggca 171780
   acatggcgaa accaagtctc tacaaaaaaa aaaaaaaaaa aaagaaaaaa ttagccaggc 171840
   atggtggcat gcacctgtag tcccagctac tcaggaggct gatgtgggag gatcacttaa 171900
   gcctgggagg tggaggctgc aatgagctgt gattgcacca ctgcactcca gcctgggcaa 171960
   cagagtgaga ttctgtctca aataaacaaa actaaacaaa aacataaaac agggaaggag 172020
   aaaacatgaa ctctaatgca ttactggtga gtggcaaatt agtatatgtt ttctaaagag 172080
   caaattttcc atacatatcc agagctttaa acacattttt gacctaagaa ttactaaatc 172140
   taatcatttt gacctaagaa tgacaattct aggaattttt ggtaagaaac catttcagga 172200
   taaatgcata gatttatcta gagtgatatt cattgcatca gtatttataa tatcaaaaat 172260
   ttgaaaatga cccaagtctc actaccaatt agagtttata taagtaaaat atattacagt 172320
   catatggtgg aatgctggcc agtgattcag tgtcacagtg tctggggata aggggaatgt 172380
   tctgtgtctt gactgggatg gtgcctatac aggtgtacac atttttcaaa actcatcaaa 172440
   ctatatgctt aaactctgta cattactgta tgtaaattat actttaataa aaagtcacag 172500
   tgattaaaat gccattggcc tggaaatgca gtcataatat attgctaaat gaaaaaaaaa 172560
   aagcagacaa caaaacatta ggtcaggtat gatagattta aaaatataaa aatatataca 172620
   catgtaaagc caaaggccac attccctaaa taaattttcg tgaatactta attctgttca 172680
   ctgtcttatg ccagacttgt tacctgcaga gctctgaagg tcactttggg atgttctgta 172740
   gctggtttct ccagttttgt ttgtttctgg ggccactcca cctctagtgc agactcaggt 172800
   ttcccactta gttcagtgag gaccaaactt gagtggcggt tccggcgtaa tcgggagtca 172860
   cttacagttc cttccaaatg tggttactct tgaggtcctc catccgctgg gagtcagctc 172920
   agttcctgga ctgagcatct tcaggtgctt ctaataagat tctggcctct gataagtctt 172980
   gacttcaatt catttttggc cccagagaag gggaatcttg gctctgccaa caaggccctg 173040
   tccatttcct gcctcagtgt gagtggcagg aagaccacct tgatcttcag ggcaaaggct 173100
   cgcttcctcc tcttggtgtc atccggtctc cttggctcag taaagcaacg taacagtcaa 173160
   gtgcactttt ctcaacgcct ggtttcttct gtcttacata accccaagtg gggacaccca 173220
   cagtgaactt tcaaaaataa tgctccttcc agggagcgaa gcagcaatga gggttgaaca 173280
   tctctcttgg gatgaccatc acctggacca aaagacccat ttacatctct caaggataag 173340
   taatgtcagg ggcaatttag tctaaatgat acagtctcca cctcccttcc tcccttcctc 173400
   ccttcttccc tctctcactt atctattatc tccctacatc tgaaagtgca ccatcctgct 173460
   gaaatcacat cctgttcaca taacactgca gtccatagct tctgaatcca cacagaagtt 173520
   ctcgtgggcg ataccaaatg ccaagaggat gccttagaag gagtctggag atactcgagg 173580
   attggcctct ctagctcaga ggttacactg tcatcaaatg tccaaagagc tcttgtacag 173640
   aatttttgtg gtgcagttct aaaggagaag taggactgag gtccatctgt gcaggctaac 173700
   gaagaacagc ccatactgac caactggttg ctacataata atattagctc cccatccgag 173760
   gatagtaaag ctcaattgcc aggcatgctg tagagggcat agctgtggta gatgtgggtt 173820
   tctaccatgt tggtggttct actagctttt gttgttttgt tgcatgcaac agaaaccatc 173880
   tttggttaac ataagttgga aaggaattca ttggaaaaga tgtggcatcc ttcagagcgt 173940
   tgaaagaaaa cttgaacaac cagaccagtt ctatctgatt ttggttcttg tgttacattc 174000
   agcctaaatt caaattccag aaggagagag attatctggc cgagctcaag ttgcctaaca 174060
   aactcttggg tcgggctttt aaaatgttct ttcaaagact aaacgctgtg ggggtaggat 174120
   ggcttttcaa aggagaatcg aagtactgat acggggagca gggggtgggg agggggaaga 174180
   aggcacagat gttgcgccaa ttggaacaac gtgtttgcta caatttctaa atgctacatt 174240
   gcagattgca gagagattgg atgacaggct tcacctgaag agatagagat caggacctgg 174300
   gaatccatgt gttaaaaagt tcttgggtaa ttctgatgga tagctgggtt tagaagccag 174360
   tgtaaggcaa tatgtataaa agctctgaaa ccagaatgac tgagtttgaa ttccagtttg 174420
   gccactaact agctgataat cttgggctag ttacctaacc tccctgcttc agtttcctca 174480
   tctataaaaa gaggataaaa tggcacccac ctcatagggt ccacgtgagg attaaatgag 174540
   ttaacctaga tggtaagagc ttcatgccgt aactggcaca taggacatgc tagttaatat 174600
   tattgccaca aatgatcctt tcaagtctta gattgtgcat ttcaaaagtc catccttatt 174660
   tttggaattg catgcattta tcttgctttc tgcaaaatgg aaaggcatct cccatgggct 174720
   ggttggagag aggggctgat aaaatgatgg taatgtgata aaatgcagtt ctcttgattg 174780
   tcaagtgaat tctgcatgat tcttctgctc tcatttgcca cgggctgact gcttctgcaa 174840
   tgcagaaccc gcttgtaaag gtcagaatga attgcttcca aacatccctt tgtccctcac 174900
   tgtcacgaag acacagagct gcatgctgtg gaggaagaag caaatccatg atgctggaag 174960
   gccagagaac agaaaccaac cgtgcacaga gaggcgagct gctgggactt gaatgaagct 175020
   gatgcgtccc atctataaat tattgtggct tctcctagtt gggaggcagg accagcctca 175080
   caatttccca gaagaccgta atttctggag cctgcaggcc tgggggcaaa tggagaaatc 175140
   aaaaggcaga tggccttgga gataggcagc cagaaaggat gtgaaaggcc ctggttctgg 175200
   actcaagaga cctcacgttt cctagtcttc tttctgtctc tgctcttttg tgttcatgag 175260
   gttcatgccc tcatgagggc tcccccacct ccagtggtct aggattctag gatgtcttgg 175320
   tattcttttt caaaaaaaaa aaactaagag ccagcattta tagatataat tctgtcctaa 175380
   acactttaca tataataatt cttcattctt cattacaccc tgtgagatag gttatcattg 175440
   ttataattcc atttcacaga tgaggaaact gaggtactca gagactaaat gacttgctca 175500
   aggtcacttg tgagaggtcc agctctaggc cctctggccc gtggggctat gctcttacct 175560
   agtacctcaa actgcctggt agacctcaac cctgtgtacc tgcacaaatg gtcctgaagc 175620
   aggcagcttt cttccctgag agctgccttt ccagatatgc actgccaaag gtctgtgacc 175680
   ctacagcctt ggcctgattt ggtccagatg cgtgtggaca tctctcccaa acaggatcaa 175740
   attggcctct tctaggagtt tagaatttgg aatcaagggt gctggtttta gtctggactg 175800
   gtcacacagt ttttgacaac tgtttattgt acgggaaaag tgtccagctt gatgaattct 175860
   cacacagtga atttttcttt gtaagcagag ccccagaagc ccttctttgc tccttctaat 175920
   ctctacacgt ccctccccat ccatcaaaat aaccactacc agaccagcaa tcctttggct 175980
   gcttttcccc ccaccccCag tgatctattt tattttattt tggaaagaac acgtaacatg 176040
   aaatctatcc tcttaacaaa gttttaagtg taaaatgcat cattgttgat gatggatgca 176100
   gtacgagaca gcagatctct ggggcttatt catcatgctt gactgaaact tgatgcccat 176160
   tggtaagtaa ttccccattt tgcccttctt ccaggctgta gccaccaccc tttcactctg 176220
   attttctatc aatttgacta tttttgttac tttacgtaag tggaatcatc tagtatttgt 176280
   tttcctgtga ctagtgtttt tcatttacca taatgtcctc agggttcatc tatgttgtct 176340
   cattgcagaa cttccttctt tttggggggc tgaaaaatat tccatcatat gcataaacac 176400
   attttcctta tccatttgtt cgtcagtgga cacctagatt gcttccacgt ttagctgttg 176460
   tgaagagtgc tgcaatgaac atggaactgc taacatttct tcaggatgtt aatttcaatt 176520
   cttttgggta aatacccaga ggtgagggtg ctgaatcata tggtagttct atttttaatg 176580
   ttttgaggaa ccttcacacg ggctaaggac ttgaataggc agttgtccga ggaagacata 176640
   caaatgacca aaaggcatat gaaaatttaa aaagctaaac atcactaatc actaggcaaa 176700
   tgcaaatcaa aaccacaatg agatattatc tcacacttgt taggatgact attttttttt 176760
   tttttttgag atggagtctt gctctgttgc ccaggctgga gtgcaatggc gtgatcctgg 176820
   ctcactgcaa cctccacctc ccgggttcaa gtgattctgc tgcctcagcc tcccagagtg 176880
   ctgggattac aggtgggagc caccacgccc ggcctatttt tttttttaaa aaagacaagt 176940
   gttagcaaag atatggagag attggaaccc ttgtacactg ttggcaggaa tacaaaatgt 177000
   tgcagatact atggaaaaca gtttgcctgc ttttgagcac ttaaaaaaca aaactaggct 177060
   gggcccggtg gctcacatct gtaatcccag cactttggga ggccgaggta ggggggatca 177120
   cttgaggtca ggagtttgag accagcctga ccaacatggc agaaccccgt ccctgctgaa 177180
   aatacaaaaa ctagcctggc atgatggttt ttgtatctgt aatcccagct actcaggagg 177240
   ctgaggcagg agaatcgctt gaggttgcgg tgagccgaga tcatgctact gcactccagc 177300
   ctgggtaaca gagcaagact ccatctcaaa caaaaaccaa aaaacagaat tatagagtag 177360
   accatctctt gtgtctggct ggtttctttc atatgtgtgt gtgtgtggcc acatgatatt 177420
   gaaaggacac atagcatcct gttcatcagg atgtccagct tcagagccag caggggccag 177480
   gcaggtgggt gaatgagtga cgtgtgtgac cacaggcatg tcccattggc ccattgaaca 177540
   ccctatctga aaggggcagc ggcactcagc tccgtttcct aattcctgct gtttaaagga 177600
   tgaaggtcta agttttccca tttttttcca gagaaatctg aaatccccta aatttagatt 177660
   ttgacagaga attcaagaat tacaactgtg gtgtatttta aacacatttt tggactggat 177720
   tccttctgtg aacctccggt ttacaacttc tgcattcgta ggtggggact gtcagtggaa 177780
   ggtctccccc accccacatc ggaggggaga agggaaaatg taaatttagt caaatgggct 177840
   ttctttggag agtagagagt gtgcaatagt ccaaggcagg gagctttaaa agaatgcaga 177900
   agtctttttt ctctcttggt gtctgagctc taaattggct gggacatatc cttagttagg 177960
   agtcagtggc tatagcgccc cctgctgtcc tttagcaccc tcttcgtgtc cagtgagatc 178020
   tgcaggtgag cctggaagcc acttgcttct cctgcaggtt ttgggaggaa gattcatcat 178080
   tttaaatatt gctcaagtgc ctgctgctgc cagtgacttc ataggctcct gttagactca 178140
   ccttcactta tatttggtca attacctact ccctcagtca gaccttgcca ctcgattttt 178200
   tttttctttt tgatacaagg tctcactgtg ttgcctacga gtgcaatggt accatcacgg 178260
   ctcactgcag cctctgcttc ccaggcttaa gtgatcctcc tgcctcagtc tcccaagtag 178320
   ctggggctac aggcatgtgc cgctacgcct ggttgatttt ttaatttttt tgtagagatg 178380
   gggtttcatc atgttgccca ggctggtctc aaactcttgg gctcaagcaa tcctcccacc 178440
   ttggcctccc aaagtgctgg gactactcag gcataagcca ccatgcccag ccaccactct 178500
   attttttttt aaattggagg tagggtaaga aggactgggc caggtcgtcc ttcccacaat 178560
   tccagtctca ctgtttctat ctattgaaaa gcatttctgg catttgcttg gtacctttcc 178620
   ctggtttctg ggctgaaata gattttatcc catttttaaa caattccttc tgatactttc 178680
   aggggtagtt tgtggatgag agcagagtga aatgcatagg ctcaggtttg tggccttgca 178740
   agttaataaa gagtgtggac tttggaacta gcctgcctgg gctccaatct tggctctggt 178800
   acctactagc tgtgtatcat tgttttcatt gttttgtttt gttttatttt gttttgtttg 178860
   agacaggatc tcactctgtt gttggggcca gagttcagtg gtgcaatcat agctcactgc 178920
   agcctccaat tcctgggatc aagtgatcct cccacctcag cctcctgagt ggttaggacc 178980
   acaggcatac accaccacac ctggctaatt ttaaattttt ttgtagagat ggggtcttgc 179040
   tatgttgccc ggatgggtct tagcctccca aagtgctggg attacaggca tgaaccattg 179100
   tgtcccacct tagctgtgca ctttaaataa gtcacttgac ctctctgttc ctgtttcttc 179160
   atttacaagg tgaagatgat aatatactac ttcttagggt gagtattaaa taagttaacc 179220
   tatgcaaaga ggttaaaaca gtgcctagca tatcatagaa atgtttgctt gacccagagg 179280
   cctgaacctg ctttgccttc agtgttggtc tttaggtcct ggcttcactc cttaatgctg 179340
   catctcagcc tttcctcatg aaactttctg tctttgatcc ttttcactcc ttaactgtga 179400
   atctcccaaa ttccactgag cagtgatcaa ttcttggggc atcttgctct gtgttcccag 179460
   acattccttc accctcattt tctctccatg gccaaatcct atagtgcctc ctaaataatt 179520
   atagctgaca tgtatgtagc acctatcata tgccacgtgc tgttctaagt gattgatata 179580
   tgctaactca tttaatcctc accctatgag aaagatgctt ttttaacacc tattttatag 179640
   aggaggaaac tgaggcccag agaagtgaac tgacttgcca ggtcacataa ctaataagca 179700
   gagtctggat ttgagtttgg ctctagagcc tgctcttagc caccatgtcc catcctcact 179760
   gagaggaccc ttgttcaact ccactaaccc tctctggaca agtagcctct ggtcctgtct 179820
   ccctgcttcc tctctgtctc actggcctac cctttccaca cctttctaaa aaccaaatgt 179880
   gatcatgtca tcctctttgt tgaaaacatg tcaatggctt cccattgttc ttgggacaaa 179940
   gtaaatactc tttgaagtgg ctgcgaagca gggtggacat acgtcctcct ctgccaagct 180000
   tgcctataaa ccaaattggt ttgttctgtt gtgtcagcgt accacaaggc tggtgccctt 180060
   gtcatcctca caagagtccc tgttggaaga tacaggatat gattgtccta ctcatgaggc 180120
   cctgcgtgct ctggcgtcag ggtccctcca gcctttgctt gaccactctg agtaccttcg 180180
   ttcactcctc tgttcatcct gccaccaggc atttgcacat acttctccct tggctgagat 180240
   actcttctct ctcttcttcc ctgtgagttc agtgggtctc aattctagtt gcattgtaga 180300
   atcttctgca gggcttttta actgaatgca gtgtctgcac ccattccaga ccaactaagt 180360
   ccaaatctct gattgattca gaacctctca gttgcatgca gggttaaggg ccctggtcaa 180420
   ctcacgctgg tcctctagac tgcagctcaa gcatcacttc ctcagggaag cccttcctga 180480
   cctccctgac caggcccatt tccaatattt tgtactcagc acaccacata cctcttcttg 180540
   ctgtcacata tcttagggac atctaaatat taggttggtg caaaagtaat tgcggttttt 180600
   gctattgtaa ataatggtgc aatagtaatt agttaaaagt aattaaaaat aatggcaaaa 180660
   gcacagttac ttctgcacca acctaatatt tacttacatg gtgacaaaga acccagattc 180720
   tggataaaaa tgaatcacta gctgtgtgac cttgggcgag ttacttaact tctctgtgtc 180780
   tcagttttct cccctataaa ataggaatag tcaaaaacct gttttacatg gtcgttgagg 180840
   atcaaattag ttcataatat gtaaggcgct tggaacagtg cctggcacat ggtgagtgct 180900
   gtatgtggta gctgttactg cactagtaat tattaccccc tgctgggatc tgtaaatccc 180960
   tgaaggtggg gttggcatcc gttgtttgat cactccacac ctaggatgct gcatgaacat 181020
   aatagctctt caatacctgt ctgttcagca gagtggcgtc ctagtgaggt tcttggtaaa 181080
   agatgaaagg tgagatgtgg aattgcaaaa aaaaaaaaaa agtatagaca tcagagttgg 181140
   cctgcacaga attcaccttc ctcagttagg gcccttgtct atgcgggcct gctggttcag 181200
   ggtcattgtt tctagcggtc atgaggatca gagcctgcct ggacttgctg tgtgcagagt 181260
   ccccggtgca gatggccacc tggcaggcat ttgctccagc agcatccttt gcaggaatgg 181320
   gaggctcctc tgtaattcac aaactcaccg tgtccttcta tacagaggat ctttctgctc 181380
   ttctgaaatg cagacttggg tgtgctgaga gcttctgtga cagagggtgc tgtcaggaag 181440
   ctgggcatcc agggctgcac ctggaaacca agctggcagc ggctttgcaa gttgaataat 181500
   cttatgttgt gatcttgcat ttacgggacc ccccacttag caaaaagggg tgctcagacc 181560
   acttccctta gttggcttac ggcactgcag ggggagaggg gtggggaggg gaggagatgg 181620
   gtgtggtttt gtgggtgatg gaggatttat ttataatgtg ggtgtgatcc gttgccatgt 181680
   gactattccc tgaaactact gtacaccctg agcaatgaat gcacaacccg gcctgcacag 181740
   gaagaggtgg ggctcactct gtgaacagcc tccaccagaa ctcgaaggcc ccctggggaa 181800
   taccaagcct aacccctttg ttttcagaag gaggaactga gaccagacca tggcagtgac 181860
   ttgcccaaga tcacagatgt catttttatt ctaagctaac atttgtcata tggatactac 181920
   atgccagtca ctgtgccaag ctaagcatca tcattgtgat ttcattaatc tgtacacatc 181980
   tagaaagcag gctcattata ttcagcaaac acttgcatag tgtatatatg atgtgccagg 182040
   cactgtgctg agtcttttac aaatattaat ccattaaatt ctcataatcc tatgaggatt 182100
   tagtgtgatt attatttcct tttatttatt tatttaagac agagtcttgc tcttgtcacc 182160
   caggctggag tgcaatggca tgatctcggc tcactgcaac ctccacctcc tgggttcgag 182220
   caattctcct gcctcaactt cctcccaagt agctgggact acaggcaccc gccaccatgc 182280
   ccagctagct aatttttgta tttttagaag agatggggtt ttaccatgtt ggccaggctg 182340
   gtctcgaact cctggcctca agtgatccgc ccgccttggc ctcccaaaat gctgggatta 182400
   caggtgtgaa ctgccgcgcc tggccttatt tccattttaa agatgaggac cctgaagctc 182460
   agagaggttg ggtaacctgt ccaaagtcac acagctggga ggtcattgag ttgggagtca 182520
   atctcagaca gtctggcctt gaagtgtctg ctccagcact gtcatttctc tccatgtgac 182580
   agctcttctc tccattttac aggtgagaaa atcaaggctt atataactca agtagcttgc 182640
   tcgaggctgc acgactggaa attaccagag ctggtgttga atttgcacat tccagctata 182700
   gaatctgacc cctgatctca tgctaaactc cctctcagtg gggctagggt cttttggctg 182760
   aggggtccaa gctgggaaca ggtcacgggg gttgaggcag agcctgaagc cagcaggtgg 182820
   aggtggggtg gtgtcagcca ggtctacctg ggttgactct gaacctcatg cagtggagca 182880
   gttactccct tcccatgggg cagagcccca gctaggtggc catcgcctgg ttacaccctc 182940
   taattcctta agatttcagc ttaaacttca ttttctcagg gaagccctcc tgactctatc 183000
   cagccgacac tctccttgta aattctctaa gcactgagtg tctcctttgc agctgttgga 183060
   agtgtacatt ggtttatgct tctttattgt tttccccaac tagactgaca tagggaaggt 183120
   actcaacgaa tatttggtga ctaaatggtt aaataagtga atggcttata gctgtagtag 183180
   aaggaattgg cacaggaaat caagactgaa gtccaagaag tggaaatata tataaggttt 183240
   attgggaaaa gtcacaactc agtaggcaca gggatgccct gagcagtgat aattaataat 183300
   taatagttaa taataatagc ttgtacttat tgagtgcctg ctctgtgtca ggcagctgtt 183360
   ttaaaggttg tgggtaattc attccctaga acctcacaaa acaaatattg ttatccccta 183420
   gggataacta ccgctagggt tattatccta gctcccccta gggttgttgt tcctagacag 183480
   ttaaggttaa ctagggataa catcaagaga gctggcccaa ggtcaccatt tcttcccagc 183540
   ttttaaccaa cgttttgaga atacagtatt ccccatcaaa atcgtggctc accacggctg 183600
   tgagtttcac ccagcggcct gggtggtcat ggtgaaagct agaaatacat ctctggaaga 183660
   cattttcagg tatgctacag aggctggcat acccgggcta agagctgctg gcctggctaa 183720
   tgaactaaga tcactcgttg aaacaacagg tataaatctc caagcagaaa agactcgctt 183780
   aacatggatg gcaaatgttg tctaccaaga agacccaaga aaataatcaa ataccctttg 183840
   ccaagaatgt ctggttataa aataaagata taaatgacaa taacttttct gtattttagc 183900
   aacaaccagt taaaataaat ttgaaaaaat cccagttaaa atagcaataa agaggtaaaa 183960
   tacctaggaa taaatactta ggccccaact aagagagttt atgggggaaa aaatcttttc 184020
   tggaaaaaca caagaaggct caaattacat gtgaattcca cccctagctc ccccaatctg 184080
   tttttctcct ggcagccgtg gttctatatc acatcccacc tcacccctta gctcagccac 184140
   agcacccagc tctgcacaat tccttattct aaattctgat tggcccagtt cttccttcat 184200
   aaatttcata tcactgaccc aaccttggtc tattcagcat tggctggaag tatgaggtca 184260
   gatgctttac tgtccactga gctggtgctg taggttggaa tttttctaga agggtcaata 184320
   agttaagcag ccaatgattg gcatttccag cacaatctgg gagaatacat ggataagaat 184380
   aaccaagaaa aatttgaaag taagcaggaa tgctagggga ggggccagtg ggtataaagg 184440
   tgtgtgcctc tagataccaa gagcttatac tatggtatgg tgcaacggtg gggagagaga 184500
   gacagatcag tggagcataa gacaatatta aaaaatgagc cgagaataca taagaaattc 184560
   gtgtatcata aaagaggcat ttcaaatcag cagggaaaag atgcatttca tacatttgct 184620
   gtctaaacat taagagtaga aataaaggtc tttcttgaga gctcatgcta aaatgaatgc 184680
   caagggaaat gatgatttca atgtaaaaaa gtgaaaccat aaaagactac cagaaaatat 184740
   caggaaggac tttctaaata cgacagcaga gacagaaacc atacaggaaa agggaaaaat 184800
   ttgcacttta ttaaacacgc aggcacacac acacacacac acagacacgc acatgcgtta 184860
   tacgttgaaa aaagaacatc gacaaattaa aaggcaaatg agaaactggg aaaacattta 184920
   ctgcatatat cataggtgaa gggttaatat ccttaaataa tacacaggaa aatttgttac 184980
   aaataatttg aaagaaacaa acttgaattt taaaacaggc aaaattaatg ataacagtga 185040
   acatttcttc tttttttgtt tgtttgtttt ggaagcagga tcttgctctg tcacccaggc 185100
   tagagtgcag tggtgtgatc ctggttcgct gtagccttga actcccggcc tcaagtgatc 185160
   ctcccacttc atccccacaa gtagctggga ctataggcat gcgccaccat aactaatttt 185220
   tttttttttt tttttttttt tagacagagt ctcgctctgt ggcccaggct ggagtgcagc 185280
   ggtgcaatct cagctcactg caagctccac ctcctgggtt catgccatac tcctgcctca 185340
   gcctcccgag tagctgggac tacaggtgcc cgccaccacg cccagctaat tttttgtatt 185400
   ttttagtaga gacggggttt caccatgttg gccaggatgg tctcgatctc ctgacctcgt 185460
   gatccgccca cctcggcctc ccaaagtgct gggattacag gtgtgagcca ctgcgcccgg 185520
   cccataactg gctaattttt aaatgttttg tagagataga gacaagatat tactatgttg 185580
   tccaggctgg tctcaaactc ctgggctcaa gcagtcctcc caccatggcc tcccaaagtg 185640
   ctgggaatac aggcgtgagc caccggacct ggcagaacat ttcttaaatg ttcatcttca 185700
   ccagcaactt tatgcagttt ccatgcagta tctcactgaa ctcttactaa cctaggaatt 185760
   aagcattatt attattccag tttccacaca aggaaacgaa aacttaaaag ggttaaataa 185820
   ctttccctag agtcagaaag ttaataactg gaagagccaa gagttaagct tagatgtggc 185880
   tactaattca cccctatatt gacatggact gcagtgcaga tcttaaccca ggaaatggta 185940
   ttttaaattc tgccaaagtt tatacaatag aagcttatcc ttcaaatgga ataattacgt 186000
   attcgccaaa tgggcggggt tagaagaggg cagggtatat caggcggttg aagcaaatcg 186060
   tcacccaaca cacaagtgtt tacatggtgt tattggctct attcattctc ctctccagga 186120
   acaaaatgaa agatcgtcac caaattatag tgtccccttt tcaaatgtgc catgcgtgtg 186180
   taattacagc ttctaacatt ctccactttt catgctcaga caatctccgt ccactcagtc 186240
   tcaattaagg tttccctact ttttttgtct tttgcaaatt tttggccttt tgtttgcagt 186300
   tgtgggcatg gcagtactga tgtgttactc acggtggtag tacaagaggt gagatttttg 186360
   taatacccag ataaacccta aaatattcta ctttacatca aaagtataga aaagtctgtg 186420
   ctgagaagtt ttactctctg tctactttat tctctcttgc ctccatagat gaacacttgt 186480
   attaggtttt ttttttcttt tttttttttt ggtgtatcct gccagaggtg taattaagag 186540
   tcgactttcc ccggttactc aggaggctca ggcaggagga tcacttgagc ccagaagttt 186600
   gaggctccag tgaactatga tctcgccact gtgctccagc ctgggcaaca tagtgagacc 186660
   ttgtctctga aaataatttt aaaaagaaag gatacacact taacttattg cacctcgctt 186720
   tctcacacac acaaaaggta ccatgtaata tacacacctc agcaccttga tttttttgtt 186780
   tgtttcactt aaaaaataca accttggtcg ggtgcagtgg ctcacgcctg taatcctagc 186840
   attctgggag gccgaagcag gcggaccact tgagcttgag ctcaggcgac cagcctgact 186900
   aatatggcga aaccctgtct ctgcaaaaaa tataaaaagt agctaggcat ggtagcgtgc 186960
   accagtagtc ccagacactt cgggggctga ggcaggagga ttgtttgaac ctgggaggtt 187020
   gaggctgcag tgagccgaga ttgcactgct gcactccagc ctgggtgaca aagtgaggcc 187080
   ttgcctaaaa aaaaaaaaga aaaaaaaatg agattttttt ccatattaat gcagagaact 187140
   tcctcattgt actttatacc tgagtagtct tccattatgt ggctacacta tgatttattt 187200
   aaacaaccct ttcccagtga cacttagcgt gtttaaatct tatgctaccc taaacagggc 187260
   ttcagtgaat aactgtatct gtatattact tcctatgtat gcaggttatt ttaccttcgt 187320
   ccaacgtgat ggagtgaaag gtgaaaagaa tttgtaattt tacaaaagca tttgtacttt 187380
   ggataggtag tgccaaattg cccccacagg atgacaccaa ttggcccacc caccaggaat 187440
   atgtgtctct gtttctccac agcttttcaa cagaaactgt cgggatgaac atttaaaatt 187500
   ttcattttaa aattttaaat tgtctacttc attttaaggt gcattagaaa aggcagtttg 187560
   catattgatc ccatgagttc acactcttag tttcattttt aaaaataggt ttatttaaat 187620
   aaaaatggca gtactgaatt gaagaaaaat atcaagtaaa tattatgttg gtgcaaaagt 187680
   aattgcggtt tttgccatta ctttcaatgg taaaatccac aatgactttt gcacctacct 187740
   aatactgtta caggttgaat gcaggcatgc gaagttatga cagctcatga ctgaagctgg 187800
   ggaaatgttg ccatgatgat tttttctctt gggaataata aaatcaagag gttttatgtt 187860
   ctgtgtgcag gacactgtgc ttgaggtttt ataaagtgga tacggttatt ggacccattt 187920
   tatggctgag gaaccagaga ctcagggtgg ttaagtcact tccctgaggt cacacagcta 187980
   actctggagc ctgcgcttgt taatttccct ctgttctgag ggtatttcca actgggacct 188040
   tcaatttaac cttgcctacc taccccatgg gaagtagagc ctcaggagct ttcatagctt 188100
   gtaaatgtcc agggatgaca cagtctgaac ttgcaatttg cttttccttt ctcctctatg 188160
   gatgctgtta gcttgtgttt tctctctgtg tgcctcattt cagcttcctt attcctgcag 188220
   ttttacttca gcccaaccag atttcagccc acctgtgggc aaagctgcac agcccattca 188280
   gactgagaga gacttttctt tttctttctt tctttctttc tttctttctt tctttctttc 188340
   tttctttctt tctttctttc tttctctttc tttctttctt tccctccctc cctcccttct 188400
   ttctttcttt cttttttcct ttctttcttt ccttctctct ctttctttct ttccttcctt 188460
   ccttccttct ttcttttcct ccctccctct ctccctccct tcccttcctt cctctttctt 188520
   tctttctttc tttctttctt tctttctttc tttctttcct tccttccttc cttcttcctt 188580
   tcttcctttc tttttctttc ttttctccct ctccctccct tccttccttc cttccttcct 188640
   tccttccttc cttccttcct tccttccttc cttccttcct ccttccctcc ctccctcccc 188700
   cctcctccat ctctctcacc tgtgcttcct ttcctcttag ctgcctgtgt ctgggattaa 188760
   gtgatgacct aggatctgcc tgtctttgat gctggggatt gtttttcact ataaagactc 188820
   tggtttgggg gtggcaggga cccaagtact ctgtcactgg ccggtgctga atttgtgtga 188880
   gatgaagagt gttattgtct ataacccccg tgttaggtgg gagactctct ggggaggctg 188940
   aggagcaggg gggcagccag gggacactga ggggttccca ctcggctggc tgcatttctg 189000
   gagtctgaca aacttggctt ccagtcctgg ctctcccatg tccttgcttt gtgaccttgg 189060
   gcaagttact tctctgagcc tcagttttct catctggaat accaggataa cagtctaact 189120
   cctccatcac tgggctgtca ggagaatctg gggacatgac atagaggact cagcacagtg 189180
   cttggcaggt agtatgtgtt caattcaata aaattggtgg cagtaaaagt cacattcgac 189240
   ggttattttg ttgttgtatt caacaatctc ttgaataaag attggaaatg gaaggaaatt 189300
   tgggaatcca agcttgccta ataccaggca tgtggagatg gagcctcagc aacaggaaat 189360
   actgggaaga ggaatcgtgc caggatcctg tggaaacctg atttccaggg gtgggcgaga 189420
   tgactgggga tgatcaaggg gtgatgctgc ggcaggagtg taatagtttc aaacctgttt 189480
   ttgagagcac ttgagactga gtcagacaaa ctcactcggg agtttagggg atgtgttcac 189540
   atggggttgg attccagcac tgttgggaag aaggtaggga gtctggcaga ggtcccaaat 189600
   ccaaattcct acagggtcca ggcaggaaag gggaaagaga gaagcaggcc tggtgtaaga 189660
   caatagggaa gagtggggac tgtggaaaac tagaaagttt attacttatc taaaggggaa 189720
   aaccactgct cagcccacag aaatgagtgt tctgtgttgt cacagctgat gatttctcaa 189780
   aaggaagcaa tatttcacat tttttattct ttctttcttt cttttttttg tttgagacag 189840
   agtctcgctc tgttgcccag gctggagtgc agtggtgcga tctcagctca ctgcaagctc 189900
   cgcctcccgg gttcatgcca ttatcctgcc tcagcctcct gagtagctgg gactagaggt 189960
   gcatgccgcc acgcccggct aattcttttt gtatttttag tagagacagg gtttcaccgt 190020
   gttagccagg atggtctcga tctcctgacc tcgtgatccg cctgcctcgg actcccaaag 190080
   tgctgggatt acaggcgtga gccactgtgc ctggccaaaa tttcacattt taatctgata 190140
   gttcctgact taaatatagt aaacttattt ggatttctaa gaagcacctt gttggccaac 190200
   ggtagcttct ttacaggctt gatatagctt ctgggttcca cactgtggcc cctgggcatg 190260
   aaggagggcc caggccaggg atgggacagg ctctaggtgg cctggaggtg tctagtcagt 190320
   gtggcctggg tgacgaggat ctgatgtaaa atgagactag gggagggatt gaggcagaga 190380
   tggaagctgt gtcttttttt ttgtttttga gtcagagttt ctctctgtca ctggagtgca 190440
   gtggtgccat catggctcac tgcagccttg aattcctggg ctcaagcgat cttcccacct 190500
   tggctgggat tacaggcatg agccactgct cccagccagg aagctgcatc tttgaatgcg 190560
   atcagaacca aaccccatag agggagtgat ggcttacagg ccatgaggat tcccagactc 190620
   atctcaggct tggtcaatac ccacagcatc aaggctagga agggaatgta ttttggttaa 190680
   aggcaggact gatacttatt tatttatttt gagacggagt ctcgctctgt cacccaggct 190740
   ggagtgcagt ggcgggatct cggctcactg caagctctgc ctcccaggtt cacaccattc 190800
   tcctgcctca gcctcctgaa tagctgggac tacaggcgcc cgccaccacg cccggctaat 190860
   tttttgtatt tttagtagag acggggtttc accgtgttag ccaggatggt cttgatctcc 190920
   tgacctcgtg atccgccagc ctcggcctcc caaagtgctg ggattacaga cgtgagccac 190980
   cgcacccggc caggcaggac tgatatttaa cctgcatgcc ctggcactac tgtaccagtg 191040
   gtggaaatat tgaaatactc tgaaaccgat gggtaaatag ccactgggtc ctctgggcct 191100
   tttcccccaa ccttccactt acccatatct gcctctgcca gctcctacca caccccctcc 191160
   atgacgtggt gactagagtg ctaactccca gcacagccag gctctgctgt ccattccgtt 191220
   tggtctgtgg gcagtccctg agccaaggca attatagata tcttgactca cccctggttg 191280
   taggtaacat gcctacctgg attggctgaa gcacaaagga caaattttag aggacacagg 191340
   gaggcagatg gaattggaag gcaggatgcc agaaccccag ggaccagaac caggagcttc 191400
   atccctgggg tcatgtagtc tttgtctttg tttctccagg ttcctcagag cgcaccataa 191460
   aaatcagcac caactgtcat ttgctacaat gtggatgaac ctggaggaca ttatgctaag 191520
   tgaaataagc caggcacaga aagacaaata ctgcatgatc tcacttagat gtggaatcta 191580
   atgaagttga actcacagaa gtagagagta gaatgatggt taccagaagc tgggggacag 191640
   caggaggcag gagtagggaa gaaatggaga gttgctgagc aaagagtaca atgtttcgga 191700
   taggaggaat tgcctttgag atctattgca cagcagggtg acattagtca ataatatgta 191760
   tgtttcaaag taattaagag agtaaatttc aaatatctca agctaagaaa atgataggca 191820
   agttaggtga tggatatgtt aattttcttg atttaatcat gccacattgt gtgtgtatat 191880
   tcagaacaat cagaacaatc acattgtacc ccataaatgc atacaattat aatttgtcaa 191940
   tcaaatataa aattagcagc ctcagccctg gaactgtatg tctgctggcc tcaggactga 192000
   ttacctgtgt cccagccact cagcatatgt gacccttgag ccaagtgtcc acctctgccc 192060
   cagtcagctg tgttcaggga atgtggatgt ctcaaagtac aagcatggct tttgggaggt 192120
   gcccatctca gagatggcat tgggctcagg caggcatgga aaggtaccct gaaagatgca 192180
   ccttgagggt cgtggaggga gtcatgagga ccacaggtgt aggagtgtgg ctttaagctc 192240
   tttcaggtag caggaaggag gagggaagaa tccactgagg atgacctggt ttgtctggtc 192300
   tgaaggattg gggcagtggg tgggtagagt tggtgcctac aagtatgatc cctcatgaaa 192360
   gcggagggag gggaatgagg tcagaggagc tctccagcag cctctaaata agccgtaaga 192420
   agtcaatgtt tccctgcaac ttgccgattg gttttcctgt ctctcatctg gctctaatct 192480
   gtgctgcaca gccccttcta agtcattttc ctaaaatagc atgtttatgg tgcaggggtt 192540
   tgcagtggcc aacagcgatg attgcagccc tacagctgtc acaacaaccc attgaccact 192600
   gtgtgcaatg ctgtgcttgc cacgggcaaa acacttcgtc tcttagttct tcggtttcct 192660
   catctgtaaa acaggggtag taagtagtat gtactttata gggctgttat gaggatcgaa 192720
   tgcattaatg cctgatgaat cccttaaaat agtccctggc atgaataaat gttccataaa 192780
   tgttaactct cacttattaa gtatttattg agcatcttct gtatgccaag tacagtttat 192840
   gccctgagag tataatgatg agcaaaattt cctcacattg catgcagtga agtgtggaag 192900
   agatgcaatt aagcgtgcat ttataagtca gtggtttaag tgccgtgatg gagaggattt 192960
   agaatataag ttccatgaaa gcagaagctc ttttgttcac agctgtatcc ccaatgccta 193020
   ggaagaactt gaccctcagt atacatttgc taggtgaatg aacgaatgag tcaagttcta 193080
   tcttagccgc acatgaagat gaggtgtcaa tggagtaaag ttgggagtga ggagagaagg 193140
   gaggggaact tggtgtgaac cccatctctg caagggtcca gctgggtggc cttggtgaag 193200
   ttactctgta gttaggatct gtttcattgt tgctaaaatg ggaacaaatg ataccttctg 193260
   catggcatgt cataaggatt ggagagagta catgtgagac acccagcgta cacatgtagc 193320
   tctcaagcag tgtgttgaga ctgggtgtgg tggttcacac ctgtaatcct agtactttgc 193380
   aaggctgagg caggcagatc acttgagccc aggagtttga gaccagcctg ggcaacatag 193440
   caaaaaccct gtctctacaa aaaataaaaa ttagccaggc atatggcatg tgccttcagt 193500
   cccagctgct caggaggctg aggtggaagg gtcgcttgaa cctgggaggc agaggctgcg 193560
   gtgagctatg atcgcaccac tgcactccag cctgcatgac agagcaagac cctgtctcaa 193620
   caagacaaaa aacaaaacac acacaaacac agaaatgtgt ttccttttcc cttcccattc 193680
   tttgtgcttc ttggtatctc ctcttgttta agagactgtt gatatggaga aaggtctgga 193740
   tcatttttgg tcagaaatgc ttgaatcctt gtcttggaga tcgctcaaga aaatatttac 193800
   atgaagaaaa gtgcaaaacc aacagaggca acttattatt agttgtgatt ttgccccaga 193860
   tcttaaagta gagctcacaa ggccttgagg aactcctttc ttctctacta ggcaggacta 193920
   aggaaattca aacctgaact caaacctaac tcttggcctc tgtaagatca atatatcaga 193980
   gaaaccagaa gggaccacaa gagccaagag tagagacaga atgtgtccta ggtcttggag 194040
   aggagctggc tcaggccact tctaccaagg gtctcagggc aatactataa gacttggacc 194100
   cagtagcgtc ctgggagaag tgaaggatgt aagggagaag gagaagagaa agatggcaag 194160
   gatgcctttt aacctccatg cgagctccac cactggggtc tggccaatcc gtgtcttgga 194220
   atccatccaa ttaaatcaac tctgcctcct tagaggttaa gtaggggtgt aagagaacct 194280
   ggagcttctc ctacaggcca ataccagtca tgaacagggg ttcctttttt attattatta 194340
   tactttaagt tctggggtac acgtgcagaa tgtgcagttt tgtttcatag gtatacacct 194400
   gccatggtgg tttgctggac ccatcaactt gtcatctaca ttaggtattt ctcctaatgc 194460
   ctcccctagt cccccacccc ccgacaggtc caggtgtatt atgttcccct ccctgtgtcg 194520
   atgtgttctc atcgttcaac tcccacttat gagtgagaac atgcggtgtt tgattttcga 194580
   acaggtgttc ctttttaggt gggtttgaag cactgaactc ccaactttaa ggtgaatgtc 194640
   caagaagcac aatgggaact gttctgtggc acagagcagg gggactctga taggaacctt 194700
   atgtattcac ttagtctcat tgtccggaga tagagacatg gctggttaag ctgccaaaag 194760
   ggatccctta gtttttgggg gggtggattt ccaagccctg ttgttgttac taggacggca 194820
   cctcaatctt tgagcaggac agatgattgc tggggtcctt ttgtagctga gagcactcag 194880
   ggtcccactg gcgacagcag aaaagacctc gcttctcaaa cttggctgtc cagtgctctg 194940
   gacgagtact tcatctgcct gactgcagag ggtgggaacg ttgtgaaaga aactttgtag 195000
   ctcattaaca agataagaag caaccgaagt ctgcagtggc tgaggatttt cagtcaggtt 195060
   tattgagaca tacgtttcct gtggtaaaag tcactttaaa caattttttt tgagacaggg 195120
   tcttgctctc ccacccatgc tggagtccag tggcgtgatc atagctcact gcagccttga 195180
   actcttgggt tcaagtgatc ctcccacctt ggcctcccta gtagctagaa ctacaggcat 195240
   gcaccactat gtctggcttt aatacatata tatagtagag acagggtctc actgtgttgc 195300
   ctaggctgat ctcaaactcc tgggttcaag ctcaagcagt cctcccgcct tggcctccta 195360
   aagtgttggg attgaaggca tgagctactg tgcccagcaa aattcacttt ttttcttttt 195420
   tctttttttt gagatggagt cttgctctgt cgcccaggct ggagtgcagt ggcatgattt 195480
   cagctcactg caacctctgc ctcttgggtt caagcaattc ttctgtctca gcctcccaag 195540
   tagctgggac tacaggtgca tgccactatg cccagctaat ttttgtattt taaatagaga 195600
   cagggtttca ccatatcggt caggctggtc ttgaactcct gacctaaggt gatccaccca 195660
   cctcggcttc ccgaagtgct gggattacag gcatgagcca ctgctccagg ccacaaattc 195720
   acttttaaaa atataaagta aaattcatca cagttaatat atagaccact ccaatatcca 195780
   aaacaaaaaa gttgctgcat ctgcctcttt ttagttaatt ccctatccac ctccaacccc 195840
   ttttaaccac tgatgttttc tgtccccata gttttgtgtt tttcagaaca tttagaaatg 195900
   aacctacact tgtgttgaac atctttgttt taccagaatc ctttcgaggt tcatgaatgc 195960
   tgctgcatgt gtcaatagtt cattcctttt tactgctgaa aagtattcca ctgtgtggat 196020
   gcctcacact gtatccattc acacgtcgaa cgacatttgg gtcctttcca gtttggggct 196080
   attataagta aggtagctat aaaccttcat gtatggatat ggttgttgtt tttttttttt 196140
   ccccttctct gtcacccagg ctggagtgca gtagcacgat ctcggctcac tgcaacctcc 196200
   gcctcctggg ttcaagtgat tctcgtgcct caccctccca agtagctggg actacaggca 196260
   tgtgccacca caccctgcta atttttgtat ttttaataga gacagggttc ctccatgttg 196320
   gccaggctgg tctcgaactc ctgacctcaa gcgatctgcc cgcctcggcc tcccaaagtg 196380
   ctggaattac aggcgtgagc cactgtgccc ggcctggatg ttttcatttc accgaaaagt 196440
   aggattcctg ggtcctagga gagttccagt ttatgttctt tcttcactaa cacttggtat 196500
   cgttggtttt agttttcaaa cattttagca actctggtag gtatgtgata tggttaggct 196560
   ttgtgtcccc acctaaatct catcttgaat tgtaatcctc ataatccaca catgtcaagg 196620
   gagagaccag gtggaggcaa ttgaatcttg gggcagtttc ccccatgctg ttctcatgat 196680
   agcgagtgag ttctcacacg atctgatggt tttataaggg gcatgtcccc ctttgctcgg 196740
   cacttctcct tcctgctgct ttgtgaagaa ggtgccttgc tttcccttcg ccttccacta 196800
   cgattttaag tttcttgagg cctcctcgtc ctcaagaaaa ttgactgtta gtcaattaaa 196860
   tctctttcct ttgtaaatta cccaatctca ggcagttctt tatagcagta tgaaagcaga 196920
   ctaatacagt atggaattat tttaaagttt tgaaaagaac agtttcctta ggagaagcac 196980
   tcatcacatt aacagcacat aaaagaatat ctatgtagta tgtctgttta tttgcttaca 197040
   tgttcatttt ctgtcactag agtacaattt taaaggaggc atctgtggca gcatcaataa 197100
   atacatgtga atggatgaat gaaggaagga agaaactggt agcatgtacc aaaggctagg 197160
   gtgtcagatc agccctggat tggaacctaa ggcccatgtt attaacttct gccatgtact 197220
   tttttttcct ttttttaaga gagagagggt cttgctctct tgcccaggct ggagtgcagt 197280
   gatgcaatca tagctcacac atagccttga attcttgggc tcaagtgatc ttcccacttc 197340
   aacttcccaa gtagctggga ctacaggcat atgccaccac acctggctaa ttttttattg 197400
   ttgtagacat ggggtctcac tatgttgccc aggctggtct tgatctcctg gcctcaagtg 197460
   atcctcccac cttggcctct caaagtgctg gggttatagg cgtgagacac tgtacctggt 197520
   ctgcaatgta taacagagaa agcaactttt acagaaagag ttctcttgaa ctaaaaccaa 197580
   ttctgtgagg aaagaatctc ttgaagaaga aagagaaatg cagagccaaa ggcttacaaa 197640
   gggcatggtc tgtttgggag cgggggtgcg ctctgagtgc tgagaatgta catagtatgt 197700
   gtgcatgtgg gtgggtggga ggaggggcct tagctgggga tcaccttggc caaatagtaa 197760
   tagcaatcat ggaaagctta tgctgcttgc tatgtgccag gtggttctaa gacctctaca 197820
   caggttcatt catttatgct tcccaacagt cttataagga gagcactagc attatcagca 197880
   ttttacagat agggaaactg aagcacaaaa agatcaaatt agctgggcat ggtggtgtgc 197940
   acctggcatt ccagcaattt tggaggctga ggtgggagga tcatttgagg ccaggaattt 198000
   gagactagcc tgggcaacat agtgagaccc ccctctctac aaaaaaaaaa aaaaaattaa 198060
   aaattagctg ggcaggttgg gcacagtggc tcatgcctgt agtcccagct actcgggagg 198120
   ctgagacaga agaatcactt gaacccagga gacagaggtt gcagtgagcc aagattgtgc 198180
   cactgcactc cagcctgggt gataagagca aaactctatc tcaaaaaaaa aaaaaagatc 198240
   agctcagcat agttgcatgc acctgtagtc ccagctactc aagaagctga ggcaggagaa 198300
   tcacttgagc ccaggaagtc aaggtcccag tgagctataa ttgtgccact gcactccagc 198360
   ctgggtgaca gagcaaaacc atgtctctta aaaaacaaaa gaaaacaaaa aaccatcaaa 198420
   caacttgcac aaactcacac agcttagagc cccggaagtt tatccaagtc tgccttttac 198480
   cactgcactg cactgccagg ctatcaaggg catatttatt gattaggaaa ttatgtaagg 198540
   ttctgtgaga aatagctccc aaaatatagt gacttaaaca aggcagaaat taggtttttc 198600
   tctcaggggt cagcccaaag ggacaaaatt caaggctggt agggtggctt tgtcctttcc 198660
   acatatggct ccaagtttgg gtccaggaca gtcacttcca gttctcacca tctttgaacc 198720
   agtggggaga gggaaaggct caaggaagta tgcatttcca tttgaaggga atggccctgg 198780
   agcacacaca tcccattggc cagaactcag caccatggcg gcacctgcaa gaggtgctgg 198840
   gaaatatggc ctctatctgg atagccatgc tctctgctaa aactcaagga ttctactatt 198900
   aaagggaaga atggatattg gtggaaaacc agcagtctct gccacaactt gcagactgtc 198960
   cactgggctt tgaactttat tctgtcagtg atgaggaact ggcaatggtt tctaagcagg 199020
   gagatttcac atccagactt gggtttttga tcactgcaga agcatgcttg gagaataaag 199080
   gtggggagaa agaaaagtca ggagccctgt ttagatgatg tgtctgttac tttgttttcc 199140
   catctctgga gtccctacta caggcaggag cctgtaggtt gagttcccag gcaggcccag 199200
   atggagggtt tgcaggcagg gtgagtgagc atggggctgc aaagggaaca atcatctgtc 199260
   tttgtccttc attccttcca caactattta tccagcaccg tttctgggca cagggctcca 199320
   gcgatggtcc caacaggtac aatgacctct ggggaccaag ttcagttctt ggtgagttct 199380
   ccagtgcctc tcgatgtagg atgaaccgtt ggcatgctcc actgacgctg gctccttctg 199440
   ttgtttctct tggctccagg acccccgcag caaacacaag tttaagatcc acacgtactc 199500
   cagccccacg ttttgtgacc actgtgggtc actgctgtat ggactcatcc accaggggat 199560
   gaaatgtgac agtaagtact ttttctctct gggggcatct gctgatggca gaagcaatgg 199620
   gaagggctgc ttccacttgg tttggggtcc aggtctgcca tacattcccc cctgtcctcg 199680
   ttggggctgg tgtaccagtt atctgttgct gcataatgat cctcccaccc caaaacactg 199740
   tgactgaaga caataaacat ttttttagct catgactctg caaggcagtt ctttgaatct 199800
   gggctggcct cagctgatgt cacgcatgtt cataaagcat gaactcatgg ttcatggtgg 199860
   attagcagat ggaggtgggc tgggagctgt ctgggccatg gtggcctcac ccacatgtcc 199920
   tggaggatgg ctggctgtca tctgggtgat ggtgatgacc aggccacgtg tctttcctcc 199980
   tcagtgggct aggctaccta gcctccgtgc atgcacggag gtcacagggt cctgagtttc 200040
   ttaggagggc aagtcccatt ggcagccaca cttcaagtct ctacttgtgt cttttgctcc 200100
   tgatccactg gtcaaagcaa gtggcatgtg agatgcacag tcacagtgtg ggaaggggca 200160
   gtccatggct gttgacaccc agaggcatga acaaatcatg ggcatttctg cagccatgca 200220
   ccaccactgt tttcagcatt gagatgtggg gctgatgcat tctttgcatg gagaagacct 200280
   gtggccctgc agcacccaca ggtttgtgct ggaggaggaa agagcatcat tctctggcca 200340
   ggatgtggta ggaaaggcag gtggggtagg actcctcttt atgaggccaa ggcccggatg 200400
   gcctgtgggg caggggagca gacaggcttt gagagctatg gccacaaagc tacagggaca 200460
   ctggaggtgc tggtggctcc cacagacctg ttggtctgca gcaggctggg tgtcatgagg 200520
   ctagtggtac tgctctccca agggtcctga ggcccagagg acagagggag aaagtcatag 200580
   ggcagagagt gacagagagg actgtgaggg tgaataaggg tggtagggag caagacagac 200640
   agcaagaaac acacatgcat agacagacac agatgcaggg gaaagatgga cacatgcaac 200700
   cctaacacag ggaggaatgt ggggcgtgga agctacagag agactcagct agagagagac 200760
   tcaggcaaaa ggagggacag gcagacacac agaccaacag ataagccagg tgcagtggtt 200820
   cactcctgta atcccaacac tttgggaggc tgaggcgggt ggatcaccta aggtcaggag 200880
   tttgagacca gcttggccaa cgtggcaaaa cccaatctct actaaaaata caaaaattag 200940
   ccagtcaagg tggtgggtac ttgtaatccc agctacttgg gaggctgagg caggaaaatc 201000
   acttgaaccc aggaagcaga ggttgcagtg agctgagatc acactactgc actccagcct 201060
   gggtgacaga gtgagactct caaagaagaa gaagaagaaa agataaaatg attggcatgg 201120
   acacagagag agacgccatt acacaggctc atgcagaggt atagagatgg aaagaccaga 201180
   gacagagaca gaagcacaca tggagagcca cagagagaca cggaggagag aggcagagag 201240
   acacagttgg agaaagggag agacaaagag gccagagaca agtgcagaga gtggaacaga 201300
   ccgattcaca gacacggcca aggcagtgag ccacgtgcag aagtgggaca gcagagacaa 201360
   gagacatgca gcaacgtgga aacagagaga cagacacaga aacatggcaa gagccacagg 201420
   gggacagtga ggttggagcc ttctgcctgc aggacaccag ctcctcttac cacacatggg 201480
   gccctaatat agggacaatg tccctgcctc agagacagtg gtgggtggga gctcaggctg 201540
   gtgctcaggt cccagagggc tctcccttcc ctccccacaa ctcatttcta gtttttgtaa 201600
   taggcctgct gcctcctggg acttgggtgg tctgagaagg gtcccttgcc cccagatccc 201660
   ttgaaatagg ggcccatggg gtgacttggc ctctccttct ggcttctcca actggaacta 201720
   gcgtccccct ggagaatcct gggtgcctgg cacaccttcc tggagtgggg cattattgct 201780
   caagacctag aagaaatcac ccaccacatt gaaatagaca cagatactct acggaggagt 201840
   gcgcagaggg agtggatatc ctataacatg agagatggcc gaaggccaaa ttcagtgtat 201900
   aggcacattt tatccaccct gccctggctc taaatctttt tccttgtaat taattatggt 201960
   ggacactatg gccagcccag accccctctt catggtccca ccccatcccc tagctgtggt 202020
   tcacagcagt gccccatcct gcataattgc ccaagataat gcctcttcca gggagaccag 202080
   atgattgtgg tgcccagccc tcttgtctca gtttggaaca actctgaagg gtcatctcag 202140
   ctctgggctc cccctaacat cagctttgat tgcatccacg tggcaaacga acttctcccc 202200
   acaagagcac tccccaaaaa ccttgtgctc aaaactcccc gtctcagact ctttccaggg 202260
   agcccaacct attagctgtt aatatttaaa aatcagagct gtcacgtaca catcgagatt 202320
   cctgcatacg ttgagaaatg gggaatgttt ttgctctgtt tcccacttgg cttggccaag 202380
   tccctgccac ccccttagct ttcctcactc atgtgaccag ccagcctgtg agcattgaag 202440
   ttggtgactc tggcttcaga gccttaaggg gtgtggggtg tgcttgcttc agactgcacc 202500
   cttggctgtg ggtggggaac agacaagttc tacctccttt gcccatcagg gttcaggggg 202560
   aagagttgga aaagcccttc tgatgagatc ctggcaggca agttggtctc agccaggctc 202620
   gtgtgtctca gctgtctcaa aggagggaaa caggaagggc tcagcggtct tgggtggggc 202680
   agatgtctgc agcccccagc ctgggccagc ctaagccaca tcccctctct ctgccctcac 202740
   agcctgcatg atgaatgtgc acaagcgctg cgtgatgaat gttcccagcc tgtgtggcac 202800
   ggaccacacg gagcgccgcg gccgcatcta catccaggcc cacatcgaca gggacgtcct 202860
   cattgtcctc ggtaggtggc cctggggctc cactggctcc tgaccttgct tgacctgtgt 202920
   gattgagaag gggagggtgg cggaggggtg gggcagtcca gtggaggggt ggggcagtcc 202980
   agggacgggg agggggtgtg gcactgcttc tgccccatgc cctgcccatg acagcccctc 203040
   cctgcagcag ggggatcttt agaaactgta caccctcaaa gcttcgtcca tcagcacccc 203100
   aggctgactt gggttcagag aggagacgtc tgagttcctt ggacttctaa cttcctcagg 203160
   atcctccact tcccttctct ccttctcatt tcccactctt ttttagtaat cttttgggat 203220
   gggtagttgc atttccttta ctcagatagc agagaaggga agcttgcttc ttccgcaaac 203280
   ttttccagca ggaaaaatgg agtcttgatg ataaacccca gatttggaga atataatatc 203340
   ttgggaagga ggagggttgg agggtgcaat tcaggggctt tccacatatt tgaaatattt 203400
   ctttcttgaa cctgatggtg gctatgctgt aattttatta ttctctatat tttctgcatg 203460
   tctaaaatat ttcatgataa ttagaaagca ggatggtaca gtctcctggg agatgcagtg 203520
   agaggaggag ctgctggtgg tggtggtggt ggtggtggtg gggctgtcac tcatgtatcc 203580
   cagcattcaa gacatacctg gctctgatat gggaagttca agcaaaattg gaactcagca 203640
   gcactggctt gaggaatact cagccataag ccccggtata accagcactg agctggctgg 203700
   gagtggagct aaaactttgc aggaatttct tctcaaaggc tagtgacaaa ctactgtgga 203760
   ctttatatat ctaatgaata taaataaggg tttatgagtt ggtaactcag gaaattgatc 203820
   atggaagaga ttaattttgc aaaaattagt gtttctgagg cctcagactg gttcagtagc 203880
   tctgatgcat tgagccctgg gttgcggagg ggctttacgt agattgtgta aaagctgaaa 203940
   aatacttatc cctgtacaat ataattcttt taaacttgag atttcgtgaa gcccagtgag 204000
   attttagaaa atgtgcaatc cattgcatta tgttttcctc caagtggtcg cctgcctccc 204060
   agtgtgatgc ttgggggccc caggggaaat cccgcagttt caggttcaag ggaagccagt 204120
   tttccccact gattgaggtt cccgatgctg gtgagacaaa tatgagactc tgcttccctc 204180
   tggtgtcatt ctgtgggaaa tacactcgtt ccaaagcatg actgcgggca gccaacattt 204240
   atgggccgca tgggatgtgc agtgctctaa accttacgtg ttatctcttt ccaccttcac 204300
   aacattgtga aatctctatt attcttgttt gagagggaga ctccatctgt tgcccaggct 204360
   ggagtgtggt ggcgccatct tagctcactg gaacctccac ctcccgggtt caagcgattc 204420
   tcctgcctca gcctcctgag tagctgggat tacaggcgcc caccaccacg cctggctaat 204480
   atttgtattt ttagtagaga cagggtttca ccacgttggc caggctggtc tagaactcct 204540
   gacctcaagt gatccgccca tctcggcctc ccaaagtgct gggattacag gtgtgagcca 204600
   ctgcgcccaa ccttctatta ttcttgtttt atgaacaaag aagccacggc tcagagaagt 204660
   tatattccaa gagctcacac catttgcaat aataataaaa caatagctaa gatgtgttta 204720
   gtttttaaaa tgtgttaatc attgtgaaaa gcacttacag gcagtatctg atttacattg 204780
   tttcaacaac tcaaatgagt taagttatca ttctacccat cagaaaacac atttcctcac 204840
   ctaggtctgt ttgactcaaa agtactatat agtaggttac ttttttgttt agtttagttt 204900
   cgtttcgttt ctctatgccc tccaccgtat ttattccttt tgtggtttgg ctagggcacc 204960
   tcctatgggc attccttagg gagtctttgt tctgatgtgg tacagtacaa tatttcttca 205020
   agttcaaggt caaagtcttc ccccttaatc atagatatgc tgtgaacttt gagaacctgg 205080
   atcacaagcc cccgcttctc aggacacact gataggaggt gtatccattt gctgtggctg 205140
   ccataacaaa ataccacaaa ctgggtggct taagacaaga cgtttaaaaa aaaaaaaaaa 205200
   gaaacgaaaa gaaaaagaaa aaaacaatag gccaggcaca gtggctcatg cctataatcc 205260
   cagcgctttg gaagaccgag gtgggtggat cacttgaact caggagtcag cctgggcaac 205320
   atgtcgaaac atcatctcta ctaaaaatac aaaaaattag ccaagtgtgg tggtgcacga 205380
   ctgtaatccc agctacttgg gaggctgagg caggaggatc acttgagcct gggaggtgga 205440
   ggttgcagtg agctgagatt gcaccactgc actccagcct gggtgacaga gcaagactcc 205500
   gtctcataaa acaaaaacaa gaaaaacaaa caaacaaaaa aggccaggtg tggtggctca 205560
   cacctgtaat cccagcactt tgggaggcta agacaggagg attgcttgag actaggcgtt 205620
   caagaccagc ctggccaaca tagtgagacc ctggcatatt gtaggatttc atttctttgg 205680
   gatgttcaga gcaggcaaat gtataaagaa gtttaatagc actttgtgct tttccaaata 205740
   ctttgatatc acaccattcg ggaaagggca cgttgttgcc atcatggaag ggaaaacgaa 205800
   gtctcagcaa ggctgaacac cctgaccaat gtcacatgct gagcaggggt gagcacctgg 205860
   gcctctggcc ccatgtcaca atgccctgca attgcccata caccatgctg ggcacagcct 205920
   gtactcagca tgggcttgtt gattgatgga tgatagaaga gagccctgat gctgtggccg 205980
   agttctctaa aaatagagcc aagatgggga ttttgttcca gtgatttatt gggctgctca 206040
   tgggaaaaga gaagtgaagg aaccatgctt ccttcctcag gcagggaaaa agcttggcga 206100
   gagtgtggtc tccgtggaag tcttcttcag cctggtatct ggtggaattt tgtaccaaac 206160
   actgtagcac agtcagtccc atcttgaggc aagggggctg ttctgtcgtg ttccaggtca 206220
   gtcagtgctg tgggatgact tgtgtctccc ccagatttgt atcttaaagc ctcaccccca 206280
   gtgtggtggt atttggagct atgggacctt tgggtggtaa ttagggttag atgaggttgt 206340
   gagggtgggg ccctcataat gggatttgtg gatttctaag aggaagaaga aagagagatc 206400
   attctgtctc caccaaagga aggatacagc aagaaggtgg ccgtctgcaa gccaggaagt 206460
   gagtccttac caggaattga agctgctggt acctcgatct tggacttccc agcccccaga 206520
   actgtgaaga aataaatgtc tttttttctt tttttctttt tattttgttt tagatggagt 206580
   cttgctctgt cacccaggct ggagtgcagt ggcatgatct ctgctcactg caacctctgc 206640
   ctctcatgtt caagcgattc tccggtctca gcctcccgag tggctaggat tacaggcacg 206700
   tgccaccatg cccagctgat tttttttatt tttagtagag acggagtttc accatgttag 206760
   ccaggatggt ctcaatctcc tgacctcaag tgatccgccc gccttggcct cccaaagtgt 206820
   tgggattaca ggcgtgagcc actgtgccgg cctaaatgtc tgttgtttaa gccacccagt 206880
   ccatggtatt ctgttatgac agcctgagct ggctgatata gtcagttact gctgtaggct 206940
   gctggggatg gaggtgggaa ggtagagtaa cttcccaggt gaggaggctt ctgtgcagtt 207000
   gaggacagtt atctggacat aggggtaact gtgagccatt aacagccatc atacacagca 207060
   cccaggggat gggtgcattg gccagcatgg aggggagctg ggcagggtcc aacagcatct 207120
   ggcacactca agtttccctt ggtctcctga ctgtcttcct ggtttcattg ctgggactcc 207180
   agccccctcc tccttggtct tggcaagaca gatcagagag gagcgagcac taaaggttgt 207240
   gcggggtcct tcccagcagt gccaggccag aagtggcctt gtggtgtttg tggaatggcc 207300
   ccacctcatc cagacccaag aagcatggtc agtattagga ttttccactg accatcagcc 207360
   caggacactg ccgtttgccc tgagtgtcat atgttctttg gaaatctcag catctctaga 207420
   ggtcaaaggt tttctaactt gcctggctac tgaaattgag ctcatatatc tgttcttagt 207480
   gttttggcag gtcaagatga gaataaatct gatcagttct cctcctttta aaaacccatc 207540
   aatggtttcc cattgtcttt ataacagact aaatctaaca tggcgatctc attctttatt 207600
   cactgtctcc tccccctttc ttttaatcct tggtcctctg cagactgcca cacaccataa 207660
   gacatctgtg cgttctgttt agaatgtttg ccattgccat cttctctttt tctagcaatt 207720
   aactcttcct ctcgatctcg gttcattttt accatctaat aagcttcctc caacttcccc 207780
   ctttcgtaga cactcatggt aacctgagct tggccttgtg aaaggggtga ttttatgttg 207840
   gatttgatta atgtctgcat ctcatcacaa ggcattagct ccatgagggc gggactgtgt 207900
   cttgccactc tactcccagc acctagcaag gtgggcatca ctaaatattt atgggctcaa 207960
   tggctgtatg tatgaatgag aaacccctgg aggatcctga tagatagata taaggctttc 208020
   actgatggtc cccagatcag tgcctcctaa agtcaggaaa tgaaggtcaa cggattgtcc 208080
   aatttatacc attattctca tgggaggtag aacatgcaga aaatcttaag taccaggtcc 208140
   aacgtcaaca gcaaatgtta ctgggactga gacccgcttg ttggagtcat gaccaccctt 208200
   gttccctgga caatgtagaa aagacatgga gcagtgggat ttgtagtatc ccaggaaacc 208260
   gtattgacat catgatgccc ataggtgtca tgagatcagg caatacaatg tccagatcag 208320
   agttgtaaaa tgctcccaag gtttgtgaag cctgcgtgtt catacatgca acaataattt 208380
   tttttgtgtg tgtgtgacag agtcttgctg tcacccaggc tggagtgcag tggtgcaatc 208440
   tcagctcact gcaacctccc cctcccaggt tcaagccatt ctcctgcctc agcctcccga 208500
   gtagctgcga ctacaggcgc atgccaccat ggccggctaa tttttttttg tatttttggt 208560
   agagatggag tttcactgtg ttagccagga tggtctcgat ctcctgacct catgatttgc 208620
   ccacctcagc ctcccaaagg gctgggatta caggcgtgag ccaccgcacc cagctaacaa 208680
   tatcttattc aatgcttttt caattcagta tctacctatc aacataacaa ctgacatcta 208740
   ttatttatac tcccatctac attacagcca cctccaaatg cttagccatg tccatcctca 208800
   tatctatctc ccaaaagttg ggagtgttct taaagaaagc agaaagaacc tttttttttt 208860
   taaatgtttc tctttggaac tcccattcta tattcttacc attatattgt gttatacaga 208920
   aactttttag actgaaacat ctccctgaca gccttaggta ctcccaatat tgtgatattt 208980
   aaatccttct taagatctga catccacaca tcaaaagaaa aagagtacca aagaggctga 209040
   gtgtggtggc tcacgtcggt aatcccagca ttttgggagg ctgaggcggc tgtatcacct 209100
   acagtcagga gttcaagacc aacgtgacaa aaccctgtct ctactaaaaa tacaaaaatt 209160
   agccaggtgt ggtggtggat gcctgtaatc ccagctactc aggaggctga gacaggagaa 209220
   tcgcttggac ccaggaggca gaggttgcag tgagctgaga ttgcatcaca gcactccagc 209280
   ctgggtaaca gagtgagact ccatcttgga aaaaaaaaaa aaaaagaatt gggaagaaaa 209340
   agggcaatgt aaacaagaaa aaaaaatcca gatagcttaa tatttatttt tgtttaactt 209400
   ttatttgaac tttggtgggg gggtacgtgt gcaggtttgt tatataggta aacttgtgtc 209460
   atgggggttt gtgtaatatt gcccagccat gcgtcaattg cagacagaaa attattataa 209520
   actaggccgc gagggcccac tagggggcag tctctgcctt caaagccatt cctcctcgga 209580
   ggctctctca ggagatgccc caatttgtga aaccacagta ctgtcattgc tcataatgga 209640
   aggtgcagca gagaccagct acgctcactg aggtccagag aaaggttact tcttcaggtc 209700
   acacacagat agggcccagt tctggtagat ctcccttccc ttttacatta ttggtttgta 209760
   attttttccc aagtctttat tttgaactaa gttgaaagaa ttttacagca aacactcatt 209820
   tatccaccac ctatattctg tcgttaacgt ttccacactt gcttaatcac atttccaact 209880
   atctcgctct ccatccatca atctgtctta tttttgacac atttcaaagt aaactgcaga 209940
   caccaattcg atttccccca aacacttcag catgcccaac atcaatagaa ttcaaaattt 210000
   acttgtaggg tttctttttt ttttttaact tttgaggtga aatgtacata caatgataca 210060
   tacaaaattt tttttttttt tgagacagtc tcactttgtc acccaagctg gagtgtagtg 210120
   gcgtaatcac ggcttactgc agcctcgacc tcccaggctc aattgattct cccacctcag 210180
   cctacaaagt agctgggact acaggcatga gccaccacac tcagctaatt tttgtagttt 210240
   tgtagagatg gggttttgcc atgttgccta ggctggtctt gaactccatg gttcaagcaa 210300
   tcctcccact ttggcctccc aaagcgctgg gattatagat gtgagccact gtgcccggtg 210360
   atgtcttaag tgtacaatgg ctgagttttg acaaatgcat atgactgtca ttctaaccct 210420
   tatcaaatta attctctttt ttgatgtcac agctgctgtt gaggatccaa gggaaggtat 210480
   gaaccagaga aatgccttta tgtccccaca cactgcactc atactcatga gctctggcta 210540
   ttgggatgtc tattatacct aaaatccctt agatgacatg aaacttaata gttgttcaat 210600
   tttttttgtt tgttttttga ggagttataa tgcagtaaag tagaaacttt gaaaattgga 210660
   aattcttgtt tacaattttt tgttatgtat tcattgaaga gcaaaatgcc tttaaaaact 210720
   caatacacct gctttatttt gctttttttt tagtattgtt gtaatgaaga agttcagtta 210780
   aatcatatct gtctatggaa catctatcat ctttctcagt gtattactga tagttgtctg 210840
   cacaccccat gaggccaggg gaagggtctc ttgcccatcc ttccttcaaa gcctatcagc 210900
   ttgttaggtt gaggtgagtg tctcaggcac tgaagttttg ggatactgga cctaaggaga 210960
   tgtagggaag ctgacgtctt gctattcagt gttccttctc ttcttccctg gtcatttaag 211020
   tctcatgaga attatcaagg ccaggccagc gtctgaggcc ttcaggatgt gctgggcgcg 211080
   gtcattgctc ccaaatgtct agggaattgc attgaagtgg gcactgctgg gacctgcagg 211140
   gctgtgactt gtgactgcct ttggggacag tggtccagca atgtctatta aaatatgtat 211200
   acatatgaac attttgactc tcacgttttc ctttagggaa tctattctct taaaatgaaa 211260
   caccagcgtg caaagaaata aaatctagaa caagcatgtt tattgatgtt ttctttgaga 211320
   tgcaaagaaa acatcagcct aatgaagggg tacccatttt atgtgatact aggtagttgt 211380
   tgaaagcatt gaattaggcc aggcgcggtg gctcacacct gtaatcccag cactttggga 211440
   ggctgaggag ggtggatcac ttgaggccag gagttccaaa ccagtctggc caacatggtg 211500
   aaaccccgtc tctactaaaa aaaatccaaa aaattagctg ggtatggtgg tgcatgcctg 211560
   taatcccacc tgctctggag ggtgagacac gagaatcact tgaaccctgc aggtggagtt 211620
   tgcagtgaac agagatcagg tcattcatgc actacagcct gggtgataga gtaagactct 211680
   gtctcaaaaa aaagaaaaga aaagactgaa ttaaaatgag aaaatgagta taatttttga 211740
   catgggaact ggttacacag gtgaatttcc tttgtgagaa ttcaagctgt aagattatga 211800
   tttatacact tttctatagg tatattatac ttcctatact tttattattt aaaagtttgt 211860
   gataaaaatt ctaaataact aaaaacaaaa caccaatggg ttagagctac agatattggt 211920
   gtaaagcaga ggttgccaaa ctggcctgct gcctgttttt ataaataaag ttttattgga 211980
   acacagccac atccattcat ttatatatta tctatggctg ctcttgtgat acaatggcag 212040
   agttcactgc gtagttgtga caaagcttgt acaatctgca aaatgtaaaa tatttgttct 212100
   ctggccttta ctgaaaacat ttggcagtcc ttgaactaga ggaacatcta tggttaagtg 212160
   aaagggttag gttgcgtaaa aatatattta gtatgatctc attttgaaga aataagcaac 212220
   agcaacagac acctcctatg tgtgaagttt tgtctgtata ttttataacc tgtgtgtgaa 212280
   gaaaggtgtg gaaggatacc aaccaggttg taaacactgg gatcgcagca ggattgggag 212340
   tgaagaggct gtggcggtga gggggattgt gttgtgtctc tgtagttttc attacgtttc 212400
   acaggttgat tgcaatacat atcatttccg caatttttga aaaataaaaa cagaaaaatc 212460
   tccaaatagg aaaaatgaac agaaataaat tgtctcaaca acaatagcag aaaaaaaaaa 212520
   tgaatggata atgccccaaa tgatggggtg ttggttgctt gaggagggtt gttgggtggt 212580
   tctcagcttg agcagagctg ggcccaggcc ctgtctgggt atttttgagt ttttattgtc 212640
   actagcttcc atcttgtgta gcatctgcct tgtgagctgg gcctgttctc cgagtttcat 212700
   ggtgtttgat gttttttgcc ttctttcagc aaggtcgctg gcatcatcct tgctgagtcg 212760
   catatgttct gggaaccacc tcctcttctc tgtgcctctc gcctggcagg tctacgctaa 212820
   actgccccct gccctcccag gcccacccca cctccaagtc tgagttcttt gacttaaact 212880
   tcaatcctgt ttcccatggg ctgtggcttc ccaaccctca gtttcagttg aagctaattt 212940
   ttttttccag aaatcactgt gggccctggc tgctctgggt gtggtctggg gaccagctgc 213000
   atcccatcgg cattacctgg gagctcgtta gcaattcggg atctcagctt cccctccagg 213060
   cctgctgaat cagaatctgc aatttaacaa catcccctgg ggatgcactg taacattcga 213120
   gcagccctgc tgcagaggat cgcagtgaga cgggaagaat gtgtcttttc cactagatgg 213180
   caggccttac ctgcgcttgg ctcctgcgca gaccaccaca ggtggatgtg ccaggggtga 213240
   tgtgaacctc cagccccgcg ttggccatgg catcttacca gtcatgaagc tcgggtgtca 213300
   gtcccgccag ggctggaatc ctctttcatt ggtgacctgc acctacctgc atgtcctggc 213360
   ctggtgcagc atgagcttct ggcttgagcc atttgtgtaa agtgacccag agcctttctt 213420
   tcttccctgc tttgcctttg tcctctacca cctggaaggt gaaggcatgt ctctggggag 213480
   cttcctagtc attagcctct gtgttgcttt tctggatggc ttgctgcacg tctctgctaa 213540
   ttttaggcca atgtggtcta tgattacaaa ggttttgcat gggctttgga gtcaggcctg 213600
   ggtttgaatc ctgcctttcc cactcctcca acaagtattt gttgggggcc tcccatgtgc 213660
   tgggcactgc ttacaggcaa ggcccctgtg ctcatggcct ctgccttctt tctaatgatg 213720
   ggagatgaag aggaagcaaa caaaacactt tgagaaaaca aggaacacta tcaaggaaat 213780
   aaaataaagc ggcatgatgt gatagagggt ggccagtggg ctgagtgtga ttagcaggga 213840
   taatggcatt tagttgagac ctcagggacc agcaggaggg agccagcttg ctgaaaattc 213900
   cagggaagag tataccaggc agagggagcg gcagaggcag aggccagagg gaagagtgaa 213960
   gcagcaggct tgtgagtgac aggaactgac agccagtggg tgtgactgga gcctcgggat 214020
   ggggaggggt gggtgacgct ggaggaaagg cagggacagc cagttaggct ttgtgagccc 214080
   catggtgggc ttggtttctt tctaagtgca tggaaattta gatcccagga gtatctgaag 214140
   gacatcatgc aaggtcagtg tgtttaggcc ccaaaaccac ccagtggggg cagtcatttt 214200
   gtttttctca ttttacaaag gagaaaacag agccttgaaa aatctgagcc tggtgtggta 214260
   gcatgtggct atggtcccag ttactcagga ggctgaagca ggatcatcag ttgaggccac 214320
   aagtttgaga ccagcctcag caacacagtg agaaaaaaag tcttaaaaat tagctgggta 214380
   ggcagggtgc ggtggctcat gcctgtaatc ccagcacttg gggagtctga ggtgggtgga 214440
   tcacaaggtc aggcgttcga gaccagcctg gtcaacatag tgaaacctca tctctactaa 214500
   aaatacaaaa aaaaaattag ctgggcgtgg tggtgcgtgt ctgcagtccc aactgctcag 214560
   gaggctgagg caggagaatc gcttgaaccc gggaggcaga agttgcagtg agctgagatt 214620
   gtgccaccgc actccagcct gggtgacaga gtgagactcc atctcaaaaa ataaataaat 214680
   aaaataaaat aaaataaaat acaaattagc tggacatgga ggcatgcacc tgtagtccca 214740
   gctacttggg aggctgaggc gggaagatga cttgagccca ggagttggtg gctgcggtga 214800
   gctatgatct cgctactgca ctctaggctg ggcgacagag cgagaccctg tctctccttc 214860
   taaaaaaaaa aaaaagaatt tgagcacccg cctctagtcc ctaaggcagt gcatggagga 214920
   ggctggattt gtaccctggg tccatctgtc tggcaggttc taacacatta agtcagatgt 214980
   tggaaagata cagtttaaga ctatgagaca ttggaacata agaaacaccc accataccag 215040
   tggggagagg aaggagatgc ttcagcactg gtagttctca tagtcttatc tttcagtggc 215100
   tttagggcac aggttgggac tagtttagcc ttaaggtctt ggagaagacc tgcttttcat 215160
   ttgcctgttg ccccagcact gtggggtcag aatctttctt gtgatcagag tttagagaag 215220
   ggagggagca gtgggctgga ggcattccag gtagccctgg agaaggcagg agaggcataa 215280
   gttagaaggg tgagggaagg gaggagggcc agtggctgac gcttactgag cccttgctgt 215340
   gaacccagca cagtgctagc catcgtgggg cacaaaagcc tgtgcaagat gcagtcgcag 215400
   ctcctgagca gcctcgctgt ctggttgggt tccatctgct ttaccactac caacaaaacc 215460
   aagggacgga cacctcttac tgatagtaac caccaggcac tgggcgaagc acgttgctca 215520
   gccatgtcgt ataatcctat caacaacctg tagtcccagc tacttgggtg ggagagagtt 215580
   actgagggca tctccatttt acagatgggg aaactgagac acagagaaag aggttaaata 215640
   acttgcccaa gccacacaac taggaagatg tggagctggg acttgaatct aactatgcca 215700
   ttccagataa ttaggctctt ggacaccgtc ttatcctggc actaagtaca tcagtggagt 215760
   gatgatgata catgatatta gtagcagcca cttttacttc tttcttttct tttctttctt 215820
   tttttttttt ttataacaga gtctcactct gtcgccaggc tggagtgcag tggcacaatc 215880
   tcagctcact gcaacctccg cctccctggt tccagcgatt ctcctgcctc agccttccaa 215940
   gtagctggga ctacaggcat gcgccaccat gcccagctaa tttttttgta tttttagtag 216000
   agacagggtt tcaccatgtt ggtcaggctg cctgacctca aatgatctgc ccacctcggc 216060
   cccccaaagt gctgggatta caggtgtgag tcaccgtgcc cagccacagc caccttttct 216120
   aaaacccttc caaatgcctg gcttttgcta ggaactctac aagcgttcct tcaggtaatc 216180
   ctcctaacaa cttttgctat tacccgtagt ccttctctcc attatatggt gaggaaactg 216240
   agactttgct tcatatgact ccattttccc caacacttcc tgctagcctg ttttgctcct 216300
   ttgggagtat gagagtcagt gtttgctgga ctgtgaggct gaagatgata acatttcttc 216360
   aaattggcct gttttttttt tttttttttt tttttttttt tgcagaaaga cagatttcac 216420
   tggccaagat ctgcccaaga ggctgaataa attcctaggt tgtgagaagc aggtggatgg 216480
   agaagacttt ctctccaaac ctccctgtgt taggaccagg gaatgggtta tgtgaatctg 216540
   gcaaccctgt cccaccctgg acctgcaccc tttaccacta ccctggcaca tccgggacct 216600
   gcacccttta ccactaccct ggcacatccg ggacctgcac ccttaaccac taccctggca 216660
   catccgggac ctgcacctta accactaccc tggcacaccc gggacctgca cccttaacca 216720
   ctaccctggc acatccagga cctgcaacct taaccactaa cctggcacat ccaggacctg 216780
   caccgttaac cactaacctg gcacaaccag gacatgtgcc cttaactatt accctggcac 216840
   aaccgggaca tgcaccctta accactaccc tggcacatct gggacatgca cgcttaacca 216900
   ctactctagc atcatccggg acctgcaccc ttaactacta ccctggtctg cctccttgag 216960
   tggcccaggg aacatctaga agctgagaga aagagaggga taaaaccaag ttcacagcag 217020
   tatcctaaga aagtgttctc acttaaaaat gcaaagatgc tcacagagtg ttcaggttac 217080
   actggaaaac tccagaaaca aagtggtttg tgtacatggg aggaagcaga atcaacttcc 217140
   caccttagca ttgtgggtgc ttagcccacg cctgtgtatc gctcagggaa tcagagtcac 217200
   tataagcatc atggaataag ggatttactg tacacgttag accttataca agcttggaga 217260
   tgctggagac gggaagatct ggaggggtga attgagaatc agaggaagag cagtggtgca 217320
   ggtggacaag taggagttgg taggggatcc aagaagccaa gcacgttgag ctgctgacat 217380
   agatattgat gaatttcatg aagcttttac aggcactggt ttcacccttg ggtaactcag 217440
   tacaggtatg ttgacagcct tccactaatt caaagatggt tcataggtgt cccagaatct 217500
   tttaattccc aagtgaggca gccccaactc cttcagccat ttactttcta atccttaatc 217560
   cacagcccac aacctcaaac acatgatagg gccgagtatg ccatcattga tgtcaacccg 217620
   gaactcttcc tagacaacac tccagttgga gtagcttcct tataaagcag ggtgcccaga 217680
   gtgaaagaga atccacctgt gttgccgagg agcgaaggat tgtcactttc cttattctag 217740
   atcagctgtt ttcaaacctt tcggcttcca gcccatctag gttttatgta acacacacac 217800
   acacacacac acacactgca caaacaagca caaactcaca aacacacaca cattgcacaa 217860
   acaagcacac actcacaacc aaaaccaaac tgccattaat gcttacctct acttatgctc 217920
   accacacact ctgatacatt ctcctctctt ttgtggccct cttttctgtt catttccatt 217980
   ctatttcctt taagaaaccc tttaaggaaa atgctcttac tctgcaaatt gatttcacta 218040
   acaattggca aatgtgtcat gacccgccgc ttgaaaaaca ctgtcctaga caacaaaatt 218100
   ctctttctgc atcctgagat gatactactc ttttgggggg tctgggggta ccaccagcca 218160
   cattgcactg ttgacttatc ttgggtttat gatcagttaa agctctcttg tctatcacat 218220
   cgggtgctgc tagcctgcag gccccacatc cttgtgcaag attttttttt tttttgagac 218280
   tccaggatga aacctgatgc atttcatctt gtagggtttg gccttttgct ccacggccag 218340
   gctgagagct tttgggtctt cctgttcttg tgccgagtgt cttccggaca tcaccttcct 218400
   cacaccattc tctgtgctac ttgttctgcc ctccttactt ttgaaaagat agtttattac 218460
   tcatagatga ggagcaggcg tgccactcca tgaaaggcca cacagggaag cagcagggtc 218520
   tgccaggagg cagaggaagc ggaaagaaaa cgtggcagga gcatttgctg ctgtttctgt 218580
   gagaaggagc aggtagggcg ggaaaccagc cttagggctg gctggtttga atgatctcag 218640
   cagactctgg gtgtagaggc tgtctctcta gttgcctggt acctgatacc tggtgctttt 218700
   acctggggta attagcacag gctggcccag agtgtaagag ccccatgaag gagttggttg 218760
   gaggtttggg ctttggattg gttggtttgc atatgaaagg tgcgcttgca ggtgagttct 218820
   ttactgtctc taggaatggg ctagccctgg gacagacagt ttctccaggg tcagcaaggc 218880
   tccgagacac caaagcatca gaaatacaga aaatagagcc aggtgcagtg gctcatgcct 218940
   gtaatcccag agactcagga ggctgaggtg aagaatcact tgaggcaagg agtttgagac 219000
   cagtctgggc aacatagtga gacccctgtc cctaaaaaaa attaaaaata aaattaccca 219060
   ggcatggtag tgcagcatgc ctgtaatctc agctactcag gagactgagg aaggaggatc 219120
   actgaatcca ggagttcaag gaaagaggcc gggcacagtg gctcacgcct gtaatcccag 219180
   cactttggga ggctgaggca ggtggcgagt gaatcaccag gtcaggaaag ggagaccatc 219240
   ctggccaaca tggtgaaacc ctgtctctac taaaaataca aaaattagct gggcatggtg 219300
   gcgggcacct gtaatcccag ctactcagga ggctgaggca ggagaatcgc ttgaacctgg 219360
   gaggtggagg ttgcagtgag caagcgagac tccgtctcaa aaaaaaaaaa aaagacataa 219420
   aattaataga ccttagacta ttttatttaa ttattttatc aaaataggat aggcatagtt 219480
   aaaaatgcca atgtgcattg gagagctgag gatgaaaagc aatgtcctgt acttgagctt 219540
   gcatcagaat catctagaga gtgccccacc ctcagagttt ctgattcagt agacattgaa 219600
   gaatgagaat ttgaatttct aacaggtccc cagagaatgc tggtgtgttc acactccggt 219660
   taccagaaag ggatcccgat ccagaaccca agagaggttc ttaggtcttt tgcaagaaag 219720
   aattcgaggc aaatccataa agtcaaagca agcttactaa gaaagtaaag gaataaagca 219780
   tggctactcc acaggcagag cagccctaaa gctgctggct agccactttt gaggtcattt 219840
   cctgattata tgctaaaaaa ggggtggatt tttcatgagt tttctgggaa aggggtggca 219900
   gattcccaga actgaggatt cctcctcttt ttagaccatg tagggtaact tcctgacgtt 219960
   gccatgacga ttgtaaactg tcatggcgct gctgggagtg tctcttagca tgctaatgca 220020
   ttacaattac tgtataatga gcagcaaaga cgaccagacg tcacttttgt tgccatcttg 220080
   gttttggagg attttgacct acttctttaa cacaacctgt tttatcagca aggtctttgt 220140
   gacctgtacc ttgtgctgac gtcctgtctc attcagtgac ttagaatccc ttaacctcct 220200
   gagaatgcag cccagtaggt ctcagcctta ttttacccag cccctattca agatggagtc 220260
   actctggttc aaacatctct gacactctga gaaccactgc attggaagac actattttca 220320
   actctttaaa ctctttcttc cagcacttga gctctaaatt ttaatcatgt gctctgtctc 220380
   tctgtcattt cactatttca tggctctgac ttcctgagaa ggttttaatt ctctgatgtg 220440
   atccctctag ttctcttccc ctcacatcca gcacggggtt actgccattt gtagttcact 220500
   cactcaaaaa aaagtgatta ttaagcatct tctgcatgtc aggcattgac ttagtcactg 220560
   gagatttgtc agagaacaag ccaaagtcct gccctcatgg agcttatatt ttagtgcagg 220620
   gcttgacaaa ctttttctgt gaagggccag atgggctttg gagctatctg gtcactgtca 220680
   caactactta actctgccat tgtaacacgg aagtggccac agtgtgccaa tgaatgggca 220740
   cgggtgtgtg ccaataaaac tttatttaca aaaacaggca ggaggctgga tttggccctt 220800
   gggctgtagt ttgtagacct ctgttgtagt gtgggagaag acaaagtagg taaataagta 220860
   aaatacgaac agtaagctaa ggggaaaaag aaaaaacgga aaaagaggat gggaaggagg 220920
   caggagggga ctggacctgc acatttagat atggtggcaa gcaggcagtc actgagatgg 220980
   ggacattgag taaagacttt caaaggagat gaaggaggga gccatgcatc tccctggcag 221040
   aagagcattc caggcacagt aacagctggt gcaaaggccc tgtggcatga gcatgttcgg 221100
   tgtgcttgaa gaaacagcaa ggaggctcgt gtgtctaagt ggggcaagtc aagggaaggg 221160
   agcaggagag gaaagcggag gaaacagaga ggcagctgct gagggctatg tgattcttgg 221220
   gagggaattt gtcttttctc ttagtccaat gggaatccat tattgtgact ctagtagttg 221280
   taatagttcc tttaagtttt atttatttat tttagataca ggttctcact gtatcactca 221340
   ggctggagta cagaggcaca atcactgctc actgcagcct caaactcctg gcctcaagca 221400
   atcctcctgc ctcagcctcc tgagtagctg ggactgcagg cacacaccac catgcccagc 221460
   caaattttta aaaattttat aatagagatg ggatctggct atgttgccca ggctagtttc 221520
   aaactcctgg cctcaagtga ccctcttgcc tcagcctccc aaagtgctgg gattacaggt 221580
   gtgagccact gaacctggct atttaagtta tgtatttgca actccatttc ttgctttccc 221640
   aatggtaggc aatatctctt aactttgtct tttgtagagc atttttatat gcatgatatc 221700
   atttactccc tatgagatgt gactattgtc cctattctgt agaggaggca actgaagctc 221760
   ggaaaggtta aatgacttgg ccagattcat atagctgata agtgctggtg ctggaattta 221820
   aacccaggtt tgtcttaata tggagatctt agcacctatt ttatatgaag agacaatcct 221880
   ttagggatac tggggaaacc tgtggctttt ggacaataaa ttaaaaaccc tttcccaaga 221940
   aggggagctg tgtgtgtatc aatcaaacaa aatgagaact attctgagag ggaggaaaag 222000
   gttcttttct gtgggaacgc tgaccaagcc gttgacagct catttatgtg atgctggtgc 222060
   agtttgacat tcatagtaca agacttgcat gcaattagca cgaatcgttc taacagtctc 222120
   ttgaaaacat ttggaatttt ccctctgtct ggccttgtgc atgatgtaaa catggcttta 222180
   tttagttggt catccatccc agaatagtgt ggtctgcatc tgtctgcatc actgtggtct 222240
   gattgaggtc ttgttttcct ggagggcaga gactgttgtg taggcttgct ttgtggatcc 222300
   tcctccccca ggatcattcg ccacaaaccc gatggcagcc tctctcttta gctcatgaca 222360
   atttctccaa gtgtaagctg ggggtcccgt tcagtatgca atgcaaatga gatgcaaatg 222420
   aggggccctc ttgggcacag gccaggaggc cagagctctg gggacttctg tacccctcct 222480
   ctgagttgcc taatgaagct gaccgttcct ttcacaatca tcttctccac ccaccaaagg 222540
   cctcttaatg agggcagaag ctgactgctc tgaaacccct tcagacacct ggtgattgga 222600
   agctggagag acagtgagct ttagatgggc tgtcagccgc tgctgagtcc tcatcagctg 222660
   cctgctgggt tctgagaagc actagggaag gaagggagat ggccctggaa ctgactgcta 222720
   ttgtctgttc ctcctcccct gacgagctgc atgcggcagc cacggatggc tgggtttggg 222780
   ctttcacgtc ggcttctctg gcccttgctg gatgcctgct tcagctcaga gaatgtggct 222840
   tcacagggac ttggaataag agctgagagc ctgttccccc catcgaggat caatgctcct 222900
   ttgatttcat ttcttctttc cagacgaatc cactgtctta gcccgttgtt ggagaaatcc 222960
   cgacctaagc aggctttcaa attggggaaa gaaaagtgaa ccaatctgaa atcatggaat 223020
   ggttggtgga gaaggacaag taggcatatc cataaagagc cagcagtccc aaaggatgtt 223080
   ccagtgcatc tttctgctta tttcttctag ttttgtaggg gtgtgtggtg tgcaatgctg 223140
   tttgcttgac agaatccttt cagtctctct ctctctcgaa atataaattt cctgcactac 223200
   aacagcctac taagtgccag acacagtgct gggctcttta cctgcatcaa cccattttgt 223260
   ctccattgac aaaaggggaa actgaggctc agagaggtta attgacttgc tcaaggtcaa 223320
   ctgggcttct gtgtggcaga actggagttt ggtcccaggt ctgtttgcat ctaacaccca 223380
   gccccttaac cactttgccg aattgagaat ttggaaatta aaatggtgat ttcaactcag 223440
   ggatgggtgt gctttctttc agccttgcag taagtgcaca tcttctttct cttttcctct 223500
   ttgaagctgc cagcctctcc cttggggttc tccttggctc tggccatgat ttccccagct 223560
   tcctcattca tacctcctgg catcagactg acctgggcta atcaccatct ctgcctgtca 223620
   ctagttcaag aggcagctta tggggacttg tgggtcatac aggttcccct agaaagatga 223680
   tgggcagact gacattggga ttgaaatgtc ttgtctccag cgttcttgaa taggggcaac 223740
   aatgcccact gggtctccag gcatggggtc taaaagtgaa tgcaaaatac ctgtgataca 223800
   gtgtggatgt tttatcccca ccaagtctca tgttgaaatg tgatcctcgg tgttggaggt 223860
   gaggcctcgt gggaggtgct tgggccattt ttgggtcatg gggcagacac ctcatgcctg 223920
   gcttggtgca ctccatgtca cctaaacgag ttctcgctcc atgttcacta gagatctggt 223980
   tgtttaaaag agtctggcac ctcccctctc tcattcttgt tcccgctcct accatgtgat 224040
   acacgggctc ccctttctcc ttgtaccatg attggaagct tcctgagtcc ctcaccagaa 224100
   gtggatgcca gcaccatgct tcctggacag cctacagaac catgaacaaa aataaacctc 224160
   ttttctttat aaagtaccca gcctcaggta ttccttgaga gcaacgcaaa cggatgaaca 224220
   cagcctattt ccatagcctt gctattggtg agtcttgggc ccgtggtgca tgcacagggg 224280
   ccagatccag cccttacccc aagagcagat tcaaacgtgt ctttccctga ttatttatgg 224340
   ccactaaaat ggaaggtgat ggttgtggtg ggtgtctatg atcagggctc aataaatatt 224400
   tgttgaaaac aaaaatgtaa acaagaaaaa gacaggacct tgtcaacaag tgttatatag 224460
   ttgcagatct ttatcttgga ttgttcagac tctgcccttc tctggcaggc acacttttgg 224520
   gaatgggaag ggccaacagt gcttctttgg tcccattaga tccccatgtc agccctatga 224580
   aataatacta tcccatactt actactattt cagctttaca gaagggaaaa atgcagctca 224640
   gagaggtgaa ttcacttgcc caaagtccca tcgctaggga gcgatgggat ttgaattaaa 224700
   aatgcagatg tgattgattc taaacgcagg ctgttaatgc atagctttcc tggggaacaa 224760
   aaagagcttt cctttcatac ctcattcatt cattcattca cttattcttt aatctcttct 224820
   gtatgggagg tcatttgcta gatactggat ggctgtagga atgaatatga ttctttacac 224880
   gctgagtatt tcatagccct actaagagag aagaagagag agctcactat aaaagggggg 224940
   attggtgaag agataatgga gtaggaagca catgctgtgg gaggtgccag gggatgagag 225000
   gttaattcca acccatgggg ttgtgatagg cttcacagag gcaaaggcag tggacttagt 225060
   tctggatggg gcagagatct aacgcttatt gatggcttgc cccttgctag gactcagctc 225120
   ggtgggcgct ttgtctattt tctactttga aagatgaata gaaactttga cggtcttttg 225180
   ccctctgaaa tccactcttc acacccagcc acaggaatct ttctaaaatg taaatcaggc 225240
   catgtcaccc ccctgcttaa atgattccaa gccttctcag agcttccaac tgaaacctaa 225300
   actcctcact cctgctctaa gagcctgcag attctgcttc cttcagcctc tttccaacct 225360
   tgtttgttct tttcccatga tagccacatc tctgagccaa caaatatttt tccttccttc 225420
   ttgcacttgc tcttccctca gcttgggttg cttttccttg ggctctatgt gttgccaact 225480
   ttccagtagc cttcagtcat cactaaatgt caccttctca gaaagcccgc ctctgagtct 225540
   tatctgaaga aacccctccc agtcactccc tatcccatta ctctgtttgc atttgtttat 225600
   ttatttgctt cttgcctgtt tctctccact ggatggagag caagaacctt ccttattttt 225660
   tatgccactt tgtgctagtg cagtgcacaa ttgacatgta cagatgcaca attagagttt 225720
   gctgagtaaa tggctaaata ggttaaaaaa aaaaaccaaa aacggtgggc tgctaggcag 225780
   aagtgtaacc acacaagtga accttgaaag ttaccatgaa acgtaacctt catgccctgt 225840
   gaaggtgcct taggtttctg gtgtgtttgg accatagatt gtagctatga agtctgatta 225900
   atgcctgaat cagacattga atgacctgaa taaatagtta aaaagttgga cttctaggct 225960
   gaagtgtaac cacatgagag aagcatgaat gttaccatga aggtgcctag ggtttctggt 226020
   gtgttcagac catagattgt agctatgaag tctgattaat gccagctcac agaagagctt 226080
   gctcaagcat gctagggatt tggtgagcca ttgtaggtgg taatgacctg atctagttac 226140
   catgggtgac aattaaaaag aaggattaaa gatgtaaggg actagagctg tagagaccca 226200
   tccaggagct attgatccct gtaggatgga ggagatgaaa catacatgaa ttaaggaagt 226260
   ggaaggataa gaataaattg atgtagagga tcagggagag gatggaggag tcaggactaa 226320
   cagagatttc tgactcagga agacatgtgg atagtgagcg tgatagccaa ttagggttag 226380
   aagtcacaga agcttaccag aagggaaagg taatcatctt gctttagtat atgttgagat 226440
   gtttttgggt tgcatgggtg actgtgtcca gtaggcagat gaatctgtat atccagagat 226500
   ccgattggaa aaacagagcc aacagtatgg atgcaggggt caccggccct aacagtggaa 226560
   gctgaattct ttgagtgggt caggaattag tgcagcactg taagagcagg aaggttcagg 226620
   ctctgggaaa tggagccact tcagtcatca gtggagcaag aggagccagt gaaggagaat 226680
   ttaaaaataa tgcttttaag tccaggtgca gtggctcatg tctgtaatcc cagcactttg 226740
   ggagtctgag gcagaaggat cgcttgagcc caggagttca agaccatcct ggatgataaa 226800
   gggagactcc atttctacaa aaaaaaattt ttttttttta atgtagccag tgtggtggtg 226860
   catgcctgtg gtccaagcca ctggggaggc taaggtggaa ggagcccttg agcccagaag 226920
   tttgaggctg cagtaagcta tgatcacagc actgcactcc agcctgagtg acagagtgac 226980
   accctatctc gaataataat tattattagt agtaataatt atagtgcttt tagagaatga 227040
   aggggaacca gccgagtaaa tggtcataga agccaaggga agagagaatt caagaaagtg 227100
   gatatggcca acaccttaga tgctgccaac tggtaatgat aaacacgagg ctaagaattg 227160
   aacagagtca ctggatttgg tcatttaact tagggacttg ggaagtgctc tttcagtggc 227220
   atagtaggag ttaagaggca ggatgccgga aagcctggat gctggatgtt gaggagggaa 227280
   ttgaaggagg ggatgtggaa agaggagtgt agacaactca tgagaatttg ggttgtggaa 227340
   gaaaggggcc agggaaggcg tcttagaagg aagttatttc tgagaggcat ctgagcaggt 227400
   ttatagcatg aatggaagaa agcaaggtgc ctgggaatag ggataggaat taagcagaag 227460
   cggtcctagg ctttgtcttc aggaagatag gtgttcaagt cccagctctg ccacatgctg 227520
   gctgtgggac tttgggcaag ttaatgaagc cccatgagtc tcagtgtcct catctggcga 227580
   gcagagagag tagcctgtac cttgcagagt catggcaagg atgaaacaag aatgtctctc 227640
   agggccttca cagcgcttag tgcataatga ggggttagtt agtgaactcc taccaagctc 227700
   ctttgtacag ggcatttagc aaagacgtca gtgaccctga gaatccctag tctaaggtag 227760
   gtaccccttc cctaagcgct gaacttcctc ccttatgcat gtatcacatc tacaatgttt 227820
   ccttgtgtag tggatactgt ggtgtgccac caggatccct ctctcagcac agaggcactc 227880
   ctgccccaag ccacgcagag tattggctct gaccactcac ggcttcagta tttccgtagg 227940
   atttgccctg agggagctgc agtgcccaag gttatgccct cttccagggg cagctcacag 228000
   tctatgattg gttgctggtg gggaatgggc ttataaatgc tcttccccag cctcagtagg 228060
   gaacaagtct aaggggccat cccagctaca gagctccctc atggatcagg tgacacctct 228120
   gttgcagcta cattgcagtt cagtttctcg atttcatcct gcttccccca ttccttgcag 228180
   gagttcctcc caaggttatt ccctgagaag ccgcctgcat acaatatcca gatcagtctt 228240
   ttttcaggga acccgactta agatgccttg tttaatgttt gtatcttttc tctaaaatat 228300
   aagctttttg tagggactga ctattctgtc ttgttcattt tgtgtcacca tgtctaggta 228360
   cataataggt gcttgttaat aatttttaat cttttttttt ttgagacagg gtcttgctct 228420
   gttgctcagg ctggagtgca gtggcatgat cttggctcac tgcaacctcc gcctcccgaa 228480
   ttcaagcaat tcttatgcct cagcctccca agtagctggg attacaggtg tgcatcacca 228540
   cacccagcta atttttgtat ttttagtaga gacggggttt tgccatgttg gccaggctga 228600
   tctcaaactc ctgagctcaa gtgatctgcc cactttggcc ccgcaaagtg ctgggattac 228660
   aggcatgagc caccacatgc ggccattaat tgaataagtg aatgaacagg tttcctaaac 228720
   acacacacag gagtgccctg atatatacag tttttcagag ttgtgattag agaataggtt 228780
   atctggaagt gccttcctgt gggagtgtga gcttgcatag gctcccccag catggagcca 228840
   tcactagcaa tggctgtgtc ttccacgagc tatttccagg gttccatcag caaacagtgg 228900
   tcacgattta taaaacgttc acacaagaac atttgggctg cattctacat cattttagta 228960
   cagagaggtt gttccttggg gcctttaatt ttctagcttt cattattgag agagtaaaat 229020
   gtatcacttg cattccaaaa ttgattttta tttcccaaaa gaagcccata aaaataaata 229080
   acttcccggg agggaaaaca gaaaagcact ctatacactg ggcaatttgt taaaaaagaa 229140
   aagaaaagga aaggaagcct ggagcgacaa agcaatctaa acatttagat tgtatctctt 229200
   gtattccccc actccccctg caccttaaat aaataaaaaa aaaaaaaaaa aaaaaaactt 229260
   gaggcctgct ggactttgaa aatgcccatg ttgttgagaa aactctgttg ggcagcttca 229320
   tgggacagca tcctttggag cactgcctca tggttcacac ccctggtatc ccaacccact 229380
   gctatgttgt tattagcaag atacgtttcc agtatctcat tactaattgt atcagtaagc 229440
   atttgctgtg taacaaattg ctgcaagggt agtgggctta aaacaacatt ctttctctca 229500
   attttgtggg tcttctggga ggttcttgtg atctgggcca gcttgtctgg ggctggaggg 229560
   tctgtgaggc cctcactctc acatctggtg gttggctcag tgtcagctgg agggacaggg 229620
   atgccttggc cattgcctgc caccacccag caggctagcc tttgcctgct cactggcatc 229680
   tcaagagcag taagtgaggg catttcaatc caagccccaa ttcccaagca cttttgagga 229740
   ttctgtttgc atcgcattgg ctaatgtccc agtcacatga ccaaacccag atgcagggaa 229800
   tggagaaacg aattgttgtt ctttccttga cattttactt tctcaagtat aaaggggggt 229860
   ttgtaggcaa ggaagatgcc aagtagtccc attttatagc taataaactg agaaccagag 229920
   aggttgagca actggtctga agtcacgcag caagcccaga acacactggg taaccaatcc 229980
   actgggggtg agttcaccct gtgcagccac atttgatctc cccttgcttc attccctcca 230040
   gcactctggc agccaggagg acagctccct gtggggactg tgacattact ggcccctcac 230100
   tcagctcttc ttgtgccaat tagaatgcat acttattttt ttttgcaggt tgtcctttgc 230160
   ttgcatccct cacctccccc atccactctg tgatcttctc tgtggctcaa gagcctgact 230220
   ccttcagagt gcatgacatg ggctcttttc ctttttagtt tctgattgag ttcaagcagt 230280
   aagaggcatt gattggcaag agattgtagg atggtaggaa tgagaagatg cggcatttct 230340
   gtcccaattc ctctgctctt ggctgtgtgt ccagcagtat cagaaggccc cttctctgtg 230400
   tctccagatt tcactgggct ctggtaacac tacttccttc tttggcctct tcaggcctag 230460
   gggtagcaat gacatctcaa tgctgctact ctctggatgt ctcaccatgc gttgttgatc 230520
   cctttaaccc tgtcttcaca tttgtgagta gtaccttaat taaaatctct agagccttct 230580
   gaattggatt ctgttttctg cttgcgccta tcctgataca cttttttttt ttttttgaga 230640
   tggagtctca ctctgttgcc caggctggag tgcagtggca tgatcttggc tctctacaac 230700
   ctccgcctcc tgagttcaag cgattctcct gcctcagcct ctcaagtagc tgggattaca 230760
   ggcgtgtgcc accatgtctg cctaattttt gtatttttag gagagacaga gtttcatcat 230820
   gttggccagg ctggcctcga actcctgacc tcatgtgatc cgcccgcctt ggcctctcag 230880
   agtgctggga ttacaggcgt gagtcaccat gcctggccac tgatgtactt tcactgggtt 230940
   ccattttttt tataagggga gaaagaatgg agtttgcagc atgcgcttag ggaacccagt 231000
   atcatgatga tagaggagtg agtagcctgc atgaatgctg ctgtggcagg aagaatgttt 231060
   gagaacttga gcactagagt gtgagatttc tggttgtctt ggctccattc agggaattct 231120
   taggactctg ggaaggacat accaggatat ttatggcatc actgtttgtg gtggcaaaaa 231180
   gctaatagcc tcctgaatgc ccgtttttga aggctgaaca tgttgtagtc tgtccatgct 231240
   gtggtataac ttgcagttga aaaagaatga gtttcaaact gactgacata ttgttgggtg 231300
   ggaaaagcat tttgcagagc agcgtgtata gtgtgattcc gtttttgtac agtaaccctc 231360
   ccagtccatt tgtttctagg tgattgtctc agccaggaga aaggtaggaa agagtaaaca 231420
   ccaggttgcc gtccttctta cctggcagtt ggggaggagg agaaagatag aatgggaaag 231480
   caagtaggta aagggagctt gaaaaaaaaa tcatagcaaa tgggaacata cacatagatt 231540
   taatttggtg tcccttttct aactttttga gatagatata gcattgattt ttcagcctat 231600
   cttctttcct aatatgtaca ttaaggctac acattttcct ttaagcacag ctttagttga 231660
   ctctcacaag ttttgaatat ttttgaattt tcatgatgat ttttcctatg atttattcaa 231720
   aagtgtatgt ttaattttca agcatttgta aatattctag ttatcttttt gttattgata 231780
   gtttgtttcc accacagtct gaaaacatac ataatttcat tcatttgaca tttgggtaaa 231840
   tattccatgt gcacttacaa agaatatgta tcctgtgttt gttggatgcc atattgtgtg 231900
   tgtgtaaatt agtttcgtta gttgtgttgt tcatattttc tatatcttgt ttgattttta 231960
   ttttgcttgc ttgttctgtt actgaaagaa ttggatttaa gtctcctatt atgattctgg 232020
   gtttttttcc tttcagtttt atcaattttt ggtttgtata ttttgaagca atgttattat 232080
   tataaaatat aattgatatc tcctcggtga attgatcctt ttatcattct gaaaggtatc 232140
   tgtttatctc aaataatgct tcttgcctta aagcctactt tgcctcttgt aaatatacca 232200
   gttttctttt ggttgatgtt ttatgatgtg tgtgtttata tgtatataca tatatacata 232260
   tatatagacc gaatatatgt attatatatg ttttatataa tgttttatgg tgtgtgtgta 232320
   tatatatata taaaaagact gaaagggaaa agggggaaat atatctatat ataaaataat 232380
   ataaatatat atatattttt cccccttttc cctttcagtc tttctgtagc cttacattta 232440
   gagtgtgtct tttataagca caatttttaa tacagtctga caatagttga ttttaattgg 232500
   agcatttaga ccatttacat tttatataat tgctgatata tttgagtcta tatctaccac 232560
   ttcctatgat ttttcgtttg tcccatgtgt tttacattcc tttttctctt tattattgcc 232620
   ttttggggat cacttaagta tattttatta tttcatattc ctttctatta actaattagt 232680
   catgtattgt ttttattatt tttatattga ttctagagat tgcaatatgc atctttggat 232740
   tttagaattt aatatattag tacttttatt acgttccaaa tgatcctagg ttctttaaca 232800
   ttttagtcct gtttacccca tcctaccata cacattgtaa ttttacatgt gatttaaact 232860
   acattatgat aatgcttcat ataatcagca tttacttgga tttaataata tatttaccct 232920
   ttctgttagt tttcattctt tcttacattt tcacatttcc atctgggatc attttctttc 232980
   tctctaaaga acgtctttta ttatttagtt taggcctgct gcaaatacat cttaagatac 233040
   ttgtatttca agctgggcac agtggcacat gcctgtagtc ccagctattc aggagtctga 233100
   gacaggagga ttgcttgagc tcaagacttc aagtccagtc tgggcaacat cgtgagatcc 233160
   catctctggg gaaaaaaaat ctttatttca cattcaccct tggatagaat ttgaaactgg 233220
   ctgttatttt ctttcggcac tttaaatata ccatcccgtt gttcttcttc catcattttt 233280
   gttgattttg tcagtcttat tgtggctctt ttgaagtcct attgttgcct ttatgcaatc 233340
   tgtatgattt tagattttct ctctctcttt gattttcagc aatgtgactg tgatgttcct 233400
   gtgtgtgtgt gtgtgtgtgt gtgtgtgtgt gtgtgtttgt atgtgtttat cttggttggt 233460
   gtttctttac ttcttgcctc tttaacttga tgtttttcat tagtattttc ttgtccattg 233520
   tctgccctat ccttttctct ccttttcatg cttgactttg attatatgta tgttagacct 233580
   tggtactgtg tcctctgtct ctgacactct cttctgtact ctctgtctct tttaatcttt 233640
   atgccatagt ctgatttttc ttaataatct gtttttcagc tcactgattt gccttctaac 233700
   tgcttccaat ctgctgctaa gccatgcatt gagttattaa ttttaagtat tgcattttaa 233760
   ataaactttt tattttagaa tagttttaga tgtacagaaa agttgcagag gatagacttc 233820
   ccgtatacct ttcactcagt tttccattat taacacgtta catgaccatg acacatccgt 233880
   caaaatgaag atgttaacac taattagtac tgtattgtta actaaactcc aaactttatt 233940
   tggattttcc tttttttttt cacgatattc ttagtgtatt ccaggatctc atgtaggata 234000
   ccaaattaca ttaagttgtc atgtttccta agtgtcctct ggtttatgac aatttctcaa 234060
   tcgtttctta tttttcatga ccttgacagt tttgagaagt actggtcaag tatctggtag 234120
   aatgtccatc cacttgggaa ttattgtatg tttaattcta aagctttcat ttgctttatt 234180
   tagttatttt gagacaaggg tcttgctctg tcacccaagc tggagtgcag tggcacaatc 234240
   atcactcatt gcagccttga actcctggcc tcaagcaatc ttcccacctc agcctcctga 234300
   gaagctggga ccacaggtgc atgccactga gcttggctaa tttttaaaaa tttttgtaga 234360
   gatgaggtct cgctgtgttg ccccaggctg gtcttgaact cctggcctca agtgatcacc 234420
   cctcctcagc ctcctaaagt gttgggatta caggtgtgag ccactgtacc cagtcagttt 234480
   cttttttgta accaatagtt ctctggtgaa actttccaca ttgtcatcct ttctcccttc 234540
   ttttaaacat cttaataagt tattttaaag ttcatgtttg ataattccag tgtctgaatc 234600
   acccatggat ctgccctctt gtctgttttt ttaaaatttt ttagacggga tcttattctg 234660
   tcacctaggc tggagtgcag tggtgcaatc atagctcgct gcagcctcta cctcctgggc 234720
   tcaagcaatc ctcccacctc agactcctga gtagctggga ctatagatgt gtatcaccac 234780
   accccactaa tgtttgtatt tttgtagaga tggggtttta ctatgttgtc caggctggtc 234840
   ttgaattcct gagctcaagc cgtctgccca tgtttgcctg tcaaagtgct gagattacag 234900
   gcatgcatca ccacgcctag gtgttttttg cttctggtcc tatttcttgg catgtttgga 234960
   agttttgttt gaatttcaga tgattccttg ctaaattaga aagctgttgg acaggtgtgg 235020
   tggctcatgc ctgtaatctc agcactttgg gaggacaagg tgggtggatc atctgagatc 235080
   aggagtttga gatcatcctg gccaacatag tgaagcccca tctctaccaa aaatacaaaa 235140
   attagcctgg catggtggca catgcctgta atcccagcta ctggggaggc tgaggcacga 235200
   gaatcacttg aacctgggag gcggaggtta cagtgagcct gggtgacaga gcgagactcc 235260
   gtctcaaaaa aaaaaaaaaa aaagagagag agagagagct gaggaagagg ttattttcca 235320
   tgcaggttgg ggtcaccctt ctggtggtga tcgagctcac tgtgtgctgg tttatagcct 235380
   tcgtaaggtt tgttctattt ctggttcatt tccactccta tggtgtagag gtttccaact 235440
   ctgaggctga agacttgcca gggccctcct tttaactggg tcctgaaccc caatttttgt 235500
   ttctctagca ccataagcct gacaaaacct ctcagctgat ttctgttttg tgtctcaacc 235560
   tctgcagtgt gattggtaga gactcaagag aaaaatcaat gcctagcgtt gagttcactt 235620
   ctctgctctt cccctcccac tatgatttgg cccctcaagt cctgccttcc ctggaagctt 235680
   tgattcctag ccccaagaat aagcttcaag ctctggtggc ctccctcctt cttagcaggt 235740
   gccctctgtc tagcttctta gtcctttgcc ctgtgttgag aattgattta tggcccaaag 235800
   gaaaaaaaaa aaaaaaccac acacagaata ttggacttat cttaattcta attccttcaa 235860
   aattccagtt tcttgaaaaa tattttttat ctggctttta tagttgctct cagtgggaga 235920
   gctactttat cattgcccca agtggtatga tattaaataa aaatataaaa tacaaaatag 235980
   atttagagtg gtataattat gtaaaaattg aatgttaaca tggaaaaaga ccagaaggtg 236040
   aatatacaaa tgaaagcatt tatttaattt tgggataaga tctaaggtaa tatttgtttg 236100
   gttcagaaaa ttatttttaa agaagggtgt tgtgagcatc agtgatagag tgaggaatcc 236160
   tcatttcttt cagatcctgg taggggaccc tgagaacttt ggggagtgaa gatagtgaag 236220
   taaaggttgc taagcattct gctagaacac tatggagaca gcgatataaa ataagagcca 236280
   ataataacaa agtagcacat agtgaatgtt taccatatgc cagacattct attaatttat 236340
   aatgattgta acattcctat gagccaacat ccttatcatt agccccattt tttaaacaag 236400
   gagactgagt taccgagaag ttaggtaact ttcaggtcac aaagctgctc tatggcagag 236460
   ctgatctgcc aggatctctg ggtttaagct cctcaccatc acacttgact aatttggtat 236520
   agaaagtggc tttagatcag ataagggtac atgaaatgca ggaggtgccg tgttagagga 236580
   cagtttggtc cagtccccat gtcctggggc aaatgaagca gatggcctca gaacagcctg 236640
   tttttaaatc atgtaagact gacttctttc agctgctgga ttcctgcctc ccagagtgag 236700
   acgttgaagt ttggcccgga agaggctgag cactggggac gagcagtgct gtgctgtgta 236760
   tagagtgctt aaaacagtgc ctgggtctga gcgtggtggc tcatgcctgt aatcccacct 236820
   ctttgggagg ccatggtggg aagattgctt gagcccagga gtttgatttc agcctgcaac 236880
   atagtgagac cccatttcta caaaaaattt taagaagtag tcaggcatgg tcgtacacct 236940
   gtagtcccag ctacttggga ggctgaggtg ggaggatcac ttgaacccca aaggttgagg 237000
   ctgcagtgag tcgtgattgc actactgtac tccagcctga gtaacaaagc aagactttgt 237060
   atcaaaacaa aacaaacaaa acaaaacagt gcctggcata tagtatgtgc tcaataaaag 237120
   ttgtcattgt tattgtcatg acatcatcag agcctagaac aaatcacaaa acctgtatat 237180
   tctctctggg ctggaggtct taagattctt ctccattccc aacgggtact cccgagatga 237240
   cgtgattcat ggccattcct tccttccttc agtgtgtggc tcacactgtg ctgggctatg 237300
   gcgtcaccac tgtcaatacg acaaataaat ggataattac agcaaagtgt taggactgct 237360
   gtgccagaca cgagtgcaag gtgtcctggg caaccaggga agggacgtta acttacactg 237420
   ggattggggc cctgtgatgg taagcttttc atcttgcagg aaaccagggg attttttttt 237480
   tttttttttt gagacaaggt cttgctctgt cgtccaggct ggagtgcagt ggcacgatct 237540
   tggctcactg caacctctgc ctcctgggtt caagcgattc ttgtgcctca gtctcccaag 237600
   tagctgggat tacaggcatg tgccatcact cttggctaat ctttgtattt tcagtagcga 237660
   cggatttttg ccatcttggc caggctggtc tcaaactcct gacctcaagt gattcgcctg 237720
   cctcagcctc ccaaaatgct ggaattacag gcatgagccg ttgcgcccgg ccttgaaacc 237780
   agggaattta atgacattta aaaatcattt attaagtata tatgaaatat atagaaaata 237840
   gtatcccagg cacctaatag aacagaataa taataatgac taggagttgt tgagtgtaaa 237900
   ataggttgca gtcattacaa cgcatataat agcatgaagt atatagctcc tcagccctct 237960
   cagccatctt atgagggaac actgttgcta ttctcatatt acagatgagg aaactgaggc 238020
   tcagagaggt taagtaatcc tccaagcctc cgcagccagg aagtggtatc actttgaact 238080
   caaactcagg tctgtttgat cctgaggcca agcccttggc cagaaaaatt aggaagtcat 238140
   agtcccttcc cttaaggagc tctatttaat atagcagaac aagtaaagca agtacatatc 238200
   tctctaaagt atactgtgtc actgaagata agaaaggaag aaatcctgtg atgggtcaga 238260
   tgaagtcagg aaaggcttct gggaagaggt ggccttatag atctaactag aaggatgaat 238320
   gagctttggg ccaaaggcaa gggagggaac catctggaca aagggtagtt agtacacact 238380
   gtgacctgaa gagggagctc aaagcccccc tccactccag ggctgatgtg aggatgtgag 238440
   ttaaggactt ggatgctgct cttggggctt tcagagatag gggaggagct taatgacctc 238500
   gtcttcatga tataccctaa gcattctctc ttggctttcc atttgagact tactctgcct 238560
   tcagacctct cagaactctg catatctggt atggtaagag gagcctgagc tacctcttct 238620
   ctgcattgcc ttgaaaacaa aacatggccg ggcacgatgg ctgacgtctg taatcctagc 238680
   actttgagag gccaaggcgg gtggatcgct tgagctcagg agtttgagac tagcctgggt 238740
   aacatggtga gaccctgtct aaaaaaaaaa aaaaaaaaaa aaaaaagaca aacaggactg 238800
   gggaaaagga ataagaggag cccgtccaac cttccaattt ccaccaattt taatgtggat 238860
   ccagaattga gaatgctagt ctagctcaac tcaacagggc cagtggttct acttgtgatt 238920
   gggggtaaag gtcctagggt agtaaagaga gtgagtacct gcctggcttg tccgctctgc 238980
   acatgtctct gggcagtagc tgtgggatgt ggtcctggac actagatccc atctgtctcc 239040
   tcctatggag gcaggatctt acagggaaca gccggcagcc aaggctagct ggagtctggc 239100
   ctacaccata taaggagatg gtgcaggaaa ggctgaaagt tatggtgggg gccgaagagg 239160
   gcgttgagtt gcagtaacca tgcccaagcc tttgtggagt taatgatcac agtggacaca 239220
   ttctggaggg gtcgtcggca tcactgggcc ctgctccagg atctcagcaa ggatgttatt 239280
   attatcatga gggtgcccaa ttcagtttgc tacttgttta tgggctgatt gcaatttgga 239340
   tagagtttaa cccccacccc acccccgcct tttgttttta aaaatttgct gaggtgaaat 239400
   tcacatgact taaaatgatc tgttttagag tgaacagttc agcggcattt agtacatgca 239460
   cagtgttgtg caaccaccac tgctatttag ttccaaaaca tttccatcat tccaaaataa 239520
   agccccatac tgattaagca gtgactaccc atccctgcct tctcccagcc cctggcaacc 239580
   atcaatctgc ttttgttttt aatttttttg agacagggtg tttctctgtc acccaggctg 239640
   gagtgcagtg gtacgatcat agctcactgc agcctggaac tcctgagctc aagcaatcct 239700
   cccacatcag cctcctaagt agctgggact gtaggcatgc accaccatgc tcagataatt 239760
   attattttta ttttttataa agatgggggt ctccaactcc tggcctcaag tgatcctccc 239820
   acctcagctt cccaaagagt tgggattaca ggtgtaagcc accacaacca gccaatctgc 239880
   tttctttaac tctctctttc tctctctctc tctttttttt ttgagatgga gtttccctct 239940
   tgtcacccag gctggagtgc aatggcatga tctctgctca ctgcaacctc tgcctcctgg 240000
   gttcaagcaa ttctcctgcc tcagcctccc aagtagctgg gattacaggc ctgtgccacc 240060
   agaccgagct aatttttgta tttttagtag agacggggtt tcaccatgtt ggccaggctg 240120
   atatcgacct cctgacctga ggtgatccac ccgcctcagc ctcccaaagc actgggatta 240180
   catgtgtgcg ccacgcacct ggcctaactc tctctctctt tttaaggctg tttaaaaatg 240240
   aaactattca gtagctcaag tataccttac tgtaaagagg aagtatttat gcagtgctca 240300
   gtcatcttga tcagagatga ttatcaaaac atcaaaatat ctctatgcca gctggcaact 240360
   aagaaatacc accagatgtc ctctgcctct ctggccttgt tagtttcttc tccaagagcc 240420
   cttggatgtg atctgataat gaaaaggtga atgtctagtt cagtgggagc cttcacatct 240480
   accccagggc tttggctgtc tccaatctga tggataaacc gcttgctaaa tattttgact 240540
   atcagccctg tgattaaggt caggattaga actgttgtat ttgtaagaac ctctcctaaa 240600
   gatggcctcc cattgcccac ctgtttgaat cctacctgcc ttaaacattt ttgccatttt 240660
   tatttatttc ttagaaacag gatcttgctc tgtctcccag gctggagtac ggtggtacaa 240720
   tgttggctca ttgcagcctc agactcctgg gctcaagtga tcctcccgcc tcagcccctt 240780
   gagttactgg gactacaggc gcatgccacc atgtctggct aatcgtgaaa attgtttttg 240840
   tagagacaaa gtcttgctat gttgcccagg ctaatctcaa attcctggcc tcaagtgatc 240900
   ctccctcctt gacctctcaa attgctggga ttacaggtgt gagccattgc gcctggccgg 240960
   ttttttaaat gtctcagtct aaagttcaat attcatcccc tagcccctaa gccatgtttt 241020
   taacacctcg ataaacgatg tgctgacttt acctaaaatg tgtccagtat tccttctgag 241080
   atggaaagat gctattttgg gggtaaatat gttgatcttt tccagaagga gtccaagaca 241140
   gctgatgata gcttttgctt gttctctata gaagagcaag tgatggccgg gcgtggtggc 241200
   tcacgcctgt aatcccaaca ctttgggagg ccgaggcagg cggatcacga ggtcaggaga 241260
   tcgagaccat cctggctaac atggtgaaac cccgatacaa aaatattagc cgggcgtggt 241320
   tggtggcggg cgcctgtagt accagctgct ggggaggctg aggcaggaga atggcatgaa 241380
   ctcaggaggt ggagcttgca gtgagccgag atcatgccac tgcacttcag tctgggtgac 241440
   agagcaagac tccatctcaa aaaaaaaaaa agaagagcaa gtgatgtgtt taagacacac 241500
   ctcgaagcta ctgccataga atgtgtgacc atggttatgg atttcataag gggcacccta 241560
   agagggaaaa ataatctgac ccagatggat cttccgaggc actgtgatgt cgtgcctaag 241620
   agtgcagcct tgggaggaag acagagaggg ttcaaatccc gctctactgt tatcagctct 241680
   ctgtatacct tggacaagtc actcaacccc ctcagctgtc aggttccaca tcagtaaaat 241740
   ggggaaaaca gtaacactca catcataagg ttcttgtaag gattcaatga gataattcat 241800
   gtgcagtcct tagaatagtg cttggctcat atagtaagag ctcattcacc gtcaactatt 241860
   attatttaat ctattttcta tcatattttg gaataaaaca ttttggctta ctaaaaaaga 241920
   acaaaaagaa catgtattta aaatcagtag gcaacagtga gtcagtgagg atgttctgag 241980
   agtaggaatg acatgataag atctgcgttc tgggaagatt aatttgtctg aagcttatag 242040
   ggtgacctga aaggagacga ggaggggagg gaaatctgta aggaggctga tgcaacggtc 242100
   taagctgctt tgtgcaatat ggcagccact agccacgtgg ggctgtttaa atttaaatta 242160
   acgagcttgg cctggtggct cacacctgta atcccaaaac tttgggaggc caaggtggaa 242220
   ggattgcttg aggccaggag tttgagacca atctgagcaa catagcaaaa acttgcctct 242280
   acaaaaaata aagttagcca aatgtggtgg gatatgccta tagtcccagc tactcgggag 242340
   gctgaggcag aaggctcaca tgagcccaag aggttgagac tacagtgagt catgattgca 242400
   ccacgcactc tagcctgggt gacagaacaa gaccctatct caaaaaaaaa aaaaaaaaat 242460
   ttaattaaag aaaattaaat aaaatgcaaa actcaatttt tccaacacaa tagccacatt 242520
   tcaggtattc agtggcctca tgtggctagt ggttactata ctggacatca aacacttcca 242580
   ttattttaga aagttctatt ggacagtgta actgtaatag gttcactgcc caatgcatgc 242640
   agcaagtcaa tacactaagc cactgagttg cagcagagaa acaggtttaa ttgtagggaa 242700
   gagacaggag gaaacctcaa atccatctcc cagaggagtc tggggctagg atttttaagg 242760
   gttttgcagt gggctgaagt gtggagattg ttgattggtg gaaaagagca ggatggagtc 242820
   atgggacagg gagataaaga agctgtgttc tcttgctgat tcagttcctc tgtttggggt 242880
   ctttaaactg gttggcatca gctattctgc tggaattcag gatctgttta agcaactctt 242940
   aaacaaaagc ctatgattct aatgtcagaa atcctatctg taggaacaac gaggttgcaa 243000
   atggtcagta tccagtgtta cctgactttt agttgcaagg aagtgggtca aagttagcct 243060
   gattaatgct gaattgtaac tatatttctg tccagaattc ttcctaccct ggtgaggctg 243120
   gcttcaacaa cactggtcaa aagcagtggt tttcatacat gagtgtcaca tcagagtcat 243180
   ctggaaagtg tgttaaaata caggttgctg ggttccatcc tcaattcagc aggtctgagc 243240
   agggcctgag atcatgcatt tctttttttt tgtttgttat tatactttaa gttctagggt 243300
   acgtgtgcac aatgtgcagg tttgttacct atgtatacat gtgccatgtt ggtgtgctgc 243360
   acccactaac tcgtcattta cattaggtgg gaggaataac attaggagat catgcatttc 243420
   taatgagttc ccaggtgatg ctgacgctgc tagtctgggc cacacccttt gagaaccact 243480
   ctctgaaaca ggacttctca gtcatggtgt gagggaaagg ggagttgttt ggagcatgtg 243540
   aaaagcctta tttatatgcc ccagttaagc ccaattggga tttggactca gcctctttcc 243600
   aggattggca gttgttggca gatgtacagg agtgcttttg gcttttctgg ttaaaagagg 243660
   tgaaaggtgg ggctaattca gagtatggag atcagagggg tgaatcatgg agcaaaaagg 243720
   gggccttatc tgcttcttca tccattctct ctgtcatcct ggagctcagc ttttggggcc 243780
   tgatagggag aaagacgaga cagaagcttt cagaactgag aagtcagaat tcactggctt 243840
   tgttcctcac taataccttg acaactttct ccctaaggca gtggatgact cacgtctagc 243900
   tgcatggtct ggggggagga gcctcagggt acaagtgaca gaattcaatg aagtgcaaag 243960
   tgggagaaac aggcacttca aacctcaacg tattaccttg ctacaagtgt gcctttaact 244020
   cactgtttga gtcacagaag gaaacagcat agggcttgct ctcagggagc cggagtatag 244080
   catctgctga tggccagggt cactggaata tttgcctctg tctgggcaga gtttagaata 244140
   acaggcagag gcatagggtg ggggctgtgc cggaactctg tctatctcat ctgtgtaccc 244200
   ttttatagat ggtcatcgga tttgtatttg catctcagtg tagtcagaca ttcttccaga 244260
   acactccagc agtgcttctg tgcctgctgg aaaatagctg cagctctctc ctgaggcagc 244320
   tcttcccaca gtcccagctg ctcccctggg atcctccttg gacctcccca tagcctggca 244380
   agtaaactta gcacctgggc atatcagggg gcctctagga ccctgttcca gtactgagca 244440
   cagcatcagt actggttagc ttgtgcttgg gctgtgccat ttggaagtat tttgaagatg 244500
   cacatgcacg tgtgtgtgtg tctgtgtgtc tacggtggtg aagaatggag ggatgtggga 244560
   tggggcagat tcagtagatg ccttgagata tggatagttt ggtaaaatta tgataataag 244620
   aaaatggaaa ggatgccagt ttccttccag ctagttaaga cccattactt tactcattga 244680
   cccacctaac ccatccatcc atccacccat gcattcatct ctccatccat ccatccatcc 244740
   atccatccat ccatccaatc catacattca tccatctaat ccatccatcc atccacacat 244800
   gcattcatct ctccatccat ccatccatcc atccatccat ccagttcata cattcatcca 244860
   tacattcatt catctgtcta acccatccat ccatccactc agccaactca tccatccagt 244920
   aatcatatat ccgtttagga taaatgtatc cagcactctc ttttagtgct tctgtgtgac 244980
   tgaggatgtt tgacaatgaa aggaggaagc attcatctgc tgggacccct tctaacagat 245040
   gttttcttct cccttcatcc ctgaacaaca gtaggagaaa caggaaactt gtcttcaaat 245100
   ggtagccctt ttccctgact gtctgaggat catggtctga gaatgtgcct ctcagttttt 245160
   aaaaatcctt gaattccatg attcagtgat gtagttaatg tagacagggc tcttctatca 245220
   aagcagggag cctattgaag atatgagaaa atatgcactg tttctctaga gaagggaagt 245280
   aaacccgcct tcctcagagc tgggaaggct tttgtcccaa gcttctgaaa attaaaccca 245340
   tcacttaacg ttctcactga gcagattgcg gggcttctgg ccctatgctg cttgggttgt 245400
   gcagagctgc cgacagacag ttttgattga atgtccaaat taagctgcca agcagcgact 245460
   ctgcagctcc agggactcca tgttagtatt tatgtcacat ctttagaaat aatcacattg 245520
   caaagcagca cccagagcca tgtctgcagc cgctgcagcg tgggagaggg ggctgttgag 245580
   cacctcctgc ctccatgctt catgctgcat ttactcagca cccaagcaag ggtggggccc 245640
   catggcacgc catcctggtt cctgtcccga ggatatgagt gaggaattgg taaacctcgt 245700
   ctgtccttct tgttttcact taacttgagg caggaggtgg tctccaaaca agaagtaaag 245760
   ctaacttatt ggatgcctga ctcccaaacc atctgtccta ggcagacaga aagaccctca 245820
   gggccaacct gaggggaaca gatgtttatg gggggcaggt ttactcttgt ttttccaagc 245880
   tgggctgaag agctcctctt ccatctgcat caagccctga aaactctgcc gtgcatgagt 245940
   aaagtagcaa gtgctgcatc ccccagtgag atccagtggt gacattgtcc acaccactgg 246000
   ggaatgctag ttattaagag actgggcttg tgagagctca aaggtggact aacccagatc 246060
   ttccctgtcg agggaaatgg tctctagact tgtggggttg tggttgtgtg gagtcagagt 246120
   aagtgtggag caacgctggg aactgtagga ccctgaatga ccagggaaac cgctgtagaa 246180
   aatagttgac actaaactgg gtttaggggg tcataaaacc tccaagattt caaagcttct 246240
   gggtcccagg aggactcagt tactgagcac aaaagttgca tggttttggt gtcaggcctc 246300
   tgagcccaag ctaagccatc atatcccctg tgacctgcac gtatacatcc agatggcctg 246360
   aagcaacgga agatccacaa aagaagtgaa aatagcctta actaatgacg ttccaccatt 246420
   gtgatttgtt cctgccccac cctaactgat acgatatatt ctcccccgcc cttaagaagg 246480
   tactttgtac acctatccca aacctataag aactaatgat aatcccatca tcctttgctg 246540
   actctctttt cggactcagc ctgcctgcac ccaggtgaaa taaacagcct tgctgctccc 246600
   acaaagcctg tttggtggtc tcttcacacg gacacacgtg acatttggca gtgttgcaaa 246660
   accctcagaa gtccgagtat cagctggact gaaccttggg gcacccgcgt tgtgtctagt 246720
   tccagtttgt taagaattag gtgcacaaga ccagcccccc ttttacacaa tgatttcttt 246780
   ctctcagatt tagagtaaca tttttttttc taactaaaaa tgtaattaat gctcaactga 246840
   gaccatatat tgaatgtctc cttcttccaa attcccactg aagtgacaaa gagattaaaa 246900
   atggaaaaaa attgatcagg gtgacaaacc agaaaaatgg tatcaatcag atgccagaat 246960
   ctttggggaa tttttctaag atacaattca gacaatccaa gctgattttg tgttctcttt 247020
   tccaacatct aagcctctta tacaatactt ttaaaaaatg taattgcgtt gattggtact 247080
   ttcagaacaa tgttaaatta tgatagtgat ggtgggcatt cttaatttga ttgtggcctt 247140
   aatagatatg cctctagagt tttacccgta agcataatga aggctgttgg tatatttatt 247200
   acgtcaagaa aagcattcat ttatttctat ttttccaagt gttaaaagga cgaatggtta 247260
   ttgaattttg tcagtcgcct ttttcgtcat tttttaagat aattatatag tttttctcct 247320
   ttgacctagt tttatagtga atcatattat taatagattt tctaaaatta tgaaagattt 247380
   ttgtttgaag agtgtgcgat cggtttctag cttttgaaag atcactttgg tgggggagtg 247440
   aaggaagggc tcaatagaag accacaggca ggaaaccaat gaggaggatc ttaatttcac 247500
   ccagagagga aaagtgagtg gccagggagc aatcatatga gcagctgctg tttatgaatc 247560
   actcatcacc tgcccagcac aataccagga ttcaaatata acttaataat cacagtaatt 247620
   ctatggcaca cttattattg ttcagatggc agtgatatga atagtaggga ctcaggtaca 247680
   caaaatgaga aagaggcaga tcaaacagca cggggttgta gattgcacca aagggaggag 247740
   accagaatga tttctagatt cttactgggg cagttgggtg gatagttcaa cactcactaa 247800
   gactggcatt gaaagacagg gaggagacat ggtggtgtcg ggggagcagg aggataatga 247860
   gcgttgttgg acatgttgag tttagcgtac atatgggaca tccagtacaa gataaccagt 247920
   ggacagttgt acatacagtt gtaaaggtca gtaaagaggt gtgggtggga gttgtcagta 247980
   tttaggtaga agtaaaatca taagagtaga gcccctggaa aaatacggaa actaacaaac 248040
   aaacttccga ataatccatg gtttagttca aagaagaagt ctcaagggaa ataagaaaat 248100
   attttgaatt gaaagaaaat aaaagtataa cacatgatac tttgtggaat ccagattaaa 248160
   aagtgcttaa aaggaaattt atattggtaa attctaccaa cacttaaaga tgaaataata 248220
   ctgattctcc taaaaagtag atgagaaggg aacatttttc aacctatttt atgaggctag 248280
   cattaccctc ataccaaaac cagacaaaaa tagtacaaga aaactacaag caaatatctc 248340
   aaatattttc ttccagttcg tcatttgtgt tttgcagaac aaatgttttt aaactttaat 248400
   aaagtacaat ttatcatttt tttcttttat aggtcatgtt tttagtgtca tgtgtaagaa 248460
   ctctttgtaa acataaatat aaaaatcttc atagaaatat tagcaaatta aattcagcaa 248520
   tatataaatg aaaaatacac tccgtccaaa tgagttttat cctgagaatg caagctggct 248580
   taataataaa aaaaaaaatc aaccaatgta atttaccata ttaataatct aaagaaagaa 248640
   aaaaaattaa catatcaatt gatgcagaaa aaattggaca aaactcaata ttcatttatg 248700
   atgaaagttc tcagcacact agaaatagaa gggaactttc ttgatccaat aagaagtatc 248760
   taccaaaaac ctgtaacata cttaatgatg aaaaactgca tgctttctca ttaagctatg 248820
   aaacaaaact aaaatgttca tcctccccag tcctattcaa catcatgtca taaccagcac 248880
   agtaagataa aaaaaatttt taaaggcatg tggggctcgg ctcggtggct catgcctgta 248940
   atcccagcac tttgggagcc tgaggtgggt ggatcatctg aggtcaggag ttcgagacca 249000
   acctggccaa catggtgaaa cccccacatc tactaaaaat acaaaaatta gtcgggcgtg 249060
   gtggcagatg cctgtagtct cacctacttg ggagtaggca ggagaattgc ttgaacctgg 249120
   gaggcagagg ttgcagtgag ctgagattgc accactgcac tccagcctgg gtgacagagc 249180
   aagactctgt ctcaaaaaag taataataaa ataaaaaata aaaaccatat ggaatggaaa 249240
   ggaaaaaaag aaaaattgtt tctatacaca gaaaacagta ttgtttatgt agaaaatcca 249300
   caggaatcta ccaagaaatt actagaaata atgagtgagt ttagctgggt tgcagcatac 249360
   aagtcaacac acaaaaatca attgtatttc tatatattaa caactagaaa ccaaaaattt 249420
   aaaaaatact acttacagca gctgcaaaac aaaggaaata tttaggtata aatctaacaa 249480
   cacatgtcta gaatctatat gctaaaaatt ccaaaacact gaaagaaatc aaagaatacc 249540
   taaataaatg aagagataac ccatgttcat ggattgaaag actcagcaga gtgaagatag 249600
   caagtcttac gattgatttg taggcttaat gaaattcaag tcaaaatccc agcaggattt 249660
   tttgtatgtt tggacacagt gattctaaaa cttgtataga agggcagtga atccagaatg 249720
   gccaaaacaa ttcttaagat gaagaataaa gttggaagaa ttactgtacc caatcttaag 249780
   acttactata taaagcttca gtaatcaaat gtgatattgg tgacaggata gatacacaag 249840
   ccattggaac acagtagaga atccagaaat agacccacac aaatacggcc tattgatttt 249900
   caacaaaagt gcgaaaacaa tgaaatggaa aaaggacggt ctttcaacaa atgatgttgg 249960
   aatgattaga cattcatatg aaaacaaaat gaaccttgac ctccatctca caccttttgc 250020
   aagaattaac tcaaaatggg tcatggagca aaatataaag gtataaaatt tcagaaaaca 250080
   caggagaaaa tctttatgac ctgtagttat acaaagagtt cttagacgtg acacttaaaa 250140
   acgtgaccca taaaagaaaa aaaaaaaagt ggtaaattgt actttattaa agtttaaaaa 250200
   cttttatttt gcaagaaaca aactataaac tggaagaaaa tatttgcaaa tcacatatct 250260
   gacaaaggac ttgtatccaa ctcattgtat cagaactctc agactcattt gtatgaagcc 250320
   agccttactt aaaatgtaat ctaaaaacct gaacaaacac tttaccaaat aatacaagga 250380
   tggccaatac attcatggaa agatattcaa tatcactaga aattacagaa atgcaaatta 250440
   aaaccacaat gagataccat tgcacactta ctagaatggc taaaataatg atgataatga 250500
   taataaatac tgtcaatatg gtgtgctaat gagtatgcag aacaagagga actctcattc 250560
   atcactgtgg gaatgcaaaa tggtacagct gctccgggaa agtgtggcag cttcttataa 250620
   agctaaacaa acgcatataa ctgaatgatc ccactcctag ttatttatct taaagaagtg 250680
   aaactttatg ctcacacaag aacccgtgag tatatcagct ctatttgtaa tcactaaaat 250740
   ttgaagacca ctgaaatatt ctttaacagt tgaatagata aaccttgata catccatgca 250800
   ataaaatact actctgcaat aaaaaagaat taactgttca tatacacaac aacatggatg 250860
   accctcaaag gccctttgcc gagaaagaga aaccaatctc aaaaggtttt cttgctgtat 250920
   gattccattt aggtattatt cttgaaaaga taaaactgta gtgatgagaa acagatcggt 250980
   ggttgccagg ggctgaggat gagaagagga tgtgactata gaggagaagt atgaggaatt 251040
   tttccgaggt gatagaactc ttctgtattc tgattgtgtg atggttacgt gaatctatac 251100
   atgcattaaa atgtataaat tttttcacaa aactgtgcac caaaaagtca attttactgc 251160
   atgtaattaa acacataaaa taaatgttat aggcagaaaa taagctgata aaagacacaa 251220
   gtacacacag agagagagaa gagagagaaa agaaggcaat agtccaagat gaagagggag 251280
   atggataaga ggaatgcaga agaaagaagg ttgtcatgtt atgatactaa aaagtaattt 251340
   aaaacagtag actaaatcaa agtgtgtaga agcagtagat agaggagtgc ctactgcaga 251400
   aaatgtaata agtaatatgg aaacaagctt aaggagtgct ccaatgggga tggcagggga 251460
   aagggacaaa taaataaata taaccactaa ataaatgaca gatgtagaga acaaatagca 251520
   tttatccatg ggtaaatgag tatcagatac tcctagagaa aagaccagag caaatggaaa 251580
   agcatctata ctgtattcaa agtagagttc aagacaaata tataatatac tatataaaat 251640
   gtgtctttta tttatggtaa gtaaagtctg gggacaaaaa gtaatcatga acctagggcc 251700
   aaaatgcata aagcaaaaac ttgaaatgaa aggagtaatt tacagaaata ccataataga 251760
   agggctttaa aaacacactt tgtcagtctt ggacagataa aagcggttaa gaaaatggat 251820
   atagataatt taaataatat aattagagtg aatataaaat acctctctat cttgaatcct 251880
   gaacccttca ataaaagaat tacatttttt ccctagtatt cacaaaatta gtgatatatt 251940
   aacttgttaa ggaaacctca ttaaattccc tccaaaatgc ccccagaaat tgctcagccc 252000
   acattttccg ataaaaataa aacagaacta gaaattaatg acaaaaacat ataataccaa 252060
   agatcaatgg atgagtgaga tggaggtagg aatcagatgg gagcctcaag aattgaaatc 252120
   ttgaaattgc ttctgtaagg aatgggaaat taactaggga cacccaatag gtttgacaag 252180
   cagtgtgcgg gactttgctg tggttggaga tcatgggtat atagtggcat gaagccccta 252240
   tgtgtgacac tttcccctgg ggacactgtc tagagcaaat ttcactcatg gttgagggta 252300
   tgcacacagg atgttgaaga gacaggaagg taaggacatt taagccactg gtcagaagcg 252360
   attgaatggc ttttccatat gggaagtggt agggaggaag taaaaccgta gcaatgcact 252420
   gatggaccag gggaaaggaa acagttgagg aactggaggg tttgatgaaa tgaaaaaaaa 252480
   aaaaaagcca ggtaaaggga gtagagacat aaaaggataa actaccactg gagagtttag 252540
   aggctatgcc atatcaggtg aaactccaaa tgcctggact ggagtggaga ggaggtcata 252600
   agaagaatgg atgatacgtt tgacttcacg tcgtctatta ttctcagacc tcctccaaga 252660
   ttcatttgta tgaaaatcac attggaggga tactctttta gccctggaat ttatcttgat 252720
   tgttacataa tttggtctaa attctctgaa aggagaaagg aaatcaacat ttgttgagcc 252780
   tctgccatgt gccttacatt ccactttgac actgttatcc tctatcatta gggtagtcat 252840
   ataatttatt gttaaaattt ggactctctt aagagtgaga ggacaacagt tctaaattga 252900
   ggttgctctg ggaaaactgg gatggatggt cacccccact attatccaag agcccatttg 252960
   agttgtctgt gtaggtttat gagaatttgg gggtggggag acaaaggctc agagaagcca 253020
   tttgagtgag atcacccagt tagtgagagt catggcagag gtagaattga caaccgtgtc 253080
   tgcctgactc tgaggtctag ccattgctaa cttagttcct gattcaccac gctagctctc 253140
   caccattgtt tcactagctg tggcttgaca ggctctcttc ttgacagaca ccatgacagt 253200
   cccagctgga agtctgtctc aacacagata attagatgtg tgataactgt accactgcca 253260
   gcaatgatgg gaagccagct gttctaggag cctttgggac cacgatgtgc tcgggaggac 253320
   tttgatgctg gatgtgggct gcccaaagac cccacgtttg ctggttttgt aatcagagcc 253380
   tatcaggtga agattataca gcatggccag ttacagaggc tgggaagggg attctgaggg 253440
   atttcagggc tcataaaagc cacagcatca gagaaccttg gggtcagaaa cccccagggt 253500
   tagaagaagt aataagtaaa caggaatcga tgggacacaa atctagattg tagagcaaag 253560
   ggctaaagaa gcttccaact tgggaaggta tggcagggca ggggggttca agcaaaggac 253620
   caggtaaact gtcagccacc aacagataac tgaaacagga gaggggcagg gcaggacagt 253680
   gaaaataggt tgatggatct gaaatgattg acctgtttag tctctccctc caaccctggt 253740
   tagggcatgt gctgaggatg tccagctgac ctcaatgagc ttatcctggg tctgtgtgtt 253800
   attcaccacg gagctcattt tctgggttct atgatgcaaa ccaaaagttt ttaagtatca 253860
   ccctgcggat ccaattaccc tataaactct ttggaggaat aggatggggt ctctgaaagc 253920
   cagggcaata gattcttgga aactgtaaaa gcacaactga atttgtaaat tgttttaata 253980
   acacaattaa tacaagaata cattctcttt gtcagaaatt taaaatgtga tggataaagc 254040
   taaaatcttt attcacaacc ctcaaatcag gttcctctac ctgttcctca gaggtggaca 254100
   ttattttaaa tcaattttgg gtatatcttt tcagactctt ttctatatat ttacattcat 254160
   atatgacctt gtagaaaatg cattttactg tacctttctt ttaaatattt ggagggcctt 254220
   taaaattttt ttattctgac ataatctcaa aatgacagaa aattgcaata acagtaaaag 254280
   aaatttctgt atacacttta cctgatgtac taattgttta tattttgctc catttggttt 254340
   agcattttct ctactaatgt atttttatat gtgtgcacat tttttttctg atccatctga 254400
   gagtaagttg gggtcatggt ttccacttat cctgaaatac ttcagttgta tttcccaaga 254460
   acaaaaatat tcttagtaca tttattcaaa tgaggaaatg caacattgac ccaatgctat 254520
   tatctgacag ttcagctcca gatttcacta attgaaccac taatgccttc acgcttcttt 254580
   cttaaacaaa gaggcattat attgccctca tcttcttttt tcaacgtgct tcatcttgct 254640
   tttttcaaca cgtttctgca tatagataac ccaatgttat cggcatgggc aaagtgcgtc 254700
   acagtatgaa tgcgccatat attatttact gtttccccca ttggtgggca ctcaggttat 254760
   ttcctacttt tataattgca cacatcctca ggctgaatat tagtgggtat tactttggat 254820
   taatgttctt ttatttttcc ttaataaatc cttcactgga atgaattact gttttgagga 254880
   cacacagtga ggacaaagca agactcacaa tattgaggaa agttccttca aaaggagtta 254940
   aataagagat gctcctgatt cacataatgt gggatcggga tatgaaagca gcacaattat 255000
   tctgagtttt ctgacagata attgtatctt cttagaaaga aaaaacaatt aaggaacttc 255060
   ttggtgaaga acaattcttg gaaaaatagt taacatgcat gctatctttt tatggattca 255120
   gtgctgtctt agagaacttg gaccagaatg cagaggcctc gcttgctcca aagaccctta 255180
   gacagaaaca ggcagacctc accctgcacc cagagcccca tgcccacggc aagcagtgtt 255240
   ctctggacca ggacacgctt ctcctgtact ctttgcaaac ttccaccagt ggggacgttt 255300
   gctatttctg ctctgtgtga agcatttggt accatctggg ctggctgagt ttcaacaaag 255360
   atagtaaaga aacatttatc aagaaaaatc aaagtcggtc tcgccaagtg tggtctagag 255420
   agatttgctt ccgcgataca tatgcggctc tcttgctgct gggaaacacg actcaacttt 255480
   gaaacacttc aggagggaca gccctttgga tcttagcctg cagctactgt caactgctca 255540
   tgatagatgg ttttagtagc cagagctctt ggagatgtga atgccagcag tctcaggatg 255600
   cattccacag cattccgttg cacagaaagt tttggcccct ttggtagaga ataagctcaa 255660
   aaggacaaaa gtagaagaaa ggacctataa aggtgaggga agagtcatgc ctcagtgagt 255720
   aatgtttggc tctgcaatga ttagattccc aagaaaaggt cattcttggc tgcacacagt 255780
   ggctcacacc tgtaatccca gcactttggg aggccgaagc gggaggatca ctcgtggcca 255840
   ggagtttgac accagcctgg gtaacatagt gagactccat ctctacaaaa attttttaaa 255900
   agttagctgg atgtagtggc acatgcctgt agtcccagct attcagggtg ctgaggtggt 255960
   agcatcgctt gatcctggga gatcaaggct gcagtgagcc gtgatcgcgc caccgcactc 256020
   cagcgagacc ctgtgtcaaa aaaaaaaaaa aaaaaaaaaa ttgctcttaa accacgagtc 256080
   ttgagattga cagcttcaaa atacaacact cttgtccagc aaagagcact ctaaaagagc 256140
   tgtaaattcc ttaagcttat gatagaagat ctttttccag tgactaccct tgtggtgtca 256200
   gttcataaga ctggggcgga ggagggggag tggccttgcc agggagaaga aagatgagct 256260
   gtttcctctt gcctctgtgt taccaatcaa ccagacagac aagcttctgg cgcatctgag 256320
   tgtctcaatc ctgtgtgtgt gcagctcact atgtgataga tgtgtcactc caagatccca 256380
   gcctagaaac tggtacatgt tattttcctt ggaaggctca agaaatgaaa gtaacagttg 256440
   gatattatat tctatgggga aaatgcatgg gtattttgag aaattattat tctccagaac 256500
   aatgttactt aatgtgtgtc ttatagactg gcagcatcag catcacatgg aaatgtttta 256560
   gaaaagcata tggacaggtt tcactgcaga cttagggaat cagaatctat ctgtgggggt 256620
   gggtgccaga tacatgtgtc tcatgagtcc tctgagtaat ttttatgcac actaaggttt 256680
   aagaagctct cctgtggaag ggttcgagta aacatcgaat gcattgttaa gaagaaaacc 256740
   acaaatggtt ccatttgatg tcagtacgtg tggaaatggt taactcttca taataaaaat 256800
   agacaaacag gcaaggcatg gtggctcaca cttgtaatcc tagcactttg ggagaccaag 256860
   gctagaggat catttgcatc caggagtttg aaaccagtct gggcaacata gagagacccc 256920
   cacctctaca aaaatttttt taaaaattag ttaggcatag gttggcaagc acctgtagtc 256980
   ccagctactt ggaaggctga gttgggagga tcacttgagc ccgggaggtc gaggctgcag 257040
   tgaaccatga tcatgccact gcactccagc ctgggtgaca gagtgagacc ttgtctctct 257100
   ctctctctct ctcacacaca cacacacaac aaaaacgtag tgaaagaggg cattgcatat 257160
   gaataagaat aataatgaaa tagcagtaat atatagcagt taacatttat tgagtatttt 257220
   ttaagttcca ggttctgtgc taaacatgta tattctttat ccaattttac tcttgtaaca 257280
   acctatttta ttgatcaaga aattgaggtt catatgtgtg aagtaactgc ttggtaagat 257340
   gcggaccctg ggatcttata gccctgtgtc catgacacta tatggtctgt cttggggaca 257400
   agattaacat tttattttgt cccctatatc cccagggcct agcagaatgc ttagcactta 257460
   gtagggactt gcttgttgaa cttaactgat caaggtcaac atcttgcttt tggtgtaaaa 257520
   atgagatgca gcttgcaagc tgagatggga gatgaattca gcctgtggaa aaatagaaga 257580
   cactgccatg ttgctggagt gcagaaaaaa tgtcagcaca gaataagtta cagcttgatc 257640
   ctggtagccg attcaatgct tctcccatct catttattcg gtctcaaccc cttactagct 257700
   gtatcatttt agggaagtca gtcttattta ttttccagca atggtcagct gtttgagtgt 257760
   aggaaaagga cagagtaaga aaggacaggt atttgaattt aagtagggct ggggtcatgt 257820
   gagacaagta aaataaaaag aaagaggggc aagggagttg agggtctttc caaaggagta 257880
   gctaaaatgc accataaaat tgaatctgga tatgaatgga gtaaaagcaa gagggaatag 257940
   ggagatcaag agaatgtgtt gggttcgagg gactgattgg tggagaatgg actgacgaat 258000
   aggggaagat ttattgcagt agaataatag aactaatgaa ttggaaaaat agaaattggt 258060
   gtaagatagt agggtacttg aaactgagat tgagaaagtg gtataatttc tgggataata 258120
   ggctccagag cgtgaccatg gtaggagata actatgattg ggggtgagca aaggttgcag 258180
   gaaatgaggg ggtgctttct ctcctacttt ccaatattct ttcttctaat ctgtttttct 258240
   tgttatcttg agtttacaaa atttctggtt ttatttatgc tgtggatatt tttccagcct 258300
   tttccctatg ggctgtctgg gtgcaggtta tgggtgatac atggataata ggtgcatctt 258360
   ggtttagagg gtaactcttt atttcttact gcacttatgg aacacgagaa aaaaaagtat 258420
   taaataatgg ctatggtaat ggatatcttt gtcttattcc ttactttaat ggaattgcat 258480
   ttatcatgaa ccatggtgct gttatttcag atagccttta tcctgttaaa attctctgct 258540
   gtttctagat aactacaatg atcttatggt tttcttcttt gatctataaa tatgatgaat 258600
   atattgaaaa tagatttcct aagattgaac catctttgca ttcctgatag gagtcattat 258660
   gtattcacct tttagcagat agttagagtc gatttggatt gaatgtatta ctgtggattg 258720
   ttctactgta agtgagatga ttgacattgt atccttaaga gctgtgcaat tctcattttc 258780
   tagtctgctt ccattgacta cctgcattac tggtttctga attgattcct aattaactac 258840
   ttcctgtttc tccaagctga tgatttagtc tccctcatcc tttctatagc cagttgcatc 258900
   ttcactgctg attccctgcc tccttttctc ctgtaagttc atcttttttt tttgacggag 258960
   tctcactctg tcgcccaggc tggagtgcag tggcatgatc ttggctcact gcaagctctg 259020
   cctcctgggt tcatgccatt ctcctgcctg ggcctcccaa gtaggtggga ccacaggcac 259080
   ctgccaccat gcctggctaa tttttttgta ttgttagtag agacagggtt tcaccatgtt 259140
   agccttatag cctctttacc tgggtgttct ggagtcaggg tggagctgag ctctaatgct 259200
   gctgacattg ttccagcctt ctctctatgg gctgtctggg tgctggttat gggtgataca 259260
   tggataatag gtgtaccttg gtttagaggg taattcttcc tttctttagc tttttctttt 259320
   tgaatggagg taaaattaac gtaacataaa attaaccatt aactgttttt aaacgtacaa 259380
   ttcagtgaca tttatagaat attcacagtg ttggacaacc atcatctcta tctagttctc 259440
   agaaattttc atcacctcta aaagaaaccc tgtactcatt aagcagtcac tccccatttc 259500
   cctttccctc cagcctctgg caactattaa tagtagtcta ctttctgtct ctatggattt 259560
   acctgtttgg gatgtttcat gtaaatggaa tcatacaata tgtgaccttt tgtgttgggg 259620
   tacacaaatg ttcatagcag cagtattcac aacagccaaa aagtggaaac aacccaacta 259680
   tgcatcaact gctgaatgga taaacaaaat gtgggatatc tatacaatgg aatattattc 259740
   agtcatgcaa aaaaatgaag tgctgataca tgctataatg tggtgaactt tgaaaacgtt 259800
   atgctaaatg aaagaagcca gcctttcctg tgggctttgt ggaaaataag cttagaaaac 259860
   ttacagttaa tcttggtatg ggaagagcag tgtaaaagtc agaaatccat ttttaaccaa 259920
   cacagaccag agttgacatc ttatcagccc atctcatttt gtgcactgct tcagctatgg 259980
   atgttaataa aattaaattt ctcactaaac aagtgtgatg ccaaagaacc ttccacaagt 260040
   tctgcagctg tcactgcttg gtgcttaaac atgttggaca gtattaatgc aaaaactgct 260100
   ttttcttttt cagtaagaga tgctaaaaac cttgtaccta tggaccccaa tggcctgtca 260160
   gatccctacg taaaactgaa actgattccc gatcccaaaa gtgagagcaa acagaagacc 260220
   aaaaccatca aatgctccct caaccctgag tggaatgaga catttagatt gtaagtggaa 260280
   atgactgcag tgagcatggg tggtggaggt tgggttatgt gccacctgaa atcagggagt 260340
   tcctcctccc ttagctcctg tttccttcct tccctacctc cctccttttc tgtcttctcc 260400
   atccctcact cctatctttc cctcccttct tttccatctg caattttgta ttgtgagcca 260460
   agaaaaaaaa ttagagctat ttttttaata gagatgagaa tgcctgtggt gtgggggagt 260520
   gggtaggaca ggaaatggtg tgatgtgttg ctaagtgaag gcaagttgtg ggagaaatga 260580
   tggggctcag caaatagggg aacataacct acagggtgtg gtgacagcct ctctagagtt 260640
   gggtttatgg atgatgcttg gcctgcagcc ttctgggaca tctccaccag cctcccttga 260700
   gggtcttccc tggccagggc tccaagaatc tgggagtctc tttcttgtac atctaggcat 260760
   caggagttgg ttataacagc caattcactg agcatatatg atgtgtgaca aacactgggt 260820
   taagcgatcc gacttcatcg gctcatttta ttcccaatgt agcaataggg cttatagagg 260880
   ttaaagagta tgcccaaagt cccatagcaa atatggggtg gagtgggatt cagcttctgg 260940
   cagtctgact ttagcggtca tactttttta gccaccagct agaagcacag ttccttgtcc 261000
   tgtctcctct tccctctctc agggcaaagc tgggcccctt ctcagcctca gtcaaggaca 261060
   tgtctcagaa cagacaattt ccccaggatt tttctccatg catttcctca gcctcattct 261120
   caacccacct gtgtgcagat atacacatgt acacacccaa ttatgcaatt gatatctgta 261180
   tttcttatag gcaataggaa ttcctgaata tcctgaaccc tattaagaca taagataaca 261240
   aaagtagtta gaactgttct tgtcatagcc agtctaagac ctctgcctgt ttaagccaca 261300
   ggctgtcaaa cagaaaaagc tctcactccc tggagagcta gcctgggatg gaaactgaat 261360
   atgtttcttt cagcagcttg taatctttaa aactttctac ctccaggtct tgtcctcttg 261420
   tacatttatt ctgcttgaga aattactaca acctccactg atgtcttttc tttttctcta 261480
   tgcagtcagc tgaaagaatc ggacaaagac agaagactgt cagtagagat ttgggattgg 261540
   gatttgacca gcaggaatga cttcatggga tctttgtcct ttgggatttc tgaacttcag 261600
   aaagccagtg ttgatggctg gtaagtaaga ttttgccttg aaagctacca tacagcttgc 261660
   tccatcaaac gcggggtcaa gtatgttttc ctttttctcc aaaaattgaa atactaaaac 261720
   agagtcccaa agtgaagtct ggttgattgg atctgccttg cagatatgtt ttgtttggtc 261780
   cttgtactaa aaacaaaaaa gaaagaggcc aggcctcgtg tctgacacct gtcatcccag 261840
   cactctggga ggccgaggca ggtagatcac ttgagctcag gagtttaaga ccagtctggg 261900
   caacatggtg aaaccccatc tctatcaaaa acatgaaaaa ttagctgggc atggtggtgt 261960
   gtgcctgtag ttccagctac tcaggaggct gagatgggag gatcacttga accctggagg 262020
   cagaggttgc agtgagcaag gtggtcagag attgagccac tgcactccag cctgggtgac 262080
   agagtgataa aaaagaaaga gagagagaga gaaggaatga aggaagggag ggaggaagga 262140
   aggaaggaag gaaggaagga aggaaggaag gaaggaaagg aagaaaaagg gaaagggaaa 262200
   ggaagaagaa agaaagaaga aagaaagacg gaaagcagga aggaaggaag ggaggaaggg 262260
   aaggagggac agaaggaggg agggagggaa ttagttctag tattttaaaa atctggggct 262320
   agaaggcaac aattgagaac agttgaatag ttaatgcccc ctttagacag gacatggatt 262380
   ttctatttgt caccgtcctc accactccct attttattac atctgggcca atttactaaa 262440
   ctacatgatg agactgtaac tgcccagtgg cttctttgtg cctgctgccc agacagagcc 262500
   gatttatcaa gatgggagaa ttgcaatagg gaaagagttt tacacacata gagccagcta 262560
   aatgggagaa cagagttttt ttatgactca aatcagcctc cctgaaaatt tggaagctag 262620
   ggcttttcaa agatagtttg gcaaggagta gggtggctag ggaatgggtg ctgctgactg 262680
   gttggggatg cagtaatagg ggtgtgaaaa atggtcctcg tgcactgagt ccacttctgg 262740
   gtggggccac aggactagtt ggcaggtcca ggtggggcag tctggttgtc aggaaggcaa 262800
   aagcctgaaa agacatctca aaaggccaat ctcaagttct acagtagtga tgttatctgt 262860
   aggagtaact taggaagttg gaagtgttgt gagctctggg ataaaggctg ataataatcc 262920
   atgtctacat cttagcagaa ttcaggctcc tttcattctc ctaacttggc aatatttcat 262980
   tagttttaca aaggcagttt agttttggga aagggctgtc ataatttaaa atatacaaca 263040
   aatttctttc aaagttaggt tggcggtctg gtcggggtgg agccattggt tacgagaaat 263100
   gcaaaaaaac ctgaagagac atctcaaaag gccagtctac aatagtgatc ttatctgcgg 263160
   gggtcattgg gaaagttgca aatcttgtga cctctggaat aatggctggt aatcctttat 263220
   gtctacacct tagcagaatt caggctcctc tcatcctcct aacctcatgg tccctcatta 263280
   gctttatgaa ggtagtttag ttttgggcaa gggctattat catttaaaat gtacactagg 263340
   ccgggcgcag tggctcaccc ctgtattccc agcactttgg gaggccgagg tgggtggatc 263400
   acgagatcgg gagatcgaga ccatcctggc caacatggtg aaaccctgtc tgtactaaaa 263460
   atacaaaaat tagttgggtg tgctggcatg tgaggatcac ttgaaccagg gaggcggagg 263520
   ttgcagtgag ccgagatcat gccactgcac tccagcctgg gtgacagagc aagactccat 263580
   ctcaaaataa aatgaaataa aataaaataa acactaaatt tgtcacaaag ttaacttggc 263640
   ccgagcccag gaaagcgtaa gggcagtttg gaggttaaag gcaagctggg ggttggttag 263700
   atcaaatctc tttcactgac gtcatcattt tctccctgtt acaatttctg caaagacagt 263760
   tccatgactg gctccatgga cactagagtg gcttgtacac ttcacctgaa agatatgagc 263820
   aacccactgt cagatggtgt ctgagaagct accgcttcct tgggagtcct tagcaagtct 263880
   ggccacagat catagatgta ctggggtgtt gttttcatag tcagctgctg tgtctccttt 263940
   gtcctgaaga ttttattttg ctcccttcct atggcaaaaa aaaaaaaaaa tatatatata 264000
   tatatatata tatatatata tatatatatc ctccaatgtt accaacagag aggctgggta 264060
   agcccccttg gcaactgttt cacagttaac tgagtcaagg agtcagaaga aatagcttaa 264120
   cctggcacta taactttggg agaagtggca attgggctgc tattacttct ttaagataaa 264180
   ttgtctcata tctgaattgt ctcattcagg accttttcag cttcaagtga aagaaaataa 264240
   aaatcaaact ggtttagcga gaaagagatg tgtcctttgg accacacaca catctttcct 264300
   ttccattgtt caccttatga cttccttgtc ttggctccat gtttcatctt aggccttttt 264360
   tttttttttt tttttttgag acggagtctt gctctgtcac ccaggctgga gtgcagtggt 264420
   gtgatctcgg ctcactgcaa cctctgcctc ctgggttcaa gcaattctcc tgcctcagcc 264480
   tcccgagtag ctgggactac aggtgcccgc caccacgccc agctaatttt ttgtaatttt 264540
   agtagagatg gggtttcacc atgttagcca ggatgatctc gatctcctga ccttgtgatc 264600
   tgcctgcctt ggcctcccaa agtgctggga ttgcaggtgt gagcctccgt gcccagccta 264660
   tcttaggctt ttcttgaaac atatattgag tgtccactag gtgctggtca ctagaaacac 264720
   atggaggtat aagacagaca tgacttcatg gagcttagct tatggcagag ggtgggttgg 264780
   ggagataaac attgaacaag gaattacgca aatgacaaat caatgacaac atgatggaag 264840
   atgtgaagaa gacaggagct gagagttttg aacccaaaag tatctgagac agatttcaat 264900
   cagtttagaa agtttatttt gccaaggtta aggatgtgcc agtgacacag actcaggaga 264960
   tcctgatgac atgggcccaa ggtggatggg gtacaacttg cttttataca ttttagggag 265020
   acgtaagaca tcaatcaata catgtgagat ttacattggc tcaatctgga aaggcaggac 265080
   aactaaaagt gggacgggga ggtgcttccg ggtcataggt agattttaca attttctgat 265140
   tggcaattgg ttgaatgagc tgttatcaat agaaaggaat gtctgggtta cataaggagt 265200
   tgtggagacc aaagttttat catgaagatg aagcctccag gtagcaggct tcagagagac 265260
   tagatggtaa ttgtttctta tcagacttaa ggtctgtgtt gatgttaatg ctggttggct 265320
   tttcctgaat tccaaaagga aggggatata atgagacatc gtgtctgact ctcccttctc 265380
   atcatggcct gaaacagttt ttcaggttaa ctttggaatg cccttggctg agagaagggg 265440
   tccatttaca tggctagggg gcttagaatt ttattttggt ttacagaatg taccccaaaa 265500
   gagttgtgtt aaagcacaat tcacaaatgg ttaaaaacat agttctcata agatagataa 265560
   tgggcattaa tgggcattgg ccaaagattt ccttagctca ctaaggagaa aagcagcagg 265620
   atgaatgaat gattccacag aaggtttgat aaactgatgt tcgtgtagtc agtagaaaaa 265680
   gatatgctga aataactttg ctcagttaaa tataaaactg actgatatac aggacaatag 265740
   aatcataagc tttggggtta tctactagaa tgaaacaaaa aaaagatttt ggccaggcat 265800
   ggcggctcac acctgtaatc ccaacacttt gggaggctga ggcgggcgga tcacctgagg 265860
   tcaggagttt gagacgagcc tggccaacat agtgaaacca tgtctctact aaaaatacaa 265920
   aaattagctg ggcatggtgg caggtgcctg tattcccagc tactcgggag gctgaggcag 265980
   aagaatcgct tgaacccagg aggcagaggt tgcagtgagc tgagatggtg ccacggcact 266040
   ccagcctggg caaagatttc acctcagttt tctaaaagtt aacatgtatc tttgcagact 266100
   agggaggcca tctggggctg acctgatggt gaataataaa ctaaacaaag taatattact 266160
   gtgaggaaag aaatagctaa ttagaaaatg ctctagcatg acattaaaag ttcacacggt 266220
   catctttttg gttttctttt tcccccaagc tttggatatt tttccctaac atgcaaatga 266280
   cttgactttg cagggtgtac atcagttact cacagtggat gtttcattct cattttaaat 266340
   caaacttaaa gaggttaaag agaaagaatg catgatcctc agggggtatc agtgtgacag 266400
   gaggaaacat aataataata attataataa caatagctac tactcattga ctcttaatag 266460
   atgccaggct ctgggttgag cttttccaga tgtcgtggag tcacaatttt acacagcact 266520
   gatcaggtgt gcagaaaaaa taaagcttat ttcttttcta taatggactt agactttctc 266580
   atgcactttt gtgagctcct ggtgaccgag gagaagggac tctatatgct gcatgtggta 266640
   ctttctgagc ttcactgttt cacttttctg actggtcact gttaacagca aaacccagct 266700
   ctgtaaaata ttttaaagaa gtttattctg agtcaatata agtgactgtg gcctggggaa 266760
   acacaaaccc aagaagcttt gaataagtgg tcctgaggtg gttagattac agtttggttt 266820
   tacatgttct ggggaggcag aagttacagg caaagacata aatcaataca tggagattat 266880
   acattggttt gggcccaaaa ggcaggatag ctttaaatgg ggcttatggg ttataggaga 266940
   ttctttaatt tgcagttggt tagagtaaga ctttgctaaa acttggagtt aacagaaaga 267000
   aatattttat gttggctggg cgtggtggct catgcctgca atcccagcac tttgggagac 267060
   cgaggcaggt ggatcatttg aagtcaggag ttcgagacca gcctggccca catggtgaaa 267120
   ccccgtctct actaaaaatg caaaaaaatt agcaagttgt ggtggcacat gcctgtaatc 267180
   tcagctactc tggaggctga ccaggagaat tgcttgacgc aggaggcgga ggttgcagtg 267240
   agctgagatc ccgccactac acccagcctg ggtgacagag tgagactcca tctcaaaaaa 267300
   aaaaaaaaaa aaagagtgtt taagttgagg atgctgtgca gcaagatggc ttgcttatgg 267360
   tgcaacttca cctttgcctg gcctggcgtt agtcctgttt ataatctgat attttattgc 267420
   cacaaagagt ctgttttgtc agtcttatga tctctacttt aacattcatg tcagtccatt 267480
   gttgtatata aactcccaaa gaaaggaggc ataacaagac gtgtccaatc tcctttctct 267540
   catggctgat aattctgttt ttaaggtttt tcttagatcc tcttggccga ggagggggtc 267600
   tgttctgtca gtggggggcc ttaagatttt atttttagcc tacgttccta aaccagcgcc 267660
   cactgaagtg ggactggtcc cacctagctt tggggcatca ttctggcttt gtgctgaaga 267720
   tcctggcctg ttgcttctcc ttgtgagttc cctccatgca gcaccctctc tgtcccactc 267780
   ataggccact gctgggcctg gttcagggag gccaggaacc tgcctgagtc ctccccctga 267840
   ggagtctttt ctgctccttc tgctgctgct ggaatgatgt tgcctggtgc atggataggt 267900
   ctctggccag cgttccagcc gaggaggagg gaaacccacc tctccccacc acctgtcctt 267960
   tctccttccc aaggttctcc aagccccaaa gggaggggct tctcctacaa tcacccaatg 268020
   agttcatcta gagctgattc actgagacgg caatatttta gcagagaaag agtttaataa 268080
   acacagtgcc agccaagctg aaggatggga gtttattact caaatcagcc tcctccaaaa 268140
   ttcaaagact agggtttttc aaggacagtt tagtggtaag tgggggctag ggaatgggga 268200
   atgctgattg gtgggttgag agtgaaatca tagggggtca aagctgtctt ctcatgttga 268260
   gtcagttcct gggtgaggat cacaagacca ggtaagccag tttcgttgta tggattccca 268320
   gtctgggttg caccagctgg tccatcagaa tgcagggtct ggaaaatatt tcaaacacca 268380
   attttaggtc tataatagtg attttatcta taggagcaat tggggagatc ctgaatcttg 268440
   tgacctctgg ctacatgtct ctggagccag aattctaact ttgtggctaa tatgttagtt 268500
   ttacaaaggc agttttagtc cccaagcaag gagatggtta gttttgggaa ggagctgtta 268560
   tcgtctttgt tttaaagtta aacgaatggc caggtgtggt ggctcatatc tgtaatcccc 268620
   gcactttggg agtgccaagg tgggaggatt gcttcagttc gggagttaga gaccagcttg 268680
   ggcaacaatg agatcctgtc tcaacaaaaa atttagccag gcatggcggc atgcacctgt 268740
   gctcccagct actcaggagc ctgaggtggg aggatcactt gggcccagga ggttgaggct 268800
   gcaatgagct gtgattgtgt catcgcactc cagcctaggc aatggagaaa gaccctgtct 268860
   caaagtaaat aaagaaacaa atacaaataa aaaatgaagt tgaactgaaa ctaacttctt 268920
   cccatagtta gcttggccta ggcccaggaa tggacaagga tagcttggag attagaagca 268980
   gatggagtta gttaggtccg atttcttaca ctgtcatagc ttttctatgt catatttctc 269040
   ttactgtcat aatttttgca aaagtggttt aactcccaca catgccatat ccttcttcct 269100
   gggctagtgc ctgccttgtc tactccccta aggctgagag cagggaagaa aaaaacggct 269160
   taagaatagg gatagtgtat caggagtaag cccaaaatat tactgcattt acacttagat 269220
   gatcatttaa ttctccagtt attctaggtt tcatcatttc caaggctcag aatagttcag 269280
   cagtgtgtta gggatacaca gcttgtaagc aacggtgctg ggttcaggtc cagatttcta 269340
   tgatgccaga gcctaagcat atacaaattc cattctgcct cctgatacag ggttaggtgg 269400
   aaggctagag ggtagacagc tgggatttta tttcggcttt cctgactgga tggatgcttt 269460
   gtgacctcag gtgagccact tcacctctct gtactacagc tttcacagca gatatgccac 269520
   ccatcagatt gattacaaca ccccctgtcc tccatccacc acttaggata gctgtgaagt 269580
   tgatgacata atagttggaa gggaactttt ccaaaataaa agccctctgc agatttttgc 269640
   agataaaagt cctcctcaga tttacggcca agacatttgc gatgctgtct tgactgtaaa 269700
   tggaccagat cctagacatt tggattcttg cccctgggat tctttgaaca ttatgtgatc 269760
   ttgagcaagt ctcacaccct ttctgaaact cagattcacc atttagaagg ggatgggctc 269820
   catcagctct tctgttgtgc tgtaattgaa gaccgtgtgg cagaaacagc agatgcagct 269880
   gtggaagcta tcagatgtgg tgcaccagcc ccaggccccc tgtctgcctc ttggccaact 269940
   gccctgtatc tgttgacaaa tccgcctctt tgtttctaat tttgtttatt tatactgtct 270000
   cctttctttc cccttcattt tccagaggtt catctgtttt atttacttat ttattttctt 270060
   caaggaatta actctgggat ttgtgtatca attttgtttg ttttctcctt ttctgtaggt 270120
   ttattttgtg tttggctttg actttcagtt gaacatttta ctaatttatt ttttcttctt 270180
   tcgcctgact ctgctcttta aaaataatga atagcttttt tgtttgtttg ttttgagacg 270240
   gagttttgct ctgatgccca ggctggagtg cagtggcaca gtctctgcag gttcaagcga 270300
   ctctcctgac tgagcctctt gagtagccag gattacaagt gcctgccacc acacccggct 270360
   aatttttgta tttttagttg agacaaggtt tcaccatgtt ggccaggctg gtctcgaact 270420
   cctgacctca ggtcatcctc tcacttcggc ctcccaaagt gttgggatta caggtgtgaa 270480
   ccaccgcacg tggccaaaaa taatgaacaa gcatttaagg gaataaatgt ttcctctgag 270540
   ttttgatatg ctaacattct ggcttttgct acttccttga gggcctgcag ttgaggtttt 270600
   agtttctttt ctaacatgct ttaatttagg catttttttt aacccctaga attagatttt 270660
   tgtttaaatt tttaatatta atgttaagtt actgttttag tgctttcaag ttagagaata 270720
   tggcctgtgc acttttcttt ttccctcctg ttttctttcc tttctttcag catagcttag 270780
   tatttgttca atgttgttaa aagttctgtg gacttgcaag aaaaagatta tattagctat 270840
   tcgtagagta tacttttttg tggagaaaga ataaaccttt tttcaacttt tattttagat 270900
   tcacgggata catgtacaaa tttgttacct gggtatattg catgatgctg aggtttgggg 270960
   tatgaatgat tccgtcaccc aggtactgag catagtaccc aacagttagt ttttcaatcc 271020
   ttgtcttccc ctcccctctc ccctctacag ttcctagtgt ctgttgttgt catctttatg 271080
   tccatgagtg tagggtatac atttattcat tccaatgtat tataaggtcc agatcttttt 271140
   tttctgaggc ggtgtttcac tcttgttgcc caggctggag tgcaacgccg cgatcttggc 271200
   tcaccatagc ctccgccttc cgggttcaag ctattctcct gcctcagcct cctgaatagc 271260
   tgggattaca ggcatgtgcc accacgctca gctaattttg tatttttagt agagatgggg 271320
   tttctccatg ttggtcaggc tggtcatgaa ctcccgacct caggtgatcc gcccgcctcg 271380
   gcctcccaaa gtgctgggat tacagatgtg agacaccacg cccagctaag gtccagatct 271440
   tttatacctt catattgtcc ttttgtttgc ttgagctaaa tgtgtagtgt ttattaagtt 271500
   tcttcaggca aaatgattca caaacattct ttttatttct aggggttttt gccttatctg 271560
   tatatggcaa tattattaac cacataactc tacaagtctt attatattga catgaagtta 271620
   tctttgttgc agctaatgcg ttttctctat aattttactt tgttgctggc attcattcat 271680
   tcattcattg aatatacaca gtgagcacct gctgtgctga agagacacta atgagcaaaa 271740
   cagacaagaa tccgtggcct catggagttt tctttttagt tgaagaaaaa taaacaacaa 271800
   ttgtaaatga acaaaaataa gtaataaaac atggcatgtt tgaggctgat aagtgtcata 271860
   gagaaaagta aacaagaaat ggggacaagg aatcttgggg agtggagaca ttcagatttt 271920
   atagggaact caggaaagaa ctcactgaaa aggtaacata gagcaaagat ctaaagcagg 271980
   tgaggaggga gctgtgtgga tatctaggga gaggttgtct taggcagcga ggacggcaag 272040
   ctcaaaggcc ctggggcagg agtgcacccg gcaagttgga agagcagtaa ggagactcct 272100
   gtagctggag ctggatccat gaggggagag tggaggagga ttaggtcttg gaggatatgg 272160
   ggagagggtt ggcggatcct gtcggccttg tcaggacttt gtcttctact ctgaagaaca 272220
   cggggagcca aggaattaat gagattgaac tgacctttgc atatgtcctc tagttgtggt 272280
   gtggtcgatc ccttttccct agtaggtcct ttcccattct tttatgtcta atctttctaa 272340
   gtcctttggt cttgtgtaac aagcggcacg ttgttggatt tttttttttt tatttttgag 272400
   acagagtctc actctatcgc ccaggctgga gtgcagtggc acgatctcag ctcactgcaa 272460
   cctctgcctc ctggattcaa gtgatttttc tgcctcagcc tcccgagtag cagggattac 272520
   aggcatgggc caccacaccc atctaatttt tgtgttttta gtagaggtgg gccttcacca 272580
   tgttggccag gctggtctcg aactcctgac ctcatgatct gcccaccttg gcctcccaaa 272640
   gtggtgctgg ggttacaggc gtgagccacc aagcctggcc tttttttctt ttttttttaa 272700
   aattttactt taagctctgg gatacatgaa cagaaagtgc aggtttgtta cataggtata 272760
   catatgccag gtggtttgct gcacctatca acctgtcatc tagatttcaa gccccgcatg 272820
   cattaggtat ttgtcttaat gctctccctc cccttgcccc tcacccccta acaggccccg 272880
   gtgtgtaatg ttcccttcct tgtgtccatg tgttctcatt gttcaactta tgagtgagaa 272940
   catgcagtgt ttggttttct gtttctgtgt tagtttgctg agaatgatgg tttccagctt 273000
   tatccatgtc cctgcaaagg acatgaactc atcctttttt atggctgcat agtattccat 273060
   ggtgtatata tgccacattt tctttatcca gtctatcatt gatgggcatt tgggttggtt 273120
   ccaagtcttt gctattgtag ctagtgctgc aataaacata catgtgcatg tgtctttata 273180
   gtaggatgat ttataattct gaatctgaag atcttttttc cacaggaaaa tgttttaatg 273240
   tttatagcta taattgccat gtttcttatg gtgtttctaa agttttcttt ttctttccta 273300
   tttcattctt tttttttcat ctccttctaa acagataact attatcttaa atcagtgtta 273360
   tattactttt ttttaaaaaa actttctgtt attattaata atcacatttt ggtcttatta 273420
   caaaaaaaaa gcaaaaaaca aaaaacaaaa aaaactaatg ctttggaatc agacttcccg 273480
   ggttagaatt tctactgtac cacttcctag ctgtgtaccc ttggcaagtt acttcgcctc 273540
   tctgtgctgt gtttcctcat ttggagataa tagtcgtaat tatgtcacag gattgttgca 273600
   gtatcaaatt agttaatata agtgaggtgc ccaatgtctg ctaaatagca ctcagtaagt 273660
   gttagtcatt attgttaatt gaataattat tagaaaaata taagaaattg gagaatttca 273720
   gagaccccag attcccagtg cccataacca ctattgaccc tatagagaga gagagtgaag 273780
   caaaaaatga aatcttactg tgcatactat ttggtacctt gcatttttca cctaaaaata 273840
   atccgcaata aacataattg tggcaaaact tttgtgccca gctttaataa tttcctaata 273900
   aatttctaag cctggaattg atgactttaa gcatatacac attattaaag ggtttgccaa 273960
   aatgtcctac aattttaaaa atcaatttac agtcttatca atcttgtatg aaacttttca 274020
   gtttattaac attaacatcc agtgctataa atttcaaaga tatgatacaa agattataca 274080
   catattgtat tagaaattat gttgaacatt ttcatttgca tattgccatt tgtttatatt 274140
   tatatatgac tatgtccttt tgcctttttc ttattgactt gtaagaactt tttatatgag 274200
   aagaatatta atatgtcttt cctgacatat gtatgttgca acgttcctcc tcctcctttt 274260
   catgttgtaa attaattttg gtaatatgca tttttcttat ggtatataaa gcattcggtt 274320
   caactccaat ctaatatgat agatatcaag ccacttgcca gaaacatttc tcaagttttt 274380
   cttttcattg actaattgtt atctgaatta tccatgctgt gttcactgtc tcaaatgaat 274440
   tttttaattc agtgattata tttttcttcc gaaagtagtc tttcccactc tcgtatttgt 274500
   tcccttttca cagatgcacc aacctcttga atctcactgc aaatatgaat taggtagttt 274560
   ctagaatgtt ctctcttttg gcagcagttg tatttcatgg gagttacttt gattctgcat 274620
   tgtttctttc tatttaaatg actaaatttt tcacacatcc tattgtgctt atttgcctat 274680
   ccttatagaa gggtgagcac agacatttct gtgtccttgt tctaacattt catatctgga 274740
   taaagggaga caggagtctt ttgtattttt tggaattctg ttttgtcaga tcaggagtcc 274800
   tgttcctgac atttgggcgg ggctacagtg gagacaggtc cataacaggc acccctcaag 274860
   ccagatggag agcccatctt cctcacgttg gttctgtgca tctcaaagtc agaaggggtc 274920
   ctgctcctct ctgagggggc acacgtgtcc tatttggagg cagtagtggc agcgtccttc 274980
   actcactctg tggcgtactg gccaccgtga acacacgctg tggcaccatg cccctgcccg 275040
   ctgcgccagg cactgacatg tctcctcttg catatgaatg ggaatcggca accctcaccc 275100
   ggtcattgat ggtctcagcc ggggtcatta ttgggctctg ttggggcacc ttttatggat 275160
   gacagctggt caattcccct ttatctgtat tttcccaagt gtgagacccc aaatactcta 275220
   gatggttcac agtcagggca taaataatat tgaattgtgc cccaagaaag caattatttt 275280
   gtcagtgttc tttcagttct tcaaattatg gcaaagagaa aatctcagag gtctctgata 275340
   tatcgttaac actctaacac ttgctcttcg ctgatctccc attaaaaagg ggcaggggca 275400
   ggctttcgat tgagagcctt tagtaagcaa caccatctac ttgcacgtgc ataaccttga 275460
   tttccttgat ttccatttgt ttattttttt ttcttttttt ttttattgga acattaatta 275520
   ggttttattt tcactttttg tttccacagt aacatgtatg ggattaggtt acagtgttta 275580
   tttgtcccca atcttttatt tatttatttt ttttattatt tttttttaat tgattgactt 275640
   tttatttttt attttatttt tttttaaatt tatttattta tttatttatt ttttattgat 275700
   aattcttggg tgtttctcac agagggggat ttggcagggt cataggacaa tagtggaggg 275760
   aaggtcagca gctaaacaag tgaacaaagg tctctggttt tcctaggcag aggaccctgc 275820
   ggccttccgc agtgtttgtg tccctgggta cttgagatta gggagtggtg atgactctta 275880
   acgagcatgc tgccttcaag catctgttta acaaagcaca tcttgcaccg cccttaatcc 275940
   attcaaccct gagtggacac agcacatgtt tcagagagca cagggttggg ggtaaggtca 276000
   tagatcaaca ggatcccaag gcagaagaat ttttcttagt acagaacaaa atgaaaagtc 276060
   tcccatgtct acttctttct acacagacac ggcaaccatc cgatttctca atcttttccc 276120
   cacctttcct gcctttctat tccacaaagc cgccattgtc atcctggccc gttctcaatg 276180
   agctgttggg cacacctccc agacggggtg gtggccgggc agaggggctc ctcacttccc 276240
   agtaggggcg gccgggcaga ggcgcccctc acttcccgga tgggggggct ggccgggcgg 276300
   gggactgacc cccccacctc cctcccggac ggggcggctg gccgggcggg gggctgaccc 276360
   ccccacctct ctcccagatg gggcggctgg ccgggcagag gggctcctca cttcccagta 276420
   ggggcggccg ggcagaggcg cccctcacct cccggacggg gcggctggcc gggcgggggg 276480
   ctgacccccc cacctccctc cccgacgggg cggctggccg ggcagagggg ctcctcgctt 276540
   cccagtaggg gcggccgggc agaggcgccc ctcacctccc ggacggggcg gctggccggg 276600
   cggggggctg acccccccac ctccctcccg gatggggtgg ctgccgggcg gagacgctcc 276660
   tcacttccca gacggggcgg ctgctaggcg gaggggctcc tcacttctca gacggggcgg 276720
   ccgggcagag atgctcctca cctcccagac ggggttgcgg ccgggcagag gtgctcctca 276780
   catcccagac ggggcggcgg ggcagaggcg ctccccacat ctcagacgat gggcggccgg 276840
   gcagagacgc tcctcacttc ctagatgtga tggccgcccg gcagaggtgc tcctcacttc 276900
   ctaggtggga tggcggccgg gcggagacgc tcctcacttt ccagactggg cagccaggca 276960
   gaggggctcc tcacatccca gatgatgggt ggccaggcag agatgctcct cacttcccag 277020
   acggggtggc ggccgggcag aggctgcaat ctcggcactt tgggaggcca aggcaggcgg 277080
   ctgggaggtg gaggttgtag cgagccgaga tcacgctact gcactccagc ctgggcacca 277140
   ttgagcactg agtgaacgag actccatctg caatcccggc acctcaggag gccgaggctg 277200
   gtggatcacg tgcggttagg ggctggagac cggcctggcc aacacagcga aaccccgtct 277260
   ccaccaaaac cagtcaggcg tggcggcacg agtctgcaat cgcaggcact cggcaggctg 277320
   agtcaggaga gtcaggcagg gaggttgcag tgagccgaga tggcagcagt acagtccagc 277380
   ttcggctcag catgagaggg agaccgtgga aagagaggga gagggagacc gtggggagag 277440
   ggagagggag agggagaggg agaggaggga gaggagggag aggagggaga gggagaggga 277500
   gagggagagg gagagagcgt ccccaatctt ttaagtctat tttttttttc ttgagacggt 277560
   gtctcgctct gttgcccagg ctggagtgca gtggagcaat ctcggttcac tgcaacctct 277620
   gcctcccggg ttcaagcgat tctcctgtct cagcctcccg aatagctggg actacgggcg 277680
   cgtgccacca tgcccaccta atttttttgt ggttttagta cagatggggt ttcaccatgt 277740
   tggccaggtt gtctcgatct cctgacctgg tgatctgccc accttggtct accaaagtac 277800
   tgggattaca ggcataagcc accatgccca accttttttt tttttttttt tttttgagag 277860
   acagggtctt gctttgtcac ccaggctgga gtgcagtggt gcaataatag ctcactgcag 277920
   ccttgacctt ctggactcaa gcgattctcc tacctcagcc gctcaagtag ctgggactac 277980
   tggcatgcgc caccatacct agctaaattt ttttaatttt tttgtagaga cagggtctcg 278040
   ctatgttgcc caggctgatc ttggactcct ggcctcaggt gatcctcctt cctcagcctc 278100
   tctaagtgct aggatgacag acatgagcca ccacgacgac ctgtttatct ttttgtttgt 278160
   tttgttttgt ttgtttttaa caattatttt ctatttgtaa caagtgatat tgaatttcct 278220
   tccaaggaag aaataaaaag tgtcctttaa aattaaattt attattaaaa aagtaaaaca 278280
   gatatttcaa cgctgatata caaatctagc aaaatccaca aaggtagcat ctgaatggat 278340
   aaagttaaag tttacccaaa atagctctgt tttctttcta ttactttaat ttgtaggaat 278400
   ccctggaagt ccctgctgtg tggatagcac ccttctcgat ttccagcaaa gatacagatg 278460
   ctctcttgtt tttatatttc ttttggtaat ttccgtggaa ttttggaaga aaatttggaa 278520
   tattaaatgt ggaggcgtag aggctcaaat tgtcttctct gctttgaatc acctagccca 278580
   tttttggaag ctgcgtaaga agattcgtga atggagagaa aacattgtga tgaaaatttt 278640
   aaactttggt taggaatgat ctttgagagc gaggagtttt ggggtctttt ttctttccca 278700
   ctgattcaaa tgcttttaga atgtcccttt tctttcctca cactcatctc atcctcttac 278760
   tagatgaaaa aatctttgtg gtcaagaaac aaactgaagc ccccatgttt attattatat 278820
   tagtagtagc tgggcatggt gggatgtgcc tataatctca gctacttggg atgctgaggc 278880
   aggaggattg cttgagccca ggagttcaag actggcctga gcaacatagc aagaccctat 278940
   ctctgccaga aaagaaaaaa aagaaaaaaa aaacagccag gtgtggtggg atgtgctttt 279000
   agtctcagct agttgaaagc tgaggccgga ggattgtttg agcccaggag gtcaaagctg 279060
   cagtgagcta tgattgtgct gctgcactcc agccttggtg acagaacaag gccctgtctc 279120
   taaaataata ataatgacat tagtcacagt aataataatc atgatgatgc cactaattga 279180
   atacttgctg aatgccaagt gctgtgctta tcattttaca tgcgtcatta catttcaatt 279240
   tcacaagaat tctatgaaaa aggaatttgt attccaattt atagatgaga aaaacagagg 279300
   cccagagagg ctaaccagcc tgtctaaggt tatgagatga gatttgcacc cagacatggt 279360
   ttgactccag ggcttgtgcg cttaaccact aagggatgcc gtttctcaaa aaggctgact 279420
   acagcacatt ttgggaaaac ttttgcagag aaaatagcat catgaagtca agtccatggt 279480
   cacctaaatg cagttgagtg ccaggatggc aaagtggcac cctatgcacc ctatgtaggg 279540
   tgaaaggtag aaatgctgga agagcatgac aagagtgtat ttacagtcag gcagtggggg 279600
   ttgcaatttg ggaggatagg gtgagaagat cacttgaggt caggaattca agactagtct 279660
   gagaaacata gccaactccc atctctacaa aaaaattttt ttttgtttaa ttagctgggc 279720
   atggtggcac atgcctatag tccccagcta ctcaggaagc tgaggcagga ggatcgcttg 279780
   aacccaggag ctcgaggctt cagtgagctg tgattgcact attgcactcc agcctgggtg 279840
   acagagtgag accctatctc taaaagcaac aacaacaata acaacaacaa caagaaattt 279900
   acgatgaggg atcttcatag cattctttca gaataagaaa ctacttaatg gacaaaaagt 279960
   aggtaaaaat atacagtaat ttaacgatat aactaagaaa actgtgtaag tgtgtataga 280020
   gagagagtga gaactttgcc tcctatgtac atgtgatata catttttttt ctgctgtcca 280080
   tggaagagtt acaaaaatac atagtgtacc tggtagtgta ccatgcacac tacccccacc 280140
   caacatactg aaaaacaaac caaaacaaac caaaaaaccc tcaacgtata ttttttcaag 280200
   tagagcttta tataggcctc cttttcatat gctgatcaat aaaataatac cgagtcgagt 280260
   catgaaatgt gtcccacccc cttgtctccc ttcaggttta agttactgag ccaggaggaa 280320
   ggcgagtact tcaatgtgcc tgtgccacca gaaggaagtg aggccaatga agaactgcgg 280380
   cagaaatttg aggtgaggtt tcttttcttt ttctcttctt tcttttttct ctttcttttt 280440
   tccttctttc ctcctgctcc ctcctctttc tttctctttc tctcttattc ttttttctct 280500
   ccttccttac ttccttcttc ctctctcttt tctttccttc tttctccttt ctttctttct 280560
   tgcttacttg cttgctttct cgctttcttc ctttctctct tgctttctct tgcttgcttt 280620
   ctcgctttct tcctttctgt cttgctttct ttcttgcttg ctttctttac cccccttttc 280680
   tttgtttttt ccttccttcc ttcctgcctt ccttcctctc tctttctctt tccctctcat 280740
   tctttctttt ctttctctct ctgtccttcc tttgtttctt tctctctcct tccttcttcc 280800
   tttcttcctt ccttcctttc ctcccttcct tctttccctc cttcctccct ccctcccccc 280860
   tttcctttct ttcttcctcc ttcctctctc ttttcattct ttctcccctt tctctctctc 280920
   ttcctctctc ttgtctctct cttctttctt catctccttt ctttcatcag tgggaaccag 280980
   ttaggtttgc tgctgtgctg attttcctgt ttatggcatg gtttctacta agacttttca 281040
   tgactctgag cctttgcaag gttgttttat ctcctagaaa actctgatta atccttcaag 281100
   accccaatta aatgcccttg ccttatgaag ttattcctgc ttccctgtct gcattcaggc 281160
   ttagacattt cttcaaaagt gctcattcac tgcccaccat tttgatggtg acttggactg 281220
   gaatggtagt catggtgaat gaaagaagta gacagagggc cgggtgcggt ggctcacacc 281280
   tgtaattcca gcactttggg aagctgaagc aggcagatca cttgaagtca ggagttggac 281340
   accagcctgg ccaatatggt taaacccagt ctctactaaa aatacaaaaa aattagctag 281400
   gcatggtggt gcacgcctgt aataccagct actcggaagg ctgaggcaca agaatttctc 281460
   tgggtggcag aggctgcagt gagccaagat tgtgccactg catgatcagt aatgctctgt 281520
   tgcccaggct gcagtgcaat ggtgcgatct cggctcactg cagcctgggc aacagagcga 281580
   gacttcatct caaaaaaaaa aaaaataagt agacagagaa gtgatatatt ttggaggctg 281640
   agtcaactag gcttgctgat ggatggaatc tggattggct ctattgacag aatgatgcta 281700
   tatagttaac gaatggaatt gattgtgcta atgacttcat gaaataccct gggggtagat 281760
   cttcccagag aatagtttta tcaaatggaa agcccttggc tgtgaccttg agttacattg 281820
   ctggctctgc tcctacctga aataaatccc tttccttcta cggggacaaa taaacaccat 281880
   tttcagctac ctaagagcag caggtgtata ggacacatcc ttgctttaca aagaaaatgt 281940
   ttgggggaag ataggtttga atacttacat tatatgtgca ttgagtgatt ttagtagttt 282000
   cctgaggaaa agcacattat tttgttccca caaactcaat acactgagtc tggtgggaga 282060
   tgatcaattc tagataaacc atgatggcct actaactccc caaaaatgta tataatagca 282120
   tagctaaaat gaaattaatt tttattattg ttattcatca aataatgaca cacacaaata 282180
   aaaaaaaaac gtgtaaagac catgtaaagt cctgggaaag agaggtcagg taccttgaga 282240
   acatgtacaa gagaaatgtc acgtagtttg agagaatggg gaggtgtcag gaaggaaaca 282300
   gcaatggaag tttaccatgt aaaaagaggg gaaaatgtat tctgttctgc aaacagcatg 282360
   tgcaaaggcc ctgtggttgg gggttatata gcagcaatgt gagactgaaa gaagaccagc 282420
   ctgggtagag cagagaaagc caaagagagt ccaaggaaag gctgaggaag taggcatgag 282480
   tcacagtacg tagggctgtg ctggccttga caaggatttt agcatttatc ccaagagtat 282540
   ttatcccaag gactttagca tttatcccaa gagtatttat cccaaggatt ttagcattta 282600
   tcccaagagc atttatccaa gagcatttat cccaaaagta tttatcccaa tgattttagc 282660
   atttatccaa agagtattta tcccaaggat tttagcattt atcccaagaa tatttatccc 282720
   aaggatttta gcatttatcc caagagcatt tacccaagag cacttatccc aagcgtattt 282780
   atcccaagga cttgagcatt tatccaaaga gtatctatcc caaggatttt agcatttatc 282840
   ccaagagcat ttatccaaga gcatttatcc caaaagtatt tatcccgatg attttagcat 282900
   ttatccgaag agtatttatc ccaaggatag catttatccc aagagcattt acccaagagc 282960
   atttttccca agactattta tcccaaagat tttagcattt atcccaagag catttaccca 283020
   agagcattta tcccaaggat tttgttttgt tttgttttgt ttttttgaga cagagtcttt 283080
   ctctgtcacc caggctggag tgcactggtg agatctcggc tcactgcaag ctccacctcc 283140
   tgggttcacg ccattctcct gcctcagcct cccgagtagc tgggactaca ggtgcccacc 283200
   accacgcctg cctaattttt tgtattttta gtagagatgg ggtttcacca tgttagccag 283260
   gatggtctcg atctcctgac ctcgtgatcc gtctgcctcg gcctcccaag tgctgggatt 283320
   acaggcgtga tatcccaagg attttagcat ttatcccaag agcccagtta ccacacgtgg 283380
   tatatttcag tacgtcagag ttggttgcct ctgtttggtt acggggcttt gagttctagg 283440
   gctctaaatt gcagacatga aaaggcagcc attagtgagt gtattcctgt ttctgagggc 283500
   acatcgtaca cagcaagata aaggacaagt ttgtttgttg aaagaaacaa actatcaagt 283560
   ttgttctccc ctcaacttga tagaaatagc gcttaggggc caggcacggt ggctcatgcc 283620
   tataatccca gcactttggg aggctgaggc aggaggattg cttgagccca ggagttcgag 283680
   accagcctca gcaacatagc gaccctacct ctattattaa aaaatatata tttaaaaaaa 283740
   aataaaaaaa gaaatagcac ttacgtattc ccaaattcct tgtcttgagc ctatgcccac 283800
   ccgacatgct ggaagatagt gatagttctg acccagtggg gtcctccctt cctgggaaca 283860
   agtggaacag acacattggc cttgagctta tcagtcattc tccagtgacc ttgataacag 283920
   aggcttgttc ccaaagtctt ggactcaagc ccaagtgaac tgaaaaaaga accttccctc 283980
   caagtagaac taaaaactgg atactgtttg ataaaaatta agacaggaag gcagtctttc 284040
   cagagaacac tttctgtctc tttcccccaa cttgctgatg aagtggtatc atttgctgct 284100
   gttatagaaa tgtactatta cgggtgaaaa acacaaggcc agtattttct tgtatataac 284160
   caagtctcag ttttgcaaaa tgattttttt ctgtcctggg gcagctgggg tgacatttca 284220
   gaacaccaag gaaaagattt gctacacaaa cagagcattt gtctgggcca gtaattggct 284280
   tggaatagga gactgtttta tccgcatgca agaaattgtg tccctttctt tctagcaaga 284340
   gcctgcaaat gcttgtttat gcctgaatta taatcgctgc atactgtact tgttgattta 284400
   aaaaaatgtt tccaacagac cgaaaattgt tctcaggctc acagaggaat ttcagttgac 284460
   cttgaacatg agaaatgtaa tccctgggcg ataaccaatt agcacccgga atgactcaga 284520
   aattactttc aagcctctta tctggaggaa ttctgagtga cagtaaaatc gagctgctag 284580
   aattggggta atagaatttc atggtagtgt gagcttggcg cagttaccac acgtggtata 284640
   cttcagaaca tcagagctgg ctgtttgctt agggggcttt cagtcgaccg tgggctttgg 284700
   aaaagaagac tccattttgg atggcgcaca gtgggatttc tcagagtctt aaaatttatt 284760
   tgtataacaa agtgcttact gtgtgccatt tatgattcta agtgctttgc aaatattaac 284820
   tcaggtaatt taatccttac aacaacctag aatggtaggt actattatca cgctatttta 284880
   cttttacaaa tgaagaaact gaagcccaca gaggtttaag taactttata aggtcccaga 284940
   gccagtcatt gtcctgggtt tgggttttcc cagaaacaga ctataaggat tcaagtgcaa 285000
   gtactttatt tgagaggtga tcctggaagc accataagtt aataaggaag cattgccaga 285060
   aaaggtggga aaccaggaaa gtaagtatta tcaagcagac tactgctgtg ggcagctagg 285120
   gtttaaccct gctggagaac tctcaagtca gcgcggaatg catgcctcag aatttcccaa 285180
   tcaagggaca ggaagctgag gggttatccg tcggctcccc ttccgttgca ggctaagggc 285240
   tgctcccagg gaaatccact cacccgcact ttcaatgtgc cctgtgcgct gtgcccgctc 285300
   ctataaccag aaaataagct acaggcaaag aatcacaggt gtttgctgtt agcagcccta 285360
   ggtgtagaga gcagcatgct ggtggtgtct tggaggaaca ccagcgattg tacctgtgca 285420
   atgtagccta atggttaagt gtgattgctt gagagccaga ctgcctagat ttgaatccca 285480
   gttctgccac ctccttgctg agtggctgta agcaagttgc tttgtctctc tgagcttctg 285540
   attccctacc tgtaaaatgg ggaattatag cacttcttac ctcatgaggt tatgtgagga 285600
   ttgaatgagg taatgcgttt agcagagtgt ctcacatgtc atacatatcc agatgttaga 285660
   tgctagcatc atggtggttg ttataattat tgtctccccc ttcgggataa ataaagctca 285720
   gaacatcctc tttctgggct ttggaagggc tgcttggagg ttagggaaaa tctacaggga 285780
   ctcctagcct acagaggtcc ctgccccact gctgggtagc tgggagtgcc tgtagtagtt 285840
   ccttgtaggg gtggagtggc agaggaatct gcagttttag ccaggacagg cattcatcat 285900
   cgggagtgga gcccagagtc tggctgccag ggcttctcca ctgtcccctc gtgctgtctg 285960
   gaaggtggct tggctctttg ctcagaaatg ctatttgctt tctctcttag cattgagttc 286020
   tagggctcta aatcgcagac atgaaaaggc aaccattagt gtattcctgt ttctgaaagc 286080
   acatcccaca cagcaagata aaggacgagt ttaatttgtg cctcaatttt aaaatgttcg 286140
   ctctttgatg cagtgtgtgc taggcattgt ccccttggaa caatgggata tttcaaagga 286200
   caaaggacag ggctcctgtc ctctgaaggc tcactgagta ggggccacaa acccaggtgt 286260
   tttcaggagc caggcaaaca acaagaaggc gaaagagctt ggcatcaccc agtgttagca 286320
   gatccccttc ttcaaaagaa gctggggatc tgggttttca tatctccaaa ttttcaacag 286380
   ttggcaacta attaaatatt aaaatgacaa caaactctgt gcagaccaga ccaacagggt 286440
   tatcagactt cttttttttt ttttccaggg ttattagact tctgatctag attctagaag 286500
   ttaaattaac atgcccatga gaaaagttat aaaagcaagc tgggagggag ggaggaaaga 286560
   ggggtgccag ccccagattc agaggagaga agggagtttc tagtgctgag ggatgaggct 286620
   gttagctgct ccttgaagga caaggaaaaa atcaacccct cttcccaccc ccccaaaaaa 286680
   gtccttatct gtccactacg caaagtcaaa tgaagaaatc aggaagtttg aggtccgtgc 286740
   tcacgtttca tggggagtgt gtgctgggac ccgcaggtgg atgggaaagt tcccaggatg 286800
   ggaaatggga actggggcag gcaggacttg gatcttcaca gtctggagaa tggagactgt 286860
   gaatcagatg ggtcctgagc caggattcaa agaaggcagg acacatggtg ctaaggtgat 286920
   ggggcagcag ggaggggagt agggaaagtg tttgggtcag gccagcttgg gggtgatgat 286980
   ttaggagggt gtgaggacat agcccgagaa aggtgagacg gcttctgtcc taccctggct 287040
   cttttgtttg cagtttccat tgcaaataag atgaccttgt agttttaact accccctcgt 287100
   tgcctctaca ccccaaatct ctgtctccag tccagatatc tttgctcaag ccccaaaccc 287160
   atgtatacaa ctgcttactg ggtatttaca cttgggttaa acctcaacat atcttactgc 287220
   aaagtaaact gaccaagtat attcacagca gcgttgttag taggccgaaa ggggaaacaa 287280
   ctcaaatgcc tgtacacagg agacgggata aacatcaaca atgagtgcca ttcagcaata 287340
   caaagagaca aattaccgat ttatgcaaaa acatggatgc atttcaagaa cattttgatg 287400
   agtgaaagaa gccaggcaca agagtacaca ctctacaatg ctgttcagat gaaattcaag 287460
   aacagacaaa actgacctat gggatagaaa ctggaacaga gattgcctta gtggcggggg 287520
   ttgcgggggg cgtcgactgg gaacttttgg agggtgatgg aaatgttccg cgtcttgttt 287580
   ggggtttggg ttacatgggt gtgcacattt gtcaaaatct acagaactgt gcacgtgaga 287640
   ttggtgcact ttgcggtgcc cacctcaagt ttcaaaacat gaataaagag aaaatggcgg 287700
   atgctttgtt agccatggtt cctatatggt aacagtggaa tatgcacccc atgagggaga 287760
   aataaacctt gaagttttaa gctgctgtgg ggaagagggg atctcaacat gcctgaaatc 287820
   acaccggtca tctctgccca ccctcttccc cacacttcca aacttgctcc tcctccgctt 287880
   cttctcatct cagtggaaac aaccagcatc ctgccagttg gtcagaacag aggctgggga 287940
   gctgtccttc atctccaatc tcccttctcc cttgcccaat ctgctaccca tctggggtca 288000
   gtctgtctcc caaatagatc cactttgttc agtctacact gcagacaact gagtccatca 288060
   tttttttttc ttttaagaca gggtctccct ttatcactca ggctggagtg cagtggtgca 288120
   atcatggctc actgcaacct ccaactcctg ggctcagggg atcctcccac ctcagcctcc 288180
   cgcgtagctg ggactacagg cacacaccac catgcctggc tcatcatttc tctccttgat 288240
   aatgcagtga cttcctagtg atcactcagc ccacgtgctg gggggtctcc tctccactca 288300
   gagtgacctg ttaaatatgt atggtcatac catagccctg cttcaaacct ttcaaaggct 288360
   gtctgtcatg ctcatagtca gcctgaattt cttaaggtcc tttcaacact ttgtctgcct 288420
   cctgtgccat atctgccttg catttcatga tccagccaga cgatgtttca ttccttgaat 288480
   gtgtcttccc ctctgttcca taaatgctgt tcctgctgcc aataccagtt tatctacttc 288540
   agcaacccca acataccttt tggacacagt atggaggacc caatcccagc tagaatgcac 288600
   gttttttcag agtatctgta tccattttgc cctctgatgt atcctcagca cctagaggag 288660
   cacttagcat gtagcaggta ctcaacaaga atttttgatt tgtttgttgg tttgtttatg 288720
   acagagtctt gctctgttgc ccagactgga gtgcaataca gtgatcagag cttactgcag 288780
   cttccaactc ctgggcttaa gggattctcc tgccttaagc ctcccaagta gctgggacta 288840
   caggcccact gccaccatgt ccagctaatt aaactttttt tttgagatag ggtctggctg 288900
   tgttgcccag gctggtcttg aactcctggc ctcaagcagt cctgcctcag ccttccaaag 288960
   ctttgtgatt acaggcatca gccaccacat ccagcctcaa taaatgtttt ttgaatgaat 289020
   gaatgaatct tccctgatat tcaaaacttt ggaaagattt attttgcctc tttatcttca 289080
   aagcaaccaa ataatgcaac aataataata gctaccattc actgagcatg tgttgtgtgc 289140
   caggaatgat actaaggact ctgtgataca tcatttcatg tcatcatctc catgtcttga 289200
   aaaggtgatg aatgtttagg aagcttcaat caccaaatgc aaattaagtg ccttctctgt 289260
   gccatactcc tctaggtacc agggaaacag tggaaagcaa aacagacaaa atccttgcca 289320
   ttggggagct tacattctgg tataaggaga aagatgataa attgggaaac aagtaaatat 289380
   atggtttgta agatggtgac aagtgtttta gagaaaatca agtaagtaag gcggatggag 289440
   attgctgaga gcaattttat gtagggtgtt cagggaagag ccctcttatc agaggtgaga 289500
   gagttctggg gaaagagtgc tctgagcaga gggaacagca ggtgcaaagg ccctggggta 289560
   agagcatgtt tggtgcattc agagagcagc aaggaggcca gtgcggaagt agaaaggagg 289620
   ctgaaatcag aggggctcag tcatctgtgg cctttggctt ttactttgag aataatggga 289680
   tggtatatat attgggcaga ggaatgttat gacttaactg atgtattctg ggccacacag 289740
   ctgattcatg tcagaggatt ctaccacgag gctttttttt tttttttttt tagtgagaga 289800
   gagatgggat ctcactctgt cacccaggct ggtgggcagt ggtgtgatta tagctcacag 289860
   cagcctctaa ctcctgggct aaagcagtcc tcccaccttg gcctcccagg tagctgggat 289920
   gacaggtgca caccaccttg cctggctaac tttctttatt tttgtagaga cagggtcttg 289980
   ttatgttgcc caggctgttc tcaaacttct atcctcaatc gaatctcctg tctcagcatc 290040
   aaaaagtgtt ggaattgcag gtgtgagcca ctgtgcccag ccacacatga gcttttaact 290100
   ccacagtttt ctctctgcta aacgtgaatc cagtaacgaa gtcctggcag ctgcaggtct 290160
   cccactcatg gcttccagtt tttgcctcat tccaaagcac ctgctgccac tctgagcccc 290220
   attccacagc tcatggcaag gagtccctct cccattgcac tgaggacaca cgtggccctc 290280
   actcatccat ttatgcaaca aatatttagt tgggaactat actagttgtc aggaaaacca 290340
   tggagaatga gaaatattcc gtgtccacaa cagtctccaa tttattggaa ggaaacaagt 290400
   gaacaggcag ttatcctcca gtatttatgg gaagagaagt ggatgaagga agaaaatcag 290460
   aggagaacct agtgcagaca gagagaatcg atgagggctt ccctgagcca tgaaaatgtg 290520
   aaccccaagc tgggttgctg aaggggagag aatagccatt tgtaccacag tagcagcagc 290580
   aggtgcaaag gccctgtggt tggaggggcc tgggacatcc aggggagtga aaggagaatg 290640
   aagctacatt gcagagaaga agcaccctag caatgatgga gctggaaagc caggtagggc 290700
   ctaaacacac aagtccatga aagcacctcg atctttaccc tgaaaatgat gggatgccac 290760
   tgagacattt taagcaggag gggacttggt tggatgagag gctcactgtg gttgtgtgtg 290820
   aaacccagtg taagggaaat cactgtgcct tgtgcaggtg agagatgatg gcaggtagtg 290880
   atggttgaga tggaaagaag tagatgaatt ctagagacag ctgggaggaa agaaatgaca 290940
   gttctggtta tggttgagac atgagggata aaagaaagat ggagttgcaa aggatgtccg 291000
   acaggtttca ggcttgtgga gtgatgcatg gctatgcttt tcatggggac agggtgccgg 291060
   agctgcagga actacagctt cccattgtgt gcctgtgcct tcctacaaga tcgtcctcaa 291120
   accctgagca tgttttctgt gtgcttcctt tttgcagagg gccaagatca gtcagggaac 291180
   caaggtcccg gaagaaaaga cgaccaacac tgtctccaaa tttgacaaca atggcaacag 291240
   agaccggatg aaactgaccg attttaactt cctaatggtg ctggggaaag gcagctttgg 291300
   caaggtatgg tatgatttgg tggctccacc tgctttggaa ggaacatcta acaacacagg 291360
   cacatttgct gttcctatgg gacggacgtc ctagtgggag agagagtaag aagtaaatag 291420
   agccacacta cactttgtaa tgaataccat gaaggaaatg aatggagtgc tgtgtcacaa 291480
   acaaaaaggc gaccctttca gtctgaggag gtgacacata tactgagacc tggatgatga 291540
   gaaagaacta gctatgcttt caaagagcta ggaaagaatg ttctcagaag agagcaagtg 291600
   caaaagttcc aaggtgggga caggctgggt gcatcctagg aaccatcaga aggtgttggg 291660
   gcaaaatgtc actagcagaa ggaggtatag gacgtgatgt tgcagagata agcagggccc 291720
   aaggatgcaa accttataag ctacaatgag gagtttgggt ttcactcaca gtgcagcggg 291780
   gagccattga acctttttaa gcagattagt gacacgatcg gatttgcctt ttggaaaaga 291840
   tcactctgac agccaagaat ggaaacggtt cctttcaaac agttgcacag tggaaggatg 291900
   accctgggac tcatacagtg tttctggacg cttttataga tactgcgaga tgctgtttta 291960
   ctgaagttga gattgtgtta taagtatttc tgactcattt agtgttgttt tggggttttt 292020
   agtctctttg cgtgccacaa agatgtctag cataaatctc ataccatgaa ctcaggtgtg 292080
   caaaaaatat aaatttcttt tccatctatg taacgtaagc caaaaacctg ggagttatcc 292140
   ctgagcttcc ctctttcctg tattcccact gttcctggcc accgcccccc acagccatgt 292200
   acaatgcatt ggcgaattct gacagttcct tcaaagtata tcatgtattc actaacttct 292260
   cttcatctct ctaataccac ccaaatctaa tcattaatat cacctggata actgcagaaa 292320
   ctcctcagtg gtttctcaat cccaatcttg ttacctataa ttaatcccaa tcccaatctt 292380
   ttttacctat aattaatcgt tacctataat taattgcaat cccaatcttg ttacctataa 292440
   ttaatcgtta cctataatta attgcaatcc caatcttgtt acctataatt aatttacttt 292500
   ctacttcgta gccgtaataa ttttttaaag cttacattag gtcaaggggc tcccctgctt 292560
   aagaccctcc aatgactttc catccaatta gaagcaattc tatgttacat ccttagcttg 292620
   tatcaccctt tatcatctgg cccttgttct ctgtgtgact tcctttcttc tttctcctcc 292680
   ccatcaccca ctgtgctctg gccacaacat cagctcttgg gtcactgaaa cacacaaagc 292740
   tccttcctcc cttagaggct ttgtgattca gctccttttg ctgtttgcaa ggccggctgc 292800
   ttcctgcaat tcacatctca acttagacgc cacttcttct gaaaaatctt ccccaactac 292860
   ccactgaaaa gttgccgctc attcactgtc cattatgttt aactctctca tggcacccat 292920
   cactgagcaa tgcttttctt gtgtccttgt tgattttttc tctcttccct ttctaaggtc 292980
   taagctccac aagagtaggg atcttctccg tcgtggtcgc cactgtgatt tcagagccta 293040
   gggatggaat tcattcagtg cccagattga atgcgtggat tctctctgtc tgtgggtggc 293100
   aagggcatcc actgagatgg actaagggat ctgaaatgat ggctcaaagt ctgaagagct 293160
   tcagcatggg atgggaatag agagtaaggg cagccagtaa gatggcccat ccagtcccta 293220
   aggttgggaa taggcagttg gcagtggtcc acaacgttgt atggttttta ccagcagctc 293280
   tgagtatccc tggggtggag atggagaagg agatggtgga tttgaactaa gcgtgggatt 293340
   agtagaatca gtgaactaga agattatgga agaaagagag ttaaggattc tgggaagaga 293400
   gatgtccact gcagatccac agagcctgta ccaagtgctc aagaaaagca tgttgaatac 293460
   atgaatgaaa atggtatcta gattgggttg gggaagatgt ctagcataaa gagggtcatc 293520
   acctgggtgg gaaaaaggga gtttagaagt ctaaggatga aaaaaaaata gatgaggtca 293580
   agatacgtaa aatgttagga agaattgagg cattgagatt tcaaacatgg agcagtttct 293640
   ctgatgatga ggaagaaagt ggcctaaata aagaagaggt tctaataagc tgagttttta 293700
   caaattaaat gagcactttt tttttctttt tgagatagag tctcactctc ttgtccaggc 293760
   tggaatgtag tggtgcatta cggttcactg cagcctcgac atgctggact caagcagtcc 293820
   agctcctacc caagttcaca tctttctctg acccccaaat aactgcatgc accaccatgc 293880
   ctggctaatt tttgtttttg tttttttgaa tcagaatctt actctgttgc ccaggctgga 293940
   gtgcaatggc gccatctcgg ctcactgcaa cctctacctc cagggttcaa gtgattctcc 294000
   tgcctcagtc tcgcgctcgg ctaatttttg tagtttttaa tagagacagg gtttcaccat 294060
   gttgaccagg ctggtcttga actcctgacc tcatgtgatc cgttcgcctc ggcctcctaa 294120
   agtgctggga ttacagacat gagccaccgc acctgaccta atttttgtat ttttttgtag 294180
   agacatggtc tcactatgtt tcccaggctg ctcaagtact tataagctca agcaatcatc 294240
   ctgccgtggc ctcagaaagt gctaggatta caggtgtgag ccaccgcacc tgtcctaaat 294300
   tatatatata tatgctttct tttttccttt tttttttttt tttttgagac ggtgtttctc 294360
   tcttgttgcc caggctggag tgcaatggca caatctcagc tcaccgcaac ctccgcctcc 294420
   caggttcaag tgattctcct gcctcagcct tttcgagtgg ctggcattac aggcacgcgc 294480
   caccacgccc agctaatttt ccacttttag tagagacggg gtttctccat gttggtcagg 294540
   ctggtcttga actcctgacc tcaggtgatc tgcctgcctc agcctcccaa agtgctggga 294600
   ttacaggtgt gagccaccac gcccggccat gatattctta ataagcaatg cattcacatg 294660
   attcatcatt aagattatat taaaaggata aacactgaga agtcctgctc ctacccaagt 294720
   tcacgtcttt ctctgaccac ttactgctac tgctttccct atgtgaacac ttttattcag 294780
   ttaatttgcg tctttccagt ccccctgagc taatttaaca ttttttattc ttgtccttat 294840
   taactcaaaa gttcatgtac tacgcctgtt tttgatcact taacaaaaca gacgagagga 294900
   tgtttgatgg aggattggaa gttctggaga gtcccatgga aaggattggg gttggggggt 294960
   agtgggtgga gtcgtaggag aatgaatcac tggggagcaa tgagagaata gatcatatgt 295020
   ctccatggaa actgttataa taattgagaa ctctgttcca gactggagat atagcatcag 295080
   ataaatcata gatgaaaaca acagtaagtc atgaaagcaa agtagaataa caaacacctc 295140
   tatgatcctt taaaacagag taatcctttg caactctagt gttgccaact agggttgtaa 295200
   attaatgcag gttttatatt tgaattccaa ctttgaacat tttgcagtct ggttaggtat 295260
   atagatcaat ctccataaac agaagaacac agctgcagtg atttgctctt ccccttcgaa 295320
   agatgaaagt gatggggctt tggggtaatc tgtttccacc tcccttaacc caggaaaaca 295380
   aaacaaaaca aaacaaaaca tgtttttaaa ccctaatggc caggtgcggt ggtttgcgcc 295440
   tgtaatccca gcactttggg aggccaaggt gggcagatca caaagtcagg agttcgaggc 295500
   cagcctggcc aatatggtga aaccctgtct ctactaaaaa tacaaaaatt agccgggcat 295560
   ggtggcaggc gcctgtattc ccagctactc aggaggctga ggcaggagaa tcgcttgaac 295620
   cgaggaggcg gaggttgcag tgagccgaga tcgtgccact gcactccagc ctgggtgaca 295680
   gagcaagaca ctgtctcaaa caacaacaac aacaacagcc ccactaataa tttgtggggc 295740
   aactagggaa tggcacaagc cttttggtaa gccctgtaaa aatgaaacaa ggcaggaagc 295800
   aatggtgaag gacattgaat ttcaaatgtg taagcccgtt tcaaaatctt tcaaaagaag 295860
   aaaactgtgg agagtttgtt tttttaaaca ggcagaagtg tttacctgga agggctatct 295920
   taccaaagca gggcctgttg gcatggctgg cttactcgga ggtagatgcc ttctaggact 295980
   tttaatacag agcattcaaa cgtgtcattc acgtttaaaa attattttat ttctcaactt 296040
   ttattttaga tacagagggt acatgcgcag gtttgtcaca tgggtatatt gtacccaggt 296100
   agtgagcata gtacccaata ggtggttttt catttagtgc ttaatggtgc ttagaatttt 296160
   atccttaact gtctgttttt ataatgcctt gaaactttga gttttgccaa cgtctttcaa 296220
   gaattgaact ttgaatgatt gctggagtca aaattatgct gcatatatat aaattgggca 296280
   gatttgtacc gtccggattg atgatacaag cttaccttat aaagtaaata cagcaaatac 296340
   ttgatcaagt atatacacag cacaaggata actctttata ttataaacgt gattcctcta 296400
   accctaatgt gctgccataa taaacatgca ttaaatattt aatgaatgct aatactgtgt 296460
   gtcttctgag atttcatatg tgctcatggt aatgatttgt atgatccacg tctttaaaaa 296520
   atagtatgca ggaaagtttg aatggaggct ttcttgttct cttatacccc tgactcttgc 296580
   ctatggatgg aactgagcta atgtacgtga gttgagatgg gatcaggggg ttagaaagga 296640
   gctgggggag gcagctggtg tgctatagtg gacatagatc tataaccata aagaagctga 296700
   aagaagcttc agcacagaag atctatgtaa aaatggtatt tactggaaaa cagaaatagc 296760
   ccaaatggta tattcataat aaatacaaaa aaaaacgaag tgactctctc ctctctcttt 296820
   ctgtccctct ctcatgcacg tgcatctatc tatctatcta tctatctatc catctgtcta 296880
   tctaatctat catctatcta cctatcatct ttctgttatc tatctaccca tcatctatct 296940
   atcatctatc atctatatct atcatctgtt tatcatctat catctattta cctattatct 297000
   gtctatctta ttatctatca tctgtctacg tatcatcaat caatcaatca tctatctgtc 297060
   gtcatagaaa gattttcata acatatcaaa taaaaataaa agcaaattat ggaacattat 297120
   gattggcaat ataaaatgtg tatagaccaa ttccttttat gtaatatact catggtccct 297180
   gctttcatta aactttttct tcaacatttc attatgaaaa tttaaaaaat ggaaaagttg 297240
   aaatgattgc ataatgaaca tctataacac ccaccacctg gattccacaa gtaacatttg 297300
   actgtatttg ctttatcatg tatccatctg tctatccgtc catcaacgct tttttaatgc 297360
   atttcaaagc aagttgcaga catccataca ctttacattt gcagataact gcaaatgcac 297420
   tttacattca gcatgtatac catttaacca gaattcaata tttgcaatga ttattttaaa 297480
   ggtaaaatta cacacactga aatgcagcta tcttaagtat atcattctat gagttttgaa 297540
   aaacgcatag tttttaaaat gcagtggagg tatacacatt catttaaaaa atcacacagg 297600
   catgaaagca actgtgatgt ttagtatgtt ccatgggcac ctgcgggagt gggatttgcc 297660
   ctggttgaag ccagagaagt cttctgtgag aaagtggctc gagctatagc taaaggacga 297720
   gttaagttaa ctatgcaaag aggggaaata gtaaatgaaa atctcagaat gtctctttgt 297780
   tcctggcccc cacccccaaa ataggcttgg ttaatctgtg tggctgacag ggcacctaaa 297840
   cttttattag ggtagattca tctgctgtaa caaatagacc cccaaatata tactggctca 297900
   gacacaatag aagtttaaaa ttttcattaa acttctaagt ttaagacgtt taagacaggc 297960
   gcgcctggcc acacccactc tctccatatg gtgattgggg acaaggtgtc tttcgtcctg 298020
   tatggctcca ttaatcccta gagctttgtt tttctctgca gtcagagggt ggaggaggca 298080
   caagctgctt cttaaaagtc tcagcttggg aagtggcacc aatcatttct gcctactctc 298140
   tattagagaa aatatagcca cgtggccaca cttcagtgca aaggaggccg ggagaacaat 298200
   ttgggtggat gacacgtcgt ctcagccaca ttccctactt ttcagtcctg gctgcaattt 298260
   ttcacggtga cacacaaaag accttctgac ttttcccttt agagctcttg atgtatcaca 298320
   ctccacacac aaaaaacttc aagtgcctct tttctgtgac atttctttgt gttcaaatca 298380
   tggagcacgc caggtgttag tggtgacttt cttgaagcta gcacgagcca ggaataagtc 298440
   aagtaggaag aagtcagcag cttatgaaat taaaaagaaa aagtgaagtt actatagaaa 298500
   ctaggtgccc aggtatcaca ttttttctct tttaaaaatc ccatccagct ctgttgctgt 298560
   ctatgaatct aagatgatct aaaatcagaa gcagagaggt gagagacaga accattaagc 298620
   ttccccatct agtgcttgac ttccctaaaa taatcccagg acactgaacc aatgacatta 298680
   aatgcctggg tgtatcacta aggaggctgt ggagggtctg gtggtggccc cttccactag 298740
   ggtaccccta gaaacttggc atttgtgtga cactctccaa gatgctggca tcaacttttg 298800
   tagctcaaaa ttcagaccgt acaacagagt tgactatagt cttggccaag ttttccaaat 298860
   attagtcatt tatgcacctg ttatgggttt ttccttgcca ttacatgcct cctttattcg 298920
   atcatatttt tctttaaagt gactttaact tgacttcctt ttcttcttct taacattgcc 298980
   ctaagcaata atatatgtga aatcacaagt ttgatgtacc agttatacat ttattttaat 299040
   gtgtttggtt tgatatacca gttatatatt tattttaatg tgttttagga aaatatatta 299100
   aaagaagagt aattttgttc ttgtactagt acctgaacca catttggtga aaaactgtca 299160
   taaaatctta cacttacaca atgtaagatt gaggtgattt ggcagctcga ttaccatggt 299220
   atttctaaga atgtaaagtc ttttgttaca attcgtgtgc gtacctggaa gttacaaaaa 299280
   gtctctgttc atccccctcc tccatatcct ttccttcaaa taaggaatat gcgcaacact 299340
   gcctgtatcc ttaaaaatct ttcgtgtgat cttgacctcc ctctaccctc ctttactcac 299400
   taaatatatc cagacagcaa agctactctt tggagatttt ctaccttcat ttcctcccca 299460
   cccactcctt ccttatcctt ttgtattatg tctcctcccc ttagcaactt tcagaaacat 299520
   ctgtctccca ggttatcagc agtcttgtaa ctcatatctc tccagatctg agcatgaggc 299580
   atcctggctt gctcagggga gtagggcagg catgagggga agggaactgg aaataagatt 299640
   ggaatccata cattattctg ggcagaagtc atcaccttta cagtaacttc ccactgaaag 299700
   gcataactaa ggattcattc attcaagaag tatttattga gcatctacta tatgccaggt 299760
   acccttgtag gtacagaggc aatgtctatg aacaaaacaa acaaatatat ctgcctgccc 299820
   tcatgaaact tatattctag tggtagtgat ggtgacgacg aagatgatga taacacctaa 299880
   catttttggg gtccttacta tgtgccaggc tctgttataa gtgcttagta tgtattcatt 299940
   agctcacact tactctatga tgcagctact gttgtcccca ttttacagat gaggagagtg 300000
   agatgcagaa agttggaata acttgctgaa gtcacacagc tagtaaatgg cattaaacag 300060
   atcagctttg atttagatgt gtatgatttg gggctttttt tttttttttt tttttcttct 300120
   agagacaggg tctcactctg tcacccaggc tggagggcag tggtgcaatc atagctcact 300180
   gcagccacga actccttggc tcaagtgatc tgcctgcctc agcctcccaa agtgctggca 300240
   ttacaggcat gagccactgt gaccagctga ttttgggatc ttgattgctt gaaacttaac 300300
   atgtaatttc ctagttttaa cagcgattcc gtggaatgtc acaaacatat tctaggaggt 300360
   tagtcatttt tccctcttat tttctcatct ttccctctaa gtgctaagca gagtttatgg 300420
   ccaggtgcag tggctcacac ctttaatctc agtgctttgg gaggccaagg caggaggatc 300480
   acttgaaccc aggagatgga gcaacaaagg gagaccccat ctctataaaa gcaaaaaaca 300540
   aaaaaaaatt gcatgcctta ttttcagtgt aagttgctgc cattagacaa accacatact 300600
   cctagtgatg gagggctctg ggttttctgt tcatcctcat cactctgtgc ttattggcta 300660
   cctgtccatg tgtaaataac acatgtgccc tgtgctcatt ggaaccgtga gtcatccgca 300720
   gggattccta agagggccca tgagaatggc tttcactcct ccagcctctg cccttctgac 300780
   ctcctccaca ggcttcagcc ttatgggagt ccccaagtct tggaagcatc tctagtgagt 300840
   gcagtgactc cttagctggt tccttttcaa ccttcaacct ccattttctt aacccttacc 300900
   aactctactg cccagctgca cccactccaa ttccactcac accctgccca ttatcgccaa 300960
   cgtttctgag ctcatgctca ttgacaagga ccatgccatg gatttgcatg cattatatca 301020
   tattaattca caccataatc ctgcaaggta gtcactgata atcactctat tatatcatat 301080
   agaaattgta aggcagagag gttaagtgac ttctccaaga aggaggcggg accttgaaag 301140
   ctggttttgt gatttcagtt gcctcttgcc atctcttctc cactttgggg tccacacatc 301200
   ttcccaaacc cctagtttaa attttttttt tttttttgac aagagtctca ctctgtcacc 301260
   caggctggag tgcagtgacg tgaccatggt tcgctgcagc ttcaacctcc caggctcaag 301320
   caatcctccc atctcagtct cccaagtagc tgggactaca ggcacatgcc accacaccca 301380
   gctaattttt ttattgtttt tgtagagaca gggtcccact ttgttgccca ggctggtctc 301440
   gaactcctgg gctcagggaa tcctcctgcc ttgacctctc aaagtgctag gattatagga 301500
   gtgaaccacc atgcccagtc caaaccacta ctctttcctc ttcctttttt cctttctttc 301560
   ttccttcctt ccctttctct ctttttgact tttttgaaac agcagttaca aaacgattac 301620
   aagagctacc taatgaggcc aggtgtggtg gctcatggct ataatcccag agcctctaga 301680
   ggccaagcct ggaggattgc ttgagctcag gagttcaaga ccagcccagc ctgagcaaca 301740
   tagcaagacc tgatctctaa aaatgtgttt aaaaattagc cagacacagc tggatgcagt 301800
   gggtcatacc tgtaattgca gcactttggg aggctgaggc aggcagatca cgaggtcagg 301860
   agtttgagac cagcctggcc aacatagtga aaccccatct ctactaaaaa tacaaataaa 301920
   aattagccgg gcgtggtggc gcatgcctgt aatcccagct actcaagagg ctgaggcagg 301980
   agaatcacct gaacctggga gtcagaggtt gcagtgagtc gagatcatgt cactgcactc 302040
   cagcctgggc gacagagcga gactccgtct caaaggaaaa aaaaaaaagc cagacacact 302100
   ggcacacagt tgtagtccta gctactctgg aggctggggc aggaagagag gttgagccca 302160
   ggagttcgag gctgaagtgg ctatgattac accattgcac tccagcctgg acagcagagg 302220
   gacaccctgt ctcttatata tttagaaagc tgcataatga catttgtggg tttctccttc 302280
   ttccatgaaa aaaaaagcaa aaaatggtat ctcatgactg cattggtatg aagacaaata 302340
   tattaatatt acatattaaa acatgtactt ccacctaggt tcactttttt ccttgtgatt 302400
   taaaaaaaaa aattaaagtg ccttatgaac cttgaaaaca gcatgggccc tgagcactgt 302460
   gcctggtggg ttttgatgga gaagtcagcc ctatgttcca gctccagcat ctcaatagca 302520
   cctcccctcg cagcctccaa acttttcttc ccaaaggcca gggtgcttaa ctaccatgct 302580
   ctgtgagcat gagagagcat tccagagttc caggccccag acctcagtgt cccttttttt 302640
   tttttttttt tttccttttt gagacagagt cttgctctgt tgcccaggct ggagtgcagt 302700
   ggggtgatct tgagtcactg cacctcccag gttcaagcaa ttctcctgcc tcagcctccc 302760
   gagtggctgg gattacaaac gcctgccacc acgcccaagt aatttttatg tttttaatag 302820
   agacagggtt tcaccatgtt ggccaggctg gtctcgaact cctggcctca agtgatccgc 302880
   ctgcctcagc ctcccaaagt gatgggatta caggcttgag ccaccacacc tggcctcaca 302940
   gcatcatctg ctggagccaa gcccggcacc agattttaac aggaagaaat tgcctgagga 303000
   gattaaaagg cagcttagtt catgccagga acaaaggcct tctctaaagt ctgagcttct 303060
   gttgtgggag tctctactaa ttttaatctc attagtggta tgtattcaca cacagacata 303120
   tacatacata cattagatgt ggttatataa tcctacagac aaaagcactt ccctgtactt 303180
   cagcctgctt tgctatcttc ctgcaccata tggtgagttc ctttatcata tggttcacag 303240
   atagctcaaa gtgtttgcta cctgtttcct taatcctctt ccagagaacc tttttgacat 303300
   ttgctgtctg atcttggatt cctgccttct ctttggttct atcgcaatta tgtcacttcc 303360
   ctgcccccac caagccttca acctttcctt ctttcatatg caattctgat tgcagcgtgt 303420
   tttttttttt ttttttgcca actcatcttc ccagatcctc gtgaggccct tcttccacct 303480
   tcccgaattt tccaggtctc tcctcaatga agtgacttac tgctcttccg cagcctctgc 303540
   cttcaattct cagaaggtag tatgttacaa gacaagataa ggttcatttt tctcaagaaa 303600
   ataaattata tcaaggaaaa tgaaatgtca ggaatttaag ttttacaacc ttccatctgc 303660
   tgcaagtaga ttgtcaagcc agagtgagta aagcgggtat ttgctgagaa gacatggctt 303720
   gtttctgtgt atctcaagac acccagaggc aagaagtgcc acctgaatga gagagcttcc 303780
   agcagtctga taaccatggc tccatctggg gggttgtagg ggggccccca gcctgtcatc 303840
   cctgcttctc gatgacatct ctctgcagat acgcatcagc accacctctc tcagccttgt 303900
   tgatgcttct caagtctcca tgtctaattc tcacatgtag aatatcctag gaatctaaaa 303960
   tcccgggaaa gaaaaactga tgggccagtg tggaccaggg atccctagga tccaattcac 304020
   tgtgaccggg gaagcggtta cttttccaac catccatcag gaaagatgac aagaaaagca 304080
   aataggatgt gggcagtttt cctatgagaa agaagaaaac gacgggcctg tctggcacac 304140
   aggccaaagt gggagtttcc tttaaatgtg tattattttt tattttaatt tttttatttt 304200
   gtttatttat ttatttttga gatggagtct cactctgtcg cccaggctgg attgcagtag 304260
   catgatctcg gctcactgca acctccacct cctgggttca agcgattctc cttcctcagc 304320
   ttcccgagta gctgggatta tatgcgcatg ccaccatgcc cggctaattt tttttttttt 304380
   tttgtatttt tagtagagac ggggtttcac catgttggcc aggctagtct tgaactcctg 304440
   acctcaaatg atccatctgc cttggcctcc caaagtgctg ggattacagg tgtgagccac 304500
   tgtgcctggc cttttatttt aattttttag agacagcatc tggctttgtt gcctaggctg 304560
   gagtgcagtg gcatgatctt agctcactgc agccccgaac tcctgggctc aagcgatcct 304620
   cctgcctcag cctcccgact agctgggact acaagtgcat gccatcgtgc ctggctcatt 304680
   tttgtttttt ttctagagat gggatctcat tatgtagacc aggctggtct taaacttctg 304740
   gccaccagcg atcctcctgc ctcagcctcc caaagcagtg ggattacaag tgtgagtcac 304800
   gtcgctgggc cctgtgtttt ctctaaaatg ttgcctgatt ctttgggaaa aatatcgcaa 304860
   agataaatca agtcctcttg tagaaggacc agatagagcc cattaacatg gaaactctgt 304920
   ctcaggtgat cattcatggc tactggtcca attattgtgc tatcatggta cctgccatcc 304980
   tctgcatatc catctcaact ttatctctat gtattatttc agaaatttac agtcctggta 305040
   aaatgaatcc ctggttacta acagcataac ccataatctt ttctataata caacaaacat 305100
   aatggcagaa atttgtggat tgtaatcatc caacacccat gataaatatt ttggatgtac 305160
   aaatgtggat ggtacctatg attattcatg ccgtagttgt ttcaaacccc caccccaccc 305220
   cacagtttca ccttatgtac tgacctcatt gaacaccact gcggagattg ataacatttt 305280
   tggagcactg ggaactcatc tgagtgtttt ataggcattg ctcatttaat cttcaccatg 305340
   ggtcttgtga agtgggtgct gttattatct gcatttttaa atctgtagat aaagagacag 305400
   agtctctatt ttgcagatga gggaatagag gctcagagag gtgagaagct tgcagtcctg 305460
   aagcggtgga ggcaggttca aaccagggag cctggctgca gaatgtgttg ccactcagct 305520
   ccatagtccc cttgcatcca gtgcccccta ctcctgctgt gctgaaaccc gttctctatt 305580
   agcagtgctc tgtaactcca acaacaaagg ggggcagtgc ttacaaggca gatcagatta 305640
   taaacaagct ttcttcctgc cacctaattg ttgccatgaa tgacaggact ttaagggttt 305700
   catttcattc tctttcagta gaagccatca attattagtt tattttccca gcaaccattt 305760
   aaaaaagagt caaattaaat ctgcttttat tttaactatc cagctttgtc aaatataaca 305820
   taggattcaa agaaatttga ctaagccata tatgtcacca atgtttattt tatttatgta 305880
   tttttttttg gcatttctgt tcttttgtga gtgtgagtgg tgagaacact tgggatctcc 305940
   tcccttagca tatttcaagt atataacaca gtattattaa ctgaattacc atgccatacc 306000
   ttttaaattg ctagtactta tttatcttgt aactgaaagt ttgtaccctt taatcaacat 306060
   ctccccattt tctctactcc cagcccctag caaccaccat tctgctctct gtttctttaa 306120
   gtttgactat tttagattcc acatatgtga gatcatacag tatttgtctt tttttttttt 306180
   tttttttttt tttgagacac agtcttgctc tgtctcccag gctagagtgc aatggcacaa 306240
   tctcggctca ctgcaacctc acctcccagg ctcaagcgat tcttctgcct cagcctcctg 306300
   agtagctgag attacaggca tgcaccacta tgcccggcta aattttatat ttttagtaga 306360
   gacagggttt caccatgttg accaggctgg tctcaaaccc ctgacctgag gtgatccacc 306420
   tgtctcagcc tccccaagtg ctgggattac aggcgtgagc caccgtgcct ggccccagta 306480
   tttgtctttt ggtgtctggc ttattttgct taggataagg ataaggtgct ccaagttcat 306540
   gaacatttat ttcagctcaa aagaaccgtt cagttactgg aattgatcaa tatcaattct 306600
   attggtaaaa atcaattcta ctggtatcaa caattagacg acacagccca gagtgtgaaa 306660
   atagtagttc tctcgcattt cttgttacaa cccaattaga catttcttat tagacccaac 306720
   cacacatcct catttaaata ccagggccag gtatttcagg tttcaatctc cttgttccac 306780
   tcctcccctt tctagcagtc actgtgagtc atcccttcta ggggtgtagt gacatttgct 306840
   taacaatagc aagtccacag aaaccagaga tgactgggct aaatgcacct tcattctaaa 306900
   cttcgtttca cactttcatg gctttctcag ctagtgggca aaaatgctac ttgattaaat 306960
   tacaggtgtg gcatggcctc attttaaaag gttgaaatct taatgcaacc tgtcaatctg 307020
   acctctgaaa tgaaaaatta gcttatctat cacacagatc catgctttta tcatctataa 307080
   agagattttt aaatacagaa aaccttacgt aataaatcaa gaaatagcca agttgtatca 307140
   tccgagttga tctatacgcc ggatatcact gctcttcttt tcatctggcc aacgtgaaga 307200
   ctgtattcga aaagtgctgc agtcctacct acatccacac tattaattcc caagcatctt 307260
   ctccccatta tatcactttc cagtttttcc tgaggtacgt cttcacttgg cagagtctac 307320
   ccagttgcct tctcctaaac agactgttag aagtctgaaa tcatttttag actgctggct 307380
   tcctctttac tgctagaggc aagtgcattt gattccttca aaccacatga cttcgcatca 307440
   gactgatgtg gccactgtca aatgtaacat ttaaacatat gtttgtgtgg acatatgtgt 307500
   ttggaacatt tcatccgaag tctgacgctt aagaatacac tttggaaaat cagaattagg 307560
   gccttcaaga taaactagaa tgtgatagga catcttcatt tctggtcaaa gttggaagtt 307620
   taggagagcc tcaaaagtga agaattcgcc aacaaccatg gtgaatgcag tgtactgagt 307680
   tcttgtaacc acccggtggg atcttcttgc ctgctcccca gatagagccg atttctcaag 307740
   aggggaatta taatagggaa agagctttac acacaaagac tcaaatcagc ctccatgaat 307800
   atttggaggc tagaattttt ccaagatagt ttggcaggga gaggaggtgg ctagggaatg 307860
   ggtgctgctg attggttgga gatgcagtca taggggtgtg gaaaatggtc cttatgctct 307920
   gtatccactt ctgggtgggg tcgcaggact ggcttatggg ttcacgtcgg ggctgtccag 307980
   tagtcagaaa gggaaaagac tgaaagacat ctcaaaaggc caatctgcag ttctacaaca 308040
   gtgatgttat ctgtgggagt aattgggaaa gttgcaagtc ttgtgacttc tggaataatg 308100
   gctagtaatc ctttatgtct acaccttagc cgaattcagg ctcctttcat cctcctaact 308160
   tggcagtctt tcattagttt tacaaaggca gtttagtttg ggggaagggc tgttaccatg 308220
   taaactatac actaaatgtt tcccaaattt agcttggccc aagtccagaa atgactaagg 308280
   gcagtttgga agtcaaaagc aagatgagga ttggttagat cagatctctt ccactgtcat 308340
   catttagtta taatttttgc aaaggcgatt tcattctttg ctatatgtta agctgttaac 308400
   atgccttatg tcatgaaatc atcatcatgg ccctactaac tcttgttaat ggactcttgt 308460
   ctatatttta ccctgatatc caacctgctg tgagacctct tgcttttgtt tgtttgtttt 308520
   tattttttgt tttttgagac agagtctccc tctgttgccc aggctggagt gcagaggtga 308580
   gatctcgggt cactgcaacg tccacctccc aggttcaagt gattctcctg cctcagcctc 308640
   ccaagtagct gggattgcaa gcatgcacca ccatgcctgg ctaatttttg tattttttag 308700
   tagagacggg gtttcaccat gttgacaagg ctggtctcga actcctggtc tcaagtgatc 308760
   cacctgcctc agcctcccaa agtgctggga ttacaggcgt gagccactgt gcccggccaa 308820
   gacctcctgt ttggaatggc tttcccctcc attccaggcc tctggttatc agagatgagg 308880
   tagcttaagc ccttcagtgc ttaatccctg gcccaggcag aaggagggct ttggcaaatc 308940
   cgcatgctag aaaagatgag tttggaaggg cctggaaacc agggtacata gttccttttc 309000
   ccttgagatt ttagaagttg acagacggtt tgctggtttt gtagggaaat cctatctgcc 309060
   acagagcatg tggattaccc agggaggatc ctggaggtca cgtgcccagt accacctcta 309120
   agcacagaca gtatgacctt gcctttcact ctgggctttg tgtaaggatc tcccaggaca 309180
   tagatttcat agcgacattg gtcgattgct ctgattcact ttctgccttt ctctttcttg 309240
   catatggaga atgtgggaaa gaatccccta tacacaccca caggagttag aacatccttc 309300
   ttatcctcca taatcgtgcc ttcaaattaa taattatggc ttgctgtgtg ttgggccctg 309360
   agcttaacct gcattatgtc acttgatctc acaacaacct tctgagggag aaactgtcat 309420
   tattcccctt ttatggattg gggaactgag actcagagag gttgagtaac tcagccaaga 309480
   acacacagca aaccatatga tagagtcagg atttgagctc atgattccaa agtctgaact 309540
   tgaaccttct atgactataa ttctaattag atataagctt ccagatcatc agactctgtc 309600
   gatgccataa tctctttctt acatggcaga gatggtcagg atacctgcca cgtggtaaag 309660
   acgtggatgc agaagttaac ctccatttga ttagcttcgg tgcaccaagc tgcacccagg 309720
   tttctctcga aatcctgctc ctgagtgggt gagtgccccg ggaagagcag gtggttggcg 309780
   tttgctcagt tttccagccc ctgatcaccc tgatggcctc tagccagctt ttctctctgt 309840
   cctcctccag tgagggccag gcctggcaca ggcagcctcc caatgtttag aaggttccga 309900
   tttaattggt ctagagtgtg gcccaagcat cggcttctaa aacctccaca gggagttcta 309960
   atatgcagca gggagaagca gcagctcagt gcagcaggca aggctcgtcc acggaagggc 310020
   tgggggaagg aaggagatca tatttgcctt aaaacttgga gttgagctca tctgacctac 310080
   cctctgcagg tcttcagggg gactgggaag aatccatgca ggagatagcc ccagagatca 310140
   aggggatcct caaagagatg ggatcttaag atagagggag ggtacactcc ttgcaacaga 310200
   tcagcaccct gaccctgtac ccagcctcgt ccacaaacct gctccctagt gctgggcatg 310260
   ggatacagag ctaggggtcg tgtggtcccc cggaggccta cagtatatac acatgagctg 310320
   taacttgctt gtctgtccct ccccaagttg acctcagttt atcacacctt actgatgctc 310380
   ttctgccaat ttagtgtttg aggaccctgt gagccctaaa ggtctctttc tcttatttta 310440
   ttattttatt ttattatttc atttatttat tttgagatgg agtctcactc tgtcatccag 310500
   tctggtgtgc agtggcatga tctcggctca ctgcaacctc cacctccttg gttcaagcga 310560
   gtctcctgcc tcagcctccc aagtagctgg gattacagat gtgcaccacc acacctggct 310620
   aatttttgta cttttagtag agacggggtt tcaccatgtt ggccaggctg ttcttgaact 310680
   cctaacctca gatgatccac ctgccgtggc ctccaaggtg ctgggactac aggcatgagt 310740
   caccgtgccc ggccgagata ggatcttaag acagagggag ggtatagtcc ttgcaacaga 310800
   tgagtaccct gatcctatac ccagtgtcat ccacaagcct gctccctagc agggatggga 310860
   gagggagcta gggagggagg ggcatgggag agggaactag ggatcgtgtg atcccctgga 310920
   ggcctacagt atatacacat gagctgtaac ttgcttgtct gtccctcccc aagttgacct 310980
   cagctcacca caccttactg tgttgaggta taatttacag aagactcact tgtttgtttc 311040
   atgtaatctt gaatgatgct cttctcccaa tttagtgtgt ggggacctaa gagccctaaa 311100
   ggtctctttg tctattttag atgcaccatc tgaattcaag ttagaatctt ttcccaatca 311160
   ctgtttgtta gtcaagcttg cagactgtga cagtgctaga tgttttggtc tcttcttcct 311220
   tgcctcctcc tcctcctttt ctgattctgt tgcagggatg ttattaagaa caccatccac 311280
   agcacagtgt gttcaaacat ccatgcaaaa catttctctt agacctaagt tctagagaaa 311340
   gcttattttt ctcctcaaaa aacgattttc attatagaaa atatgaattt tgttatgttg 311400
   catatatttc tgattgcttt ggtttccagg aagcagaaag agaaagtagg agagagagaa 311460
   agagagagag agaaagagag agactgagaa gaaaagaggg agagaaaaaa aagaaaagag 311520
   aacgaaggag agagggagat ggcaaaggag ggaaagagag agtatatatc tataaagtat 311580
   atatgcatac atgtacacat acacgtgtat ttactatatt ctttttttct ttttgagacc 311640
   gagtctcact ctgtcaccca ggctggagtg cagtggcatg atgatctctc ggctcactgc 311700
   aacctccacc tctcgggttc aagtgatact cctgcctcag cctcccaagt aactgggatt 311760
   acaggcgtgt gctatcatgc ccagctaatt cttttgtatt tttagttgag acagggtttc 311820
   accatgttgg ccaggctggt ctcgaaatcc tgacctcaag tgatccaccc tccttggcct 311880
   cccaaagtgc cgggattaca ggcatgagcc accaagcccg gccactacat tcatttttaa 311940
   ccacctgtgt tgaggtataa tttacagaag attcacttgt ttgtttcatg taatcttgaa 312000
   tgatccctca aattctttgt gaaatatgag ttggatggag ccttatgtaa attaaacaat 312060
   gctgacaccc aaggaactga agatctttag tgaccttgat ctgggagatt tttaaagcaa 312120
   cttctcctac tcatgaaatc ctttcattca ttgattcaac ggatatgtat tgagtgtcaa 312180
   ctgtgtgcta gatgttgggg acatgacagt gggcagctca aagaaaatgc ctgccctcat 312240
   ggagcttcca ctcttgtgag ggataaaaat aaacagatag atattaatat aatgcccgtg 312300
   tggtaagtgc tgtcaagaaa aacaaaggag gggaagggga tggaaagtga caatggggcc 312360
   tatgagttta gagaaggtgg tagggagggt gttggtgaca gggtcacatt ggagtgacct 312420
   gaacaagatg agggagcaaa ctgttgagtg tccctagggg aagagtcatg caggcaggaa 312480
   gagcaaaagg cagtgattct gagaggtgag attgagtccc aagtacagta ggaggacaga 312540
   ggaaagcctt aggaaggtgg agtgacatga acaaacttaa gtttctaaag catcgttcag 312600
   gtgtatagag aatagaatgg accaggtgca gtggttcatg cctgtaatcc agcactttgg 312660
   gaggccaagg taggaggatc tcctgaggcc aggagttcca gacaagctgg ggcaacagag 312720
   tgagacctaa tgtctaccaa aaaagagaga aaaagagaat agaatgttta tgagagcgag 312780
   aagaggggaa gcagaaaaca gggtaggaga caactgcagg aaaccaggct aaggatcaag 312840
   gtggctcaga tcagcaaaga ggttgaactt agtatatatt ttgaatgtag agctgacaaa 312900
   tcttgtgatt gtactggatg agagatacag gagagaggaa ctgagggtga ctggatttgg 312960
   ctttggactt gtggcaagtg tatgttgaag ttgagcagtt gggaagcaag atcttgggtc 313020
   cctggctgtc tctgcccttg cacactagtg tggccatttg cactgctccc tcaggacaaa 313080
   gctcccgggc cagttgaaaa ccggaggttg gtgctttgta caaagctcac catcagcccc 313140
   ctttgttgtt tggtgaaaag atgatgttga cgatcaccac tgtaatgggt agaattgtgt 313200
   cctcgtaaac aatgtatgtt caatttctaa tgaaggttgc ctcacttgga catagggtct 313260
   ttgcagttat aagcaagtta agatgtggtc actctggatt agggtgggcc tgaatccaat 313320
   ggctggtgtc ctcataagaa gagggatatt tggataccga cagaacacac agggaagaat 313380
   accaggggaa gacagaggca gagattggag tgatgcgctt aaaagccagg gagtgccaac 313440
   ggtagctggc agccagcagg acctaggagg gaggtgtgga tggaacagat cctccctcag 313500
   aatcttcaga aggaaccaac cctgccagca ccttgatttg ggacttccag cctccagaac 313560
   tctgaaataa taaattcctt ttgtgtgaag tcactcaggt tttggctctt ccttttggtc 313620
   atgctaggaa actagtagaa ccaccaggta ttaaatattt ataatttgcc atgcacagtg 313680
   ctaagttaag gtgtgcacta agatacttta tcccataaaa ctctctcagc agcaccaaaa 313740
   gcaaagtatt gttggcatct ccattttata agatgaggag aagattgagg ttcagaaaac 313800
   agtgaatttc tcaaggtcgt acagcttgta tgtggcaaaa cttggacacg taccaggttt 313860
   tctaactcta aaagtccatg ccctaaccat tatgcaacgc tgggccacag tcaggggccg 313920
   agattccagg cacgctttgc cattgggatt cgccagtaaa gttcatcacc acaaactttc 313980
   cagccaggaa tccctgggct actttcctct gttttatttg ctgtcatcag agttctgcta 314040
   tttatcacat ctctcccaga agattcttgg gggaacagat gtggcttgga atttgataag 314100
   aacgtcactt caacacatcc tttcagtctg tttggaatcc tcaccctgga gagagaggga 314160
   cagctgaaca aacagcccac gccctctatg ttggtattaa agtttctgga agctccagaa 314220
   actgcttgag acaattaata agagatcatt tccatttaaa ggaacatttt tgaacgtggg 314280
   catcaccagc cagggcaatg gctacttttc tgctcttttc ctctcccatg gcaaaacttc 314340
   acagcatatg gccgggcgcg gtggctcatg cctgtaatcc cagcactttg ggaggctgag 314400
   gcgggcggat cacgaggtca ggagattgag accatcctgg ctaacacgat gaaaccccgt 314460
   ctctactaaa aatacaaaaa attagccggg catcgtggcg ggcgcctgta gtcccagcta 314520
   ctcgggaggc tgaggcagga gaatggagtg aactcgggag gcggagcttg cagtgagccg 314580
   agatcgcacc actgcactcc agcctgggca acagaggaga ctctgtctca aaaaaaaaaa 314640
   aaaaacttca cagcatatgt ggaggcagaa aggtctgccc aagccacttg atggaagcag 314700
   ggccgtgttt gtcctgctta ttgggcacat caccagtgcc tagcatcatg cctagaacaa 314760
   aattcccaat agagcttgta gacctgatgg aagaactgga atcgtgactg cagcatgaat 314820
   gcacaccaag ctcatctcgc caagcaaatg ttagtggttg tataagaaat agaaaaagga 314880
   aatgactgtt ctctctgtgg ctatgtgcaa ggtggatagg aaaagaggaa taggaccagg 314940
   atctaaaact gaatttaaaa tattccagca ctgatttgct caccactgaa cacttggcac 315000
   ttagggcttg atgatgggtg tgtttaaggg catggattta agggacaggt gactaggtgt 315060
   cacttcctag tggaatacgg acgagtcacc tagctttgtt aagccctccc ttcttcatct 315120
   gtaaaatagg gatagcagca gtttccacct tacgtattta ctgtgggaat taaataagat 315180
   aatgcatatt ggaagtaatc agtgggcatg tacatggtta ttgttataaa gtccaaactg 315240
   tcccaacgtt gccctttttt tccttggcac aaacactagg cctttgagta tcacaaattc 315300
   tttgtccagc caccctactt ggtggctgga tctctgtgat tctattagag acaattgtct 315360
   gctacatctc agagagatgg gagtcagcat ctgcaatttg atttctgaag cttaagtgct 315420
   catatcctta taggaatcat aggccaaggt ttgtagatcc agatcgttta atcctaagga 315480
   agcactgttc ttttcccttt ggctgaaata tgcctcctgt gtccccgcct tgctagtcct 315540
   attgatactt caggtgtcag cttaaccatc acttcccgaa gagcatcttt tgctgaccca 315600
   ctcagcgatg ttaagtcttc catttaaaca ctccaagagc aatgtgtact tctgtttcag 315660
   gggtctcatt atgattgatg ttaggtagtt atttgtacag gagtgtaagc ttcatgaggg 315720
   caggaacctt gtcgttctgc attcctggtg cctatctcag tgtctgacac atcctagacc 315780
   ctaaattaat gttaactgaa agaattgtct agtcttctag gtgctggata aaactaggaa 315840
   agttgatgca tttggaaggc ccaatgaatt ccatctctgt tgcctctctc agttgaagct 315900
   ttaaacaata ttgttattat tgattgaaaa gagcatgcat aaccaaaccc aaggtggcag 315960
   ggaccactgc tgaccaagct gggttgcaaa ttaaactgca gccatttttg atcatgtggt 316020
   tactcttctt ttccctgatc cttcttgaaa cacttattct aaaacgaggg tcagcagact 316080
   attggaaccc aggctactgc ttatttttgt aaataaagtt ttattagacc atagccacac 316140
   tcattcactt gatccagacc gccctgcaaa gcctataata tttcctgctt ggccctttat 316200
   agagaaagag ggacttgata aatctgttct agttatctat tgctatgtag caagtcattc 316260
   caaaacatag aagcttaaaa caacagccat cctatattat ctgtcatggt ttctgtgggt 316320
   caggagattg ggaatgatca ctggttggtt gtggcttgag gtttttcctg cactggctgg 316380
   agcacctggg gctgcctggt catcaagctt atcaggtagt accagggttt cttcatgggg 316440
   tcttgttccc tggtctagca ttgtgtcttg gggtagaaag gaaagaaact gacagaagct 316500
   acagcctcag aagtcttgca gggtctcttc accacattct gttcattaag gcagccgcag 316560
   tgcctgccca ggttcagaga ctctggctct gattggagga gtatcaaata tttgtagaca 316620
   tgttttaaaa cctctataaa gtatcacttc cttcgtgcta tggggacagc tctcaaaatg 316680
   taactccaag agaggacaga gagatttata tcgaaaatga aagcttgatt agaatattaa 316740
   gaagaaaagg aaagttttct ctcatcctat taatgactgc tgagaaagtg ttaggaaaat 316800
   caggatggaa ggattctgtc agctttacct actgacatct cctagccacc ttattaaatg 316860
   aatcaatttt gataatatct ggtgattata atcaacatag gaagaatatg tttattaaaa 316920
   tgcagactcc caggtcacag cccagagctc ctgaaggaga gggagtgctt ggaatctata 316980
   tggtaagcaa agaccctcaa gtgattctga cataggcaag aggcactgtc ctttataaag 317040
   actggaattt gccttgtttt aggtcacata ctgacatgat gctacttggc ttcatgtatt 317100
   ttaattatta gtaagaattt gagctaccta tgtaataatt ttgttttagg gatctctaag 317160
   atacataatc cattcaaagt aatataaata aaaataactt tattggagga acataaaggt 317220
   ttcaaacaga aaatggctaa gcctcatgag agattggaaa ctgaaagcaa ggcctctgct 317280
   tgttctctta ccttctttgt ttttttcccc ctgcctcaaa catctatttt attcctcttt 317340
   gcctgcacag tggtagaaaa ggaccacgct aggctgagtg tggtggctta cgcctgtaat 317400
   cccagcactt tgggagacct agatgggcgg atcactgaag gtctggagtt tgagaacagc 317460
   tggggcaaca tggtgaaacc ccatttctac taaaaataca aaaattaacc aggtgtggtg 317520
   gtggacgcct gtaatcccag ctacttggga ggttgaggca agagaattgc ttgaactcgg 317580
   gaggaggtga acgaagatca tgccactgca ccccagcctg ggcaacaggg tgagaatctg 317640
   tctcaaaaac gaaaaacaga aaacaaaaca aaacaaaaca aaaaaggacc actctaggaa 317700
   gatctgtgta ttctctgttg agggacccag gactgactcc tcagagtacc ctaattcaaa 317760
   ttcttgggag ggggaatccg attggcttgg ctcaggttga ggaacttacc tctgatccaa 317820
   tcagctgtga ccagctgtat ccactgccca atcagaaggt tcttctaata acacagagtt 317880
   ggaagaagtg aaaggcatgc tagggttttc atttccaaat taaactgcag tcatttttta 317940
   tcatgtggtt actcttattt tccctgatct tcttgaaaca cttgttgcaa aacaagggtc 318000
   agcaaactat tggagcccag gctattgctt attgttgtaa ataaagtttt attagactac 318060
   ggccacactc attctcttga tccagaccgt cccgcaaagc ctataatatt tcctgtttgg 318120
   ccctttatag agaaagtgtc ctgactcctg ctctagaatt cagttcgctt ctatgtctaa 318180
   atttttgcta tgccttatgc atgcccgttc tgttttccgt taatcagtat agggcatgtt 318240
   gttgagggca gggcaagaca ctgggaatct tcacacaccc ctgtttattg atttttaaaa 318300
   ctatctgtac aaatgcaata gtatctccta cgatgcggag cacatagcat tgctgatttg 318360
   caagaagatt tcaactgcta cttgagattg ttttaggtgc cgtgtagttg cactatgaaa 318420
   taaaattaaa tcacgttatg ataaggttat ttctgtgtct ccttttactc tttctaatta 318480
   cttgaacaag caaatctcat ttgatgacaa tatgctctct aacaactttc taatacaatt 318540
   ttttccttta acaaggagaa gatgcagagc ctgactcaga accttcagca gccagcagta 318600
   tatagctaga atttactggc accgctttgc ttttatttat ttttatgctt atcttccatt 318660
   tatgacttgt gatactggct tgccattaat agtggtgata taaagtttcc ttttcaaatt 318720
   tagttaagtt gaaatgtgac tagattttta aaatgctaag caaataacag tttaggtggc 318780
   aggaagaaat gtgaaaaaac ggatatgaat gattgaagtg cggaaatgcc tgagctacag 318840
   taactgacat agttacaaag cagccctggt gcccagagga ggtagtggcc gatttggtct 318900
   gaatggaacc tccagattag aggaggagat aacagcccag tggaatctca gattttcaaa 318960
   gcagtgcaag agcatggaag gacgtcatag acagtgggag tcgaatgttc aaaggaaaag 319020
   aaagaacagg atgtggtcat ggaattgttt ggctttcaag agccactgaa gggctttcag 319080
   cattcaagtg gcacagtcag agctgcattt tcagaacagt cattagctga agcaagttaa 319140
   tctccctgag cctcagtgtg tttatctacg aaatgggggt aattgacaca gatgtaggat 319200
   gtccacccct gtccctggtg aagggggaaa ttcccagcca ttctgccctg tctctcttcc 319260
   atgtggaagg ctaagatgtc ctatcatgac tgcatctgca ctgaaggtgt ctagggcccc 319320
   tccttctctc gttttggttg ggggaatggg agatagttgc agatattcag gattgttgga 319380
   tggtgccttg accggtggtg atgggtcagg gagagtgtat tagtccgttt tcatgctgct 319440
   gataaagaca tacccgagac tgtgtaattt atgaaaaaga ggtttaatgg acctggggag 319500
   gcctcaatca tggtggaagg ctaaagtcac gtcttacatg gtagcagtca agagagaatg 319560
   agagctaagc gaaaggggaa accccttata aaatcatccg atcttgtgag acttattcac 319620
   taccactaga acagtaggca ggaaaccgct cccatgattc aattatctcc caccaggtcc 319680
   ctcccacaac acgtggaaat tacgggagct acaattcaag acgagatttg ggtggggaca 319740
   cagccaaacc atatcagagg gtgtccactg tcaaggacag ctcagcacca agcccttctt 319800
   actgggctct ggagtttctc agctgagaac tcttcttctt tccatctcca attcctctgc 319860
   tcacttgtct tccagctaag tcacctccct gccccctccc tgcacatgtc ccggccccag 319920
   ctctctctgg aatccaggag caggaagtcc ctgtctccac ctgactctac atcttaggaa 319980
   agtgactccc ttgagtcatt gtttctgtgt tagtaagcat tgaggaagga aaatacatcc 320040
   cttgggtacc agggagagaa cagagggagg caagaaataa actgaaggtg cccatcctag 320100
   cacttggagc cctgtcttgc catgcacgta agcagagggt ctgcaggagc cactcagact 320160
   ggcagctttt cttttttatt gtgctgggga agaaagaggt taaaaagagt tctcttgctg 320220
   tggaagtgtg tgacagggac cctcttccta aggtgtccca aatccctgca tctgcctggc 320280
   ctttgtcact gtggctgtga tgagagagaa gatggtgagg acaggagata ggaatgctgt 320340
   gagctggcaa gctcagctcg gaagcttcag tgcatgggtg catgatctgg aagcttctgt 320400
   gcatgagttt agccagacct tttgctgctg ggataagagc tgagtggggt gtgcagagct 320460
   gccttcctgc tgctaagctt attctatatg tcaccccttc tagcctgagt cctctgttgc 320520
   atcctctggt cctccagctg agttgagtgt agcatctaat ttcctcaatc acctgaactc 320580
   tggccctgat tggttccctt accctcctga gccttaattt cctgcaatat gggattcaca 320640
   atggcaccta cttcctatga tgttatgcaa tacaaatgag taacaataag tgaaaacctg 320700
   gaattgtttg gtcagtagaa aagtgctcaa aaatgtaatg gtggttagta ttgttgcctg 320760
   caaaatatct gacacagtgc atgcagtgaa tacttcaatc tgattctttc ttcctccagg 320820
   aagccttcct agaagtcccc aaaccaggtt aggactcttc gtaatggggt tctgccttgt 320880
   gcttcctaca gggcttcttg agacaatctg ctggtcattg ttctgtctgg cataatcaca 320940
   accaaagaat gttcaaagaa tattcccccg gttgggagag tgggagaggg ggtgacacgc 321000
   agaaaatacc gatgtgtctg tgagccacac tggttaactg gccctgaata tgaccacagc 321060
   acagctcata gaaggaagcc ccaagacaac aggagaggct gttaaaatat taaattaatc 321120
   atctctgtaa ttaatttgca aaatatttct tcctctctgg agttaagggt cagagaacgt 321180
   gcttgtttct ttattgctgt atccccagtg cttagcaggt tcttagtgaa cattaaatac 321240
   ttgaattaat aaaggaacaa gtgccctaat tttagttttg tcagtcattc actcattctg 321300
   tcattcaaca aacatttatt gaacatgtac tatgagccag gagctattgc aggctcttgg 321360
   gttacaccag taaaccaaaa cagcaaagat acttgccctc atggggctta ccttctagct 321420
   gggcattgac acattgcctg ttacctcaga gggatcaata tacaatcatc tcccctggaa 321480
   ccatcctctc ttgattgagt tcagctattc aaagtgcgag gtcagggctg gatgcagaat 321540
   cacccctgta atcttagcac tttgggaagc cagggcaggt ggattgcttg aggccaggat 321600
   accagcctgg gcaacatagt gagaccttgt ctctacaaaa aataaaaaat aaaaaaatta 321660
   gccggacatg gtggcactta cctgtagccc tagctacttg ggaggctgag gcaggaggat 321720
   cactgagccc aggatttcga gtccgcagtg agctatgatc atgttactac cctctaggct 321780
   gcagtgagtc atgatcatgc tattgcaatc taggctgggt ggcagagcaa gaccctgtgt 321840
   ctgaaaatat aaaagtgtga agtcaataga aaacattacc tgttgtttca gagtcaacag 321900
   aaggcaccta tacaaagcta agtgtggcta caaatgaaca ccagctgctc ataactggcc 321960
   cccagactcc ttccaactgc cctgacatgc ttgctctctt tcccaggtca tgctttcaga 322020
   acgaaaaggc acagatgagc tctatgctgt gaagatcctg aagaaggacg ttgtgatcca 322080
   agatgatgac gtggagtgca ctatggtgga gaagcgggtg ttggccctgc ctgggaagcc 322140
   gcccttcctg acccagctcc actcctgctt ccagaccatg gtaacttgtc ccatggccct 322200
   gggtattcca cctccagggc ttcaccaggc tttggccaaa gctatcttag cagcacccag 322260
   cacagagata cctgcaccct tccctttcta gacacagaaa gatgtaaggc ccagggaagt 322320
   cagctgagag ttctgaggtt gctactgaga aacgctctcc cctgagacag atgctgtctc 322380
   aacttagata aaggaattca ggcaactggt tttataatta acagagcagg gcctttctct 322440
   gacagcaagg agtgacttgg ggacaggagc tgggtgaaaa ggttcacagc ttgtggatct 322500
   agatggttcc attcagccct ctagaccttt ggataaatgc acaagtacat ctctgcaggt 322560
   tgcctgaggc ctttgcatgg gcctcattgg agggaggatg ttgcctgaga acacagccct 322620
   cgaggactct cagggaaggt acacagtgtc ctgggaagga gctagtgagt tcagagccat 322680
   ggctgccaag gtgagaaggg agcacttgtc catttgtcca ttttctgctc ttgactgtga 322740
   tttcaaccct atctgcaaga gcaagagtcc ctgctttaag taacctctta cctttccctg 322800
   ttcaaattag accttggttt ccatacccag ggatggagag gccagagccc aaatgcaaag 322860
   gggtacaaag ttaattgatg ccttttagga ttctcagggc caatgacctg gtgacttaag 322920
   atgttttggg gacttttcaa gattaatttt gactctatcc agttctcaac ttactgagta 322980
   gagtgactga ttttagagcc caacccatat atgagatggg acactacagt actttgcatc 323040
   taatttgatg tctttagtga tttccctctc actttaattc ttttctttta tctgttctat 323100
   tatctgctga agatatcctg ggctgtgtga cctgtagcat tttcacagtc tggatttcac 323160
   tgactgtacg gtcttggggc acctcaacat gtttcctctg tcttctgtat ttcgtgcaat 323220
   tggcagctgg ttctggaggc ttggtcagac tcagctagga tctctttggc aagaccatag 323280
   atggtgacct gttctttcat ctgaagtcgt ataatggctt gttgtctctc atagtgaagt 323340
   tggcagcgac tgatggttgg tgcctagatc tgttaactca ttagggattg taaaatggtg 323400
   atatcctaat tctatcattt atttttcatt tactagtgag aatgccttta taaagaaggg 323460
   cctcccttca cctattactt agttccctag tggtatagtt cctgtgagaa aggcaggata 323520
   aatgcttgat tctttctctt tatttaatag ttctcaagat aataaattgg ttcactgttg 323580
   tgctccaagg gtggctaaag ttggcttttt tttttctaaa tgggtctggc tctgtctccc 323640
   cagctagagt gcagtggcat gatcatggct gactgcagcc tcgatctccc atgctcaagt 323700
   gatcttccca cctcaacctc cctagtagct aggactacag gcacacacca ccacgcccag 323760
   ctaatttttg aacttttagt agagaccggg tttcaccatg ttgtccaggc tggtcttgaa 323820
   ctcctggact caagtgattc acctgcttcg gcctcccaag gtgctgggat tacaggcatg 323880
   agtcaccatg cccggccaaa gttggctttt aaaataaact gttttgagtg gaagaagaga 323940
   caatagaaag aaaaatatta agtgaatgat aatttacatc atgcaatgga gacttggcaa 324000
   agaccaggaa aggggtacac ctaaccttac tagtccaaat ctctagtctt accatgggga 324060
   tttaaggcct aaaggtgttc aagtggtgat agagacaagg ctgggattta aacctctagg 324120
   ctcccctact cccttacctt ctcccttttc cattggttat ccccagatgg attcattgac 324180
   atcatcaggc aaaggacagt ctccgtccag taaatcaact aagtaagaaa gggaccttgt 324240
   gcccagcata gaggctgagc agcagcagtt ttccatttta gttggatatg ttgagagtgg 324300
   gagaaatgaa tcatgggtga ctaatgttca ttgtagtgtc tttgtggttt attattttaa 324360
   aaatgattaa atagagtaga tttgagatta ttcttttaat attgaaccaa cctactcacc 324420
   tatatttcaa tatgctttct ggaattagag agtaggggct gggtgtacat ggaaattttg 324480
   cagaagtctg ggtcccctcc agggattgtc atgtgccagg gaagagagac tgagaaccag 324540
   gagagcctag aacacaagag cccagtgggt ttgggtcttg catggtgaca tgatttggct 324600
   gtgtccccac ccaaatctca tcttgaattg tagttcccat aatccccata tgtcgtggga 324660
   gggaccaagt gggaggtaat tgaatcatgg gggcggttac ctccatgcta gttatgatag 324720
   tgagtgaatt ctcacaagat ctgatggttt aagagggact tttaccccac ttcgctctgc 324780
   acttctcctt gctgctgcca cgtgaagaaa gacacgtgtg cttccctttc tgccatgatt 324840
   gtaagtttcc tgaggcctct ctagccatgc tgaactccga gtcaattaaa cccctttcct 324900
   ttataaatta tcccatctca ggtatgtcta atggactaat gagaatggac taatacacat 324960
   gggaatgaag gcctcccatg ttggagacca atgcaagtaa ttgaatcgtg ggggcagtca 325020
   cctccatgct agtcatgaga gtgagtgaat tctcacgaga tctgatggtt ttatgaggga 325080
   cttttacccc atttcgctct gcacttctcc ttgctggtgc catgtgaagg acacgtttgc 325140
   ttccccttct gccgtgattc taagtttcct gaggcctctc tagccgtgct gaactccaag 325200
   tcaattaaac ccctttcctt tataaattac cgagtctcag gtatgtctaa tggactaatg 325260
   agaatggact aatacacatg ggaatgaagg ccttcccatg tggagaccaa tgcagaatct 325320
   ccccaaccga caagtccaag gatggaacag attgctttct cagttgttga gggtctgatg 325380
   aagtctttaa cctatctcta gggattgtat cgattgaaag aaaaaagtgc ttggagagac 325440
   gcactcactg caagcaagag accctgagat gccaagtgca gttggctgaa ggaagagcag 325500
   atccccttcc atctcacctg cactctgcgc atatgctcat tgagtgaaat gctccaattt 325560
   aaattatttt ttctcacttc tttgtgggct aagcacatat attttaatgt tgcataaatt 325620
   aaatttattt tcaaagactt ataataatgc ctactatatg tgaggcacaa ggcaaacaat 325680
   tttaagtctc ttgacaactt agataggttc tgtcatcatt cccattgtgt agaccagggg 325740
   actgaggcca cacagctagt acatttcagg gccaggattt gaacccaggc agtctggctt 325800
   taaggagttt tgcatttaat tgctatgctc tgagatcttt ttaaaaaaac atgtatatgt 325860
   ataagtatat gtatatgtat atgtatatgt atatgtatat gtatatgtat atgtatatgt 325920
   gtgtgtatgt gtgtgtgtgt gtatgtgtat gtgtgtgtat gtgtatgtgt gtgtatatgt 325980
   ataagtatat gtatttttag agacagggtc ttgctctgtg gcccaggctt gagtgcagtg 326040
   aaacaatcat agctcactgc agccttgggc tcctgggctc aatagatcct cctgcctcgg 326100
   cctccctagt agctaggact acaagcatgc atcaccacgc ccagttaata tttaaaaata 326160
   ttttgtagaa acagggtctt gctatgttac ctaggctggt ctcaatcttc taggctcaag 326220
   caatcctctt acctcggcct cccaaagtgc tgggattaca agtatgagcc accacaccca 326280
   gcctgaagtc ttttgagtgt ggatcaccca aagaccactc cacctgacac ccctgggtta 326340
   gaatccctcc tgcaggcctg tctccaggct ccaccaggag cctgtgctct aatctagcca 326400
   tggtcacctc tcacctgttc agtgaagcca cactacctcc acctcttagc ttccctatct 326460
   ctgcataaaa cttgatgttc catctgagcc tctaccatgc atgggtcttc aacaacgttg 326520
   cttcattcca gcctcacaaa aatcttgcag tgaaagggat tatctactct cccattttgc 326580
   aggtgaaagg attgagaatc agggctgcat ggtagttctt cctcgacaca gcactgggtc 326640
   attggctcat tgagctcaga gcccaagctt ttgttagtac atcatgttga gttgccacca 326700
   aaatggcctt gacaatgatg atacaaacaa gaacaaaaag cagcagctaa cagtaatagg 326760
   ttgggcactc tactcagcac tctagctgta ttatctcatt ttatcctcct gatacctcta 326820
   ttggaaaggt actattacta actttaattt acagacaaga atgctgacat tcagagaagt 326880
   taagtaactg gtgcactgtc acacagctgc taagtagtgg agctaagatt tgctctgcaa 326940
   gactctagaa ccccaaaccc ggaagccttt gctaacctac ctgtgcctta acttcacatt 327000
   cctggttttg gcttatagaa tattttcact ttacagccag gcacagtggc tcacacctgt 327060
   aacaccagca ctttgggagg tgtaggcaag tggattgctt gaatccagga ggtcaagacc 327120
   atcctgggca acatggcaag atcccatctc tacaaaaaat tagctaggag tgatgcacgc 327180
   acccgtagtc ccacctaccc aggaggctgt ggtgggaggg tcaactgagc ctgggaggtc 327240
   aaggctgcag tgagccatga ttgcatcact gcactccagc ctgagctaca gactgatact 327300
   ttgtctcaaa aaataaaata aaataaaata aaatataaaa tattttcact cttacgatca 327360
   catctaatct tcacaaaagc actataagta ttatggtcca catttctatt caatggaaac 327420
   tgacccccag aaagatgaaa tggctcaagt tccacaatca gtaagtggca gttccagggc 327480
   ttgagctcag gttttctaat agccctttgg gtgttttttt tttttttttt ttttaatact 327540
   atggaagtta ctccgcttat cctacaggga tattgcaagg attagataag attttatcca 327600
   tgggaatgat tattaaaatt tagggagcac tgcatataca tgaggcaagc cattgttatc 327660
   atgactaaaa tgagcaggaa ggtgacacct ctttctgggg atgtccttga aaatggtcat 327720
   ctggctcctg ctcattttct agcattgcat ggcctggatc tgcagagcag ggcttcagag 327780
   gaggcagtgg agctggaata ggccatatcc ctaaaagggc attactgcct gtatacagag 327840
   ggtattactg cctgtatgct gcctgtatac agagggtact aatgcttatt gattatttca 327900
   acaaagactt attcattgaa ataattgtat caggccccat tgcatcacca tgatgaggta 327960
   caaaacagat aaagtccctg tttctttgga atttacattc tcttggggga gactgataat 328020
   aaactataat aatgacacaa agtaaagagg atggagatgg atggattggg gcagagagat 328080
   gctatttttg ataggatcat caaggaaggt ctctctgata aggatgacat ctgagcagag 328140
   accagggcaa gcaagggagc ggggcatgtg gctatctggg ggcagagtgt tccaggcagc 328200
   aggaagagtg agtgtgaatg cccgcggcat gagcatgctc cgcgtgtttg aggaacagcg 328260
   aggaggccgg tgtcgccccg gtggattgtc attttgtatt ttgtgaccca gaattctttt 328320
   tgaatcagct acctttaaaa aaaaaagaga caaatccaat ataacatgct gtaaatttga 328380
   tgtttttctt tctagagact cagaatttta aagataaaag attcttaata ggctcttaaa 328440
   aaatctcaaa agtggccaaa atactggata aaagtgaaag agacaaccag caatgtacaa 328500
   aaccattttt taaaaatcaa tttttaaatg atgtaaattc gatttttaaa aacttattta 328560
   aaaaagcaaa gttacatcag aaataatctt atattggaat aaaaatatct ttcaaacata 328620
   tgcacagcac ggtgttaaca gaaaatgtag gatattaaat cctatttaag atattatttt 328680
   aagtgtgtta aaattgaata ttcacatgga aaagaatcag aagggggcat aaacaaatca 328740
   aaatattgat ttgatttagg gaggggtctg tggataagtg ccttcttttt gaacttacat 328800
   cattgcagaa gtagttataa tatagttgtt taagtttttt ttaatgtacc acaagatggc 328860
   aaagttaata tccaaataga tgcaaaagct cccagaaggg ggttgagttt tcagagattt 328920
   gctcattaga tggtttaggc aaattgtcct agaagcacaa agggataata agcctgtact 328980
   ccacagtcag gtgaatctgg atctgtgatt gcttgctaga tgacctctag gaagtcactt 329040
   aacagctctg aatatggatt tcatcatctt taaagtgggc ataatcaggg aggattgaaa 329100
   gagatggtga ataaaatata ataaacatga aatggaggtt gccggcatta ttagagctgg 329160
   actggaggtg caccttgctg cataggtacg aaagaattat gtttcttcca ttccttttgc 329220
   agtttttctc agggacaatt gtcagcccgg cacattcatg caatttccca actgccagtg 329280
   ggcacaggca gttaagtttc tttagcaaaa catctatttt aactgttttc ttttcaattg 329340
   aacatatgca cctctttttt tttttaaata gaattagatt ttatgagtca ttgaaggaca 329400
   gaagttactt cctccccaaa tttgctgcca tggcccagca ataactcagt tgcaaggcat 329460
   gctgggagga acgtcaattc agaatgcaag agaggacagg gatggggtga ggatgattcc 329520
   cacatgtaga gtggacccct gatggcccaa gaatgagccc cgagaatgtc ccaggggtcc 329580
   tggtaactcg ggctcagctc tgggctggca gcagtaccca aggatatggt tggacttaag 329640
   ggcattgctg caggtggctc tggctgttgt ctcagtcagc agtgatcaag ttgtaagaaa 329700
   cagaacaccc cttcccctgc cccccaccaa actggctcca atagctccaa aaaaacagag 329760
   aagacttttt tttttttttt tttttttttg agacaggatc tccctttgtc acccaggctg 329820
   gagtgcagtg gtataatcat ggcttactgc agcctggacc tcctgggctc aagcgatccc 329880
   cctgcctcga cttccggagt agctgggacc acaggcatgc accaccaagc ctgactaatt 329940
   ttttaaaatt tttttgtaga gatggggttg agacctatgt tgctcaggct gctctcaaac 330000
   tcctaggctc aagtagtccc catacctcag cctcccaaag tgttggaatt acaggcatga 330060
   gccaccgcgc ccagcctgga gaggattgtt gatgagaaat tagttaatgc gtgcaatgta 330120
   ccttattcag gtgatgggtg ccctaaaagc cctgacttga ccactatgca atctatgcag 330180
   gtaaccaaaa aaagaggatt ttattggaaa gaaaccaccc aaagacaaag gtgtacctgg 330240
   gcctcatgag gagctagaat ctgtaatcct aaagcagtct agaatagatt tctaggtttc 330300
   tttgtccttg ctttctttgt ttctcactgc tcctggtaaa catgacttcg ttagctcctt 330360
   gcagtttgcc tatctggcaa attccctgca tttcagatca tagtcctggg aaagagaatc 330420
   tggttggttc agcttgggtc aggtgtttat tcctggccca atcagctggc aattggttgc 330480
   tactaactca ctaggaacct tttaaaaggg gttgtatcta cagggaggga ataattggtg 330540
   ctcatgccag gtgagaggca ccatcttgag tccaaggaga ctaactggat tcaagcagga 330600
   catagttata ggctagtggt acaaacatgg catcattgcc tcccagggga ggaaactggt 330660
   gagagcttgg caggtgttag tgctttagcc aactaaagcc atccaaggat cttgagtaca 330720
   ggaaatgaat cagaaatgca acccaggcaa atagagctgg ggcaggtggg aatagacaca 330780
   gccttatgct ctctgttttc ttgatggtgg cctttgcata ttcattcaat aatcaaagat 330840
   ctgtggaatt ccgaggaaga atagaggtag ttctagcctt tgaaaacttt ggcctctatg 330900
   tggagggaca aggcttagaa agctgaaaac aaacaaacaa aaaagctaga ggagcatcaa 330960
   ttgcttgatt ctaacagtga ttgcaaggct ataaaataca gaggaaggga aagttgtgtg 331020
   tgaacacatg tcattgagtg cacacttact tgggtaaggc tttttgagtt tgcatggact 331080
   tgacatgcgt catctgatgt atcttggcaa cacccctatg aagaacaagc tgtcattacc 331140
   ccctttgggg aaggtagaga ctctaagtaa ctgacccaaa gtctcagagc aagtatccag 331200
   tataactgtg actagaaccc aggactagct gataccagaa ccagggctct taaccacagc 331260
   actacacagc ctctctggag agaaacagat ggttctgagc aataaaggga tcagcttcca 331320
   gacgggatga atccacagac cagcttggaa aatcattaaa gatcattaaa tagacaaatt 331380
   cttgcagcaa cttcccttgg agcatggtac atagagctgc ccacgtggtc ggttttctct 331440
   gttcatggta tctgattaaa agtggcaagt gtccccctgg cagtcacaca ggcatcatgt 331500
   tacactccac tttcagctgt cttaggggaa gattgtctct ttgaattccc ttgcttcctc 331560
   tggagacctc caggtgaatt ctgcacctcc cacctagtca tcattttaaa tcagactatc 331620
   ctcatactca tgttccaaat ttgggaaatt ctagccaacc attttgcata atgtagtgac 331680
   agacagacat ttagttttat ttatttactg attatgagag attaaggaga aagggaattt 331740
   taaattacct gaaggcactt gaagcttaga gggcagcggt aatagtgtaa tgaagaatca 331800
   caataacaat gacaattttg aagaagccct ttaaagtcaa gcagactagc cagtttgaag 331860
   agaaagatgg tgagtttgcc tttccgagct tttaattgga gttgacggtg gaacacccac 331920
   gtagaaacgt cctgtagcca gctggagctg actggagctc aggagagagg gagaggatag 331980
   aaatataagt caagactcat cagcccagcg agatgagagt tgaaattctg agctgtgaca 332040
   agttcttctt ggaaaattga gtaagtagga gagagccaat ctggacttca agtcgatacg 332100
   caagaaatac tttttttgat tttcatatat tagagtccaa caaataagcc ctaatcaaaa 332160
   aacactgagt gtggtgataa ggaagatttt gcaataattt cgtaggagga atggggaaca 332220
   aaggcaaatt taagggtgct atggagggga ataatatgta agaattagag gcagtacgtg 332280
   aggtccattt agttagccga agcccccata gttcactctt tatgcctctc atgcactttc 332340
   ttttatcgca ttctatttta tttatttatt tacttttatt tgaaacaggg tctcactctg 332400
   tcacccaggc tggagtgcaa tggtgcaatc actgctcatt gcagccttga cctcctgggc 332460
   acaagccgtc ctctcacctg agcctcctga gtagctgaga ctacaggtgt gcgccaccat 332520
   gcctggctaa tttttttatt ttttgtagag ccagggtctt gccatgttgc ccaggctgac 332580
   ctggaacttc tgggctcaaa agcaatcctt ccacctcagc ctctcaaagt gctgggattg 332640
   caggcatgag cccctgtgcc cagccacatt ctattttaga ttagggtttg aattagttgt 332700
   atacttgttt taaccaagat tctgaattcc ctgagggcaa gcctgaacct tattcatcgt 332760
   tttcatcctt ttaatgcctg cctaatgcca tgacttgcaa aaataagcgc tcgatgaaag 332820
   ttctaaaact cacttctctg ctccagattg gctctggatt gcctctgaca atattttcca 332880
   gattttgatg ttgcttactg tggagtggga gatgaacctg gtggtacatg aacacattgc 332940
   atttattttt aaaataagtc atttaaagga ttgatgtacc agagccagac tggatattgc 333000
   ataaagatga tggactccag atatgcactt cttttctaca gagaggatga atttccattt 333060
   atgactttag tatgaaattt aaaattttta aaataattgt gtttaagttt tttttaagtg 333120
   agttaataaa aatactttta aaataagtca attttagtag aaaaggcaga caaacttagt 333180
   aaagattttt ttcttttctt tctttctttt tttttttttt tttttttttg acacagggtc 333240
   tcactcttcc ccaggctgga gtgcagtggc atgatctggg ttcactgcaa cctccacatc 333300
   ctgggatcaa gccattcttg tgcctcagcc ttccgaatag ctgacattag aggtgtgtgt 333360
   caccatacct ggctaatttt tgtattttta gtagagatag ggtttctcca tgttggccag 333420
   gctggtctca agctcttggc cttaagtgat ctgtccacct taacctccca aagggcttac 333480
   aggtgggagc cactgcaccc ggtccagata tgtacttgtt ttctatagag gggatgaatt 333540
   tccatttatg actttggtat gaagttaaaa tattttaaaa taattgtgtg taagtttttt 333600
   taacggagtt aataaaaata tttttaaaaa ataagtcaat tttagtagag gaggcagaga 333660
   aacttggtaa agatcttaaa gctggttggg agacaagtca cgtttgggtg gatggtctta 333720
   ggcagtgcat cataatctgg tcttacccta cctttcccct tttttctttt atatgactcc 333780
   atgaatttta ctccccctct ccagcaataa tagccatcaa atcaggaacg taagtgctta 333840
   gttgtatttt ccacaatata cgttaaaaca aatacaaagc cataaaggtg aaaccatcat 333900
   acatccatgg actacctaaa atgatctcct acccattttg ggaaacacgg ccgtttggaa 333960
   atttatctgg cccctgtccc tcgcctggga atccttaaca tctggcactg tggattatcc 334020
   acagaaacat ctctggcaat agagttgctc ctggcataga gtgggcatca ttaagtgttt 334080
   gttcagaaat aaaaggacag tgagatgcca accgtaacat ttaatagctt aggcactggt 334140
   gtgttgaagc cacatgctca gtacagttca aagatgatag atcttagggt ttccctgcat 334200
   atggttagcg gtagaaacat accacaaaac acgaccttac ctacagtgga gcgatcccct 334260
   gatgtgggga ttccgactaa tgagtctggg gggcaagccg agttcttcca agcagaaagc 334320
   aagcaagtga aatctctctc tcacttactt ctctctctgt ggccaatgcc atctgctagc 334380
   tttaaaaata atttttaaaa caagtgtttt ggttctcttc aacgtgcagc aaagacagat 334440
   gctattttta ggcttgtttt ctattaaaaa aaaaaaaaaa gccaaagaac caggggctgg 334500
   gtgtcggcag ttctggctta ctgtctgcat tttgccacca acttgtatgt gaccttttgg 334560
   caaatctcct atgccttgga ccctgagaat ataagtgatc atttcacctc cttttctcat 334620
   tagacatgtt gagagggtga agcagtattg gtctgagaaa taaaaaccat tttctaaaaa 334680
   ttagtcagtg tgggcaacat ggcaaaaccc tgtctctaca aaaaatacaa aaattagcca 334740
   agtgtggttg tgtgaacctg tagtcccagc tactggagag gctgagacag gaggattact 334800
   tgagcctggg aggttgaggc tgcagtgagc agagatcacg ccattgtact ccagactggg 334860
   cgacagagtg agaccctgcc acagaaaaaa aaaaacaaaa aaattggtta acttgtgctc 334920
   tcctgtaacc tgccagcttc tgtcttgatg taccactaaa tctccatgtt ccttctctcc 334980
   catcctggac tggaagctct ttgaggggag gacacatcca cacatccata caatgtctag 335040
   ctcagaacct ttcacacaag atgtacccac tggaggcagc atcatgaggg gttgagagtg 335100
   tagagtctgg aacccaatgc ctaaggttca gaccctggct ttgcagcagc ttatcagctg 335160
   agagaccact ggcaagttac ttaccctttc tgagcttcaa ctttctcctc tttagaaggg 335220
   agagaatgat tatacttgtc acattgagtt ttgtagaggg acaagtgaga caaaatatgt 335280
   aaagcattca gctcatcacg tggtaagtac ccaagaaagc ttaatttcat tattgttgtt 335340
   gttataatta ttaggcactc ccacatttga taaatgcaca tatgactgtt acatataaga 335400
   taaagttcta tgtgctgtat ggaagatttt tttaaatggt gaattaaatg cttgttatat 335460
   ggccagcatc atgttagaca ctctacacat gtgtccttgt ttaatattct gtgggcaagt 335520
   gaaaaatgtc ccatctacag gtgaagagaa agcccaaaat tagattaaat aactgctcag 335580
   tgttgcagtt agtaagtagt gcaacaagac ttcaaatact gtattgactt aaaaggtgaa 335640
   atctttgctt cactttccca ccacctcttc tcccggtttt atagcccctc acaatagctt 335700
   tgcaactttt gacagcaagt caccttttac gttgtgactc agccatagca acatataaat 335760
   ataaccaaaa catatattcc acaaaaagta cttagtggtg ttctgtgcag catgcactga 335820
   taatttccat ttcatcattt cattattttt ctttcttctt ctactttttt tttttttttt 335880
   tttttttttt tttgagacag tttcactctg ttgcctgggc tggagtgcag tggcaccacc 335940
   atggctcact atagccttga actcctgggc tcaagcgatc ctcttgcctc agcctcccga 336000
   gtagctggga ctacaggtgc tcaccaccat gcctggctat tttttttttt tttttttttt 336060
   tggtagaggc agagtcttgc tatgttcccc aggctgttct caaactccta gcctcaagtg 336120
   attttcctgt ctcggccctg ccaaagttct gggattacag gagccagcca ccatgcctga 336180
   cctctttttt ctattatttt tttttaagtt gatcatgacc caccaaattg attcaatcat 336240
   caattaaagt gagatacaac ttgagtttgg aaaacacttg tctggcttca atactcaggc 336300
   agtattgcca ggacatctca ttttcatcac atttacagtt tgcagccctg tgtatgtttc 336360
   attccagccc ctcagtcagt ctgttagaga ttatcgcata tgttatcctc attttataaa 336420
   tgaagatatc aaggctcagg gatgtaaaat ggtcttgcca agcctcccca gtagtcagaa 336480
   aacgtggagt cagagctttg attccaacct tctgactgta tgaatgctgc tttggagacc 336540
   cagtaactcc tgctgcctcg aggatcaccc atttattcac ttaattaatg tatattgatg 336600
   gctcgctttg gtcaggcatg gtgctgggct gttggtacac agcggtaaac aaattttaca 336660
   tggctcctgg atttgtggaa cgttcagtca catttcgcat tagtccattt ctttcagagt 336720
   gagaccccga acctttgtgt tagcctacgt gaccttacat gatgcgtccc tcagcaccac 336780
   cctgggctca tctcctaccc tgtccccctt cttctttctt ctccaagcac atgaggtgtg 336840
   ctctagcctc agggccttcg cacagcctgt ttctccgcct ggtaagctct cactctcaat 336900
   acctgcatag ctgctttcgt catctccttc aagtctttgc tgaaggatcg ccttctaaat 336960
   aagattatcc tgaatacttt attgaaactg gaactccttt ctcccatctc atccagctgg 337020
   tactcccaat cctccatact ttgctctgtt tttcccccgt ggcacttttt aatcttttag 337080
   ccttctattt ttatttttat ttatttattt attttttgag acagagtctc actctgttgc 337140
   ccaggctgga gtgcagtggt gtgatcttgg ctcactgcaa cctccacctc ccaggttcaa 337200
   gcaactctgc tgcaccagcc tctcgagtag ctgggactac aggcgcacac catcatgccc 337260
   agctaatttt tgtatttata ttttagtaga gacagggttt cactatgtta gccaggctgg 337320
   tctcgagctc ctgacctcag gagatctgcc cgcctcagtc tcccaaagtg ctgggattac 337380
   aggcacgagc cgctgtgccc ggctatcttt taatattcta tataatttgt ttattatgtt 337440
   tagcatttat tgccttttct gtgtctactt gctagaatga aagacaaagg ccaggaattt 337500
   ttgttggttt tgttcactgc tctatcctca gcacccatct ataacctggt acctagtagg 337560
   actctgtaaa tttgttttta cttgaataga tagatgaaat aaataaccat caagttcaaa 337620
   atataattaa caaattatga tgtggtcaga gattaaaaaa agaaaagaaa agaaaaaagc 337680
   ccacaggctt tcatgaaaga gtataacggc agacctaatt tagattgggt gggttagagg 337740
   aagtgacttt tttaggctga gaacataaga gggacttcag ttcccaaatt cgttagcgca 337800
   gactctgcca ttgcaagcca aaggaacgta gcgtaaagga gagatttcac ccaagcagaa 337860
   gtctctaaga agagataaag tgattcattc attcatttag caaacaccct ctgatggaag 337920
   gagagaaacg agtcacatat tggtgttgcc caagggccaa aacacacatc tgacttattt 337980
   aattccagca acatcactga ggttctacta ttatctcctc ttgacacaca aggaaaccca 338040
   ggtgttgaat aacttctcca aatacatcca gctattaaga gacagagctg gatttccaaa 338100
   tgaagtatct ttggcaccaa agcttgtgcc ctttccctct ctgggcacct gctatgtgct 338160
   agttgtgcta tcagaaacag ggcttacaca gccaacaagg gcatagcccc tgacttgaag 338220
   aagttcacaa tatcccttga aagacatgct gaaaaacttc cctgattagg acacagattt 338280
   ttttttgtcc ccagttcttt tacttctgga catgccttcc tagattactg tttttcaaac 338340
   cttgataatt catctttttg tttttgatag ccactatgtt atctattggt gagtacctca 338400
   aaattcagtg gcttaaaaca ataagcattt attatctcag tttctgtggg gcaggaattt 338460
   gggaatgctt tacctagttg atctggctct gggtctcaaa ggaagctgca ctcagattgc 338520
   cagctggaat tttagtcatc tgaagccttg tctcgagtga aggatctgtt tccaaggtga 338580
   ttctctcaca ggactggagt tggtgctggc tgctggcagg aggcctcagt tctttgccaa 338640
   atgtaccttc ccatagtact gcttgagcat cctcatggca tggcggcagg cttcccaaaa 338700
   agcaagtgat ctgagagaga gcagggtgga agttgtaggt cttttacgcg ctactcctgg 338760
   aagtcattca ttgtcatttt tgcagtatcc aatgggttac acaggtcagt cctattcatt 338820
   aggggaaggt actacacaag aacataaata ccaagagact gaaaaatcac tgtgggtggg 338880
   gggagcatca taaaggctgg ctactacagc tatctatcac ctgtaacatt atttacttaa 338940
   tattttccac tttttgttaa aatcagcctt atcctaaaca ataatatcag ttaagtcaca 339000
   agattaatat gctaattgta ttttccagca aaaacaaata taaacttgta attattatta 339060
   ttattattat ttttgaaatg gagtctcact ctgtcaccca agctggagtg cagtggcatg 339120
   atcatggctc actgcaacat ccccctcccg ggttcaagtg agtctcctgc ctcagcctcc 339180
   cgagtagctg ggattacagg tgcctgccac cacgattcgc taatttttgt attttgtttt 339240
   ttttcagtag agatggagtt ttgccctgtt ggccaggctg gccttgaact cctgacctca 339300
   ggtgatctgc ccacctcagc ctcccaaagt gctgggatta cagctgtgag ccaccatgcc 339360
   tggccaaagt tgtaattatt aatgaaaaaa attatctgtg tatcacctat gctcacttgc 339420
   gtagttccag tgacacaaac cttacccttg gggaaacagg aagcccagca aacagggatc 339480
   tgtcagagag ccctggtagt tttcatcata aattccagct cttcccccac tgtccatttg 339540
   gagccccaga agctacggga tgctaatgtt gctgtttgct gtcctcttcc aggaccgcct 339600
   gtactttgtg atggagtacg tgaatggggg cgacctcatg tatcacatcc agcaagtcgg 339660
   ccggttcaag gagccccatg ctgtgtaagt gagaactagt gctgtgcttt ctccttggga 339720
   taaatgggta ggttagtggt tcctttgagg gtgttttttt gccaaagagg gatctttaaa 339780
   cacctcgggc atctttttga aaaaatattt agccagggaa gaaaggtgtt ctgcttaata 339840
   tgagatactt cttttttttc tgacaccagc taagaacctg aagctcagac agacaccatg 339900
   gctcatacct gtaatctcag tgttttggga ggctgaggtt ggaggatcac ttgagctcag 339960
   gagttagagg ccagcctggg caccatgacg agatctcgtc tctataaaaa ataaaaaact 340020
   tagctgggca tggtggcgca cagctgtagt ctctcctaaa aataaaacaa aactcaaagc 340080
   tctaaaacca cagctattta tttatttcta attcatgtcc ctgactgctt agaaaggccc 340140
   tatatcttcc ttggggatta tcattgtaga ttttatgtgt ccatttgcta atatttattg 340200
   agaacttact ataagccagg ccctaagtgc ttaacaaaca tcgtctcact taaccttgag 340260
   aataactctg ggagggagat gctaccatcc ctgctttaca aacaaggaaa tggagatgca 340320
   aggttttgaa agcttgccca aaatcatgga gctagtaaga agcagttccc aaattctgac 340380
   cccggttttt ctttgcccat gttctttatc ttacaccact ctccgtctta gatgccttca 340440
   ctccatgggg gtgaaggatc aaggtgctcg gtggcaatgg tctgtggttc ctgtgatggg 340500
   gagactgatt ctgggttgtt ggaaatgtgt ttcctctgct gagtcaattc catccccttc 340560
   ggttcaaaag gcagctacag tctcttcttt ttagaacaca gatttcatca tgtcattccc 340620
   ctgcttagga acatggcaag ttataggaat tagcaatgat aacctctcct ggggagtttt 340680
   ctaagatctt attgcctctc cagagagccc ccagagctca tctagaaccc caccttgtca 340740
   tactagaggc ttggtggctc ctacccagtt cagcttgccc cctctcctcc cacctggaca 340800
   ggttgtgagc actatgttct gataggattt gcattattct tgtgcttcca aatctagcca 340860
   tttgtatctc ctggcatgac tctaatagga ctagctttgc ttttacttct aacatctaat 340920
   tgaccacagg ctttaattcc attgtcacac aagacatgtc aagacacttt acatagttcc 340980
   ccaaagccct gcccaacctt tgtggcataa ctgggcttcc cagttctctt gaaacctctt 341040
   tctttctgtg ctgaccttca ataagaggtt acactttaga cagatgttgt ttcttattcc 341100
   agctgcgcca ttcattcact atataacttg cccaagttaa tctctctaac cctcagtttc 341160
   ctcatttgag gaatggggag atactagtag gacctacctt acaggattgt tattaggatt 341220
   aagtcaggta atgcctaaaa tggtgctggg gagatggtaa accttcaaca gatgttagct 341280
   gctattgtta agataattta acttgctata tagtctgaaa gtataattct ctcttttttg 341340
   ggggggtggg ggacagggtc tcactctgtt gctcagactg gaatgcattg gtcctgacca 341400
   tagctcactg cagccttgat cacctgggat caagcgatcc tcttgcctca gccttccgag 341460
   tagctgggat cacaggcatg tgccaccaca cccagttatt ttttttcttt tatttttagt 341520
   agagacaagg tctcactatg ttgcccagct agtgaaacta taatccttca tccagttcat 341580
   catatcccat gtccccttct ccactataca attgctgaac ttctgcctac ccccagtttg 341640
   tccttaaggc aaccccatca ccctctaaac caccctttga atccatctgt actttcagca 341700
   ctttatgagc tatatagctg accatccatg ggttctttaa tgtcctacac ctagttcaat 341760
   attgtattga attctgagca ttgccaagca tatggtgtcc ttgagtttct ccatcgcttt 341820
   tttttttttt ttaatttcag attttacgct gcagaaattg ccatcggtct gttcttctta 341880
   cagagtaagg gcatcattta ccggtaagtg aacactgctg tactttccat ctcttcatct 341940
   ccctcagctc ctccctgccc tgcctctttc ttcagctgct tttaaaatta gaatccgcgg 342000
   tgggggagga atcctccaga actaacttgg gcatgtgttc tgccaggcag catcgcccac 342060
   tgccctggca aagtttgggc agctctatat ggtggtagaa aatctcggtg gactgtgtca 342120
   ccaatgatgg ttcagagcct caaacaggag actgtttgct ctaagaggag actcacagag 342180
   aggacctcag tacttctttc tcttgcttgt ggaccctaca tttgaggagc aggcaaagaa 342240
   ttttggctgg cagaatgtga tggatcacat tattatgaaa cattttttat ttatttattt 342300
   ttttctgcta tgtaagtaca acttggaaat ggatctctga gtggctgccc tgaaagacag 342360
   gattaagttc aggacttggc atttgctggt ttttggagga gcaaatggat gatcatagct 342420
   gcatgagtca taccatttta tatttcatgc aaggcagacg attgcacccg aggctgctat 342480
   ttgtcagcaa gtccagggcg tacaattctc cttgtactac attgatgcaa atggcatagc 342540
   cagggcaggc ttgggctggt ttgttatgca agagaggctg caaggtatga ggcctccaaa 342600
   tagcatttgg catgggtggg ggtctatttc aaagattatg aagaaccaca agagtctctt 342660
   ccattcatgt gtttgacaca catttatcaa atcctactat attctaggca ctcttctggg 342720
   tggtgggcat acagaagtga ataaaacaga tgaaacccct tgcccccgtg gagtttatct 342780
   ttctcatggt gggagttaga taagtgaatc ataaaaacat agtctgccaa gtagtgataa 342840
   gtactatgga gataaatcaa gcaggggagg gtgctggtgt acactgggtg ttggagggtt 342900
   ctctctcatt ctgtagagag catgatgatg agggcctgga gagttagggg tgacttgaga 342960
   gtcctgggcc ttttgcctta actgcgggat atgggattaa aaggcatgag gggcctgggc 343020
   acagtagcta gcacctgtaa tcccaacgct ttgggaggct gaggcaggag gggtgcttca 343080
   agctaggagc tccagaccag cctaagcaac atagtgaggc ccacatctct aaaaataaaa 343140
   taaaattagc caggtgtgat ggtgcatacc tatagaccta gctactcagg aggctgaggt 343200
   gggaggatca attgagccca ggaattcaag gatgcaatga gctgtgatca tactactgca 343260
   ttccagcctg cgtgacacag caagaccctg tctcaaaaaa aaaaaaaaaa aaaaaaggca 343320
   tgttaagatt agccctggta agtacaaggc agatagcgga ggccaggcta tgggatgctg 343380
   gtaggatgga gaaatgggaa gtggggacct tgtcagaggc tttcagagtc ctggggcttt 343440
   tagtcacacc tgaagtggtg atatggaatc tataggggga aggatgatct tattctgagg 343500
   gcacaaagtc cccgccattc ctactttctc ctaatgatgg agggaagaag gtcaagaaga 343560
   ggagtggttt tacttggcat gtgggtgatc ctagtgaaaa tgtaaaaata gagagaagaa 343620
   gaagaattta gaagctgagg caagaggatc acttgagccc aggagttcga aactgcagtg 343680
   agctatgatt gtgccactgc actccagcct gggcaacaga gcaagaccat gtctctaaaa 343740
   aaagaagaat ttaaatttta aatagtatga tcattctgcc catgcccatg tttgccctca 343800
   atgaacacgt tcactatgtg tgcatgaaat ggcacatgga aatcctctaa ttcccttagt 343860
   ttctcttttg actctcatag tgacaacatc tttttgtgct gaatattatt ctggttttga 343920
   aaggtgcaca tgaggctggg cgcagtggct catgcctgta aacccaacac atagggaggc 343980
   cgaggcaggc ggattgcctg aggtcaggag ttggtgacca gcctggctaa catggtgaaa 344040
   cctcatctct actaaaaaat acgaatatta gctgggtgtg gttgcacatg tctgtaatcc 344100
   cagccactcg ggaggctgag gcaggagaat tgcttgaacc cgggaggcag aggttgcagt 344160
   gagctgagat caccccgttg cactccaacc tgggcaacaa gaatgaaact ccatttcaaa 344220
   aaaaaaaaaa aaaagtacac atgatccgcc atatgacatg atccctaaat tcaagccgat 344280
   gacttcagct ggtgcttaac caaagagaca ggcatcatgg gtgatttttc actctacatg 344340
   attttcaact tgggtgctga cattaggacc taactcctat gtgggatgtg cctctactaa 344400
   aatagaacca actgggttct tgagaagatt aaatatggaa ctacaagttt attggaagaa 344460
   aatccactcc cattgtagga aacaggagag gaaataatta gtgttccggt caattgagtt 344520
   gtgattcagg gctagtttct aatcaatcag gaagccgcac tgggatctgg gatccatgga 344580
   tccggctcac tggccaggca ggtggtaaaa ttaaaagatt agcatttcag gtgctaagat 344640
   gatacctatt ccaaagcccc ttttcctaaa ctaactacac actctccctt tcatcttcca 344700
   ctattaatat ggtctttaaa ggggtattta tcaatctttt aagccagtct gcatctgaac 344760
   ccttcttttg tgtatgtgga agaacagagt caatcttcta ctataaataa tgaaaatgac 344820
   agatactcat ttcacagccc acctgtagcc agggcaggca cacaacccaa gctctgacag 344880
   ttggtcatac ttactcaaga ccttgaatca gaaactagcg gccaaggaag cagaaagcat 344940
   agaatcaaga gtgaccatag taattatcag caactacttt ccagaggcaa gagaagaggg 345000
   tcttaatagc aataggcaga gtccagcatt agggcttcgt ggaacaaggg gtgatgtgtg 345060
   tgtcccatgg cagcttgggt ggtggtgtca ctggacaagc cctgagttgc agttaggcat 345120
   tgtttcaggc tgtatagact cttaatctgg ttcttcagtc tcctcaaaga ttctatgagc 345180
   tccccaatat cctttgatga attccttttc aggtaaaaat catcatagtt ggtttttgtt 345240
   gctcaccatc gagaacgctg atggaaatag agttcttgaa attgatagtc aaatggaaat 345300
   ggtaaaaaat aaattacttg acaaaagatg tatggtttta tcatttcaca gagattgatt 345360
   tcatagtggt aagatctttt aagatgttac aaatcactac ctccttcatc taaatctcta 345420
   cttaattgaa cagttcttaa gtaaagagga tgaaggatta tgttagactt accatcttta 345480
   aggaactgga gatgttgggt tgtggggtgg gaagggaaaa agattcttcc tatcaaactt 345540
   agtgtctgat ttactgacca ctgtctctgg aatgtgtgtt ggttttgccc ttgatggttt 345600
   tctgaaaaat gaaaataatt tctccatcca ccttttgaag cagaaatgcg gatttattac 345660
   acagaacaaa agattagggt gtgatatcaa aacactgtat gtgacagtga agatgacaga 345720
   ggtgccctcc agcctcagga ctgcctggct cttggcacct gaaacttctt ttctataata 345780
   gatgaagatt ttatcacttc cctagtctac ccagctgcct gcttctgagc atgtggtcga 345840
   gctgaagggt tgtcatttgt cactattaga gaaatcacat atatcacata tagacctacc 345900
   tagtaagttt atgaaaagca aattgctgca aagcctgggt tttctgagtt ccaaactttc 345960
   acacaaatca tggtggcttg tagatgagca tgaaggctgg atatgagcag caccacggac 346020
   agcgccaaag gcatggcgtg ttgggattgg caaacatcag tgggcaagtg caagtggacc 346080
   tccccatccc tttgaataag gagaccccac agacacagat aggagatgcc aggatatagt 346140
   tttcagattg tattctttta gttggaaatg atgacagcaa ctcaattcaa agtaacttag 346200
   gttaaaagag gcatttattg gtgacataac tgaaaaatcc aggtgcagat ctgactttag 346260
   gcctggataa atgcagaggc tcaagtgacg ttatgagggc acattttatc accctccctc 346320
   taggctctgc tttctctata ttagattgct tcttttcatc tggtgacccc tggagtttct 346380
   agacttccat aataccacct gaacaacccc aatgtaaaat gcacttatcc ctttcctgat 346440
   tatgccagcc aaagtgccag cactaatact taacagcttt gattggccca gattggatca 346500
   catgcccatt cctgaatcaa tcactgtgac cagcaggatg gaatatttcc attggctggg 346560
   acaggcccac ttgaccactg ctggagccat gttacatgga agccatgtta cacccatgtt 346620
   acatggaaga attgaaggag ggtggttctt ctaagaaata ttagaatgct gtcatcacaa 346680
   aacactagaa tagacagaac cacagatgcc cactacccaa tcaagatgac cgaagggaaa 346740
   gatctgaatt aaggaataga tccccctacc tctcaggaaa tcggatttcc ccatcttcta 346800
   ttaggaccga tccatctctc agccttgctc actggtgcag aaatataaag cattgacatt 346860
   ggagtaggtg tttagggagg aggaaagtgt gttgatattg gagcaatctt gtgtgctaaa 346920
   gacctacaac tgttccccaa aatatcaagc tcttctcatc aacacagatg aaaataaaag 346980
   agaatacagc acatatcctt taggttagag ctcagatgtc accgccttag acaggtcttt 347040
   tctgactgac accctgctta aagtgacttc ctccaatcac tatcttatgt cctctttatt 347100
   ttcatcatac cacttatcag aatctgtaat aatcatgctt acagagtaat tgttatcttt 347160
   ttcccagcac tgaaatttaa tctgaagaac aggaggcttg cttatcttgt ttactgctac 347220
   atactatacc tggctccagg catagtgcct gtcacatagt aattgtgcaa catttttttt 347280
   ttttcaagac ggagtctcgc tctgtcaccc aggctggagt gcagtggcgt gatctcggct 347340
   cactgcaacc tctgcctccc aggttcaagc tattctcctg cctcagcttc ccgagtagct 347400
   gggactacag gcgcccacga ccacgcccag ctaatttttg tatttttact agagacgggg 347460
   tttcaccata ttggccaggc tggtcttgag ctcctgacct tgtgatccac ctgccttggc 347520
   ctcccgaagt gctggaatcg caggcgtgag ccaatgtgcc ctgccaaatg tattttgatt 347580
   caaagaattg atggaaactc ctgggtcatg tcctagaagg tggcccaaga gatcagggaa 347640
   cacttagcat taaatatttc tttcaatcag cttctttttt tctttgtaat ttttagagat 347700
   attttaaaaa ataataattt tctgaaaaat gtatcttctt acaaaatata aggagtcttt 347760
   aagtggcaaa ggaaaattct tggaattttc ctggatccaa gaagcgtcat ggatgctctg 347820
   ccctttgctt aactttctgt ggatctccgt cttttcagaa gtgctgtctc tttgtgtcct 347880
   tgtgagacac ttggcacagt tggctgtgta gaaaaagaaa agcacggagg cttgcggacc 347940
   tctttttatc tccctgccag actgtaagct gttggaggtc aaaggctggt ttctactgac 348000
   ctttgtgccc acacaacacc taacacagtg ttttatgcat aatgagtggc tgttggttgg 348060
   cttgaaatgc atagcgttta attaaatctc taaattcttg tgtctgccat agtgacctaa 348120
   aacttgacaa cgtgatgctc gattctgagg gacacatcaa gattgccgat tttggcatgt 348180
   gtaaggaaaa catctgggat ggggtgacaa ccaagacatt ctgtggcact ccagactaca 348240
   tcgcccccga ggtgagagct gctgggcaca cgttcacatt gcggtgatgt accctctgct 348300
   ccccagatgg ctgtgtgaga gctgggaagg gttgagggtg gtaccttgca agggaacctc 348360
   ggacagattc ttattctata ctctaaatcc tccctttgag atcacacatg caaattaatt 348420
   tagcacacat ccagaagccc aagcccaaaa tacatcaagg tttgggagaa aatttaaata 348480
   aaacccaaaa gaaccatcca acacacatca gtaaaattct tcgtcaaact gttgtggctc 348540
   acggccatgt gataccgcct aattatgaca ttgaaatgtt tgttgagaga aaaagagcat 348600
   atcccctggg ctggcatatg acatgggatc tgtgtcctgt atatgtccct cagcaggaaa 348660
   atggacaata gcccgtgatt tatttatagc atgaaatact accaggcacc aattcagaag 348720
   agtgagttag atatatatgt agatgtgaaa agatcttcaa gacacattgt tagagaaaaa 348780
   aatcaaggta cagaatgaca aatacaaggc caggtgtggc agctcgcgcc tgtaatccca 348840
   gcactttggg aggcagaggc aggtggatca cttgagccca ggagttccag accagcctgg 348900
   ccaacatggc gagacctcgt ctctacaaaa aatacaaaaa ttagccgggt gtggtggcac 348960
   atgcctgtag tcccagctac ttgggaggct gaggtaagag aatcacctga gccccaggag 349020
   gtccaggctg cagtgagccg tgattatgcc actgcactcc agcctgcctg ggtgacacag 349080
   taagaccctg tctcaaaaaa aaaaaaaaaa aaaagaagaa gaatgacaaa taacactatg 349140
   atactattta tggatgaaac agaaatacac agaacactgt tctgttttca atgagtaaaa 349200
   gcatacaaaa atctggaaga tttcaaactg attttataac actggttacc tctgaggata 349260
   agggaattgt aaagagaaat ttagctattt ctgtatactg ctgacatttt ttacaaggta 349320
   aatgtatttg ggtattactt gagtaataaa gtaattcttt aaaaataata aggctttact 349380
   cggacccagt gaagttttcc ctatgtgata tgctttggtc tattatcatt ttgttaatta 349440
   attagatagg acacaatcca taacacaact tcctgatagg ccaggggttc cataatctct 349500
   ggttctcaac tctaactgca gtttagagtc gtgagggagc ttttacaatg taggatgcag 349560
   ctggggcatg gtggctcact cctgtaatct cagcactttg ggagcctgag gcaagaagat 349620
   cgcttgaggg tagaagtttg agaccagact ggacaacata gtgagaccct aatctctaca 349680
   aaaaaaatta aaaattcgcc aggtgcacta ggtcacccct gctgtcctga ctactcagga 349740
   ggctaaggtg ggaggatcac ttgagcccag caggtagagg ctgcagtaag cagtgatcat 349800
   gccactgcac cccaccctgg gcgactgagc gagactctgt ctcaaaaaaa gaaagaaaga 349860
   aagaaagaaa gtgtttccac acttttaaag tacttcaagt cttatttgat cctcatgaca 349920
   agccaacaag ttaatccttt tacaaaggaa actagagcct gggatgtgac agcagttgcc 349980
   gaaggtgatg tggttagctc atgacaaagc ctgacctgct gcttatccct cccatctgtg 350040
   ggctggaggg gaggcaactt caccagccac acagttgagg gtttcttgtg cttgcctgaa 350100
   tcacgaccag cgaggaactt ctgagaacca cagatcctgg gcctccacct gcagagattc 350160
   taatgggatg atgcttgggc atcccacatt gtttcttgtg tgtgtgtgtg tgtgtgtgtg 350220
   tgtgtttgag acagggtctc actctgtcta ctcactgcaa gctctgcctc ctggattcat 350280
   gccattctcc tgcctcagcc tcccgagtag ctgggactac aggtgcccat caccacgccc 350340
   agctaatttt ttttgtattt ttttagtaga gacggggttt caccgtgtta gccaggatgg 350400
   tcgcgatctc ctgacctcgt gatccgcccg cctcggcctc ccaaagtgga aaacacgttc 350460
   aatgagaagt gttctcctgg aatgctttcc cttccttgtc ttgagggtta cttctcagaa 350520
   tgcatttcat tcattcataa ttgaccagta aaattgtata catttatcat gtgcaacatg 350580
   ttgttctgaa atatgcatac attgtggaat ggctaagttg agttaattaa catatttatt 350640
   gtctcacata cttatttttt tatggtaaaa acacttaaaa atccctctta gcaattttca 350700
   agaatacaat agattgttat taactatggt tactgtgtta cacgataagt ctcttgaatt 350760
   tattcctgct acttaactga aattttgtat cctgtaatca acatctctgc atttctgatt 350820
   ttggaataga aatcaaagtg ggagatagag atcaagaaac aaacattcac tttatagcca 350880
   agtttgctca aatacccttt ttgagttaaa caagcaataa ctgaattata aaacattcta 350940
   ggattagtta acattttgag ggcataatat gtgaaagcca ccgagtatct cattcatcct 351000
   cacataactt catgatatag agattataga agttaagtaa cttctcctgt ggtcagacag 351060
   ccaaccggta gacagcagtc aggattcaaa gtcagactgc ctaaccctca gacctcagcc 351120
   tttagccttt gctgtattac ctcttaacct ctgacatggt tagttttaca tccctgcata 351180
   ctgaaagatg gacaatcaca tagctcttct agacttttcc atgttcttgc agtttgggaa 351240
   tatgaattaa aaacttcaca accatatata ccatatgata caccaattct acatctagga 351300
   gtttatctta aggaaatact tggacagatg tccaaatgtc taattacaag aatatatgtt 351360
   acagcattgg aagaaaccta atcaactgga aatttataag atagattttg gtatatccat 351420
   tcaacggaat gctatgcaca gataaaaatt gattatgttc atatacaaat attaaattag 351480
   caaggaaaac ttaagtgaat aactggcttt attaaagatc tattgcccat aatcccagtg 351540
   ctttgggagg ccaagatggg agctttgctt gagtttgaga tcagtctggg caatatagag 351600
   agaccccatc cctacaaaaa attaaataat tagcatggtg gtgagcacct gtagtcccaa 351660
   ctacacagga ggctgaggtg cgaggattgc ttgagcccag gaggccaagg ctgtgagctg 351720
   tgagctatga tctcaccact gcactccagc ctgggtgaca aaaagagaca ctatctcaaa 351780
   aaaaaaagaa aagaaaaaaa gaaaaaaaat cgtccaaata ctaaagtgtt tatgtaaagc 351840
   acacacatac acacagagtt taatatgtat tcagagaaaa gagtatggaa tgaagtagac 351900
   caaatattag tcataatttt tctcttaata aaggattatg ggtgattttc atgttattat 351960
   ctcagctgag ctatctttta taaagaaata ttaaaatagt gacaaatgtt gttatctctt 352020
   aaacgttttt atgtttaatt atgttttaaa aacatgtaac gtaaaattta ctattttaac 352080
   cagttctaag tatacaattt agtagtgtta agtatattta cattattgtg taaatgttgt 352140
   aacttttaca gaggagtaaa gacagttttt ccttttaaaa attccatgct cactgttttg 352200
   aaaataataa taacaataca ccttacttag tgcttcgaaa aggcaatgtg acagccacag 352260
   gtgtttgccc agctgcaggt gcctctgcaa agcatgtctg tcattccctg aatagctaat 352320
   ataaagaaac agttcaggtg ggcactggcc ttcttgtaaa agaagagtga aatctgtctc 352380
   cagaagaaac atgaactgca aacctccctg atagggtgcc ttctcttttc tagataattg 352440
   cttatcagcc ctatgggaag tccgtggatt ggtgggcatt tggagtcctg ctgtatgaaa 352500
   tgttggctgg gcaggtaatt gaattttaag tgatcgataa gagaagtcct ccctaccccc 352560
   atccacatgg tttctttagg agaatgcatg tttggtaaca gctcataaat tggaacctct 352620
   atgactttcc cggccttggg gtcatacaag tctgtggtca tttgaaagcc tgggtgtgtg 352680
   gcttcactgt gggccccagg gaggagggtc tgccagttga ggggagctag ggggagggtt 352740
   caagcaggga ttcttctcct cccaccctcc cctgtcatac aggcaccctt tgaaggggag 352800
   gatgaagatg aactcttcca atccatcatg gaacacaacg tagcctatcc caagtctatg 352860
   tccaaggaag ctgtggccat ctgcaaaggg gtaagtgcat ctcttaaagc tgtgaccagg 352920
   accaccacat aaaggcatgt gggctgtacc ttgcccaaga acaccccacc aggggggaac 352980
   acagcctgaa ctccaccctt cactccattt gccaatatga gaaccctgat gtggagctgc 353040
   tacctcccgg aggagagaga gctttttttc tgatttgcac agaagtgctg tagagagctg 353100
   cctgcagctg cgtcttgctg ctggctgggc tgcagtctct gaaccagcac aggggaatgc 353160
   ttattgaatg aatgaggtag aaagcacaga gattttgtca aagagctgga agtcatggga 353220
   aagggcacaa gaggtctttt gcacaactca ggctatcttg ccctgctctg ctgtatgtgt 353280
   ttagttaata ctaagagcgt gcaacttttg ttctaaaaaa taccacgccg tgttgaggtt 353340
   aaggaattct ctagataagc agactgaaat tcaatgtact caaaatccca ctgtctcctc 353400
   tccccagaca cacacacaaa tggctaaaag ggcatgcacg cttggacatc tacggtttat 353460
   gcacagggca cctgtgaaga aagcaggatg tgtgtttatg gggaaggatt gtttgttttg 353520
   catcttgtgt tggaccaggg catactcaca gtgacttctg tgagatccta aagatcagcg 353580
   accctcacct ttggagccat cagaataacc cgaagtgcct taccagaatg cacatgcttc 353640
   tgccttaccc cagagattaa gtctgggtat gagtattttg gaaaagtctc catttggctt 353700
   gcagaccact gcctttaaaa atactgtcca aatgaagtga tttttcaact ctgatggaag 353760
   cctgaaagca atagaattag acgcccagaa aatatgttct acacataata tactaataaa 353820
   ggatttgctc tccctccacc cagtggtaca ggttagaaca tggccgtgcc ttaatcagtg 353880
   ggttcccacc tgtgtgtctg aataaaccag ggctgccagt cctagatctg tgggatgctt 353940
   gcagactttt catgccctac tttgttcttc ctgaccccag ccagatgctg gatgcactgt 354000
   cttgggtgaa ggaaagctgg gaaactcagg caggccaatc tgaggcttgg cccagtttgg 354060
   aatagctggg ccagcctctc agcctaaaag cgccttgtca cactgcctaa attcccactc 354120
   tcaatgaatc acctaaggtt tgcacctcag ctgcataggc taagatgcat gtatccctgg 354180
   ggaaaccacc tgtgaattag tctcaatccc gaacatctaa agtgaattgc ttgaaaatat 354240
   tttctttgag ggtgtgaggc agggagaaag aaagtgaaga atagagaggg gagtcccaga 354300
   ggtgcattag gaacccaagc aaagcccata aagaaaaaaa aggcacacac gaaggcctcc 354360
   acaggccccc gtagacaagt tattgaaggg tgggggcggg tagagagaag gtggtacctg 354420
   gagcttttag tttgtttaat aattaccagc atataaggat ccagagattt ctcaaaaaca 354480
   ttcagctttc aggtttctct tgaaaaatca gatcttgcca cattggcctg tcattcttac 354540
   aaagcaccag tcagctggat tggaaaccat ggccctctgt agttggagct tgccctctgt 354600
   aattctcaga cgttctcatt tggccaattc tctccaagtt caaatcctgg ttcaggcatt 354660
   aattgccgca ggaccttggg gaagttgttt gacggcatct gcagattggg gataataata 354720
   gcactccaca ttgggttctt tgatgactaa acagctgaat actgtaaatc acttagaata 354780
   gaggctggca ctctgctcca agccccttgg aagttattat tactattact agtgctgacg 354840
   ctattgtgtt atctgccagg ttcctcttgg catttgagtt tgcatcccct ggaattgcaa 354900
   cttttaaagt aggggatttt ggagatcaag ggaccctttg caaatcttga cccttctact 354960
   ttctaggcac aaggtgtttt tgttttgttt tgttttgttt tgttttgttt tgttttgttt 355020
   tatcaatgtc tcgctctgtc gcccaggcta gagtgccgtg gagtgatcat ggctcactgc 355080
   agccttgatc tcccaggctc aagggatcct cctgcctctg cctcccaaag tgctgggatt 355140
   acaggcatgg gccactgcac ccagcctagg tgcaagatct taagccactt cctttgcatc 355200
   tctgggcttc tgtttatttt aaaaaatgga atagtagtac tcattgaaaa gagtttttga 355260
   gagcattaaa tgagataaca taagagaaag ctccttgcat gatgtgggca catagtaggt 355320
   gtttaataca tccacagggt cctccccttc acacgcccgc cctgcctgga cagctggggg 355380
   ttctaaatgg gtcatggagg aaagagcatt ttccaggtca gctgccttca gctcctgctc 355440
   caattagcag tttccctggt agagccgttt tctattaatt caggacccat aaccttcatg 355500
   attaactggg gtgactctct tactattgag tttggggacc attattaatt gagtgggctg 355560
   agtccaatgg ggctcttcct tcaaggaggg tactccaaaa acgagtgatg ctcaagcaaa 355620
   cagccaaatg cttcctctgg aacagcgagc cccaggggat acgtctggga ggtgatcaga 355680
   gattggaaca gtgattaaat ccagctgtct acatggcttc gggcacatta gagcagagtg 355740
   cgggaactgg gcagagactc cgaattaatg ctggatgttt ttgcagtacc ttttctttgc 355800
   ttggcatcct cctgcttaga cagaaattca gttgagcttt tgaaaacagt cacctgttta 355860
   attgaagcag tgtcctttat atgtccattc ccttaaaggc ccgattttct actaaaaaaa 355920
   aaaaattagt aaaaccttac tcagatggct attcccactt tgaacaaact tgaagcaaac 355980
   agaagcccaa atagacagga ccttagggtg aaaaccagca gacactgaac agctgagact 356040
   gaacataaag ctataagagt cctgagccag gcagagctgt tcaaagcctt gccctgctcc 356100
   ttagctgttt gaccttaggc agatgacttt acttctctgc acccacatcc tttacctgga 356160
   aaatggggga taatagttgc tgaggcctta gagtattgtt gcaaagttta aagagtaatg 356220
   ggtaatggcc gggaagcctt tagcacggag cctgaacgca tatgatgttg tcatactcag 356280
   tacacggaag ccgttattga tgttattatc agtgtgaacc aaaacctcac tgccctttct 356340
   ttcctcccat cctaatctta cacaaatgtc aagccttggc tcaatttcct caccctccat 356400
   aaacctctcc tgacaaagcc aggccacaaa aattcctgag aaaaccacgt gtgaaatgca 356460
   ctcccatttt tcttaaaagc ccaagtttta gagctaggca gaggtggagt ctgcacccag 356520
   ctcttactga ctctaccact tcctctctgg agtccttact gttcctcaac ttctctgagt 356580
   gttcattgcc tttttattca aatgggaaaa atagtatact cacctcattg ggttattatg 356640
   atgatcaggg aagctcatgc atgtaaataa agaagtagca gctcggcgcg gtggctcttg 356700
   cctgtaatcc cagcactttg ggaggctgag gcaggcagat catgaggtca ggagatcgag 356760
   accatcctgg ctaacatggt gaaaccccgt ctctactaaa aatacaagaa aattattagc 356820
   tgggcatggt ggtgggcgcc tgtaatccca gctactcggg aggctgaggc aggagaatgg 356880
   cgtgaacccg ggaggtggag cttgcagtga cccgagatcg cgcctctgca ctccagcctg 356940
   ggtgacagag tgagactccg tctcaaaaaa aaaaaaaaaa aaaaagaagt agctagctta 357000
   gaatgtaagt gctcagttaa tttccccacc ctggaggata cactttatat gacaagtaca 357060
   tgcttgtgtg tgagaccaca aattatcttg cgagggggca acaggctgag tgactgtcca 357120
   acagtgtagg aatgaatttc tgggttcatt ccttccccat catcagcaag tcctgggcag 357180
   aagattctgc catcatgggc ttctcaatgt gatgtcaact ctggaaagct tttggacttg 357240
   tatgcttctt tctttttttt taattaatca ccaactcttc tgtctttact ccctgtgcat 357300
   ttgctcaagt cttgagatca gagatatttt catgctgctt tggactcatt tacaagagga 357360
   agcctgtctg gtttcatctt ggcgcatcta ttcatcttgt tatttagcac tcagagaccc 357420
   aaagatggag ggttttgata attaagtaat ttaaacggaa caccaaagaa acctcagcgg 357480
   ctttgtgtgt aatgtatgat acaagggaaa tgagctgggc taggttttga ctatttaatg 357540
   ggctttcaga aagtaattac ttgaaacttg ggcccctttg actaatactt tttcttctgt 357600
   tttgtcattt ttgcaagtgc tttactgaga aatgcaaatg gtatctgaat gaatgtagcc 357660
   tttgcttttg tcaaactcca gttttcttgt tgtgggagga gggaagtttg aagatattga 357720
   aactgtccta attaaatgca ctggtaattg cacaaaactt attcttaaac caaaatggat 357780
   aaatggatca tgtggcattt attttttctt ttctgttttt ttttttttgg ttgttttttt 357840
   ggttttgttt tttttttttg agatggagtc tctctctgtc gccagactgt agagtgcagt 357900
   ggcgcgatct cagctcactg caacctccgc ttcccgggtt caacaattct cctgcctcag 357960
   cctcccaagt agctgggact acaggtgtgt gccaccatac ccagctaatt tttgtatttt 358020
   tagtagagac agggtttcac catgttggcc aggatggtct caatctcttg accttgtgat 358080
   tcgcccgcct aggcctccca aagtgctggg attacaggtg tgagccactg tgcctggccc 358140
   tcatgtggca tttctaactt gaagttcacg gccacattca ggcttcgttt tgattaacca 358200
   taacagatga ttggcagtgg gtcctattat caggtcctct cctgcaaatt cctggctggg 358260
   attttttgct gggataaaaa gcaaaaaaca aaaatgagtt gagattcttc atttgcgctt 358320
   tctttcctaa tgcattggag taataatttc tgagtgtctt acatttcctc ttctgaaact 358380
   cagcaaattc aatgtttttc tttctctcga agctgatgac caaacaccca ggcaaacgtc 358440
   tgggttgtgg acctgaaggc gaacgtgata tcaaagagca tgcatttttc cggtatattg 358500
   attgggagaa acttgaacgc aaagagatcc agccccctta taagccaaaa gctgtaagta 358560
   gcccattctc tctgactgcc gggtattcac acacaaggta ttcttgcctg tgcctcatgt 358620
   tttccaaatg ctccctgagg ggtagacgtc aatgtgtcta ctggaacatg ggtgtatgta 358680
   ttcacacata tgcacaaaat cactggttac taagatggac attttccatt ggcccagctt 358740
   agtctctata aagatggaaa cgacgatgcc tattgaattt ttgtttcttc gattcaatta 358800
   ttgtaactgt cttagctgac aactttctga gaatggttct tgggtgagca gagttcagat 358860
   ccatttttgg actgtgataa aggatctaat tttgccctca ttctttctct tggttcaact 358920
   gtgtcatttt ttcttcatac tgacattgaa aaggtcatgt tgtttatctc aacagtattt 358980
   aaatttaatt ctgaaacata cactatgtct gagtcagtaa ataagatggc agttcagcag 359040
   ccagctccca cccaaggcaa aacagagtga agcagagact gaagtcctct tgaactgaag 359100
   accccctccg ggagggtttg ggtgtggaga tggctttgac ataagtttta taatgcaaga 359160
   ataaaactct cacatttccc acagtccatg taccaacagg aaggttttct atccccaaag 359220
   actcagcagg aagtacttgt cggcatcagg aaagaagagt ttacacctga attaatcaaa 359280
   ctgttattct gctgggagga tatttccttg tgaaattaaa tagagggcag acttggtgac 359340
   tcaatgatgg gttgtaaaac tactgactga ggctgaagag tgaacataaa caccatcatt 359400
   gaaacctaac gttgtaaggg tttgagtgtt aagatgaaga agataataat ggtagttatt 359460
   ttgatactag tattgttatt catttcttga gcacttacgt gctaggcacg taatgataat 359520
   agttttgaca tcagagggtt gttatgatgc ataaagcact ttgtacaagc ctggtacaga 359580
   ggaggcactc acagagagct gatcatggtt ttcgttagct gtgtcataca ttaattaatg 359640
   tcccggtgta gagtctttga ggggcagaat cctgctgctg ctattcctta agataacatc 359700
   tgtgtgtaca ttggggtcga agagggcccc acatgtcatg agcagcatca cagcttttat 359760
   gggaccattt ggaaaaagat gaaaaggtaa cctggaaagg aagagtgagc tctttgttca 359820
   ggaaacaggg aactcagagg tgagatgcat aactcatatg gtttttgtgg ggttatttat 359880
   gagatcttga aatgtggacc tccctttcct ctcttggcca gatgcacacg ctcccatccc 359940
   caccagcata gaacccagcg gacatctggg ggtcatgtta tagctgtagt gcagtggttt 360000
   gcaaagtgtg ctgtctatca gtatgcagta tcagtatgca gtgtcagagc cagcggtatt 360060
   gacatctcct gagcacttta ggaatgaagt tcttggtctc caccccagac ctcctgaatc 360120
   agacactcta ggtgtggggc ccagtgttct gtgtttaata ggctctccag gtgacttgat 360180
   gagctctcaa ggttgagaat cactgctgtg gagtttggca ttaggagctc agccagggaa 360240
   gctaggcaga gaccagaggg aggtagcatg gagaatcatc tccttttttt tcttttttta 360300
   aagacagggt cttcttctgt tacccaggtt ggagtgcagt agcgcgatca tagcttactg 360360
   caacctcgac ctccagggct catgcgatgc tctcacctca gcctcctgag tagctgggac 360420
   tacaggagtg taccaccatg tctgcctaat tttttatttt tatttttaaa taaatggggt 360480
   cttggccggg tgcggtgcct cacgcctata atctcagcac tttgggaagc cgaggtgggt 360540
   ggatcacctg agctcaggag ttcgagatca gcctggccaa cgtggtaaaa ccccatctct 360600
   actaaaaaag tacaaaaaat agctaggtgt gatggtgggt gcctataatc ccagctattt 360660
   gtgaggctga ggcaggagaa tcacttgaac cgaggaggtg gaggttgcag tgagctgaga 360720
   ttgcgccact gcactccagc ctgggcgaca gagtgagact ccatctcaaa taaataaata 360780
   aataaataaa taaataaata aataaataaa taaataaata aatgtggtct tgccatgttg 360840
   cccaggctag tctcaaattc ctgggctcaa gtgatcctcc aaaaacctca gcctcccaaa 360900
   gtactaggat tacaggtgtg agccactgca cccagccaag agaatcatct ctttaagtgg 360960
   aagacactgg tcctccttct agtcagggca tggggtgatg gcttccagaa gaggtcacag 361020
   actcagatgc ctacagaggc caagcagata atatgaatga gggaagaggg ctggtgagat 361080
   agaagagaaa gtgttggaga tcgatgcaag ccagagagca ggtgatctac ctaacaggct 361140
   cagctgatgt tcacctcccg atggcctgcc ccgcaggatg cagcctcctc agggctgctc 361200
   catcttcatg gtatttgggg ctagaactcg gtgctgtggg atgcagcaga cctgtgagtt 361260
   gattgacaga tcctgttaca gtcagtgtag tatgtaggcc ctgtgtagta agggcttagt 361320
   gaaagggtgt ataaagttaa gatgtttata taaaatctca caattttggc tgggtatagt 361380
   ggctcacacc cgtaatccca gcactttggg aggctgagcc gggcagatca cctgaggtca 361440
   ggagttcgag accagcctgg ccaacatagt gaaaccccat ctgtactaaa aatacaaaaa 361500
   ttagctagat gcgatggcat gcacctgtag tcccaactac ttgggaggct gaggatagag 361560
   aatcgcttga acctggaggc agaggttgca gtgagctgag attgtgccat tgcactctag 361620
   cctgggtgac agagcaagac tccgtctcaa aaacaaacaa acaaacaaaa aaacaaaaaa 361680
   caacaaaaaa aaaacctcac aatttctttt atatatcaga acaaattttt gaaaattaaa 361740
   cacagtttgg ggcaaataaa acacacccac acccatatgt atcccatgca ttgttggttt 361800
   atagcctcag gggtagtgat ggttcaaagt caggttgcta tttagccagc atctgttagc 361860
   aatggttatt tacaaccaga gtccattttg accggttaaa tattttgaat agccctgcta 361920
   agcagtaatg aatataagag gtctagcctc gtgcctggct catagaaagc actcaggaaa 361980
   aggtagctgt tattatcatt gataatataa aaagattaca atacttagtc attatcaacg 362040
   agatccctca actgttctgc atttgatatg atttcccact gacctgtgtc ttgaattcct 362100
   ccatgtgggt ttaacaatga ctttcaaagt acagtgatca ggctgtgcta catacttcaa 362160
   agtgttctgg taccagctct aaaggagcag gtgctacctt cagtctcctt agactgaatt 362220
   agaaataata ataaatgata cattatccag gtacagtggc tcatgcctgt aatccccaca 362280
   ctttggaagg ctgaggcagg caaattgctt gagcccagga atttgagacc agtctgggca 362340
   acatggcaaa accccgtctc tacaaaaaat acaaacgttg gtgggcgtga tagcatccac 362400
   ctgtagtccc agctactcag gaggctgagg tgggaggatc ttgatcctgg gaggttgagg 362460
   ctgcagtgag ccatgatcat gccactgcac tctagcctgg gcaacagagc aagaccctgt 362520
   ctcagaaaaa aaaaaaaaga tacatcacct acccatttga tccagcctgc ctgtatgcct 362580
   tttacattgc aaatgcattg catgagagcc ctcctccccc caatcccagg attttatatt 362640
   tctccttcct caccccttct taactgtctg ccacttccaa aaggcccatg gtgagcgtgt 362700
   gcagagttcc atgtgccttg tgggatgtga aaaggcatgc atgtatctgt cagcgtttct 362760
   gattcatgca tgctattaaa tagagtgtgg tgagagaaaa aacaaggaac aataacagag 362820
   gattatgttc aaacatcagt gaagggtgct attattggag gttcattaaa ataactttaa 362880
   atgggttttt ctaagttgtt ctcaatgcca tcttttattg tggcaaggag ctgttcacag 362940
   agcattgttt gaaactgtat tgaaatacac tagatttggt gtattgttgc agggaacaag 363000
   agtcatttct tttttaatgt tcgcctggtt gtgtgttgct tttgctttaa aacacccaaa 363060
   tggctctctg ttgccccagt ctcgtatcta ttgctccttg gcatatatgg cttttgaggt 363120
   cttccagtgc tagatctctg tcctcttcct agctttagct cctgactcca agaacagcat 363180
   tcctgggctt ctttcagtcc ctcaaacatg cagtgccttg ttttcccttg cctgcttcat 363240
   ctgtgcagag ccatgcacct gggatgcccg aagccctttc ctatgctctc accagccccc 363300
   tgatccccac cgtgccttgc cttttccccc tcatcaaatc agtagaacat caaccgctca 363360
   gggaggctct tgttgattcc tgagtagata tctgctactg tcccatcaag aaattcatca 363420
   caggctgtaa taacatgttc aatctcatgc ctgtcttttt gactacttta agattgtgac 363480
   tttctctgtg tcaaatccca gtgcctagca tagggtttag cacattatag gtgctcagga 363540
   attcttactg aatgagtgaa tggccataga acaagatgct gtaacacagt gtgctgtgga 363600
   acataaagga gtcttttggg atatatatta gagtgcatgg tgtttttctt atgtacagca 363660
   gggtatcgca aataaattgg ttgttgtgta agttactacc ctatttataa aggactataa 363720
   tttggctgct gattttgcaa actgtcatgg catcgtgtgg ttttgcagtg tgtgctggga 363780
   gagggtgctg gtgaaggatc tgaagaggca atgcattgaa tcacaggact ttgggtagag 363840
   agatctgcag cttcctgcag cctaaccttg cacagtccaa tccagtgtta cagatgccat 363900
   gatgtagccc tgcagagtgt cttaaatttt aatgtgcatg cacgagtcac ctgggtatct 363960
   ggctaaaatt cttactcagc cagtctgcgg tggggtttca gattctgcat ttctcacaag 364020
   ctcccagggg ataggacact gtggggccag ggactccacc tttatgtagc aggggctaga 364080
   actcagtgct gcagggtgca gcaggcctgt gagttgattg gcagaacctg ttgtagcagt 364140
   gcagtaggta gaccctgtgt agtaaggact gtagtgaagg ggtatataca gttaagtata 364200
   gaatgttctg gtgaagttat tgttcagggc acacagcagg ctgttgctgg ccagcatgtg 364260
   gtaggaggaa ggtggtggtt tgggtactaa agggcagact ctgtcgctga gtgcatgatg 364320
   gagcagtggc ttgggaagaa gcgtgtgctg gttctgtcct ctggtgggga tgttgtcaga 364380
   tattgagtta tccattcagt gggtgatgag gttaggtaat gttctaggtg atggaaacat 364440
   agcactaaaa ataattaaca tccctgcctt catggagctt atattctagg gagagtgggc 364500
   aaaataataa acatagaaac aagtaaaata tacgatgtgt cagaagttga taaacattat 364560
   gcaggaagct gaagtaggga tggagattgg aagagccagg tcagggagat gtccttatga 364620
   tagagtaggt ggagaaaatg acgtttcagc aaatacagga agaagatgag ggaggtggga 364680
   gccatgtata tatctgggtg aggggggaga gtgatttggt tggtagtagc agatgcaaag 364740
   gccctgaggc aggagcttgc ctggcatggt caagggagag caaggagtgc aggtgcctga 364800
   gccaaggggt tgagggagaa aggaaaaggg ggtgtgagat cagagaggcc aaaggaaagc 364860
   acaggccact gaaggatctt tggcttttac accaactcat gagccaagaa ataacatgtt 364920
   ctgactttgg gtttttaaag gatccctctg agtgctgcag ggacaagggt gaaatcaggg 364980
   agactggttg agactcttgc cataatccag gcaaaaggtg ataggagctt gggcaggttg 365040
   gggctgtttg agaaccccca agttggcatc ctcaggtcct ccccagttcc tggcatcttg 365100
   taggcactca gtcattgtct catggagaca tgaaggcgct gatgaacatg agtccctgca 365160
   gactgcccac atgattgctc ctcaagtata aaacatggta gccaagagtt tggtcccagt 365220
   agcaatagga tgaatgaatg aaatagatgg tcaggaaagc ttctgtgata cagagcgctg 365280
   gcttgcaggg catagagcag gaggacatgg ttgtgctctt ggttggactt cctcctgtcc 365340
   atcctgttgc aaagaatgta gtgatatcgg agggtgcagt ggctgagggc tgtacccagg 365400
   gagttgcttt gtgtctgggt gatccagctc agaattttgc tgacttgtag caaaaggtgc 365460
   attgagcctt tggcatggat gagcatattt tattgcagaa gtggtagtac atggagctat 365520
   aatgtcacag gacaagatat gctaggtaat atgttcacat tgaaatgcct aatggagagt 365580
   ttggtggcat tcatgtattg tcacgaattg gcttaatgaa gtcctgtcac tcttcctctg 365640
   aaaggctccc atagtgtcca taacacctaa tgaagggctt actgtgaaaa ctgggtaggg 365700
   ggatgagccc agggtacaag gattcagccg cctcagaaga caacccaggt agcagcatag 365760
   aagcagtgag gtgaagagga agagaaagct aagtaggctt cattagagtc cttggagtct 365820
   tttctgcttt ttatttattt gttttttcat tgtttttaat taattgccca ggctggtgtc 365880
   aaaatcctgg cctcaagtga tcctcccacc tctgcctctg aaagtgctgg gggttacagg 365940
   cgtgagccac tgtgcctggc acctgtggag tcttaagcgc taaagtgtgt gtacaatgat 366000
   ctgcccagta gtaaacatgg ctctagtatt cttttaaaat attatcaatc agatcatcaa 366060
   ttgttctaaa atatggtcaa ccagattgtc aagtgttcta aaatatgatg catctgatcg 366120
   tcaatgctct ctgggcaggg cttgaggagt acaacctgat gagaagcaga cgttaagatc 366180
   ccctttcctc cactcctgac attggagcaa ccatccagtc ctgtctagac atcactacaa 366240
   tgctcattcc ctccttcctt ccattctagc ctgtgtcagg cctcactaac tccaaaccca 366300
   cttgtatgac agactgattc caatcatttc tgctcttgtt accgattctt atctgcactt 366360
   tacttaaagc cccataattt tgaggcatct ggacagtagg aaaggctgaa aacctggcaa 366420
   gcttttatag cttgcaaagg tccttgtcct atgctctgtt agtatcattt ccaaaatact 366480
   acttgggcta tctgatcaca tcccattgcc cgtatcctct gagagtctaa atgctgaact 366540
   gcttcagaaa ctaaatcata ctcacaggac ttggtttctc agcaatttta tcatctgaaa 366600
   acattgttta tgtagctgag gtcaaaagct tcaagtactg ctttaatgag ctgcgaatct 366660
   gttcatgtga cattctcttc caaacccaat tgccctttac actgtatgag taagtgcctc 366720
   tgtgtgtgtg tgtctgaatg tgtgtgtttg atgtgctgac atttctgaat tgaatccaga 366780
   aagtgtaacc tttgtatgtt tgctgttaaa actccaaatt caagccttaa gaaaaggatc 366840
   taacacttac tcaatgtgat ttatttaagc acagaaccta actcccccaa agtatcaaat 366900
   actcaagaga acaattcatt tttgaaaatt gtgatttaaa ataattctca taaaaccttt 366960
   actgttcaaa tatattagaa aatatattgc atttttgtct gcatcaggca ctgttttaac 367020
   tgccttccat gaattgcctc atttaattct ctcaaccctg taaggtaggg ttatcaccac 367080
   agtatagatg aggaactaga gcttggaaaa gttaaggcat ttgcccaagg tccagcaact 367140
   agaaagcaga ggagccagga cttgatccct ggactgtaca ctgcacaaga cccttgtaaa 367200
   gattattaga agcacttgga gcccctggta aggtttgtga ttgtgctacc aggagagtca 367260
   tgataacacc atcgaatggt gtctgttaca taatagaaaa agactgagta ttcttacaat 367320
   tcaaatgatt taaatttatt ttaaaatatt gcaacaaata agtagcatct tcacagcttt 367380
   tataattcgt tgtcaggatt ttaaaagatg caaatcttat ttgttcctta gggctttgat 367440
   tttgaggctg acaaagacgg catgcattga ctaacctctg aaagaagggc atggcagcat 367500
   tgaaatgagg cagccgagac aggctcatta aggagtagag gtttccctgc tcatttctgg 367560
   ttgttctttt ggggtatcca ctgtgtggag tttgcagaga tcttaataaa aatatcaacc 367620
   tacatttata tactgacaaa caaatagcca agagccattt aggacacaca gcttggagtc 367680
   tgtttatctc tctccaccta tctacacacc catggagaac ccatttaaac ttcagatcaa 367740
   accaggctcc ctttagatgg ttggaattgt caggttcctg agccaggtga agttcctaaa 367800
   tcagtttctg tcttagtcca tgttgtgttg ctatgactga ataccacaga ctagataatt 367860
   tataaagaaa agaaatttat ttcttacagt tctggaggct gggaagtcca agggtgaggg 367920
   gcttgcatct ggtgagagac ttcttgctgt gtcatcccat ggcagaaggt gaagggcaag 367980
   agagagtatg cgtgcatgtg tgtgcatata tatgcatatg catacatgtg tgtgcacatg 368040
   caggggaaag atacggagcc aaactcatcc cttttattag gatcccacta ccaagataat 368100
   gttgttaagt tctcatgagg tgccacctct caacactgtt gcattggaga ttcagtttct 368160
   aacacatgaa ctttggcggg gcaagtcaac catagaacct accagataca tgactttgag 368220
   tcatgtacta ttatattatt atttattatt attattgtta ttttgatact ggtccatggc 368280
   ccagggattg gggatccctg ccctaaatcg tgaggaagat tgaggcccat gggtgccact 368340
   ggttggtagc cgtcagcttc ttaccaccta ccggcacctc catagtcatc ttgacctcct 368400
   tgcccagctt ccatctctgt cttagcaaat atgttttccc tcccccaact catgtgagac 368460
   ttaccctcac tcctgctctg gactccagcc tcttgatcct tcatgcttat ctcccctgtt 368520
   tagtctgtct acagcttctc tctgtatcaa caaaactcct atggtttcaa ataggaatct 368580
   gatttaaact gacttttaag caaaaaataa tgatagttag tattcagatg tagatggatc 368640
   atctacatct ggtcccaaac aatgccagca gggaaacatt tatctatatc ccttaggatt 368700
   actttcccca ctgtgttgac ttcattcttc attctcagaa ggctttcttg ggcacatagc 368760
   aactcccaat ttatgtccca ttatctctgc aacttctacc aaagaaaagg gcttcctttt 368820
   tataaaatcc cagcagccta ggactcaatc tttttggaag attattgggt catatgccca 368880
   tctctaaggg aattgctcac tgcaccaggg ggatggaatg tgctgattga ccgggcctgg 368940
   gtcaccagcc cctcttagcc agagagctgg atctaatcca gacattttga aaaaagagaa 369000
   ggaatgtcct atctaaatca gacattttga aagaagagaa ggaatatctc ctttaagaaa 369060
   aaaaagccac tgtcatggcc gggtgtggtg gctcacgcct ataatcccag cactttggga 369120
   ggctgaggcg ggtggatcat gaggtcagga gatcgagacc atcttggcta acacggtgaa 369180
   accccatctc cactaaaaaa tacaaaaaat tagctgggcg cggtggcggg cacctgtagt 369240
   cccagctact cgggaggctg aggcaggaga atggcgtgaa cccaggaggc ggagcttgca 369300
   gtgagctgag atagcgccac tgcagtccag cctgggcaaa agagcgagac tccgtctcaa 369360
   aaaaaaaaaa aaagaaaaaa agaaaaataa agccactgtc actagaagaa aagacaatgc 369420
   ctgctggctg ggcaaaaaca gcagctgtcc tttctgactc tatcctggcc ttctcctttg 369480
   tgcataaacc tgctatgtct ttttcatgtt aaaaatcccc tacctcaaac ctgggaatcc 369540
   tccttactac tactcttact actgctatga ttactaccct accttacttt gcatacaaac 369600
   acacttttga aagagttgcc agttcctgct atttccactt gatgacaatt tattgtttcc 369660
   tcagtttggt gcaatctggc ttctgctccc gccatcctat tgatattttt ctcattgagt 369720
   catcaatgac ctgaaaactg cctcttctca taagcttgtc tcatctttct tgacctcacc 369780
   atattattag gtgctctaat cctttctcct ttaaaaaaat gtgaaaaaat aaaatattca 369840
   agcagcatca gaagctacaa agtgaaatta agattcccct cacccatggc tcctagctag 369900
   gcttcctggt tctccttccc agagacaccc attgcaacca ggttcttgtg tgaccttcta 369960
   gagaagatgt gttcaggccc agaaatagtc tacatcacac acaaggatgt tagcatgctg 370020
   tgtacaattt ctgtatcttg cattattccc ctctgtcttg gagattgttt cttatctgct 370080
   tatctaactc tacatcaccc tttgaataac ttcctagttt cctatataag tgagtttatc 370140
   tgtaggaaaa gttcagagtg ccttctttct ttttaagccc ctcttctcct tcatttttat 370200
   gacattgtgt gtctgtctta gtctgttttg tgctgctata agaatacctg aaactggggc 370260
   ctggtgcagt ggctcatgcc tgtaattcca gcactttggg aggttaacat gggcagatca 370320
   cttgagcttg agcccaggag tttgagacca gcttggggaa catggcaaaa tctgtcttta 370380
   cacacacaaa aattacaaaa atcaaccagg tggggtggca catgcctgta gtcccagcta 370440
   cttgggtgac taaggcagga ttgcttgagc ctgggaggtc aaggctgcag tgtcgtggtc 370500
   acagcttgct gcagactcga cctcccaggc ttaagcaatc ctcctgctcg agtgacagag 370560
   tgagaccctg tctcaaaaaa aaaaaagtgg ataacttaca atgaaaagaa atttatttgg 370620
   ctcatggttc tggagggtgg gaagtacaag atcaggggct gtatctgggg aggaccttct 370680
   tgctgcatag taacctgata gaaggcatca catggcaaga gaacacatga gggagggtaa 370740
   cttacttttt aaataaattc actcccacaa taataacatc aatccagtca cctccaaaag 370800
   ttcccacctc tcaacactgt tgcattgggg attaagtgtc cagcacaaga actttgggag 370860
   aacacattca aaccatatca ccacatttgc tgatgttctt cctaccttca cagccactcc 370920
   caatagaaat tccaactcca caaagacttc cagtgacagt tgaatttgga gggttgagac 370980
   cagctcatga cccagaaagg aaaacagaaa gcttactttt tgttttgtta caagtgcagt 371040
   ctcttaaaaa taaccacaaa atgccccccc aaatggcata tggaaaacag cacagagatg 371100
   gtgtgttaat ctacctgaaa tgtctttcta aggaagtgag gcttggatgg gaacggaaat 371160
   gcatcccttt ttcttcccag ggtacacgtc tttttctcac ttcctctaac tgggagcccc 371220
   agggcctggc tttgtcttgt agcttcctgc cgtgatgcac caggctaagt gggaaggttt 371280
   ctgttctatg tctaaagtag cactggcagg tcctcattct gcttgtaatt gggatgggaa 371340
   tatgggtagg aatttagggt ttggcagagc gtacaggacc tctttcaagc cagagcacag 371400
   ccctgtcaga cttactcact ggacatgaat gggtcagtca gccctcagca gtaaccacca 371460
   gctaccatgt taagtatttg atgtgaattt tatcaagtca ttctcgccac accctacaag 371520
   aaacttgagc ttaggggact tagctaactt gcttgaaggt tgtatagctg ggaattgttg 371580
   gagccaggat tggtacccag gttagcttaa cttttaagtc cgtgccacac aatcagatac 371640
   atagtatgag gcaatgggat gtaatggaaa gcccatgggc tttggaattg aacagatggg 371700
   ctctggatct tggctctcct acttgaccct atatgaccct gaacaaactg ttaactctta 371760
   aaattatctg ttaagtagag ataatccttt tcctgcctat ctcacagcct tgtgggaatc 371820
   aaagctataa aaagataggc aaatagaagg gataattact atttgagggt gagtttactt 371880
   gaagccaaca atgtgggttt aggatgaatg gtgaagaata gataagaaaa aaaatgatat 371940
   tctgttgaca tgggaagatg tttagagtgt atttcatcta gtttctttta aaaagcaagc 372000
   aaaagaacag agtcatgatt ccacctttgt ctaaaaacta aaaaacatcc attcatctat 372060
   tcatccagcc atctaggata caaaccaaga tgttaacata atacagagat ttggggtggc 372120
   gggattaaga aggattcttt tgtgtgtttt aaaatttttt atggccaggc acagaggctc 372180
   atgcctgtaa tcctagcact ttgggaggcc aaggcgtgca gatcacgagg tcaggagttc 372240
   gagaccagcc tagccaacat agtgaaaccc cgtctctact aaaaatacaa aaattagctg 372300
   ggcatggtga tgggtgcctg tagtcccagc tacttgggag gctgaggcag gagaatcgct 372360
   tgaacccggg aggtggaggt tgcagtgagc cgagatcgtg ccactgcaca ccagcctggg 372420
   cgacaaagtg agacttcatc tcaaaaaaaa aattattatt ataattcctt tttttttttt 372480
   tttttttttt agagacaggg tcttactctg ttacccaggc tgaagtgaag tggaacagtc 372540
   atagctcact gcagcctcca gttcctgggc tcaagggatt ccccgtgcct cagcctcttg 372600
   aacagctggg accacaggca tgcaccacca tgcctggcca atttttaaaa aattctttgt 372660
   agagacaggt tctctctatg ttgcccaggc tggtctcaaa ctcctgatct caagtgatcc 372720
   ttccacctgg gcctcccaaa gtgctggtat tataggcatg gcaccaccat gcctggcatc 372780
   ttggtgcttt ttaatagctt gtttgtttct gcgataaatg taggagactg atagaagagt 372840
   ctggatctct tgtcaaatct aggatcaaat cgtggtcaga tacttaaact aaacccattt 372900
   tctcattgca cagcaaggac ttcatagagc ttttgggggg attaaaggag gtaaagcttg 372960
   caaaacactt aacacagtat ctggctcata gttagtgctt aacatacact attatttgtc 373020
   catttgattt tttttcatcc ctgggttcta gaaacgcagt ggtgaataaa acagatgtga 373080
   ttttgctctc atgtggctta cagtctagat gagaacatag acaatcaaca gagaatgaac 373140
   atgtattaat aattatagac tgagatatac cagattttat ttctgttaaa gaaatgaaca 373200
   ggatatcttg acagagaata acaggaggga agaatgagaa tcctctttat ttgaaggtgg 373260
   tctggaacag cttctctgac agggtgccat ttaaactgag atctggagga aatcaaaaca 373320
   ctggatgtta ttttttaatg aaaattaaga aaagcaagag caaaacaaaa cagacttagt 373380
   atcaaacaca aaagacccct ctcctactga cttcctccct aaataaacat aagagataaa 373440
   agatccctga ttgtaaggga ccttcattat tagcagaaaa aggagactta aatctctctt 373500
   tttttctgcc aaaacccaga ccacaaatga ctttttaaaa tgatcaaata tgtatgcagc 373560
   tttgagcatc tgattctccc tggagtgggt ccctcctgta ttcggctgtg ggaaactgga 373620
   aggcatagga acgaccctgc atttcactgg atgtgcacat gtgaaaaaga aagatgcgct 373680
   tgtctttttg cctaggctat gtagcttgga tgtcttttca tcgtgacctt ctggtcttgt 373740
   taacctgaag gtgtcctggt gtcttgatgg aggctgggca ggtagctccc ctgagccact 373800
   agcctcatgt tatcaaagtc ggctaggcaa cagacagcag ggaggagcct tatcctctcc 373860
   tccaccatct taccttgtcc atgccctctt tttacctcca tttcttctcc aggctctttc 373920
   ctttcctcct ccaaagaagc aatgtctctc ttgcaggtcc cactcaagtg agatgtcact 373980
   ccctccagac agagttcctg gcccttcttg ggttcctttc ctctgtgctc aatggcagca 374040
   cctgtcccat ggtcccatgc agtggcctgt gtgtgcatct gtgtcctctg ctagatctta 374100
   agctcaacgg ggacaagaac aggtaccctc ttatgtatcg ccagagtcca gcatgctgct 374160
   cggcagagga tctacaacag ccaaatatta gccttcctta ttatccctta gcaccaaaat 374220
   acacgtcaaa gcagccactt gttttagagt ttttttgctt cattttgttt tgagacaggg 374280
   tctcactctg tcacccaggc tgaagtgcag aggcacgatc atagcttgct gcagtctaaa 374340
   cctcctgggc tcaagtaatc ctccaacttc agccttccaa gtagctggga ctacaggcat 374400
   gtggcgtcat gccctgagaa gttttaaatt tttgtagaga cagggtcttg ctatgttgcc 374460
   caggctggtc tcacactcct agcctcaagc gatcctcctg ctgccttaga gtgttttact 374520
   aagatgaagc atgttaaagt agcatcaggc taaccttaac aatttaccca aatactccca 374580
   ttctctttat ttctattttt caatttcttc catgataatt gctgtctcat ttaaaatagc 374640
   tttcacagct tcttttctta ttacaaaata ataataacaa tacctgattg ttatgtaaat 374700
   ttttaaaata taatcaagca taaaggggaa aaggaaaaaa ttaaaaccat ctgtaattct 374760
   tcgaatctcc ataagcctgt gtataaaatg tgtacagata ttacgtatac atttgtgtat 374820
   ataatttata gaatgggaag atattgtaca cactgttagt aattatataa ctatgtcatt 374880
   taatattctt ctacatcttc ttttaatttt taagaatgtt ttgcatggta atacatcaaa 374940
   tagatgtatc attatttatt taactgtttt ccctattggc ccatagtaga ttcccaattt 375000
   ttctttcctc tgacgattct ttgatgaaca ccctgtattt tgatggggta aatccaggag 375060
   tatagaggac aggtcaggga aaacctcgtg gaggaagtga cactgctctg gagacagaaa 375120
   ggatgcacag gagatagctg gatgagtgga gaaggagtgg aaggcaagtg ttcctggcag 375180
   aggaggcagc ctgtgcaagg gcctggagtc acctccaaga gttcatgagt gttctaggaa 375240
   ctggaaatac ttcagtatgg ctggagggtg tctcaggggg tgggggacac aggcagggaa 375300
   ggggccattg accaagtgtc caggaacagg tcctaaatgg ccccttaagc catgctaagg 375360
   agtgtgggct ttgggcaatg gggagctatt gcagataaga gcagggaaat ggcatactcc 375420
   agtcagcgtc tttaaaagat ccctttagct ggccatggtg gtgtgcagct gtggtcccaa 375480
   gctactcaca aggccaaggc aagaggatcc cttgagcctt ggaattcaag gtggcagtga 375540
   gctatgatca cgccactgca ccccaacctg ggtaagagag caagaccttg tctcaaaaaa 375600
   caaaacaaaa caaaacaaaa aagagatccc tgtggcagtg gtgaccaagc caactccaca 375660
   ttcaacagca agactctgtt attaattcaa tgaaacagaa attaaaagag aagagaaaaa 375720
   gaaaaagagc ttacgtgtaa caggtcacag ataagctttt caaactgtct agattcttaa 375780
   attcaggagc gtaattcaac aaatcaacct tcttactcct ctccttaatc ctgaaagagg 375840
   tgcttgtggt ttaaataacc cagttggttt ctggccccca ttcctgttcc aggagcccca 375900
   ttgatgtacc ctgatgacaa gggagtcgga ttttcttttt ttttttaagc tgagtgaggc 375960
   tgaagtgttc acccaagaca agccagagag gcaggtgcaa tccatggtgc gtttttaatt 376020
   agatcccagc cactgagctg cccatacttc aaggacacag caggcaggaa actgatctgg 376080
   gctgtgtctc ccaagggctg acgtctccat ttagggagcg ctcctgaagc taatggtcag 376140
   tgacagctcc atttgaaaaa cacagaaaca cttaacaggg aggctgtgat ctgccagccc 376200
   tgtaagctgc tgattagcag ggaaatcacg ctccctctgt atcgcacccc cttggttaat 376260
   ccctgttctt gtgtctcctc tgtgctatct cacttaaaga tttcattgtt ctctgaggca 376320
   ggagggagtg gggagaggga gggggaggtt gtggagctgc cagaagctgt ttcactgtct 376380
   cccatcacag cctctgggga ggcccaggcg aagaaacagc cttctggtgt ggatgggaaa 376440
   ttgatggttt ttaaagaaaa atggtccctg aagatgctct gtgtctcagc aaatgtgctt 376500
   cctagactgg ctggcttggg ttccaacacc caggccaaag aaaaacacgc acgggtctgc 376560
   cttatagtca caccaggatg ccataccagc agtccctgcc accatcacct ctttgttgtc 376620
   tgctctgcct cccctttcta cagtcctaag ccaccattcc tctcctgtgg atgactgcgg 376680
   cagtctctcc ccgatgccac ggccaccaat ctagctcccc aaacctctgc aatccccaca 376740
   ctgcagctga ggggactcct tgacgtgtcc tctggaattt tgaacttatc aaaaaggaaa 376800
   gcaaatacta aatttccccc ctcaaatagg atctcctcct cgtgttcctc tcttactaaa 376860
   tagctcaggc caaaaatgcc agggtcacca acaatgcctc tcttcctcac ataccccaca 376920
   cccaatccat cagcaaatct tgtcaactct gaattcagaa tataccccac atccgaatgc 376980
   atctttccat ccctccacca atcaccttcc ttcaagcccc catcattctt aactggatta 377040
   tcataaccac ctcctcactg gttgtactgt ttccactatt gtcccccgct catttaatct 377100
   atccttgtac accacaccag tgatcctgtt taaatgtaaa tcagggccag tcttggtggc 377160
   tgacacctgg aattccagaa ctttgggagg ctgaggtggg aggatcactt gaggatagga 377220
   gttcgagacc agcctgggca acatagcaag accccacaca cacaccctgt gtctacaaaa 377280
   aaaaaaaaat tagtcctgcg tggtggtgca cacctgtagt cctagctact catgaggcta 377340
   aagtgggagg attgcttgag cccaggagtt tgaggctgca gtgagctgtg atcatgcctc 377400
   tgactcaagc atgggcaaca gagcaagacc atgtctctaa aacaatgaaa tattataaaa 377460
   acaaaacata cgtcatcata tcattcaggg ctcaacattc tccagcagtt tctcatctag 377520
   ctgagatgaa aatccactgt ccttacatgg ccatcaagga ccctgctctg gctcgtggcc 377580
   cgctgtgtgg ccccttggtc actctgctct ggccatacca tactgacctc attgctgctt 377640
   ggtgactgtg ccaggtgagc tctgtcttgg ggcctttgca cttgctgctc cctctgccta 377700
   gcatgccttt cctcccgaca tccaaagggc accctctctc acttcactga ggtcttgctc 377760
   aaaggccatt tcctccaagc tgaaaatggc atcactctca ctccatcccc tggactttct 377820
   tcttcttttt tttttttttg agacagagtc tcattctgtc gcccaggctg gagtgcagtg 377880
   gcttgatctc cgctcactgc aacctctgcc tcctgggctc aagtgatccc cccaccacag 377940
   tcctcctgag tagctgggac cacaggtgtg caccaccaca cccagctaat atttgtattt 378000
   ttggtagaga cggggtttca ccatgttgcc caggctggtc atgaattcct gagctcaagt 378060
   gatccaccat cctcggcctc tcaaagtgct gggattatag gcgtgagcta ccctgcccgg 378120
   ccttggcttt atttttcttc acatcactta tctccctctg gcactaaaac atatattcac 378180
   atttacagtt acagtctcgt ttgttgcttc tcttccccag taaaatgtaa gttccatgaa 378240
   gcatggattt tgtatcattt tctactagca cagagcctgg catgtagtgg gtgctcagta 378300
   aatccgtctt gaatgagaaa atgaataaac aaatgaatga atctggacaa caattctaac 378360
   cacagtgagt ttaccgaggg gtcatccttc tgtgggagct tccctccact ggccagcaga 378420
   aaagacccca ggtcttcata ctatgtcaga gagttctcat tttctgagac ctgaagccaa 378480
   gataacaaaa acagcagaaa caatttagct tggaatgaca aataggtttc cttaagtctg 378540
   acaagagtct tttaaaaatt caagactgtt tccttagtat ttaaaactta aacaccatgc 378600
   aaatgcccta ttataggaaa catacgctgt ctgtctgtag ggatttgtag tagaaaatgg 378660
   gaactaaata atacttggag ccatgatgcg ttgatcacat actcagtacc tgttacttgc 378720
   attctctcat tttatcctca tatcacaacc ccagtgtgat aggaattaaa attgctccca 378780
   ttttacaaaa gaggaaactg aaactttaag gaacttgccc agagttgcac agatagtggg 378840
   tagcataatg ccagggtttg aattcagtct acagactcac tttttttttt ttttttttgc 378900
   gatggagttt cccttttgtt gcccaggctg gagtgcaatg gtgccatctc agctcactgt 378960
   aacctctgcc tcctgggttt aagtgattct cctgcctcag cctcccgagt agctgggatt 379020
   acaggcacct gacaccacgc ctggctaatt ttttgtattt ttagtagaga tgggatttca 379080
   ccatgttggc caggctggtc tcgaactctt gatctcaggt gatccacccg cctcggcttc 379140
   ccaaagttca gggattacag gcatgagcca ccgcgcccgg ccccagactc acttttactc 379200
   agataagaag tgagcaggag gcagaggctc ttggagacct aagtcccttg catgcacagc 379260
   aaatacttct gcttccctca acaaaaaggc tttgaagaag agcgtgcacc tcttgacttt 379320
   ctcaatgtac cttcactcct ccagcaactg aagttcagtg gcagccctcc atttctcatc 379380
   agtatcaccc cagttgccaa ttgcaatgat cctttttcgg caactctcct atttgacctc 379440
   tgaaagatat tttagttata ccaatgccct tcacctgggg tttctcccag tccttagctt 379500
   ggctgatact gttctctcct ggttctcctc ctgctgtcct aagacacttt aaggagcatt 379560
   tttatcatgg gcaaatggaa ataaaacacc tatttcacag agtttttaca tgaatgaaaa 379620
   aggtcatata ttcaaaaaca cttggcaaat gtctgtttca ttgaaagtgc tcactggttt 379680
   ttattactct tgttatctct ttccactccc catattctgt gctttaaaaa aaaaaaaaaa 379740
   aaaaaggttt ttcaagacct ctctcctcct gtgctttttt ttcctttttt tttttttttt 379800
   tttttttttt cctttttgga gtcaaggtct caatcactgc ccaggctgga gtgcagtggt 379860
   acaatccctg ctcactgcaa cctctacctc gctggctcaa gcaattctct cacctcagtt 379920
   tcctgagtag ctgggactac aggcatgcac caccatgccc agctaatttt ttaaagacag 379980
   ggttttgcca tgttggtcag gctggtctca aactcatgac ctcaggtgat ctgcccacct 380040
   cggcctccca aagtgctggg attacaggtg tgaaccacca cgcctggccc agaacctgtg 380100
   cttctgactc cctctggtca tcaccaggac tctcattggc ctctcaaagt cgttatccaa 380160
   aatggaatta atcatctttc tcagttttca cactttctct gtctcagttc atacaaccac 380220
   agtctctgtc attcaaacca gagacttcat catccccatc cttgtcttcc cgctggcccc 380280
   ttccccactt cattagttag tcagccacca ggctgccacc gtacttgtat atttatttat 380340
   ttatttattt atttatttat ttatttattt attttgagag agagtctcgc tctgtcaccc 380400
   aggctggagt gcagtggcgc gatctcagcc gactgcaacc tccgcctccc gggttcaagc 380460
   tattctcctg cttcagcctc ctgagtagct gggactacag gtgcccatca ccacgcctgg 380520
   ctaattttta tatttttagt agagacgggg tttcatcatg ttggctagga tggtctcgat 380580
   ctcctgactt tgtgatccac ccgcctcctc ctcccaaagt gctgggatta caggcgtgag 380640
   ccactgcgcc cagtcactgt acttgtaaaa atggagccag gaacttgggc tctgatgtca 380700
   gatggacctg gttcatcttg ctctgctatt ggtttgctga gattatgaac aaattacata 380760
   gtctccgtgg tctcagttcc gtcatctata aaatggagat cataataatg cccacttcat 380820
   agggccattg ggaagattaa ataaggtaat gcaagtaaag aactttccag taaacagcat 380880
   tttgcactgg tatatagcaa atgcataata aatatcagca gtcataattg ttattactcc 380940
   tactactatt atactattag gttgtggtta cctcgtagag atactgtgtt gaaaacacat 381000
   agagcttttg tgcaaatggg ttttgctggc aaagaacaag gaaccccaaa ttagcggttt 381060
   gccccttctc attaggtggg aattagcatg tacaggtgtg agcacccatg catgcatgca 381120
   cacatttgca gacatgtaca tacacatatg tgttataact ctgcctcaga tgggggcctt 381180
   tttaatatag aaacaccagc aggatgggtg agaaatggca atggttttct ctctagaacc 381240
   tgattgcttc accaaacaat ggtttgtatt tcctcccaca tccctccttc ctaattgaag 381300
   agggaatggc tgaatttacc tcctggcacc ttgcatttga tgttcagtga ctgatccagc 381360
   taaccagggg cctcgtggtt gcccaggcgt tgcaagcatc aaacccagat cttcactggg 381420
   atcttcctcc agtggctgga gtttataaat tgtattctgt tgagttggaa aacatccact 381480
   gctcctgatt gagagctgga ggacagcact ttgaggggtc tgctgctata aatactggga 381540
   tctcccaggt gatgtataaa tgggtcacta ctttgcatct tgcaccacct gaaacatctc 381600
   aggtgcctta atcccatgtt ttccaacaga tgagcttggc tgacttctcg gggtgggttg 381660
   gggggacatg ccacatgcct ctggccccac catctcatgc ctttgccatc catctcctga 381720
   ctggccagaa ggacaccagg agcagctcat gagctacaaa aactgaaggt cgggaaccca 381780
   aggtcgtgca ccaaaggtca tgagaatttt gcattttatg ttgctctcat ttctgatcat 381840
   ttgaaacaac ctctctgagt ttctgaaagg gaagggaatg gagaaaagca cacccaatta 381900
   tgctaggttc tgttttattt tggcttttgt ttttgttttt tttcccaccc accacaagtt 381960
   cttttcttcc cctctcatag tgtgggcgaa atgctgaaaa cttcgaccga tttttcaccc 382020
   gccatccacc agtcctaaca cctcccgacc aggaagtcat caggaatatt gaccaatcag 382080
   aattcgaagg attttccttt gttaactctg aatttttaaa acccgaagtc aagagctaag 382140
   tagatgtgta gatctccgtc cttcatttct gtcattcaag ctcaacggct attgtggtga 382200
   catttttatg tttttcattg ccaagttgca tccatgtttg attttctgat gagactagag 382260
   tgacagtgtt tcagaaccca aatgtcctca ggtagtttgg agcatctcta tgagatggga 382320
   ttatgcagat ggcctatgga aaatgcagct gcataattaa cacattatca aagtcctctt 382380
   acaatttatt ttccgcagca tgtcagctaa gtagacccaa tggggagaga aaatgcctgc 382440
   tttctttccc tctttttctg cactgccata ttcaccccca accatccaat ctgtggataa 382500
   ttggatgtta gcggtactct tccacttccg ggcctggagc ttggcttgta tccaagtgta 382560
   tggttgcttt gcctaagagg aatccctcta tttcacctgt tctggaggca ccagaccttg 382620
   aaaagaacat gctcaaaata aaatgttatc tgttattttt gtaaactcaa agttaagatg 382680
   atcaaagttc taaaattcca agaatgtgct tttagacggt ctcaatctaa aagcacttca 382740
   aggggtcaaa gggcaaccag cttgggtgct acctcagtgt tgtagtttct gatactttat 382800
   gtctttgctc accctcatcc ccaaactact tgaaaagggc atttggcacc actctctgaa 382860
   acaacacagt cactctagca aggcccccaa agggccctgg ttttacatta catttcaaac 382920
   tttatttgct ttggggtttt gtttctgttg ttgttcaaat gcaaaaaaaa gaaaaaaaaa 382980
   gaaaaaaaaa ggtgactcac attgttacac atgctttaaa atatgtattc aaatgttatt 383040
   aaccacaatg acgacctgct ttgatttaac caagaagacg gctgcggagc ctagcagact 383100
   caggcctgtg ggaatgggat ttgttacaaa tctaggtttg ttactggctt cagaaagcta 383160
   attaagtgct ctgaaaaaga caccgtttct tgaaacaaag atggttgtat tcctcacttt 383220
   gatgttgttt tgcaagatgt ttgtggaaat gttcatttgt atctggatct ctgttatgtg 383280
   ccatttttct tctagcatcg agatacaata aaaaaaaaaa aaaagaaaag aagaagaaat 383340
   actatttcaa ggaaaactgc tctttttgag aaacgtggac ctaaactaca aagtgggaac 383400
   tgaggaggga actcaggaga aaggaactaa ctgcggagct ttaatcttgg ccccagtgtt 383460
   cagccactcg gaggggcggg ggctgtggcc cattcagggg ctgctggtgg gctgtagtgg 383520
   ggtgggatga cctggccaga gccaacgagg atactggagc ccaaagtcaa gtttagagac 383580
   cagctgggaa cgtgaatggg gctcttgatt ttcttatcaa aatcaccact cctcccagct 383640
   tggactaaat attctttcta gcaagcagct ttgtgagctc cctgaagccc aaggaaaccc 383700
   ttcggtggga gaaatttcat ttctgtctga gaggattaag gcagcaggtg actccccctc 383760
   ctcgcctgcc gtgtcctgct attctcaggc agctctaagg agaattctta tcacagttca 383820
   agtgatttcc agaagttcca gggcttctga gagaccatca agggaacttt aacaacttga 383880
   caaatgtcct tgaagtaaga tgcctcatct ttagggaaaa atggggtttg gatttctgct 383940
   taggcaaagt ctcctgcagt tcatccttct ctgtcctctt cttgcttcag gcttggggac 384000
   cgtccctgct gtccccactg tggtggcaat caggacctaa ggtgaagcaa acttgaagtt 384060
   ctatctgaca agtttaggca gtaagagaag gagggaaatc ggagcaaagc tccctcactt 384120
   tattgttgag aaactggcat ctggaaagag gaaggaattt gcccaaagtc agtcagctgg 384180
   gataaaaacc tgggtgtcct gtccagaaag tgcagggtgc tttctgctct gtagcaaggc 384240
   agcagacatc tctgagccag gcccaccaac aggcccttat ctggtggttg gatcatgatc 384300
   ccattttgct tggacatgct ctcaggaaga taaaaaccat ggagaaacac taggccattg 384360
   acaaatgatc tgagacaact ttagaaaaca atgtaggatg aatggaaaga gaaagaaagg 384420
   aaagaaagaa gaaaaagaaa gaaggaaaga aagaaagaga aaggaaggaa ggaaagaagg 384480
   aaggaaaaga aggaaggaag gaaggaatat agtgttataa atactgcact caacattttc 384540
   caaattcttg ccattatttt tcaaaagttt aatagtttgc agaaatagat actcaagcca 384600
   aagtctgttt tagagaaact ttccatggaa agtcagaatt tctaccactt ccttttctat 384660
   ccacatttcc agtgcagaag aaactgagaa acagagcttt ttgaagagag gacagggcca 384720
   tagcaacaag gaccttcttg ggggattaat gggaggtcag tagaattaat aaccctcctt 384780
   ggatgagtgc tactgttttc acatggcttc agatgctatc aacctcaaag aaatgatctc 384840
   aacagagaag cttattctct cccaacttct acggtaaaat ccaggagtat tttctctggg 384900
   gatctgccca caggacaaag tccataaaag caagtcctgt ctggaccatg tggttatctg 384960
   aagcattagc catcaccagc acaacaaacg gggcagggct ttccaaggtg gggctggtca 385020
   gaagggaatc tttgataaga ggcccacagg cagggaaagc gaaatagggt tgatgagacc 385080
   aggggagacc taaaaaaaag gcagctttgt gtcttctagc tccaaatata cctgcctttt 385140
   agctcacaca ctgtcctgga gttctcagac ctttaggggc cctaacacag ttcagttcat 385200
   acaggggttc aaaagggaca gtggcccatt tgggagacct ttaggatcaa tgggaatcaa 385260
   ttccattgtt ttgcctcaga gtaaagtttc tggctcgggg acaattataa gttgcaaaaa 385320
   ggatagaggc atatcccaag tcttccttca ttccacaaat aattacaaac aacctactgt 385380
   gtgccaggca ctattcttag cactggaaat acactagtga agaagcagat gaggaccctg 385440
   tttattgttt ctctccaaga aattctccaa gaatattgtt tcttggagag aaataataaa 385500
   taaacaagac aatttctgaa agcaataagt gcaatcaaga taattaaagg atgctaaagt 385560
   gtgacttgtg gggattggga gagagatgca cagacaatat taaagaggag gcattcgagc 385620
   tttgttgtga acaccggaag taacatgccg agcgcctggg ggatggaaac tcctatagca 385680
   ccccacaggc taacagcaag caggacaaga caaaaagggc aggtgggaca tggtagagat 385740
   ggaccctacc caggaaacag ctccatcagc atcttagcct gccccactct agccacacat 385800
   acccacgtgt gctcctgagt tcagtgtgcc cacctcactc ccacaccctc acatagactt 385860
   ggcaagagta aggagggaac tccatagaga cattttacct atctcagggg agcagccaca 385920
   aagaagcaag tcttgtaaaa ggtcttttgc aaaggagagt gaacccagca atgagagatc 385980
   cttaacagct agtgcccatt agggggctaa acctaaagcc tgggtggtga tggctcaaac 386040
   gctaatgagt cagtgaatcc ttaccgaccc cctggccttt ataatctgag gcaactttgg 386100
   ctgcagcccg ggaatgtgca gggcactagg gaatacaagg ccttcttccc tggttgtctt 386160
   gtaataaaac agccatgggg ttgtccctcc agtccgagag actgtgatga ggcctacata 386220
   gcagcgatgt ggtcaggtaa aaatcaggaa cccactgaaa tcttgggcaa gccaccctgc 386280
   ctgcttgtgc ctcggttctc tcatatgtca tatataggag gtgaggactc cagctccacc 386340
   tgccccaggt gggtgtggtg atgatgagga aagacaagag gcttgcaagg accctgaaga 386400
   ggtcggagca tcatacagat tcctttatta gcccacattc tgatgttccc tggtgagact 386460
   tgccccaagc aattgctagt aaatgggggt taatttcttc tccacctccc tactgaacaa 386520
   aaaaagaaat gccagactta ctaggagaat cgagttgctt tgagtttctt ttgttttgtt 386580
   ttgttttgtt ttgttttaag gctcccctta cacaccctcc tttaagcttt gggttttctc 386640
   tcttatagtt tgttgacaca tgctaaaaat gtctttggag agaacttctg cctgataaac 386700
   acccaattct agactgtggg tggattttcg agctgacggt ggtcaattcc tttcattaag 386760
   cagtgatctg atttctccac atggccattc tgccttcttg ggggcagagt agatgggcag 386820
   cagttcacct tttcagagaa agaggtcttc tagccacctg ggctgctact gaatggtttt 386880
   ctccaggacg ctctacctaa tgattatttc tataacatta agcatggtaa taagtagctt 386940
   ccaattcaat tcatcctaaa gccaaagaaa atacagcaac acacacacac acacacacac 387000
   acacacacac acacacacac acacaccact ttatggcaat tcttaactga cattcaatga 387060
   cttacttctt ttcttagaaa atttccacca catttctatc cccaagccaa catacaatgt 387120
   gaaatgaaag ccagtgcgtg gagtgcagct gctaaaaatt ttcagcacag ggctctttct 387180
   gactctgctc atgagatggt atcagccacc caatgactgg cgtatcttgg tcctgtgtct 387240
   ttcttcttac gctgtgttaa tgtgtttact ttccatttgg cagagagaca agagagacac 387300
   ctccaacttc gacaaagagt tcaccagaca gcctgtggaa ctgaccccca ctgataaact 387360
   cttcatcatg aacttggacc aaaatgaatt tgctggcttc tcttatacta acccagagtt 387420
   tgtcattaat gtgtaggtga atgcaaactc catcgttgag cctggggt 387468

## Description

### FIELD OF THE INVENTION

The present invention relates generally to the fields of genetics and medicine.

### BACKGROUND OF THE INVENTION

Autism is a neuropsychiatric developmental disorder characterized by impairments in reciprocal social interaction and verbal and non-verbal communication, restricted and stereotyped patterns of interests and activities, and the presence of developmental abnormalities by 3 years of age (Bailey et al., 1996). In his pioneer description of infantile autism, Kanner (1943) included the following symptoms: impaired language, lack of eye contact, lack of social interaction, repetitive behavior, and a rigid need for routine. He noted that in most cases the child's behavior was abnormal from early infancy. On this basis, he suggested the presence of an inborn, presumably genetic, defect. One year later, Hans Asperger in Germany described similar patients and termed the condition "autistic psychopathy".

Autism is defined using behavioral criteria because, so far, no specific biological markers are known for diagnosing the disease. The clinical picture of autism varies in severity and is modified by many factors, including education, ability and temperament. Furthermore, the clinical picture changes over the course of the development within an individual. In addition, autism is frequently associated with other disorders such as attention deficit disorder, motor in coordination and psychiatric symptoms such as anxiety and depression. There is some evidence that autism may also encompass epileptic, metabolic and immune disorder. In line with the clinical recognition of the variability, there is now general agreement that there is a spectrum of autistic disorders, which includes individuals at all levels of intelligence and language ability and spanning all degrees of severity.

Part of the autism spectrum, but considered a special subgroup, is Asperger syndrome (AS). AS is distinguished from autistic disorder by the lack of a clinically significant delay in language development in the presence of the impaired social interaction and restricted repetitive behaviors, interests, and activities that characterize the autism spectrum disorders (ASDs).

ASDs are types of pervasive developmental disorders (PPD). PPD, "not otherwise specified" (PPD-NOS) is used to categorize children who do not meet the strict criteria for autism but who come close, either by manifesting atypical autism or by nearly meeting the diagnostic criteria in two or three of the key areas.

To standardize the diagnosis of autism, diagnostic criteria have been defined by the World Health, Organisation (International Classification of Diseases, 10^{th} Revision. (ICD-10), 1992) and the American Psychiatric Association (Diagnostic and Statistical Manual of Mental Disorders, 4th edition (DSM-IV), 1994). An Autism Diagnostic Interview (ADI) has been developed (Le Couteur et al., 1989; Lord et al., 1994). The ADI is the only diagnostic tool available to diagnose ASD that has been standardized, rigorously tested and is universally recognized. The ADI is a scored, semi-structured interview of parents that is based on ICD-10 and DSM-IV criteria for the diagnosis of autism. It focuses on behavior in three main areas: qualities of reciprocal social interaction; communication and language; and restricted and repetitive, stereotyped interests and behaviors. Using these criteria, autism is no longer considered a rare disorder. Higher rates of 10-12 cases per 10,000 individuals have been reported in more recent studies (Gillberg and Wing, 1999) compared to the previously reported prevalence rate of 4-5 patients per 10,000 individuals based on Kanner's criteria (Folstein and Rosen-Sheidley, 2001). Estimates for the prevalence rate of the full spectrum of autistic disorders are 1.5 to 2.5 times higher. Reports of a four times higher occurrence in males compared to females are consistent. Mental retardation is present in between 25% and 40% of cases with ASD (Baird et al. 2000; Chakrabarti and Fornbonne, 2001). Additional medical conditions involving the brain are seen in ca. 10% of the population (Gillberg and Coleman, 2000).

The mechanisms underlying the increase in reported cases of autism are unknown. It is highly debated whether this difference reflects an increase in the prevalence of autism, a gradual change in diagnostic criteria, a recognition of greater variability of disease expression, or an increased awareness of the disorder. In addition, there is a widespread public perception that the apparent increase is due primarily to environmentally factors (Nelson, 1991; Rodier and Hyman, 1998). However, it seems likely that most of the increased prevalence can be explained by a broadening of the diagnostic criteria, in combination witch a broader application of these criteria.

Although there are effective treatments for ameliorating the disease, there are no cures available and benefits of treatment tend to be modest. Promising results have been obtained for several programs utilizing various behavioral and developmental strategies. Among the most promising are programs based on applied behavior analysis (ABA). Several medications appeared to improve various symptoms associated with autism, thereby increasing individuals' ability to benefit from educational and behavioral interventions. The most extensively studied agents are the dopamine antagonists. Several studies suggest the usefulness of various selective serotonin reuptake inhibitors.

Three twin studies have been performed to estimate heritability of autism (Folstein and Rutter, 1977; Bailey et al., 1995; Steffenburg et al., 1989). All twins who lived in a geographically defined population were sought out. In the combined data 36 monozygotic (MZ) and 30 dizygotic (DZ) twins were studied. The average MZ concordance rate is 70% compared to a DZ rate of 0%. A heritability of more than 90% was calculated from the MZ to DZ concordance ratio and the sibling recurrence risk that has been estimated to be ca 2%-4% (Jorde et al., 1991 Szatmari et al., 1998). Studies of non-autistic relatives have clearly shown that several characteristics of the ASDs are found more often in the parents of autistic children than the parents of controls including social reticence, communication difficulties, preference for routines and difficulty with change (Folstein and Rutter, 1977). Delayed onset of speech and difficulty with reading are also more common in family members of individuals with autism, as are recurrent depression, anxiety disorders, elevated platelet serotonin and increased head circumference (Folstein and Rosen-Sheidley, 2001).

The incidence of autism falls significantly with decreasing degree of relatedness to an affected individual indicating that a single-gene model is unlikely to account for most cases of autism (Jorde et al., 1990). A reported segregation analysis was most consistent with a polygenic mode of inheritance (Jorde et al., 1991). The most parsimonious genetic model is one in which several genes interact with one another to produce the autism phenotype (Folstein and Rosen-Sheidley, 2001).

Considerable indirect evidence indicates a possible role for autoimmunity in autism. One study found more family members with autoimmune diseases in families with an autistic proband compared with control probands (Comi et al., 1999). A few studies reported that haplotypes at the Major Histocompatibility Complex (MHC) locus present in some children with autism, or their mothers, might predipose their autistic children to autoimmunity (Burger and Warren, 1998). In two studies, autoantibodies to certain brain tissues and proteins, including myelin basic protein, neurofilament proteins and vascular epithelium were found more often in autistic children compared to controls (Singh et al., 1993; Connolly et al., 1999; Weizman et al., 1982).

Although most autism cases are consistent with the proposed mechanism of oligogenicity and epistasis, a minority have been seen in association with chromosomal abnormalities and with disorders that have specific etiologies. Smalley (1997) stated that approximately 15 to 37% of cases of autism have a comorbid medical condition, including 5 to 14% with a known genetic disorder or chromosomal anomaly. Chromosome anomalies involving almost all human chromosomes have been reported. These include autosomal aneuploidies, sex-chromosome anomalies, deletions, duplications, translocations, ring chromosomes, inversions and marker chromosomes (Gillberg, 1998). Most common are abnormalities of the Prader Willi/Angelman Syndrome region on chromosome 15. Association of autism and a Mendelian condition or genetic syndrome included untreated phenylketonuria, fragile X syndrome, tuberous sclerosis and neurofibromatosis. Recently, Carney et al. (2003) identified mutations in the MECP2 (methyl CpG-binding protein 2) gene in two females with autism who do not have manifestations of Rett syndrome caused in 80% of the cases by mutations in the MECP2 gene.

Different groups are conducting genome scans related to autism or the broader phenotypes of ASDs. This approach appears very promising, because it is both systematic and model free. In addition, it has already been shown to be successful. Thus, positive linkage results have been obtained even by analysing comparatively small study groups. More important, some findings have already been replicated. The most consistent result was obtained for chromosome 7q, but there is also considerable overlap on chromosomes 2q and 16p (Folstein and Rosen-Sheidley, 2001). Considerable progress in identifying chromosomal regions have also been made on chromosome 15 and X. Mutations in two X-linked genes encoding neuroligins NLGN3 and NLGN4 have been identified in siblings with autism spectrum disorders (Jamain et al., 2003). Several lines of evidence support the fact that mutations in neuroligins are involved in autistic disorder. First, the reported mutations cause severe alterations of the predicted protein structure. Second, deletions at Xp22.3 that include NLGN4 have been reported in several autistic children. Third, a mutation in NLGN4 appeared *de novo* in one affected individual's mother.

### SUMMARY OF THE INVENTION

The present invention now discloses the identification of a human autism susceptibility gene, which can be used for the diagnosis of autism, autism spectrum disorders, as well as for the screening of therapeutically active drugs.

The present invention more particularly discloses the identification of a human autism susceptibility gene, which can be used for the diagnosis of autism and autism spectrum disorders, as well as for the screening of therapeutically active drugs. The invention more specifically discloses certain alleles of the protein kinase C, beta-1 (PRKCB1) gene related to susceptibility to autism and representing novel targets for therapeutic intervention. The present invention relates to particular mutations in the PRKCB 1 gene and expression products; as well as to diagnostic tools and kits based on these mutations. The invention can be used in the diagnosis of predisposition to, detection, prevention and/or treatment of Asperger syndrome, pervasive developmental disorder, childhood disintegrative disorder, mental retardation, anxiety, depression, attention deficit hyperactivity disorders, speech delay or language impairment, epilepsy, metabolic disorder, immune disorder, bipolar disease and other psychiatric and neurological diseases including schizophrenia.

The invention can be used in the diagnosis of predisposition to or protection from, detection, of autism, or an autism spectrum disorder, the method comprising detecting in a sample from the subject the presence of an alteration in the PRKCB 1 gene or polypeptide, the presence of said alteration being indicative of the presence or predisposition to autism, an autism spectrum disorder, or an autism-associated disorder. The presence of said alteration can also be indicative for protecting from autism.

A particular object of this invention resides in a method of detecting the presence of or predisposition to autism, or an autism spectrum disorder, the method comprising detecting the presence of an alteration in the PRKCB 1 gene locus in a sample from the subject, the presence of said alteration being indicative of the presence of or the predisposition to autism, or an autism spectrum disorder. An alteration being indicative of the presence of or the predisposition to autism, or an autism spectrum disorder is an alteration with a preferential transmission to autists. Alternatively, an alteration being indicative of the presence of or the predisposition to autism, or an autism spectrum disorder is an alteration with a higher frequency in autists compared to non affected individuals.

An additional particular object of this invention resides in a method of detecting the protection from autism, or an autism spectrum disorder in a subject, the method comprising detecting the presence of an alteration in the PRKCB1 gene locus in a sample from the subject, the presence of said alteration being indicative of the protection from autism, or an autism spectrum disorder. An alteration being indicative of the protection from autism, or an autism spectrum disorder, is an alteration with a preferential non-transmission to autists. Alternatively, an alteration being indicative of the protection from autism or an autism spectrum disorder is an alteration with a lower frequency in autists compared to non affected individuals.

Another particular object of this invention resides in a method of assessing the response of a subject to a treatment of autism, or an autism spectrum disorder the method comprising detecting the presence of an alteration in the PRKCB1 gene locus in a sample from the subject, the presence of said alteration being indicative of a particular response to said treatment.

A further particular object of this invention resides in a method of assessing the adverse effect in a subject to a treatment of autism, or an autism spectrum disorder, the method comprising detecting the presence of an alteration in the PRKCB1 gene locus in a sample from the subject, the presence of said alteration being indicative of an adverse effect to said treatment.

It is further described a method for preventing autism, or an autism spectrum disorder, in a subject, comprising detecting the presence of an alteration in the PRKCB1 gene locus in a sample from the subject, the presence of said alteration being indicative of the predisposition to autism or an autism spectrum disorder, and, administering a prophylactic treatment against autism, or an autism spectrum disorder.

In a preferred embodiment, said alteration is one or several SNP(s) or a haplotype of SNPs associated with autism. Preferably, said SNP(s) are selected from those disclosed in Tables 1a and 1b, more preferably those disclosed in Tables 3-10. More preferably, said SNP associated with autism can be selected from the group consisting of SNP106, SNP134, SNP128, SNP138, SNP140 and SNP149. Preferably, said haplotype associated with autism comprises or consists of several SNPs selected from SNP disclosed in Tables 1a and 1b. Preferably, said SNPs are selected from the group consisting of those disclosed in Tables 3-10. In a preferred embodiment, said haplotype associated with autism comprises or consists of several SNPs selected from the group consisting of SNP106, SNP134, SNP128, SNP138, SNP140, SNP139, SNP141, SNP149, SNP150 and SNP151. In a particular embodiment, said haplotype associated with autism comprises or consists of several SNPs selected from the group consisting of SNPs72, SNP75, SNP76, SNP79, SNP89, SNP1O6, SNP107, SNP109 and SNP111. Still more preferably, said haplotype is selected from the haplotypes disclosed in Tables 4, 6, 7, 9 and/or 10, in particular Tables 4, 6, 9 and 10. In a most preferred embodiment, said haplotype consists of or comprises SNP139, SNP140 and SNP141, preferably with the alleles 1-2-2, respectively. In an other most preferred embodiment, said haplotype consists of or comprises SNP149, SNP150 and SNP151, preferably with the alleles 1-2-1, respectively. When said alteration is indicative for protecting from autism, said haplotype is preferably selected from the haplotypes disclosed in Table 7. The present invention considers any particular allele of a SNP disclosed in the present invention and any combination of particular alleles of SNPs disclosed in the present invention for use in a method according to the present invention.

Preferably, the alteration in the PRKCB1 gene locus is determined by performing a hydridization assay, a sequencing assay, a microsequencing assay, an oligonucleotide ligation assay, a confirmation based assay, a melting curve analysis, a denaturing high performance liquid chromatography (DHPLC) assay or an allele-specific amplification assay.

This invention may employ compositions of matter comprising primers, probes, and/or oligonucleotides, which are designed to specifically detect at least one SNP or haplotype associated with autism in the genomic region including the PRKCB1 gene, or a combination thereof. Preferably, said SNP(s) are selected from those disclosed in Tables 1a and 1b, more preferably those disclosed in Tables 3-10. More preferably, said SNP associated with autism can be selected from the group consisting of SNP106, Snip134, SNP128, SNP138, SNP140 and SNP149. Preferably, said haplotype associated with autism comprises or consists of several SNPs selected from SNP disclosed in Tables 1a and 16. Preferably, said SNPs are selected from the group consisting of those disclosed in Tables 3-10. In a preferred embodiment, said haplotype associated with autism comprises or consists of several SNPs selected from the group consisting of SNP106, SNP134, SNP128, SNP138, SNP140, SNP139, SNP 141, SNP149, SNP 150 and SNP 151. In a particular embodiment, said haplotype associated with autism comprises or consists of several SNPs selected from the group consisting of SNP72, SNP75, SNP76, SNP79, SNP89, SNP106, SNP107, SNP109 and SNP111. Still more preferably, said haplotype is selected from the haplotypes disclosed in Tables 4, 6, 7, 9 and/or 10, in particular Tables 4, 6, 9 and 10. In a most preferred embodiment, said haplotype consists of or comprises SNP139, SNP140 and SNP141, preferably with the alleles C-G-T, respectively. In an other most preferred embodiment, said haplotype consists of or comprises SNP149, SNP150 and SNP151, preferably with the alleles C-T-A, respectively.

It is further described methods of treating autism and/or autism spectrum disorders in a subject through a modulation of PRKCB1 expression or activity. Such treatments use, for instance, PRKCB 1 polypeptides, PRKCB1 DNA sequences (including antisense sequences and RNAi directed at the PRKCB1 gene locus), anti- PRKCB1 antibodies or drugs that modulate PRKCB1 expression or activity.

It is also described methods of treating individuals who carry deleterious alleles of the PRKCB1 gene, including pre-symptomatic treatment or combined therapy, such as through gene therapy, protein replacement therapy or through the administration of PRKCB 1 protein mimetics and/or inhibitors.

A further aspect of this invention resides in the screening of drugs for therapy of autism or autism spectrum disorder, based on the modulation of or binding to an allele of PRKCB 1 gene associated with autism or associated disorder or gene product thereof.

It is further described antibodies specific of PRKCB 1 polypeptide fragments and derivatives of such antibodies, hybridomas secreting such antibodies, and diagnostic kits comprising those antibodies. More preferably, said antibodies are specific to a PRKCB1 polypeptide or a fragment thereof comprising are alteration, said alteration modifying the activity of PRKGB1.

A PRKCB1 gene or a fragment thereof comprising an alteration is also described as well as a PRKCB1 polypeptide or a fragment thereof comprising an alteration. Preferably, said, alteration modifies the activity of PRKCB1. In a particular embodiment, said alteration is selected from the mutation disclosed in Table 12.

### LEGEND TO THE FIGURES

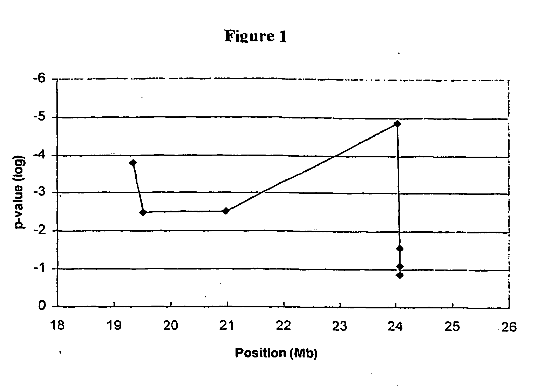

Figure 1 : High density mapping using Genomic Hybrid Identity Profiling (GenomeHIP).

A total of 2263 BAC clones with an average spacing of 1.2 Mega base pairs between clones representing the whole human genome were tested for linkage using GenomeHIP. Each point corresponds to a clone. Significant evidence for linkage was calculated for clone BACA7ZDOG (p-value 1.4x10⁻⁵). The whole linkage region encompasses a region from 134095595 base pairs to 135593528 base pairs on human chromosome 16. The p-value 2x10⁻⁵ corresponding to the significance level for significant linkage was used as a significance level for whole genome screens as proposed by Lander and Kruglyak (1995).

### DETAILED DESCRIPTION OF THE INTENTION

The present invention discloses the identification of PRKCB1 as a human autism susceptibility gene. Various nucleic acid samples from 114 families with autism were submitted to a particular GenomeHIP process. This process led to the identification of particular identical-by-descent fragments in said populations that are altered in autistic subjects. By screening of the IBD fragments, we identified the protein kinase C, beta-1 1 gene on chromosome 16p11.2 (PRKCB1) as a candidate for autism and related phenotypes. This gene is indeed present in the critical interval and expresses a functional phenotype consistent with a genetic regulation of autism. SNPs of the PRKCB1 gene were also identified, as being correlated to autism in human subjects. Among others (see Tables 3, 5 and 8), SNP106, SNP 134, SNP138, SNP 140, SNP149 and SNP 128, located in the PRKCB gene locus, was found to be associated with autism. Haplotypes disclosed in Tables 4, 6, 9 and 10 have also been identified as associated with autism.

The present invention thus proposes to use PRKCB1 1 genre and corresponding expression products for the diagnosis of autism, and autism spectrum disorders, as well as for the screening of therapeutically active drugs.

### DEFINITIONS

Autism and autism spectrum disorders (ASDs): Autism is typically characterized as part of a spectrum of disorders (ASDs) including Asperger syndrome (AS) and other pervasive developmental disorders (PPD). Autism shall be construed as any condition of impaired social interaction and communication with restricted repetitive and stereotyped patterns of behavior, interests and activities present before the age of 3, to the extent that health may be impaired. AS is distinguished from autistic disorder by the lack of a clinically significant delay in language development in the presence of the impaired social interaction and restricted repetitive behaviors, interests, and activities that characterize the autism-spectrum disorders (ASDs). PPD-NOS (PPD, not otherwise specified) is used to categorize children who do not meet the strict criteria for autism but who come close, either by manifesting atypical autism or by nearly meeting the diagnostic criteria in two or three of the key areas.

The invention may be used in various subjects, particularly human, including adults, children and at the prenatal stage.

Within the context of this invention, the PRKCB1 gene locus designates all PRKCB1 sequences or products in a cell or organism, including PRKCB1 coding sequences, PRKCB 1 non-coding sequences (e.g., introns), PRKCB 1 regulatory sequences controlling transcription, translation and/or stability (e.g., promoter, enhancer, terminator, etc.), as well as all corresponding expression products, such as PRKCB1 RNAs (e.g., mRNAs) and PRKCB1 polypeptides (e.g., a pre-protein and a mature protein). The PRKCB1 gene locus also comprise surrounding sequences of the PRKGB1 gene which include SNPs that are in linkage disequilibrium with SNPs located in the PRKCB 1 gene. For example, the PRKCB1 1 locus comprises surrounding sequences disclosed in Tables 1a and/or 1b.

As used in the present application, the term "PRKCB1 gene" designates the protein kinase C, beta-1 gene on human chromosome 16p11.2, as well as variants, analogs and fragments thereof, including alleles thereof (e.g., germline mutations) which are related to susceptibility to autism and autism spectrum disorders. The PRKCB 1 gene may also be referred to as HGNC:9395, MGC41878, PKC-beta, PKCB, PRKCB, PRKCB2, protein kinase C, beta, protein kinase C, beta 1 polypeptide.

The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding PRKCB1, i.e., any non naturally occurring nucleic acid molecule created artificially, e.g., by assembling, cutting, ligating or amplifying sequences. A PRKCB1 gene is typically double-stranded, although other forms may be contemplated, such as single-stranded. PRKCB 1 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable PRKCB1 gene sequences may be found on gene banks, such as Unigene Cluster for PRKCB 1 (Hs.349845) and Unigene Representative Sequence NM 002738. A particular example of a PRKCB 1 gene comprises SEQ ID No: 1 or 63.

The term "PRKCB1 gene" includes any variant, fragment or analog of SEQ ID No 1 or 63 or of any coding sequence as identified above. Such variants include, for instance, naturally-occurring variants due to allelic variations between individuals (e.g., polymorphisms), mutated alleles related to autism, alternative splicing forms, etc. The term variant also includes PRKCB1 gene sequences from other sources or organisms. Variants are preferably substantially homologous to SEQ ID No 1 or 63, i.e., exhibit a nucleotide sequence identity of at least about 65%, typically at least about 75%, preferably at least about 85%, more preferably at least about 95% with SEQ ID No 1. Variants and analogs of a PRKCB1 gene also include nucleic acid sequences, which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a PRKCB1 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide lengths between 8 and 100 nucleotides, preferably between 15 and 100, more preferably between 20 and 100.

A PRKGB1 polypeptide designates any protein or polypeptide encoded by a PRKCB1 gene as disclosed above. The term "polypeptide" refers to any molecule comprising a stretch of amino acids. This term includes molecules of various lengths, such as peptides and proteins. The polypeptide may be modified, such as by glycosylations and/or acetylations and/or chemical reaction or coupling, and may contain one or several non-natural or synthetic amino acids. A specific example of a PRKCB 1 polypeptide comprises all or part of SEQ ID No: 2 (SNP_002729).

The terms "response to a treatment" refer to treatment efficacy, including but not limited to ability to metabolise a therapeutic compound, to the ability to convert a pro-drug to an active drug, and to the pharmacokinetics (absorption, distribution, elimination) and the pharmacodynamics (receptor-related) of a drug in an individual.

The terms "adverse effects to a treatment" refer to adverse effects of therapy resulting from extensions of the principal pharmacological action of the drug or to idiosyncratic adverse reactions resulting from an interaction of the drug with unique host factors. "Side effects to a treatment" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

### DIAGNOSIS

The invention now provides diagnosis methods based on a monitoring of the PRKCB 1 gene locus in a subject. Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmacogenetics), etc.

The present invention provides diagnostic methods to determine whether an individual is at risk of developing autism, or an autism spectrum disorder, or suffers from autism, or an autism spectrum disorder, resulting from a mutation or a polymorphism in the PRKCB1 gene locus. The present invention also provides methods to determine whether an individual is likely to respond positively to a therapeutic agent or whether an individual is at risk of developing an adverse side effect to a therapeutic agent.

A particular object of this invention resides in a method of detecting the presence of or predisposition to autism, or an autism spectrum disorder in a subject, the method comprising detecting in a sample from the subject the presence of an alteration in the PRKCB1 gene locus in said sample. The presence of said alteration is indicative of the presence or predisposition to autism, or an autism spectrum disorder. Optionally, said method comprises a previous step of providing a sample from a subject. Preferably, the presence of an alteration in the PRKCB 1 gene locus in said sample is detected through the genotyping of a sample.

Another particular object of this invention resides in a method of detecting the protection from autism, or an autism spectrum disorder, in a subject, the method comprising detecting the presence of an alteration in the PRKCB1 gene locus in a sample from the subject, the presence of said alteration being indicative of the protection from autism, or an autism spectrum disorder.

In a preferred embodiment, said alteration is one or several SNP(s) or a haplotype of SNPs associated with autism. Preferably, said SNP(s) are selected from those disclosed in Tables 1a and 1b, more preferably those disclosed in Tables 3-10. More preferably, said SNP associated with autism can be selected from the group consisting of SNP106, SNP134, SNP128, SNP138, SNP140 and SNP149. Preferably, said haplotype associated with autism comprises or consists of several SNPs selected from SNP disclosed in Tables 1a and 1b. Preferably, said SNPs are selected from the group consisting of those disclosed in Tables 3-10. In a preferred embodiment, said haplotype associated with autism comprises or consists of several SNPs selected from the group consisting of SNP106, SNP134, SNP128, SNP138, SNP140, SNP139, SNP141, SNP149, SNP150 and SNP151. In a particular embodiment, said haplotype associated with autism comprises or consists of several SNPs selected from the group consisting of SNP71, SNP72, SNP75, SNP76, SNP79, SNP89, SNP106, SNP107, SNP109 and SNP111. Still more preferably, said haplotype is selected from the haplotypes disclosed in Tables 4, 6, 7, 9 and/or 10, in particular Tables 4, 6, 9 and 10. In a most preferred embodiment, said haplotype consists of or comprises SNPs39, SNP140 and SNP141, preferably with the alleles 1-2-2, respectively. In an other most preferred embodiment, said haplotype consists of or comprises SNP149, SNP150 and SNP151, preferably with the alleles 1-2-1, respectively. When said alteration is indicative for protecting from autism, said haplotype is preferably selected from the haplotypes disclosed in Table 7.

Another particular object of this invention resides in a method of assessing the response of a subject to a treatment of autism, or an autism spectrum disorder, the method comprising (i) providing a sample from the subject and (ii) detecting the presence of an alteration in the PRKCB1 gene locus in said sample.

Another particular object of this invention resides in a method of assessing the response of a subject to a treatment of autism, or an autism spectrum disorder, the method comprising detecting in a sample from the subject the presence of an alteration in the PRKCB1 gene locus in said sample. The presence of said alteration is indicative of a particular response to said treatment. Preferably, the presence of an alteration in the PRKCB1 gene Locus in said sample is detected through the genotyping of a sample.

A further particular object of this invention resides in a method of assessing the adverse effects of a subject to a treatment of autism, or an autism spectrum disorder, the method comprising detecting in a sample from the subject the presence of an alteration in the PRKCB1 gene locus in said sample. The presence of said alteration is indicative of adverse effects to said treatment. Preferably, the presence of an alteration in the PRKCB1 gene locus in said sample is detected through the genotyping of a sample.

In a preferred embodiment, said alteration is one or several SNP(s) or a haplotype of SNPs associated with autism. Preferably, said SNP(s) are selected from those disclosed in Tables 1a and 1b, more preferably those disclosed in Tables 3-10. More preferably, said SNP associated with autism can be selected from the group consisting of SNP106, SNP134, SNP128, SNP138, SNP140 and SNP149. Preferably, said haplotype associated with autism comprises or consists of several SNPs selected from SNP disclosed in Tables 1a and 1b. Preferably, said SNPs are selected from the group consisting of those disclosed in Tables 3-10. In a preferred embodiment, said haplotype associated with autism comprises or consists of several SNPs selected from the group consisting of SNP106, SNP134, SNP128, SNP138, SNP140, SNP139, SNP141, SNPs149, SNP150 and SNP151. In a particular embodiment, said haplotype associated with autism comprises or consists of several SNPs selected from the group consisting of SNP72, SNP75, SNP76, SNP79, SNP89, SNP106, SNP107, SNPI09 and SNP111. Still more preferably, said haplotype is selected from the haplotypes disclosed in Tables 4, 6, 7, 9 and/or 10, in particular Tables 4, 6, 9 and 10. In a most preferred embodiment, said haplotype consists of or comprises SNP139, SNP140 and SNP141, preferably with the alleles 1-2-2, respectively. In an other most preferred embodiment, said haplotype consists of or comprises SNP149, SNP150 and SNP151, preferably with the alleles 1-2-1, respectively.

In an additional embodiment, it is disclosed a method for preventing autism or an autism spectrum disorder in a subject, comprising detecting the presence of an alteration in the PRKCB1 gene locus in a sample from the subject, the presence of said alteration being indicative of the predisposition to autism, or an autism spectrum disorder ; and, administering a prophylactic treatment against autism, an autism spectrum disorder. Said prophylactic treatment can be a drug administration. Said prophylactic treatment can also be a behavioral therapy.

Diagnostics, which analyse and predict response to a treatment or drug, or side effects to a treatment or drug, may be used to determine whether an individual should be treated with a particular treatment drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug or behavioral therapy, an alternative course of treatment may be prescribed. A negative response may be defined as either the absence of an efficacious response or the presence of toxic side effects.

Clinical drug trials represent another application for the PRKCB1 SNPs. One or more PRKCB1 SNPs indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

Clinical trials to assess the utility of a behavioural therapy are also an application for the PRKCB1 SNPs. One or more PRKCB1 SNPs indicative of response to a behavioural therapy or to side effects to a behavioral therapy may be identified using the methods described above. Thereafter, potential participants in clinical trials of such a therapy may be screened to identify those individuals most likely to respond favorably to the therapy and exclude those likely to experience side effects. In that way, the effectiveness of behavioral treatment may be measured in individuals who respond positively to the therapy, without lovering the measurement as a result of the inclusion of individuals who are unlikely to respond positively in the study and without risking undesirable safety problems.

The alteration may be determined at the level of the PRKCB1 gDNA, RNA or polypeptide. Optionally, the detection is determined by performing a hydridization assay, a sequencing assay, a microsequencing assay, an oligonucleotide ligation assay, a confirmation based assay, a melting curve analysis, a denaturing high performance liquid chromatography (DHPLC) assay (Jones et al, 2000) or an allele-specific amplification assay. In a particular embodiment, the detection is performed by sequencing all or part of the PRKCB1 gene or by selective hybridisation or amplification of all or part of the PRKCB1 gene. More preferably a PRKCB1 gene specific amplification is carried out before the alteration identification step.

An alteration in the PRKCB1 gene locus may be any form of mutation(s), deletion(s), rearrangement(s) and/or insertions in the coding and/or non-coding region of the locus, alone or in various combination(s). Mutations more specifically include point mutations. Deletions may encompass any region of one, two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The PRKCB1 gene locus alteration may result in the creation of stop codons, frameshift mutations, amino acid substitutions, particular RNA splicing or processing, product instability, truncated polypeptide production, etc. The alteration may result in the production of a PRKCB1 polypeptide with altered function, stability, targeting or structure. The alteration may also cause a reduction in protein expression or, alternatively, an increase in said production.

In a particular embodiment of the method according to the present invention, the alteration in the PRKCB1 gene locus is selected from a point mutation, a deletion and an insertion in the PRKCB1 gene or corresponding expression product, more preferably a point mutation and a deletion. The alteration may be determined at the level of the PRKGB 1 gDNA, RNA or polypeptide.

In this regard, the present invention now discloses a SNP in the PRKCB1 gene and certain haplotypes, which include SNPs selected from the group consisting of SNP71, SNP72, SNP75, SNP76, SNP79, SNP89, SNP106, SNP107, SNP109 and SNP111, that are associated with autism. The SNPs are reported in the following Table 1a.

**Table 1a**

| Nucleotide position in genomic sequence of chromosome 16 (Build34) | SNP identity | dbSNP or Celera reference | Polymorphism | Position in locus and type of amino acid change | Sequence reference |
|---|---|---|---|---|---|
| 22122182 | SNP72 | hCV2844131 | A/G | 5' of PRKCB1 locus | 3 |
| 22198979 | SNP75 | hCV3079140 | G/T | 5' of PRKCB1 locus | 4 |
| 22296591 | SNP76 | rs2926362 | C/T | 5' of PRKCB1 locus | 5 |
| 22825556 | SNP79 | hCV2613285 | A/G | 5' of PRKCB1 locus | 6 |
| 23297260 | SNP89 | rs886113 | A/G | 5' of PRKCB1 locus | 7 |
| 23870016 | SNP106 | rs2878156 | C/T | PRKCB1 intron | 8 |
| 23911036 | SNP107 | rs3785392 | A/G | PRKCB1 intron | 9 |
| 23962728 | SNP109 | hCV11191069 | A/G | PRKCB1 intron | 10 |
| 23989995 | SNP111 | hCV1936109 | C/G | PRKCB1 intron | 11 |

**Table 1b**

| Nucleotide position in , genomic sequence of chromosome 16 (Build34) | SNP identity | dbSNP or Celera reference | Polymorphism | Sequence reference |
|---|---|---|---|---|
| 23817666 | SNP114 | rs916678 | A=1/C=2 | 12 |
| 23833950 | SNP117 | hCV2192055/rs3826262 | C=1/T=2 | 13 |
| 23834328 | SNP118 | rs2188355 | C=1/T=2 | 14 |
| 23842130 | SNP119 | hCV2192062/rs12928700 | A=1/C=2 | 15 |
| 23843728 | SNP120 | rs3826261 | C=1/T=2 | 16 |
| 23845935 | SNP121 | rs6497692 | C=1/T=2 | 17 |
| 23849100 | SNP122 | rs1468130 | A=1/G=2 | 18 |
| 23850031 | SNP123 | hCV2192075/rs11646426 | A=1/G=2 | 19 |
| 23863612 | SNP126 | C_11192702_10/rs9924860 | A=1/C=2 | 20 |
| 23870016 | SNP128 | hCV2192108/rs2878156 | C=1/T=2 | 21 |
| 23888016 | SNP131 | rs6497703 | A=1/G=2 | 22 |
| 23893027 | SNP133 | rs3785394 | C=1/T=2 | 23 |
| 23898266 | SNP134 | C_2192130_10/rs2188356 | C=1/G=2 | 24 |
| 23898266 | SNP135 | hCV2192130/rs2188356 | C=1/G=2 | 25 |
| 23911036 | SNP136 | hCV11192725/rs3785392 | A=1/G=2 | 26 |
| 23912938 | SNP137 | rs195990 | A=1/C=2 | 27 |
| 23925972 | SNP138 | rs3890662 | A=1/G=2 | 28 |
| 23928846 | SNP139 | rs3785387 | C=1/T=2 | 29 |
| 23929790 | SNP140 | hCV946275/rs196002 | A=1/G=2 | 30 |
| 23937230 | SNP141 | rs1873423 | C=1/T=2 | 31 |
| 23942845 | SNP142 | rs4238948 | A=1/G=2 | 32 |
| 23962728 | SNP145 | hCV11191069/rs4788103 | A=1/G=2 | 33 |
| 23967460 | SNP147 | hCV1936120/rs7194004 | A=1/G=2 | 34 |
| 23981619 | SNP149 | hCV9609165/rs14907.54 | C=1/T=2 | 35 |
| 23984187 | SNP150 | rs195992 | C=1/T=2 | 36 |
| 23986260 | SNP151 | rs6497712 | A=1/G=2 | 37 |
| 23989995 | SNP152 | hCV1936109/rs8058691 | C=1/G=2 | 38 |
| 23998657 | SNP155 | C_11191083_10/rs12922749 | C=1/T=2 | 39 |
| 24001130 | SNP156 | rs1021385 | A=1/G=2 | 40 |
| 24085389 | SNP177 | rs6497722 | C=1/G=2 | 41 |
| 24090173 | SNP178 | rs582161 | A=1/G=2 | 42 |
| 24092299 | SNP179 | rs6497725 | G=1/T=2 | 43 |
| 24094916 | SNP180 | rs174828 | A=1/C=2 | 44 |
| 24096736 | SNP181 | C_2976331_10/rs9937112 | A=1/G=2 | 45 |
| 24098935 | SNP183 | rs198182 | A=1/G=2 | 46 |
| 24101233 | SNP184 | rs 182068 | C=1/G=2 | 47 |
| 24107610 | SNP186 | rs183204 | A=1/G=2 | 48 |
| 24109398 | SNP187 | rs420414 | A=1/G=2 | 49 |
| 24137526 | SNP197 | rs2051684 | C=1/T=2 | 50 |
| 24139500 | SNP198 | rs1126289 | C=1/T=2 | 51 |
| 24144684 | SNP199 | rs198207 | A=1/G=2 | 52 |
| 24145792 | SNP200 | rs2283548 | C=1/T=2 | 53 |
| 24150994 | SNP201 | rs198163 | C=1/T=2 | 54 |
| 24155057 | SNP203 | hCV2976238/rs12448206 | A=1/G=2 | 55 |
| 24167134 | SNP206 | rs198143 | C=1/T=2 | 56 |
| 24169166 | SNP207 | hCV2976229/rs198145 | A=1/G=2 | 57 |
| 24176913 | SNP209 | hCV8918943/rs1015408 | A=1/T=2 | 58 |
| 24196360 | SNP210 | hCV2976211/rs2239338 | A=1/G=2 | 59 |
| 24196754 | SNP211 | rs411103 | A=1/T=2 | 60 |
| 24197799 | SNP213 | hCV2976205/rs3729908 | C=1/T=2 | 61 |
| 24201448 | SNP214 | rs198148 | C=1/T=2 | 62 |

In any method according to the present invention, one or several SNP in the PRKCB1 gene and certain haplotypes comprising SNP in the PRKCB1 gene and surrounding regions can be used in combination with other SNP or haplotype associated with autism, or an autism spectrum disorder and located in other gene(s).

In another variant, the method comprises detecting the presence of an altered PRKCB 1 RNA expression. Altered RNA expression includes the presence of an altered RNA sequence, the presence of an altered RNA splicing or processing, the presence of an altered quantity of RNA, etc. These may be detected by various techniques known in the art, including by sequencing all or part of the PRKCB1 RNA or by selective hybridisation or selective amplification of all or part of said RNA, for instance.

In a further variant, the method comprises detecting the presence of an altered PRKCB1 polypeptide expression. Altered PRKCB1 polypeptide expression includes the presence of an altered polypeptide sequence, the presence of an altered quantity of PRKCB1 polypeptide, the presence of an altered tissue distribution, etc. These may be detected by various techniques known in the art, including by sequencing and/or binding to specific ligands (such as antibodies), for instance.

As indicated above, various techniques known in the art may be used to detect or quantify altered PRKCB1 gene or RNA expression or sequence, including sequencing, hybridisation, amplification and/or binding to specific ligands (such as antibodies). Other suitable methods include allele-specific oligonucleotide (ASO), oligonucleotide ligation, allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, denaturing HLPC, melting curve analysis, heteroduplex analysis, RNase protection, chemical or enzymatic mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild type or altered PRKCB1 gene or RNA. The probe may be in suspension or immobilized on a substrate. The probe is typically labeled to facilitate detection of hybrids.

Some of these approaches are particularly suited for assessing a polypeptide sequence or expression level, such as Northern blot, ELISA and RIA. These latter require the use of a ligand specific for the polypeptide, more preferably of a specific antibody.

In a particular, preferred, embodiment, the method comprises detecting the presence of an altered PRKCB1 gene expression profile in a sample from the subject. As indicated above, this can be accomplished more preferably by sequencing, selective hybridisation and/or selective amplification of nucleic acids present in said sample.

### Sequencing

Sequencing can be carried out using techniques well known in the art, using automatic sequencers. The sequencing may be performed on the complete PRKCB 1 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerise chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the PRKCB1 gene or locus are able to specifically hybridize with a portion of the PRKCB1 gene locus that flank a target region of said locus, said target region being altered in certain subjects having autism, an autism spectrum disorder, or an autism-associated disorder. Examples of such target regions are provided in Tables 1a and 1b.

Primers that can be used to amplify PRKCB1 target region comprising SNPs as identified in Table 1 may be designed based on the sequence of Seq Id No 1 or 63 or on the genomic sequence of PRKCB1. In a particular embodiment, primers may be designed based on the sequence of SEQ ID Nos 3-62.

Another particular object of this invention resides in a nucleic acid primer useful for amplifying sequences from the PRKCB1 gene or locus including surrounding regions. Such primers are preferably complementary to, and hybridize specifically to nucleic acid sequences in the PRKCB1 gene locus. Particular primers are able to specifically hybridise with a portion of the PRKCB1 gene locus that flank a target region of said locus, said target region being altered in certain subjects having autism, an autism spectrum disorder, or an autism-associated disorder.

The invention also relates to a nucleic acid primer, said primer being complementary to and hybridizing specifically to a portion of a PRKCB1 coding sequence (e.g., gene or RNA) altered in certain subjects having autism, an autism spectrum disorder, or an autism-associated disorder. In this regard, particular primers of this invention are specific for altered sequences in a PRKCB1 gene or RNA. By using such primers, the detection of an amplification product indicates the presence of an alteration in the PRKCB1 gene locus. In contrast, the absence of amplification product indicates that the specific alteration is not present in the sample.

Typical primers of this invention are single-stranded nucleic acid molecules of about 5 to 60 nucleotides in length, more preferably of about 8 to about 25 nucleotides in length. The sequence can be derived directly from the sequence of the PRKCB1 gene locus. Perfect complementarity is preferred, to ensure high specificity. However, certain mismatch may be tolerated.

The invention also concerns the use of a nucleic acid primer or a pair of nucleic acid primers as described above in a method of detecting the presence of or predisposition to autism, an autism spectrum disorder, or an autism-associated disorder in a subject or in a method of assessing the response of a subject to a treatment of autism, an autism spectrum disorder, or an autism-associated disorder.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild type or altered PRKCB1 gene or RNA, followed by the detection of the presence of a hybrid. The probe may be in suspension or immobilized on a substrate or support (as in nucleic acid array or chips technologies). The probe is typically labeled to facilitate detection of hybrids.

In this regard, a particular embodiment of this invention comprises contacting the sample from the subject with a nucleic acid probe specific for an altered PRKCB 1 gene locus, and assessing the formation of an hybrid. In a particular, preferred embodiment, the method comprises contacting simultaneously the sample with a set of probes that are specific, respectively, for wild type PRKCB1 gene locus and for various altered forms thereof. In this embodiment, it is possible to detect directly the presence of various forms of alterations in the PRKCB1 gene locus in the sample. Also, various samples from various subjects may be treated in parallel.

Within the context of this invention, a probe refers to a polynucleotide sequence which is complementary to and capable of specific hybridisation with a (target portion of a) PRKCB1 gene or RNA, and which is suitable for detecting polynucleotide polymorphisms associated with PRKCB1 alleles which predispose to or are associated with autism, an autism spectrum disorder, or an autism-associated disorder. Probes are preferably perfectly complementary to the PRKCB1 gene, RNA, or target portion thereof. Probes typically comprise single-stranded nucleic acids of between 8 to 1000 nucleotides in length, for instance of between 10 and 800 more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridise to a region of a PRKCB1 gene or RNA that carries an alteration.

A specific embodiment of this invention is a nucleic acid probe specific for an altered (e.g., a mutated) PRKCB1 gene or RNA, i.e., a nucleic acid probe that specifically hybridises to said altered PRKGB1 gene or RNA and essentially does not hybridise to a PRKCB1 gene or RNA lacking said alteration. Specificity indicates that hybridisation to the target sequence generates a specific signal which can be distinguished from the signal generated through non-specific hybridisation. Perfectly complementary sequences are preferred to design probes according to this invention. It should be understood, however, that certain a certain degree of mismatch may be tolerated, as long as the specific signal may be distinguished from non-specific hybridisation.

Particular examples of such probes are nucleic acid sequences complementary to a target portion of the genomic region including the PRKCB1 gene or RNA carrying a point mutation as listed in Tables 1a and 1b above. More particularly, the probes can comprise a sequence selected from the group consisting of SEQ ID Nos 3-62 or a fragment thereof comprising the SNP or a complementary sequence thereof.

The sequence of the probes can be derived from the sequences of the PRKCB 1 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labeling, etc.

The invention also concerns the use of a nucleic acid probe as described above in a method of detecting the presence of or predisposition to autism, an autism spectrum disorder, or an autism associated disorder in a subject or in a method of assessing the response of a subject to a treatment of autism, or an autism spectrum disorder.

### Oligonuleotide legation

The oligonucleotide ligation assay is a method consists of designing 3 specific primers per SNP with two primers carrying the SNP-base specific 3' end and one common primer that starts 5' with the next base in the target sequence. The two allele specific primers carry a tag of unique sequences that determine each allele. Primers are annealed to the target sequence and a ligation reaction will join the allele specific primer with the common primer if the allele specific 3'-base is present. A short fluorescent dye labelled probe homologous to the tag of unique sequence, is then hybridised to the immobilized product enabling the detection of the corresponding allele. An oligo-ligation kit is commercially available (SNPlex, Applied Biosystems, Foster City).

### Specific Ligand Binding

As indicated above, alteration in the PRKCB1 gene locus may also be detected by screening for alteration(s) in PRKCB1 polypeptide sequence or expression levels. In this regard, a specific embodiment of this invention comprises contacting the sample with a ligand specific for a PRKCB1 polypeptide and determining the formation of a complex.

Different types of ligands may be used, such as specific antibodies. In a specific embodiment, the sample is contacted with an antibody specific for a PRKCB1 polypeptide and the formation of an immune complex is determined. Various methods for detecting an immune complex can be used, such as ELISA, radioimmunoassays (RIA) and immuno-enzymatic assays (IEMA).

Within the context of this invention, an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly-functional antibodies, etc.

An antibody specific for a PRKCB 1 polypeptide designates an antibody that selectively binds a PRKCB1 polypeptide, namely, an antibody raised against a PRKCB1 polypeptide or an epitope-containing fragment thereof. Although non-specific binding towards other antigens may occur, binding to the target PRKCB1 polypeptide occurs with a higher affinity and can be reliably discriminated from non-specific binding.

In a specific embodiment, the method comprises contacting a sample from the subject with (a support coated with) an antibody specific for an altered form of a PRKCB 1 polypeptide, and determining the presence of an immune complex. In a particular embodiment, the sample may be contacted simultaneously, or in parallel, or sequentially, with various (supports coated with) antibodies specific for different forms of a PRKCB1 polypeptide, such as a wild type and various altered forms thereof.

The invention also concerns the use of a ligand, preferably an antibody, a fragment or a derivative thereof as described above, in a method of detecting the presence of or predisposition to autism, or an autism spectrum disorder, or in a method of assessing the response of a subject to a treatment of autism, or an autism spectrum disorder.

It is further described a diagnostic kit comprising products and reagents for detecting in a sample from a subject the presence of an alteration in the PRKCB1 gene or polypeptide, in the PRKCB1 gene or polypeptide expression, and/or in PRKCB1 activity. Said diagnostic kit according to the present invention comprises any primer, any pair of primers, any nucleic acid probe and/or any ligand, preferably antibody, described in the present invention. Said diagnostic kit according to the present invention can further comprise reagents and/or protocols for performing a hybridization, amplification or antigen-antibody immune reaction.

The diagnosis methods can be performed in vitro, ex vivo or in vivo, preferably in vitro or ex vivo. They use a sample from the subject, to assess the status of the PRKCB1 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Pre-natal diagnosis may also be performed by testing fetal cells or placental cells, for instance. The sample may be collected according to conventional techniques and used directly for diagnosis or stored. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered PRKCB 1 gene locus. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered PRKCB1 polypeptide, RNA or DNA in the sample is indicative of the presence of an altered PRKCB1 gene locus in the subject, which can be correlated to the presence, predisposition or stage of progression of autism, an autism spectrum disorder, or an autism-associated disorder. For example, an individual having a germ line PRKCB1 mutation has an increased risk of developing autism, an autism spectrum disorder, or an autism-associated disorder. The determination of the presence of an altered PRKCB1 gene locus in a subject also allows the design of appropriate therapeutic intervention, which is more effective and customized. Also, this determination at the pre-symptomatic level allows a preventive regimen to be applied.

### Linkage Disequilibirum

Once a first SNP has been identified in a genomic region of interest, more particularly in PRKCB1 gene locus, the practitioner of ordinary skill in the art can easily identify additional SNPs in linkage disequilibrium with this first SNP. Indeed, any SNP in linkage disequilibrium with a first SNP associated with autism or an associated disorder will be associated with this trait. Therefore, once the association has been demonstrated between a given SNP and autism or an associated disorder, the discovery of additional SNPs associated with this trait can be of great interest in order to increase the density of SNPs in this particular region.

Identification of additional SNPs in linkage disequilibrium with a given SNP involves: (a) amplifying a fragment from the genomic region comprising or surrounding a first SNP from a plurality of individuals; (b) identifying of second SNPs in the genomic region harboring or surrounding said first SNP; (c) conducting a linkage disequilibrium analysis between said first SNP and second SNPs; and (d) selecting said second SNPs as being in linkage disequilibrium with said first marker. Subcombinations comprising steps (b) and (c) are also contemplated.

Methods to identify SNPs and to conduct linkage disequilibrium analysis can be carried out by the skilled person without undue experimentation by using well-known methods.

These SNPs in linkage disequilibrium can also be used in the methods according to the present invention, and more particularly in the diagnosic methods according to the present invention.

For example, a linkage locus of Crohn's disease has been mapped to a large region spanning 18cM on chromosome 5q31 (Rioux et al., 2000 and 2001). Using dense maps of microsatellite markers and SNPs across the entire region, strong evidence of linkage disequilibrium (LD) was found. Having found evidence of LD, the authors developed an ultra-high-density SNP map and studied a denser collection of markers selected from this map. Multilocus analyses defined a single common risk haplotype characterised by multiple SNPs that were each independently associated using TDT. These SNPs were unique to the risk haplotype and essentially identical in their information content by virtue of being in nearly complete LD with one another. The equivalent properties of these SNPs make it impossible to identify the causal mutation within this region on the basis of genetic evidence alone.

### Causal Mutation

Mutations in the PRKCB1 gene which are responsible for autism or an associated disorder may be identified by comparing the sequences of the PRKCB1 gene from patients presenting autism or an associated disorder and control individuals. Based on the identified association of SNPs of PRKCB1 and autism or an associated disorder, the identified locus can be scanned for mutations. In a preferred embodiment, functional regions such as exons and splice sites, promoters and other regulatory regions of the PRKCB1 gene are scanned for mutations. Preferably, patients presenting autism or an associated disorder carry the mutation shown to be associated with autism or an associated disorder and controls individuals do not carry the mutation or allele associated with autism or an associated disorder. It might also be possible that patients presenting autism or an associated disorder carry the mutation shown to be associated with autism or an associated disorder with a higher frequency than controls individuals.

The method used to detect such mutations generally comprises the following steps: amplification of a region of the PRKCB1 gene comprising a SNP or a group of SNPs associated with autism or an associated disorder from DNA samples of the PRKCB1 gene from patients presenting autism or an associated disorder and control individuals; sequencing of the amplified region; comparison of DNA sequences of the PRKCB1 gene from patients presenting autism or an associated disorder and control individuals; determination of mutations specific to patients presenting autism or an associated disorder.

Therefore, identification of a causal mutation in the PRKCB1 gene can be carried out by the skilled person without undue experimentation by using well-known methods.

For example, the causal mutations have been identified in the following examples by using routine methods.

Hugot et al. (2001) applied a positional cloning strategy to identify gene variants with susceptibly to Crohn's disease in a region of chromosome 16 previously found to be linked to susceptibility to Crohn's disease. To refine the location of the potential sucecptibility locus 26 microsatellite markers were genotyped and tested for association to Crohn's disease using the transmission disequilibrium test. A borderline significant association was found between one allele of the microsatellite marker D16S136. Eleven additional SNPs were selected from surrounding regions and several SNPs showed significant association. SNP5-8 from this region were found to be present in a single exon of the NOD2/CARD15 gene and shown to be non-synonymous variants. This prompted the authors to sequence the complete coding sequence of this gene in 50 CD patients. Two additional non-synonymous mutations (SNP12 and SNP13) were found. SNP13 was most significant associated (p=6x10-6) using the pedigree transmission disequilibrium test. In another independent study, the same variant was found also by sequencing the coding region of this gene from 12 affected individuals compared to 4 controls (Ogura et al., 2001). The rare allele of SNP13 corresponded to a 1-bp insertion predicted to truncate the NOD2/CARD15 protein. This allele was also present in normal healthy individuals, albeit with significantly lower frequency as compared to the controls.

Similarly, Lesage et al. (2002) performed a mutational analyses of CARD15 in 453 patents with CD, including 166 sporadic and 287 familial cases, 159 patients with ulcerative colitis (UC), and 103 healthy control subjects by systematic sequencing of the coding region. Of 67 sequence variations identified, 9 had an allele frequency >5% in patients with CD. Six of them were considered to be polymorphisms, and three (SNP12-R702W, SNP8-G908R, and SNP13-1007fs) were confirmed to be independently associated with susceptibility to CD. Also considered as potential disease-causing mutations (DCMs) were 27 rare additional mutations. The three main variants (R702W, G908R, and 1007fs) represented 32%, 18%, and 31%, respectively, of the total CD mutations, whereas the total of the 27 rare mutations represented 19% of DCMs. Altogether, 93% of the mutations were located in the distal third of the gene. No mutations were found to be associated with UC. In contrast, 50% of patients with CD carried at least one DCM, including 17% who had a double mutation.

### DRUG SCREENING

The present invention also provides novel targets and methods for the screening of drug candidates or leads. The methods include binding assays and/or functional assays, and may be performed in vitro, in cell systems, in animals, etc.

A particular object of this invention resides in a method of selecting biologically active compounds, said method comprising contacting in vitro a test compound with a PRKCB1 gene or polypeptide according to the present invention and determining the ability of said test compound to bind said PRKCB 1 gene or polypeptide. Binding to said gene or polypeptide provides an indication as to the ability of the compound to modulate the activity of said target, and thus to affect a pathway leading to autism, or an autism spectrum disorder, in a subject. In a preferred embodiment, the method comprises contacting in vitro a test compound with a PRKCB1 polypeptide or a fragment thereof according to the present invention and determining the ability of said test compound to bind said PRKCB polypeptide or fragment. The fragment preferably comprises a binding site of the PRKCB1 polypeptide. Preferably, said PRKCB1 gene or polypeptide or a fragment thereof is an altered or mutated PRKCB1 gene or polypeptide or a fragment thereof comprising the alteration or mutation.

A particular object of this invention resides in a method of selecting compounds active on autism, and autism spectrum disorders said method comprising contacting in vitro a test compound with a PHKCB1 polypeptide according to the present invention or binding site-containing fragment thereof and determining the ability of said test compound to bind said PRKCB1 polypeptide or fragment thereof. Preferably, said PRKCB1 polypeptide or a fragment thereof is an altered or mutated PRKCB1 polypeptide or a fragment thereof comprising the alteration or mutation.

In a further particular embodiment, the method comprises contacting a recombinant host cell expressing a PRKCB1 polypeptide according to the present invention with a test compound, and determining the ability of said test compound to bind said PRKCB1 and to modulate the activity of PRKCB1 polypeptide. Preferably, said PRKCB1 polypeptide or a fragment thereof is an altered or mutated PRKCB1 polypeptide or a fragment thereof comprising the alteration or mutation.

The determination of binding may be performed by various techniques, such as by labeling of the test compound, by competition with a labeled reference ligand, etc.

A further object of this invention resides in a method of selecting biologically active compounds, said method comprising contacting in vitro a test compound with a PRKCB 1 polypeptide according to the present invention and determining the ability of said test compound to modulate the activity of said PRKCB1 polypeptide. Preferably, said PRKCB1 polypeptide or a fragment thereof is an altered or mutated PRKCB1 polypeptide or a fragment thereof comprising the alteration or mutation.

A further object of this invention resides in a method of selecting biologically active compounds, said method comprising contacting in vitro a test compound with a PRKCB1 gene according to the present invention and determining the ability of said test compound to modulate the expression of said PRKCB 1 gene. Preferably, said PRKCB 1 gene or a fragment thereof is an altered or mutated PRKCB1 gene or a fragment thereof comprising the alteration or mutation.

In an other embodiment, this invention relates to a method of screening, selecting or identifying active compounds, particularly compounds active on autism, or an autism spectrum disorder, the method comprising contacting a test compound with a recombinant host cell comprising a reporter construct, said reporter construct comprising a reporter gene under the control of a PRKCB1 gene promoter, and selecting the test compounds that modulate (e.g. stimulate or reduce) expression of the reporter gene. Preferably, said PRKCB1 gene promoter or a fragment thereof is an altered or mutated PRKCB1 gene promoter or a fragment thereof comprising the alteration or mutation.

In a particular embodiment of the methods of screening, the modulation is an inhibition. In another particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

Further aspects and advantages of the present invention will be disclosed in the following experimental section, which should be regarded as illustrative and not limiting the scope of the present application.

### GENE, VECTORS, RECOMBINANT CELLS AND POLYPEPTIDES

It is further described products for use in diagnosis or screening. These products comprise nucleic acid molecules encoding a PRKCB1 polypeptide or a fragment thereof, vectors comprising the same, recombinant host cells and expressed polypeptides.

More particularly, it is described an altered or mutated PRKCB1 gene or a fragment thereof comprising said alteration or mutation. The invention also concerns nucleic acid molecules encoding an altered or mutated PRKCB1 polypeptide or a fragment thereof comprising said alteration or mutation. Said alteration or mutation modifies the PRKCB1 activity. The modified activity can be increased or decreased. The invention further discloses a vector comprising an altered or mutated PRKCB 1 gene or a fragment thereof comprising said alteration or mutation or a nucleic acid molecule encoding an altered or mutated PRKCB 1 polypeptide or a fragment thereof comprising said alteration or mutation, recombinant host cells and expressed polypeptides.

It is also disclosed a vector comprising a nucleic acid encoding a PRKCB1 polypeptide according to the present invention. The vector may be a cloning vector or, more preferably, an expression vector, i.e., a vector comprising regulatory sequences causing expression of a PRKCB1 polypeptide from said vector in a competent host cell.

These vectors can be used to express a PRKCB1 polypeptide in vitro, ex vivo or in vivo, to create transgenic or "Knock Out" non-human animals, to amplify the nucleic acids, to express antisense RNAs, etc.

The vectors typically comprise a PRKCB1 coding sequence according to the present invention operably linked to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and may provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

In this regard, it is described a recombinant virus encoding a PRKCB 1 polypeptide as defined above. The recombinant virus is preferably replication-defective, even more preferably selected from E1- and/or E4-defective adenoviruses, Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

It is further disclosed a recombinant host cell comprising a recombinant PRKCB 1 gene or a vector as defined above. Suitable host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

It is also described a method for producing a recombinant host cell expressing a PRKCB1 polypeptide according to the present invention, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the PRKCB 1 polypeptide.

Such recombinant host cells can be used for the production of PRKCB 1 polypeptides, as well as for screening of active molecules, as described below. Such cells may also be used as a model system to study autism. These cells can be maintained in suitable culture media, such as DMEM, RPMI, HAM, etc., in any appropriate culture device (plate, flask, dish, tube, pouch, etc.).

### EXAMPLES

### 1. GenomeHIP platform to identify the chromosome 16 susceptibility gene

The GenomeHIP platform was applied to allow rapid identification of an autism susceptibility gene.
Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.

The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here autism). The linked interval can be delimited by the two most distant clones showing significant p-values.

In the present study, 114 families from the United States (114 independent sib-pairs) concordant for strict autism (as defined by ADI-R) were submitted to the GenomeHIP process. The resulting IBD enriched DNA fractions were then labeled with Cy5 fluorescent dyes and hybridised against a DNA array consisting of 2263 BAC clones covering the whole human genome with an average spacing of 1.2 Mega base pairs. Non-selected DNA labeled with Cy3 was used to normalize the signal values and compute ratios for each clone. Clustering of the ratio results was then performed to determine the IBD status for each clone and pair.

By applying this procedure, several BAC clones were identified (BACA18ZG03, BACA7ZF09, BACA7ZH09, BACA7ZD06, BACA27ZA05, BACA27ZD05 and BACA27ZC05) spanning approximately 5 megabases in the region on chromosome 16 (bases19333164 to 24295063), which showed evidence for linkage to autism. Significant evidence for linkage was observed for BAC clone BACA7ZD06 (p < 1.40 x 10⁻⁵)_{.}

Table 2: Linkage results for chromosome 16 in the PRKCB 1 locus: Indicated is the region corresponding to 7 BAC clones with evidence for linkage. The start and stop positions of the clones correspond to their genomic locations based on NCB1 Build34 with respect to the start of the chromosome (p-ter).

**Table 2**

| Human chromosome | Clones | Start | End | Proportion of informative pairs | p-value |
|---|---|---|---|---|---|
| 16 | BACA18ZG03 | 19333164 | 19483995 | 0.9 | 0.00016 |
| 16 | BACA7ZF09 | 19508175 | 19709301 | 0.91 | 0.0032 |
| 16 | BACA7ZH09 | 20995594 | 21170320 | 0.9 | 0.0031 |
| 16 | BACA7ZD06 | 24037143 | 24228972 | 0.91 | 1.40E-05 |
| 16 | BACA27ZA05 | 24076832 | 24271067 | 0.89 | 0.028 |
| 16 | BACA27ZD05 | 24077350 | 24222224 | 0.86 | 0.083 |
| 16 | BACA27ZC05 | 24077364 | 24295063 | 0.87 | 0.14 |

### 2. Identification of an autism susceptibility gene on chromosome 16

By screening the aforementioned 5 Megabases in the linked chromosomal region, we identified the protein kinase C, beta-1 (PRKCB1) gene as a candidate for autism and related phenotypes. This gene is indeed present in the critical interval, with evidence for linkage delimited by the clones outlined above.

Protein kinase C (PKC) is a family of serine- and threonine-specific protein kinases that can be activated by calcium and second messenger diacylglycerol. Coussens et al. (1986) defined a family of PKC-related genes in bovine, human and rat genomes. Three of these, termed alpha, beta and gamma, are highly homologous. PKC family members phosphorylate a wide variety of protein targets and are known to be involved in diverse cellular signaling pathways. PKC family members also serve as major receptors for phorbol esters, a class of tumor promoters. Each member of the PKC family has a specific expression profile and is believed to play a distinct role in cells. The protein encoded by PRKCB1 is one of the PKC family members. This protein kinase has been reported to be involved in many different cellular functions, such as B cell activation, apoptosis induction, endothelial cell proliferation, and intestinal sugar absorption. Studies in mice also suggest that this kinase may also regulate neuronal functions and correlate fear-induced conflict behavior after stress. Alternatively spliced transcript variants encoding distinct isoforms have been reported.

Two transcriptional variants are the longer isoform, protein kinase C, beta isoform 2, and isoform 1 that uses an alternate exon at the 3' end, which includes a part of the coding region.

The PKC-beta I isoform is a 76.8 kDa protein that is expressed in a limited range of normal tissues but highly expressed in brain and hematopoietic cells. PKC beta I is calcium and phosphatidylserine dependent, and is activated by diacylglycerol. It is the isoform that is most rapidly translocated to platelet membranes following exposure to phorbol esters.

Guadagno et al. (1992) established rat embryo fibroblasts with stable overproduction of protein kinase C beta I. The excess PRKCB1 results in multiple cellular growth abnormalities. They examined radio-labeled cellular phosphoproteins from either untreated or cultures treated with a phorbol ester. The most prominent phosphoprotein was MARCKS (myristoylated alanine-rich C kinase substrate). Phorbol ester treatment increased the basal level of phosphorylation of MARCKS and a prolonged increase in both the cytosolic and total cellular level of the MARCKS protein. These altered parameters of the MARCKS protein may be responsible, at least in part, for the altered phenotype of these cells.

Watterson et al. (2002) examined the effects of valproic acid (VPA), a broad-spectrum anticonvulsant, on the expression of two prominent substrates for protein kinase C in the brain, MARCKS and GAP-43, which have been implicated in actin-membrane plasticity and neurite outgrowth during neuronal differentiation, respectively, and are essential to normal brain development. Immortalized hippocampal HN33 cells exposed to VPA exhibited reduced MARCKS protein expression and demonstrated increased GAP-43 protein expression, with concomitant alterations in cellular morphology, including an increase in the number and length of neurites and accompanied by a reduction in cell growth rate. In addition, VPA-induced alteration in PKC activity, as well as temporal changes in individual PKC isozyme expression. Inhibition of PKC with the PKC-selective inhibitor, LY333531, prevented the VPA-induced down-regulation of membrane-associated MARCKS, but had no effect on the cytosolic MARCKS reduction or the GAP-43 up-regulation. Inhibition of PKC by LY333531 enhanced the differentiating effects of VPA; additionally, LY333531 alone induced greater neurite outgrowth in this cell line. Collectively, these data indicate that VPA induces neuronal differentiation, associated with a reduction in MARCKS expression and an increase in GAP-43 expression, consistent with the hypothesis that a reduction in MARCKS at the membrane may be permissive for cytoskeletal plasticity during neurite outgrowth.

Manji and Chen (2002) have reviewed the converging body of preclinical data showing that chronic lithium and VPA, at concentrations therapeutically relevant to treatment of bipolar disorder, regulate the protein kinase C signaling cascade. This has led to the investigation of the antimanic efficacy of tamoxifen (at doses sufficient to inhibit protein kinase C), with very encouraging preliminary results. A growing body of data also suggests that impairments of neuroplasticity and cellular resilience may also underlie the pathophysiology of bipolar disorder. It is thus noteworthy that mood stabilizers, such as lithium and valproate, indirectly regulate a number of factors involved in cell survival pathways--including cAMP response element binding protein (CREB), brain derived neurotrophic factor (BDNF), B-cell lymphoma protein 2 (BCL2) and mitogen-activated protein kinases (MAPK), and may thus bring about some of their delayed long-term beneficial effects via under-appreciated neurotrophic effects. The development of novel treatments, which more directly target molecules involved in critical central nervous system cell survival and cell death pathways, has the potential to enhance neuroplasticity and cellular resilience.

Birikh et al. (2003) found that in mice, the stress-induced splice variant of acetylcholinesterase, AChE-R, interacts intraneuronally with the scaffold protein RACK1 (receptor for activated C-kinase) and through it, with its target, protein kinase C beta II (isoform 2 of PRKCB1), which is known to be involved in fear conditioning. Stress-associated changes in cholinergic gene expression may regulate neuronal PKCbetaII functioning, promoting fear-induced conflict behavior after stress.

Brandon et al. (2002) described a central role for RACK-1 in potentiating PKC-dependent phosphorylation and functional modulation of gamma-aminobutyric acid receptors, alpha (GABA(A)) receptors. RACK-1 enhances functional cross talk between GABA(A) receptors and G-protein-coupled receptors and therefore may have profound effects on neuronal excitability. Various reports, for example, (Martin ER et al., 2000), describe evidence for linkage disequilibrium near the GABA(A)) receptor beta3-subunit gene on chromosome 15q11-q13 in autistic subjects.

In summary, protein kinase C (PKC) is an upstream actor in various signaling cascades, including pathways with key proteins such as MAPK or MARCKS, and thus variation in PKC structure or expression levels could affect numerous downstream events with dramatic changes in the types, levels and timing of the expression of many genes, various protein functions and interactions of the cell with its environment. Such changes, affecting for example neurite growth, neuronal differentiation or synaptic transmission, could produce a cell predisposed to a phenotype contributing to a resulting clinical autism. Particular variants of PRKCB1 or other protein kinase C isoforms may predispose to either favorable or to non-responsive outcome by agents such as VPA in the treatment of autism or autism-associated disorders.

### 3. Association study

The same families that have been used for the linkage study were also used to test for association between a specific phenotype (here autism) in question and the genetic marker allele or haplotypes containing a specific marker allele using the transmission disequilibrium test (TDT). The TDT is a powerful association test as it is insensitive to population stratification problems in the tested sample. Briefly, the segregation of alleles from heterozygous parents to their affected offspring is tested. The portion of alleles transmitted to the affected offspring compared to the non-transmitted alleles is compared to the ratio expected under random distribution. A significant excess of allele transmission over the expected value is evidence for an association of the respective allele or haplotype with the studied autism phenotype.

The results of this analysis show that certain alleles of the PRKCB1 gene are positively associated with autism and therefore increase the susceptibility to disease. In the tested population, the allele T of SNP106 that is located within an intron of the PRKCB 1 gene is correlated with autism as determined by TDT (p-value = 0.0055). In contrast, the allele C of SNP106 is under-transmitted to autistic individuals showing that this allele helps protect from the disease.

The example of the transmission to autists of the allele T of SNP106 is given in Table 3.

**Table 3**

| SNP | Allele | Frequency of allele transmitted to autists | Frequency of allele not transmitted to autists | p- value |
|---|---|---|---|---|
| SNP106 | C | 0.3679 | 0.5078 | 0.005521 |
| SNP106 | T | 0.6321 | 0.4922 | 0.005521 |
| SNP107 | A | 0.7568 | 0.6324 | 0.009242 |
| SNP107 | G | 0.2432 | 0.3676 | 0.009242 |
| SNP111 | C | 0.4124 | 0.299 | 0.01945 |
| SNP111 | G | 0.5876 | 0.701 | 0.01945 |

In addition, haplotypes were constructed for SNP71, SNP72, SNP75, SNP76, SNP79, SNP89, SNP106, SNP107, SNP109 and SNP111 to identify the phase for all SNPs.

The results of this analysis in the tested population showed that certain haplotypes, all characterized by the presence of allele T at SNP 106 are strongly associated with autism, while certain haplotypes devoid of allele G are preferentially not transmitted to autists. Examples are the haplotypes T-C for SNP106-SNP111, p = 2.97 x 10⁻⁵, and T-T-C for SNP76-SNP106-SNP111, p = 4.51 x 10⁻⁵. Haplotypes that carry allele C instead of allele T at SNP106 show evidence to be under-represented in autistic subjects. An example is the haplotype T-C-C for SNP76-SNP106-SNP111. Examples of haplotypes with preferential transmission and non-transmission of SNP106 to autists are given in Table 4.

**Table 4**

| SNPs used to construct haplotype | Haplotype | Frequency of haplotype transmitted to autists | Frequency of haplotype not transmitted to autists | p- value |
|---|---|---|---|---|
| SNP75-SNP 106 | G-T | 0.4247 | 0.2861 | 0.004344 |
| SNP75-SNP106 | T-C | 0.1005 | 0.2147 | 0.002245 |
| SNP79-SNP106 | A-C | 0.1167 | 0.2038 | 0.04621 |
| SNP79-SNP106 | A-T | 0.2619 | 0.1126 | 0.0005695 |
| SNP106-SNP107 | C-G | 0.2337 | 0.3525 | 0.009865 |
| SNP106-SNP107 | T-A | 0.6563 | 0.4773 | 0.0007929 |
| SNP106-SNP111 | C-G | 0.2291 | 0.3056 | 0.02958 |
| SNP106-SNP111 | T-C | 0.288 | 0.1078 | 2.97E-05 |
| SNP71-SNP106-SNP111 | T-C-G | 0.1773 | 0.2729 | 0.009115 |
| SNP71-SNP106-SNP111 | T-T-C | 0.2308 | 0.08357 | 0.0001321 |
| SNP72-SNP75-SNP106 | A-G-T | 0.4289 | 0.2221 | 9.64E-05 |
| SNP76-SNP106-SNP111 | T-C-C | 0.04425 | 0.1157 | 0.02953 |
| SNP76-SNP106-SNP111 | T-T-C | 0.1549 | 0.02145 | 4.51E-05 |
| SNP79-SNP106-SNP109 | A-C-A | 0.03078 | 0.1284 | 0.003604 |
| SNP79-SNP106-SNP109 | A-T-A | 0.1558 | 0.03867 | 0.0004731 |
| SNP79-SNP106-SNP111 | A-T-C | 0.1273 | 0.02865 | 0.0005642 |
| SNP89-SNP106-SNP111 | A-T-C | 0.1272 | 0.02243 | 0.0003164 |

To increase the information content and narrow down the interval of association, the SNP density in the PRKCB1 gene was increased to approximately one SNP every 2.5 kb. Several additional markers showed positive single point results in the PRKCB1 gene. The strongest association was observerd for marker SNP 134 with allele 2 being transmitted more frequently to autists than expected by chance, while allele 1 was preferentially non-transmitted to autists (p=0.002). Interestingly, marker SNP128 which is identical to SNP106 was among the markers most strongly associated in this analysis. Examples of alleles transmitted and non-transmitted to autists are shown in Table 5.

**Table 5**

| Marker | Allele | N transmitted | N non transmitted | p |
|---|---|---|---|---|
| SNP117 | 1 | 65 | 86 | 0.03109 |
| | 2 | 139 | 118 | 0.03109 |
| SNP118 | 1 | 130 | 108 | 0.01839 |
| | 2 | 58 | 80 | 0.01839 |
| SNP120 | 1 | 72 | 96 | 0.0115 |
| | 2 | 110 | 86 | 0.0115 |
| SNP121 | 1 | 109 | 86 | 0.01351 |
| | 2 | 67 | 90 | 0.01351 |
| SNP122 | 1 | 111 | 86 | 0.01037 |
| | 2 | 80 | 105 | 0.01037 |
| SNP123 | 1 | 127 | 110 | 0.02674 |
| | 2 | 31 | 48 | 0.02674 |
| SNP126 | 1 | 84 | 106 | 0.02421 |
| | 2 | 107 | 85 | 0.02421 |
| SNP128 | 1 | 72 | 99 | 0.005778 |
| | 2 | 123 | 96 | 0.005778 |
| SNP131 | 1 | 50 | 71 | 0.0202 |
| | 2 | 138 | 117 | 0.0202 |
| SNP133 | 1 | 50 | 73 | 0.01139 |
| | 2 | 139 | 116 | 0.01139 |
| SNP134 | 1 | 62 | 91 | 0.002361 |
| | 2 | 128 | 99 | 0.002361 |
| SNP135 | 1 | 57 | 83 | 0.004693 |
| | 2 | 121 | 95 | 0.004693 |
| SNP136 | 1 | 140 | 117 | 0.009242 |
| | 2 | 45 | 68 | 0.009242 |
| SNP137 | 1 | 123 | 100 | 0.0152 |
| | 2 | 64 | 87 | 0.0152 |
| SNP138 | 1 | 46 | 71 | 0.00517 |
| | 2 | 141 | 116 | 0.00517 |
| SNP139 | 1 | 140 | 116 | 0.006729 |
| | 2 | 45 | 69 | 0.006729 |
| SNP140 | 1 | 58 | 84 | 0.006405 |
| | 2 | 141 | 115 | 0.006405 |
| SNP 152 | 1 | 79 | 60 | 0.04431 |
| | 2 | 116 | 135 | 0.04431 |
| SNP156 | 1 | 39 | 56 | 0.04164 |
| | 2 | 141 | 124 | 0.04164 |
| SNP177 | 1 | 181 | 167 | 0.01562 |
| | 2 | 12 | 826 | 0.01562 |
| SNP179 | 1 | 141 | 117 | 0.002907 |
| | 2 | 32 | 56 | 0.002907 |
| SNP181 | 1 | 11 | 22 | 0.04372 |
| | 2 | 189 | 178 | 0.04372 |

Haplotypes were also constructed to determine the phase and analysed for association. The results of this analysis in the tested population showed that certain haplotypes are strongly associated with autism, the majority of which are characterized by the presence of allele 2 at SNP128, which is identical to allele T of SNP 1 06, or the presence of allele 2 of SNP138 or the presence of allele 2 at SNP140, respectively. The most significant result was obtained for haplotype 1-2-2 for markers SNP139, SNP140 and SNP141 with a p-value of 2.96 x 10⁻⁰⁵. While certain haplotypes characterised by allele 1 at SNP128 (allele G of SNP106), or allele 1 of SNP 138 or SNP140, respectively, are preferentially not transmitted to autists.

Examples of haplotypes with preferential transmission to autists are given in Table 6.

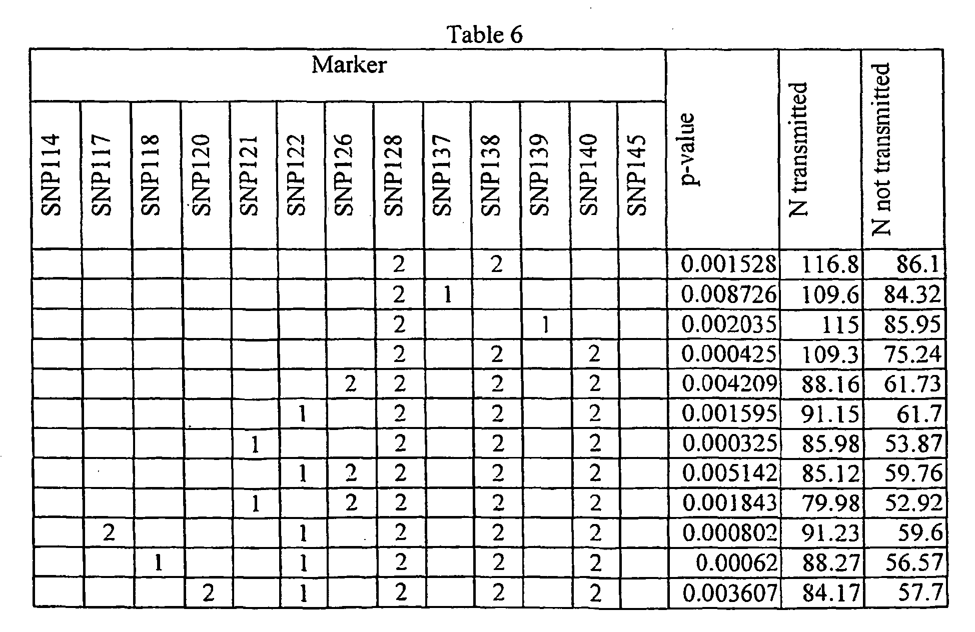

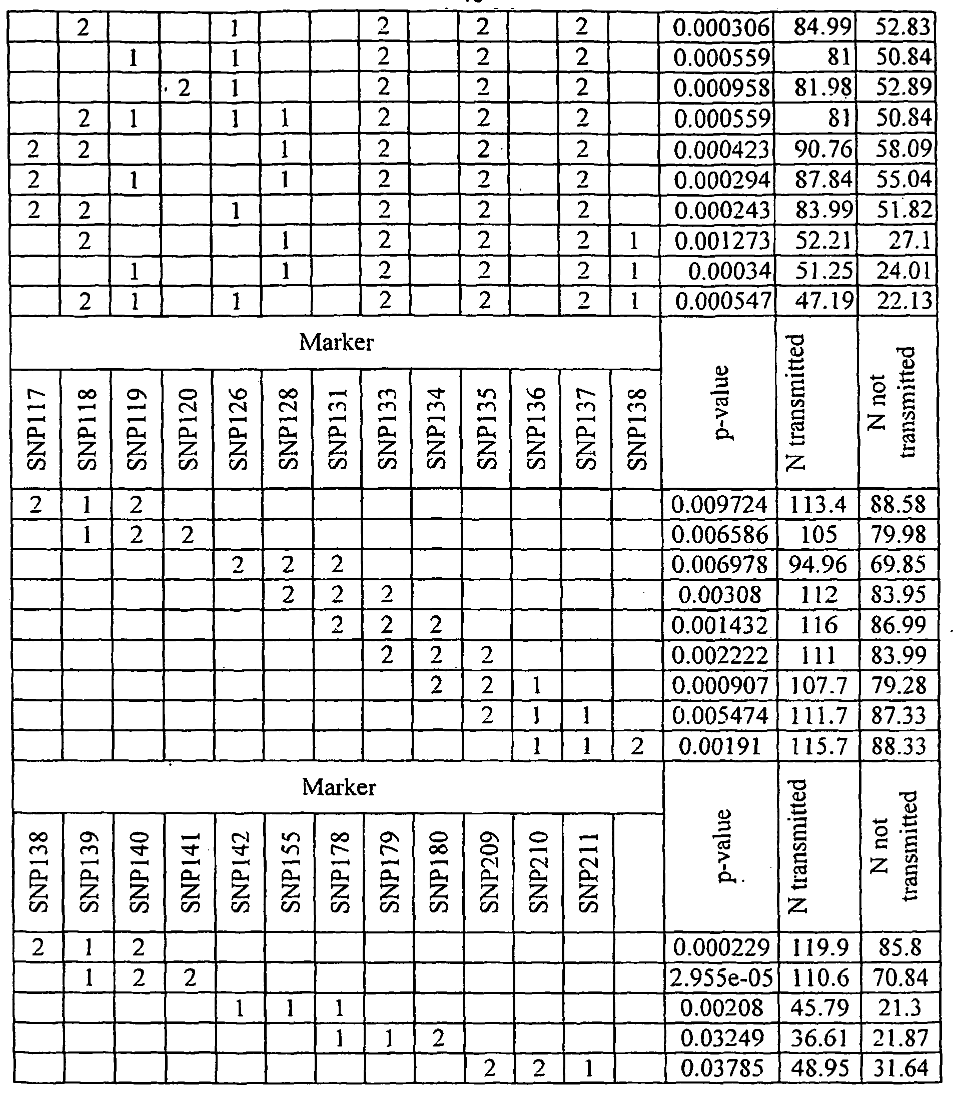

Examples of haplotypes with preferential non-transmission to autists are given in Table 7.

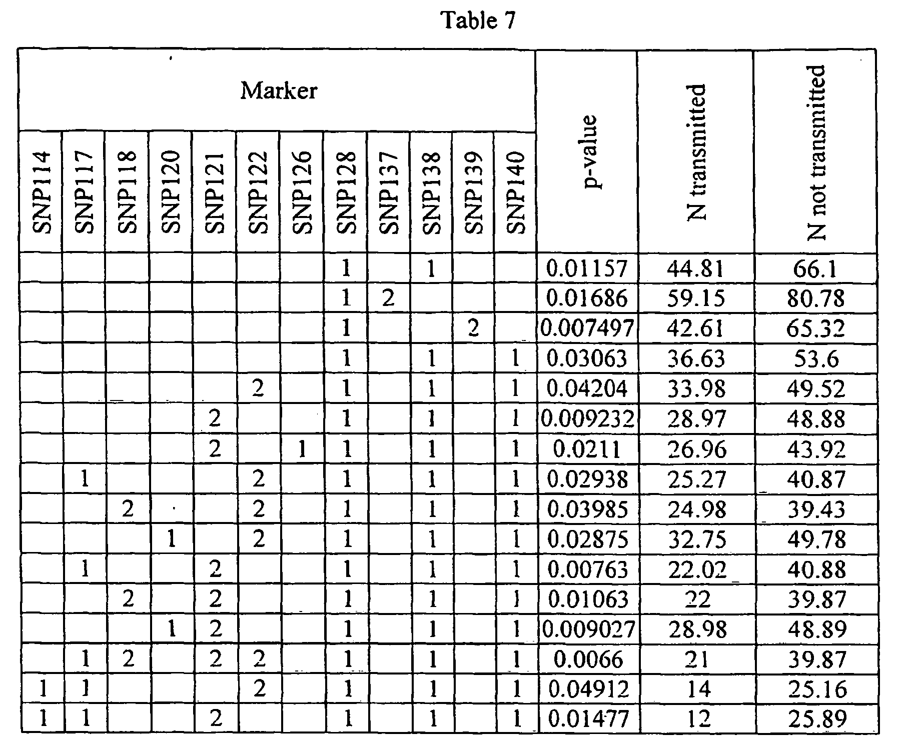

A second independent set of 167 trio families (set 2) was studied for replication of the association that has been observed in the families providing evidence for linkage (set 1).

Several single SNPs were found to be positively associated with autism in the independent set of trio families. For example, the allele 1 of marker SNP149 was more often transmitted to autists (p=0.01). On the contrary, the allele 2 of marker SNP149 was more often not transmitted to autists. Examples of alleles significantly more often transmitted or non-transmitted to autists are shown in Table 8.

**Table 8**

| Marker | Allele | N transmitted | N non transmitted | p |
|---|---|---|---|---|
| SNP149 | 1 | 147 | 116 | 0.01296 |
| | 2 | 176 | 207 | 0.01296 |
| SNP 180 | 1 | 146 | 174 | 0.02556 |
| | 2 | 169 | 141 | 0.02556 |
| SNP201 | 1 | 112 | 89 | 0.03932 |
| | 2 | 153 | 176 | 0.03932 |
| SNP209 | 1 | 66 | 46 | 0.03717 |
| | 2 | 256 | 276 | 0.03717 |
| SNP211 | 1 | 135 | 107 | 0.0226 |
| | 2 | 187 | 215 | 0.0226 |

Haplotypes were also constructed to determine the phase and analysed for association in the second set of independent trio families. The results of this analysis in the tested population showed that certain haplotypes are strongly associated with autism. The most significant result was obtained for haplotype 1-2-1 for markers SNP149, SNP150 and SNP 151 with a p-value of 8.7 x 10⁻³.

Examples of haplotypes with preferential transmission to autists are given in Table 9.

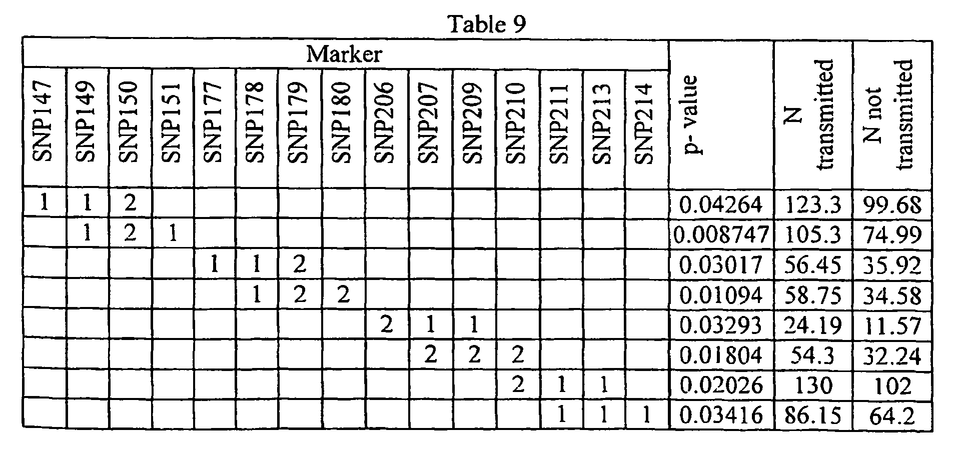

Both family sets include samples from different ethnic groups represented within the population of the USA. In order to adjust for any population stratification effects, the haplotype analysis was repeated for each ethnic group. Haplotype analysis in Caucasians only confirmed the association of haplotype 1-2-2 for markers SNP139, SNP140 and SNP141 in set 1 (p = 9.0 x 10⁻⁰³ ) and showed positive replication of this haplotype in set 2 (p = 3.6 x 10⁻³) as shown in Table 10 below.

**Table 10: Haplotype analysis of marker SNP139, SNP140 and SNP141 in Caucasians in families of set 1 and set 2.**

| **Set 1 Caucasian** | | | | | | |
|---|---|---|---|---|---|---|
| SNP # | SNP139 | SNP140 | SNP141 | T | NT | p-value |
| Allele | 1 | 2 | 2 | 60 | 41 | 0.009 |

| **Set 2 Caucasian** | | | | | | |
|---|---|---|---|---|---|---|
| SNP # | SNP139 | SNP140 | SNP141 | T | NT | p-value |
| Allele | 1 | 2 | 2 | 111 | 73 | 0.0036 |

### 4. Identification of nucleotide changes

96 unrelated affected individuals were included in the mutation screen. Primers to amplify the coding region of the PRKCB1 gene were obtained from Applied Biosystems. The positions of the primers corresponding to their position in sequence ID No. 63 are provided below in Table 11.

**Table 11**

| PCR primer | start position in sequence No. 63 | end position in sequence No. 63 |
|---|---|---|
| TR2 | 4 367 | 4 826 |
| TR3 | 155690 | 156160 |
| TR4 | 199 340 | 199 790 |
| TR6 | 259 840 | 260 390 |
| TR7 | 261360 | 261730 |
| TR9 | 291040 | 291490 |
| TR10 | 321 910 | 322460 |
| TR12 | 341710 | 342010 |
| TR13 | 347 990 | 348 520 |
| TR14-15 | 52310 | 353030 |
| TR16 | 358 187 | 358 724 |
| T17 | 381 880 | 382390 |
| TR18 | 387 160 | 387 470 |

The resulting amplification products were directly sequenced in both directions using dye-terminator sequencing chemistry to identify rare nucleotide changes (mutations) and polymorphisms (allele frequency >1%) in the gene.

A total of 29 nucleotide changes were detected in the coding region of the gene plus the flanking intron regions in close proximity of the splice sites (for positions see table 12). None of these resulted in changes of the amino-acids in the respective codons, as illustrated in table 12.

**Table 12**

| ID | Nucleotide position in Seq No ID | Alleles | Location of nucleotide variation in the gene | Variation and position in Seq No ID | Minor allele frequency |
|---|---|---|---|---|---|
| MUT1 | 4400 | G/T | intron 1-2 | | 45% |
| MUT2 | 4502 | G/A | intron 1-2, individual AU0203-5 homozygote A/A | | 2% |
| MUT3 | 4765 | G/A | intron 2-3, +34 bp from 3' splice site | | 48% |
| MUT4 | 155786 | C/T | intron 2-3, -45 bp from 5' splice site | | 3% |
| MUT5 | 155931 | G/T | intron 3-4, +16 bp from 3' splice site | | 1% |
| MUT6 | 156064 | T/G | intron 3-4 | | 3% |
| MUT7 | 199417 | T/C | intron 3-4, -41 bp from 5' splice site | | 1% |
| MUT8 | 199479 | G/A | exon 4 | K103K | 1% |
| MUT9 | 199658 | C/T | intron 4-5 | | 1% |
| MUT10 | 199678 | C/G | intron 4-5 | | 44% |
| MUT11 | 199789 | T/C | intron 4-5 | | 2% |
| MUT12 | 259978 | G/A | intron 4-5 | | 7% |
| MUT13 | 260189 | C/T | exon 6 | P202P | 5% |
| MUT14 | 260329 | A/G | intron 6-7, +59 bp from 3' splice site | | 43% |
| MUT15 | 260377 | C/A | intron 6-7 | | 2% |
| MUT16 | 261670 | G/A | intron 7-8, +50 bp from 3' splice site | | 49% |
| MUT17 | 291113 | T/C | intron 8-9, -43 bp from 5' splice site | | 36% |
| MUT18 | 322131 | T/C | exon 10 | P397P | 37% |
| MUT19 | 322341 | C/T | intron 10-11 | | 3% |
| MUT20 | 322449 | G/C | intron 10-11 | | 24% |
| MUT21 | 341998 | G/A | intron 12-13, individual AU0203-5 homozygote A/A | | 1% |
| MUT22 | 348108 | A/G | intron 12-13, individual AU0203-5 homozygote G/G | | 1% |
| MUT23 | 352393 | A/G | intron 13-14, -39 bp from 5' splice site | | 1 % |
| MUT24 | 352965 | C/T | intron 15-16 | | 1% |
| MUT25 | 358459 | C/T | exon 16 | G590G | 22% |
| MUT26 | 358616 | A/G | intron 16-16a | | 37% |
| MUT27 | 382266 | T/A | 3'UTR of isoform 1, intron of isoform 2 | | 4% |
| MUT28 | 382352 | A/G | 3'UTR of isoform 1, intron of isoform 2 | | 1% |
| MUT29 | 387249 | C/T | 3'UTR of isoform 1, intron of isoform 2 | | 8% |

Three mutations were detected in the 3' untranslated region of the transcript of the PRKCB1 isoform 1. These mutations could affect the stability of the RNA.

Three mutations, MUT2, MUT21 and MUT22, respectively, were detected only in inidividual AU0203-5. This individual carried only the mutant allele at these sites. However, this allele was not detected in any additional individual. As the mutation appears to be a rare event one would assume to detect a heterozygous genotype at this site unless the mutation has occurred independently at both chromosomes which appears to be extremely unlikely. However, homozygosity at these sites could be due to a deletion including these mutations.

### REFERENCES

Asperger H. (1944) Die autistischen Psychopathen im Kindesalter. Archiv für Psychiatrie und Nervenkrankheiten, 2:217-250.
Bailey A, Le Couteur A, Gottesman I, et al. (1995) Autism as a strongly genetic disorder: evidence from a British twin study. Psychol Med, 25:63-77.
Bailey A, Phillips W, Rutter M (1996) Autism: towards an integration of clinical, genetic, neuropsychological, and neurobiological perspectives. J Child Psychol Psychiatry 37(1):89-126.
Baird G, Charman T, Baron-Cohen S et al. (2000) A screening instrument for autism at 18 months of age: a 6-year follow-up study. J Am Acad Child Adolesc Psychiatry, 39(6):694-702.
Birikh, K.R., Sklan, E.H., Shoham, S., and Soreq, H. (2003). Interaction of "readthrough" acetylcholinesterase with RACK1 and PKCbeta II correlates with intensified fear-induced conflict behavior. Proc Natl Acad Sci U S A 100, 283-288.
Brandon, N., Jovanovic, J., and Moss, S. (2002). Multiple roles of protein kinases in the modulation of gamma-aminobutyric acid(A) receptor function and cell surface expression. Pharmacol Ther 94, 113-122.
Burger R and Warren R (1998) Possible immunogenetic basis for autism. Ment Retard Dev Disabil Res Rev, 4:137-141.
Carney RM, Wolpert CM, Ravan SA et al. (2003) Identification of MeCP2 mutations in a series of females with autistic disorder. Pediatr Neurol, 28(3):205-211.
Chakrabarti S and Fombonne E (2001) Pervasive developmental disorders in preschool children. JAMA, 285(24):3093-9
Comi AM, Zimmerman AW, Frye VH et al. (1999) Familial clustering of autoimmune disorders and evaluation of medical risk factors in autism. J Child Neurol, 14(6):388-394.
Connolly AM, Chez MG, Pestronk A et al. (1999) Serum autoantibodies to brain in Landau-Kleffner variant, autism, and other neurologic disorders. J Pediatr, 134(5):607-613.
Coussens, L., Parker, P.J., Rhee, L., Yang-Feng, T.L., Chen, E., Waterfield, M.D., Francke, U., and Ullrich, A. (1986). Multiple, distinct forms of bovine and human protein kinase C suggest diversity in cellular signaling pathways. Science 233, 859-866.
Folstein S and Rutter M (1977) Infantile autism: a genetic study of 21 twin pairs. J Child Psychol Psychiatry Allied Disciplines, 18:297-321.
Folstein SE and Rosen-Sheidley BR (2001) Genetics of autism: complex aetiology for a heterogeneous disorder. Nat Rev Genet, 2:943-955.
Gillberg C (1998) Chromosomal disorders and autism. J Autism Dev Disord, 28(S):415-425.
Gillberg C and Coleman M (2000) The biology of the autistic syndromes, 3rd edn London: MacKeith Press.
Gillberg C and Wing L (1999) Autism: not an extremely rare disorder. Acta Psychiatr Scand, 99:339-406.
Guadagno, S.N., Borner, C., and Weinstein, I.B. (1992). Altered regulation of a major substrate of protein kinase C in rat 6 fibroblasts overproducing PKC beta I. J Biol Chem 267,2697-2707.
Hugot JP, Chamaillard M, Zouali H et al. (2001) Association of NOD2 leucine-rich repeat variants with susceptibility to Crohn's disease. Nature 411(6837):599-603.
Jamain S, Quach H, Betancur C et al. (2003) Mutations of the X-linked genes encoding neuroligins NLGN3 and NLGN4 are associated with autism. Nat Genet, 34(1):27-29.
Jones AC, Sampson JR, Hoogendoorn B, Cohen D, Cheadle JP. (2000) Application and evaluation of denaturing HPLC for molecular genetic analysis in tuberous sclerosis. Hum Genet., 106(6):663-8.
Jorde LB, Hasstedt SJ, Ritvo ER et al. (1991) Complex segregation analysis of autism. Am J Hum Genet, 49(5):932-938.
Jorde LB, Mason-Brothers A, Waldmann R et al. (1990) The UCLA-University of Utah epidemiologic survey of autism: genealogical analysis of familial aggregation. Am J Med Genet, 36(1):85-88.
Kanner L (1943) Autistic disturbances of affective contact. Nervous Child, 2:217-250.
Lander E and Kruglyak L (1995) Genetic dissection of complex traits: guidelines for interpreting and reporting linkage results. Nat Genet, 11(3):241-247.
Le Couteur A, Rutter M, Lord C et al. (1989) Autism diagnostic interview: a standardized investigator-based instrument. J Autism Dev Disord, 19(3):363-357.
Lesage S, Zouali H, Cezard Jpet al. (2002) CARD15/NOD2 mutational analysis and genotype-phenotype correlation in 612 patients with inflammatory bowel disease. Am J Hum Genet. 70(4):845-857.
Lord C, Rutter M, Le Couteur A (1994) Autism Diagnostic Interview-Revised: a revised version of a diagnostic interview for caregivers of individuals with possible pervasive developmental disorders. J Autism Dev Disord, 24(5):659-685.
Manji, H.K., and Chen, G. (2002). PKC, MAP kinases and the bcl-2 family of proteins as long-term targets for mood stabilizers. Mol Psychiatry 7 Suppl 1, S46-56.
Martin, E.R., Menold, M.M., Wolpert, C.M., Bass, M.P., Donnelly, S.L., Ravan, S.A., Zimmerman, A., Gilbert, J.R., Vance, J.M., Maddox, L.O., Wright, H.H., Abramson, R.K., DeLong, G.R., Cuccaro, M.L., and Pericak-Vance, M.A. (2000a). Analysis of linkage disequilibrium in gamma-aminobutyric acid receptor subunit genes in autistic disorder. Am J Med Genet 96, 43-48.
Nelson KB (1991) Prenatal and perinatal factors in the etiology of autism. Pediatrics, 87(5 Pt 2):761-766.
Ogura Y, Bonen DK, Inohara N (2001) A framshift mutation in NOD2 associated with susceptibility to Crohn's disease. Nature 411(6837):603-606.
Rioux JD, Daly MJ, Silverberg MS et al. (2001) Genetic variation in the 5q31 cytokine gene cluster confers susceptibility to Crown disease. Nat Genet 29(2): 223-228.
Rioux JD, Silverberg MS, Daly MJ (2000) Genomewide search in Canadian families with inflammatory bowel disease reveals two novel susceptibility loci. Am J Hum Genet 66(6):1863-1870.
Rodier P and Hyman S (1998) Early environmental factors in autism. Mental Retard Dev Disord Res Rev, 4:121-128.
Singh VK, Warren RP, Odell JD et al. (1993) Antibodies to myelin basic protein in children with autistic behavior. Brain Behav Immun, 7(1):97-103.
Smalley SL (1997) Genetic influences in childhood-onset psychiatric disorders: autism and attention-deficit/hyperactivity disorder. Am J Hum Genet, 60(6): 1276-1282.
Steffenburg S, Gillberg C, Hellgren L et al. (1989) A twin study of autism in Denmark, Finland, Iceland, Norway and Sweden. J Child Psychol Psychiatry, 30(3):405-416.
Szatmari P, Jones MB, Zwaigenbaum L et al. (1998) Genetics of autism: overview and new directions. J Autism Dev Disord, 28(5):351-368.
Watterson, J.M., Watson, D.G., Meyer, E.M., and Lenox, R.H. (2002). A role for protein kinase C and its substrates in the action of valproic acid in the brain: implications for neural plasticity. Brain Res 934, 69-80.
Weizman A, Weizman R, Szekely GA et al. (1982) Abnormal immune response to brain tissue antigen in the syndrome of autism. Am J Psychiatry, 139(11):1462-1465.

## Claims

1. An *in vitro* method of detecting the presence of or predisposition to autism or to an autism spectrum disorder, in a subject, the method comprising detecting the presence of an alteration in the sequence of the PRKCB1 (protein kinase C beta-1) gene locus in a sample from the subject, wherein the alteration is a mutation of a base, an insertion of a base, or a deletion of one or more bases.

2. An *in vitro* method of detecting the protection from autism, or from an autism spectrum disorder, in a subject, the method comprising detecting the presence of an alteration in the sequence of the PRKCB1 (protein kinase C beta-1) gene locus in said sample in a sample from the subject, wherein the alteration is a mutation of a base, an insertion of a base, or a deletion of one or more bases.

3. An *in vitro* method of assessing the response of a subject to a treatment of autism or to an autism spectrum disorder the method comprising detecting the presence of an alteration in the sequence of the PRKCB1 (protein kinase C beta-1) gene locus in a sample from the subject, wherein the alteration is a mutation of a base, an insertion of a base, or a deletion of one or more bases.

4. An *in vitro* method of assessing the adverse effect in a subject to a treatment of autism or of an autism spectrum disorder the method comprising detecting the presence of an alteration in sequence of the the PRKCB1 (protein kinase C beta-1) gene locus in a sample from the subject, wherein the alteration is a mutation of a base, an insertion of a base, or a deletion of one or more bases.

5. The method of any one of claims 1-4, wherein the presence of an alteration in the PRKCB1 gene locus is detected by sequencing, oligonucleotide ligation, selective hybridisation and/or selective amplification.

6. The method of any one of claims 1-4, wherein said alteration is one or several SNP(s) or a haplotype of SNPs associated with autism.

7. The method of claim 6, wherein said SNP(s) are selected from the group consisting of those disclosed in Tables 1a and 1b, more preferably those disclosed in Tables 3-10.

8. The method of claim 7, wherein said SNP(s) associated with autism are selected from the group consisting of SNP106, SNP134, SNP128, SNP138, SNP140 and SNP149, as identified below
| Nucleotide position in genomic sequence of chromosome 16 (Build34) | SNP identity | dbSNP or Celera reference | Polymorphism | Position in locus and type of amino acid change | Sequence reference |
|---|---|---|---|---|---|
| 23870016 | SNP106 | rs2878156 | C/T | PRKCB1 intron | 8 |
| Nucleotide position in genomic sequence of chromosome 16 (Build34) | SNP identity | dbSNP or Celera reference | Polymorphism | Sequence reference |
|---|---|---|---|---|
| 23870016 | SNP128 | hCV2192108/rs2878156 | C=1/T=2 | 21 |
| 23898266 | SNP134 | C_2192130_10/rs2188356 | C=1/G=2 | 24 |
| 23925972 | SNP138 | rs3890662 | A=1/G=2 | 28 |
| 23929790 | SNP140 | hCV946275/rs196002 | A=1/G=2 | 30 |
| 23981619 | SNP149 | hCV9609165/rs1490754 | C=1/T=2 | 35 |

9. The method of claim 7, wherein said haplotype associated with autism comprises or consists of several SNPs selected from the group consisting of SNP106, SNP134, SNP128, SNP 138, SNP 140, SNP 139, SNP 141, SNP 149, SNP 150 and SNP 151
| Nucleotide position in genomic sequence of chromosome 16 (Build34) | SNP identity | dbSNP or Celera reference | Polymorphism | Position in locus and type of amino acid change | Sequence reference |
|---|---|---|---|---|---|
| 23870016 | SNP106 | rs2878156 | C/T | PRKCB1 intron | 8 |
| Nucleotide position in genomic sequence of chromosome 16 (Build34) | SNP identity | dbSNP or Celera reference | Polymorphism | Sequence reference |
|---|---|---|---|---|
| 23870016 | SNP128 | hCV2192108/rs2878156 | C=1/T=2 | 21 |
| 23925972 | SNP138 | rs3890662 | A=1/G=2 | 28 |
| 23928846 | SNP139 | rs3785387 | C=1/T=2 | 29 |
| 23929790 | SNP140 | hCV946275/rs196002 | A=1/G=2 | 30 |
| 23937230 | SNP141 | rs1873423 | C=1/T=2 | 31 |
| 23981619 | SNP149 | hCV9609165/rs1490754 | C=1/T=2 | 35 |
| 23984187 | SNP150 | rs195992 | C=1/T=2 | 36 |
| 23986260 | SNP151 | rs6497712 | A=1/G=2 | 37 |

10. The method of claim 9, wherein said haplotype consists of or comprises SNP139, SNP140 and SNP141, preferably with the alleles C-G-T.

11. The method of claim 9, wherein said haplotype consists of or comprises SNP149, SNP150 and SNP151, preferably with the alleles C-T-A.

12. The method of claim 6, wherein said haplotype associated with autism said haplotype is selected from the haplotypes disclosed in Tables 4, 6, 7, 9 and/or 10.

13. An *in vitro* method of selecting biologically active compounds on autism or autism spectrum disorders, said method comprising contacting a test compound with a PRKCB1 (protein kinase C beta-1) polypeptide or gene and determining the ability of said test compound to bind the PRKCB1 polypeptide or gene.

14. An *in vitro* method of selecting biologically active compounds on autism or autism spectrum disorders, said method comprising contacting a recombinant host cell expressing a PRKCB1 (protein kinase C beta-1) polypeptide with a test compound, and determining the ability of said test compound to bind said PRKCB1 polypeptide and to modulate the activity of PRKCB1 polypeptide.

15. An *in vitro* method of selecting biologically active compounds on autism or autism spectrum disorders, said method comprising contacting a test compound with a PRKCB1 (protein kinase C beta-1) gene and determining the ability of said test compound to modulate the expression of said PRKCB1 gene.

16. An *in vitro* method of selecting biologically active compounds on autism or autism spectrum disorders,said method comprising contacting a test compound with a recombinant host cell comprising a reporter construct, said reporter construct comprising a reporter gene under the control of a PRKCB1 (protein kinase C beta-1) gene promoter, and selecting the test compounds that modulate (e.g. stimulate or reduce) expression of the reporter gene.

17. Method according any one of claims 13-16, wherein said PRKCB1 gene or polypeptide is an altered or mutated PRKCB1 gene or polypeptide comprising the alteration or mutation, wherein the alteration is a mutation of a base, an insertion of a base, or a deletion of one or more bases.

18. Method according any one of claims 13-17, wherein said modulation is an activation.

19. Method according any one of claims 13-17, wherein said modulation is an inhibition.

## Patentansprüche

1. Ein *in vitro*-Verfahren zum Nachweis von oder Prädisposition für Autismus oder einer Autismus-Spektrum-Störung in einem Patienten, wobei das Verfahren umfasst: Nachweis der Gegenwart einer Veränderung in der Sequenz des PRKCB1 (Proteinkinase C beta-1)-Genlocus in einer Probe des Patienten, wobei die Veränderung eine Mutation einer Base, eine Insertion einer Base oder eine Deletion einer oder mehrerer Basen ist.

2. Ein *in vitro*-Verfahren zum Nachweis des Schutzes vor Autismus oder vor einer Autismus-Spektrum-Störung in einem Patienten, wobei das Verfahren umfasst: Nachweis der Gegenwart einer Veränderung in der Sequenz des PRKCB1 (Proteinkinase C beta-1)-Genlocus in der Probe des Patienten, wobei die Veränderung eine Mutation einer Base, eine Insertion einer Base oder eine Deletion einer oder mehrerer Basen ist.

3. Ein *in vitro*-Verfahren zum Feststellen der Reaktion bei einem Patienten zur Behandlung von Autismus oder einer Autismus-Spektrum-Störung, wobei das Verfahren umfasst: Nachweis der Gegenwart einer Veränderung in der Sequenz des PRKCB1 (Proteinkinase C beta-1)-Genlocus in einer Probe des Patienten in einer Probe des Patienten, wobei die Veränderung eine Mutation einer Base, eine Insertion einer Base oder eine Deletion einer oder mehrerer Basen ist.

4. Ein *in vitro*-Verfahren zum Feststellen der Gegenwirkung in einem Patienten bei einer Behandlung von Autismus oder einer Autismus-Spektrum-Störung, wobei das Verfahren umfasst: Nachweisen der Gegenwart einer Veränderung in der Sequenz des PRKCB1 (Proteinkinase C beta-1)-Genlocus in einer Probe des Patienten, wobei die Veränderung eine Mutation einer Base, eine Insertion einer Base oder eine Deletion einer oder mehrerer Basen ist.

5. Das Verfahren nach einem der Ansprüche 1 bis 4, wobei die Gegenwart einer Veränderung im PRKCB1-Genlocus durch Sequenzieren, Oligonukleotid-Ligation, selektive Hybridisierung und/oder selektive Amplifikation nachgewiesen wird.

6. Das Verfahren nach einem der Ansprüche 1 bis 4, wobei die Veränderung ein oder einige SNP(s) oder ein Haplotyp von SNP(s), die mit Autismus assoziiert sind, ist.

7. Das Verfahren nach Anspruch 6, wobei die SNP(s) aus der Gruppe ausgewählt werden, die aus denen besteht, die in den Tabellen 1a und 1b offenbart sind, mehr bevorzugt diese die in den Tabellen 3 bis 10 offenbart sind.

8. Das Verfahren nach Anspruch 7, wobei die SNP(s), die mit Autismus assoziiert sind, aus der Gruppe ausgewählt werden bestehend aus SNP106, SNP134, SNP128, SNP138, SNP140 und SNP149, wie nachstehend identifiziert sind
| Nukleotid-Position in der genomischen Sequenz von Chromosom 16 (Build34) | SNP-Identität | dbSNP oder Celera-Referenz | Polymorphismus | Position im Locus and Art der Aminosäure-Änderung | Sequenz-Referenz |
|---|---|---|---|---|---|
| 23870016 | SNP106 | rs2878156 | C/T | PRKCB1 intron | 8 |
| Nukleotid-Position in der genomischen Sequenz von Chromosom 16 (Build34) | SNP-Identität | dbSNP oder Celera-Referenz | Polymorphismus | Sequenz-Referenz |
|---|---|---|---|---|
| 23870016 | SNP128 | hCV2192108/rs2878156 | C=1/T=2 | 21 |
| 23898266 | SNP134 | C_2192130_10/rs2188356 | C=1/G=2 | 24 |
| 23925972 | SNP138 | rs3890662 | A=1/G=2 | 28 |
| 23929790 | SNP140 | hCV946275/rs196002 | A=1/G=2 | 30 |
| 23981619 | SNP149 | hCV9609165/rs1490754 | C=1/T=2 | 35 |

9. Das Verfahren nach Anspruch 7, wobei der Haplotyp, der mit Autismus assoziiert ist, umfasst oder besteht aus einigen SNP(s), aus der Gruppe ausgewählt bestehend aus SNP106, SNP134, SNP128, SNP138, SNP140, SNP139, SNP141, SNP149, SNP150, und SNP151
| Nukleotid-Position in der genomischen Sequenz von Chromosom 16 (Build34) | SNP-Identität | dbSNP oder Celera-Referenz | Polymorphismus | Position im Locus and Art der Aminosäure-Änderung | Sequenz-Referenz |
|---|---|---|---|---|---|
| 23870016 | SNP106 | rs2878156 | C/T | PRKCB1 intron | 8 |
| Nukleotid-Position in der genomischen Sequenz von Chromosom 16 (Build34) | SNP-Identität | dbSNP oder Celera-Referenz | Polymorphismus | Sequenz-Referenz |
|---|---|---|---|---|
| 23870016 | SNP128 | hCV2192108/rs2878156 | C=1/T=2 | 21 |
| 23925972 | SNP138 | rs3890662 | A=1/G=2 | 28 |
| 23928846 | SNP139 | rs3785387 | C=1/T=2 | 29 |
| 23929790 | SNP140 | hCV946275/rs196002 | A=1/G=2 | 30 |
| 23937230 | SNP141 | rs1873423 | C=1/T=2 | 31 |
| 23981619 | SNP149 | hCV9609165/rs1490754 | C=1/T=2 | 35 |
| 23984187 | SNP150 | rs195992 | C=1/T=2 | 36 |
| 23986260 | SNP151 | rs6497712 | A=1/G=2 | 37 |

10. Das Verfahren nach Anspruch 9, wobei der Haplotyp besteht aus oder umfasst SNP139, SNP140 und SNP141, vorzugsweise mit den Allelen C-G-T.

11. Das Verfahren nach Anspruch 9, wobei der Haplotyp besteht aus oder umfasst SNP149, SNP150 und SNP151, vorzugsweise mit den Allelen C-T-A.

12. Das Verfahren nach Anspruch 6, wobei der Haplotyp, der mit Autismus assoziiert ist, aus den Haplotypen ausgewählt ist, die in den Tabellen 4, 6, 7, 9 und/oder 10 offenbart sind.

13. Ein *in* vitro-Verfahren zum Auswählen biologisch aktiver Verbindungen für Autismus oder Autismus-Spektrum-Störungen, wobei das Verfahren umfasst: In Kontakt bringen einer Testverbindung mit einem PRKCB1 (Proteinkinase C beta-1)-Polypeptid oder -Gen und Bestimmen der Fähigkeit der Testverbindung das PRKCB1-Polypeptid oder -Gen zu binden.

14. Ein *in vitro*-Verfahren zum Auswählen biologisch aktiver Verbindungen für Autismus oder Autismus-Spektrum-Störungen, wobei das Verfahren umfasst: In Kontakt bringen einer rekombinanten Wirtszelle, die ein PRKCB1 (Proteinkinase C beta-1)-Polypeptid exprimiert, mit einer Testverbindung und Bestimmen der Fähigkeit der Testverbindung das PRKCB1-Polypeptid zu binden und die Aktivität des PRKCB1-Polypeptids zu modulieren.

15. Ein *in* vitro-Verfahren zum Auswählen biologisch aktiver Verbindungen für Autismus oder Autismus-Spektrum-Störungen, wobei das Verfahren umfasst: In Kontakt bringen einer Testverbindung mit einem PRKCB1 (Proteinkinase C beta-1)-Gen und Bestimmen der Fähigkeit der Testverbindung die Expression des PRKCB1-Gens zu modulieren.

16. Ein *in* vitro-Verfahren zum Auswählen biologisch aktiver Verbindungen für Autismus oder Autismus-Spektrum-Störungen, wobei das Verfahren umfasst: In Kontakt bringen einer Testverbindung mit einer rekombinanten Wirtszelle, die ein Reporterkonstrukt umfasst, wobei das Reporterkonstrukt ein Reportergen unter der Kontrolle eines PRKCB1 (Proteinkinase C beta-1)-Genpromotors umfasst, und Auswählen der Testverbindung, die die Expression des Reportergens moduliert (z. B. stimuliert oder reduziert).

17. Verfahren nach einem der vorhergehenden Ansprüche 13 bis 16, wobei das PRKCB1-Gen oder -Polypeptid ein verändertes oder mutiertes PRKCB1-Gen oder Polypeptid ist, umfassend die Veränderung oder Mutation, wobei die Veränderung eine Mutation einer Base, eine Insertion einer Base oder eine Deletion einer oder mehrerer Basen ist.

18. Verfahren nach einem der Ansprüche 13 bis 17, wobei die Modulation eine Aktivierung ist.

19. Verfahren nach einem der Ansprüche 13 bis 17, wobei die Modulation eine Inhibition ist.

## Revendications

1. Méthode *in vitro* pour détecter la présence de ou la prédisposition à l'autisme ou un trouble du spectre de l'autisme chez un sujet, la méthode comprenant la détection de la présence d'une altération dans la séquence du locus du gène PRKCB1 (protéine kinase C bêta-1) dans un échantillon du sujet, dans laquelle l'altération est une mutation d'une base, une insertion d'une base ou une délétion d'une ou plusieurs bases.

2. Méthode *in vitro* pour détecter la protection contre l'autisme ou un trouble du spectre de l'autisme chez un sujet, la méthode comprenant la détection de la présence d'une altération dans la séquence du locus du gène PRKCB1 (protéine kinase C bêta-1) dans un échantillon du sujet, dans laquelle l'altération est une mutation d'une base, une insertion d'une base ou une délétion d'une ou plusieurs bases.

3. Méthode *in vitro* pour évaluer la réponse d'un sujet à un traitement contre l'autisme ou un trouble du spectre de l'autisme, la méthode comprenant la détection de la présence d'une altération dans la séquence du locus du gène PRKCB1 (protéine kinase C bêta-1) dans un échantillon du sujet, dans laquelle l'altération est une mutation d'une base, une insertion d'une base ou une délétion d'une ou plusieurs bases.

4. Méthode *in vitro* pour évaluer un effet secondaire chez un sujet d'un traitement contre l'autisme ou un trouble du spectre de l'autisme, la méthode comprenant la détection de la présence d'une altération dans la séquence du locus du gène PRKCB1 (protéine kinase C bêta-1) dans un échantillon du sujet, dans laquelle l'altération est une mutation d'une base, une insertion d'une base ou une délétion d'une ou plusieurs bases.

5. Méthode selon l'une quelconque des revendications 1-4, dans laquelle la présence d'une altération dans le locus du gène PRKCB1 est détectée par séquençage, ligation d'oligonucléotides, hybridation sélective et/ou amplification sélective.

6. Méthode selon l'une quelconque des revendications 1-4, dans laquelle ladite altération est un ou plusieurs SNP(s) ou un haplotype de SNPs associés à l'autisme.

7. Méthode selon la revendication 6, dans laquelle le(s)dit(s) SNP(s) sont sélectionnés parmi le groupe consistant en ceux décrits dans les Tableaux 1a et 1b, de préférence ceux décrits dans les Tableaux 3-10.

8. Méthode selon la revendication 7, dans laquelle le(s)dit(s) SNP(s) associé(s) à l'autisme sont sélectionnés parmi le groupe consistant en SNP106, SNP134, SNP128, SNP 13 8, SNP 140 et SNP 149, tels que décrits ci-dessous
| Position nucléotidique dans la séquence génomique du chromosome 16 (Build34) | Identité du SNP | Référence de dbSNP ou Celera | Polymorphisme | Position dans le locus et type de changement d'acide aminé | Référence de séquence |
|---|---|---|---|---|---|
| 23870016 | SNP106 | rs2878156 | C/T | intron de PRKCB1 | 8 |
| Position nucléotidique dans la séquence génomique du chromosome 16 (Build34) | Identité du SNP | Référence de dbSNP ou Celera | Polymorphism ee | Référence de séquence |
|---|---|---|---|---|
| 23870016 | SNP128 | hCV2192108/rs2878156 | C=1/T=2 | 21 |
| 23898266 | SNP134 | C_2192130_10/rs2188356 | C=1/G=2 | 24 |
| 23925972 | SNP138 | rs3890662 | A=1/G=2 | 28 |
| 23929790 | SNP140 | hCV946275/rs196002 | A=1/G=2 | 30 |
| 23981619 | SNP149 | hCV9609165/rs1490754 | C=1/T=2 | 35 |

9. Méthode selon la revendication 7, dans laquelle ledit haplotype associé à l'autisme comprend ou consiste en plusieurs SNPs sélectionnés parmi le groupe consistant en SNP106, SNP134, SNP128, SNP138, SNP140, SNP139, SNP141, SNP149, SNP150 and SNP151
| Position nucléotidique dans la séquence génomique du chromosome 16 (Build34) | Identité du SNP | Référence de dbSNP ou Celera | Polymorphisme | Position dans le locus et type de changement d'acide aminé | Référence de séquence |
|---|---|---|---|---|---|
| 23870016 | SNP106 | rs2878156 | C/T | intron de PRKCB1 | 8 |
| Position nucléotidique dans la séquence génomique du chromosome 16 (Build34) | Identité du SNP | Référence de dbSNP ou Celera | Polymorphism e | Référence de séquence |
|---|---|---|---|---|
| 23870016 | SNP128 | hCV2192108/rs2878156 | C=1/T=2 | 21 |
| 23925972 | SNP138 | rs3890662 | A=1/G=2 | 28 |
| 23928846 | SNP139 | rs3785387 | C=1/T=2 | 29 |
| 23929790 | SNP140 | hCV946275/rs196002 | A=1/G=2 | 30 |
| 23937230 | SNP141 | rs1873423 | C=1/T=2 | 31 |
| 23981619 | SNP149 | hCV9609165/rs1490754 | C=1/T=2 | 35 |
| 23984187 | SNP150 | rs195992 | C=1/T=2 | 36 |
| 23986260 | SNP151 | rs6497712 | A=1/G=2 | 37 |

10. Méthode selon la revendication 9, dans laquelle ledit haplotype consiste en ou comprend SNP139, SNP140 et SNP141, de préférence avec les allèles C-G-T.

11. Méthode selon la revendication 9, dans laquelle ledit haplotype consiste en ou comprend SNP 149, SNP 15 0 et SNP151, de préférence avec les allèles C-T-A.

12. Méthode selon la revendication 6, dans laquelle ledit haplotype associé à l'autisme est sélectionné parmi les haplotypes décrits dans les Tableaux 4, 6, 7, 9 et/ou 10.

13. Méthode *in vitro* pour sélectionner des composés biologiquement actifs sur l'autisme ou des troubles de spectre de l'autisme, ladite méthode comprenant la mise en contact d'un composé test avec un polypeptide ou un gène PRKCB1 (protéine kinase C bêta-1) et la détermination de la capacité dudit composé test à se lier au polypeptide ou gène PRKCB 1.

14. Méthode *in vitro* pour sélectionner des composés biologiquement actifs sur l'autisme ou des troubles de spectre de l'autisme, ladite méthode comprenant la mise en contact d'une cellule hôte recombinante exprimant un polypeptide PRKCB1 (protéine kinase C bêta-1) avec un composé test et la détermination de la capacité dudit composé test à se lier au polypeptide PRKCB1 et de moduler l'activité du polypeptide PRKCB1.

15. Méthode *in vitro* pour sélectionner des composés biologiquement actifs sur l'autisme ou des troubles de spectre de l'autisme, ladite méthode comprenant la mise en contact d'un composé test avec un gène PRKCB1 (protéine kinase C bêta-1) et la détermination de la capacité dudit composé test à moduler l'expression dudit gène PRKCB1.

16. Méthode *in vitro* pour sélectionner des composés biologiquement actifs sur l'autisme ou des troubles de spectre de l'autisme, ladite méthode comprenant la mise en contact d'un composé test avec une cellule hôte recombinante comprenant une construction rapporteur, ladite construction rapporteur comprenant un gène rapporteur sous le contrôle du promoteur du gène PRKCB1 (protéine kinase C bêta-1) et la sélection du composé test qui module (par exemple stimule ou diminue) l'expression du gène rapporteur.

17. Méthode selon l'une quelconque des revendications 13-16, dans laquelle ledit gène ou polypeptide PRKCB1 est un gène ou polypeptide PRKCB1 altéré ou muté comprenant une altération ou mutation, ladite altération étant une mutation d'une base, une insertion d'une base ou une délétion d'une ou plusieurs bases.

18. Méthode selon l'une quelconque des revendications 13-17, dans laquelle ladite modulation est une activation.

19. Méthode selon l'une quelconque des revendications 13-17, dans laquelle ladite modulation est une inhibition.
